# EUROPEAN PATENT SPECIFICATION

(11) **EP 3 806 888 B1**
(45) Date of publication and mention of the grant of the patent: **31.01.2024**
(21) Application number: 19819609.9
(22) Date of filing: 12.06.2019
(51) Int. Cl.: A61K 38/17, C07K 14/705, C07K 16/28, C07K 19/00, C12N 15/09, C12N 15/12, A61K 39/00, A61K 38/00, A61P 35/00, C07K 14/725, C12N 9/16

(54) **PDE5 DERIVED REGULATORY CONSTRUCTS AND METHODS OF USE IN IMMUNOTHERAPY**
VON PDE5 ABGELEITETE REGULATORISCHE KONSTRUKTE UND VERFAHREN ZUR VERWENDUNG IN DER IMMUNTHERAPIE
CONSTRUCTIONS RÉGULATRICES DÉRIVÉES DE PDE5 ET PROCÉDÉS D'UTILISATION EN IMMUNOTHÉRAPIE

(30) Priority: 12.06.2018 US 201862683972 P; 06.11.2018 US 201862756124 P; 11.12.2018 US 201862777893 P; 18.04.2019 US 201962835545 P
(43) Date of publication of application: 21.04.2021
(73) Proprietor: Obsidian Therapeutics, Inc., Cambridge, MA 02138 (US)
(72) Inventor: SURI, Vipin, Belmont, Massachusetts 02478 (US); RICHARDSON, Celeste, Brookline, Massachusetts 02467 (US); DOLINSKI, Brian, Cambridge, Massachusetts 02138 (US); KULKARNI, Abhishek, Brookline, Massachusetts 02445 (US); INNISS, Mara Christine, Beverly, Massachusetts 01915 (US); SUN, Dexue, Cambridge, Massachusetts 02138 (US); LI, Dan Jun, Cambridge, Massachusetts 02138 (US); OLINGER, Grace Y., Cambridge, Massachusetts 02141 (US); HELLER, Scott Francis, Stoughton, Massachusetts 02072 (US); GORI, Jennifer Leah, Jamaica Plain, Massachusetts 02130 (US); DELABARRE, Byron, Arlington, Massachusetts 02474 (US)
(74) Representative: Carpmaels & Ransford LLP
(86) International application number: PCT/US2019/036654
(87) International publication number: WO 2019/241315

(56) References cited:
- WO-A1-2017/024318
- WO-A1-2018/231759
- WO-A1-2018/237323
- WO-A2-2017/180587
- WO-A2-2017/210617
- US-A1- 2005 048 573
- US-A1- 2009 087 871
- US-A1- 2014 255 361
- ALEXANDRE JUILLERAT ET AL: "Design of chimeric antigen receptors with integrated controllable transient functions", SCIENTIFIC REPORTS, vol. 6, 11 January 2016 (2016-01-11), pages 1-7, XP055271820, DOI: 10.1038/srep18950 Retrieved from the Internet: URL:https://www.nature.com/articles/srep18 950>
- YUSUKE MIYAZAKI ET AL: "Destabilizing Domains Derived from the Human Estrogen Receptor", JOURNAL OF THE AMERICAN CHEMICAL SOCIETY, vol. 134, no. 9, 7 March 2012 (2012-03-07) , pages 3942-3945, XP055387238, ISSN: 0002-7863, DOI: 10.1021/ja209933r

## Description

### CROSS REFERENCE TO RELATED APPLICATIONS

This application claims priority to the US Provisional Patent Application No. 62/835,545 filed April 18, 2019 entitled PDE5 DERIVED REGULATORY CONSTRUCTS AND METHODS OF USE IN IMMUNOTHERAPY, the US Provisional Patent Application No. 62/777,893 filed December 11, 2018 entitled PDE5 DERIVED REGULATORY CONSTRUCTS AND METHODS OF USE IN IMMUNOTHERAPY, the US Provisional Patent Application No. 62/756,124 filed November 6, 2018 entitled PDE5 DERIVED REGULATORY CONSTRUCTS AND METHODS OF USE IN IMMUNOTHERAPY, and the US Provisional Patent Application No. 62/683,972 filed June 12, 2018 entitled PDE5 DERIVED REGULATORY CONSTRUCTS AND METHODS OF USE IN IMMUNOTHERAPY.

### REFERENCE TO THE SEQUENCE LISTING

The present application is being filed along with a Sequence Listing in electronic format. The Sequence Listing is provided as a file entitled 2095_1008PCT_SL.txt, created on June 11, 2019, which is 20,431,626 bytes in size.

### FIELD

The present disclosure relates to tunable biocircuit systems for the development of controlled and/or regulated therapeutic systems. In particular, regulatable biocircuits containing destabilizing domains (DD) derived from mutant human cGMP-specific phosphodiesterase type 5 (PDE5) are disclosed. The present disclosure also relates to compositions and methods for immunotherapy.

Provided in the present disclosure include polypeptides of biocircuit systems, effector modules, stimulus response elements (SREs) and immunotherapeutic agents, polynucleotides encoding the same, vectors and cells containing the polypeptides and/or polynucleotides for use in cancer immunotherapy. In one embodiment, the compositions comprise destabilizing domains (DDs) which tune protein stability.

### BACKGROUND

Safe and effective gene therapy requires tightly regulated expression of a therapeutic transgenic product (e.g., the protein product). Similarly, the analysis of gene function in development, cell differentiation and other physiological activities requires the controllable expression of a protein under investigation. However, current technologies do not allow titration of levels of target protein induced or kinetics of induction. Inadequate exogenous and/or endogenous gene control is a critical issue in numerous settings including *ex vivo* and *in vivo* gene therapy. This lack of tunability also makes it difficult to safely express proteins with narrow or uncertain therapeutic windows or those requiring more titrated, controlled or transient expression.

One approach to regulated protein expression or function is the use of Destabilizing Domains (DDs). Destabilizing domains are small protein domains that can be appended to a payload such as a protein of interest (POI). DDs render the attached protein of interest (POI) unstable in the absence of a DD-binding ligand such that the protein is rapidly degraded by the ubiquitin-proteasome system of the cell (Stankunas, K., et al., (2003). Mol. Cell 12, 1615-1624; Banaszynski, L.A., et al., (2006) Cell; 126(5): 995-1004; reviewed in Banaszynski, L.A., and Wandless, T.J. (2006) Chem. Biol. 13, 11-21; Iwamoto, M., et al. (2010). Chem Biol. 17(9):981-8; Egeler, E.L.et al. (2011). J Biol Chem. 286(36):31328-36; and Rakhit R, Navarro R, Wandless TJ (2014) Chem Biol. Sep 18;21(9):1238-52; Navarro, R. et al. (2016) ACS Chem Biol. 11(8): 2101-2104). In some cases, the protein of interest is not completely processed and may not be secreted or presented on the membrane in the absence of DD-binding ligand (Sellmeyer et al., (2012), doi.org/10.1371/journal.pone.0043297). However, when a specific small molecule ligand binds its intended DD as a ligand binding partner, the instability is reversed and protein function is restored or, in some cases, processing is restored and the protein of interest is presented on the membrane or secreted. Such a system is herein referred to as a biocircuit, with the canonical DD-containing biocircuit described above being the prototypical model biocircuit

It is believed that improvements of biocircuits, including those containing DDs can form the basis of a new class of cell and gene therapies that employ tunable and temporal control of gene expression and function. Such novel moieties are described by the present inventors as stimulus response elements (SREs) which act in the context of an effector module to complete a biocircuit arising from a stimulus and ultimately producing a signal or outcome. When properly formatted with a polypeptide payload, and when activated by a particular stimulus, e.g., a small molecule, biocircuit systems can be used to regulate transgene and/or protein levels either up or down by perpetuating a stabilizing signal or destabilizing signal. This approach has many advantages over existing methods of regulating protein function and/or expression, which are currently focused on top level transcriptional regulation via inducible promoters.

The present disclosure provides novel protein domains, in particular, destabilizing domains (DDs) derived from mutant human cGMP-specific phosphodiesterase type 5 (PDE5), particularly the catalytic domain of human PDE5, that display small molecule dependent stability, and the biocircuit systems and effector modules comprising such DDs. Methods for tuning transgene functions using the same are also provided.

Cancer immunotherapy aims to eradicate cancer cells by rejuvenating the tumoricidal functions of tumor-reactive immune cells, predominantly T cells. Strategies of cancer immunotherapy including the recent development of checkpoint blockade, adoptive cell transfer (ACT) and cancer vaccines which can increase the anti-tumor immune effector cells have produced remarkable results in several tumors.

The impact of host anti-tumor immunity and cancer immunotherapy is impeded by three major hurdles: 1) low number of tumor antigen-specific T cells due to clonal deletion; 2) poor activation of innate immune cells and accumulation of tolerogenic antigen-presenting cells in the tumor microenvironment; and 3) formation of an immunosuppressive tumor microenvironment. Particularly, in solid tumors the therapeutic efficacy of immunotherapeutic regimens remains unsatisfactory due to lack of an effective an anti-tumor response in the immunosuppressive tumor microenvironment. Tumor cells often induce immune tolerance or suppression and such tolerance is acquired because even truly foreign tumor antigens will become tolerated. Such tolerance is also active and dominant because cancer vaccines and adoptive transfer of pre-activated immune effector cells (e.g., T cells), are subject to suppression by inhibitory factors in the tumor microenvironment (TME).

In addition, administration of engineered T cells could result in on/off target toxicities as well as a cytokine release syndrome (reviewed by Tey Clin. Transl. Immunol., 2014, 3: e17 10.1038).

Development of a tunable switch that can turn on or off the transgenic immunotherapeutic agent expression is needed in case of adverse events. For example, adoptive cell therapies may have a very long and an indefinite half-life. Since toxicity can be progressive, a safety switch is desired to eliminate the infused cells. Systems and methods that can tune the transgenic protein level and expression window with high flexibility can enhance therapeutic benefit, and reduce potential side effects.

In an effort to develop regulatable therapeutic agents for disease therapy, in particular cancer immunotherapy, the present disclosure provides biocircuit systems to control the expression of immunotherapeutic agents. The biocircuit system comprises a stimulus and at least one effector module that responds to the stimulus. The effector module may include a stimulus response element (SRE) that binds and is responsive to a stimulus and an immunotherapeutic agent operably linked to the SRE. In one example, a SRE is a destabilizing domain (DD) which is destabilized in the absence of its specific ligand and can be stabilized by binding to its specific ligand.

WO 2018/231759 describes PDE5 compositions and methods for immunotherapy. Juillerat et al. (2016) Scientici Reports, 6: 1-7 describes design of chimeric antigen receptors with integrated controllable transient functions. Miyazaki et al. (2012) Journal of the American Chemical Society, 134(9): 3942-3945 describes destabilizing domains derivd from the human estrogen receptor. US 2009/087871 describes a method for identifying PDE5-modulators.

### SUMMARY

The invention is defined by the appended claims.

The present disclosure relates to compositions useful in methods for immunotherapy. The compositions relate to tunable systems and agents that induce anti-cancer immune responses in a cell or in a subject. The tunable system and agent is a biocircuit system comprising at least one effector module that is responsive to at least one stimulus as defined in the appended claims. The biocircuit system is a destabilizing domain (DD) biocircuit system. These systems are further taught in co-owned U.S. Provisional Patent Application No. 62/320,864 filed April 11, 2016, 62/466,596 filed March 3, 2017 and the International Publication WO2017/180587.

The present disclosure provides an effector module. The effector module includes (a) a stimulus response element (SRE), wherein the SRE is a DD, said DD comprising a cGMP-specific 3',5'-cyclic phosphodiesterase (hPDE5; SEQ ID NO:1), in whole or in part, wherein said whole or part is capable of binding to a stabilizing ligand and wherein the DD further comprises: i) a mutation at amino acid position 732 (R732) of SEQ ID NO: 1 and ii) a mutation at amino acid position 736 (F736) of SEQ ID NO: 1, selected from the group consisting of F736G, F736L, F736M, F736R, F736W, F736K, F736Q, F736E, F736S, F736P, F736V, F736I, F736C, F736Y, F736H, F736N, F736D, and F736T, and (b) at least one payload, which is attached, appended or associated with said SRE. The SRE is responsive to one or more stimuli.

In some embodiments, the SRE may include three mutations.

In some embodiments, the SRE may include four mutations.

In some embodiments, the SRE may include five mutations.

In one embodiment, the SRE may include six mutations. In one embodiment, the SRE may include eight mutations.

The mutation pairs may be selected from but not limited to R732D, F736S; R732E, F736D; R732V, F736G; R732W, F736G; R732W, F736V; R732L, F736W; R732P, F736Q; R732A, F736A; R732S, F736G; R732T, F736P; R732M, F736H; R732Y, F736M; R732P, F736D; R732P, F736G; R732W, F736L; R732L, F736S; R732D, F736T; R732L, F736V; R732G, F736V; and R732W, F736A.

The payload of the effector module may be a chimeric antigen receptor (CAR). The CAR may include (a) an extracellular target moiety; (b) a hinge and transmembrane domain; (c) an intracellular signaling domain; and (d) optionally, one or more co-stimulatory domains. The CAR may comprise the mutations H653A and R732L.

In some embodiments the CAR is a CD19 CAR.

The one or more stimuli of the effector modules as described herein can be responsive to a small molecule. In some embodiments, the small molecule is selected from Tadalafil, Vardenafil, Sildenafil, Avanafil, Lodenafil, Mirodenafil, Udenafil, Benzamidenafil, Dasantafil, and Beminafil.

Also provided herein are pharmaceutical compositions which include effector modules as defined in the claims and pharmaceutically acceptable excipients, polynucleotides encoding effector modules as defined in the claims, pharmaceutical compositions comprising polynucleotides as defined in the claims, and vectors comprising polynucleotides as defined in the claims.

### BRIEF DESCRIPTION OF THE DRAWINGS

The drawings are not necessarily to scale; emphasis instead being placed upon illustrating the principles of various embodiments of the disclosure.
Figure 1 shows an overview diagram of a biocircuit system of the disclosure. The biocircuit comprises a stimulus and at least one effector module responsive to a stimulus, where the response to the stimulus produces a signal or outcome. The effector module comprises at least one stimulus response element (SRE) and one payload.
Figure 2 shows representative effector modules carrying one payload. The signal sequence (SS), SRE and payload may be located or positioned in various arrangements without (A to F) or with (G to Z, and AA to DD) a cleavage site. An optional linker may be inserted between each component of the effector module.
Figure 3 shows representative effector modules carrying two payloads without a cleavage site. The two payloads may be either directly linked to each other or separated.
Figure 4 shows representative effector modules carrying two payloads with a cleavage site. In one embodiment, an SS is positioned at the N-terminus of the construct, while other components: SRE, two payloads and the cleavage site may be located at different positions (A to L). In another embodiment, the cleavage site is positioned at the N-terminus of the construct (M to X). An optional linker may be inserted between each component of the effector module.
Figure 5 shows effector modules of the disclosure carrying two payloads, where an SRE is positioned at the N-terminus of the construct (A to L), while SS, two payloads and the cleavage site can be in any configuration. An optional linker may be inserted between each component of the effector module.
Figure 6 shows effector modules of the disclosure carrying two payloads, where either the two payloads (A to F) or one of the two payloads (G to X) is positioned at the N-terminus of the construct (A to L), while SS, SRE and the cleavage site can be in any configuration. An optional linker may be inserted between each component of the effector module.

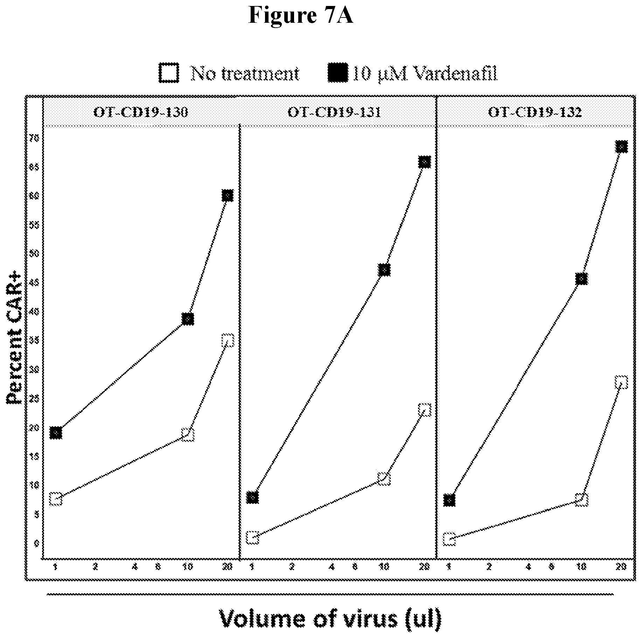

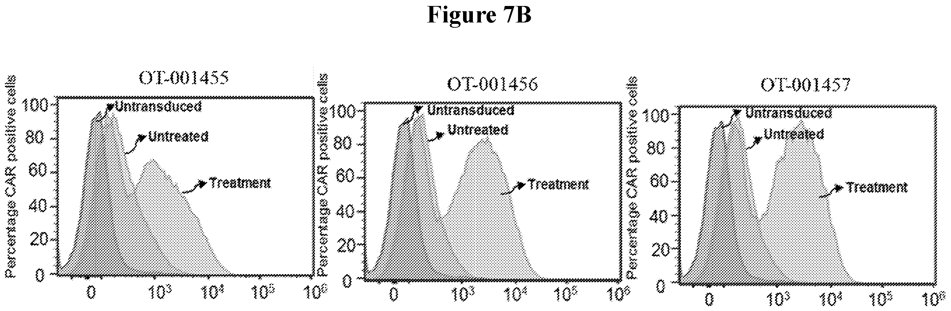

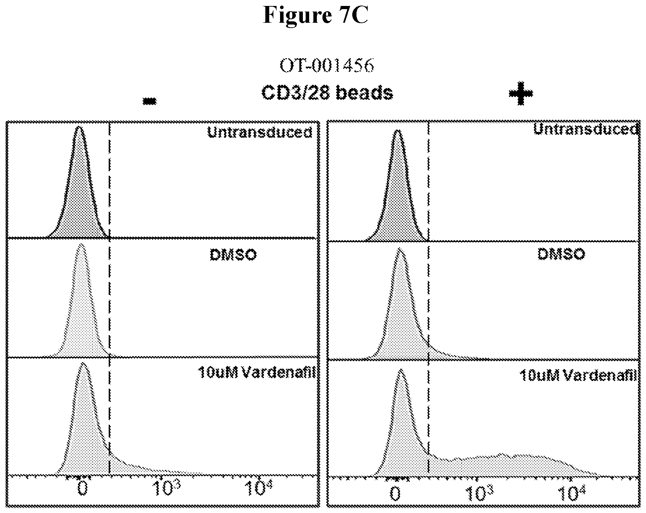

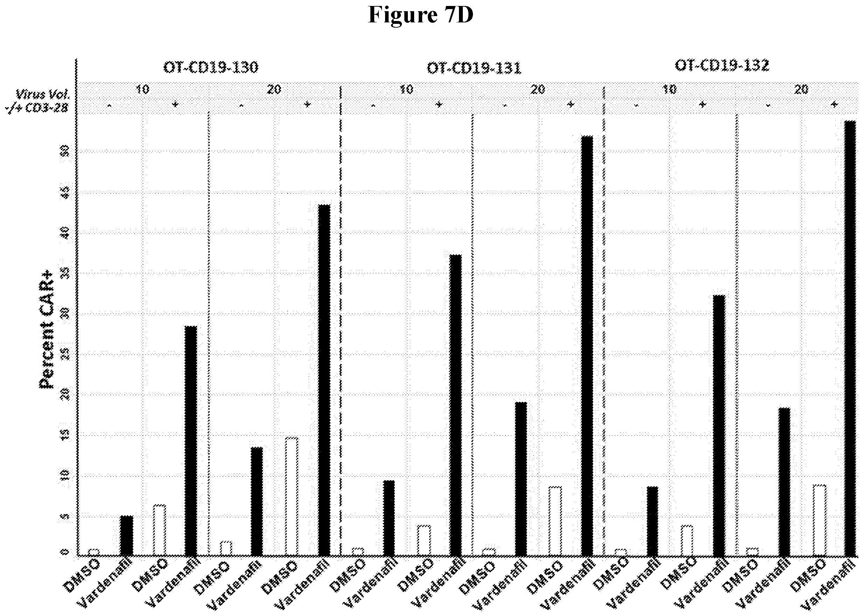

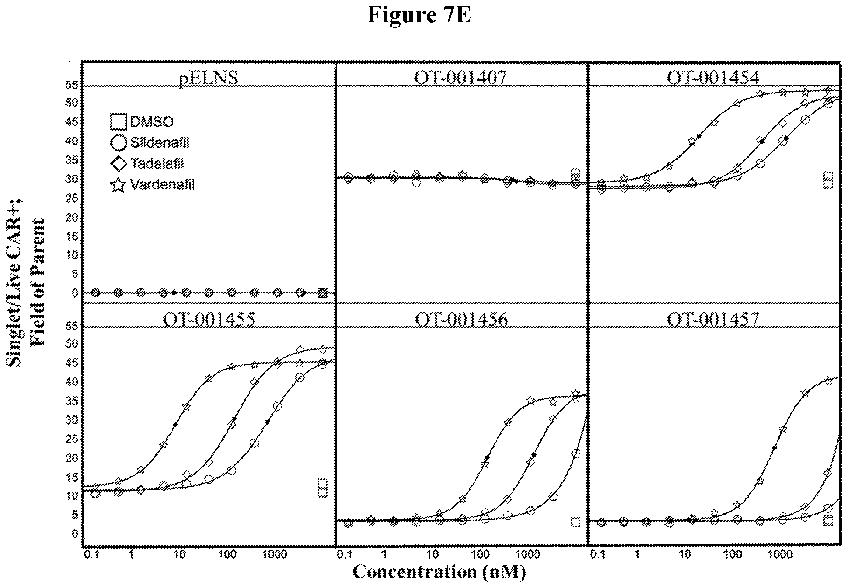

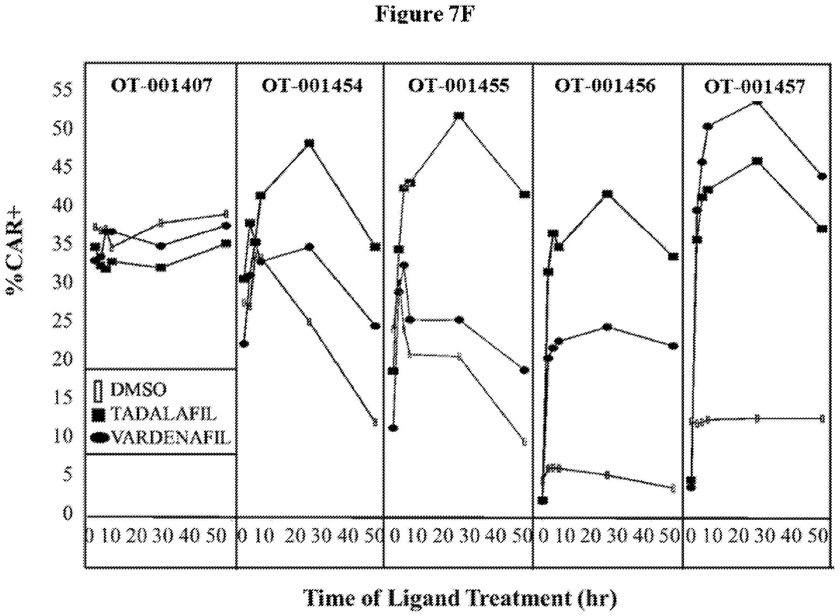

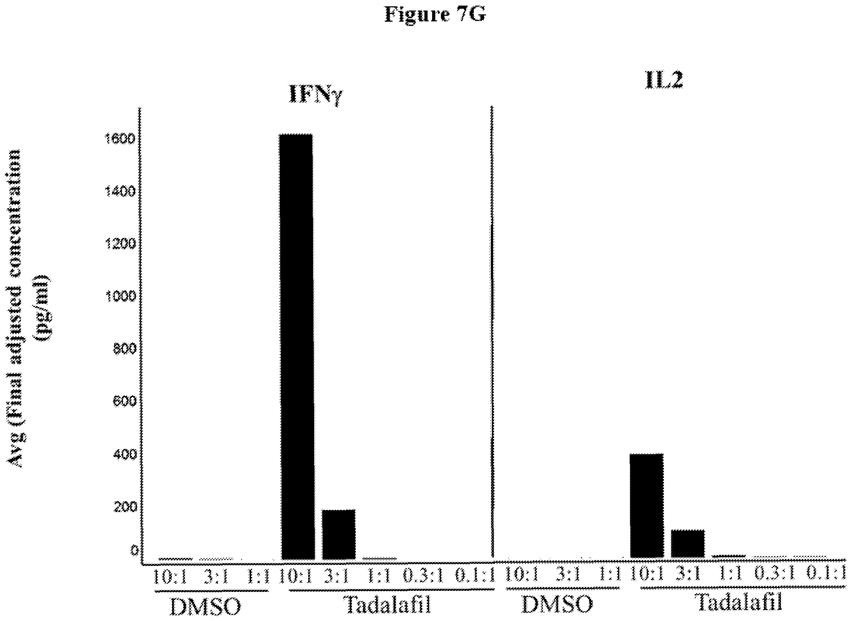

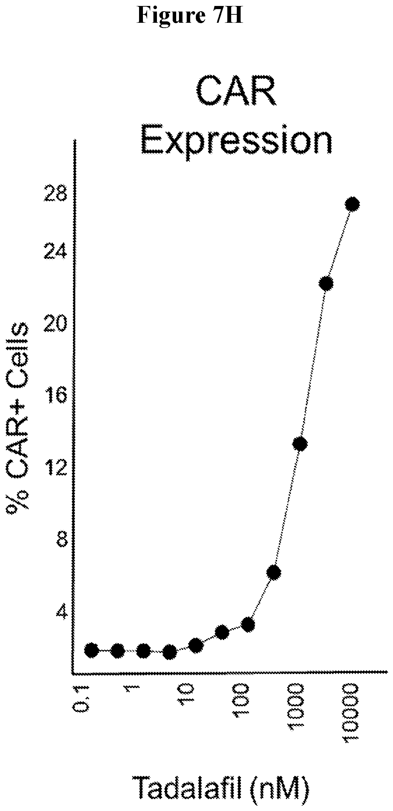

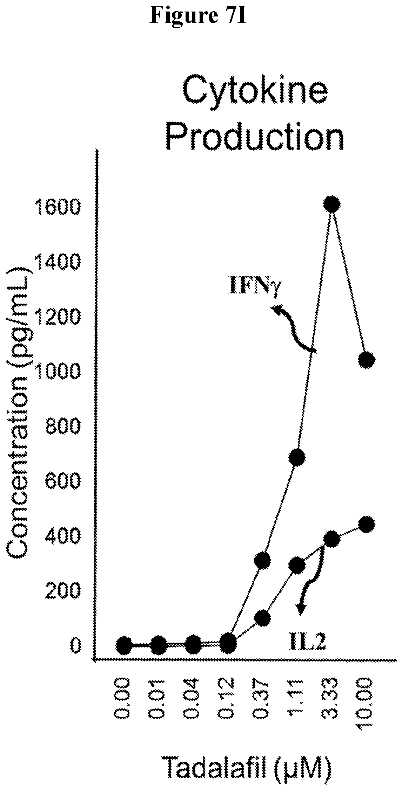

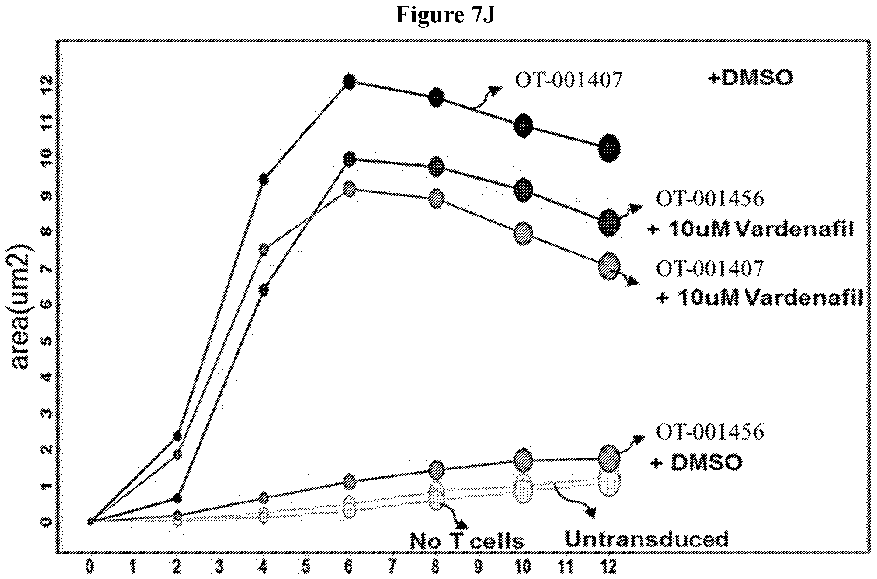

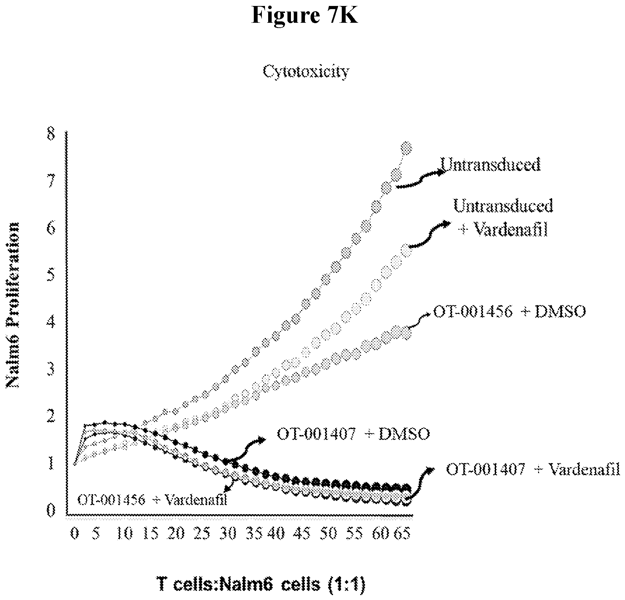

Figure 7A shows the percent of CAR positive cells obtained with cells transduced with different volumes of virus related to the CAR constructs. Figure 7B shows the percentage of CAR positive cells with vardenafil treatment. Figure 7C shows the expression of the CAR with CD3/CD28 bead restimulation. Figure 7D shows the percentage of CAR positive cells with different concentrations of virus and in the presence or absence of CD3/CD28 bead restimulation. Figure 7E shows the dose response of CD19 CAR constructs to sildenafil, vardenafil and tadalafil. Figure 7F shows the response of CD19 CAR constructs to different ligands and with varying the duration of ligand treatment. Figure 7G shows IL2 and IFNγ levels obtained from the supernatants of cocultures of effector and target cells in the presence of vardenafil. Figure 7H shows the percentage of CAR positive T cells obtained with Tadalafil treatment. Figure 7I shows the IL2 and IFNγ levels obtained from the supernatants of cocultures of effector and target cells in the presence of tadalafil. Figure 7J shows the target cell killing by CAR expressing T cells in the presence of vardenafil. Figure 7K shows the proliferation of target cells cocultured with CAR expressing T cells in the presence of vardenafil.
Figure 8A shows the total flux of Nalm6 luc cells in mice, in the presence of tadalafil. Figure 8B and Figure 8C show the pharmacokinetics of vardenafil and tadalafil in plasma of mice after injections of ligand at indicated doses.
Figure 9A shows antitumor efficacy of Vardenafil. Figure 9B shows that increased Tadalafil dose density significantly increases DD regulated CAR-Ts antitumor efficacy.
Figure 10 shows the total flux of Nalm6 luc cells in mice with initial high dose of tadalafil compared to lower dose. A high initial dose of tadalafil with later shift to maintenance dosing does not improve efficacy of PDE5-regulated CD19 CAR-mediated cytotoxicity.
Figure 11 shows the total flux of Nalm6 luc cells in mice with pre-infusion tadalafil exposure. Ligand pre-treatment prior to freezing CAR T cells increases basal CAR activity in vehicle treated animals but does not improve efficacy of ligand-regulated CAR-mediated cytotoxicity.
Figure 12 shows the total flux of Nalm6 luc cells in mice with variable cell dosing. Increased cell dose fractionated over 3 days led to increased anti-tumor efficacy as compared to a single cell infusion.
Figure 13 shows the total flux of Nalm6 luc cells in mice comparing efficacy of OT-001455 and OT-001456 constructs. OT-001455 showed higher increased efficacy but higher basal activity compared to OT-001456.
Figure 14A shows a time course of CD19 CAR expression upon exposure to saturating dose of tadalafil. PDE5-regulated CD19 CAR expression responded similarly to constitutively expressed CD19 CAR, with low and high tadalafil concentrations achieving similar timecourse.
Figure 14B shows a timecourse of CD19 CAR expression upon exposure to saturating dose of vardenafil. PDE5-regulated CD19 CAR expression responded similarly to constitutively expressed CD19 CAR, with low and high vardenafil concentrations achieving similar timecourse.
Figure 15 shows CD19 CAR expression upon washout of tadalafil or vardenafil. PDE5-regulated CD19 CAR expression on the surface of T cells drops rapidly following removal of ligand.
Figure 16 shows that OT-001456 allows reversibility of CAR expression based on ligand exposure time course, along with the ability to perform multiple rounds of pulsed ligand dosing while maintaining equal expression levels.

### DETAILED DESCRIPTION

Some of the instances and disclosures below encompass embodiments of the present invention, which is defined by the appended claims.

Any references herein to methods of treatment using products should be interpreted as references to said products for use in said methods.

Although any materials and methods similar or equivalent to those described herein can be used in the practice or testing of the present disclosure, the preferred materials and methods are now described. Other features, objects and advantages of the disclosure will be apparent from the description. In the description, the singular forms also include the plural unless the context clearly dictates otherwise. Unless defined otherwise, all technical and scientific terms used herein have the same meaning as commonly understood by one of ordinary skill in the art to which this disclosure belongs. In the case of conflict, the present description will control.

### I. INTRODUCTION

### Protein regulation

The ability to conditionally control protein levels is a powerful tool in gene and cell therapy. Techniques to control protein expression on a genetic level have been widely studied. The Cre-Lox technology provides a useful approach to activate or inactivate genes. Tissue or cell specific promoters can be used to control spatial and temporal expression of genes of interest. However, this system is limited in application due to the irreversible nature of the perturbation. The transcription of the gene of interest can be conditionally regulated using tools such as Doxycycline (Dox)-inducible system. Alternatively, the stability of mRNA can be regulated using RNA interference techniques. However, methods targeting DNA or RNA are slow acting, irreversible and have low efficiency.

Direct manipulation of activities at the protein level provides significant advantages in flexibility, reversibility and speed. Strategies which directly trigger a cell's natural degradation system have been developed. Szostak and colleagues showed that a small peptide sequence could be fused to the N-terminus of a protein of interest to modulate protein stability (Park, E-C., et al., Proc. Natl. Acad. Sci. U.S.A. 1992, 89:1249-1252). Varshavsky and coworkers isolated a temperature-sensitive peptide sequence that greatly reduced the half-life of dihydrofolate reductase (DHFR) at the non-permissive temperatures (Dohmen et al. Science 1994, 263:1273-1276). These mutants have been widely used to study protein functions in yeast (Labib et al. Science 2000, 288:1643-1646; and Kanemaki et al. Nature 2003, 423:720-724).

Subsequently, reversible systems employing a rapamycin derivative for the regulation of GSK-3β kinase fused to an unstable triple-mutant of the FRB domain (FRB*) were developed. The rapamycin derivative induces dimerization of the FRB*-GSK-3β and endogenous FKBP12 and stabilizes the FRB* fusion thus restoring the function of the fused kinase. (Stankunas et al., Mol Cell. 2003; 12:1615-1624 and Liu et al., Nature. 2007; 446:79-82).

Building on the FRB* domain system, Banaszynski, *et al.,* developed a cell-permeable ligand system using mutants of FKBP12 protein which were engineered to be unstable in the absence of a high-affinity ligand, Shield-1. (Banaszynski et al., Cell. 2006; 126:995-1004). They termed these unstable domains, destabilizing domains (DDs).

The FKBP/shield-1 tuning system has been successfully used in several studies to control target proteins. For example, Dettwier et al., fused FKBP to tune the express of NADPH P450 oxidoreductase (POR) (Dettwier et al., PLoS One, 2014, 9(11): e113540).

The FKBP DD-shield system has been used in cell lines, transgenic mice, *protozoan Entamoeba histolytica,* the flatworm *Caenorhabditis elegans,* the medaka, and transgenic xenografts to investigate the activity of a protein of interest (Maynard-Smith et al., J Biol Chem. 2007, 282(34): 24866-24872; Liu et al., Int J Parasitol. 2014, 44(10):729-735; Cho et al., PLoS One. 2013, 8(8): e72393); Banaszynski et al. Nat Med. 2008, 14(10):1123-1127; Rodriguez and Wolfgang, Chem Biol. 2011, 19(3):391-398; and Froschauer et al., PLoS One, 2015, 10(7): e0131252), for iPSC reprogramming (Sui et al., Stem cell Reports., 2014, 2(5): 721-733).

In addition, the destabilizing domain has been used for the conditional knock-down or knock-out of the target gene fused with the destabilizing domain. Park et al achieved this genomic engineering by CRISPR/Cas9-mediated homologous recombination and a donor template coding for a resistance cassette and the DD-tagged TCOF1 sequence (Park et al., PLoS One. 2014, 9(4): e95101).

More recently protein switches useful as biosensors as well as new chimeric antigen receptors and other small molecule stabilization frameworks have been disclosed (An W, et al. PLoS ONE, 2015, 10(12): e0145783. doi: 10.1371/journal.pone.0145783; Nicholes, et al., Protein Engineering, Design & Selection, 2016, vol. 29 no. 2, pp. 77-85; Nath, et al., Biochemical and Biophysical Research Communications, 2016, 470: 411e416); Stevers, et al., PNAS, 2016, vol. 119, no. 9, pp. E112-1161; Juillerat, A. et al., Sci. Rep. 2016, 6: 18950; Roybal, Cell, 2016, vol. 164, pp. 1-10; and Morsut, Cell, 2016, vol. 164, pp. 1-12).

One drawback of the FKBP/Shield-1 is that Shield-1 is a novel drug whose biodistribution is not fully characterized and it is not known to what extent Shield-1 crosses the blood-brain barrier.

Other DD ligand pairs include estrogen receptor domains which can be regulated by several estrogen receptor antagonists (Miyazaki et al., J Am Chem. Soc., 2012, 134(9): 3942-3945), and fluorescent destabilizing domain (FDD) derived from bilirubin-inducible fluorescent protein, UnaG. A FDD and its cognate ligand bilirubin (BR) can induce degradation of a protein fused to the FDD (Navarro et al., ACS Chem Biol., 2016, June 6, Epub). Other known DDs and their applications in protein stability include those described in U.S. Pat. NO. 8,173,792 and U.S. Pat. NO. 8,530,636.

In an orthogonal approach, the destabilizing domains of the bacterial dihydrofolate reductase (ecDHFR) were explored. (Iwamoto et al., Chem Biol. 2010, 17(9):981-988; and Tai et al., PLoS One. 2012, 7(9): e46269). Numerous inhibitors of DHFR have been developed as drugs and one such inhibitor Trimethoprim (TMP), inhibits ecDHFR much more potently than mammalian DHFR providing specificity to the interaction. Additionally, TMP is commercially available and has desirable pharmacological properties making this protein-ligand pair ideal for development for use as a biocircuit (Iwamoto, et al., Chem Biol. (2010) September 24; 17(9): 981-988).

The present disclosure provides novel protein domains displaying small molecule dependent stability. Such protein domains are called destabilizing domains (DDs). In the absence of its binding ligand, the DD is destabilizing and causes degradation of a payload fused to the DD (e.g., a protein of interest (POI), while in the presence of its binding ligand, the fused DD and payload can be stabilized and its stability is dose dependent. Methods for tuning the expression level and activity of a protein of interest using the DDs, effector modules, biocircuit systems and compositions of the disclosure are also provided. In some instances, the SRE may be a destabilizing domain (DD). In some examples, the DD may be a portion or region of human protein PDE5. In this context, the biocircuit system is a DD biocircuit system.

The present disclosure expands upon the technology of tuning protein stability using novel destabilizing domains derived from human PDE5 protein. The destabilization and stabilization of a protein of interest, e.g., a transgene for gene therapy, can be controlled by PDE5 mutant DDs having destabilizing or stabilizing properties and their ligands, e.g. Sildenafil and Vardenafil specifically binding to such protein domains. The presence and/or absence of a small molecule ligand can tune the activity of a payload (e.g., a protein of interest) that is operably linked to the destabilizing domain.

### Immunotherapy

Cancer immunotherapy aims' at the induction or restoration of the reactivity of the immune system towards cancer. Significant advances in immunotherapy research have led to the development of various strategies which may broadly be classified into active immunotherapy and passive immunotherapy. In general, these strategies may be utilized to directly kill cancer cells or to counter the immunosuppressive tumor microenvironment. Active immunotherapy aims at induction of an endogenous, long-lasting tumor-antigen specific immune response. The response can further be enhanced by non-specific stimulation of immune response modifiers such as cytokines. In contrast, passive immunotherapy includes approaches where immune effector molecules such as tumor-antigen specific cytotoxic T cells or antibodies are administered to the host. This approach is short lived and requires multiple applications.

Despite significant advances, the efficacy of current immunotherapy strategies is limited by associated toxicities. These are often related to the narrow therapeutic window associated with immunotherapy, which in part, emerges from the need to push therapy dose to the edge of potentially fatal toxicity to get a clinically meaningful treatment effect. Further, dose expands *in vivo* since adoptively transferred immune cells continue to proliferate within the patient, often unpredictably.

A major risk involved in immunotherapy is the on-target but off tumor side effects resulting from T-cell activation in response to normal tissue expression of the tumor associated antigen (TAA). Clinical trials utilizing T cells expressing T-cell receptor against specific TAA reported skin rash, colitis and hearing loss in response to immunotherapy.

Immunotherapy may also produce on target, on-tumor toxicities that emerge when tumor cells are killed in response to the immunotherapy. The adverse effects include tumor lysis syndrome, cytokine release syndrome and the related macrophage activation syndrome. Importantly, these adverse effects may occur during the destruction of tumors, and thus even a successful on-tumor immunotherapy might result in toxicity. Approaches to regulatably control immunotherapy are thus highly desirable since they have the potential to reduce toxicity and maximize efficacy.

The present disclosure provides systems, compositions, immunotherapeutic agents and methods for cancer immunotherapy. These compositions provide tunable regulation of gene expression and function in immunotherapy. The present disclosure also provides biocircuit systems, effector modules, stimulus response elements (SREs) and payloads, as well as polynucleotides encoding any of the foregoing. In one aspect, the systems, compositions, immunotherapeutic agents and other components of the disclosure can be controlled by a separately added stimulus, which provides a significant flexibility to regulate cancer immunotherapy. Further, the systems, compositions and the methods of the present disclosure may also be combined with therapeutic agents such as chemotherapeutic agents, small molecules, gene therapy, and antibodies.

The tunable nature of the systems and compositions of the disclosure has the potential to improve the potency and duration of the efficacy of immunotherapies. Reversibly silencing the biological activity of adoptively transferred cells using compositions of the present disclosure allows maximizing the potential of cell therapy without irretrievably killing and terminating the therapy.

In particular, present disclosure provides methods for fine tuning of immunotherapy after administration to patients. This in turn improves the safety and efficacy of immunotherapy and increases the subject population that may benefit from immunotherapy.

### II. COMPOSITIONS OF THE DISCLOSURE

A variety of strategies that can directly control protein, e.g., a transgene, expression and function are available. The present disclosure provides novel protein domains displaying small molecule dependent stability. Such protein domains are called destabilizing domains (DDs). In the absence of its binding ligand, the DD causes degradation of a payload such as a protein of interest (POI) that is operably linked to the DD, while in the presence of its binding ligand, the fused DD and payload can be stabilized and its stability is dose dependent.

According to the present disclosure, novel destabilizing domains derived from human hPDE5 (cGMP-specific phosphodiesterase type 5; also referred to as cGMP-specific 3',5'-cyclic phosphodiesterase) protein are provided. The destabilizing domain (DD) mutants are derived from the human PDE5 protein, comprising the amino acid sequence of SEQ ID NO: 1 (encoded by the nucleic acid sequence of SEQ ID NO: 2). The hPDE5 DD mutant may also comprise more than one mutation in the catalytic domain of human PDE5 (SEQ ID NO: 3), encoded by nucleic acid sequence of SEQ ID NO:339, e.g., two, three, four, five or more mutations. These hPDE5 DDs can bind to Sildenafil and/or Vardenafil and be stabilized. In some instances, the hPDE5 DD may include a methionine appended to the N terminus of catalytic domain (SEQ ID NO: 237), encoded by SEQ ID NO: 4.

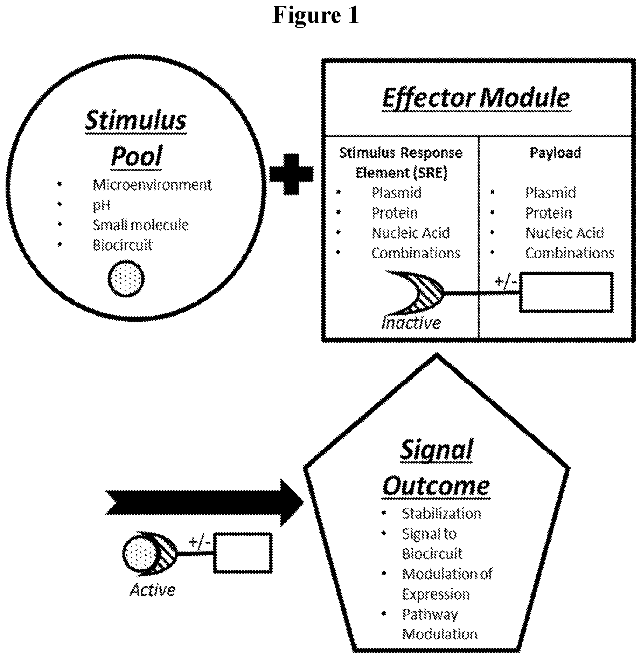

According to the present disclosure, biocircuit systems are provided which comprise, at their core, at least one effector module system. Such effector module systems comprise at least one effector module having associated, or integral therewith, one or more stimulus response elements (SREs). The overall architecture of a biocircuit system of the disclosure is illustrated in Figure 1. In general, a stimulus response element (SRE) may be operably linked to a payload construct which could be any protein of interest (POI) (e.g., an immunotherapeutic agent), to form an effector module. The SRE, when activated by a particular stimulus, e.g., a small molecule, can produce a signal or outcome, to regulate transcription and/or protein levels of the linked payload either up or down by perpetuating a stabilizing signal or destabilizing signal, or any other types of regulation. A much-detailed description of a biocircuit system can be found in co-owned U.S. Provisional Patent Application No. 62/320,864 filed April 11, 2016, 62/466,596 filed March 3, 2017 and the International Publication WO2017/180587. In accordance with the present disclosure, biocircuit systems, effector modules, SREs and components that tune expression levels and activities of any agents used for immunotherapy are provided. In particular, biocircuit systems and effector modules comprising the novel hPDE5 destabilizing domains discussed herein are provided.

As used herein, a "biocircuit" or "biocircuit system" is defined as a circuit within or useful in biologic systems comprising a stimulus and at least one effector module responsive to a stimulus, where the response to the stimulus produces at least one signal or outcome within, between, as an indicator of, or on a biologic system. Biologic systems are generally understood to be any cell, tissue, organ, organ system or organism, whether animal, plant, fungi, bacterial, or viral. It is also understood that biocircuits may be artificial circuits which employ the stimuli or effector modules taught by the present disclosure and effect signals or outcomes in acellular environments such as with diagnostic, reporter systems, devices, assays or kits. The artificial circuits may be associated with one or more electronic, magnetic, or radioactive components or parts.

In accordance with the present disclosure, a biocircuit system may be a destabilizing domain (DD) biocircuit system, a dimerization biocircuit system, a receptor biocircuit system, and a cell biocircuit system. Any of these systems may act as a signal to any other of these biocircuit systems. In some instances, the present disclosure provides biocircuit systems, effector modules and compositions comprising the DDs of the present disclosure. In one aspect, the biocircuit system is a DD biocircuit system.

In one aspect of the present disclosure, the biocircuit system is a DD biocircuit system. The DD is a hPDE5 derived DD. The present disclosure provides isolated polypeptide variants. Such variants may include one to ten mutations relative to SEQ ID NO:3. In some instances, the isolated polypeptide variant comprises mutations that may independently selected from, but not limited to L573M, F735L, R658H, S562G, L727P, R847T, Q666H, L781P, A767E, T571S, V585M, T723S, Q743L, Y709H, F787V, N605Y, I715K, F564I, N662Y, H685L, L693P, F736I, A722V, F755L, M760I, V594I, H592R, E858V, T784I, S542L, E708V, W772C, I700F, E796G, D724G, K848N, T712M, M758I, Q859R, I556S, E796D, I680S, H613L, D724Y, F755Y, A611T, N661S, L569M, T833S, I768N, R607W, N620S, L554R, Q589E, M691I, W615R, T723R, A762T, E808G, C574Y, L554R, Q586P, K710N, K730E, H613R, Q586L, S663Y, A762T, E808G, V548M, D803N, L554R, Q589E, A823T, T571I, K604R, I706N, E795R, G659A, T784I, Q666H, H617L, A722V, N609Y, I799L, F561L, G659A, T784I, K795N, K770Q, K848N, N605D, I680S, V548M, F820I, I799L, R607Q, S550F, L554R, P549S, F564I, R658H, A779T, and R847G.

In some instances, the isolated polypeptide variant may comprise the mutations, L554R, Q589E and A823T. In one instance, the isolated polypeptide variant may be SEQ ID NO: 8423.

In some instances, the isolated polypeptide variant may comprise the mutations K770Q, and K848N. In one instance, the isolated polypeptide variant may be SEQ ID NO: 8431.

In some instances, the isolated polypeptide variant may comprise the mutations L573M, and F735L. In one instance, the isolated polypeptide variant may comprise the SEQ ID NO: 8387.

In some instances, the isolated polypeptide variant may be a stimulus response element (SRE). In one aspect, the SRE may be responsive to one or more stimuli.

The present disclosure also provides polynucleotides encoding the polypeptides described herein. In some instances, the polynucleotide may be codon optimized. In some aspects, the polynucleotide may comprise spatiotemporally selected codons.

### Effector Modules and SREs for Immunotherapy

In accordance with the present disclosure, biocircuit systems, effector modules, SREs, and components that tune expression levels and activities of any agents used for immunotherapy are provided. As non-limiting examples, an immunotherapeutic agent may be an antibody and fragments and variants thereof, a cancer specific T cell receptor (TCR) and variants thereof, an anti-tumor specific chimeric antigen receptor (CAR), a chimeric switch receptor, an inhibitor of a co-inhibitory receptor or ligand, an agonist of a co-stimulatory receptor and ligand, a cytokine, chemokine, a cytokine receptor, a chemokine receptor, a soluble growth factor, a metabolic factor, a suicide gene, a homing receptor, or any agent that induces an immune response in a cell and a subject.

As stated, the biocircuits of the disclosure include at least one effector module as a component of an effector module system. As used herein, an "effector module" is a single or multi-component construct or complex comprising at least (a) one or more stimulus response elements (SREs) and (b) one or more payloads (i.e. proteins of interest (POIs)). In the context of the present disclosure, the SRE is a DD derived from human PDE5 protein.

As used herein a "stimulus response element (SRE)" is a component of an effector module which is joined, attached, linked to or associated with one or more payloads of the effector module and in some instances, is responsible for the responsive nature of the effector module to one or more stimuli. As used herein, the "responsive" nature of an SRE to a stimulus may be characterized by a covalent or non-covalent interaction, a direct or indirect association or a structural or chemical reaction to the stimulus. Further, the response of any SRE to a stimulus may be a matter of degree or kind. The response may be a partial response. The response may be a reversible response. The response may ultimately lead to a regulated signal or output. Such output signal may be of a relative nature to the stimulus, e.g., producing a modulatory effect of between 1% and 100% or a factored increase or decrease such as 2-fold, 3-fold, 4-fold, 5-fold, 10-fold or more.

In some instances, the biocircuit system of the present disclosure comprising a stimulus and an effector module of the disclosure. The DD of the effector module binds to the stimulus and regulates the stability of the linked payload. The DD may destabilize the protein of interest by a destabilization ratio between 0, and 0.09, wherein the destabilization ratio comprises the ratio of expression, function or level of a protein of interest in the absence of the stimulus specific to the DD to the expression, function or level of the protein of interest that is expressed constitutively, and in the absence of the stimulus specific to the DD. In some instances, the DD may stabilize the protein of interest by a stabilization ratio of 1 or more, wherein the stabilization ratio comprises the ratio of expression, function or level of a protein of interest in the presence of the stimulus to the expression, function or level of the protein of interest in the absence of the stimulus.

In some instances, the present disclosure provides methods for modulating protein expression, function or level. In some aspects, the modulation of protein expression, function or level refers to modulation of expression, function or level by at least about 20%, such as by at least about 30%, 40%, 50%, 60%, 70%, 80%, 85%, 90%, 95% and 100%, or at least 20-30%, 20-40%, 20-50%, 20-60%, 20-70%, 20-80%, 20-90%, 20-95%, 20-100%, 30-40%, 30-50%, 30-60%, 30-70%, 30-80%, 30-90%, 30-95%, 30-100%, 40-50%, 40-60%, 40-70%, 40-80%, 40-90%, 40-95%, 40-100%, 50-60%, 50-70%, 50-80%, 50-90%, 50-95%, 50-100%, 60-70%, 60-80%, 60-90%, 60-95%, 60-100%, 70-80%, 70-90%, 70-95%, 70-100%, 80-90%, 80-95%, 80-100%, 90-95%, 90-100% or 95-100%.

According to the present disclosure, biocircuit systems and effector modules of the disclosure can be used to regulate the expression and activity of a payload in response to the presence or absence of a ligand that specifically binds to the DD integrated within the biocircuit system and effector module.

In some aspects, DDs, effector modules and biocircuit systems of the disclosure may be used to regulate the expression, function and activity of a payload in a cell or a subject. The regulation refers to a level of change of its expression, function and activity, by at least about 10%, or at least about 20%, or at least about 30%, or at least about 40%, or at least about 50%, or at least about 60%, or at least about 70%, or at least about 80%, or at least about 85%, or at least about 90%, or at least about 95%, or at least about 100%, or at least 20-30%, 20-40%, 20-50%, 20-60%, 20-70%, 20-80%, 20-90%, 20-95%, 20-100%, 30-40%, 30-50%, 30-60%, 30-70%, 30-80%, 30-90%, 30-95%, 30-100%, 40-50%, 40-60%, 40-70%, 40-80%, 40-90%, 40-95%, 40-100%, 50-60%, 50-70%, 50-80%, 50-90%, 50-95%, 50-100%, 60-70%, 60-80%, 60-90%, 60-95%, 60-100%, 70-80%, 70-90%, 70-95%, 70-100%, 80-90%, 80-95%, 80-100%, 90-95%, 90-100% or 95-100%.

In some instances, the mutation co-efficient of the biocircuits of the disclosure may also be measured as a way of evaluating the ability of the DD to modulate protein expression, function or level. As used herein, the "mutation co-efficient" refers to the expression, function or level or a protein of interest, appended to a particular DD mutant, in the absence of the stimulus specific to the SRE; to the protein expression, function or level of the protein of interest, appended to the corresponding wildtype sequence from which the particular DD mutant is derived and in the absence of the stimulus. The mutation co-efficient is indicative of the contribution of the destabilizing mutations towards the basal expression of the protein independent of the whether the corresponding wildtype protein can be destabilized without any mutations. In some aspects, the mutation co-efficient ratio is at least 0, such as by at least 0.1, 0.2, 0.3, 0.4, 0.5, 0.6, 0.7, 0.8, 0.9, or at least, 0-0.1, 0-0.2, 0 -0.3, 0-0.4, 0-0.5, 0-0.6, 0-0.7, 0-0.8, 0-0.9, 0.1-0.2, 0.1 -0.3, 0.1-0.4, 0.1-0.5, 0.1-0.6, 0.1-0.7, 0.1-0.8, 0.1-0.9, 0.2 -0.3, 0.2-0.4, 0.2-0.5, 0.2-0.6, 0.2-0.7, 0.2-0.8, 0.2-0.9, 0.3-0.4, 0.3-0.5, 0.3-0.6, 0.3-0.7, 0.3-0.8, 0.3-0.9, 0.4-0.5, 0.4-0.6, 0.4-0.7, 0.4-0.8, 0.4-0.9, 0.5-0.6, 0.5-0.7, 0.5-0.8, 0.5-0.9, 0.6-0.7, 0.6-0.8, 0.6-0.9,0.7-0.8, 0.7-0.9 or 0.8-0.9.

In some instances, the present disclosure provides methods for modulating protein, expression, function or level by measuring the stabilization ratio, destabilization ratio, and/or destabilizing mutation co-efficient. As used herein, the "stabilization ratio" is the ratio of expression, function or level of a protein of interest in response to the stimulus to the expression, function or level of the protein of interest in the absence of the stimulus specific to the SRE. In some aspects, the stabilization ratio is at least 1, such as by at least 1-10, 1-20, 1 -30, 1-40, 1-50, 1- 60,1-70, 1-80,1- 90,1-100, 20-30, 20-40, 20-50, 20-60, 20-70, 20-80, 20-90, 20-95, 20-100, 30-40, 30-50, 30-60, 30-70, 30-80, 30-90, 30-95, 30-100, 40-50, 40-60, 40-70, 40-80, 40-90, 40-95, 40-100, 50-60, 50-70, 50-80, 50-90, 50-95, 50-100, 60-70, 60-80, 60-90, 60-95, 60-100, 70-80, 70-90, 70-95, 70-100, 80-90, 80-95, 80-100, 90-95, 90-100 or 95-100. As used herein, the "destabilization ratio" is the ratio of expression, function or level of a protein of interest in the absence of the stimulus specific to the effector module to the expression, function or level of the protein of interest, that is expressed constitutively and in the absence of the stimulus specific to the SRE. As used herein "constitutively" refers to the expression, function or level a protein of interest that is not linked to an SRE, and is therefore expressed both in the presence and absence of the stimulus to the SRE. As used herein, the "destabilizing mutation co-efficient" may be defined as the ratio of expression or level of a protein of interest that is appended to a DD, in the absence of the stimulus specific to the effector module to the expression, function or level of the protein that is appended to the wild type protein from which the DD is derived. In some aspects, the destabilization ratio and/or the destabilizing mutation co-efficient is at least 0, such as by at least 0.1, 0.2, 0.3, 0.4, 0.5, 0.6, 0.7, 0.8, 0.9, or at least, 0-0.1, 0-0.2, 0 -0.3, 0-0.4, 0-0.5, 0-0.6, 0-0.7, 0-0.8, 0-0.9, 0.1-0.2, 0.1 -0.3, 0.1-0.4, 0.1-0.5, 0.1-0.6, 0.1-0.7, 0.1-0.8, 0.1-0.9, 0.2 -0.3, 0.2-0.4, 0.2-0.5, 0.2-0.6, 0.2-0.7, 0.2-0.8, 0.2-0.9, 0.3-0.4, 0.3-0.5, 0.3-0.6, 0.3-0.7, 0.3-0.8, 0.3-0.9, 0.4-0.5, 0.4-0.6, 0.4-0.7, 0.4-0.8, 0.4-0.9, 0.5-0.6, 0.5-0.7, 0.5-0.8, 0.5-0.9, 0.6-0.7, 0.6-0.8, 0.6-0.9,0.7-0.8, 0.7-0.9 or 0.8-0.9.

The SRE of the effector module may be selected from, but is not limited to, a peptide, peptide complex, peptide-protein complex, protein, fusion protein, protein complex, protein-protein complex. The SRE may comprise one or more regions derived from any natural or mutated protein, or antibody. In this aspect, the SRE is an element, when responding to a stimulus, can tune intracellular localization, intramolecular activation, and/or degradation of payloads.

In some instances, effector modules of the present disclosure may comprise additional features that facilitate the expression and regulation of the effector module, such as one or more signal sequences (SSs), one or more cleavage and/or processing sites, one or more targeting and/or penetrating peptides, one or more tags, and/or one or more linkers. Additionally, effector modules of the present disclosure may further comprise other regulatory moieties such as inducible promoters, enhancer sequences, microRNA sites, and/or microRNA targeting sites. Each aspect or tuned modality may bring to the effector module or biocircuit a differentially tuned feature. For example, an SRE may represent a destabilizing domain, while mutations in the protein payload may alter its cleavage sites or dimerization properties or half-life and the inclusion of one or more microRNA or microRNA binding site may impart cellular detargeting or trafficking features. Consequently, the present disclosure embraces biocircuits which are multifactorial in their tenability. Such biocircuits may be engineered to contain one, two, three, four or more tuned features.

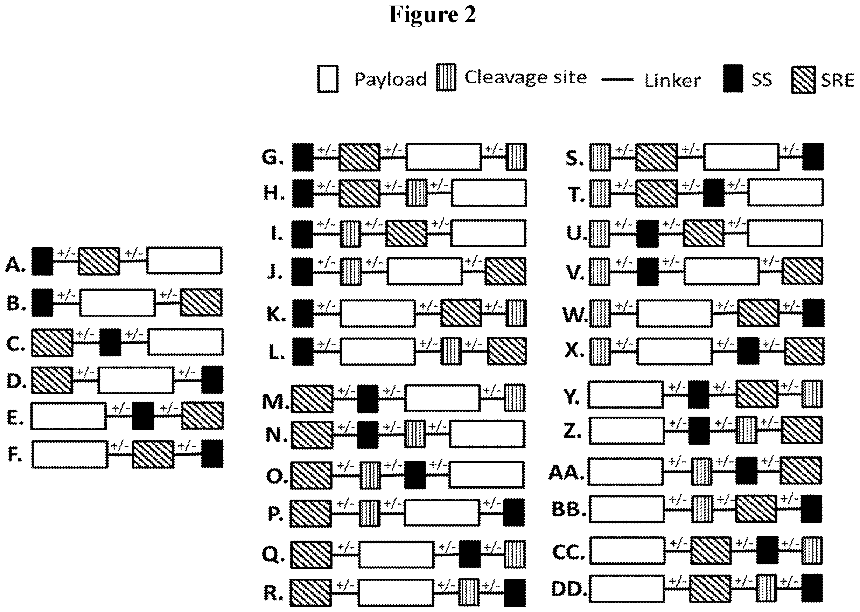

As shown in Figure 2, representative effector module instances comprising one payload, i.e. one immunotherapeutic agent are illustrated. Each components of the effector module may be located or positioned in various arrangements without (A to F) or with (G to Z, and AA to DD) a cleavage site. An optional linker may be inserted between each component of the effector module.

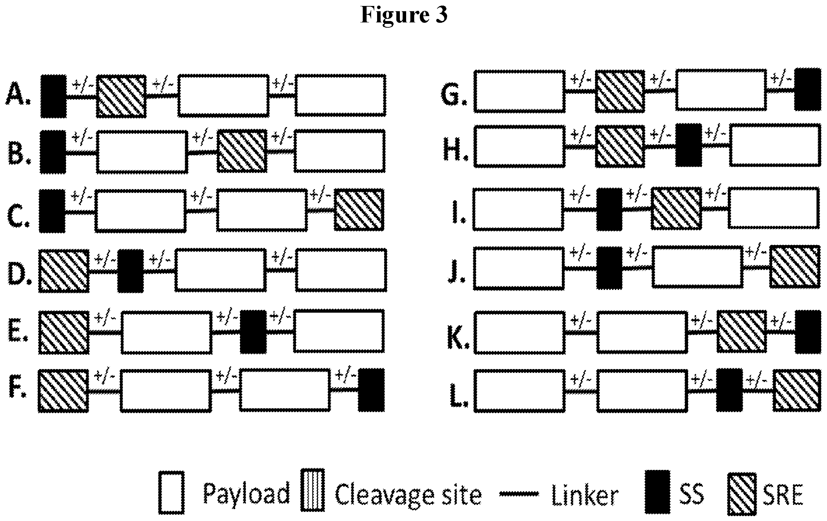

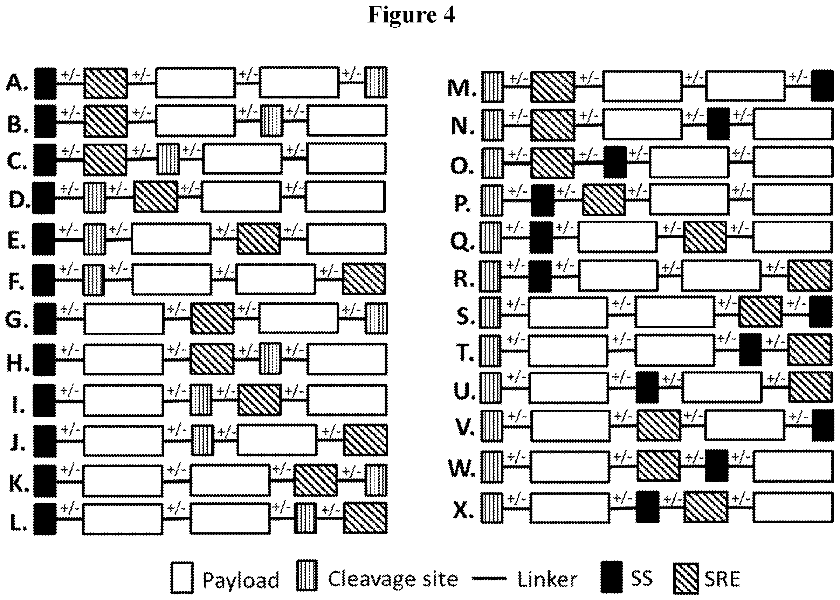

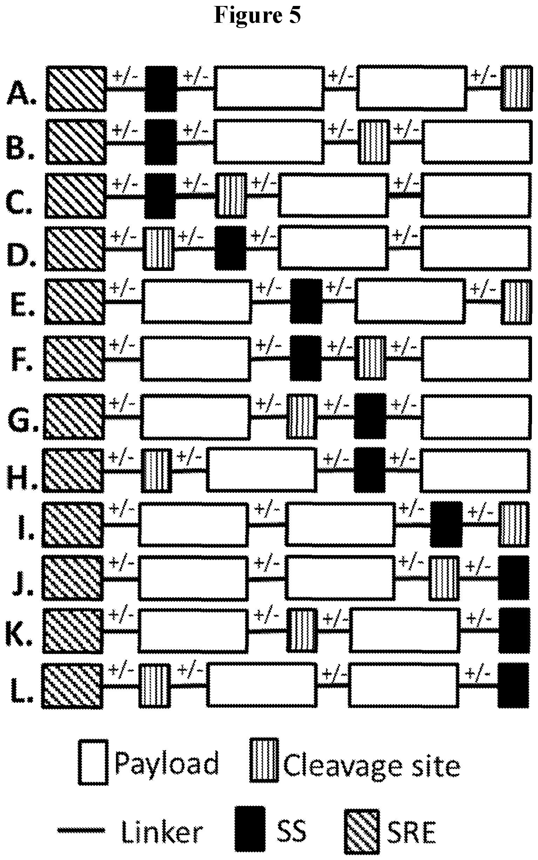

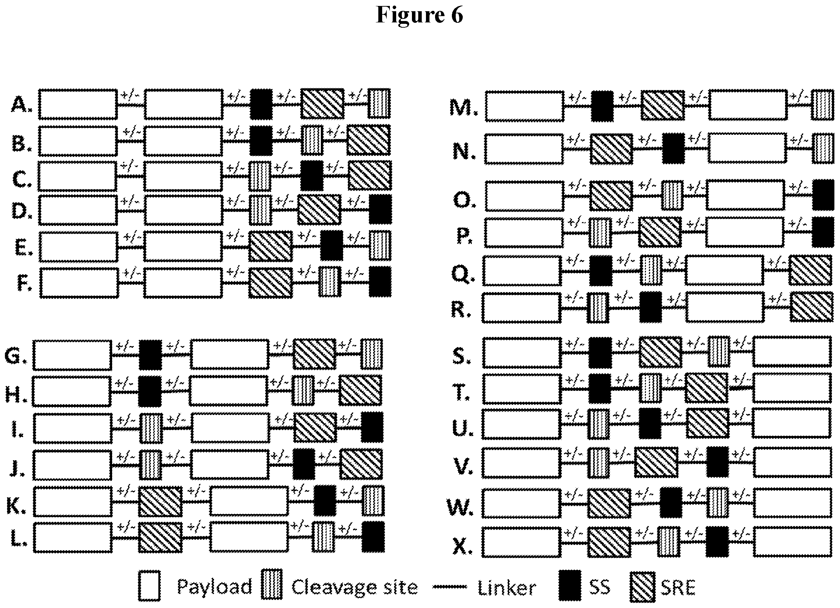

Figures 3 to 6 illustrate representative effector module instances comprising two payloads, i.e. two immunotherapeutic agents. In some aspects, more than two immunotherapeutic agents (payloads) may be included in the effector module under the regulation of the same SRE (e.g., the same DD). The two or more agents may be either directly linked to each other or separated (Figure 3). The SRE may be positioned at the N-terminus of the construct, or the C-terminus of the construct, or in the internal location.

In some aspects, the two or more immunotherapeutic agents may be the same type such as two antibodies, or different types such as a CAR construct and a cytokine IL12. Biocircuits and components utilizing such effector molecules are given in Figures 7-12 in International Publication No. WO2017/180587.

Figure 7 in International Publication No. WO2017/180587 depicts representative configurations of the stimulus and effector module within a biocircuit system. A trans-membrane effector module is activated either by a free stimulus (A) or a membrane bound stimulus (B) which binds to SRE. The response to the stimulus causes the cleavage of the intracellular signal/payload, which activates down-stream effector/payload.

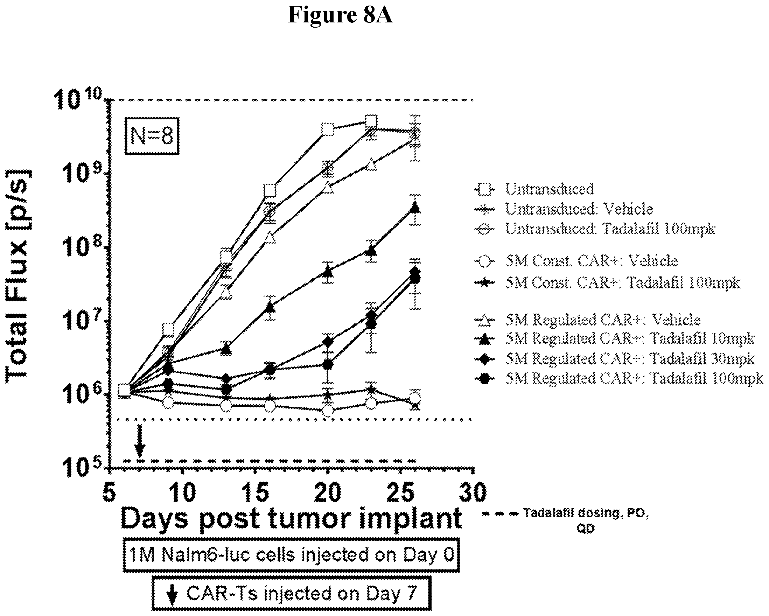

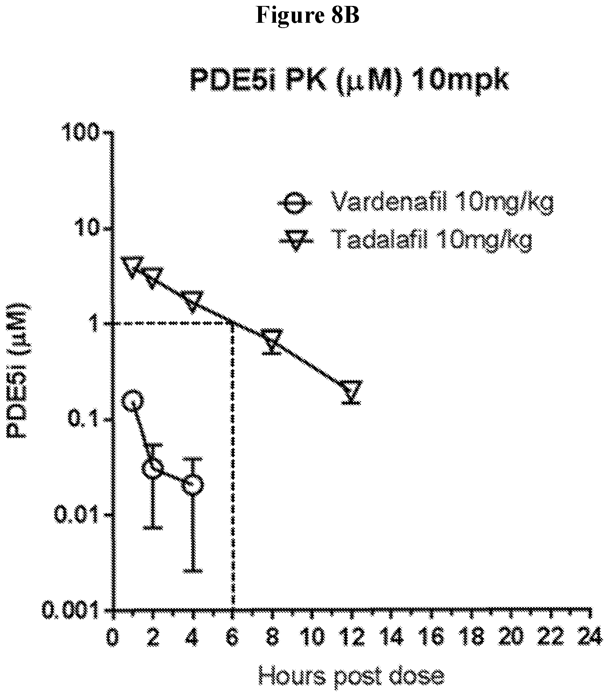

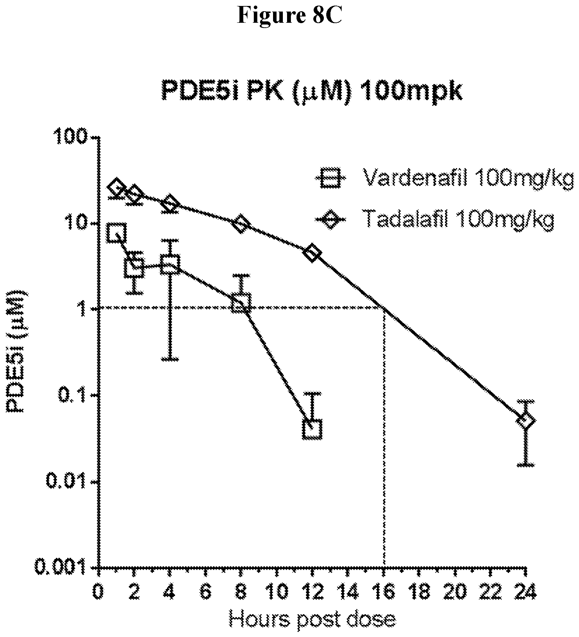

Figure 8 in International Publication No. WO2017/180587 depicts a dual stimulus-dual presenter biocircuit system, where two bound stimuli (A and B) from two different presenters (e.g., different cells) bind to two different effector modules in a single receiver (e.g., another single cell) simultaneously and create a dual-signal to downstream payloads.

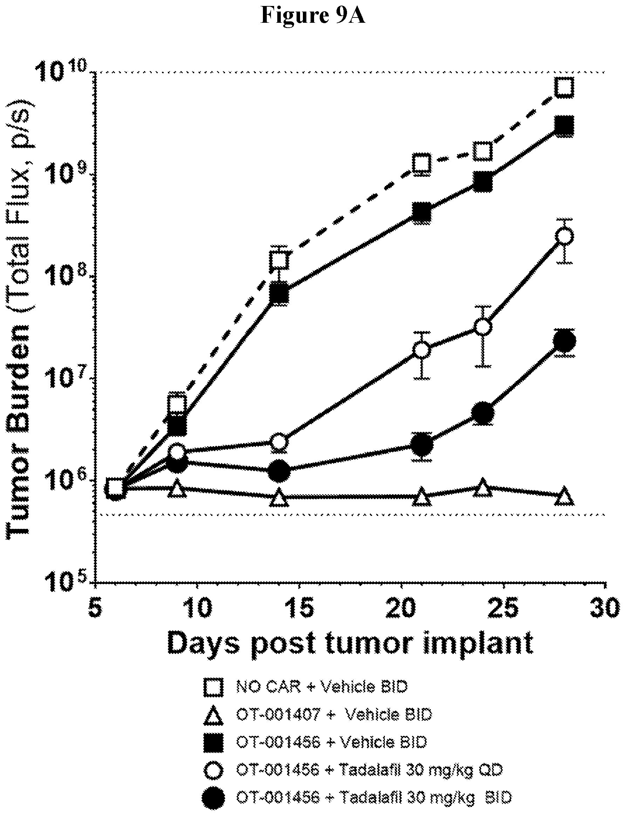

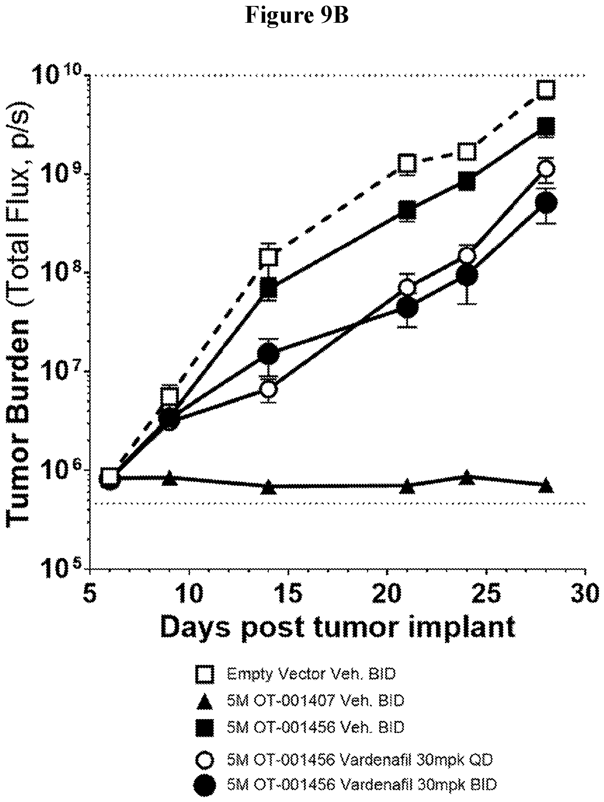

Figure 9 in International Publication No. WO2017/180587 depicts a dual stimulus-single presenter biocircuit system, where two bound stimuli (A and B) from the same presenter (e.g., a single cell) bind to two different effector modules in another single cell simultaneously and create a dual-signal.

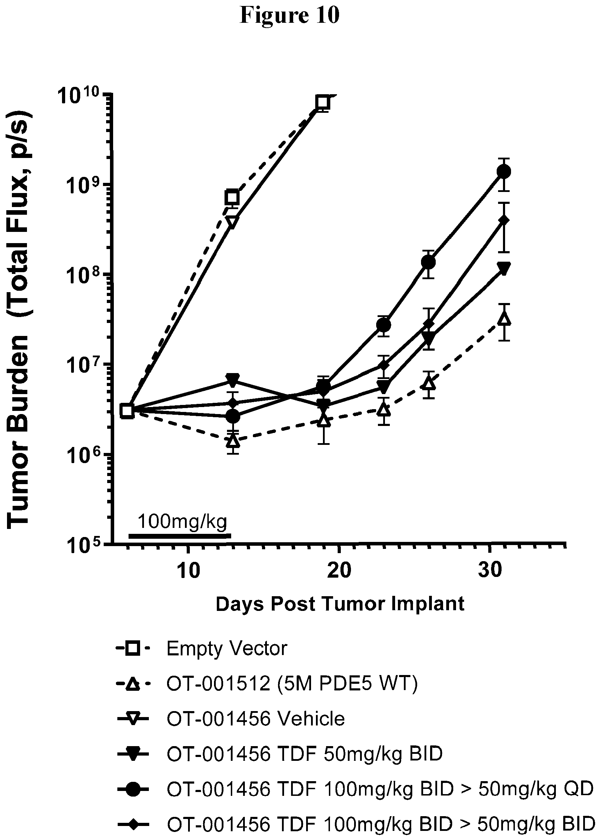

Figure 10 in International Publication No. WO2017/180587 depicts a single-stimulus-bridged receiver biocircuit system. In this configuration, a bound stimulus (A) binds to an effector module in the bridge cell and creates a signal to activate a payload which is a stimulus (B) for another effector module in the final receiver (e.g., another cell).

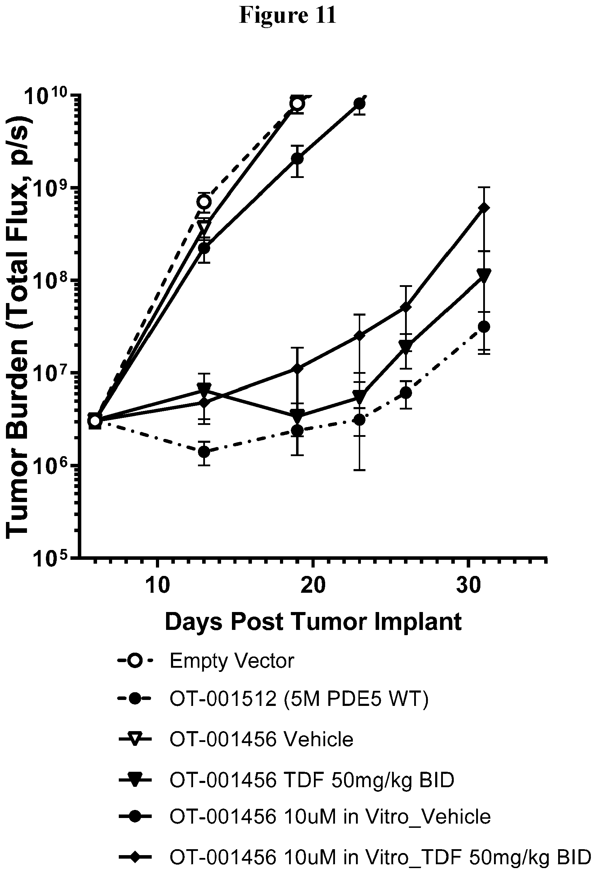

Figure 11 in International Publication No. WO2017/180587 depicts a single stimulus-single receiver biocircuit system, wherein the single receiver contains the two effector modules which are sequentially activated by a single stimulus.

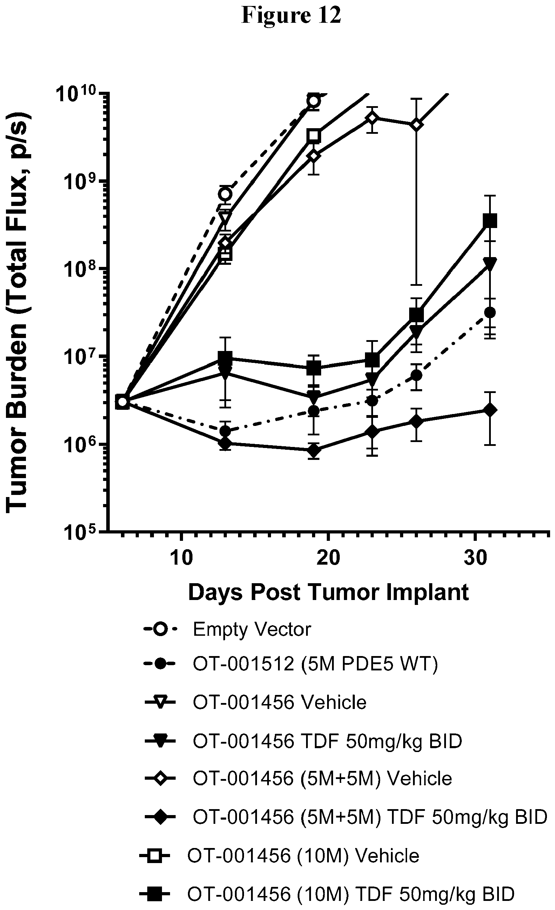

Figure 12 in International Publication No. WO2017/180587 depicts a biocircuit system which requires a dual activation. In this instance, one stimulus must bind the transmembrane effector module first to prime the receiver cell being activated by the other stimulus. The receiver only activates when it senses both stimuli (B).

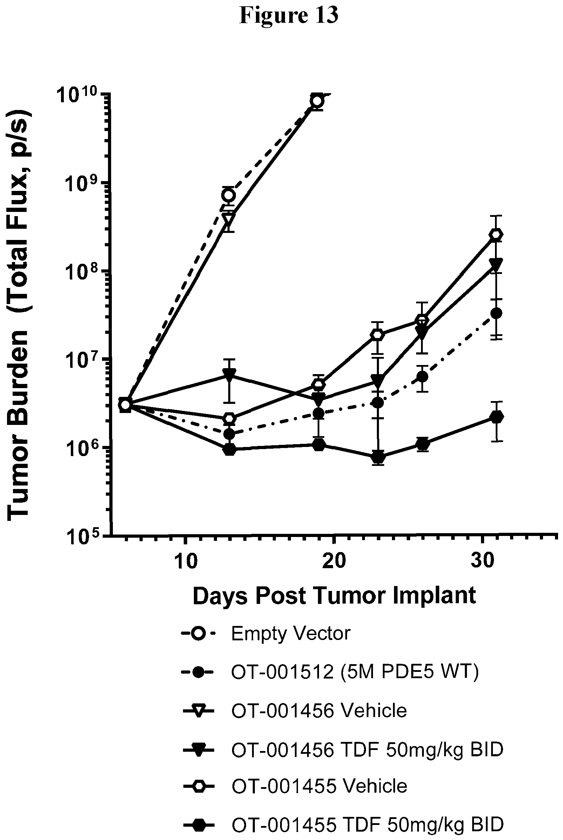

Figure 13 in International Publication No. WO2017/180587 depicts a standard effector module of a chimeric antigen receptor (CAR) system which comprises an antigen binding domain as an SRE, and signaling domain(s) as payload.

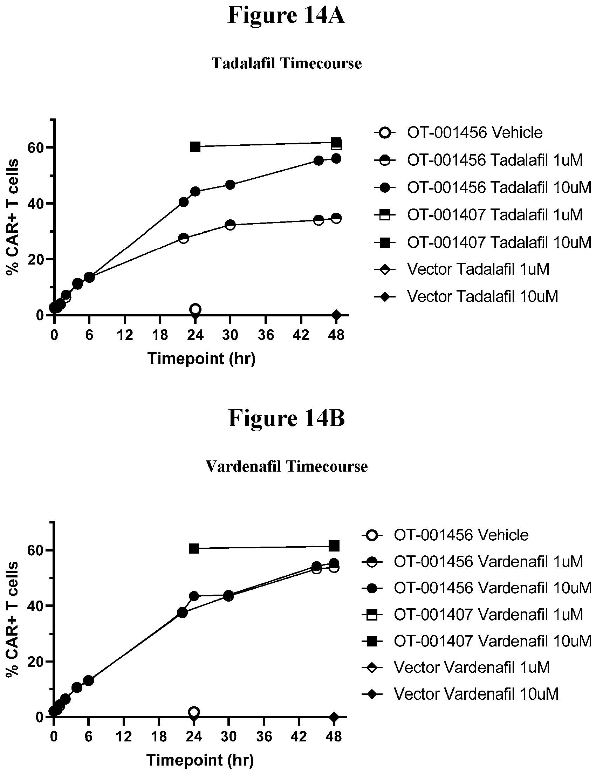

Figure 14 in International Publication No. WO2017/180587 depicts the structure design of a regulatable CAR system, where the trans-membrane effector modules comprise antigen binding domains sensing an antigen and a first switch domain and the intracellular module comprises a second switch domain and signaling domains. A stimulus (e.g., a dimerization small molecule) can dimerize the first and second switch domains and assemble an activated CAR system.

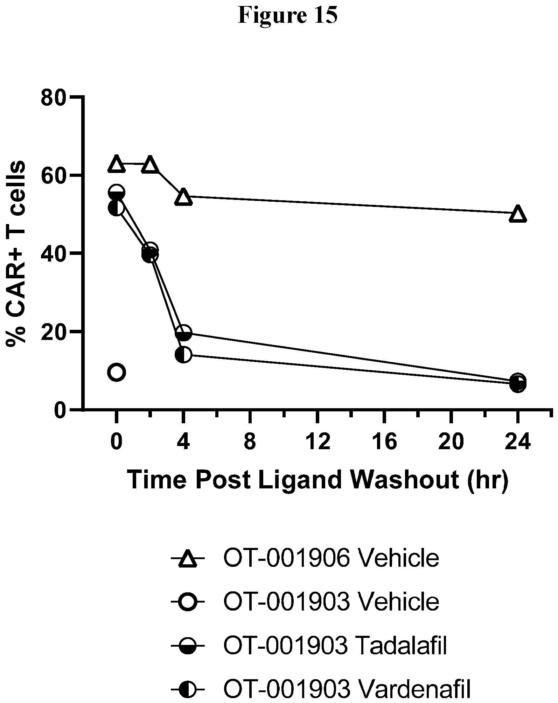

Figure 15 in International Publication No. WO2017/180587 shows schematic representation of CAR systems having one (A) or two (B and C) SREs incorporated into the effector module.

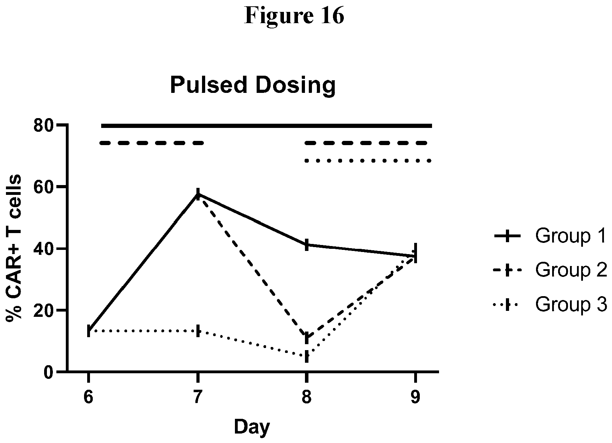

Figure 16 in International Publication No. WO2017/180587 depicts a split CAR design to control T cell activation by a dual stimulus (e.g., an antigen and small molecule). (A) shows normal T cell activation which entails a dual activation of TCR and co-stimulatory receptor. The regular CAR design (B) combines the antigen recognition domain with TCR signaling motif and co-stimulatory motif in a single molecule. The split CAR system separates the components of the regular CAR into two separate effector modules which can be reassembled when a heterodimerizing small molecule (stimulus) is present.

Figure 17 in International Publication No. WO2017/180587 depicts the positive and negative regulation of CAR engineered T cell activation. The absence or presence of a second stimulus can negatively (A) or positively (B) control T cell activation.

Figure 18 in International Publication No. WO2017/180587 shows schematic representation of gated activation of CAR engineered T cells. If a normal cell that has no stimulus (e.g., an antigen) (A) or an antigen that cannot bind to the trans-membrane effector module (B), or only an antigen that activates the trans-membrane effector module and primes the receiver T cell to express the second effector (Fig 18C), the receiver T cell remains inactive. When both stimuli (e.g. two antigens) that bind the trans-membrane effector module and the primed effector, are present on the presenter cell (e.g. a cancer cell), the T cell is activated (D).

As used herein a "payload" or "target payload" is defined as any protein or nucleic acid whose function is to be altered. Payloads may include any coding or non-coding gene or any protein or fragment thereof, or fusion constructs, or antibodies.

Payloads are often associated with one or more SREs (e.g., DDs) and may be encoded alone or in combination with one or more DD in a polynucleotide of the disclosure. Payloads themselves may be altered (at the protein or nucleic acid level) thereby providing for an added layer of tenability of the effector module. For example, payloads may be engineered or designed to contain mutations, single or multiple, which affect the stability of the payload or its susceptibility to degradation, cleavage or trafficking. The combination of a DD which can have a spectrum of responses to a stimulus with a payload which is altered to exhibit a variety of responses or gradations of output signals, e.g., expression levels, produce biocircuits which are superior to those in the art. For example, mutations or substitutional designs such as those created for IL12 in WO2016048903 (specifically in Example 1 therein) may be used in any protein payload in conjunction with a DD of the present disclosure to create dual tunable biocircuits. The ability to independently tune both the DD and the payload greatly increases the scope of uses of the effector modules of the present disclosure.

Effector modules may be designed to include one or more payloads, one or more DDs, one or more cleavage sites, one or more signal sequences, one or more tags, one or more targeting peptides, and one or more additional features including the presence or absence of one or more linkers. Representative effector module instances of the disclosure are illustrated in Figures 2-6. In some aspects, the DD can be positioned at the N-terminal end, or the C-terminal end, or internal of the effector module construct. Different components of an effector module such as DDs, payloads and additional features are organized linearly in one construct, or are separately constructed in separate constructs.

Additionally, effector modules of the present disclosure may further comprise other regulatory moieties such as inducible promoters, enhancer sequences, microRNA sites, and/or microRNA targeting sites that provide flexibility on controlling the activity of the payload. The payloads of the present disclosure may be any natural proteins and their variants, or fusion polypeptides, antibodies and variants thereof, transgenes and therapeutic agents.

The stimulus of the biocircuit system may be, but is not limited to, a ligand, a small molecule, an environmental signal (e.g., pH, temperature, light and subcellular location), a peptide or a metabolite. In one aspect of the present disclosure, the stimulus is a hPDE5 DD binding ligand including Sildenafil and Vardenafil.

Polypeptides of DDs, biocircuit systems and effector modules comprising such DDs and payload constructs, other components, polynucleotides encoding these polypeptides and variants thereof, vectors comprising these polynucleotides, are provided in the present disclosure. The vector may be a plasmid or a viral vector including but not limited to a lentiviral vector, a retroviral vector, a recombinant AAV vector and oncolytic viral vector.

The position of the payload with respect to the DD, within the SRE may be varied to achieve optimal DD regulation. In some instances, the payload may be fused to the N terminus of the DD. In another instance, the payload may be fused to the C terminus of the DDs. An optional start codon nucleotide sequence encoding for methionine may be added to the DD and/or payload.

In some instances, more than one biocircuit system may be used in combination to control various protein functions in the same cell or organism, each of which uses different DD and ligand pair and can be regulated separately.

In some instances, biocircuits of the disclosure may be modified to reduce their immunogenicity. Immunogenicity is the result of a complex series of responses to a substance that is perceived as foreign and may include the production of neutralizing and non-neutralizing antibodies, formation of immune complexes, complement activation, mast cell activation, inflammation, hypersensitivity responses, and anaphylaxis. Several factors can contribute to protein immunogenicity, including, but not limited to protein sequence, route and frequency of administration and patient population. In a preferred instance, protein engineering may be used to reduce the immunogenicity of the compositions of the disclosure. In some instances, modifications to reduce immunogenicity may include those that reduce binding of the processed peptides derived from the sequence of the compositions of the disclosure, to the MHC proteins. For example, amino acid may be modified such that virtually none or a minimal of number of immune epitopes predicted to bind to any prevalent MHC alleles are present in the compositions of the disclosure. Several methods to identify MHC binding epitopes of known protein sequences are known in the art and may be used to score epitopes in the compositions of the present disclosure. Such methods are disclosed in US Patent Publication No. US20020119492, US20040230380, and US 20060148009.

Epitope identification and subsequent sequence modification may be applied to reduce immunogenicity. The identification of immunogenic epitopes may be achieved either physically or computationally. Physical methods of epitope identification may include, for example, mass spectrometry and tissue culture/cellular techniques. Computational approaches that utilize information related to antigen processing, loading and display, structural and/or proteomic data for identifying peptides that may result from antigen processing, and that are likely to have good binding characteristics in the groove of the MHC may also be utilized. One or more mutations may be introduced into the biocircuits of the disclosure to render the identified epitope less or non-immunogenic, while maintaining functionality.

In some instances, protein modifications engineered into the structure of the compositions of the disclosure to interfere with antigen processing and peptide loading such as glycosylation and PEGylation, may also be useful in the present disclosure. Compositions of the disclosure may also be engineered to include non-classical amino acid sidechains. Any of the methods discussed in International Patent Publication No. WO2005051975 for reducing immunogenicity may be useful in the present disclosure.

In one instance, patients may also be stratified according to the immunogenic peptides presented by their immune cells and may be utilized as a parameter to determine patient cohorts that may therapeutically benefit from the compositions of the disclosure.

In some instances, reduced immunogenicity may be achieved by limiting immunoproteasome processing. The proteasome is an important cellular protease that is found in two forms: the constitutive proteasome, which is expressed in all cell types and which contains catalytic subunits and the immunoproteasome that is expressed in cells of the hematopoietic lineage, and which contains different active subunits termed low molecular weight proteins (LMP) namely LMP-2, LMP- 7 and LMP-10. Immunoproteasomes exhibit altered peptidase activities and cleavage site preferences that result in more efficient liberation of many MHC class I epitopes. A well described function of the immunoproteasome is to generate peptides with hydrophobic C terminus that can be processed to fit in the groove of MHC class I molecules. Deol P et al. have shown that immunoproteasomes may lead to a frequent cleavage of specific peptide bonds and thereby to a faster appearance of a certain peptide on the surface of the antigen presenting cells; and enhanced peptide quantities (Deol P et al. (2007) J Immunol 178 (12) 7557-7562). This study indicates that reduced immunoproteasome processing may be accompanied by reduced immunogenicity. In some instances, immunogenicity of the compositions of the disclosure may be reduced by modifying the sequence encoding the compositions of the disclosure to prevent immunoproteasome processing. Biocircuits of the present disclosure may also be combined with immunoproteasome-selective inhibitors to achieve the same effects. Examples of inhibitors useful in the present disclosure include UK-101 (B1i selective compound), IPSI-001, ONX 0914 (PR-957), and PR-924 (IPSI).

In some instances, effector modules of the present disclosure may include one or more degrons to tune expression. As used herein, a "degron" refers to a minimal sequence within a protein that is sufficient for the recognition and the degradation by the proteolytic system. An important property of degrons is that they are transferrable, that is, appending a degron to a sequence confers degradation upon the sequence. In some instances, the degron may be appended to the destabilizing domains, the payload or both. Incorporation of the degron within the effector module of the disclosure, confers additional protein instability to the effector module and may be used to reduce basal expression. In some instances, the degron may be an N-degron, a phospho degron, a heat inducible degron, a photosensitive degron, an oxygen dependent degron. As a non-limiting example, the degron may be an Ornithine decarboxylase degron as described by Takeuchi et al. (Takeuchi J et al. (2008). Biochem J. 2008 Mar 1; 410(2):401-7). Other examples of degrons useful in the present disclosure include degrons described in International patent publication Nos. WO2017004022, WO2016210343, and WO2011062962.

In some instances, the effector modules of the present disclosure may include degrons at their C termini. The degrons may comprise -GG, -RG, -KG, -QG, -WG, -PG, and -AG as the penultimate and the ultimate amino acids of the SREs. Furthermore, certain -2 amino acids (D, E, V, I and L) may be more enriched in the C terminus of the of the effector modules. Other degrons include, but are not limited, to RxxG motif, wherein x is any amino acid, C-terminal twin glutamic acid (EE) motif, and motifs that comprise an arginine at the -3 positions. Degrons may also be selected from the R-3 motif, G-end, R at -3, A-end, A at -2, V at -2 positions. Any of the degrons described in Koren et al., 2018, Cell 173, 1-14, may be useful in the present disclosure. In some aspects, the expression of the effector module may be tuned by altering its overall amino acid composition. In some aspects, the amino acid composition of the effector module may be tuned to reduce basal expression. In some instances, basal expression may be tuned by increasing the number of bulky aromatic residues such as tryptophan (W), phenylalanine (F), and tyrosine (Y) in the effector module. Such bulky amino acids are known to reduce protein stability. In some instances, the amino acid composition of the SREs may be enriched with acidic residues such as, but not limited to, aspartic acid (D) and glutamic acid (E), and positively charged lysine (K), if an increase in the basal expression of the SRE is desired.

In some instances, the endoplasmic reticulum associated degradation (ERAD) pathway may be used to optimize degradation of the payloads described herein e.g. secreted and membrane cargos. In one instance, the effector modules of the disclosure may be directed to the ER E3 ligases by using adaptor proteins or protein domains. The endoplasmic reticulum is endowed with a specialized machinery to ensure proteins deployed to the distal secretory pathway are correctly folded and assembled into native oligomeric complexes. Proteins failing to meet this conformational standard are degraded by the ERAD pathway, a process through which folding defective proteins are selected and ultimately degraded by the ubiquitin proteasome system. ERAD proceeds through four main steps involving substrate selection, dislocation across the ER membrane, covalent conjugation with polyubiquitin, and proteasome degradation. Any of these steps may be modulated to optimize the degradation of the payloads and the effector modules described herein. Protein adaptors within the ER membrane, link substrate recognition to the ERAD machinery (herein referred to as the "dislocon"), which causes the dislocation of the proteins from the ER. Non-limiting examples of protein adaptors that may be used to optimize ERAD pathway degradation include, but are not limited to SEL1L (an adaptor that links glycan recognition to the dislocon), Erlins (intermembrane substrate adaptors), Insigs (client specific adaptors), F-Box proteins (act as adaptors for dislocated glycoproteins in the cytoplasm) and viral-encoded adaptors.

According to the present disclosure, novel destabilizing domains derived from human hPDE5 (cGMP-specific phosphodiesterase type 5) protein are provided. The destabilizing mutants are derived from the human PDE5 protein, comprising the amino acid sequence of SEQ ID NO: 1 (encoded by the nucleic acid sequence of SEQ ID NO: 2). The hPDE5 DD mutant may also comprise more than one mutation in the catalytic domain of human PDE5 of SEQ ID NO: 3 (encoded by nucleic acid sequence of SEQ ID NO: 339), e.g., two, three, four, five or more mutations. These hPDE5 DDs can bind to Sildenafil and/or Vardenafil and be stabilized.

### Destabilizing Domains (DDs)

As used herein, the term "destabilizing domains (DDs)" refers to protein domains that are unstable and degraded in the absence of ligand, but whose stability is rescued by binding to a high affinity cell-permeable ligand. Destabilizing domains (DDs) can be appended to a target protein of interest (POI) and can convey its destabilizing property to the protein of interest, causing protein degradation. The presence, absence or an amount of a small molecule ligand that binds to or interacts with the DD, can, upon such binding or interaction modulate the stability of the payload(s) and consequently the function of the payload. A protein domain with destabilizing property (e.g. a DD) is used in conjunction with a cell-permeable ligand to regulate any protein of interest when it is fused with the destabilizing domain. DDs render the attached protein of interest unstable in the absence of a DD-binding ligand such that the protein is rapidly degraded by the ubiquitin-proteasome system of the cell. However, when a specific small molecule ligand binds its intended DD as a ligand binding partner, the instability is reversed and protein function is restored. The conditional nature of DD stability allows a rapid and non-perturbing switch from stable protein to unstable substrate for degradation. Moreover, its dependency on the concentration of its ligand further provides tunable control of degradation rates. Depending on the degree of binding and/or interaction the altered function of the payload may vary, hence providing a "tuning" of the payload function.

Due to its reversibility, specificity and the fast and easy regulation on protein level, the post-transcriptional tuning system provides a useful system for gene regulation. Furthermore, the regulation may be dose-dependent, thereby altering the protein-turnover rate to transform a short-lived or no detectable protein into a protein that functions for a precisely controlled period of time (Iwamoto et al., Chem. Biol. 2010, 17: 981-988).

In some instances, the desired characteristics of the DDs may include, but are not limited to, low protein levels in the absence of a ligand of the DD (i.e. low basal stability), large dynamic range, robust and predictable dose-response behavior, and rapid kinetics of degradation. Candidate DDs that bind to a desired ligand but not endogenous molecules may be preferred.

Candidate destabilizing domain sequence identified from protein domains of known wildtype proteins (as a template) may be mutated to generate libraries of mutants based on the template candidate domain sequence. Mutagenesis strategies used to generate DD libraries may include site-directed mutagenesis e.g. by using structure guided information, or random mutagenesis e.g. using error-prone PCR, or a combination of both. In some instances, destabilizing domains identified using random mutagenesis may be used to identify structural properties of the candidate DDs that may be required for destabilization, which may then be used to further generate libraries of mutations using site directed mutagenesis.

In some instances, novel DDs may be identified by mutating one or more amino acids in the candidate destabilizing domain to an amino acid that is vicinal to the mutation site. As used herein a vicinal amino acid refers to an amino acid that is located 1, 2, 3, 4, 5 or more amino acids upstream or downstream of the mutation site in the linear sequence and/or the crystal structure of the candidate destabilizing domain. In some instances, the vicinal amino acid may be a conserved amino acid (with similar physicochemical properties as the amino acid at the mutation site), a semi-conserved amino acid (e.g. negatively to positively charge amino acid) or a non-conserved amino acid (with different physicochemical properties than the amino acid at the mutation site).

In some instances, DD mutant libraries may be screened for mutations with altered, preferably higher binding affinity to the ligand, as compared to the wild type protein. DD libraries may also be screened using two or more ligands and DD mutations that are stabilized by some ligands but not others may be preferentially selected. DD mutations that bind preferentially to the ligand compared to a naturally occurring protein may also be selected. Such methods may be used to optimize ligand selection and ligand binding affinity of the DD. Additionally, such approaches can be used to minimize deleterious effects caused by off-target ligand binding.

In some instances, suitable DDs may be identified by screening mutant libraries using barcodes. Such methods may be used to detect, identify and quantify individual mutant clones within the heterogeneous mutant library. Each DD mutant within the library may have distinct barcode sequences (with respect to each other). In other instances, the polynucleotides can also have different barcode sequences with respect to 2, 3, 4, 5, 6, 7, 8, 9, 10 or more nucleic acid bases. Each DD mutant within the library may also comprise a plurality of barcode sequences. When used in plurality barcodes may be used such that each barcode is unique to any other barcode. Alternatively, each barcode used may not be unique, but the combination of barcodes used may create a unique sequence that can be individually tracked. The barcode sequence may be placed upstream of the SRE, downstream of the SRE, or in some instances may be placed within the SRE. DD mutants may be identified by barcodes using sequencing approaches such as Sanger sequencing, and next generation sequencing, but also by polymerase chain reaction and quantitative polymerase chain reaction. In some instances, polymerase chain reaction primers that amplify a different size product for each barcode may be used to identify each barcode on an agarose gel. In other instances, each barcode may have a unique quantitative polymerase chain reaction probe sequence that enables targeted amplification of each barcode.

Inventors of the present disclosure investigated several human proteins and identified novel human DDs which can confer its instability features to the fused payload and facilitate the rapid degradation of the fusion polypeptide in the absence of its ligand but stabilize the fused payload in response to the binding to its ligand. Specifically, the new DDs are derived from human PDE5 protein.

### Human PDE5 mutants

In some instances, DDs of the disclosure may be derived from human cGMP-specific phosphodiesterase type 5 (PDE5); gene name: PDE5A (herein referred to as "hPDE5"). hPDE5 (phosphodiesterase 5) is a member of the 3,5'-cyclic nucleotide phosphodiesterase family which degrades/hydrolyzes cyclic GMP (cGMP) into its inactive form, GMP, and regulates cGMP signaling. The cGMP/PDE 5 signaling contributes to the development of hypertension in the vasculature, the central nervous system, and the kidney. hPDE5 is known to bind to small molecule such as Sildenafil, Vardenafil, Tadalafil, Avanafil, Lodenafil, Mirodenafil, Udenafil, Benzamidenafil, Dasantafil, Beminafil, SLx-2101, LAS 34179, UK-343,664, UK-357903, UK-371800, and BMS-341400. Sildenafil, Vardenafil, Avanafil, and Tadalafil are hPDE5 inhibitors clinically approved for the treatment of erectile dysfunction. In particular, the binding of these small molecules to hPDE5 occurs within the catalytic domain. In some instances, the DDs of the disclosure may be derived from the catalytic domain of hPDE5 (SEQ ID NO: 3) includes residues 535 to 860 of hPDE5 (SEQ ID NO: 1; GenBank Access NO. 076074.2) which may be stabilized by ligands such as small molecule inhibitors of hPDE5 e.g. Sildenafil and Vardenafil. As used herein the term "PDE5 WT" or "hPDE5 WT", refers to the human wildtype PDE5 protein sequence, which is defined as SEQ ID NO: 1, with the GenBank Access NO. 076074.2. In some instances, DDs of the present disclosure may be identified by utilizing a cocktail of hPDE5 inhibitors. In other instances, the suitable DDs may be identified by screening first with one hPDE5 inhibitor and subsequently screening with a second hPDE5 inhibitor.

In one instance, the hPDE5 derived DD may comprise the amino acid sequence of UniProt ID: 076074 (SEQ ID NO: 1) or a portion or a fragment thereof. In another instance, the hPDE5 derived DD may comprise the catalytic domain of UniProt ID: 076074, spanning from amino acid position 535 to position 860 (SEQ ID NO: 3); encoded by SEQ ID NO: 339. In addition to the catalytic domain, hPDE5 derived DDs may also comprise one or more GAF domains and/or the C terminal portion that extends beyond the catalytic domain. In some instance, the hPDE5 derived DD may be identified by testing constructs generated by truncating the 5' and/or the 3' end of hPDE5. In one instance, the hPDE5 derived DD may be truncated and the smallest hPDE5 based DD may be identified. In another instance, the hPDE5 derived DD may include amino acids from position 535 to 836 of SEQ ID NO: 1 which removes the C terminal helix. In another instance, the hPDE5 derived DD may consist of amino acids from position 567 to 860 of UniProt ID: 076074 (SEQ ID NO: 1), or position 590 to 860 of UniProt ID: 076074 (SEQ ID NO: 1), which removes a portion of the N terminal domain. In another instance, the hPDE5 derived DD may consist of amino acids from position 590 to 836 of UniProt ID: 076074 (SEQ ID NO: 1), which removes a portion of the N terminal domain and the C terminal helix. The DD may include amino acids from position 535 to position 875 of UniProt ID: 076074 (SEQ ID NO: 1). In another instance, the hPDE5 derived DD may consist of amino acids from position 466 to 875 of UniProt ID: O76074 (SEQ ID NO: 1) or position 420 to 875 of UniProt ID: 076074 (SEQ ID NO: 1).

According to the present disclosure, several hPDE5 destabilizing mutations were discovered by site directed mutagenesis of the catalytic domain of wildtype human PDE5 using site directed mutagenesis. The destabilization of the mutants in the absence of its binding ligands is tested. Binding to hPDE5 ligands, Sildenafil, Tadalafil and Vardenafil to human PDE5 was tested and ligand dependent stabilization was characterized. Based on the structural analysis of hPDE5 bound to Sildenafil, several residues were selected for mutagenesis. In some instances one or more of the residues described herein may be mutagenized to obtain hPDE5 derived DDs. The tryptophan at position 853 of SEQ ID NO:1 may be mutated to phenylalanine to induce hydrophobic packing near binding site, while maintaining pi bond with the nearby tryptophan at position 772. The isoleucine at position 821 may be mutated to valine or alanine, which results in hydrophobic packing near binding site. The tyrosine at position 829 may be mutated to isoleucine, valine or alanine, which results in hydrophobic packing near binding site. The aspartate at position 656 may be mutated to asparagine or leucine to break up the charge and the salt bridge on loop/helix. The tyrosine at position 728 may be mutated to phenylalanine or leucine to break the salt bridges at position 732 and to affect hydrophobic packing. Alternatively, the arginine at position 732 may be mutated to lysine or leucine to generate the inverse of the effects of mutating tyrosine at position 728. The methionine at position 625 may be mutated to leucine or isoleucine to alter packing in away from the binding site. In some instances, destabilizing mutations that do not affect ligand binding may be preferentially selected.

In some instances, new destabilizing domains of the present disclosure are derived from the catalytic domain of human PDE5 protein comprising the amino acid sequence of SEQ ID NO: 3. In some aspects, the destabilizing mutant domain may comprise one, two, three, four, five or more mutations such as but not limited to M625I, D656L, Y728L, R732L, F736A, F787A, I821A, Y829A and W853F. The DDs of the disclosure may further include additional mutations e.g. E535D, E536G, Q541R, K555R, S560G, F559L, F561L, F564L, F564S, S766F, V585A, N587S, K591E, I599V, K604E, K608E, N609H, K630R, K633E, N636S, 1648V, N661S, S663P, L675P, Y676D, Y676N, C677R, H678R, D687A, T711A, T712S, D724N, L738H, N742S, F744L, L746S, F755L, A762S, D764V, D764N, D764G, K795E, L797F, I799T, L804P, T802P, S815C, M816A, M816T, I824T, C839S, F840S, and K852E. In some instances, any of the mutation sites disclosed herein may be mutated to any of the known amino acids such as Histidine, Alanine, Isoleucine, Arginine, Leucine, Aspartic acid, Lysine, Cysteine, Methionine, Glutamic acid, Phenylalanine, Glutamine, Threonine, Glycine, Tryptophan, Proline, Valine, Serine, Tyrosine, Asparagine, Selenocysteine, Pyrrolysine. In some instances, DDs of the present disclosure may be stabilized by ligands such as Sildenafil, Vardenafil, Tadalafil, Avanafil, Lodenafil, Mirodenafil, Udenafil, Benzamidenafil, Dasantafil, and Beminafil.

The amino acid sequences of the destabilizing domains encompassed in the disclosure have at least about 40%, 50 or 60% identity, further at least about 70% identity, preferably at least about 75% or 80% identity, more preferably at least about 85%, 86%, 87%, 88%, 89% or 90% identity, and further preferably at least about 91%, 92%, 93%, 94%, 95%, 96%, 97%, 98% or 99% identity to the amino acid sequence set forth therein. Percent identity may be determined, for example, by comparing sequence information using the advanced BLAST computer program, including version Magic-BLAST 1.2.0, available from the National Institutes of Health. The BLAST program is based on the alignment method discussed in Karl and Altschul (1990) Proc. Natl. Acad. Sci USA, 87:2264-68.

Several hPDE5 destabilizing mutants were identified by structure guided mutagenesis and are provided in Table 1. The position of the mutated amino acids listed in Table 1 is relative to the full length hPDE5 of SEQ ID NO:1. The domains described in Table 1 include the catalytic domain of hPDE5 (e.g. 535-860 of SEQ ID NO:1). In some instances, the hPDE5 DDs described in Table 1 may include a methionine at the N terminal of the catalytic domain of hPDE5, i.e. amino acid 535-860 of PDE5 WT. In Table 1, the mutated amino acids are in bold and underlined.

**Table 1: hPDE5 DDs**

| **hPDE5 mutant description** | **Amino Acid Sequence** | **AA SEQ ID NO** | **NA SEQ ID NO** |
|---|---|---|---|
| hPDE5 (Amino acid 535-860 of WT, W853F) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKFQALAEQQ | 5 | 350 |
| hPDE5 (Amino acid 535-860 of WT, I821A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFADAICLQLYEALTHVSEDCFPLLDGCRKNRQKWQALAEQQ | 6 | 351 |
| hPDE5 (Amino acid 535-860 of WT, Y829A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLAEALTHVSEDCFPLLDGCRKNRQKWQALAEQQ | 7 | 352 |
| hPDE5 (Amino acid 535-860 of WT, F787A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFADQGDRERKELNIEPTDLMNREKKN KIPSMQVGFIDAICLQLYEALTHVSEDCFPLLDGCRKNRQKWQALAEQQ | 8 | 353 |
| hPDE5 (Amino acid 535-860 of WT, F736A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFAELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 9 | 354; 355 |
| hPDE5 (Amino acid 535-860 of WT, D656L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLLHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 10 | 356 |
| hPDE5 (Amino acid 535-860 of WT, Y728L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALLIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 11 | 357 |
| hPDE5 (Amino acid 535-860 of WT, R732L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 12 | 358; 359 |
| hPDE5 (Amino acid 535-860 of WT, M625I) | EETRELQSLAAAVVPSAQTLKITDFSFSDFELSDLETALCTIRMFTDLNLVQNFQM KHEVLCRWILSVKKNYRKNVAYHNWRHAFNTAQCIFAALKAGKIQNKLTDLEIL ALLIAALSHDLDHRGVNNSYIQRSEHPLAQLYCHSIMEHHHFDQCLMILNSPGNQI LSGLSIEEYKTTLKIIKQAILATDLALYIKRRGEFFELIRKNQFNLEDPHQKELFLAM LMTACDLSAITKPWPIQQRIAELVATEFFDQGDRERKELNIEPTDLMNREKKNKIP SMQVGFIDAICLQLYEALTHVSEDCFPLLDGCRKNRQKWQALAEQQ | 13 | 360 |
| Methionine; hPDE5 (Amino acid 535-860 of WT; W853F) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKFQALAEQQ | 340 | 14 |
| Methionine; hPDE5 (Amino acid 535-860 of WT, I821A) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFFELIRKNQFNLEDPHQKELF LAMLMTACDLSAITKPWPIQQRIAELVATEFFDQGDRERKELNIEPTDLMNREKK NKIPSMQVGFADAICLQLYEALTHVSEDCFPLLDGCRKNRQKWQALAEQQ | 341 | 15 |
| Methionine; hPDE5 (Amino acid 535-860 of WT, Y829A) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFFELIRKNQFNLEDPHQKELF LAMLMTACDLSAITKPWPIQQRIAELVATEFFDQGDRERKELNIEPTDLMNREKK NKIPSMQVGFIDAICLQLAEALTHVSEDCFPLLDGCRKNRQKWQALAEQQ | 342 | 16 |
| Methionine; hPDE5 (Amino acid 535-860 of WT, F787A) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFFELIRKNQFNLEDPHQKELF LAMLMTACDLSAITKPWPIQQRIAELVATEFADQGDRERKELNIEPTDLMNREKK NKIPSMQVGFIDAICLQLYEALTHVSEDCFPLLDGCRKNRQKWQALAEQQ | 343 | 17 |
| Methionine; hPDE5 (Amino acid 535-860 of WT, F736A) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFAELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 344 | 18 |
| Methionine; hPDE5 (Amino acid 535-860 of WT, D656L) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLLHRGVNNSYIQRSEHPLAQLYCHSIMEHHHFDQCLMILNSPG NQILSGLSIEEYKTTLKIIKQAILATDLALYIKRRGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 345 | 19 |
| Methionine; hPDE5 (Amino acid 535-860 of WT, Y728L) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALLIKRRGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 346 | 20 |
| Methionine; hPDE5 (Amino acid 535-860 of WT, R732L) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRLGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 238 | 21 |
| Methionine; hPDE5 (Amino acid 535-860 of WT, M625I) | MEETRELQSLAAAVVPSAQTLKITDFSFSDFELSDLETALCTIRMFTDLNLVQNFQ MKHEVLCRWILSVKKNYRKNVAYHNWRHAFNTAQCIFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 347 | 22 |
| hPDE5 (Amino acid 535-860 of WT, H653A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSADLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 348 | 361 |
| hPDE5 (Amino acid 535-860 of WT, D764A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFFELIRKNQFNLEDPHQKELFL AMLMTACALSAITKPWPIQQRIAELVATEFFDQGDRERKELNIEPTDLMNREKKN KIPSMQVGFIDAICLQLYEALTHVSEDCFPLLDGCRKNRQKWQALAEQQ | 349 | 362 |
| hPDE5 (Amino acid 535-860 of WT, E682A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMAHHHFDQCLMILNSPGN QILSGLSIEEYKTTLKIIKQAILATDLALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 8383 | 8385 |
| hPDE5 (Amino acid 535-860 of WT, D724A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATALALYIKRRGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 8384 | 8386 |

Also provided herein are hPDE5 mutants identified by random mutagenesis screening, using a combination of nucleotide analog mutagenesis and error-prone PCR, to generate libraries of mutants. The hPDE5 DDs identified by random mutagenesis are provided in Table 2.

**Table 2: hPDE5 DDs generated by random mutagenesis**

| **Description** | **Library ID** | **Amino acid sequence** | **AA SEQ ID NO:** | **NA SEQ ID NO:** |
|---|---|---|---|---|
| hPDE5 (Amino acid 535-860 of WT, L573M, F735L) | LibC000009 | EETRELQSLAAAVVPSAQTLKITDFSFSDFELSDLETAMCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGELFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8387 | 8439 |
| hPDE5 (Amino acid 535-860 of WT, R658H) | LibC000010 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHHGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8388 | 8440 |
| hPDE5 (Amino acid 535-860 of WT, S562G, L727P, R847T) | LibC000011 | EETRELQSLAAAVVPSAQTLKITDFSFGDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLAPYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCTKNRQKWQALAEQQ | 8389 | 8441 |
| hPDE5 (Amino acid 535-860 of WT, Q666H) | LibC000012 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIHRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8390 | 8442 |
| hPDE5 (Amino acid 535-860 of WT, L781P) | LibC000013 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAEPVATEFFDQGDRERKELNIEPTDLMNREKKNKIPSM QVGFIDAICLQLYEALTHVSEDCFPLLDGCRKNRQKWQALAEQQ | 8391 | 8443 |
| hPDE5 (Amino acid 535-860 of WT, A767E) | LibC000014 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSEIT KPWPIQQRIAELVATEFFDQGDRERKELNIEPTDLMNREKKNKIPSM QVGFIDAICLQLYEALTHVSEDCFPLLDGCRKNRQKWQALAEQQ | 8392 | 8444 |
| hPDE5 (Amino acid 535-860 of WT, T571S, V585M, T723S) | LibC000015 | EETRELQSLAAAVVPSAQTLKITDFSFSDFELSDLESALCTIRMFTDL NLMQNFQMKHEVLCRWILSVKKNYRKNVAYHNWRHAFNTAQCM FAALKAGKIQNKLTDLEILALLIAALSHDLDHRGVNNSYIQRSEHPL AQLYCHSIMEHHHFDQCLMILNSPGNQILSGLSIEEYKTTLKIIKQAIL ASDLALYIKRRGEFFELIRKNQFNLEDPHQKELFLAMLMTACDLSAI | 8393 | 8445 |
| | | TKPWPIQQRIAELVATEFFDQGDRERKELNIEPTDLMNREKKNKIPS MQVGFIDAICLQLYEALTHVSEDCFPLLDGCRKNRQKWQALAEQQ | | |
| hPDE5 (Amino acid 535-860 of WT, Q743L) | LibC000016 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNLFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8394 | 8446 |
| hPDE5 (Amino acid 535-860 of WT, Y709H, F787V) | LibC000017 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEHKTTLKIIKQAILA TDLALYIKRRGEFFELIRKNQFNLEDPHQKELFLAMLMTACDLSAIT KPWPIQQRIAELVATEFVDQGDRERKELNIEPTDLMNREKKNKIPSM QVGFIDAICLQLYEALTHVSEDCFPLLDGCRKNRQKWQALAEQQ | 8395 | 8447 |
| hPDE5 (Amino acid 535-860 of WT, N605Y, I715K) | LibC000018 | EETRELQSLAAAVVPSAQTLKITDFSFSDFELSDLETALCTIRMFTDL NLVQNFQMKHEVLCRWILSVKKYYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKKIKQAIL ATDLALYIKRRGEFFELIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ | 8396 | 8448 |
| hPDE5 (Amino acid 535-860 of WT, F564I, N662Y, H685L, L693P, F736I) | LibC000019 | EETRELQSLAAAVVPSAQTLKITDFSFSDIELSDLETALCTIRMFTDL NLVQNFQMKHEVLCRWILSVKKNYRKNVAYHNWRHAFNTAQCMF AALKAGKIQNKLTDLEILALLIAALSHDLDHRGVNYSYIQRSEHPLA QLYCHSIMEHHLFDQCLMIPNSPGNQILSGLSIEEYKTTLKIIKQAILA TDLALYIKRRGEFIELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8397 | 8449 |
| hPDE5 (Amino acid 535-860 of WT, A722V, F755L, M760I) | LibC000020 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILV TDLALYIKRRGEFFELIRKNQFNLEDPHQKELLLAMLITACDLSAITK PWPIQQRIAELVATEFFDQGDRERKELNIEPTDLMNREKKNKIPSMQ VGFIDAICLQLYEALTHVSEDCFPLLDGCRKNRQKWQALAEQQ | 8398 | 8450 |
| hPDE5 (Amino acid 535-860 of WT, V594I) | LibC000021 | EETRELQSLAAAVVPSAQTLKITDFSFSDFELSDLETALCTIRMFTDL NLVQNFQMKHEILCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8399 | 8451 |
| hPDE5 (Amino acid 535-860 of WT, H592R) | LibC000022 | EETRELQSLAAAVVPSAQTLKITDFSFSDFELSDLETALCTIRMFTDL NLVQNFQMKREVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8400 | 8452 |
| hPDE5 (Amino acid 535-860 of WT, E858V) | LibC000023 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAVQQ | 8401 | 8453 |
| hPDE5 (Amino acid 535-860 of WT, T784I) | LibC000024 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVAIEFFDQGDRERKELNIEPTDLMNREKKNKIPSM QVGFIDAICLQLYEALTHVSEDCFPLLDGCRKNRQKWQALAEQQ | 8402 | 8454 |
| hPDE5 (Amino acid 535-860 of WT, S542L, E708V, W772C) | LibC000001 | EETRELQLLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEVYKTTLKIIKQAILA TDLALYIKRRGEFFELIRKNQFNLEDPHQKELFLAMLMTACDLSAIT KPCPIQQRIAELVATEFFDQGDRERKELNIEPTDLMNREKKNKIPSM QVGFIDAICLQLYEALTHVSEDCFPLLDGCRKNRQKWQALAEQQ | 8403 | 8455 |
| hPDE5 (Amino acid 535-860 of WT, I700F, E796G) | LibC000002 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQFLSGLSIEEYKTTLKIIKQAIL ATDLALYIKRRGEFFELIRKNQFNLEDPHQKELFLAMLMTACDLSAI TKPWPIQQRIAELVATEFFDQGDRERKGLNIEPTDLMNREKKNKIPS MQVGFIDAICLQLYEALTHVSEDCFPLLDGCRKNRQKWQALAEQQ | 8404 | 8456 |
| hPDE5 (Amino acid 535-860 of WT, D724G, K848N) | LibC000003 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TGLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRNNRQKWQALAEQQ | 8405 | 8457 |
| hPDE5 (Amino acid 535-860 of WT, T712M, M758I, Q859R) | LibC000004 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTMLKIIKQAIL ATDLALYIKRRGEFFELIRKNQFNLEDPHQKELFLAILMTACDLSAIT KPWPIQQRIAELVATEFFDQGDRERKELNIEPTDLMNREKKNKIPSM QVGFIDAICLQLYEALTHVSEDCFPLLDGCRKNRQKWQALAERQ | 8406 | 8458 |
| hPDE5 (Amino acid 535-860 of WT, I556S, E796D) | LibC000005 | EETRELQSLAAAVVPSAQTLKSTDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKDLNIEPTDLMNREKKNKIPSM QVGFIDAICLQLYEALTHVSEDCFPLLDGCRKNRQKWQALAEQQ | 8407 | 8459 |
| hPDE5 (Amino acid 535-860 of WT, I680S) | LibC000006 | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSSMEHHHFDQCLMILNSPGNQILSGLSIEEYKTTLKIIKQAIL ATDLALYIKRRGEFFELIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ | 8408 | 8460 |
| hPDE5 (Amino acid 535-860 of WT, H613L, D724Y, F755Y) | LibC000007 | EETRELQSLAAAVVPSAQTLKITDFSFSDFELSDLETALCTIRMFTDL NLVQNFQMKHEVLCRWILSVKKNYRKNVAYLNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TYLALYIKRRGEFFELIRKNQFNLEDPHQKELYLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8409 | 8461 |
| hPDE5 (Amino acid 535-860 of WT, A611T) | LibC000008 | EETRELQSLAAAVVPSAQTLKITDFSFSDFELSDLETALCTIRMFTDL NLVQNFQMKHEVLCRWILSVKKNYRKNVTYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA | 8410 | 8462 |
| | | TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | | |
| hPDE5 (Amino acid 535-860 of WT, N661S) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVSNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8411 | 8463 |
| hPDE5 (Amino acid 535-860 of WT, L569M, T833S) | - | EETRELQSLAAAVVPSAQTLKITDFSFSDFELSDMETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALSHVSEDCFPLLDGCRKNRQKWQALAEQQ | 8412 | 8464 |
| hPDE5 (Amino acid 535-860 of WT, I768N) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSANT KPWPIQQRIAELVATEFFDQGDRERKELNIEPTDLMNREKKNKIPSM QVGFIDAICLQLYEALTHVSEDCFPLLDGCRKNRQKWQALAEQQ | 8413 | 8465 |
| hPDE5 (Amino acid 535-860 of WT, R607W) | - | EETRELQSLAAAVVPSAQTLKITDFSFSDFELSDLETALCTIRMFTDL NLVQNFQMKHEVLCRWILSVKKNYWKNVAYHNWRHAFNTAQCM FAALKAGKIQNKLTDLEILALLIAALSHDLDHRGVNNSYIQRSEHPL AQLYCHSIMEHHHFDQCLMILNSPGNQILSGLSIEEYKTTLKIIKQAIL ATDLALYIKRRGEFFELIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ | 8414 | 8466 |
| hPDE5 (Amino acid 535-860 of WT, N620S) | - | EETRELQSLAAAVVPSAQTLKITDFSFSDFELSDLETALCTIRMFTDL NLVQNFQMKHEVLCRWILSVKKNYRKNVAYHNWRHAFSTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8415 | 8467 |
| hPDE5 (Amino acid 535-860 of WT, L554R, Q589E, M691I) | - | EETRELQSLAAAVVPSAQTRKITDFSFSDFELSDLETALCTIRMFTDL NLVQNFEMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLIILNSPGNQILSGLSIEEYKTTLKIIKQAILAT DLALYIKRRGEFFELIRKNQFNLEDPHQKELFLAMLMTACDLSAITK PWPIQQRIAELVATEFFDQGDRERKELNIEPTDLMNREKKNKIPSMQ VGFIDAICLQLYEALTHVSEDCFPLLDGCRKNRQKWQALAEQQ | 8416 | 8468 |
| hPDE5 (Amino acid 535-860 of WT, W615R, T723R, A762T, E808G) | - | EETRELQSLAAAVVPSAQTLKITDFSFSDFELSDLETALCTIRMFTDL NLVQNFQMKHEVLCRWILSVKKNYRKNVAYHNRRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA RDLALYIKRRGEFFELIRKNQFNLEDPHQKELFLAMLMTTCDLSAIT KPWPIQQRIAELVATEFFDQGDRERKELNIEPTDLMNRGKKNKIPSM QVGFIDAICLQLYEALTHVSEDCFPLLDGCRKNRQKWQALAEQQ | 8417 | 8469 |
| hPDE5 (Amino acid 535-860 of WT, C574Y) | - | EETRELQSLAAAVVPSAQTLKITDFSFSDFELSDLETALYTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8418 | 8470 |
| hPDE5 (Amino acid 535-860 of WT, L554R, Q586P, K710N, K730E) | - | EETRELQSLAAAVVPSAQTRKITDFSFSDFELSDLETALCTIRMFTDL NLVPNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYNTTLKIIKQAILA TDLALYIERRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8419 | 8471 |
| hPDE5 (Amino acid 535-860 of WT, H613R) | - | EETRELQSLAAAVVPSAQTLKITDFSFSDFELSDLETALCTIRMFTDL NLVQNFQMKHEVLCRWILSVKKNYRKNVAYRNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8420 | 8472 |
| hPDE5 (Amino acid 535-860 of WT, Q586L, S663Y, A762T, E808G) | - | EETRELQSLAAAVVPSAQTLKITDFSFSDFELSDLETALCTIRMFTDL NLVLNFQMKHEVLCRWILSVKKNYRKNVAYHNWRHAFNTAQCMF AALKAGKIQNKLTDLEILALLIAALSHDLDHRGVNNYYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTTCDLSAIT KPWPIQQRIAELVATEFFDQGDRERKELNIEPTDLMNRGKKNKIPSM QVGFIDAICLQLYEALTHVSEDCFPLLDGCRKNRQKWQALAEQQ | 8421 | 8473 |
| hPDE5 (Amino acid 535-860 of WT, V548M, D803N) | - | EETRELQSLAAAVMPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTNLMNREKKNKIPSM QVGFIDAICLQLYEALTHVSEDCFPLLDGCRKNRQKWQALAEQQ | 8422 | 8474 |
| hPDE5 (Amino acid 535-860 of WT, L554R, Q589E, A823T) | - | EETRELQSLAAAVVPSAQTRKITDFSFSDFELSDLETALCTIRMFTDL NLVQNFEMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDTICLQLYEALTHVSEDCFPLLDGCRKNRQKWQALAEQQ | 8423 | 8475 |
| hPDE5 (Amino acid 535-860 of WT, T571I, K604R, I706N, E795R) | - | EETRELQSLAAAVVPSAQTLKITDFSFSDFELSDLEIALCTIRMFTDLN LVQNFQMKHEVLCRWILSVKRNYRKNVAYHNWRHAFNTAQCMFA ALKAGKIQNKLTDLEILALLIAALSHDLDHRGVNNSYIQRSEHPLAQ LYCHSIMEHHHFDQCLMILNSPGNQILSGLSNEEYKTTLKIIKQAILA TDLALYIKRRGEFFELIRKNQFNLEDPHQKELFLAMLMTACDLSAIT KPWPIQQRIAELVATEFFDQGDRERRELNIEPTDLMNREKKNKIPSM QVGFIDAICLQLYEALTHVSEDCFPLLDGCRKNRQKWQALAEQQ | 8424 | 8476 |
| hPDE5 (Amino acid 535-860 of WT, G659A, T784I) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRAVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVAIEFFDQGDRERKELNIEPTDLMNREKKNKIPSM QVGFIDAICLQLYEALTHVSEDCFPLLDGCRKNRQKWQALAEQQ | 8425 | 8477 |
| hPDE5 (Amino acid 535-860 of WT, Q666H) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIHRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8426 | 8478 |
| hPDE5 (Amino acid 535-860 of WT, H617L, A722V) | - | EETRELQSLAAAVVPSAQTLKITDFSFSDFELSDLETALCTIRMFTDL NLVQNFQMKHEVLCRWILSVKKNYRKNVAYHNWRLAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILV | 8427 | 8479 |
| | | TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | | |
| hPDE5 (Amino acid 535-860 of WT, N609Y, I799L) | - | EETRELQSLAAAVVPSAQTLKITDFSFSDFELSDLETALCTIRMFTDL NLVQNFQMKHEVLCRWILSVKKNYRKYVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNLEPTDLMNREKKNKIPSM QVGFIDAICLQLYEALTHVSEDCFPLLDGCRKNRQKWQALAEQQ | 8428 | 8480 |
| hPDE5 (Amino acid 535-860 of WT, F561L, G659A, T784I) | - | EETRELQSLAAAVVPSAQTLKITDFSLSDFELSDLETALCTIRMFTDL NLVQNFQMKHEVLCRWILSVKKNYRKNVAYHNWRHAFNTAQCMF AALKAGKIQNKLTDLEILALLIAALSHDLDHRAVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVAIEFFDQGDRERKELNIEPTDLMNREKKNKIPSM QVGFIDAICLQLYEALTHVSEDCFPLLDGCRKNRQKWQALAEQQ | 8429 | 8481 |
| hPDE5 (Amino acid 535-860 of WT, K795N) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERNELNIEPTDLMNREKKNKIPSM QVGFIDAICLQLYEALTHVSEDCFPLLDGCRKNRQKWQALAEQQ | 8430 | 8482 |
| hPDE5 (Amino acid 535-860 of WT, K770Q, K848N) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT QPWPIQQRIAELVATEFFDQGDRERKELNIEPTDLMNREKKNKIPSM QVGFIDAICLQLYEALTHVSEDCFPLLDGCRNNRQKWQALAEQQ | 8431 | 8483 |
| hPDE5 (Amino acid 535-860 of WT, N605D) | - | EETRELQSLAAAVVPSAQTLKITDFSFSDFELSDLETALCTIRMFTDL NLVQNFQMKHEVLCRWILSVKKDYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8432 | 8484 |
| hPDE5 (Amino acid 535-860 of WT, I680S) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSSMEHHHFDQCLMILNSPGNQILSGLSIEEYKTTLKIIKQAIL ATDLALYIKRRGEFFELIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ | 8433 | 8485 |
| hPDE5 (Amino acid 535-860 of WT, V548M, F820I) | - | EETRELQSLAAAVMPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGIIDAICLQLYEALTHVSEDCFPLLDGCRKNRQKWQALAEQQ | 8434 | 8486 |
| hPDE5 (Amino acid 535-860 of WT, I799L) | - | EETRELQSLAAAVVPSAQTLKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNLEPTDLMNREKKNKIPSM QVGFIDAICLQLYEALTHVSEDCFPLLDGCRKNRQKWQALAEQQ | 8435 | 8487 |
| hPDE5 (Amino acid 535-860 of WT, R607Q) | - | EETRELQSLAAAVVPSAQTLKITDFSFSDFELSDLETALCTIRMFTDL NLVQNFQMKHEVLCRWILSVKKNYQKNVAYHNWRHAFNTAQCM FAALKAGKIQNKLTDLEILALLIAALSHDLDHRGVNNSYIQRSEHPL AQLYCHSIMEHHHFDQCLMILNSPGNQILSGLSIEEYKTTLKIIKQAIL ATDLALYIKRRGEFFELIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ | 8436 | 8488 |
| hPDE5 (Amino acid 535-860 of WT, S550F, L554R) | - | EETRELQSLAAAVVPFAQTRKITDFSFSDFELSDLETALCTIRMFTDL NLVQNFQMKHEVLCRWILSVKKNYRKNVAYHNWRHAFNTAQCMF AALKAGKIQNKLTDLEILALLIAALSHDLDHRGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRIAELVATEFFDQGDRERKELNIEPTDLMNREKKNKIPSM QVGFIDAICLQLYEALTHVSEDCFPLLDGCRKNRQKWQALAEQQ | 8437 | 8489 |
| hPDE5 (Amino acid 535-860 of WT, P549S, F564I, R658H, A779T, R847G) | - | EETRELQSLAAAVVSSAQTLKITDFSFSDIELSDLETALCTIRMFTDL NLVQNFQMKHEVLCRWILSVKKNYRKNVAYHNWRHAFNTAQCMF AALKAGKIQNKLTDLEILALLIAALSHDLDHHGVNNSYIQRSEHPLA QLYCHSIMEHHHFDQCLMILNSPGNQILSGLSIEEYKTTLKIIKQAILA TDLALYIKRRGEFFELIRKNQFNLEDPHQKELFLAMLMTACDLSAIT KPWPIQQRITELVATEFFDQGDRERKELNIEPTDLMNREKKNKIPSM QVGFIDAICLQLYEALTHVSEDCFPLLDGCGKNRQKWQALAEQQ | 8438 | 8490 |

In some instances, DDs derived from hPDE5 may include one, two, three, four, five, or more of the mutations described in the previous Tables (Table 1 or Table 2).

In some instances, DDs derived from hPDE5 may further comprise one, two, three, four, five or more mutations to the catalytic domain of hPDE5 and may be selected from E535D, E536G, Q541R, K555R, S560G, F559L, F561L, F564L, F564S, S766F, V585A, N587S, K591E, I599V, K604E, K608E, N609H, K630R, K633E, N636S, 1648V, N661S, S663P, L675P, Y676D, Y676N, C677R, H678R, D687A, T711A, T712S, D724N, L738H, N742S, F744L, L746S, F755L, A762S, D764V, D764N, D764G, K795E, L797F, I799T, L804P, T802P, S815C, M816A, M816T, I824T, C839S, F840S, and K852E (as listed in Table 3). The position of the mutated amino acid is with respect to the hPDE5 of SEQ ID NO: 1. In Table 3, the mutated amino acids are underlined and in bold. The position of the mutated amino acids listed in Table 3 is relative to the full length hPDE5 of SEQ ID NO:1

**Table 3: Additional hPDE5 DDs**

| **hPDES mutant description** | **Amino Acid Sequence** | **AA SEQ ID NO** |
|---|---|---|
| hPDE5 (Amino acid 535-860 of WT, E535D) | DETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 23 |
| hPDE5 (Amino acid 535-860 of WT, E536G) | EGTRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 24 |
| hPDE5 (Amino acid 535-860 of WT, Q541R) | EETRELRSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 25 |
| hPDE5 (Amino acid 535-860 of WT, K555R) | EETRELQSLAAAVVPSAQTLRITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 26 |
| hPDE5 (Amino acid 535-860 of WT, F559L) | EETRELQSLAAAVVPSAQTLKITDLSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 27 |
| hPDE5 (Amino acid 535-860 of WT, F561L) | EETRELQSLAAAVVPSAQTLKITDFSLSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 28 |
| hPDE5 (Amino acid 535-860 of WT, F564L) | EETRELQSLAAAVVPSAQTLKITDFSFSDLELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 29 |
| hPDE5 (Amino acid 535-860 of WT, F564S) | EETRELQSLAAAVVPSAQTLKITDFSFSDSELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 30 |
| hPDE5 (Amino acid 535-860 of WT, K591E) | EETRELQSLAAAVVPSAQTLKITDFSFSDFELSDLETALCTIRMFTDLNLVQNFQMEHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 31 |
| hPDE5 (Amino acid 535-860 of WT, N587S) | EETRELQSLAAAVVPSAQTLKITDFSFSDFELSDLETALCTIRMFTDLNLVQSFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 32 |
| hPDE5 (Amino acid 535-860 of WT, K604E) | EETRELQSLAAAVVPSAQTLKITDFSFSDFELSDLETALCTIRMFTDLNLVQNFQMKHEVLC RWILSVKENYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 33 |
| hPDE5 (Amino acid 535-860 of WT, K608E) | EETRELQSLAAAVVPSAQTLKITDFSFSDFELSDLETALCTIRMFTDLNLVQNFQMKHEVLC RWILSVKKNYRENVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 34 |
| hPDE5 (Amino acid 535-860 of WT, N609H) | EETRELQSLAAAVVPSAQTLKITDFSFSDFELSDLETALCTIRMFTDLNLVQNFQMKHEVLC RWILSVKKNYRKHVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 35 |
| hPDE5 (Amino acid 535-860 of WT, K630R) | EETRELQSLAAAVVPSAQTLKITDFSFSDFELSDLETALCTIRMFTDLNLVQNFQMKHEVLC RWILSVKKNYRKNVAYHNWRHAFNTAQCMFAALRAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 36 |
| hPDE5 (Amino acid 535-860 of WT, K633E) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGEIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 37 |
| hPDE5 (Amino acid 535-860 of WT, N636S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQSKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 38 |
| hPDE5 (Amino acid 535-860 of WT, N661S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVSNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 39 |
| hPDE5 (Amino acid 535-860 of WT, Y676D) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLDCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 40 |
| hPDE5 (Amino acid 535-860 of WT, Y676N) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLNCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 41 |
| hPDE5 (Amino acid 535-860 of WT, C677R) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYRHSIMEHHHFDQCLMILNSPGNQILSGLSIEEYKTTLKIIKQA ILATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 42 |
| hPDE5 (Amino acid 535-860 of WT, H678R) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCRSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 43 |
| hPDE5 (Amino acid 535-860 of WT, D687A) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFAQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 44 |
| hPDE5 (Amino acid 535-860 of WT, T712S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTSLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 45 |
| hPDE5 (Amino acid 535-860 of WT, D724N) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATNLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 46 |
| hPDE5 (Amino acid 535-860 of WT, D724G) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATGLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 47 |
| hPDE5 (Amino acid 535-860 of WT, L738H) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFEHIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 48 |
| hPDE5 (Amino acid 535-860 of WT, N742S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKSQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 49 |
| hPDE5 (Amino acid 535-860 of WT, A762S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTSCDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 50 |
| hPDE5 (Amino acid 535-860 of WT, D764N) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACNLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 51 |
| hPDE5 (Amino acid 535-860 of WT, D764G) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACGLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 52 |
| hPDE5 (Amino acid 535-860 of WT, D764V) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACVLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 53 |
| hPDE5 (Amino acid 535-860 of WT, S766F) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLFAITKPWPIQQRIAELV ATEFFDQGDRERKELNIEPTDLMNREKKNKIPSMQVGFIDAICLQLYEALTHVSEDCFPLLD GCRKNRQKWQALAEQQ | 54 |
| hPDE5 (Amino acid 535-860 of WT, K795E) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERE ELNIEPTDLMNREKKNKIPSMQVGFIDAICLQLYEALTHVSEDCFPLLD GCRKNRQKWQALAEQQ | 55 |
| hPDE5 (Amino acid 535-860 of WT, L797F) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKEFNIEPTDLMNREKKNKIPSMQVGFIDAICLQLYEALTHVSEDCFPLLD GCRKNRQKWQALAEQQ | 56 |
| hPDE5 (Amino acid 535-860 of WT, I799T) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNTEPTDLMNREKKNKIPSMQVGFIDAICLQLYEALTHVSEDCFPLLD GCRKNRQKWQALAEQQ | 57 |
| hPDE5 (Amino acid 535-860 of WT, T802P) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPPDLMNREKKNKIPSMQVGFIDAICLQLYEALTHVSEDCFPLLD GCRKNRQKWQALAEQQ | 58 |
| hPDE5 (Amino acid 535-860 of WT, S815C) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPCMQVGFIDAICLQLYEALTHVSEDCFPLLD GCRKNRQKWQALAEQQ | 59 |
| hPDE5 (Amino acid 535-860 of WT, M816A) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSAQVGFIDAICLQLYEALTHVSEDCFPLLD GCRKNRQKWQALAEQQ | 60 |
| hPDE5 (Amino acid 535-860 of WT, I824T) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDATCLQLYEALTHVSEDCFPLLD GCRKNRQKWQALAEQQ | 61 |
| hPDE5 (Amino acid 535-860 of WT, C839S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDSFPLLD GCRKNRQKWQALAEQQ | 62 |
| hPDE5 (Amino acid 535-860 of WT, K852E) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQEWQALAEQQ | 63 |
| hPDE5 (Amino acid 535-860 of WT, S560G) | EETRELQSLAAAVVPSAQTLKITDFGFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 64 |
| hPDE5 (Amino acid 535-860 of WT, V585A) | EETRELQSLAAAVVPSAQTLKITDFSFSDFELSDLETALCTIRMFTDLNLAQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 65 |
| hPDE5 (Amino acid 535-860 of WT, I599V) | EETRELQSLAAAVVPSAQTLKITDFSFSDFELSDLETALCTIRMFTDLNLVQNFQMKHEVLC RWVLSVKKNYRKNVAYHNWRHAFNTAQCMFAALKAGKIQNKLTDLEILALLIAALSHDL DHRGVNNSYIQRSEHPLAQLYCHSIMEHHHFDQCLMILNSPGNQILSGLSIEEYKTTLKIIKQ AILATDLALYIKRRGEFFELIRKNQFNLEDPHQKELFLAMLMTACDLSAITKPWPIQQRIAEL VATEFFDQGDRERKELNIEPTDLMNREKKNKIPSMQVGFIDAICLQLYEALTHVSEDCFPLL DGCRKNRQKWQALAEQQ | 66 |
| hPDE5 (Amino acid 535-860 of WT, I648V) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLVAALSHDL DHRGVNNSYIQRSEHPLAQLYCHSIMEHHHFDQCLMILNSPGNQILSGLSIEEYKTTLKIIKQ AILATDLALYIKRRGEFFELIRKNQFNLEDPHQKELFLAMLMTACDLSAITKPWPIQQRIAEL VATEFFDQGDRERKELNIEPTDLMNREKKNKIPSMQVGFIDAICLQLYEALTHVSEDCFPLL DGCRKNRQKWQALAEQQ | 67 |
| hPDE5 (Amino acid 535-860 of WT, S663P) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNPYIQRSEHPLAQLYCHSIMEHHHFDQCLMILNSPGNQILSGLSIEEYKTTLKIIKQA ILATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 68 |
| hPDE5 (Amino acid 535-860 of WT, L675P) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQPYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 69 |
| hPDE5 (Amino acid 535-860 of WT, T711A) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKATLKIIKQA ILATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 70 |
| hPDE5 (Amino acid 535-860 of WT, F744L) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQLNLEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 71 |
| hPDE5 (Amino acid 535-860 of WT, L746S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNSEDPHQKELFLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 72 |
| hPDE5 (Amino acid 535-860 of WT, F755L) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELLLAMLMTACDLSAITKPWPIQQRIAELV ATEFFDQGDRERKELNIEPTDLMNREKKNKIPSMQVGFIDAICLQLYEALTHVSEDCFPLLD GCRKNRQKWQALAEQQ | 73 |
| hPDE5 (Amino acid 535-860 of WT, L804P) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDPMNREKKNKIPSMQVGFIDAICLQLYEALTHVSEDCFPLLD GCRKNRQKWQALAEQQ | 74 |
| hPDE5 (Amino acid 535-860 of WT, M816T) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSTQVGFIDAICLQLYEALTHVSEDCFPLLD GCRKNRQKWQALAEQQ | 75 |
| hPDE5 (Amino acid 535-860 of WT, F840S) | EETRELQSLAAAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILALLIAALSHDLD HRGVNNSYIQRSEHPLAQLYCHSIMEHHHFDQCLMILNSPGNQILSGLSIEEYKTTLKIIKQAI LATDLALYIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSEDCSPLLD GCRKNRQKWQALAEQQ | 76 |

In some instances, DDs derived from hPDE5 may include the combination of at least one, two, three, four or five mutations listed in Table 1 or Table 2 with at least 0, 1, 2, 3, 4, 5 or more mutations listed in Table 3.

In some instances, the DDs may be derived from hPDE5 (SEQ ID NO: 3), by mutating one or more amino acids residues between positions 530 -550, 550-570, 570-590, 590-610, 620- 640, 640-660, 660-680, 680-700, 710- 730, 730- 750, 750-770, 770-790, 790- 810- 830, 830- 850, 850-860 of the catalytic domain of human PDE5 (SEQ ID NO: 1). In some instances, the mutation may be a conserved (with similar physicochemical properties as the amino acid at the mutation site), a semi conserved (e.g., negatively to positively charge amino acid) or a non-conserved (amino acid with different physicochemical properties than the amino acid at the mutation site). In some instances, the amino acid lysine may be mutated to glutamic acid or arginine; the amino acid phenylalanine may be mutated to leucine; the amino acid leucine may be mutated to phenylalanine; or the amino acid asparagine may be mutated to serine. Regions or portions or domains of wild type proteins may be utilized as SREs/DDs in whole or in part. They may be combined or rearranged to create new peptides, proteins, regions or domains of which any may be used as SREs/DDs or the starting point for the design of further SREs and/or DDs.

In some instances, DDs may be derived from hPDE5 by mutating amino acid residues conserved within all members of the PDE family. Exemplary conserved residues include but are not limited to, E682, H613, H617, H653, D654, and H657 residues of full length human PDE5 (SEQ ID NO: 1) and are taught in Sung et al. Nature (2003) 425, 98-102. In other instances, residues that are critical for binding to metals such as zinc and magnesium may be mutated to identify novel hPDE5 DDs. In some instances, hPDE5 derived DDs may be identified from a library of mutants of hPDE5 catalytic domain generated using a combination of error-prone PCR and nucleotide analog mutagenesis through random mutagenesis. Any of the mutations identified by site directed mutagenesis may be combined with the mutations identified by random mutagenesis. In some instances, DDs described herein may be derived by mutating the Y612 amino acid of hPDE5 (SEQ ID NO:1). In some instances, the mutations to the Y612 amino acid may be combined with any of the mutations described herein. Independent co-crystals of hPDE5 with vardenafil and with sildenafil, have demonstrated that one of the rings of the both ligand, interacts with Y612 of hPDE5, an amino acid located within the catalytic site of hPDE5. Interactions occur via a hydrogen bond with a water molecule and via hydrophobic bonds. Y612F mutation, which ablates the hydrogen bonding potential, increases the inhibition of hPDE5 activity by both ligands. The Y612A mutation, which leads to the ablation of both hydrogen bonding and hydrophobic bonding potential has been shown to weaken the inhibition of hPDE5 catalytic activity by vardenafil and sildenafil to a lesser extent. These studies suggest that hydrophobic bonding involving Y612 is stronger for vardenafil than for sildenafil (Corbin et al. 2006, International Journal of Impotence Research 18, 251-257).

The destabilization domains described herein may also include amino acid and nucleotide substitutions that do not affect stability, including conservative, non-conservative substitutions and or polymorphisms.

In some instances, hPDE5 DDs described herein may also be fragments of the above destabilizing domains, including fragments containing variant amino acid sequences. Preferred fragments are unstable in the absence of the stimulus and stabilized upon addition of the stimulus. Preferred fragments retain the ability to interact with the stimulus with similar efficiency as the DDs described herein.

In one instance, hPDE5 mutants are fused to AcGFP through a linker sequence at either the N-terminal or the C-terminal end of the fusion constructs. The AcGFP of Uniprot ID BAE93141 (SEQ ID NO: 363), may be used as the GFP template and is referred to as the wildtype or "WT" version of AcGFP. In some instances, the hPDE5 mutants described herein may also be operably linked to a luciferase (luc) gene, such as the firefly luciferase (Flue) or Renilla luciferase (Rluc). The position of the mutations in the Fluc protein sequence described herein are based on the comparison of the SEQ ID NO: 223 with the wildtype luciferase sequence of Photinus pyralis (Uniprot ID: P08659.1) or "Flue WT", comprising the amino acid sequence of SEQ ID NO: 364. The destabilizing and ligand dependent stabilization properties of the fusion proteins may be evaluated by methods such as western blotting, and FACS. hPDE5 mutants that are fused to the N terminus of GFP are provided in Table 4. Constructs may be cloned into any vector known in the art, such as, but not limited to pLVX.IRES. Puro vectors. OT-001232 was placed under the transcriptional control of the EF1a promoter, while the other constructs described in Table 4 were placed under the transcriptional control of CMV promoter. In Table 4, an asterisk indicates the translation of the stop codon.

**Table 4: hPDES-AcGFP constructs**

| **Construct ID/ Description** | **Amino Acid Sequence** | **Amino acid SEQ ID NO:** | **Nucleic Acid SEQ ID NO:** |
|---|---|---|---|
| Linker | GGSGGGSGG | 77 | 92 |
| Linker | GGSGGG | 78 | 93; 300 |
| Linker | SG | - | AGTGGT |
| AcGFP (Amino acid 2-239 of WT) | VSKGAELFTGIVPILIELNGDVNGHKFSVSGEGEGDATYGKLTLKFICTTGK LPVPWPTLVTTLSYGVQCFSRYPDHMKQHDFFKSAMPEGYIQERTIFFEDD GNYKSRAEVKFEGDTLVNRIELTGTDFKEDGNILGNKMEYNYNAHNVYIM TDKAKNGIKVNFKIRHNIEDGSVQLADHYQQNTPIGDGPVLLPDNHYLSTQ SALSKDPNEKRDHMIYFGFVTAAAITHGMDELYK | 79 | 372 |
| AcGFP (Amino acid 1-239 of WT) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYK | 365 | 94, 373 |
| Flue (Amino acid 2-549 of WT, N50D, N119G, S548I, K549A, L550V) | EDAKNIKKGPAPFYPLEDGTAGEQLHKAMKRYALVPGTIAFTDAHIEVDIT YAEYFEMSVRLAEAMKRYGLNTNHRIVVCSENSLQFFMPVLGALFIGVAV APANDIYNERELLNSMGISQPTVVFVSKKGLQKILNVQKKLPIIQKIIIMDSK TDYQGFQSMYTFVTSHLPPGFNEYDFVPESFDRDKTIALIMNSSGSTGLPKG VALPHRTACVRFSHARDPIFGNQIIPDTAILSVVPFHHGFGMFTTLGYLICGF RVVLMYRFEEELFLRSLQDYKIQSALLVPTLFSFFAKSTLIDKYDLSNLHEIA SGGAPLSKEVGEAVAKRFHLPGIRQGYGLTETTSAILITPEGDDKPGAVGK VVPFFEAKVVDLDTGKTLGVNQRGELCVRGPMIMSGYVNNPEATNALIDK DGWLHSGDIAYWDEDEHFFIVDRLKSLIKYKGYQVAPAELESILLQHPNIFD AGVAGLPDDDAGELPAAVVVLEHGKTMTEKEIVDYVASQVTTAKKLRGG VVFVDEVPKGLTGKLDARKIREILIKAKKGGKIAV | 366 | 374 |
| OT-001075 (Methionine; hPDE5 (Amino acid 535-860 of WT); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 80 | 95 |
| OT-001078 (Methionine; hPDE5 (Amino acid 535-860 of WT, W853F); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKFQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | 81 | 96 |
| OT-001080 (Methionine; hPDE5 (Amino acid 535-860 of WT, 1821A); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFADAICLQLYEALTHVSEDCFP LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | 82 | 97 |
| OT-001081 (Methionine; hPDE5 (Amino acid 535-860 of WT, Y829A); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLAEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 83 | 98 |
| OT-001074 (Methionine; hPDE5 (Amino acid 535-860 of WT, F787A); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFADQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 84 | 99 |
| OT-001076 (Methionine; hPDE5 (Amino acid 535-860 of WT, F736A); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFAELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 85 | 100 |
| OT-001082 (Methionine; hPDE5 (Amino acid 535-860 of WT, D656L); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLLHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 86 | 101 |
| OT-001083 (Methionine; hPDE5 (Amino acid 535-860 of WT, Y728L); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALLIKRRGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL DGCRKNRQKWQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | 87 | 102 |
| OT-001084 (Methionine; hPDE5 (Amino acid 535-860 of WT, R732L); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRLGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL DGCRKNRQKWQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | 88 | 103 |
| OT-001070 (Methionine; hPDE5 (Amino acid 535-860 of WT, M625I); linker (GGSGGGSGG (SEQ ID NO: 77)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCIFAALKAGKI QNKLTDLEILALLIAALSHDLDHRGVNNSYIQRSEHPLAQLYCHSIMEHHHF DQCLMILNSPGNQILSGLSIEEYKTTLKIIKQAILATDLALYIKRRGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL DGCRKNRQKWQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | 89 | 104 |
| OT-001224 (Methionine; hPDE5 (Amino acid 535-860 of WT); linker (GGSGGG (SEQ ID NO: 78)); AcGFP (Amino acid 2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGVSKGAELFTGIVPILIELNGDVNGHK FSVSGEGEGDATYGKLTLKFICTTGKLPVPWPTLVTTLSYGVQCFSRYPDH MKQHDFFKSAMPEGYIQERTIFFEDDGNYKSRAEVKFEGDTLVNRIELTGT DFKEDGNILGNKMEYNYNAHNVYIMTDKAKNGIKVNFKIRHNIEDGSVQL ADHYQQNTPIGDGPVLLPDNHYLSTQSALSKDPNEKRDHMIYFGFVTAAAI THGMDELYK* | 90 | 105 |
| OT-001232 (AcGFP (Amino acid 1-239 of WT); linker (SG); hPDE5 (Amino acid 535-860 of WT); stop) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYKSGEETRELQSLAAAV VPSAQTLKITDFSFSDFELSDLETALCTIRMFTDLNLVQNFQMKHEVLCRWI LSVKKNYRKNVAYHNWRHAFNTAQCMFAALKAGKIQNKLTDLEILALLIA ALSHDLDHRGVNNSYIQRSEHPLAQLYCHSIMEHHHFDQCLMILNSPGNQI LSGLSIEEYKTTLKIIKQAILATDLALYIKRRGEFFELIRKNQFNLEDPHQKEL FLAMLMTACDLSAITKPWPIQQRIAELVATEFFDQGDRERKELNIEPTDLM NREKKNKIPSMQVGFIDAICLQLYEALTHVSEDCFPLLDGCRKNRQKWQA LAEQQ* | 91 | 106 |
| OT-001227 (Methionine; hPDE5 (Amino acid 535-860 of WT); linker (SG); Flue (N50D, N119G, S548I, K549A, L550V); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQSGEDAKNIKKGPAPFYPLEDGTAGEQLHKAM KRYALVPGTIAFTDAHIEVDITYAEYFEMSVRLAEAMKRYGLNTNHRIVVC SENSLQFFMPVLGALFIGVAVAPANDIYNERELLNSMGISQPTVVFVSKKGL QKILNVQKKLPIIQKIIIMDSKTDYQGFQSMYTFVTSHLPPGFNEYDFVPESF DRDKTIALIMNSSGSTGLPKGVALPHRTACVRFSHARDPIFGNQIIPDTAILS VVPFHHGFGMFTTLGYLICGFRVVLMYRFEEELFLRSLQDYKIQSALLVPTL FSFFAKSTLIDKYDLSNLHEIASGGAPLSKEVGEAVAKRFHLPGIRQGYGLT ETTSAILITPEGDDKPGAVGKVVPFFEAKVVDLDTGKTLGVNQRGELCVRG PMIMSGYVNNPEATNALIDKDGWLHSGDIAYWDEDEHFFIVDRLKSLIKYK GYQVAPAELESILLQHPNIFDAGVAGLPDDDAGELPAAVVVLEHGKTMTE KEIVDYVASQVTTAKKLRGGVVFVDEVPKGLTGKLDARKIREILIKAKKGG KIAV* | 367 | 375 |
| OT-001228 (Methionine; hPDE5 (Amino acid 535-860 of WT, F736A); linker (SG); Flue (N50D, | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFAELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL | 368 | 376 |
| N119G, S548I, K549A, L550V); stop) | LDGCRKNRQKWQALAEQQSGEDAKNIKKGPAPFYPLEDGTAGEQLHKAM KRYALVPGTIAFTDAHIEVDITYAEYFEMSVRLAEAMKRYGLNTNHRIVVC SENSLQFFMPVLGALFIGVAVAPANDIYNERELLNSMGISQPTVVFVSKKGL QKILNVQKKLPIIQKIIIMDSKTDYQGFQSMYTFVTSHLPPGFNEYDFVPESF DRDKTIALIMNSSGSTGLPKGVALPHRTACVRFSHARDPIFGNQIIPDTAILS VVPFHHGFGMFTTLGYLICGFRVVLMYRFEEELFLRSLQDYKIQSALLVPTL FSFFAKSTLIDKYDLSNLHEIASGGAPLSKEVGEAVAKRFHLPGIRQGYGLT ETTSAILITPEGDDKPGAVGKVVPFFEAKVVDLDTGKTLGVNQRGELCVRG PMIMSGYVNNPEATNALIDKDGWLHSGDIAYWDEDEHFFIVDRLKSLIKYK GYQVAPAELESILLQHPNIFDAGVAGLPDDDAGELPAAVVVLEHGKTMTE KEIVDYVASQVTTAKKLRGGVVFVDEVPKGLTGKLDARKIREILIKAKKGG KIAV* | | |
| OT-001229 (Methionine; hPDE5 (Amino acid 535-860 of WT, R732L); Linker (SG); Flue (N50D, N119G, S548I, K549A, L550V); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRLGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL DGCRKNRQKWQALAEQQSGEDAKNIKKGPAPFYPLEDGTAGEQLHKAMK RYALVPGTIAFTDAHIEVDITYAEYFEMSVRLAEAMKRYGLNTNHRIVVCS ENSLQFFMPVLGALFIGVAVAPANDIYNERELLNSMGISQPTVVFVSKKGL QKILNVQKKLPIIQKIIIMDSKTDYQGFQSMYTFVTSHLPPGFNEYDFVPESF DRDKTIALIMNSSGSTGLPKGVALPHRTACVRFSHARDPIFGNQIIPDTAILS VVPFHHGFGMFTTLGYLICGFRVVLMYRFEEELFLRSLQDYKIQSALLVPTL FSFFAKSTLIDKYDLSNLHEIASGGAPLSKEVGEAVAKRFHLPGIRQGYGLT ETTSAILITPEGDDKPGAVGKVVPFFEAKVVDLDTGKTLGVNQRGELCVRG PMIMSGYVNNPEATNALIDKDGWLHSGDIAYWDEDEHFFIVDRLKSLIKYK GYQVAPAELESILLQHPNIFDAGVAGLPDDDAGELPAAVVVLEHGKTMTE KEIVDYVASQVTTAKKLRGGVVFVDEVPKGLTGKLDARKIREILIKAKKGG KIAV* | 369 | 377 |
| OT-001211 (AcGFP (Amino acid 1-239 of WT); linker (SG); hPDE5 (Amino acid 535-860 of WT, H653A); stop) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYKSGEETRELQSLAAAV VPSAQTLKITDFSFSDFELSDLETALCTIRMFTDLNLVQNFQMKHEVLCRWI LSVKKNYRKNVAYHNWRHAFNTAQCMFAALKAGKIQNKLTDLEILALLIA ALSADLDHRGVNNSYIQRSEHPLAQLYCHSIMEHHHFDQCLMILNSPGNQI LSGLSIEEYKTTLKIIKQAILATDLALYIKRRGEFFELIRKNQFNLEDPHQKEL FLAMLMTACDLSAITKPWPIQQRIAELVATEFFDQGDRERKELNIEPTDLM NREKKNKIPSMQVGFIDAICLQLYEALTHVSEDCFPLLDGCRKNRQKWQA LAEQQ* | 370 | 378 |
| OT-001208 (AcGFP (Amino acid 1-239 of WT); linker (SG); hPDE5 (Amino acid 535-860 of WT, D764A); stop) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYKSGEETRELQSLAAAV VPSAQTLKITDFSFSDFELSDLETALCTIRMFTDLNLVQNFQMKHEVLCRWI LSVKKNYRKNVAYHNWRHAFNTAQCMFAALKAGKIQNKLTDLEILALLIA ALSHDLDHRGVNNSYIQRSEHPLAQLYCHSIMEHHHFDQCLMILNSPGNQI LSGLSIEEYKTTLKIIKQAILATDLALYIKRRGEFFELIRKNQFNLEDPHQKEL FLAMLMTACALSAITKPWPIQQRIAELVATEFFDQGDRERKELNIEPTDLM NREKKNKIPSMQVGFIDAICLQLYEALTHVSEDCFPLLDGCRKNRQKWQA LAEQQ* | 371 | 379 |
| OT-001209 (AcGFP (1-239 of WT); linker (SG); hPDE5 (Amino acid 535-860 of WT, E682A); stop) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYKSGEETRELQSLAAAV VPSAQTLKITDFSFSDFELSDLETALCTIRMFTDLNLVQNFQMKHEVLCRWI | 8491 | 8521 |
| | LSVKKNYRKNVAYHNWRHAFNTAQCMFAALKAGKIQNKLTDLEILALLIA ALSHDLDHRGVNNSYIQRSEHPLAQLYCHSIMAHHHFDQCLMILNSPGNQI LSGLSIEEYKTTLKIIKQAILATDLALYIKRRGEFFELIRKNQFNLEDPHQKEL FLAMLMTACDLSAITKPWPIQQRIAELVATEFFDQGDRERKELNIEPTDLM NREKKNKIPSMQVGFIDAICLQLYEALTHVSEDCFPLLDGCRKNRQKWQA LAEQQ* | | |
| OT-001210 (AcGFP (1-239 of WT); Linker (SG); hPDE5 (Amino acid 535-860 of WT, D724A); stop) | MVSKGAELFTGIVPILIELNGDVNGHKFSVSGEGEGDATYGKLTLKFICTTG KLPVPWPTLVTTLSYGVQCFSRYPDHMKQHDFFKSAMPEGYIQERTIFFED DGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLST QSALSKDPNEKRDHMIYFGFVTAAAITHGMDELYKSGEETRELQSLAAAV VPSAQTLKITDFSFSDFELSDLETALCTIRMFTDLNLVQNFQMKHEVLCRWI LSVKKNYRKNVAYHNWRHAFNTAQCMFAALKAGKIQNKLTDLEILALLIA ALSHDLDHRGVNNSYIQRSEHPLAQLYCHSIMEHHHFDQCLMILNSPGNQI LSGLSIEEYKTTLKIIKQAILATALALYIKRRGEFFELIRKNQFNLEDPHQKEL FLAMLMTACDLSAITKPWPIQQRIAELVATEFFDQGDRERKELNIEPTDLM NREKKNKIPSMQVGFIDAICLQLYEALTHVSEDCFPLLDGCRKNRQKWQA LAEQQ* | 8492 | 8522 |
| OT-001358 (Met; hPDE5 (535- 860 of WT, N661S); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVSNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8493 | 8523 |
| OT-001359 (Met; hPDE5 (535-830 of WT, L569M, T833S); linker (GGSGGGSGG (SEQ ID NO: 77)); AcGFP (2-239 of WT); stop) | MEETRELQSLAAAVVPSAQTLKITDFSFSDFELSDMetALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALSHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8494 | 8524 |
| OT-001360 (Met; hPDE5 (535-860 of WT, I768N); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSANTKPWPIQQRIAELVATEFFDQ GDRERKELNIEPTDLMNREKKNKIPSMQVGFIDAICLQLYEALTHVSEDCFP LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | 8495 | 8525 |
| OT-001361 (Met; hPDE5 (535-860 of WT, R607W); linker (GGSGGGSGG (SEQ ID NO: 77)); | MEETRELQSLAAAVVPSAQTLKITDFSFSDFELSDLETALCTIRMFTDLNLV QNFQMKHEVLCRWILSVKKNYWKNVAYHNWRHAFNTAQCMFAALKAG KIQNKLTDLEILALLIAALSHDLDHRGVNNSYIQRSEHPLAQLYCHSIMEHH HFDQCLMILNSPGNQILSGLSIEEYKTTLKIIKQAILATDLALYIKRRGEFFEL IRKNQFNLEDPHQKELFLAMLMTACDLSAITKPWPIQQRIAELVATEFFDQ GDRERKELNIEPTDLMNREKKNKIPSMQVGFIDAICLQLYEALTHVSEDCFP | 8496 | 8526 |
| AcGFP (2-239 of WT); stop) | LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | | |
| OT-001362 (Met; hPDE5 (535-860 of WT, N620S); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFSTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8497 | 8527 |
| OT-001363 (Met; hPDE5 (535-860 of WT, L554R, Q589E, M691I); linker (GGSGGGSGG (SEQ ID NO: 77)); AcGFP (2-239 of WT); stop) | MEETRELQSLAAAVVPSAQTRKITDFSFSDFELSDLETALCTIRMFTDLNLV QNFEMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLIILNSPGNQILSGLSIEEYKTTLKIIKQAILATDLALYIKRRGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL DGCRKNRQKWQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | 8498 | 8528 |
| OT-001364 (Met; hPDE5 (535-860 of WT, W615R, T723R, A762T, E808G); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNRRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILARDLALYIKRRGEFFELI RKNQFNLEDPHQKELFLAMLMTTCDLSAITKPWPIQQRIAELVATEFFDQG DRERKELNIEPTDLMNRGKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8499 | 8529 |
| OT-001365 (Met; hPDE5 (535-860 of WT, C574Y); linker (GGSGGGSGG (SEQ ID NO: 77)); AcGFP (2-239 of WT); stop) | MEETRELQSLAAAVVPSAQTLKITDFSFSDFELSDLETALYTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8500 | 8530 |
| OT-001366 (Met; hPDE5 (535-860 of WT, L554R, Q586P, K710N, K730E); linker (GGSGGGSGG | MEETRELQSLAAAVVPSAQTRKITDFSFSDFELSDLETALCTIRMFTDLNLV PNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYNTTLKIIKQAILATDLALYIERRGEFFELIR KNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLL | 8501 | 8531 |
| (SEQ ID NO: 77)); AcGFP (2-239 of WT); stop) | DGCRKNRQKWQALAEQQGGSGGGSGGVSKGAELFTGIVPILIELNGDVNG HKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELT GTDFKEDGNILGNKMEYNYNAHNVYIMTDKAKNGIKVNFKIRHNIEDGSV QLADHYQQNTPIGDGPVLLPDNHYLSTQSALSKDPNEKRDHMIYFGFVTA AAITHGMDELYK* | | |
| OT-001367 (Met; hPDE5 (535-860 of WT, H613R); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYRNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8502 | 8532 |
| OT-001368 (Met; hPDE5 (535-860 of WT, Q586L, S663Y, A762T, E808G); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV LNFQMKHEVLCRWILSVKKNYRKNVAYHNWRHAFNTAQCMFAALKAGK IQNKLTDLEILALLIAALSHDLDHRGVNNYYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTTCDLSAITKPWPIQQRIAELVATEFFDQG DRERKELNIEPTDLMNRGKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8503 | 8533 |
| OT-001369 (Met; hPDE5 (535-860 of WT, V548M, D803N); linker (GGSGGGSGG (SEQ ID NO: 77)); AcGFP (2-239 of WT); stop) | MEETRELQSLAAAVMPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTNLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8504 | 8534 |
| OT-001370 (Met; hPDE5 (535-860 OF WT, L554R, Q589E, A823T); linker (GGSGGGSGG (SEQ ID NO: 77)); AcGFP (2-239 of WT); stop) | MEETRELQSLAAAVVPSAQTRKITDFSFSDFELSDLETALCTIRMFTDLNLV QNFEMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDTICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8505 | 8535 |
| OT-001371 (Met; hPDE5 (535-860 of WT, T571I, K604R, I706N, E795R); linker (GGSGGGSGG | MEETRELQSLAAAVVPSAQTLKITDFSFSDFELSDLEIALCTIRMFTDLNLV QNFQMKHEVLCRWILSVKRNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSNEEYKTTLKIIKQAILATDLALYIKRRGEFFELI RKNQFNLEDPHQKELFLAMLMTACDLSAITKPWPIQQRIAELVATEFFDQG DRERRELNIEPTDLMNREKKNKIPSMQVGFIDAICLQLYEALTHVSEDCFPL | 8506 | 8536 |
| (SEQ ID NO: 77)); AcGFP (2-239 of WT); stop) | LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | | |
| OT-001372 (Met; hPDE5 (535-860 of WT, G659A, T784I); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRAVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVAIEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8507 | 8537 |
| OT-001373 (Met; hPDE5 (535-860 of WT, Q666H); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIHRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8508 | 8538 |
| OT-001374 (Met; hPDE5 (535-860 of WT, H617L, A722V); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRLAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILVTDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8509 | 8539 |
| OT-001375 (Met; hPDE5 (535-860 of WT, N609Y, I799L); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKYVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNLEPTDLMNREKKNKIPSMQVGFIDAICLQLYEALTHVSEDCFP LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | 8510 | 8540 |
| OT-001376 (Met; hPDE5 (535-860 of WT, F561L, G659A, T784I); linker (GGSGGGSGG | MEETRELQSLAAAVVPSAQTLKITDFSLSDFELSDLETALCTIRMFTDLNLV QNFQMKHEVLCRWILSVKKNYRKNVAYHNWRHAFNTAQCMFAALKAGK IQNKLTDLEILALLIAALSHDLDHRAVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVAIEFFDQG DRERKELNIEPTDLMNREKKNKIPSMQVGFIDAICLQLYEALTHVSEDCFPL | 8511 | 8541 |
| (SEQ ID NO: 77)); AcGFP (2-239 of WT); stop) | LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | | |
| OT-001377 (Met; hPDE5 (535-860 of WT, K795N); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERNELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8512 | 8542 |
| OT-001378 (Met; hPDE5 (535-860 of WT, K770Q, K848N); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITQPWPIQQRIAELVATEFFDQG DRERKELNIEPTDLMNREKKNKIPSMQVGFIDAICLQLYEALTHVSEDCFPL LDGCRNNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8513 | 8543 |
| OT-001379 (Met; hPDE5 (535-860 of WT, N605D); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKDYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8514 | 8544 |
| OT-001380 (Met; hPDE5 (535-860 of WT, I680S); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSSMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8515 | 8545 |
| OT-001381 (Met; hPDE5 (535-860 of WT, V548M, F820I); linker (GGSGGGSGG (SEQ ID NO: 77)); | MEETRELQSLAAAVMPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGIIDAICLQLYEALTHVSEDCFPL | 8516 | 8546 |
| AcGFP (2-239 of WT); stop) | LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | | |
| OT-001382 (Met; hPDES(535-860 of WT, I799L); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNLEPTDLMNREKKNKIPSMQVGFIDAICLQLYEALTHVSEDCFP LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | 8517 | 8547 |
| OT-001383 (Met; hPDE5 (535-860 of WT, R607Q); linker (GGSGGGSGG (SEQ ID NO: 77)); AcGFP (2-239 of WT); stop) | MEETRELQSLAAAVVPSAQTLKITDFSFSDFELSDLETALCTIRMFTDLNLV QNFQMKHEVLCRWILSVKKNYQKNVAYHNWRHAFNTAQCMFAALKAG KIQNKLTDLEILALLIAALSHDLDHRGVNNSYIQRSEHPLAQLYCHSIMEHH HFDQCLMILNSPGNQILSGLSIEEYKTTLKIIKQAILATDLALYIKRRGEFFEL IRKNQFNLEDPHQKELFLAMLMTACDLSAITKPWPIQQRIAELVATEFFDQ GDRERKELNIEPTDLMNREKKNKIPSMQVGFIDAICLQLYEALTHVSEDCFP LLDGCRKNRQKWQALAEQQGGSGGGSGGVSKGAELFTGIVPILIELNGDV NGHKFSVSGEGEGDATYGKLTLKFICTTGKLPVPWPTLVTTLSYGVQCFSR YPDHMKQHDFFKSAMPEGYIQERTIFFEDDGNYKSRAEVKFEGDTLVNRIE LTGTDFKEDGNILGNKMEYNYNAHNVYIMTDKAKNGIKVNFKIRHNIEDG SVQLADHYQQNTPIGDGPVLLPDNHYLSTQSALSKDPNEKRDHMIYFGFVT AAAITHGMDELYK* | 8518 | 8548 |
| OT-001384 (Met; hPDE5 (535-860 of WT, S550F, L554R); linker (GGSGGGSGG (SEQ ID NO: 77)); AcGFP (2-239 of WT); stop) | MEETRELQSLAAAVVPFAQTRKITDFSFSDFELSDLETALCTIRMFTDLNLV QNFQMKHEVLCRWILSVKKNYRKNVAYHNWRHAFNTAQCMFAALKAGK IQNKLTDLEILALLIAALSHDLDHRGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPL LDGCRKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8519 | 8549 |
| OT-001385 (Met; hPDE5 (535-860 of WT, P549S, F564I, R658H, A779T, R847G); linker (GGSGGGSGG (SEQ ID NO: 77)); AcGFP (2-239 of WT); stop) | MEETRELQSLAAAVVSSAQTLKITDFSFSDIELSDLETALCTIRMFTDLNLV QNFQMKHEVLCRWILSVKKNYRKNVAYHNWRHAFNTAQCMFAALKAGK IQNKLTDLEILALLIAALSHDLDHHGVNNSYIQRSEHPLAQLYCHSIMEHHH FDQCLMILNSPGNQILSGLSIEEYKTTLKIIKQAILATDLALYIKRRGEFFELI RKNQFNLEDPHQKELFLAMLMTACDLSAITKPWPIQQRITELVATEFFDQG DRERKELNIEPTDLMNREKKNKIPSMQVGFIDAICLQLYEALTHVSEDCFPL LDGCGKNRQKWQALAEQQGGSGGGSGGVSKGAELFTGIVPILIELNGDVN GHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIEL TGTDFKEDGNILGNKMEYNYNAHNVYIMTDKAKNGIKVNFKIRHNIEDGS VQLADHYQQNTPIGDGPVLLPDNHYLSTQSALSKDPNEKRDHMIYFGFVT AAAITHGMDELYK* | 8520 | 8550 |

In addition to human PDE5, other phosphodiesterases may also be used to generate novel destabilizing domains. The human PDE superfamily includes 11 structurally related but functionally distinct gene families (hPDE1 to hPDE11). These differ in their cellular functions, primary structures, affinities for cAMP and cGMP, catalytic properties and response to specific activators, inhibitors, effectors and mechanisms of regulation. As modular proteins, hPDEs exhibit a common structural organization with divergent amino-terminal regulatory regions and conserved carboxy-terminal catalytic core. The hPDE family proteins contain an N terminal regulatory region and a C terminal catalytic region. The N-terminal regulatory regions contain structural determinants that target individual hPDEs to different subcellular locations, and allow individual hPDEs to specifically respond to different post translational modification. The structural elements include dimerization domains, auto-inhibitory modules, binding sites for ligands and allosteric effectors. In contrast, the X ray crystal structure isolated catalytic domains of nine hPDE families (hPDE1 to hPDE5 and hPDE7 to hPDE10) have demonstrated that the catalytic domains of hPDE share a similar topography, composed of -350 amino acids folded into 16 helices. Across the hPDE families, the active site forms a deep hydrophobic pocket that contains a hPDE specific, histidine-containing signature motif, HD(X₂) H(X₄) N (SEQ ID NO: 8377), and binding sites for two divalent metal ions that are essential for catalytic function. The affinity of hPDEs to specific cyclic nucleotides varies within the family-some hPDEs specifically hydrolyze cAMP (hPDE4, hPDE7 and hPDE8), whereas others hydrolyze cGMP (hPDE5, hPDE6 and hPDE9), and some hydrolyze both cAMP and cGMP (hPDE1, hPDE2, hPDE3, hPDE10 and HPDE11).

Similar to hPDE5, the catalytic domain or other functional domain of any hPDE family member may be mutagenized and screened for destabilizing mutations. Known inhibitors for each hPDE protein may also be tested for ligand-dependent stabilization.

In some instances, known mutations in phosphodiesterases that affect protein stability may be utilized to identify novel hPDE derived DDs. Mutations previously identified include, but are not limited to, hPDE5 (I778T), or hPDE6C (H602L), hPDE6C (E790K), hPDE6C (R104W), hPDE6C (Y323N), and hPDE6C (P391L) or hPDE4D (S752A), hPDE4D (S754A), hPDE4D (S752A, S754A), and hPDE4D (E757A, E758A, D759A) (Zhu et al. (2010) Mol Cell Biol. 4379-4390; Alexandre et al. (2015). Endocr. Relat. Cancer 22(4):519-30; Cheguru P. et al. (2015) Mol Cell Neurosci; 64: 1-8).

In some instances, novel DDs may be generated from hPDE1 which is also known as calcium and calmodulin dependent phosphodiesterase. It has three subtypes hPDE1A, hPDEIB and hPDE1C. The enzyme contains three functional domains; a conserved catalytic core, a regulatory N-terminus and a C-terminus. The catalytic domains of hPDE1 have three helical subdomains; an N-terminal cyclin fold region, a linker region and a C-terminal helical bundle. Vinpocetine is a known inhibitor of hPDE1.

In some instances, novel DDs may be generated from hPDE2, a dual substrate enzyme that hydrolyzes both cAMP and cGMP. The distinguishing feature of this hPDE is that it is allosterically stimulated by cGMP binding to one of its GAF domains. The crystal structure of hPDE2 GAF-B domain reveals that the GAF-B domain binds cGMP with high affinity and selectivity. Exemplary hPDE2 inhibitors include EHNA (erythro-9-(2-hydroxy-3-nonyl) adenine), Oxindole, PDP and BAY 60-7550. Inhibitors that selectively inhibit a hPDE2 isoform may also be used, for example, substituted pyrido (2,3-b) pyrazines having a hPDE2A selective inhibitory action (See, e.g., US Pat. NO.: 9,527,841.)

In some instances, novel DDs may be generated from hPDE3 which preferentially hydrolyses cAMP. Like other hPDE family members, it comprises three functional domains, a conserved catalytic core, a regulatory terminus and the C-terminus. The catalytic core of hPDE is characterized by a unique 44-amino acid insert. Amrinone, Cilostazol, Milrinone, Enoximone and Pimobendan are inhibitors for hPDE3 enzyme.

In some instances, novel DDs may be generated from hPDE4, the principal second messenger for immune response regulation and is responsible for the hydrolysis of cAMP. Four isoforms of hPDE4 exist with each isoform having a unique N terminal region that specifies cellular localization by mediating interactions with scaffolding proteins which may further comprise upstream conserved regions (UCRs). All isoforms share invariant catalytic domain. The catalytic pocket is lined with highly conserved and invariant residues, including an invariant glutamine (Q369) that forms crucial hydrogen bond with substrates. Analyses of crystal structure of hPDE-inhibitor complexes suggest that two conserved residues are essential for inhibitor binding. The formation of hydrogen bonds with invariant glutamine determines the orientation of the inhibitors and conserved hydrophobic residues (1336 and F340) form a hydrophobic clamp that anchors inhibitors in the pocket. A number of small molecules can inhibit hPDE 4 activity, some of which are FDA approved such as AN2728 (4-[(1-hydroxy-1,3-dihydro-2,1-benzoxaborol-5-yl) oxy]benzonitrile), Apremilast/ CC10004 (N-{2-[(1S)-1-(3-Ethoxy-4-methoxyphenyl)-2-(methyl sulfonyl) ethyl]-1,3-dioxo-2,3-dihydro-1H-isoindol-4-yl}acetamide), and Roflumilast. Other small molecules that inhibit hPDE4 also include E6005/RVT501, Cilomilast/SB-207,499, Ibudilast (AV-411 or MN-166), Mesembrenone, Piclamilast / RP 73401, Rolipram, Atizoram / CP-80633, Arofylline, CC-1088, Catramilast, CGH-2466, Cipamfylline, Drotaverine, FilaminasUWAY-PDA 641, HT-0712, DNS-001, ICI-63197, Indimilast, Irsogladine/MN 1695, Lirimilast/ BAY 19-8004, Oglemilast, Revamilast, Ro 20-1724, Ronomilast, GSK256066, DC-TA-46, AWD 12-281 and YM-976.

In some instances, novel DDs may be generated from hPDE6. This holoenzyme includes hPDE6 alpha, hPDE6 beta and/or two identical inhibitory subunits of hPDE6 gamma. hPDE6 alpha and beta forms comprise three domains: two N-terminal GAF domains and one C-terminal catalytic domain. The non-catalytic GAF domains are responsible for cGMP binding.

In some instances, novel DDs may be generated from hPDE7, a cAMP specific hPDE which consists of two genes, hPDE7A and hPDE7B. There are no known regulatory domains on the N terminus as established for most of the other hPDE families, although consensus PKA phosphorylation sites exist in this region. Several small molecules can inhibit PDE7, including BRL-50481(N, N,2-Trimethyl-5-nitrobenzenesulfonamide) and ASB16165 (1H-Thieno(2,3-C) pyrazole-5-carboxamide, 1-cyclohexyl-N-(6-(4-hydroxy-1-piperidinyl)-3-pyridinyl)-3-methyl).

In some instances, novel DDs may be generated from hPDE8. Two subfamilies of hPDE8 exist and both they have very high affinity for the substrate cAMP and are insensitive to the non-specific PDE inhibitor IBMX. Each protein contains a catalytic core, a PAS (Per, Arnt and Sim) and a REC (receiver) domain. The crystal structure of the catalytic core of hPDE8 identified Tyr748 residue as a unique residue that distinguishes hPDE8 inhibitor binding from other hPDE proteins bound to inhibitors. PF-04957325 (Pfizer) is a small molecule inhibitor of hPDE8.

In some instances, novel DDs may be generated from hPDE9, which has the highest affinity for cGMP. The primary structure of hPDE9A is simple as it does not appear to contain any GAF domains or other N-terminal regulatory sequences found in other hPDEs. The catalytic pocket is lined with highly conserved and invariant residues, including an invariant glutamine Gln 453 in hPDE9A2 that forms crucial hydrogen bond with substrates. The formation of hydrogen bonds with invariant glutamine determines the orientation of the inhibitors and conserved hydrophobic residues form a hydrophobic clamp that anchors inhibitors in the pocket and wedges their ring structures against F456 in hPDE9A. Tested hPDE9 inhibitors may include BAY73-6691 (1-(2-chlorophenyl)-6-[(2R)-3,3,3-trifluoro-2-methylpropyl]-1,5-dihydro-4H-pyrazolo[3,4-d]pyrimidine-4-one), PF-04447943 (6-[(3S,4S)-4-methyl-1-(pyrimidin-2-ylmethyl)pyrrolidin-3-yl]-1-(oxan-4-yl)-2H-pyrazolo[3,4-d]pyrimidin-4-one) and WYQ-C28L.

In some instances, novel DDs may be generated from hPDE10, which can hydrolyze both cAMP and cGMP. Like some other hPDE family proteins, hPDE10 comprises 2 GAF domains in the N terminal region, a Protein Kinase A phosphorylation site and a catalytic domain. The catalytic pocket is lined with highly conserved and invariant residues, including an invariant glutamine Q726 in hPDE10A2 that forms crucial hydrogen bond with substrates. Several PDE10 inhibitors are under clinical trials including OMS 824, Papaverine and PF-2545920 (2-(4-(1-methyl-4-pyridin-4-yl-1H-pyrazol-3-yl) phenoxymethyl) quinolone).

In some instances, novel DDs may be generated from hPDE11. Like hPDE10, the recently discovered hPDE11 can hydrolyze both cAMP and cGMP. It comprises of only one gene with four isoform variants. The longest variant, HPDE11A4, has two N-terminal GAF domains, whereas the other variants are truncations of this variant of varying lengths.

In some instances, any of the destabilizing mutations related to hPDE5 described herein may be structurally mapped onto other phosphodiesterases to generate destabilizing domains. In one instance, mutations that destabilize hPDE5, and which subsequently result in stabilization in the presence of sildenafil and/or vardenafil may be engineered onto hPDE6. In one instance, mutations that destabilize hPDE5, and which subsequently result in stabilization in the presence of Tadalafil may be engineered onto hPDE11.

The full length hPDEs and their catalytic domains that may be utilized to derive novel DDs are listed in Table 5.

**Table 5: Sequences of human PDE proteins and their catalytic domains**

| **PDE protein (Uniprot ID or domain description)** | **Amino Acid sequence** | **AA SEQ ID NO:** |
|---|---|---|
| hPDE1A (Uniprot ID: P54750) | MDDHVTIRKKHLQRPIFRLRCLVKQLERGDVNVVDLKKNIEYAASVLEAVYIDETRRLLDT EDELSDIQTDSVPSEVRDWLASTFTRKMGMTKKKPEEKPKFRSIVHAVQAGIFVERMYRKT YHMVGLAYPAAVIVTLKDVDKWSFDVFALNEASGEHSLKFMIYELFTRYDLINRFKIPVSC LITFAEALEVGYSKYKNPYHNLIHAADVTQTVHYIMLHTGIMHWLTELEILAMVFAAAIHD YEHTGTTNNFHIQTRSDVAILYNDRSVLENHHVSAAYRLMQEEEMNILINLSKDDWRDLR NLVIEMVLSTDMSGHFQQIKNIRNSLQQPEGIDRAKTMSLILHAADISHPAKSWKLHYRWT MALMEEFFLQGDKEAELGLPFSPLCDRKSTMVAQSQIGFIDFIVEPTFSLLTDSTEKIVIPLIE EASKAETSSYVASSSTTIVGLHIADALRRSNTKGSMSDGSYSPDYSLAAVDLKSFKNNLVDI IQQNKERWKELAAQEARTSSQKCEFIHQ | 107 |
| hPDE1A Catalytic domain (Amino acid 193 - 515 of PDE1A) | FKIPVSCLITFAEALEVGYSKYKNPYHNLIHAADVTQTVHYIMLHTGIMHWLTELEILAMV FAAAIHDYEHTGTTNNFHIQTRSDVAILYNDRSVLENHHVSAAYRLMQEEEMNILINLSKD DWRDLRNLVIEMVLSTDMSGHFQQIKNIRNSLQQPEGIDRAKTMSLILHAADISHPAKSWK LHYRWTMALMEEFFLQGDKEAELGLPFSPLCDRKSTMVAQSQIGFIDFIVEPTFSLLTDSTE KIVIPLIEEASKAETSSYVASSSTTIVGLHIADALRRSNTKGSMSDGSYSPDYSLAAVDLKSF KNNLVDIIQQNKERW | 108 |
| hPDE1B (Uniprot ID: Q01064) | MELSPRSPPEMLEESDCPSPLELKSAPSKKMWIKLRSLLRYMVKQLENGEINIEELKKNLEY TASLLEAVYIDETRQILDTEDELQELRSDAVPSEVRDWLASTFTQQARAKGRRAEEKPKFR SIVHAVQAGIFVERMFRRTYTSVGPTYSTAVLNCLKNLDLWCFDVFSLNQAADDHALRTI VFELLTRHNLISRFKIPTVFLMSFLDALETGYGKYKNPYHNQIHAADVTQTVHCFLLRTGM VHCLSEIELLAIIFAAAIHDYEHTGTTNSFHIQTKSECAIVYNDRSVLENHHISSVFRLMQDD EMNIFINLTKDEFVELRALVIEMVLATDMSCHFQQVKTMKTALQQLERIDKPKALSLLLHA ADISHPTKQWLVHSRWTKALMEEFFRQGDKEAELGLPFSPLCDRTSTLVAQSQIGFIDFIVE PTFSVLTDVAEKSVQPLADEDSKSKNQPSFQWRQPSLDVEVGDPNPDVVSFRSTWVKRIQE NKQKWKERAASGITNQMSIDELSPCEEEAPPSPAEDEHNQNGNLD | 109 |
| hPDE1B Catalytic domain (Amino acid 197 - 496 of PDE1B) | FKIPTVFLMSFLDALETGYGKYKNPYHNQIHAADVTQTVHCFLLRTGMVHCLSEIELLAIIF AAAIHDYEHTGTTNSFHIQTKSECAIVYNDRSVLENHHISSVFRLMQDDEMNIFINLTKDEF VELRALVIEMVLATDMSCHFQQVKTMKTALQQLERIDKPKALSLLLHAADISHPTKQWLV HSRWTKALMEEFFRQGDKEAELGLPFSPLCDRTSTLVAQSQIGFIDFIVEPTFSVLTDVAEK SVQPLADEDSKSKNQPSFQWRQPSLDVEVGDPNPDVVSFRSTWVKRIQENKQKW | 110 |
| bPDEIC (Uniprot ID: Q14123) | MESPTKEIEEFESNSLKYLQPEQIEKIWLRLRGLRKYKKTSQRLRSLVKQLERGEASVVDLK KNLEYAATVLESVYIDETRRLLDTEDELSDIQSDAVPSEVRDWLASTFTRQMGMMLRRSD EKPRFKSIVHAVQAGIFVERMYRRTSNMVGLSYPPAVIEALKDVDKWSFDVFSLNEASGD HALKFIFYELLTRYDLISRFKIPISALVSFVEALEVGYSKHKNPYHNLMHAADVTQTVHYLL YKTGVANWLTELEIFAIIFSAAIHDYEHTGTTNNFHIQTRSDPAILYNDRSVLENHHLSAAY RLLQDDEEMNILINLSKDDWREFRTLVIEMVMATDMSCHFQQIKAMKTALQQPEAIEKPK ALSLMLHTADISHPAKAWDLHHRWTMSLLEEFFRQGDREAELGLPFSPLCDRKSTMVAQS QVGFIDFIVEPTFTVLTDMTEKIVSPLIDETSQTGGTGQRRSSLNSISSSDAKRSGVKTSGSEG SAPINNSVISVDYKSFKATWTEVVHINRERWRAKVPKEEKAKKEAEEKARLAAEEQQKEM EAKSQAEEGASGKAEKKTSGETKNQVNGTRANKSDNPRGKNSKAEKSSGEQQQNGDFKD GKNKTDKKDHSNIGNDSKKTDGTKQRSHGSPAPSTSSTCRLTLPVIKPPLRHFKRPAYASSS YAPSVSKKTDEHPARYKMLDQRIKMKKIQNISHNWNRK | 111 |
| hPDElC Catalytic domain (Amino acid 202 - 521 of PDE1C) | FKIPISALVSFVEALEVGYSKHKNPYHNLMHAADVTQTVHYLLYKTGVANWLTELEIFAIIF SAAIHDYEHTGTTNNFHIQTRSDPAILYNDRSVLENHHLSAAYRLLQDDEEMNILINLSKDD WREFRTLVIEMVMATDMSCHFQQIKAMKTALQQPEAIEKPKALSLMLHTADISHPAKAW DLHHRWTMSLLEEFFRQGDREAELGLPFSPLCDRKSTMVAQSQVGFIDFIVEPTFTVLTDM TEKIVSPLIDETSQTGGTGQRRSSLNSISSSDAKRSGVKTSGSEGSAPINNSVISVDYKSFKAT WTEVVHINRERW | 112 |
| hPDE2A (Uniprot ID: O00408) | MGQACGHSILCRSQQYPAARPAEPRGQQVFLKPDEPPPPPQPCADSLQDALLSLGSVIDISG LQRAVKEALSAVLPRVETVYTYLLDGESQLVCEDPPHELPQEGKVREAIISQKRLGCNGLG FSDLPGKPLARLVAPLAPDTQVLVMPLADKEAGAVAAVILVHCGQLSDNEEWSLQAVEK HTLVALRRVQVLQQRGPREAPRAVQNPPEGTAEDQKGGAAYTDRDRKILQLCGELYDLD ASSLQLKVLQYLQQETRASRCCLLLVSEDNLQLSCKVIGDKVLGEEVSFPLTGCLGQVVED | 113 |
| | KKSIQLKDLTSEDVQQLQSMLGCELQAMLCVPVISRATDQVVALACAFNKLEGDLFTDED EHVIQHCFHYTSTVLTSTLAFQKEQKLKCECQALLQVAKNLFTHLDDVSVLLQEIITEARNL SNAEICSVFLLDQNELVAKVFDGGVVDDESYEIRIPADQGIAGHVATTGQILNIPDAYAHPL FYRGVDDSTGFRTRNILCFPIKNENQEVIGVAELVNKINGPWFSKFDEDLATAFSIYCGISIA HSLLYKKVNEAQYRSHLANEMMMYHMKVSDDEYTKLLHDGIQPVAAIDSNFASFTYTPR SLPEDDTSMAILSMLQDMNFINNYKIDCPTLARFCLMVKKGYRDPPYHNWMHAFSVSHFC YLLYKNLELTNYLEDIEIFALFISCMCHDLDHRGTNNSFQVASKSVLAALYSSEGSVMERH HFAQAIAILNTHGCNIFDHFSRKDYQRMLDLMRDIILATDLAHHLRIFKDLQKMAEVGYDR NNKQHHRLLLCLLMTSCDLSDQTKGWKTTRKIAELIYKEFFSQGDLEKAMGNRPMEMMD REKAYIPELQISFMEHIAMPIYKLLQDLFPKAAELYERVASNREHWTKVSHKFTIRGLPSNN SLDFLDEEYEVPDLDGTRAPINGCCSLDAE | |
| hPDE2A Catalytic domain (Amino acid 633 - 891 of PDE2A) | KIDCPTLARFCLMVKKGYRDPPYHNWMHAFSVSHFCYLLYKNLELTNYLEDIEIFALFISC MCHDLDHRGTNNSFQVASKSVLAALYSSEGSVMERHHFAQAIAILNTHGCNIFDHFSRKD YQRMLDLMRDIILATDLAHHLRIFKDLQKMAEVGYDRNNKQHHRLLLCLLMTSCDLSDQ TKGWKTTRKIAELIYKEFFSQGDLEKAMGNRPMEMMDREKAYIPELQISFMEHIAMPIYKL LQDLFPKAAELYERVASN | 114 |
| hPDE3A (Uniprot ID: Q14432) | | 115 |
| hPDE3A Catalytic domain (Amino acid 728 - 1086 of PDE3A) | FKIPIREFMNYFHALEIGYRDIPYHNRIHATDVLHAVWYLTTQPIPGLSTVINDHGSTSDSDS DSGFTHGHMGYVFSKTYNVTDDKYGCLSGNIPALELMALYVAAAMHDYDHPGRTNAFL VATSAPQAVLYNDRSVLENHHAAAAWNLFMSRPEYNFLINLDHVEFKHFRFLVIEAILATD LKKHFDFVAKFNGKVNDDVGIDWTNENDRLLVCQMCIKLADINGPAKCKELHLQWTDGI VNEFYEQGDEEASLGLPISPFMDRSAPQLANLQESFISHIVGPLCNSYDSAGLMPGKWVEDS DESGDTDDPEEEEEEEAPAPNEEETCENNESPKKKTFKRRKIYCQITQHLLQNHKMW | 116 |
| hPDE3B (Uniprot ID: Q13370) | MRRDERDAKAMRSLQPPDGAGSPPESLRNGYVKSCVSPLRQDPPRGFFFHLCRFCNVELRP PPASPQQPRRCSPFCRARLSLGALAAFVLALLLGAEPESWAAGAAWLRTLLSVCSHSLSPL FSIACAFFFLTCFLTRTKRGPGPGRSCGSWWLLALPACCYLGDFLVWQWWSWPWGDGDA GSAAPHTPPEAAAGRLLLVLSCVGLLLTLAHPLRLRHCVLVLLLASFVWWVSFTSLGSLPS ALRPLLSGLVGGAGCLLALGLDHFFQIREAPLHPRLSSAAEEKVPVIRPRRRSSCVSLGETA ASYYGSCKIFRRPSLPCISREQMILWDWDLKQWYKPHYQNSGGGNGVDLSVLNEARNMV SDLLTDPSLPPQVISSLRSISSLMGAFSGSCRPKINPLTPFPGFYPCSEIEDPAEKGDRKLNKG LNRNSLPTPQLRRSSGTSGLLPVEQSSRWDRNNGKRPHQEFGISSQGCYLNGPFNSNLLTIP KQRSSSVSLTHHVGLRRAGVLSSLSPVNSSNHGPVSTGSLTNRSPIEFPDTADFLNKPSVILQ RSLGNAPNTPDFYQQLRNSDSNLCNSCGHQMLKYVSTSESDGTDCCSGKSGEEENIFSKES FKLMETQQEEETEKKDSRKLFQEGDKWLTEEAQSEQQTNIEQEVSLDLILVEEYDSLIEKM SNWNFPIFELVEKMGEKSGRILSQVMYTLFQDTGLLEIFKIPTQQFMNYFRALENGYRDIPY HNRIHATDVLHAVWYLTTRPVPGLQQIHNGCGTGNETDSDGRINHGRIAYISSKSCSNPDE SYGCLSSNIPALELMALYVAAAMHDYDHPGRTNAFLVATNAPQAVLYNDRSVLENHHAA SAWNLYLSRPEYNFLLHLDHVEFKRFRFLVIEAILATDLKKHFDFLAEFNAKANDVNSNGI EWSNENDRLLVCQVCIKLADINGPAKVRDLHLKWTEGIVNEFYEQGDEEANLGLPISPFMD RSSPQLAKLQESFITHIVGPLCNSYDAAGLLPGQWLEAEEDNDTESGDDEDGEELDTEDEE MENNLNPKPPRRKSRRRIFCQLMHHLTENHKIWKEIVEEEEKCKADGNKLQVENSSLPQA DEIQVIEEADEEE | 117 |
| hPDE3B Catalytic domain (Amino acid 713 - 1072 of PDE3B) | FKIPTQQFMNYFRALENGYRDIPYHNRIHATDVLHAVWYLTTRPVPGLQQIHNGCGTGNE TDSDGRINHGRIAYISSKSCSNPDESYGCLSSNIPALELMALYVAAAMHDYDHPGRTNAFL VATNAPQAVLYNDRSVLENHHAASAWNLYLSRPEYNFLLHLDHVEFKRFRFLVIEAILAT DLKKHFDFLAEFNAKANDVNSNGIEWSNENDRLLVCQVCIKLADINGPAKVRDLHLKWTE GIVNEFYEQGDEEANLGLPISPFMDRSSPQLAKLQESFITHIVGPLCNSYDAAGLLPGQWLE AEEDNDTESGDDEDGEELDTEDEEMENNLNPKPPRRKSRRRIFCQLMHHLTENHKIW | 118 |
| hPDE4A (Uniprot ID: P27815) | MEPPTVPSERSLSLSLPGPREGQATLKPPPQHLWRQPRTPIRIQQRGYSDSAERAERERQPH RPIERADAMDTSDRPGLRTTRMSWPSSFHGTGTGSGGAGGGSSRRFEAENGPTPSPGRSPL DSQASPGLVLHAGAATSQRRESFLYRSDSDYDMSPKTMSRNSSVTSEAHAEDLIVTPFAQV LASLRSVRSNFSLLTNVPVPSNKRSPLGGPTPVCKATLSEETCQQLARETLEELDWCLEQLE TMQTYRSVSEMASHKFKRMLNRELTHLSEMSRSGNQVSEYISTTFLDKQNEVEIPSPTMKE REKQQAPRPRPSQPPPPPVPHLQPMSQITGLKKLMHSNSLNNSNIPRFGVKTDQEELLAQEL ENLNKWGLNIFCVSDYAGGRSLTCIMYMIFQERDLLKKFRIPVDTMVTYMLTLEDHYHAD VAYHNSLHAADVLQSTHVLLATPALDAVFTDLEILAALFAAAIHDVDHPGVSNQFLINTNS ELALMYNDESVLENHHLAVGFKLLQEDNCDIFQNLSKRQRQSLRKMVIDMVLATDMSKH MTLLADLKTMVETKKVTSSGVLLLDNYSDRIQVLRNMVHCADLSNPTKPLELYRQWTDRI MAEFFQQGDRERERGMEISPMCDKHTASVEKSQVGFIDYIVHPLWETWADLVHPDAQEIL DTLEDNRDWYYSAIRQSPSPPPEEESRGPGHPPLPDKFQFELTLEEEEEEEISMAQIPCTAQE ALTAQGLSGVEEALDATIAWEASPAQESLEVMAQEASLEAELEAVYLTQQAQSTGSAPVA PDEFSSREEFVVAVSHSSPSALALQSPLLPAWRTLSVSEHAPGLPGLPSTAAEVEAQREHQA AKRACSACAGTFGEDTSALPAPGGGGSGGDPT | 119 |
| hPDE4A Catalytic domain (Amino acid 330-723 of PDE4A) | QPMSQITGLKKLMHSNSLNNSNIPRFGVKTDQEELLAQELENLNKWGLNIFCVSDYAGGRS LTCIMYMIFQERDLLKKFRIPVDTMVTYMLTLEDHYHADVAYHNSLHAADVLQSTHVLLA TPALDAVFTDLEILAALFAAAIHDVDHPGVSNQFLINTNSELALMYNDESVLENHHLAVGF KLLQEDNCDIFQNLSKRQRQSLRKMVIDMVLATDMSKHMTLLADLKTMVETKKVTSSGV LLLDNYSDRIQVLRNMVHCADLSNPTKPLELYRQWTDRIMAEFFQQGDRERERGMEISPM CDKHTASVEKSQVGFIDYIVHPLWETWADLVHPDAQEILDTLEDNRDWYYSAIRQSPSPPP EEESRGPGHPPLPDKFQFELTLEEEEEEEISM | 120 |
| hPDE4B (Uniprot ID: Q07343) | MKKSRSVMTVMADDNVKDYFECSLSKSYSSSSNTLGIDLWRGRRCCSGNLQLPPLSQRQS ERARTPEGDGISRPTTLPLTTLPSIAITTVSQECFDVENGPSPGRSPLDPQASSSAGLVLHATF PGHSQRRESFLYRSDSDYDLSPKAMSRNSSLPSEQHGDDLIVTPFAQVLASLRSVRNNFTIL TNLHGTSNKRSPAASQPPVSRVNPQEESYQKLAMETLEELDWCLDQLETIQTYRSVSEMAS NKFKRMLNRELTHLSEMSRSGNQVSEYISNTFLDKQNDVEIPSPTQKDREKKKKQQLMTQI SGVKKLMHSSSLNNTSISRFGVNTENEDHLAKELEDLNKWGLNIFNVAGYSHNRPLTCIMY AIFQERDLLKTFRISSDTFITYMMTLEDHYHSDVAYHNSLHAADVAQSTHVLLSTPALDAV FTDLEILAAIFAAAIHDVDHPGVSNQFLINTNSELALMYNDESVLENHHLAVGFKLLQEEH CDIFMNLTKKQRQTLRKMVIDMVLATDMSKHMSLLADLKTMVETKKVTSSGVLLLDNYT DRIQVLRNMVHCADLSNPTKSLELYRQWTDRIMEEFFQQGDKERERGMEISPMCDKHTAS VEKSQVGFIDYIVHPLWETWADLVQPDAQDILDTLEDNRNWYQSMIPQSPSPPLDEQNRD CQGLMEKFQFELTLDEEDSEGPEKEGEGHSYFSSTKTLCVIDPENRDSLGETDIDIATEDKSP VDT | 121 |
| hPDE4B Catalytic domain (Amino acid 330-682 of PDE4B) | VNTENEDHLAKELEDLNKWGLNIFNVAGYSHNRPLTCIMYAIFQERDLLKTFRISSDTFITY MMTLEDHYHSDVAYHNSLHAADVAQSTHVLLSTPALDAVFTDLEILAAIFAAAIHDVDHP GVSNQFLINTNSELALMYNDESVLENHHLAVGFKLLQEEHCDIFMNLTKKQRQTLRKMVI DMVLATDMSKHMSLLADLKTMVETKKVTSSGVLLLDNYTDRIQVLRNMVHCADLSNPT KSLELYRQWTDRIMEEFFQQGDKERERGMEISPMCDKHTASVEKSQVGFIDYIVHPLWET WADLVQPDAQDILDTLEDNRNWYQSMIPQSPSPPLDEQNRDCQGLMEKFQFEL | 122 |
| hPDE4C (Uniprot ID: Q08493) | MENLGVGEGAEACSRLSRSRGRHSMTRAPKHLWRQPRRPIRIQQRFYSDPDKSAGCRERD LSPRPELRKSRLSWPVSSCRRFDLENGLSCGRRALDPQSSPGLGRIMQAPVPHSQRRESFLY RSDSDYELSPKAMSRNSSVASDLHGEDMIVTPFAQVLASLRTVRSNVAALARQQCLGAAK QGPVGNPSSSNQLPPAEDTGQKLALETLDELDWCLDQLETLQTRHSVGEMASNKFKRILN RELTHLSETSRSGNQVSEYISRTFLDQQTEVELPKVTAEEAPQPMSRISGLHGLCHSASLSSA TVPRFGVQTDQEEQLAKELEDTNKWGLDVFKVAELSGNRPLTAIIFSIFQERDLLKTFQIPA DTLATYLLMLEGHYHANVAYHNSLHAADVAQSTHVLLATPALEAVFTDLEILAALFASAI HDVDHPGVSNQFLINTNSELALMYNDASVLENHHLAVGFKLLQAENCDIFQNLSAKQRLS LRRMVIDMVLATDMSKHMNLLADLKTMVETKKVTSLGVLLLDNYSDRIQVLQNLVHCA DLSNPTKPLPLYRQWTDRIMAEFFQQGDRERESGLDISPMCDKHTASVEKSQVGFIDYIAH PLWETWADLVHPDAQDLLDTLEDNREWYQSKIPRSPSDLTNPERDGPDRFQFELTLEEAEE EDEEEEEEGEETALAKEALELPDTELLSPEAGPDPGDLPLDNQRT | 123 |
| hPDE4C Catalytic domain (Amino acid 312-677 of PDE4C) | VQTDQEEQLAKELEDTNKWGLDVFKVAELSGNRPLTAIIFSIFQERDLLKTFQIPADTLATY LLMLEGHYHANVAYHNSLHAADVAQSTHVLLATPALEAVFTDLEILAALFASAIHDVDHP GVSNQFLINTNSELALMYNDASVLENHHLAVGFKLLQAENCDIFQNLSAKQRLSLRRMVI DMVLATDMSKHMNLLADLKTMVETKKVTSLGVLLLDNYSDRIQVLQNLVHCADLSNPTK PLPLYRQWTDRIMAEFFQQGDRERESGLDISPMCDKHTASVEKSQVGFIDYIAHPLWETWA DLVHPDAQDLLDTLEDNREWYQSKIPRSPSDLTNPERDGPDRFQFELTLEEAEEEDEEEEEE GE | 124 |
| hPDE4D (Uniprot ID: Q08499) | | 125 |
| hPDE4D Catalytic domain (Amino acid 386-751 of PDE4D) | VKTEQEDVLAKELEDVNKWGLHVFRIAELSGNRPLTVIMHTIFQERDLLKTFKIPVDTLITY LMTLEDHYHADVAYHNNIHAADVVQSTHVLLSTPALEAVFTDLEILAAIFASAIHDVDHPG VSNQFLINTNSELALMYNDSSVLENHHLAVGFKLLQEENCDIFQNLTKKQRQSLRKMVIDI VLATDMSKHMNLLADLKTMVETKKVTSSGVLLLDNYSDRIQVLQNMVHCADLSNPTKPL QLYRQWTDRIMEEFFRQGDRERERGMEISPMCDKHNASVEKSQVGFIDYIVHPLWETWAD LVHPDAQDILDTLEDNREWYQSTIPQSPSPAPDDPEEGRQGQTEKFQFELTLEEDGESDTEK D | 126 |
| hPDE6A (Uniprot ID: P16499) | | 127 |
| hPDE6A Catalytic domain (Amino acid 483-819 of PDE6A) | EEEELAEILQAELPDADKYEINKFHFSDLPLTELELVKCGIQMYYELKVVDKFHIPQEALVR FMYSLSKGYRKITYHNWRHGFNVGQTMFSLLVTGKLKRYFTDLEALAMVTAAFCHDIDH RGTNNLYQMKSQNPLAKLHGSSILERHHLEFGKTLLRDESLNIFQNLNRRQHEHAIHMMDI AIIATDLALYFKKRTMFQKIVDQSKTYESEQEWTQYMMLEQTRKEIVMAMMMTACDLSA ITKPWEVQSQVALLVAAEFWEQGDLERTVLQQNPIPMMDRNKADELPKLQVGFIDFVCTF VYKEFSRFHEEITPMLDGITNNRKEWKALADEYDAK | 128 |
| hPDE6B (Uniprot ID: P35913) | | 129 |
| | | |
| hPDE6B Catalytic domain (Amino acid 476-817 of PDE6B) | PADCDEDELGEILKEELPGPTTFDIYEFHFSDLECTELDLVKCGIQMYYELGVVRKFQIPQE VLVRFLFSISKGYRRITYHNWRHGFNVAQTMFTLLMTGKLKSYYTDLEAFAMVTAGLCH DIDHRGTNNLYQMKSQNPLAKLHGSSILERHHLEFGKFLLSEETLNIYQNLNRRQHEHVIH LMDIAIIATDLALYFKKRAMFQKIVDESKNYQDKKSWVEYLSLETTRKEIVMAMMMTAC DLSAITKPWEVQSKVALLVAAEFWEQGDLERTVLDQQPIPMMDRNKAAELPKLQVGFIDF VCTFVYKEFSRFHEEILPMFDRLQNNRKEWKALADEYEAK | 130 |
| hPDE6C (Uniprot ID: P51160) | | 131 |
| hPDE6C Catalytic domain (Amino acid 483-822 of PDE6C) | DDCEEKQLVAILKEDLPDPRSAELYEFRFSDFPLTEHGLIKCGIRLFFEINVVEKFKVPVEVL TRWMYTVRKGYRAVTYHNWRHGFNVGQTMFTLLMTGRLKKYYTDLEAFAMLAAAFCH DIDHRGTNNLYQMKSTSPLARLHGSSILERHHLEYSKTLLQDESLNIFQNLNKRQFETVIHL FEVAIIATDLALYFKKRTMFQKIVDACEQMQTEEEAIKYVTVDPTKKEIIMAMMMTACDLS AITKPWEVQSQVALMVANEFWEQGDLERTVLQQQPIPMMDRNKRDELPKLQVGFIDFVC TFVYKEFSRFHKEITPMLSGLQNNRVEWKSLADEYDAK | 132 |
| hPDE7A (Uniprot ID: Q13946) | MEVCYQLPVLPLDRPVPQHVLSRRGAISFSSSSALFGCPNPRQLSQRRGAISYDSSDQTALYI RMLGDVRVRSRAGFESERRGSHPYIDFRIFHSQSEIEVSVSARNIRRLLSFQRYLRSSRFFRG TAVSNSLNILDDDYNGQAKCMLEKVGNWNFDIFLFDRLTNGNSLVSLTFHLFSLHGLIEYF HLDMMKLRRFLVMIQEDYHSQNPYHNAVHAADVTQAMHCYLKEPKLANSVTPWDILLSL IAAATHDLDHPGVNQPFLIKTNHYLATLYKNTSVLENHHWRSAVGLLRESGLFSHLPLESR QQMETQIGALILATDISRQNEYLSLFRSHLDRGDLCLEDTRHRHLVLQMALKCADICNPCR TWELSKQWSEKVTEEFFHQGDIEKKYHLGVSPLCDRHTESIANIQIGFMTYLVEPLFTEWA RFSNTRLSQTMLGHVGLNKASWKGLQREQSSSEDTDAAFELNSQLLPQENRLS | 133 |
| hPDE7A Catalytic domain (Amino acid 187-451 of PDE7A) | FHLDMMKLRRFLVMIQEDYHSQNPYHNAVHAADVTQAMHCYLKEPKLANSVTPWDILLS LIAAATHDLDHPGVNQPFLIKTNHYLATLYKNTSVLENHHWRSAVGLLRESGLFSHLPLES RQQMETQIGALILATDISRQNEYLSLFRSHLDRGDLCLEDTRHRHLVLQMALKCADICNPC RTWELSKQWSEKVTEEFFHQGDIEKKYHLGVSPLCDRHTESIANIQIGFMTYLVEPLFTEW ARFSNTRLSQTMLGHVGLNKASW | 134 |
| hPDE7B (Uniprot ID: Q9NP56) | MSCLMVERCGEILFENPDQNAKCVCMLGDIRLRGQTGVRAERRGSYPFIDFRLLNSTTYSG EIGTKKKVKRLLSFQRYFHASRLLRGIIPQAPLHLLDEDYLGQARHMLSKVGMWDFDIFLF DRLTNGNSLVTLLCHLFNTHGLIHHFKLDMVTLHRFLVMVQEDYHSQNPYHNAVHAADV TQAMHCYLKEPKLASFLTPLDIMLGLLAAAAHDVDHPGVNQPFLIKTNHHLANLYQNMS VLENHHWRSTIGMLRESRLLAHLPKEMTQDIEQQLGSLILATDINRQNEFLTRLKAHLHNK DLRLEDAQDRHFMLQIALKCADICNPCRIWEMSKQWSERVCEEFYRQGELEQKFELEISPL CNQQKDSIPSIQIGFMSYIVEPLFREWAHFTGNSTLSENMLGHLAHNKAQWKSLLPRQHRS RGSSGSGPDHDHAGQGTESEEQEGDSP | 135 |
| hPDE7B Catalytic domain (Amino acid 172-410 of PDE7B) | YHNAVHAADVTQAMHCYLKEPKLASFLTPLDIMLGLLAAAAHDVDHPGVNQPFLIKTNH HLANLYQNMSVLENHHWRSTIGMLRESRLLAHLPKEMTQDIEQQLGSLILATDINRQNEFL TRLKAHLHNKDLRLEDAQDRHFMLQIALKCADICNPCRIWEMSKQWSERVCEEFYRQGEL EQKFELEISPLCNQQKDSIPSIQIGFMSYIVEPLFREWAHFTGNSTLSENMLGHLAHNK | 136 |
| hPDE8A (Uniprot ID: O60658) | MGCAPSIHISERLVAEDAPSPAAPPLSSGGPRLPQGQKTAALPRTRGAGLLESELRDGSGKK VAVADVQFGPMRFHQDQLQVLLVFTKEDNQCNGFCRACEKAGFKCTVTKEAQAVLACFL DKHHDIIIIDHRNPRQLDAEALCRSIRSSKLSENTVIVGVVRRVDREELSVMPFISAGFTRRY VENPNIMACYNELLQLEFGEVRSQLKLRACNSVFTALENSEDAIEITSEDRFIQYANPAFETT | 137 |
| | MGYQSGELIGKELGEVPINEKKADLLDTINSCIRIGKEWQGIYYAKKKNGDNIQQNVKIIPV IGQGGKIRHYVSIIRVCNGNNKAEKISECVQSDTHTDNQTGKHKDRRKGSLDVKAVASRA TEVSSQRRHSSMARIHSMTIEAPITKVINIINAAQESSPMPVTEALDRVLEILRTTELYSPQFG AKDDDPHANDLVGGLMSDGLRRLSGNEYVLSTKNTQMVSSNIITPISLDDVPPRIARAMEN EEYWDFDIFELEAATHNRPLIYLGLKMFARFGICEFLHCSESTLRSWLQIIEANYHSSNPYH NSTHSADVLHATAYFLSKERIKETLDPIDEVAALIAATIHDVDHPGRTNSFLCNAGSELAIL YNDTAVLESHHAALAFQLTTGDDKCNIFKNMERNDYRTLRQGIIDMVLATEMTKHFEHV NKFVNSINKPLATLEENGETDKNQEVINTMLRTPENRTLIKRMLIKCADVSNPCRPLQYCIE WAARISEEYFSQTDEEKQQGLPVVMPVFDRNTCSIPKSQISFIDYFITDMFDAWDAFVDLPD LMQHLDNNFKYWKGLDEMKLRNLRPPPE | |
| hPDE8A Catalytic domain (Amino acid 531-813 of PDE8A) | LHCSESTLRSWLQIIEANYHSSNPYHNSTHSADVLHATAYFLSKERIKETLDPIDEVAALIAA TIHDVDHPGRTNSFLCNAGSELAILYNDTAVLESHHAALAFQLTTGDDKCNIFKNMERND YRTLRQGIIDMVLATEMTKHFEHVNKFVNSINKPLATLEENGETDKNQEVINTMLRTPENR TLIKRMLIKCADVSNPCRPLQYCIEWAARISEEYFSQTDEEKQQGLPVVMPVFDRNTCSIPK SQISFIDYFITDMFDAWDAFVDLPDLMQHLDNNFKYW | 138 |
| hPDE8B (Uniprot ID: 095263) | MGCAPSIHVSQSGVIYCRDSDESSSPRQTTSVSQGPAAPLPGLFVQTDAADAIPPSRASGPPS VARVRRARTELGSGSSAGSAAPAATTSRGRRRHCCSSAEAETQTCYTSVKQVSSAEVRIGP MRLTQDPIQVLLIFAKEDSQSDGFWWACDRAGYRCNIARTPESALECFLDKHHEIIVIDHRQ TQNFDAEAVCRSIRATNPSEHTVILAVVSRVSDDHEEASVLPLLHAGFNRRFMENSSIIACY NELIQIEHGEVRSQFKLRACNSVFTALDHCHEAIEITSDDHVIQYVNPAFERMMGYHKGEL LGKELADLPKSDKNRADLLDTINTCIKKGKEWQGVYYARRKSGDSIQQHVKITPVIGQGG KIRHFVSLKKLCCTTDNNKQIHKIHRDSGDNSQTEPHSFRYKNRRKESIDVKSISSRGSDAPS LQNRRYPSMARIHSMTIEAPITKVINIINAAQENSPVTVAEALDRVLEILRTTELYSPQLGTK DEDPHTSDLVGGLMTDGLRRLSGNEYVFTKNVHQSHSHLAMPITINDVPPCISQLLDNEES WDFNIFELEAITHKRPLVYLGLKVFSRFGVCEFLNCSETTLRAWFQVIEANYHSSNAYHNS THAADVLHATAFFLGKERVKGSLDQLDEVAALIAATVHDVDHPGRTNSFLCNAGSELAVL YNDTAVLESHHTALAFQLTVKDTKCNIFKNIDRNHYRTLRQAIIDMVLATEMTKHFEHVN KFVNSINKPMAAEIEGSDCECNPAGKNFPENQILIKRMMIKCADVANPCRPLDLCIEWAGRI SEEYFAQTDEEKRQGLPVVMPVFDRNTCSIPKSQISFIDYFITDMFDAWDAFAHLPALMQH LADNYKHWKTLDDLKCKSLRLPSDS | 139 |
| hPDE8B Catalytic domain (Amino acid 590-868 of PDE8B) | LNCSETTLRAWFQVIEANYHSSNAYHNSTHAADVLHATAFFLGKERVKGSLDQLDEVAAL IAATVHDVDHPGRTNSFLCNAGSELAVLYNDTAVLESHHTALAFQLTVKDTKCNIFKNIDR NHYRTLRQAIIDMVLATEMTKHFEHVNKFVNSINKPMAAEIEGSDCECNPAGKNFPENQILI KRMMIKCADVANPCRPLDLCIEWAGRISEEYFAQTDEEKRQGLPVVMPVFDRNTCSIPKSQ ISFIDYFITDMFDAWDAFAHLPALMQHLADNYKHW | 140 |
| hPDE9A (Uniprot ID: O76083) | MGSGSSSYRPKAIYLDIDGRIQKVIFSKYCNSSDIMDLFCIATGLPRNTTISLLTTDDAMVSID PTMPANSERTPYKVRPVAIKQLSAGVEDKRTTSRGQSAERPLRDRRVVGLEQPRREGAFES GQVEPRPREPQGCYQEGQRIPPEREELIQSVLAQVAEQFSRAFKINELKAEVANHLAVLEKR VELEGLKVVEIEKCKSDIKKMREELAARSSRTNCPCKYSFLDNHKKLTPRRDVPTYPKYLL SPETIEALRKPTFDVWLWEPNEMLSCLEHMYHDLGLVRDFSINPVTLRRWLFCVHDNYRN NPFHNFRHCFCVAQMMYSMVWLCSLQEKFSQTDILILMTAAICHDLDHPGYNNTYQINAR TELAVRYNDISPLENHHCAVAFQILAEPECNIFSNIPPDGFKQIRQGMITLILATDMARHAEI MDSFKEKMENFDYSNEEHMTLLKMILIKCCDISNEVRPMEVAEPWVDCLLEEYFMQSDRE KSEGLPVAPFMDRDKVTKATAQIGFIKFVLIPMFETVTKLFPMVEEIMLQPLWESRDRYEEL KRIDDAMKELQKKTDSLTSGATEKSRERSRDVKNSEGDCA | 141 |
| hPDE9A Catalytic domain (Amino acid 288-550 of PDE9A) | FSINPVTLRRWLFCVHDNYRNNPFHNFRHCFCVAQMMYSMVWLCSLQEKFSQTDILILMT AAICHDLDHPGYNNTYQINARTELAVRYNDISPLENHHCAVAFQILAEPECNIFSNIPPDGF KQIRQGMITLILATDMARHAEIMDSFKEKMENFDYSNEEHMTLLKMILIKCCDISNEVRPM EVAEPWVDCLLEEYFMQSDREKSEGLPVAPFMDRDKVTKATAQIGFIKFVLIPMFETVTKL FPMVEEIMLQPLWESRDRY | 142 |
| hPDE10A (Uniprot ID: Q9Y233) | MRIEERKSQHLTGLTDEKVKAYLSLHPQVLDEFVSESVSAETVEKWLKRKNNKSEDESAP KEVSRYQDTNMQGVVYELNSYIEQRLDTGGDNQLLLYELSSIIKIATKADGFALYFLGECN NSLCIFTPPGIKEGKPRLIPAGPITQGTTVSAYVAKSRKTLLVEDILGDERFPRGTGLESGTRI QSVLCLPIVTAIGDLIGILELYRHWGKEAFCLSHQEVATANLAWASVAIHQVQVCRGLAKQ TELNDFLLDVSKTYFDNIVAIDSLLEHIMIYAKNLVNADRCALFQVDHKNKELYSDLFDIGE EKEGKPVFKKTKEIRFSIEKGIAGQVARTGEVLNIPDAYADPRFNREVDLYTGYTTRNILCM PIVSRGSVIGVVQMVNKISGSAFSKTDENNFKMFAVFCALALHCANMYHRIRHSECIYRVT MEKLSYHSICTSEEWQGLMQFTLPVRLCKEIELFHFDIGPFENMWPGIFVYMVHRSCGTSC FELEKLCRFIMSVKKNYRRVPYHNWKHAVTVAHCMYAILQNNHTLFTDLERKGLLIACLC HDLDHRGFSNSYLQKFDHPLAALYSTSTMEQHHFSQTVSILQLEGHNIFSTLSSSEYEQVLEI IRKAIIATDLALYFGNRKQLEEMYQTGSLNLNNQSHRDRVIGLMMTACDLCSVTKLWPVT KLTANDIYAEFWAEGDEMKKLGIQPIPMMDRDKKDEVPQGQLGFYNAVAIPCYTTLTQIL PPTEPLLKACRDNLSQWEKVIRGEETATWISSPSVAQKAAASED | 143 |
| hPDE10A Catalytic domain (Amino acid 458-760 of PDE10A) | CKEIELFHFDIGPFENMWPGIFVYMVHRSCGTSCFELEKLCRFIMSVKKNYRRVPYHNWKH AVTVAHCMYAILQNNHTLFTDLERKGLLIACLCHDLDHRGFSNSYLQKFDHPLAALYSTST MEQHHFSQTVSILQLEGHNIFSTLSSSEYEQVLEIIRKAIIATDLALYFGNRKQLEEMYQTGS LNLNNQSHRDRVIGLMMTACDLCSVTKLWPVTKLTANDIYAEFWAEGDEMKKLGIQPIP MMDRDKKDEVPQGQLGFYNAVAIPCYTTLTQILPPTEPLLKACRDNLSQWEKVIRGEE | 144 |
| HPDE11A (Uniprot ID: Q9HCR9) | MAASRLDFGEVETFLDRHPELFEDYLMRKGKQEMVEKWLQRHSQGQGALGPRPSLAGTS SLAHSTCRGGSSVGGGTGPNGSAHSQPLPGGGDCGGVPLSPSWAGGSRGDGNLQRRASQK ELRKSFARSKAIHVNRTYDEQVTSRAQEPLSSVRRRALLRKASSLPPTTAHILSALLESRVN LPRYPPTAIDYKCHLKKHNERQFFLELVKDISNDLDLTSLSYKILIFVCLMVDADRCSLFLV EGAAAGKKTLVSKFFDVHAGTPLLPCSSTENSNEVQVPWGKGIIGYVGEHGETVNIPDAYQ DRRFNDEIDKLTGYKTKSLLCMPIRSSDGEIIGVAQAINKIPEGAPFTEDDEKVMQMYLPFC GIAISNAQLFAASRKEYERSRALLEVVNDLFEEQTDLEKIVKKIMHRAQTLLKCERCSVLLL EDIESPVVKFTKSFELMSPKCSADAENSFKESMEKSSYSDWLINNSIAELVASTGLPVNISDA YQDPRFDAEADQISGFHIRSVLCVPIWNSNHQIIGVAQVLNRLDGKPFDDADQRLFEAFVIF CGLGINNTIMYDQVKKSWAKQSVALDVLSYHATCSKAEVDKFKAANIPLVSELAIDDIHFD DFSLDVDAMITAALRMFMELGMVQKFKIDYETLCRWLLTVRKNYRMVLYHNWRHAFNV CQLMFAMLTTAGFQDILTEVEILAVIVGCLCHDLDHRGTNNAFQAKSGSALAQLYGTSAT LEHHHFNHAVMILQSEGHNIFANLSSKEYSDLMQLLKQSILATDLTLYFERRTEFFELVSKG EYDWNIKNHRDIFRSMLMTACDLGAVTKPWEISRQVAELVTSEFFEQGDRERLELKLTPSA IFDRNRKDELPRLQLEWIDSICMPLYQALVKVNVKLKPMLDSVATNRSKWEELHQKRLLA STASSSPASVMVAKEDRN | 145 |
| hPDE11A Catalytic Domain (Amino acid 640-905 of PDE11A) | FKIDYETLCRWLLTVRKNYRMVLYHNWRHAFNVCQLMFAMLTTAGFQDILTEVEILAVIV GCLCHDLDHRGTNNAFQAKSGSALAQLYGTSATLEHHHFNHAVMILQSEGHNIFANLSSK EYSDLMQLLKQSILATDLTLYFERRTEFFELVSKGEYDWNIKNHRDIFRSMLMTACDLGAV TKPWEISRQVAELVTSEFFEQGDRERLELKLTPSAIFDRNRKDELPRLQLEWIDSICMPLYQ ALVKVNVKLKPMLDSVATNRSKW | 146 |

In some instances, the hPDE5 derived destabilizing domains may be derived from variants, and/or isoforms of hPDE5. The three isoforms hPDE5 Isoform 1, hPDE5 Isoform 2, and hPDE5 Isoform 3 differ at their N terminal regions, and all 3 have unique first exons followed by a common sequence of 823 amino acids. Accordingly, hPDE5 derived DDs may be derived from hPDE5 Isoform 1 (SEQ ID NO: 1; encoded by the nucleotide sequence of SEQ ID NO: 2; hPDE5 Isoform 2 (SEQ ID NO: 147; encoded by the nucleotides 113- 2611 of the nucleotide sequence of SEQ ID NO: 148 or the nucleotide sequence of SEQ ID NO: 380) and/or hPDE5 Isoform 3 (SEQ ID NO: 149; encoded by the nucleotides 95-2563 of nucleotide sequence of SEQ ID NO: 150 or SEQ ID NO: 381). In some instances, the hPDES DDs may be derived from hPDE5 Isoform X1 (SEQ ID NO: 382).

In some instances, the DD mutations identified herein may be mapped back to the hPDE5 sequence to identify DD hotspots. DD hotspots as used herein refer to amino acids within the hPDE5 of SEQ ID NO: 1 whose mutation results in the "responsive" nature of the stimulus responsive element generated from hPDE5.The DD characteristics may be improved by saturation mutagenesis, which involves mutating the amino acids at the hotspot position to any of the known amino acids, including, but not limited to lysine, aspartic acid, glutamic acid, glutamine, asparagine, histidine, serine, threonine, tyrosine, cysteine, methionine, tryptophan, alanine, isoleucine, leucine, phenylalanine, valine, proline, and glycine. In some instances, a library of hotspot mutations may be generated by site directed mutagenesis and each of the mutants in the library is fused to a reporter protein e.g. AcGFP (SEQ ID NO: 79) via a linker such as SG. The properties of the DDs may be analyzed in the presence and absence of ligands such as Sildenafil, Vardenafil and Tadalafil. In some instances, the arginine at position 732 (also referred to as R732) of the hPDE5 of SEQ ID NO: 1, may be mutated to any of the known amino acids and are provided in Table 6. The mutations of the amino acid R732 may include but are not limited to R732L, R732A, R732G, R732V, R732I, R732P, R732F, R732W, R732Y, R732H, R732S, R732T, R732D, R732E, R732Q, R732N, R732M, R732C, and R732K. The hPDE5 mutants fused either at the N terminus or the C terminus to a linker and GFP are provided in Table 8. In Table 6, the mutations are in bold. Table 7 provides the additional components that may be combined with the mutants listed in Table 6 to generate the constructs described in Table 8. In Table 8, asterisk indicates the translation of the stop codon.

**Table 6: hPDE5 R732 mutants**

| **Construct ID/ Description** | **Sequence** | **Amino acid SEQ ID NO:** | **Nucleic Acid SEQ ID NO:** |
|---|---|---|---|
| hPDE5 (Amino acid 590-836 of WT, R732G) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRGGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 151 | 169 |
| hPDE5 (Amino acid 590-836 of WT, R732A) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRAGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 152 | 170 |
| hPDE5 (Amino acid 590-836 of WT, R732V) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRVGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 153 | 171 |
| hPDE5 (Amino acid 590-836 of WT, R732I) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRIGEFFELIRKNQFNLEDPHQKELFLAML MTACDLSAITKPWPIQQRIAELVATEFFDQGDRERKELNIEPTDLMNREKKNKIPSM QVGFIDAICLQLYEALTHVS | 154 | 172 |
| hPDE5 (Amino acid 590-836 of WT, R732P) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRPGEFFELIRKNQFNLEDPHQKELFLAML | 155 | 173 |
| | MTACDLSAITKPWPIQQRIAELVATEFFDQGDRERKELNIEPTDLMNREKKNKIPSM QVGFIDAICLQLYEALTHVS | | |
| hPDE5 (Amino acid 590-836 of WT, R732F) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRFGEFFELIRKNQFNLEDPHQKELFLAML MTACDLSAITKPWPIQQRIAELVATEFFDQGDRERKELNIEPTDLMNREKKNKIPSM QVGFIDAICLQLYEALTHVS | 156 | 174 |
| hPDE5 (Amino acid 590-836 of WT, R732W) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRWGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 157 | 175 |
| hPDE5 (Amino acid 590-836 of WT, R732Y) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRYGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 158 | 176 |
| hPDE5 (Amino acid 590-836 of WT, R732H) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRHGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 159 | 177 |
| hPDE5 (Amino acid 590-836 of WT, R732S) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRSGEFFELIRKNQFNLEDPHQKELFLAML MTACDLSAITKPWPIQQRIAELVATEFFDQGDRERKELNIEPTDLMNREKKNKIPSM QVGFIDAICLQLYEALTHVS | 160 | 178 |
| hPDE5 (Amino acid 590-836 of WT, R732T) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRTGEFFELIRKNQFNLEDPHQKELFLAML MTACDLSAITKPWPIQQRIAELVATEFFDQGDRERKELNIEPTDLMNREKKNKIPSM QVGFIDAICLQLYEALTHVS | 161 | 179 |
| hPDE5 (Amino acid 590-836 of WT, R732D) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRDGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 162 | 180 |
| hPDE5 (Amino acid 590-836 of WT, R732E) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKREGEFFELIRKNQFNLEDPHQKELFLAML MTACDLSAITKPWPIQQRIAELVATEFFDQGDRERKELNIEPTDLMNREKKNKIPSM QVGFIDAICLQLYEALTHVS | 163 | 181 |
| hPDE5 (Amino acid 590-836 of WT, R732Q) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRQGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 164 | 182 |
| hPDE5 (Amino acid 590-836 of WT, R732N) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRNGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 165 | 183 |
| hPDE5 (Amino acid 590-836 of WT, R732M) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRMGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 166 | 184 |
| hPDE5 (Amino acid 590-836 of WT, R732C) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRCGEFFELIRKNQFNLEDPHQKELFLAM | 167 | 185 |
| | LMTACDLSAITKPWPIQQRIAELVATEFFDQGDRERKELNIEPTDLMNREKKNKIPS MQVGFIDAICLQLYEALTHVS | | |
| hPDE5 (Amino acid 590-836 of WT, R732K) | MKHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGNQI LSGLSIEEYKTTLKIIKQAILATDLALYIKRKGEFFELIRKNQFNLEDPHQKELFLAM LMTACDLSAITKPWPIQQRIAELVATEFFDQGDRERKELNIEPTDLMNREKKNKIPS MQVGFIDAICLQLYEALTHVS | 168 | 186 |
| hPDE5 (Amino acid 535-860 of WT, R732G) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRGGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 383 | 401; 402 |
| hPDE5 (Amino acid 535-860 of WT, R732A) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRAGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 384 | 403; 404 |
| hPDE5 (Amino acid 535-860 of WT, R732L) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRLGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 12 | 405 |
| hPDE5 (Amino acid 535-860 of WT, R732V) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRVGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 385 | 406; 407 |
| hPDE5 (Amino acid 535-860 of WT, R732I) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRIGEFFELIRKNQFNLEDPHQKELFLAMLMT ACDLSAITKPWPIQQRIAELVATEFFDQGDRERKELNIEPTDLMNREKKNKIPSMQV GFIDAICLQLYEALTHVSEDCFPLLDGCRKNRQKWQALAEQQ | 386 | 408; 8551 |
| hPDE5 (Amino acid 535-860 of WT, R732P) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRPGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 387 | 409; 410 |
| hPDE5 (Amino acid 535-860 of WT, R732F) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRFGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 388 | 411 |
| hPDE5 (Amino acid 535-860 of WT, R732W) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRWGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 389 | 412 |
| hPDE5 (Amino acid 535-860 of WT, R732Y) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRYGEFFELIRKNQFNLEDPHQKELFLAMLM | 390 | 413; 414 |
| | TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | | |
| hPDE5 (Amino acid 535-860 of WT, R732H) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRHGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 391 | 415; 416 |
| hPDE5 (Amino acid 535-860 of WT, R732S) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRSGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 392 | 417; 418 |
| hPDE5 (Amino acid 535-860 of WT, R732T) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRTGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 393 | 419; 8552 |
| hPDE5 (Amino acid 535-860 of WT, R732D) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRDGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 394 | 420; 421 |
| hPDE5 (Amino acid 535-860 of WT, R732E) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKREGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 395 | 422; 423 |
| hPDE5 (Amino acid 535-860 of WT, R732Q) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRQGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 396 | 424; 425 |
| hPDE5 (Amino acid 535-860 of WT, R732N) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRNGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 397 | 426; 8553 |
| hPDE5 (Amino acid 535-860 of WT, R732M) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRMGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 398 | 427 |
| hPDE5 (Amino acid 535-860 of WT, R732C) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRCGEFFELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 399 | 428; 8554 |
| hPDE5 (Amino acid 535-860 of WT, R732K) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRKGEFFELIRKNQFNLEDPHQKELFLAMLM | 400 | 429 |
| | TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | | |

**Table 7: Additional hPDES R732 construct components**

| **Component** | **Amino Acid Sequence** | **Amino acid SEQ ID NO** | **Nucleic Acid SEQ ID NO** |
|---|---|---|---|
| AcGFP (Amino acid 2-239 of WT) | VSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIF FEDDGNYKSRAEVKFEGDTLVNRIELTGTDFKEDGNILGNKMEYNYNA HNVYIMTDKAKNGIKVNFKIRHNIEDGSVQLADHYQQNTPIGDGPVLLP DNHYLSTQSALSKDPNEKRDHMIYFGFVTAAAITHGMDELYK | 79 | 372 |
| AcGFP (Amino acid 1-239 of WT) | MVSKGAELFTGIVPILIELNGDVNGHKFSVSGEGEGDATYGKLTLKFICT TGKLPVPWPTLVTTLSYGVQCFSRYPDHMKQHDFFKSAMPEGYIQERTI FFEDDGNYKSRAEVKFEGDTLVNRIELTGTDFKEDGNILGNKMEYNYN AHNVYIMTDKAKNGIKVNFKIRHNIEDGSVQLADHYQQNTPIGDGPVLL PDNHYLSTQSALSKDPNEKRDHMIYFGFVTAAAITHGMDELYK | 365 | 373 |
| linker (SG) | SG | - | AGTGGT |
| linker (GS) | GS | - | GGATCC |

**Table 8: hPDE5 R732 constructs**

| **Construct ID/ Description** | **Sequence** | **Amino acid SEQ ID NO:** | **Nucleic Acid SEQ ID NO:** |
|---|---|---|---|
| OT-001233 (hPDE5 (Amino acid 590-836 of WT, R732G); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRGGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 187 | 205 |
| OT-001234 (hPDE5 (Amino acid 590-836 of WT, R732A); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRAGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 188 | 206 |
| OT-001235 (hPDE5 (Amino acid 590-836 of WT, R732V); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRVGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 189 | 207 |
| OT-001236 (hPDE5 (Amino acid 590-836 of WT, R732I); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRIGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSSGVSKGAELFTGIVPILIELNGDVNGHKFSVS GEGEGDATYGKLTLKFICTTGKLPVPWPTLVTTLSYGVQCFSRYPDHMKQHDFFK | 190 | 208 |
| | SAMPEGYIQERTIFFEDDGNYKSRAEVKFEGDTLVNRIELTGTDFKEDGNILGNKM EYNYNAHNVYIMTDKAKNGIKVNFKIRHNIEDGSVQLADHYQQNTPIGDGPVLLP DNHYLSTQSALSKDPNEKRDHMIYFGFVTAAAITHGMDELYK* | | |
| OT-001237 (hPDE5 (Amino acid 590-836 of WT, R732P); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRPGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSSGVSKGAELFTGIVPILIELNGDVNGHKFSVS GEGEGDATYGKLTLKFICTTGKLPVPWPTLVTTLSYGVQCFSRYPDHMKQHDFFK SAMPEGYIQERTIFFEDDGNYKSRAEVKFEGDTLVNRIELTGTDFKEDGNILGNKM EYNYNAHNVYIMTDKAKNGIKVNFKIRHNIEDGSVQLADHYQQNTPIGDGPVLLP DNHYLSTQSALSKDPNEKRDHMIYFGFVTAAAITHGMDELYK* | 191 | 209 |
| OT-001238 (hPDE5 (Amino acid 590-836 of WT, R732F); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRFGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSSGVSKGAELFTGIVPILIELNGDVNGHKFSVS GEGEGDATYGKLTLKFICTTGKLPVPWPTLVTTLSYGVQCFSRYPDHMKQHDFFK SAMPEGYIQERTIFFEDDGNYKSRAEVKFEGDTLVNRIELTGTDFKEDGNILGNKM EYNYNAHNVYIMTDKAKNGIKVNFKIRHNIEDGSVQLADHYQQNTPIGDGPVLLP DNHYLSTQSALSKDPNEKRDHMIYFGFVTAAAITHGMDELYK* | 192 | 210 |
| OT-001239 (hPDE5 (Amino acid 590-836 of WT, R732W); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRWGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 193 | 211 |
| OT-001240 (hPDE5 (Amino acid 590-836 of WT, R732Y); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRYGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 194 | 212 |
| OT-001241 (hPDE5 (Amino acid 590-836 of WT, R732H); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRHGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 195 | 213 |
| OT-001242 (hPDE5 (Amino acid 590-836 of WT, R732S); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRSGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSSGVSKGAELFTGIVPILIELNGDVNGHKFSVS GEGEGDATYGKLTLKFICTTGKLPVPWPTLVTTLSYGVQCFSRYPDHMKQHDFFK SAMPEGYIQERTIFFEDDGNYKSRAEVKFEGDTLVNRIELTGTDFKEDGNILGNKM EYNYNAHNVYIMTDKAKNGIKVNFKIRHNIEDGSVQLADHYQQNTPIGDGPVLLP DNHYLSTQSALSKDPNEKRDHMIYFGFVTAAAITHGMDELYK* | 196 | 214 |
| OT-001243 (hPDE5 (Amino acid 590-836 of WT, | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRTGEFFELIRKNQFNLEDPHQKELF | 197 | 215 |
| R732T); linker (SG); AcGFP (Amino acid 2-239 of WT); stop) | LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | | |
| OT-001244 (hPDE5 (Amino acid 590-836 of WT, R732D); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRDGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 198 | 216 |
| OT-001245 (hPDE5 (Amino acid 590-836 of WT, R732E); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKREGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 199 | 217 |
| OT-001246 (hPDE5 (Amino acid 590-836 of WT, R732Q); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRQGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 200 | 218 |
| OT-001247 (hPDE5 (Amino acid 590-836 of WT, R732N); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRNGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 201 | 219 |
| OT-001248 (hPDE5 (Amino acid 590-836 of WT, R732M); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRMGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 202 | 220 |
| OT-001249 (hPDE5 (Amino acid 590-836 of WT, R732C); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRCGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 203 | 221 |
| OT-001250 (hPDE5 (Amino acid 590-836 of WT, R732K); linker (SG); AcGFP (Amino acid 2-239 of WT); stop) | MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRKGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSSGVSKGAELFTGIVPILIELNGDVNGHKFSV SGEGEGDATYGKLTLKFICTTGKLPVPWPTLVTTLSYGVQCFSRYPDHMKQHDFF KSAMPEGYIQERTIFFEDDGNYKSRAEVKFEGDTLVNRIELTGTDFKEDGNILGNK MEYNYNAHNVYIMTDKAKNGIKVNFKIRHNIEDGSVQLADHYQQNTPIGDGPVL LPDNHYLSTQSALSKDPNEKRDHMIYFGFVTAAAITHGMDELYK* | 204 | 222 |
| OT-001186 (Methionine; (hPDE5 (Amino acid 535-860 of WT, R732G); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRGGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 430 | 467 |
| OT-001187 (Methionine; (hPDE5 (Amino acid 535-860 of WT, R732A); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRAGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 431 | 468 |
| OT-001188 (Methionine; (hPDE5 (Amino acid 535-860 of WT, R732η; linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRVGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 432 | 469 |
| OT-001189 (Methionine; (hPDE5 (Amino acid 535-860 of WT, R732I); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRIGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQSGVSKG AELFTGIVPILIELNGDVNGHKFSVSGEGEGDATYGKLTLKFICTTGKLPVPWPTL VTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRH NIEDGSVQLADHYQQNTPIGDGPVLLPDNHYLSTQSALSKDPNEKRDHMIYFGFV TAAAITHGMDELYK* | 433 | 470 |
| OT-001190 (Methionine; (hPDE5 (Amino acid 535-860 of WT, R732P); linker (SG); AcGFP | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRPGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQSGVSKG AELFTGIVPILIELNGDVNGHKFSVSGEGEGDATYGKLTLKFICTTGKLPVPWPTL | 434 | 471 |
| (Amino acid 2-239 of WT); stop) | VTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRH NIEDGSVQLADHYQQNTPIGDGPVLLPDNHYLSTQSALSKDPNEKRDHMIYFGFV TAAAITHGMDELYK* | | |
| OT-001191 (Methionine; (hPDE5 (Amino acid 535-860 of WT, R732F); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRFGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQSGVSKG AELFTGIVPILIELNGDVNGHKFSVSGEGEGDATYGKLTLKFICTTGKLPVPWPTL VTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRH NIEDGSVQLADHYQQNTPIGDGPVLLPDNHYLSTQSALSKDPNEKRDHMIYFGFV TAAAITHGMDELYK* | 435 | 472 |
| OT-001192 (Methionine; (hPDE5 (Amino acid 535-860 of WT, R732W); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRWGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 436 | 473 |
| OT-001193 (Methionine; (hPDE5 (Amino acid 535-860 of WT, R732Y); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRYGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 437 | 474 |
| OT-001195 (Methionine; (hPDE5 (Amino acid 535-860 of WT, R732H); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRHGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 438 | 475 |
| OT-001 196 (Methionine; (hPDE5 (Amino acid 535-860 of WT, R732S); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRSGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQSGVSKG AELFTGIVPILIELNGDVNGHKFSVSGEGEGDATYGKLTLKFICTTGKLPVPWPTL VTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRH NIEDGSVQLADHYQQNTPIGDGPVLLPDNHYLSTQSALSKDPNEKRDHMIYFGFV TAAAITHGMDELYK* | 439 | 476 |
| OT-001 197 (Methionine; (hPDE5 (Amino acid 535-860 of WT, R732T); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRTGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 440 | 477 |
| OT-001198 (Methionine; (hPDE5 (Amino acid 535-860 of WT, R732D); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRDGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 441 | 478 |
| OT-001199 (Methionine; (hPDE5 (Amino acid 535-860 of WT, R732E); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKREGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 442 | 479 |
| OT-001200 (Methionine; (hPDE5 (Amino acid 535-860 of WT, R732Q); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRQGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 443 | 480 |
| OT-001201 (Methionine; (hPDES (Amino acid 535-860 of WT, R732N); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRNGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 444 | 481 |
| OT-001202 (Methionine; (hPDE5 (Amino acid 535-860 of WT, R732M); | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRMGEFFELIRKNQFNLEDPHQKELF LAMLMTACDLSAITKPWPIQQRIAELVATEFFDQGDRERKELNIEPTDLMNREKK | 445 | 482 |
| linker (SG); AcGFP (Amino acid 2-239 of WT); stop) | NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | | |
| OT-001203 (Methionine; (hPDE5 (Amino acid 535-860 of WT, R732C); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRCGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 446 | 483 |
| OT-001204 (Methionine; (hPDE5 (Amino acid 535-860 of WT, R732K); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRKGEFFELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 447 | 484 |
| OT-001279 (AcGFP (Amino acid 1-239 of WT); linker (GS); (hPDE5 (Amino acid 535-860 of WT, R732G); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRGGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 448 | 485 |
| OT-001280 (AcGFP (Amino acid 1-239 of WT); linker (GS); (hPDE5 (Amino acid 535-860 of WT, R732A); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRAGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 449 | 486 |
| OT-001281 (AcGFP (Amino acid 1-239 of WT); linker (GS); (hPDE5 (Amino acid 535-860 of WT, R732L); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD | 450 | 487 |
| | RERKELNIEPTDLMNREKKNKIPSMQVGFIDAICLQLYEALTHVSEDCFPLLDGCR KNRQKWQALAEQQSR* | | |
| OT-001282 (AcGFP (Amino acid 1-239 of WT); linker (GS); (hPDE5 (Amino acid 535-860 of WT, R732M); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRMGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 451 | 488 |
| OT-001283 (AcGFP (Amino acid 1-239 of WT); linker (GS); (hPDE5 (Amino acid 535-860 of WT, R732F); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRFGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 452 | 489 |
| OT-001284 (AcGFP (Amino acid 1-239 of WT); linker (GS); (hPDE5 (Amino acid 535-860 of WT, R732W); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRWGE FFELIRKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPLLDGC RKNRQKWQALAEQQSR* | 453 | 490 |
| OT-001285 (AcGFP (Amino acid 1-239 of WT); linker (GS); (hPDE5 (Amino acid 535-860 of WT, R732K); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRKGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 454 | 491 |
| OT-001286 (AcGFP (Amino acid 1-239 of WT); linker (GS); (hPDE5 (Amino acid 535-860 of WT, R732Q); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRQGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 455 | 492 |
| OT-001287 (AcGFP (Amino acid 1-239 of WT); | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN | 456 | 493 |
| linker (GS); (hPDE5 (Amino acid 535-860 of WT, R732E); Xba I site (TCTAGA); stop) | FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKREGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | | |
| OT-001288 (AcGFP (Amino acid 1-239 of WT); linker (GS); (hPDE5 (Amino acid 535-860 of WT, R732S); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRSGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 457 | 494 |
| OT-001289 (AcGFP (Amino acid 1-239 of WT); linker (GS); (hPDE5 (Amino acid 535-860 of WT, R732P); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRPGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 458 | 495 |
| OT-001290 (AcGFP (Amino acid 1-239 of WT); linker (GS); (hPDE5 (Amino acid 535-860 of WT, R732V); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRVGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 459 | 496 |
| OT-001291 (AcGFP (Amino acid 1-239 of WT); linker (GS); (hPDE5 (Amino acid 535-860 of WT, R732I); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRIGEFF ELIRKNQFNLEDPHQKELFLAMLMTACDLSAITKPWPIQQRIAELVATEFFDQGDR ERKELNIEPTDLMNREKKNKIPSMQVGFIDAICLQLYEALTHVSEDCFPLLDGCRK NRQKWQALAEQQSR* | 460 | 497 |
| OT-001292 (AcGFP (Amino acid 1-239 of WT); linker (GS); (hPDE5 (Amino acid 535-860 of WT, R732C); Xba I | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRCGEF | 461 | 498 |
| site (TCTAGA); stop) | FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | | |
| OT-001293 (AcGFP (Amino acid 1-239 of WT); linker (GS); (hPDE5 (Amino acid 535-860 of WT, R732Y); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRYGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 462 | 499 |
| OT-001294 (AcGFP (Amino acid 1-239 of WT); linker (GS); (hPDE5 (Amino acid 535-860 of WT, R732H); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRHGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 463 | 500 |
| OT-001295 (AcGFP (Amino acid 1-239 of WT); linker (GS); (hPDE5 (Amino acid 535-860 of WT, R732N); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRNGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 464 | 501 |
| OT-001296 (AcGFP (Amino acid 1-239 of WT); linker (GS); (hPDE5 (Amino acid 535-860 of WT, R732D); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRDGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 465 | 502 |
| OT-001297 (AcGFP (Amino acid 1-239 of WT); linker (GS); (hPDE5 (Amino acid 535-860 of WT, R732T); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGSEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRTGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 466 | 503 |

In some instances, the phenylalanine at position 736 (also referred to as F736) of the hPDE5 (SEQ ID NO: 1), may be mutated to any of the known amino acids and are provided in Table 9. The mutations of the amino acid F736 may include, but are not limited to F736G, F736A, F736L, F736M, F736R, F736W, F736K, F736Q, F736E, F736S, F736P, F736V, F736I, F736C, F736Y, F736H, F736N, F736D, and F736T. The hPDE5 mutants fused either at the N terminus or the C terminus to a linker and GFP are provided in Table 9. Also provided in Table 9 are the components that may be combined with the hPDE5 F736 mutants to generate constructs. In Table 9, the "*" indicates the translation of the stop codon.

**Table 9: hPDE5 F736 constructs**

| **Construct ID/Description** | **Amino acid sequence** | **AA SEQ ID** | **NA SEQ ID** |
|---|---|---|---|
| AcGFP (Amino acid 1-239 of WT) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYK | 365 | 373 |
| linker (GS) | GS | - | GGATC C |
| Xba I site | SR | - | TCTAG A |
| hPDE5 (Amino acid 535-860 of WT, F736G) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFGELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8555 | 8592 |
| hPDE5 (Amino acid 535-860 of WT, F736A) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFAELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 9 | 8593 |
| hPDE5 (Amino acid 535-860 of WT, F736L) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFLELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8556 | 8594 |
| hPDE5 (Amino acid 535-860 of WT, F736M) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFMELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8557 | 8595 |
| hPDE5 (Amino acid 535-860 of WT, F736R) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFRELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8558 | 8596 |
| hPDE5 (Amino acid 535-860 of WT, F736W) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS | 8559 | 8597 |
| | PGNQILSGLSIEEYKTTLKIIKQAILATDLALYIKRRGEFWELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | | |
| hPDE5 (Amino acid 535-860 of WT, F736K) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFKELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8560 | 8598 |
| hPDE5 (Amino acid 535-860 of WT, F736Q) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFQELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8561 | 8599 |
| hPDE5 (Amino acid 535-860 of WT, F736E) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFEELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8562 | 8600 |
| hPDE5 (Amino acid 535-860 of WT, F736S) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFSELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8563 | 8601 |
| hPDE5 (Amino acid 535-860 of WT, F736P) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFPELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8564 | 8602 |
| hPDE5 (Amino acid 535-860 of WT, F736V) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFVELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8565 | 8603 |
| hPDE5 (Amino acid 535-860 of WT, F736I) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFIELIRKNQFNLEDPHQKE LFLAMLMTACDLSAITKPWPIQQRIAELVATEFFDQGDRERKELNIEPTDLMNRE KKNKIPSMQVGFIDAICLQLYEALTHVSEDCFPLLDGCRKNRQKWQALAEQQ | 8566 | 8604 |
| hPDE5 (Amino acid 535-860 of WT, F736C) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFCELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8567 | 8605 |
| hPDE5 (Amino acid 535-860 of WT, F736Y) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFYELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8568 | 8606 |
| hPDE5 (Amino acid 535-860 of WT, F736H) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS | 8569 | 8607 |
| | PGNQILSGLSIEEYKTTLKIIKQAILATDLALYIKRRGEFHELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | | |
| hPDE5 (Amino acid 535-860 of WT, F736N) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFNELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8570 | 8608 |
| hPDE5 (Amino acid 535-860 of WT, F736D) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFDELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8571 | 8609 |
| hPDE5 (Amino acid 535-860 of WT, F736T) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFTELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8572 | 8610 |
| OT-001259 (AcGFP (1-239 of WT); Linker (GS); hPDE5 (Amino acid 535-860 of WT, F736G); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFGELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8573 | 8611 |
| OT-001260 (AcGFP (1-239 of WT); Linker (GS); hPDE5 (Amino acid 535-860 of WT, F736A); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFAELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8574 | 8612 |
| OT-001261 (AcGFP (1-239 of WT); Linker (GS); hPDE5 (Amino acid 535-860 of WT, F736L); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFLELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8575 | 8613 |
| OT-001262 (AcGFP (1-239 of WT); Linker (GS); hPDE5 (Amino acid 535-860 of WT, F736M); Xba | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR | 8576 | 8614 |
| I site (TCTAGA); stop) | HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFMELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | | |
| OT-001263 (AcGFP (1-239 of WT); Linker (GS); hPDE5 (Amino acid 535-860 of WT, F736R); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFRELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8577 | 8615 |
| OT-001264 (AcGFP (1-239 of WT); Linker (GS); hPDE5 (Amino acid 535-860 of WT, F736W); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFWELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8578 | 8616 |
| OT-001265 (AcGFP (1-239 of WT); Linker (GS); hPDE5 (Amino acid 535-860 of WT, F736K); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFKELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8579 | 8617 |
| OT-001266 (AcGFP (1-239 of WT); Linker (GS); hPDE5 (Amino acid 535-860 of WT, F736Q); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFQELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8580 | 8618 |
| OT-001267 (AcGFP (1-239 of WT); Linker (GS); hPDE5 (Amino acid 535-860 of WT, F736E); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8581 | 8619 |
| OT-001268 (AcGFP (1-239 of WT); Linker (GS); hPDE5 (Amino acid 535-860 of WT, F736S); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFSELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8582 | 8620 |
| OT-001269 (AcGFP (1-239 of WT); Linker (GS); hPDE5 (Amino acid 535-860 of WT, F736P); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFPELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8583 | 8621 |
| OT-001270 (AcGFP (1-239 of WT); Linker (GS); hPDE5 (Amino acid 535-860 of WT, F736V); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFVELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8584 | 8622 |
| OT-001271 (AcGFP (1-239 of WT); Linker (GS); hPDE5 (Amino acid 535-860 of WT, F736I); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFIELIRKNQFNLEDPHQKELFLAMLMTACDLSAITKPWPIQQRIAELVA TEFFDQGDRERKELNIEPTDLMNREKKNKIPSMQVGFIDAICLQLYEALTHVSED CFPLLDGCRKNRQKWQALAEQQSR* | 8585 | 8623 |
| OT-001272 (AcGFP (1-239 of WT); Linker (GS); hPDE5 (Amino acid 535-860 of WT, F736C); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFCELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8586 | 8624 |
| OT-001273 (AcGFP (1-239 of WT); Linker (GS); hPDE5 (Amino acid 535-860 of WT, F736Y); Xba | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF | 8587 | 8625 |
| I site (TCTAGA); stop) | ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFYELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | | |
| OT-001274 (AcGFP (1-239 of WT); Linker (GS); hPDE5 (Amino acid 535-860 of WT, F736H); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFHELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8588 | 8626 |
| OT-001275 (AcGFP (1-239 of WT); Linker (GS); hPDE5 (Amino acid 535-860 of WT, F736N); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFNELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8589 | 8627 |
| OT-001276 (AcGFP (1-239 of WT); Linker (GS); hPDE5 (Amino acid 535-860 of WT, F736D); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFDELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8590 | 8628 |
| OT-001277 (AcGFP (1-239 of WT); Linker (GS); hPDE5 (Amino acid 535-860 of WT, F736T); Xba I site (TCTAGA); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFTELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSR* | 8591 | 8629 |

In some instances, any of the mutations taught in Table 1, Table 3, Table 6, Table 9 may be combined. In one instance, the combination mutations are provided in Table 10. Combination mutations may be linked to AcGFP (Amino acid 2-239 of WT) (SEQ ID NO: 79); encoded by the nucleotide sequence of SEQ ID NO: 372; AcGFP (Amino acid 1-239 of WT) (SEQ ID NO: 365); encoded by the nucleotide sequence of SEQ ID NO: 94; or firefly luciferase, Flue (N50D, N119G, S548I, K549A, L550V) (SEQ ID NO: 223) encoded by the nucleotide sequence of SEQ ID NO: 224. In some instances, the portions of the construct may be linked through a linker e.g. SG linker, encoded by the nucleotide sequence (AGTGGT) or a GGSGGG linker (SEQ ID NO: 8630), encoded by (SEQ ID NO: 8631). In some instances, the constructs described in Table 10 may comprise an Xba I restriction site, SR, encoded by the nucleotide sequence, TCTAGA. Table 10 provides the constructs comprising the combination mutations. In Table 10, translation of the stop codon is indicated by asterisk.

**Table 10: hPDE5 combination mutations**

| **Construct ID/ Description** | **Amino Acid Sequence** | **Amino acid SEQ ID NO:** | **Nucleic Acid SEQ ID NO:** |
|---|---|---|---|
| hPDE5 (Amino acid 535-860 of WT, F736A, D764N) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRRGEFAELIRKNQFNLEDPHQKELFL AMLMTACNLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 504 | 519 |
| Methionine; hPDE5 (Amino acid 535-860 of WT, F736A, D764N) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFAELIRKNQFNLEDPHQKELF LAMLMTACNLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 225 | 231 |
| hPDE5 (Amino acid 535-860 of WT, R732L, D764N) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACNLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 505 | 520 |
| Methionine; hPDE5 (Amino acid 535-860 of WT, R732L, D764N) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRLGEFFELIRKNQFNLEDPHQKELF LAMLMTACNLSAITKPWPIQQRIAELVATEFFDQGDRERKELNIEPTDLMNREKK NKIPSMQVGFIDAICLQLYEALTHVSEDCFPLLDGCRKNRQKWQALAEQQ | 226 | 232 |
| hPDE5 (Amino acid 535-860 of WT, R732L, F736A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFAELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 227 | 233 |
| hPDE5 (Amino acid 535-860 of WT, Y612F, R732L) | EETRELQSLAAAVVPSAQTLKITDFSFSDFELSDLETALCTIRMFTDLNLVQNFQM KHEVLCRWILSVKKNYRKNVAFHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 506 | 521 |
| hPDE5 (Amino acid 535-860 of WT, Y612W, R732L) | EETRELQSLAAAVVPSAQTLKITDFSFSDFELSDLETALCTIRMFTDLNLVQNFQM KHEVLCRWILSVKKNYRKNVAWHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 507 | 522 |
| hPDE5 (Amino acid 535-860 of WT, Y612A, R732L) | EETRELQSLAAAVVPSAQTLKITDFSFSDFELSDLETALCTIRMFTDLNLVQNFQM KHEVLCRWILSVKKNYRKNVAAHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL | 508 | 523 |
| | AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | | |
| hPDE5 (Amino acid 535-860 of WT, H653A, R732L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSADLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 509 | 524 |
| hPDE5 (Amino acid 535-860 of WT, R732L, D764A) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKELFL AMLMTACALSAITKPWPIQQRIAELVATEFFDQGDRERKELNIEPTDLMNREKKN KIPSMQVGFIDAICLQLYEALTHVSEDCFPLLDGCRKNRQKWQALAEQQ | 510 | 525 |
| hPDE5 (Amino acid 535-860 of WT, D724G, D764G) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATGLALYIKRRGEFFELIRKNQFNLEDPHQKELFL AMLMTACGLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 8632 | 8640 |
| hPDE5 (Amino acid 535-860 of WT, Y728L, D764N) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATDLALLIKRRGEFFELIRKNQFNLEDPHQKELFLA MLMTACNLSAITKPWPIQQRIAELVATEFFDQGDRERKELNIEPTDLMNREKKNKI PSMQVGFIDAICLQLYEALTHVSEDCFPLLDGCRKNRQKWQALAEQQ | 8633 | 8641 |
| hPDE5 (Amino acid 535-860 of WT, E682A, R732L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMAHHHFDQCLMILNSPGN QILSGLSIEEYKTTLKIIKQAILATDLALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 8634 | 8642 |
| hPDE5 (Amino acid 535-860 of WT, D724A, R732L) | EETRELQSLAAAVVPSAQTLKITDFSFSDFELSDLETALCTIRMFTDLNLVQNFQM KHEVLCRWILSVKKNYRKNVAYHNWRHAFNTAQCMFAALKAGKIQNKLTDLEI LALLIAALSHDLDHRGVNNSYIQRSEHPLAQLYCHSIMEHHHFDQCLMILNSPGN QILSGLSIEEYKTTLKIIKQAILATALALYIKRLGEFFELIRKNQFNLEDPHQKELFL AMLMTACDLSAITKPWPIQQRIAELVATEFFDQGDRERKELNIEPTDLMNREKKN KIPSMQVGFIDAICLQLYEALTHVSEDCFPLLDGCRKNRQKWQALAEQQ | 8635 | 8643 |
| OT-001222 (Methionine; hPDE5 (Amino acid 535-860 of WT, F736A, D764N); linker (SG); AcGFP (Amino acid 2-239 of WT); stop | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRRGEFAELIRKNQFNLEDPHQKELF LAMLMTACNLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 228 | 234 |
| OT-001223 (Methionine; hPDE5 (Amino acid 535-860 of WT, R732L, D764N); linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRLGEFFELIRKNQFNLEDPHQKELF LAMLMTACNLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 229 | 235 |
| OT-001231 (Flue (N50D, N119G, S5481, K549A, L550V); linker (SG); hPDE5 (Amino acid 535-860 of WT, R732L; F736A); stop) | MEDAKNIKKGPAPFYPLEDGTAGEQLHKAMKRYALVPGTIAFTDAHIEVDITYAE YFEMSVRLAEAMKRYGLNTNHRIVVCSENSLQFFMPVLGALFIGVAVAPANDIYN ERELLNSMGISQPTVVFVSKKGLQKILNVQKKLPIIQKIIIMDSKTDYQGFQSMYTF VTSHLPPGFNEYDFVPESFDRDKTIALIMNSSGSTGLPKGVALPHRTACVRFSHAR DPIFGNQIIPDTAILSVVPFHHGFGMFTTLGYLICGFRVVLMYRFEEELFLRSLQDY KIQSALLVPTLFSFFAKSTLIDKYDLSNLHEIASGGAPLSKEVGEAVAKRFHLPGIR QGYGLTETTSAILITPEGDDKPGAVGKVVPFFEAKVVDLDTGKTLGVNQRGELCV RGPMIMSGYVNNPEATNALIDKDGWLHSGDIAYWDEDEHFFIVDRLKSLIKYKG YQVAPAELESILLQHPNIFDAGVAGLPDDDAGELPAAVVVLEHGKTMTEKEIVDY VASQVTTAKKLRGGVVFVDEVPKGLTGKLDARKIREILIKAKKGGKIAVSGEETR ELQSLAAAVVPSAQTLKITDFSFSDFELSDLETALCTIRMFTDLNLVQNFQMKHEV LCRWILSVKKNYRKNVAYHNWRHAFNTAQCMFAALKAGKIQNKLTDLEILALLI AALSHDLDHRGVNNSYIQRSEHPLAQLYCHSIMEHHHFDQCLMILNSPGNQILSG LSIEEYKTTLKIIKQAILATDLALYIKRLGEFAELIRKNQFNLEDPHQKELFLAMLM TACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ* | 230 | 236 |
| OT-001225 (Methionine; hPDE5 (Amino acid 535-860 of WT, R732L, F736A); Linker (SG); AcGFP (Amino acid 2-239 of WT); stop) | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALYIKRLGEFAELIRKNQFNLEDPHQKELF LAMLMTACDLSAITKPWPIQQRIAELVATEFFDQGDRERKELNIEPTDLMNREKK NKIPSMQVGFIDAICLQLYEALTHVSEDCFPLLDGCRKNRQKWQALAEQQSGVSK GAELFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAEVKF EGDTLVNRIELTGTDFKEDGNILGNKMEYNYNAHNVYIMTDKAKNGIKVNFKIR HNIEDGSVQLADHYQQNTPIGDGPVLLPDNHYLSTQSALSKDPNEKRDHMIYFGF VTAAAITHGMDELYK* | 511 | 526 |
| OT-001226 (AcGFP (Amino acid 1-239 of WT); linker (SG); PDE5 (Amino acid 535-860 of WT, R732L, F736A); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF AELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 512 | 527 |
| OT-001205 (AcGFP (Amino acid 1-239 of WT); linker (SG); hPDE5 (Amino acid 535-860 of WT, Y612F, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAFHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 513 | 528 |
| OT-001206 (AcGFP (Amino acid 1-239 of WT); linker (SG); hPDE5 (Amino acid 535-860 of WT, Y612W, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAWHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 514 | 529 |
| OT-001207 (AcGFP (Amino acid 1-239 of WT); linker (SG); hPDE5 (Amino acid 535-860 of WT, Y612A, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAAHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 515 | 530 |
| OT-001212 (AcGFP (Amino acid 1-239 of WT); linker (SG); hPDE5 (Amino acid 535-860 of WT, H653A, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSADLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 516 | 531 |
| OT-001213 (AcGFP (Amino acid 1-239 of WT); linker (SG); hPDE5 (Amino acid 535-860 of WT, R732L, D764A); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACALSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 517 | 532 |
| OT-001253 (AcGFP (Amino acid 1-239 of WT); linker (SG); hPDE5 (Amino acid 535-860 of WT; R732L); Xba I site (TCTAGA); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQSR* | 518 | 533 |
| OT-001073 (AcGFP (Amino acid 1-239 of WT); linker (GGSGGG (SEQ ID NO: 78)); hPDE5 (535-860 of WT, D724G, D764G); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKGGSGGGEETRELQSLAAAVVPSAQTLKITDFSFSDF ELSDLETALCTIRMFTDLNLVQNFQMKHEVLCRWILSVKKNYRKNVAYHNWRH AFNTAQCMFAALKAGKIQNKLTDLEILALLIAALSHDLDHRGVNNSYIQRSEHPL AQLYCHSIMEHHHFDQCLMILNSPGNQILSGLSIEEYKTTLKIIKQAILATGLALYIK RRGEFFELIRKNQFNLEDPHQKELFLAMLMTACGLSAITKPWPIQQRIAELVATEF FDQGDRERKELNIEPTDLMNREKKNKIPSMQVGFIDAICLQLYEALTHVSEDCFPL LDGCRKNRQKWQALAEQQ* | 8636 | 8644 |
| OT-001251 (Methionine; hPDES (Amino acid 535-860 of WT, Y728L, | MEETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTDL EILALLIAALSHDLDHRGVNNSYIQRSEHPLAQLYCHSIMEHHHFDQCLMILNSPG NQILSGLSIEEYKTTLKIIKQAILATDLALLIKRRGEFFELIRKNQFNLEDPHQKELFL AMLMTACNLSAITKPWPIQQRIAELVATEFFDQGDRERKELNIEPTDLMNREKKN | 8637 | 8645 |
| D764N); linker (SG); AcGFP (2-239 of WT); stop) | KIPSMQVGFIDAICLQLYEALTHVSEDCFPLLDGCRKNRQKWQALAEQQSGVSKG AELFTGIVPILIELNGDVNGHKFSVSGEGEGDATYGKLTLKFICTTGKLPVPWPTL VTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRH NIEDGSVQLADHYQQNTPIGDGPVLLPDNHYLSTQSALSKDPNEKRDHMIYFGFV TAAAITHGMDELYK* | | |
| OT-001214 (AcGFP (1-239 of WT); Linker (SG); hPDE5 (Amino acid 535-860 of WT, E682A, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMAHHHFDQCLMILNSPGNQILSGLSIEEYKTTLKIIKQAILATDLALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 8638 | 8646 |
| OT-001215 (AcGFP (1-239 of WT); linker (SG); hPDE5 (Amino acid 535-860 of WT, D724A, R732L); stop) | MVSKGAELFTGIVPILIELNGDVNGHKFSVSGEGEGDATYGKLTLKFICTTGKLPV PWPTLVTTLSYGVQCFSRYPDHMKQHDFFKSAMPEGYIQERTIFFEDDGNYKSRA EVKFEGDTLVNRIELTGTDFKEDGNILGNKMEYNYNAHNVYIMTDKAKNGIKVN FKIRHNIEDGSVQLADHYQQNTPIGDGPVLLPDNHYLSTQSALSKDPNEKRDHMI YFGFVTAAAITHGMDELYKSGEETRELQSLAAAVVPSAQTLKITDFSFSDFELSDL ETALCTIRMFTDLNLVQNFQMKHEVLCRWILSVKKNYRKNVAYHNWRHAFNTA QCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATALALYIKRLGEF FELIRKNQFNLEDPHQKELFLAMLMTACDLSAITKPWPIQQRIAELVATEFFDQGD RERKELNIEPTDLMNREKKNKIPSMQVGFIDAICLQLYEALTHVSEDCFPLLDGCR KNRQKWQALAEQQ* | 8639 | 8647 |

In some instances, any of the mutations described herein may be tested in the context of the truncated hPDE5 domains. As used herein truncated hPDE5 domains refers to regions and/or portions of hPDE5 of SEQ ID NO: 1, and are exemplified in Table 11. Also provided in Table 11 are the constructs utilizing the regions or portions of hPDE5, fused to AcGFP via linkers. In Table 11, translation of the stop codon is indicated by asterisk.

**Table 11: hPDE5 truncation constructs and its components**

| **Construct ID/ Description** | **Sequence** | **Amino acid SEQ ID NO** | **Nucleic Acid SEQ ID NO** |
|---|---|---|---|
| Methionine; hPDES (Amino acid 535-860 of WT) | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGNQILSGL SIEEYKTTLKIIKQAILATDLALYIKRRGEFFELIRKNQFNLEDPHQKELFLAMLMTAC DLSAITKPWPIQQRIAELVATEFFDQGDRERKELNIEPTDLMNREKKNKIPSMQVGFI DAICLQLYEALTHVSEDCFPLLDGCRKNRQKWQALAEQQ | 237 | 4 |
| Methionine; hPDES (Amino acid 535-860 of WT, R732L) | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGNQILSGL SIEEYKTTLKIIKQAILATDLALYIKRLGEFFELIRKNQFNLEDPHQKELFLAMLMTAC DLSAITKPWPIQQRIAELVATEFFDQGDRERKELNIEPTDLMNREKKNKIPSMQVGFI DAICLQLYEALTHVSEDCFPLLDGCRKNRQKWQALAEQQ | 238 | 250 |
| Methionine; hPDE5 (Amino acid 535-836 of WT, R732L) | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGNQILSGL SIEEYKTTLKIIKQAILATDLALYIKRLGEFFELIRKNQFNLEDPHQKELFLAMLMTAC | 239 | 251 |
| | DLSAITKPWPIQQRIAELVATEFFDQGDRERKELNIEPTDLMNREKKNKIPSMQVGFI DAICLQLYEALTHVS | | |
| Methionine; hPDE5 (Amino acid 567-860 of WT, R732L) | MSDLETALCTIRMFTDLNLVQNFQMKHEVLCRWILSVKKNYRKNVAYHNWRHAFN TAQCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEFFE LIRKNQFNLEDPHQKELFLAMLMTACDLSAITKPWPIQQRIAELVATEFFDQGDRER KELNIEPTDLMNREKKNKIPSMQVGFIDAICLQLYEALTHVSEDCFPLLDGCRKNRQ KWQALAEQQ | 240 | 252 |
| hPDE5 (Amino acid 590-860 of WT, R732L) | MKHEVLCRWILSVKKNYRKNVAYHNWRHAFNTAQCMFAALKAGKIQNKLTDLEIL ALLIAALSHDLDHRGVNNSYIQRSEHPLAQLYCHSIMEHHHFDQCLMILNSPGNQILS GLSIEEYKTTLKIIKQAILATDLALYIKRLGEFFELIRKNQFNLEDPHQKELFLAMLMT ACDLSAITKPWPIQQRIAELVATEFFDQGDRERKELNIEPTDLMNREKKNKIPSMQVG FIDAICLQLYEALTHVSEDCFPLLDGCRKNRQKWQALAEQQ | 241 | 253 |
| hPDE5 (Amino acid 590-836 of WT, R732L) | MKHEVLCRWILSVKKNYRKNVAYHNWRHAFNTAQCMFAALKAGKIQNKLTDLEIL ALLIAALSHDLDHRGVNNSYIQRSEHPLAQLYCHSIMEHHHFDQCLMILNSPGNQILS GLSIEEYKTTLKIIKQAILATDLALYIKRLGEFFELIRKNQFNLEDPHQKELFLAMLMT ACDLSAITKPWPIQQRIAELVATEFFDQGDRERKELNIEPTDLMNREKKNKIPSMQVG FIDAICLQLYEALTHVS | 242 | 254 |
| hPDE5 (Amino acid 535-860 of WT) | EETRELQSLAAAVVPSAQTLKITDFSFSDFELSDLETALCTIRMFTDLNLVQNFQMKH EVLCRWILSVKKNYRKNVAYHNWRHAFNTAQCMFAALKAGKIQNKLTDLEILALLI AALSHDLDHRGVNNSYIQRSEHPLAQLYCHSIMEHHHFDQCLMILNSPGNQILSGLSI EEYKTTLKIIKQAILATDLALYIKRRGEFFELIRKNQFNLEDPHQKELFLAMLMTACD LSAITKPWPIQQRIAELVATEFFDQGDRERKELNIEPTDLMNREKKNKIPSMQVGFID AICLQLYEALTHVSEDCFPLLDGCRKNRQKWQALAEQQ | 3 | 339 |
| hPDE5 (Amino acid 535-860 of WT, R732L) | EETRELQSLAAAVVPSAQTLKITDFSFSDFELSDLETALCTIRMFTDLNLVQNFQMKH EVLCRWILSVKKNYRKNVAYHNWRHAFNTAQCMFAALKAGKIQNKLTDLEILALLI AALSHDLDHRGVNNSYIQRSEHPLAQLYCHSIMEHHHFDQCLMILNSPGNQILSGLSI EEYKTTLKIIKQAILATDLALYIKRLGEFFELIRKNQFNLEDPHQKELFLAMLMTACD LSAITKPWPIQQRIAELVATEFFDQGDRERKELNIEPTDLMNREKKNKIPSMQVGFID AICLQLYEALTHVSEDCFPLLDGCRKNRQKWQALAEQQ | 12 | 536 |
| hPDE5 (Amino acid 535-836 of WT, R732L) | EETRELQSLAAAVVPSAQTLKITDFSFSDFELSDLETALCTIRMFTDLNLVQNFQMKH EVLCRWILSVKKNYRKNVAYHNWRHAFNTAQCMFAALKAGKIQNKLTDLEILALLI AALSHDLDHRGVNNSYIQRSEHPLAQLYCHSIMEHHHFDQCLMILNSPGNQILSGLSI EEYKTTLKIIKQAILATDLALYIKRLGEFFELIRKNQFNLEDPHQKELFLAMLMTACD LSAITKPWPIQQRIAELVATEFFDQGDRERKELNIEPTDLMNREKKNKIPSMQVGFID AICLQLYEALTHVS | 534 | 537 |
| hPDE5 (Amino acid 567-860 of WT, R732L) | SDLETALCTIRMFTDLNLVQNFQMKHEVLCRWILSVKKNYRKNVAYHNWRHAFNT AQCMFAALKAGKIQNKLTDLEILALLIAALSHDLDHRGVNNSYIQRSEHPLAQLYCH SIMEHHHFDQCLMILNSPGNQILSGLSIEEYKTTLKIIKQAILATDLALYIKRLGEFFELI RKNQFNLEDPHQKELFLAMLMTACDLSAITKPWPIQQRIAELVATEFFDQGDRERKE LNIEPTDLMNREKKNKIPSMQVGFIDAICLQLYEALTHVSEDCFPLLDGCRKNRQKW QALAEQQ | 535 | 538 |
| Linker (SG) | SG | - | AGTGGT |
| AcGFP | VSKGAELFTGIVPILIELNGDVNGHKFSVSGEGEGDATYGKLTLKFICTTGKLPVPWP TLVTTLSYGVQCFSRYPDHMKQHDFFKSAMPEGYIQERTIFFEDDGNYKSRAEVKFE GDTLVNRIELTGTDFKEDGNILGNKMEYNYNAHNVYIMTDKAKNGIKVNFKIRHNIE DGSVQLADHYQQNTPIGDGPVLLPDNHYLSTQSALSKDPNEKRDHMIYFGFVTAAAI THGMDELYK | 79 | 372 |
| OT-001216 (Methionine; hPDE5 (Amino acid 535-860 of WT); linker (SG); AcGFP (Amino acid 2-239 of WT); stop) | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGNQILSGL SIEEYKTTLKIIKQAILATDLALYIKRRGEFFELIRKNQFNLEDPHQKELFLAMLMTAC DLSAITKPWPIQQRIAELVATEFFDQGDRERKELNIEPTDLMNREKKNKIPSMQVGFI DAICLQLYEALTHVSEDCFPLLDGCRKNRQKWQALAEQQSGVSKGAELFTGIVPILIE LNGDVNGHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIELTGTDFK EDGNILGNKMEYNYNAHNVYIMTDKAKNGIKVNFKIRHNIEDGSVQLADHYQQNTP IGDGPVLLPDNHYLSTQSALSKDPNEKRDHMIYFGFVTAAAITHGMDELYK* | 243 | 255 |
| OT-001217 (Methionine; | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL | 244 | 256 |
| hPDE5 (Amino acid 535-860 of WT, R732L); linker (SG); AcGFP; stop (Amino acid 2-239 of WT); stop) | LIAALSHDLDHRGVNNSYIQRSEHPLAQLYCHSIMEHHHFDQCLMILNSPGNQILSGL SIEEYKTTLKIIKQAILATDLALYIKRLGEFFELIRKNQFNLEDPHQKELFLAMLMTAC DLSAITKPWPIQQRIAELVATEFFDQGDRERKELNIEPTDLMNREKKNKIPSMQVGFI DAICLQLYEALTHVSEDCFPLLDGCRKNRQKWQALAEQQSGVSKGAELFTGIVPILIE LNGDVNGHKFSVSGEGEGDATYGKLTLKFICTTGKLPVPWPTLVTTLSYGVQCFSRY PDHMKQHDFFKSAMPEGYIQERTIFFEDDGNYKSRAEVKFEGDTLVNRIELTGTDFK EDGNILGNKMEYNYNAHNVYIMTDKAKNGIKVNFKIRHNIEDGSVQLADHYQQNTP IGDGPVLLPDNHYLSTQSALSKDPNEKRDHMIYFGFVTAAAITHGMDELYK* | | |
| OT-001218 (Methionine; hPDE5 (Amino acid 535-836 of WT, R732L); linker (SG); AcGFP (Amino acid 2-239 of WT); stop) | MEETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGNQILSGL SIEEYKTTLKIIKQAILATDLALYIKRLGEFFELIRKNQFNLEDPHQKELFLAMLMTAC DLSAITKPWPIQQRIAELVATEFFDQGDRERKELNIEPTDLMNREKKNKIPSMQVGFI DAICLQLYEALTHVSSGVSKGAELFTGIVPILIELNGDVNGHKFSVSGEGEGDATYGK LTLKFICTTGKLPVPWPTLVTTLSYGVQCFSRYPDHMKQHDFFKSAMPEGYIQERTIF FEDDGNYKSRAEVKFEGDTLVNRIELTGTDFKEDGNILGNKMEYNYNAHNVYIMTD KAKNGIKVNFKIRHNIEDGSVQLADHYQQNTPIGDGPVLLPDNHYLSTQSALSKDPN EKRDHMIYFGFVTAAAITHGMDELYK* | 245 | 257 |
| OT-001219 (Methionine; hPDE5 (Amino acid 567-860 of WT, R732L); linker (SG); AcGFP (Amino acid 2-239 of WT); stop) | MSDLETALCTIRMFTDLNLVQNFQMKHEVLCRWILSVKKNYRKNVAYHNWRHAFN TAQCMFAALKAGKIQNKLTDLEILALLIAALSHDLDHRGVNNSYIQRSEHPLAQLYC HSIMEHHHFDQCLMILNSPGNQILSGLSIEEYKTTLKIIKQAILATDLALYIKRLGEFFE LIRKNQFNLEDPHQKELFLAMLMTACDLSAITKPWPIQQRIAELVATEFFDQGDRER KELNIEPTDLMNREKKNKIPSMQVGFIDAICLQLYEALTHVSEDCFPLLDGCRKNRQ KWQALAEQQSGVSKGAELFTGIVPILIELNGDVNGHKFSVSGEGEGDATYGKLTLKF ICTTGKLPVPWPTLVTTLSYGVQCFSRYPDHMKQHDFFKSAMPEGYIQERTIFFEDD GNYKSRAEVKFEGDTLVNRIELTGTDFKEDGNILGNKMEYNYNAHNVYIMTDKAK NGIKVNFKIRHNIEDGSVQLADHYQQNTPIGDGPVLLPDNHYLSTQSALSKDPNEKR DHMIYFGFVTAAAITHGMDELYK* | 246 | 258 |
| OT-001220 (hPDE5 (Amino acid 590-860 of WT, R732L); linker (SG); AcGFP (Amino acid 2-239 of WT); stop) | MKHEVLCRWILSVKKNYRKNVAYHNWRHAFNTAQCMFAALKAGKIQNKLTDLEIL ALLIAALSHDLDHRGVNNSYIQRSEHPLAQLYCHSIMEHHHFDQCLMILNSPGNQILS GLSIEEYKTTLKIIKQAILATDLALYIKRLGEFFELIRKNQFNLEDPHQKELFLAMLMT ACDLSAITKPWPIQQRIAELVATEFFDQGDRERKELNIEPTDLMNREKKNKIPSMQVG FIDAICLQLYEALTHVSEDCFPLLDGCRKNRQKWQALAEQQSGVSKGAELFTGIVPIL IELNGDVNGHKFSVSGEGEGDATYGKLTLKFICTTGKLPVPWPTLVTTLSYGVQCFS RYPDHMKQHDFFKSAMPEGYIQERTIFFEDDGNYKSRAEVKFEGDTLVNRIELTGTD FKEDGNILGNKMEYNYNAHNVYIMTDKAKNGIKVNFKIRHNIEDGSVQLADHYQQ NTPIGDGPVLLPDNHYLSTQSALSKDPNEKRDHMIYFGFVTAAAITHGMDELYK* | 247 | 259 |
| OT-001221 (hPDE5 (Amino acid 590-836 of WT, R732L); linker (SG); AcGFP (Amino acid 2-239 of WT); stop) | MKHEVLCRWILSVKKNYRKNVAYHNWRHAFNTAQCMFAALKAGKIQNKLTDLEIL ALLIAALSHDLDHRGVNNSYIQRSEHPLAQLYCHSIMEHHHFDQCLMILNSPGNQILS GLSIEEYKTTLKIIKQAILATDLALYIKRLGEFFELIRKNQFNLEDPHQKELFLAMLMT ACDLSAITKPWPIQQRIAELVATEFFDQGDRERKELNIEPTDLMNREKKNKIPSMQVG FIDAICLQLYEALTHVSSGVSKGAELFTGIVPILIELNGDVNGHKFSVSGEGEGDATY GKLTLKFICTTGKLPVPWPTLVTTLSYGVQCFSRYPDHMKQHDFFKSAMPEGYIQER TIFFEDDGNYKSRAEVKFEGDTLVNRIELTGTDFKEDGNILGNKMEYNYNAHNVYI MTDKAKNGIKVNFKIRHNIEDGSVQLADHYQQNTPIGDGPVLLPDNHYLSTQSALSK DPNEKRDHMIYFGFVTAAAITHGMDELYK* | 248 | 260 |

Mutant library wherein both the amino acid at the position 732 (R732) as well as at position 736 (F736) were mutated to any of the known amino acids, including, but not limited to, aspartic acid, glutamic acid, glutamine, asparagine, histidine, serine, threonine, tyrosine, cysteine, methionine, tryptophan, alanine, isoleucine, leucine, phenylalanine, valine, proline, and glycine were generated. Mutants were screened by appended DDs to payloads such as GFP and mcherry. Table 12 provides the R732, F736 mutants and related constructs. In Table 12, * indicates the translation of the stop codon.

**Table 12: hPDE5(R732, F736) DDs, constructs and its components**

| **Construct ID/ Description** | **Sequence** | **AA SEQ ID NO** | **NA SEQ ID NO** |
|---|---|---|---|
| **DDs** | | | |
| hPDE5 (aa 535 to 860 of WT, R732D, F736S) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRDGEFSELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8721 | 8763 |
| hPDE5 (aa 535 to 860 of WT, R732E, F736D) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKREGEFDELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8722 | 8764 |
| hPDE5 (aa 535 to 860 of WT, R732V, F736G) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRVGEFGELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8723 | 8765 |
| hPDE5 (aa 535 to 860 of WT, R732W, F736G) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRWGEFGELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8724 | 8766 |
| hPDE5 (aa 535 to 860 of WT, R732W, F736V) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRWGEFVELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8725 | 8767 |
| hPDE5 (aa 535 to 860 of WT, R732L, F736W) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRLGEFWELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8726 | 8768 |
| hPDE5 (aa 535 to 860 of WT, R732P, F736Q) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRPGEFQELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8727 | 8769 |
| hPDE5 (aa 535 to 860 of WT, R732A, F736A) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRAGEFAELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8728 | 8770 |
| hPDE5 (aa 535 to 860 of WT, R732S, F736G) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRSGEFGELIRKNQFNLEDPHQKELFLAMLM | 8729 | 8771 |
| | TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | | |
| hPDE5 (aa 535 to 860 of WT, R732T, F736P) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRTGEFPELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8730 | 8772 |
| hPDE5 (aa 535 to 860 of WT, R732M, F736H) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRMGEFHELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8731 | 8773 |
| hPDE5 (aa 535 to 860 of WT, R732Y, F736M) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRYGEFMELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8732 | 8774 |
| hPDE5 (aa 535 to 860 of WT, R732P, F736D) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRPGEFDELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8733 | 8775 |
| hPDE5 (aa 535 to 860 of WT, R732P, F736G) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRPGEFGELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8734 | 8776 |
| hPDE5 (aa 535 to 860 of WT, R732W, F736L) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRWGEFLELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8735 | 8777 |
| hPDE5 (aa 535 to 860 of WT, R732L, F736S) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRLGEFSELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8736 | 8778 |
| hPDE5 (aa 535 to 860 of WT, R732D, F736T) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRDGEFTELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8737 | 8779 |
| hPDE5 (aa 535 to 860 of WT, R732L, F736V) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRLGEFVELIRKNQFNLEDPHQKELFLAMLM TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | 8738 | 8780 |
| hPDE5 (aa 535 to 860 of WT, R732G, F736V) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRGGEFVELIRKNQFNLEDPHQKELFLAMLM | 8739 | 8781 |
| | TACDLSAITKPWPIQQRIAELVATEFFDQGDRERKELNIEPTDLMNREKKNKIPSMQ VGFIDAICLQLYEALTHVSEDCFPLLDGCRKNRQKWQALAEQQ | | |
| hPDE5 (aa 535 to 860 of WT, R732W, F736A) | EETRELQSLAAAVVPSAQTLKITDFSFSDFELSDLETALCTIRMFTDLNLVQNFQMK HEVLCRWILSVKKNYRKNVAYHNWRHAFNTAQCMFAALKAGKIQNKLTDLEILA LLIAALSHDLDHRGVNNSYIQRSEHPLAQLYCHSIMEHHHFDQCLMILNSPGNQILS GLSIEEYKTTLKIIKQAILATDLALYIKRWGEFAELIRKNQFNLEDPHQKELFLAML MTACDLSAITKPWPIQQRIAELVATEFFDQGDRERKELNIEPTDLMNREKKNKIPSM QVGFIDAICLQLYEALTHVSEDCFPLLDGCRKNRQKWQALAEQQ | 8740 | 8782 |

| **Construct components** | | | |
|---|---|---|---|
| AcGFP (1-239 of WT) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYK | 365 | 373 |
| Linker (GS); BamH1 Site | GS | | |
| GSLDG Linker | GSLDG | 8741 | 8783 |
| mCherry (M1L) | LSKGEEDNMAIIKEFMRFKVHMEGSVNGHEFEIEGEGEGRPYEGTQTAKLKVTKG GPLPFAWDILSPQFMYGSKAYVKHPADIPDYLKLSFPEGFKWERVMNFEDGGVVT VTQDSSLQDGEFIYKVKLRGTNFPSDGPVMQKKTMGWEASSERMYPEDGALKGEI KQRLKLKDGGHYDAEVKTTYKAKKPVQLPGAYNVNIKLDITSHNEDYTIVEQYER AEGRHSTGGMDELYK | 8240 | 8269 |
| P2A cleavage site | ATNFSLLKQAGDVEENPGP | 8742 | 8784 |

| **Constructs** | | | |
|---|---|---|---|
| OT-001876(AcGFP (1-239 of WT); Linker (GS); hPDE5 (aa 535 to 860 of WT, R732D, F736S); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRDGEFSELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | 8743 | 8785 |
| OT-001875(AcGFP (1-239 of WT); Linker (GS); hPDE5 (aa 535 to 860 of WT, R732E, F736D); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKREGEFDELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | 8744 | 8786 |
| OT-001877(AcGFP (1-239 of WT); Linker (GS); hPDE5 (aa 535 to 860 of WT, R732V, F736G); GSLDG Linker (SEQ ID NO: 8741); P2A | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRVGEFGELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS | 8745 | 8787 |
| cleavage site; mCherry (M1L); stop) | VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | | |
| OT-001879(AcGFP (1-239 of WT); Linker (GS); hPDE5 (aa 535 to 860 of WT, R732W, F736G); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRWGEFGELIRK NQFNLEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKNRQKW QALAEQQGSLDGATNFSLLKQAGDVEENPGPLSKGEEDNMAIIKEFMRFKVHMEG SVNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8746 | 8788 |
| OT-001880(AcGFP (1-239 of WT); Linker (GS); hPDE5 (aa 535 to 860 of WT, R732W, F736V); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRWGEFVELIRK NQFNLEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKNRQKW QALAEQQGSLDGATNFSLLKQAGDVEENPGPLSKGEEDNMAIIKEFMRFKVHMEG SVNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8747 | 8789 |
| OT-001882(AcGFP (1-239 of WT); Linker (GS); hPDE5 (aa 535 to 860 of WT,, R732L, F736W); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRLGEFWELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8748 | 8790 |
| OT-001881(AcGFP (1-239 of WT); Linker (GS); hPDE5 (aa 535 to 860 of WT, R732P, F736Q); GSLDG Linker (SEQ ID NO: 8741); P2A | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRPGEFQELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS | 8749 | 8791 |
| cleavage site; mCherry (M1L); stop) | VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | | |
| OT-001874(AcGFP (1-239 of WT); Linker (GS); hPDE5 (aa 535 to 860 of WT, R732A, F736A); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRAGEFAELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | 8750 | 8792 |
| OT-001883(AcGFP (1-239 of WT); Linker (GS); hPDE5 (aa 535 to 860 of WT, R732S, F736G); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRSGEFGELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8751 | 8793 |
| OT-001884(AcGFP (1-239 of WT); Linker (GS); hPDE5 (aa 535 to 860 of WT, R732T, F736P); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRTGEFPELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8752 | 8794 |
| OT-001885(AcGFP (1-239 of WT); Linker (GS); hPDE5 (aa 535 to 860 of WT, R732M, F736H); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRMGEFHELIRK NQFNLEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKNRQKW QALAEQQGSLDGATNFSLLKQAGDVEENPGPLSKGEEDNMAIIKEFMRFKVHMEG SVNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8753 | 8795 |
| OT-001886(AcGFP (1-239 of WT); Linker (GS); hPDE5 (aa 535 to 860 of WT, R732Y, F736M); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRYGEFMELIRK NQFNLEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKNRQKW QALAEQQGSLDGATNFSLLKQAGDVEENPGPLSKGEEDNMAIIKEFMRFKVHMEG SVNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8754 | 8796 |
| OT-001887(AcGFP (1-239 of WT); Linker (GS); hPDE5 (aa 535 to 860 of WT, R732P, F736D); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRPGEFDELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8755 | 8797 |
| OT-001873(AcGFP (1-239 of WT); Linker (GS); hPDE5 (aa 535 to 860 of WT, R732P, F736G); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRPGEFGELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | 8756 | 8798 |
| OT-001878(AcGFP (1-239 of WT); Linker (GS); hPDE5 (aa 535 to 860 of WT, R732W, F736L); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRWGEFLELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8757 | 8799 |
| OT-001872(AcGFP (1-239 of WT); Linker (GS); | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG | 8758 | 8800 |
| hPDE5 (aa 535 to 860 of WT, R732L, F736S); stop) | FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRLGEFSELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | | |
| OT-001889(AcGFP (1-239 of WT); Linker (GS); hPDE5 (aa 535 to 860 of WT, R732D, F736T); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRDGEFTELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8759 | 8801 |
| OT-001871(AcGFP (1-239 of WT); Linker (GS); hPDE5 (aa 535 to 860 of WT, R732L, F736V); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRLGEFVELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQ* | 8760 | 8802 |
| OT-001888(AcGFP (1-239 of WT); Linker (GS); hPDE5 (aa 535 to 860 of WT, R732G, F736V); GSLDG Linker (SEQ ID NO: 8741); P2A cleavage site; mCherry (M1L); stop) | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRGGEFVELIRKN QFNLEDPHQKELFLAMLMTACDLSAITKPWPIQQRIAELVATEFFDQGDRERKELNI EPTDLMNREKKNKIPSMQVGFIDAICLQLYEALTHVSEDCFPLLDGCRKNRQKWQ ALAEQQGSLDGATNFSLLKQAGDVEENPGPLSKGEEDNMAIIKEFMRFKVHMEGS VNGHEFEIEGEGEGRPYEGTQTAKLKVTKGGPLPFAWDILSPQFMYGSKAYVKHP ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | 8761 | 8803 |
| OT-001890(AcGFP (1-239 of WT); Linker (GS); hPDE5 (aa 535 to 860 of WT, R732W, F736A); GSLDG Linker (SEQ ID NO: 8741); P2A cleavage site; | MVSKGAELFTGIVPILIELNGDVNGHKFSVSGEGEGDATYGKLTLKFICTTGKLPVP WPTLVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIKVNFK IRHNIEDGSVQLADHYQQNTPIGDGPVLLPDNHYLSTQSALSKDPNEKRDHMIYFG FVTAAAITHGMDELYKGSEETRELQSLAAAVVPSAQTLKITDFSFSDFELSDLETAL CTIRMFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRWGEFAELIRK NQFNLEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKNRQKW QALAEQQGSLDGATNFSLLKQAGDVEENPGPLSKGEEDNMAIIKEFMRFKVHMEG SVNGHEFEIEGEGEGRPYEGTQTAKLKVTKGGPLPFAWDILSPQFMYGSKAYVKHP | 8762 | 8804 |
| mCherry (M1L); stop) | ADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKK PVQLPGAYNVNIKLDITSHNEDYTIVEQYERAEGRHSTGGMDELYK* | | |

Saturation mutagenesis screens were performed using mutant library wherein every amino acid within position 535 to 860 of SEQ ID NO: 1 was mutated. Mutants were screened by appended DDs to payloads such as GFP and mcherry Table 13 provides hPDE5 mutants identified by this screen. In Table 13, * indicates the translation of the stop codon.

**Table 13: hPDE5 saturation mutagenesis DD and constructs**

| **Construct ID/ Description** | **Sequence** | **AA SEQ ID NO** | **NA SEQ ID NO** |
|---|---|---|---|
| **DDs** | | | |
| hPDE5 (aa 535 to 860 of WT, C574N) | EETRELQSLAAAVVPSAQTLKITDFSFSDFELSDLETALNTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8805 | 8813 |
| hPDE5 (aa 535 to 860 of WT, E536K, I739W) | EKTRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELWRKNQFNLEDPHQ KELFLAMLMTACDLSAITKPWPIQQRIAELVATEFFDQGDRERKELNIEPTDLMN REKKNKIPSMQVGFIDAICLQLYEALTHVSEDCFPLLDGCRKNRQKWQALAEQQ | 8806 | 8814 |
| hPDE5 (aa 535 to 860 of WT, H678F, S702F) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCFSIMEHHHFDQCLMILNSP GNQILFGLSIEEYKTTLKIIKQAILATDLALYIKRRGEFFELIRKNQFNLEDPHQKE LFLAMLMTACDLSAITKPWPIQQRIAELVATEFFDQGDRERKELNIEPTDLMNRE KKNKIPSMQVGFIDAICLQLYEALTHVSEDCFPLLDGCRKNRQKWQALAEQQ | 8807 | 8815 |

| **Construct components** | | | |
|---|---|---|---|
| AcGFP (1-239 of WT) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYK | 365 | 373 |
| Linker (GS); BamH1 Site | GS | | |
| SLDG Linker | SLDG | 8808 | 8816 |
| mCherry (M1L) | LSKGEEDNMAIIKEFMRFKVHMEGSVNGHEFEIEGEGEGRPYEGTQTAKLKVTK GGPLPFAWDILSPQFMYGSKAYVKHPADIPDYLKLSFPEGFKWERVMNFEDGG VVTVTQDSSLQDGEFIYKVKLRGTNFPSDGPVMQKKTMGWEASSERMYPEDGA LKGEIKQRLKLKDGGHYDAEVKTTYKAKKPVQLPGAYNVNIKLDITSHNEDYTI VEQYERAEGRHSTGGMDELYK | 8240 | 8269 |
| P2A cleavage site | ATNFSLLKQAGDVEENPGP | 8809 | 8817 |

| **Constructs** | | | |
|---|---|---|---|
| OT-001869 (AcGFP (1-239 of WT); Linker | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI | 8810 | 8818 |
| (GS); hPDE5 (aa 535 to 860 of WT, C574N); SLDG Linker (SEQ ID NO: 8808); P2A cleavage site; mCherry (M1L); stop) | KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALNTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSLDGATNFSLLKQAGDVEENPGPLSKGEED NMAIIKEFMRFKVHMEGSVNGHEFEIEGEGEGRPYEGTQTAKLKVTKGGPLPFA WDILSPQFMYGSKAYVKHPADIPDYLKLSFPEGFKWERVMNFEDGGVVTVTQD SSLQDGEFIYKVKLRGTNFPSDGPVMQKKTMGWEASSERMYPEDGALKGEIKQ RLKLKDGGHYDAEVKTTYKAKKPVQLPGAYNVNIKLDITSHNEDYTIVEQYER AEGRHSTGGMDELYK* | | |
| OT-001868 (AcGFP (1-239 of WT); Linker (GS); hPDE5 (aa 535 to 860 of WT E536K, I739W); SLDG Linker (SEQ ID NO: 8808); P2A cleavage site; mCherry (M1L); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEKTRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCHSIMEHHHFDQCLMILNSPGNQILSGLSIEEYKTTLKIIKQAILATDLAL YIKRRGEFFELWRKNQFNLEDPHQKELFLAMLMTACDLSAITKPWPIQQRIAEL VATEFFDQGDRERKELNIEPTDLMNREKKNKIPSMQVGFIDAICLQLYEALTHVS EDCFPLLDGCRKNRQKWQALAEQQSLDGATNFSLLKQAGDVEENPGPLSKGEE DNMAIIKEFMRFKVHMEGSVNGHEFEIEGEGEGRPYEGTQTAKLKVTKGGPLPF AWDILSPQFMYGSKAYVKHPADIPDYLKLSFPEGFKWERVMNFEDGGVVTVTQ DSSLQDGEFIYKVKLRGTNFPSDGPVMQKKTMGWEASSERMYPEDGALKGEIK QRLKLKDGGHYDAEVKTTYKAKKPVQLPGAYNVNIKLDITSHNEDYTIVEQYE RAEGRHSTGGMDELYK* | 8811 | 8819 |
| OT-001870 (AcGFP (1-239 of WT); Linker (GS); hPDE5 (aa 535 to 860 of WT, H678F, S702F); SLDG Linker (SEQ ID NO: 8808); P2A cleavage site; mCherry (M1L); stop) | MVSKGAELFTGIVPILIELNGDVNGHKFSVSGEGEGDATYGKLTLKFICTTGKLP VPWPTLVTTLSYGVQCFSRYPDHMKQHDFFKSAMPEGYIQERTIFFEDDGNYKS RAEVKFEGDTLVNRIELTGTDFKEDGNILGNKMEYNYNAHNVYIMTDKAKNGI KVNFKIRHNIEDGSVQLADHYQQNTPIGDGPVLLPDNHYLSTQSALSKDPNEKR DHMIYFGFVTAAAITHGMDELYKGSEETRELQSLAAAVVPSAQTLKITDFSFSDF ELSDLETALCTIRMFTDLNLVQNFQMKHEVLCRWILSVKKNYRKNVAYHNWR HAFNTAQCMFAALKAGKIQNKLTDLEILALLIAALSHDLDHRGVNNSYIQRSEH PLAQLYCFSIMEHHHFDQCLMILNSPGNQILFGLSIEEYKTTLKIIKQAILATDLAL YIKRRGEFFELIRKNQFNLEDPHQKELFLAMLMTACDLSAITKPWPIQQRIAELV ATEFFDQGDRERKELNIEPTDLMNREKKNKIPSMQVGFIDAICLQLYEALTHVSE DCFPLLDGCRKNRQKWQALAEQQSLDGATNFSLLKQAGDVEENPGPLSKGEED NMAIIKEFMRFKVHMEGSVNGHEFEIEGEGEGRPYEGTQTAKLKVTKGGPLPFA WDILSPQFMYGSKAYVKHPADIPDYLKLSFPEGFKWERVMNFEDGGVVTVTQD SSLQDGEFIYKVKLRGTNFPSDGPVMQKKTMGWEASSERMYPEDGALKGEIKQ RLKLKDGGHYDAEVKTTYKAKKPVQLPGAYNVNIKLDITSHNEDYTIVEQYER AEGRHSTGGMDELYK* | 8812 | 8820 |

The position of hPDES in a construct comprising hPDE5 fused to GFP may affect the resultant library of mutants generated by mutagenesis screening. The effect of positioning the DD at the c terminal construct on the identification of DD clones was tested. PDES library generated by random mutagenesis fused GFP to generate AcGFP-PDE5-IRES-Puro constructs. Mutants were screened by appended DDs to payloads such as GFP and mcherry Table 14 provides PDE5 DDs generated in C terminal screens and their related constructs. In Table 14, * indicates the translation of the stop codon.

**Table 14: hPDE5 mutagenesis by C terminal positioning**

| **Construct ID/ Description** | **Sequence** | **AA SEQ ID NO** | **NA SEQ ID NO** |
|---|---|---|---|
| **DDs** | | | |
| hPDE5 (aa 535 to 860 of WT, E669G, I700T) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLEILALLIAALSHDLDHRGVNNSYIQRSGHPLAQLYCHSIMEH HHFDQCLMILNSPGNQTLSGLSIEEYKTTLKIIKQAILATDLALYIKRRGEF FELIRKNQFNLEDPHQKELFLAMLMTACDLSAITKPWPIQQRIAELVATEF FDQGDRERKELNIEPTDLMNREKKNKIPSMQVGFIDAICLQLYEALTHVS EDCFPLLDGCRKNRQKWQALAEQQ | 8821 | 8841 |
| hPDE5 (aa 535 to 860 of WT, hPDE5 (G632S, I648T) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAS KIQNKLTDLEILALLTAALSHDLDHRGVNNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTACDLSAITKPWPIQQRIAELVATEFF DQGDRERKELNIEPTDLMNREKKNKIPSMQVGFIDAICLQLYEALTHVSE DCFPLLDGCRKNRQKWQALAEQQ | 8822 | 8842 |
| hPDE5 (aa 535 to 774 of WT, hPDE5 (L646S) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLEILASLIAALSHDLDHRGVNNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTACDLSAITKPWPI | 8823 | 8843 |
| hPDE5 (aa 535 to 860 of WT, hPDE5 (A762V) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTVCDLSAITKPWPIQQRIAELVATEFF DQGDRERKELNIEPTDLMNREKKNKIPSMQVGFIDAICLQLYEALTHVSE DCFPLLDGCRKNRQKWQALAEQQ | 8824 | 8844 |
| hPDE5 (aa 535 to 860 of WT, hPDE5 (D640N) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTNLEILALLIAALSHDLDHRGVNNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTACDLSAITKPWPIQQRIAELVATEFF DQGDRERKELNIEPTDLMNREKKNKIPSMQVGFIDAICLQLYEALTHVSE DCFPLLDGCRKNRQKWQALAEQQ | 8825 | 8845 |
| hPDE5 (aa 535 to 860 of WT, hPDE5 (N636S) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQSKLTDLEILALLIAALSHDLDHRGVNNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTACDLSAITKPWPIQQRIAELVATEFF DQGDRERKELNIEPTDLMNREKKNKIPSMQVGFIDAICLQLYEALTHVSE DCFPLLDGCRKNRQKWQALAEQQ | 8826 | 8846 |
| hPDE5 (aa 535 to 860 of WT, hPDE5 (Q623R, D654G, K741N) | EETRELQSLAAAVVPSAQTLKITDFSFSDFELSDLETALCTIRMFTDLNLV QNFQMKHEVLCRWILSVKKNYRKNVAYHNWRHAFNTARCMFAALKAG KIQNKLTDLEILALLIAALSHGLDHRGVNNSYIQRSEHPLAQLYCHSIMEH HHFDQCLMILNSPGNQILSGLSIEEYKTTLKIIKQAILATDLALYIKRRGEFF ELIRNNQFNLEDPHQKELFLAMLMTACDLSAITKPWPIQQRIAELVATEFF DQGDRERKELNIEPTDLMNREKKNKIPSMQVGFIDAICLQLYEALTHVSE DCFPLLDGCRKNRQKWQALAEQQ | 8827 | 8847 |
| hPDE5 (aa 535 to 860 of WT, hPDE5 (A673T, L756V, C846Y) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLEILALLIAALSHDLDHRGVNNSYIQRSEHPLTQLYCHSIMEH HHFDQCLMILNSPGNQILSGLSIEEYKTTLKIIKQAILATDLALYIKRRGEFF ELIRKNQFNLEDPHQKELFVAMLMTACDLSAITKPWPIQQRIAELVATEFF DQGDRERKELNIEPTDLMNREKKNKIPSMQVGFIDAICLQLYEALTHVSE DCFPLLDGYRKNRQKWQALAEQQ | 8828 | 8848 |
| hPDE5 (aa 535 to 860 of WT, hPDE5 (V660A, L781F, R794G, C825R, E858G) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLEILALLIAALSHDLDHRGANNSYIQRSEHPLAQLYCHSIMEH HHFDQCLMILNSPGNQILSGLSIEEYKTTLKIIKQAILATDLALYIKRRGEFF ELIRKNQFNLEDPHQKELFLAMLMTACDLSAITKPWPIQQRIAEFVATEFF DQGDREGKELNIEPTDLMNREKKNKIPSMQVGFIDAIRLQLYEALTHVSE DCFPLLDGCRKNRQKWQALAGQQ | 8829 | 8849 |
| hPDE5 (aa 535 to 860 of WT, hPDE5 (E642G, G697D, I813T) | EETRELQSLAAAVVPSAQTLKITDFSFSDFELSDLETALCTIRMFTDLNLV QNFQMKHEVLCRWILSVKKNYRKNVAYHNWRHAFNTAQCMFAALKAG KIQNKLTDLGILALLIAALSHDLDHRGVNNSYIQRSEHPLAQLYCHSIMEH HHFDQCLMILNSPDNQILSGLSIEEYKTTLKIIKQAILATDLALYIKRRGEFF ELIRKNQFNLEDPHQKELFLAMLMTACDLSAITKPWPIQQRIAELVATEFF DQGDRERKELNIEPTDLMNREKKNKTPSMQVGFIDAICLQLYEALTHVSE DCFPLLDGCRKNRQKWQALAEQQ | 8830 | 8850 |

| **Components** | | | |
|---|---|---|---|
| AcGFP (1-239 of WT) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYK | 365 | 373 |
| Linker (GS); BamH1 Site | GS | - | GGAT CC |
| Linker (SR) | SR | - | TCTA GA |

| **Constructs** | | | |
|---|---|---|---|
| OT-001947 (AcGFP (1-239 of WT); Linker (GS); hPDE5 (aa 535 to 860 of WT, E669G, I700T); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LEILALLIAALSHDLDHRGVNNSYIQRSGHPLAQLYCHSIMEHHHFDQCL MILNSPGNQTLSGLSIEEYKTTLKIIKQAILATDLALYIKRRGEFFELIRKNQ FNLEDPHQKELFLAMLMTACDLSAITKPWPIQQRIAELVATEFFDQGDRE RKELNIEPTDLMNREKKNKIPSMQVGFIDAICLQLYEALTHVSEDCFPLLD GCRKNRQKWQALAEQQSR* | 8831 | 8851 |
| OT-001948 (AcGFP (1-239 of WT); Linker (GS); hPDE5 (aa 535 to 860 of WT, hPDE5 (G632S, I648T); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKASKIQNKLTDL EILALLTAALSHDLDHRGVNNSYIQRSEHPLAQLYCHSIMEHHHFDQCLM ILNSPGNQILSGLSIEEYKTTLKIIKQAILATDLALYIKRRGEFFELIRKNQFN LEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGC RKNRQKWQALAEQQSR* | 8832 | 8852 |
| OT-001945 (AcGFP (1-239 of WT); Linker (GS); hPDE5 (aa 535 to 860 of WT, hPDE5 (L646S); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LEILASLIAALSHDLDHRGVNNSYIQRSEHPLAQLYCHSIMEHHHFDQCL MILNSPGNQILSGLSIEEYKTTLKIIKQAILATDLALYIKRRGEFFELIRKNQ FNLEDPHQKELFLAMLMTACDLSAITKPWPISR* | 8833 | 8853 |
| OT-001944 (AcGFP (1-239 of WT); Linker (GS); hPDE5 (aa 535 to 860 of WT, A762V); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCL MILNSPGNQILSGLSIEEYKTTLKIIKQAILATDLALYIKRRGEFFELIRKNQ FNLEDPHQKELFLAMLMTVCDLSAITKPWPIQQRIAELVATEFFDQGDRE RKELNIEPTDLMNREKKNKIPSMQVGFIDAICLQLYEALTHVSEDCFPLLD GCRKNRQKWQALAEQQSR* | 8834 | 8854 |
| OT-001943 (AcGFP (1239 of WT); Linker (GS); hPDE5 (aa 535 to 860 of WT, hPDE5 (D640N); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTN LEILALLIAALSHDLDHRGVNNSYIQRSEHPLAQLYCHSIMEHHHFDQCL MILNSPGNQILSGLSIEEYKTTLKIIKQAILATDLALYIKRRGEFFELIRKNQ FNLEDPHQKELFLAMLMTACDLSAITKPWPIQQRIAELVATEFFDQGDRE RKELNIEPTDLMNREKKNKIPSMQVGFIDAICLQLYEALTHVSEDCFPLLD GCRKNRQKWQALAEQQSR* | 8835 | 8855 |
| OT-001941 (AcGFP (1-239 of WT); Linker (GS); hPDE5 (aa 535 to 860 of WT, N636S); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQSKLTDL EILALLIAALSHDLDHRGVNNSYIQRSEHPLAQLYCHSIMEHHHFDQCLMI LNSPGNQILSGLSIEEYKTTLKIIKQAILATDLALYIKRRGEFFELIRKNQFN LEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGC RKNRQKWQALAEQQSR* | 8836 | 8856 |
| OT-001942 (AcGFP (1-239 of WT); Linker (GS); hPDE5 (aa 535 to 860 of WT, Q623R, D654G, K741N); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTARCMFAALKAGKIQNKLTDL EILALLIAALSHGLDHRGVNNSYIQRSEHPLAQLYCHSIMEHHHFDQCLMI LNSPGNQILSGLSIEEYKTTLKIIKQAILATDLALYIKRRGEFFELIRNNQFN LEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGC RKNRQKWQALAEQQSR* | 8837 | 8857 |
| OT-001940 (AcGFP (1-239 of WT); Linker (GS); hPDE5 (aa 535 to 860 of WT, A673T, L756V, C846Y); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LEILALLIAALSHDLDHRGVNNSYIQRSEHPLTQLYCHSIMEHHHFDQCL MILNSPGNQILSGLSIEEYKTTLKIIKQAILATDLALYIKRRGEFFELIRKNQ FNLEDPHQKELFVAMLMTACDLSAITKPWPIQQRIAELVATEFFDQGDRE RKELNIEPTDLMNREKKNKIPSMQVGFIDAICLQLYEALTHVSEDCFPLLD GYRKNRQKWQALAEQQSR* | 8838 | 8858 |
| OT-001946 (AcGFP (1-239 of WT); Linker (GS); hPDE5 (aa 535 to 860 of WT, V660A, L781F, R794G, C825R, E858G); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LEILALLIAALSHDLDHRGANNSYIQRSEHPLAQLYCHSIMEHHHFDQCL MILNSPGNQILSGLSIEEYKTTLKIIKQAILATDLALYIKRRGEFFELIRKNQ FNLEDPHQKELFLAMLMTACDLSAITKPWPIQQRIAEFVATEFFDQGDRE GKELNIEPTDLMNREKKNKIPSMQVGFIDAIRLQLYEALTHVSEDCFPLLD GCRKNRQKWQALAGQQSR* | 8839 | 8859 |
| OT-001939 (AcGFP (1-239 of WT); Linker (GS); hPDE5 (aa 535 to 860 of WT, hPDE5 (E642G, G697D, I813T); Linker (SR); stop) | MVSKGAELFTGIVPILIELNGDVNGHKFSVSGEGEGDATYGKLTLKFICTT GKLPVPWPTLVTTLSYGVQCFSRYPDHMKQHDFFKSAMPEGYIQERTIFF EDDGNYKSRAEVKFEGDTLVNRIELTGTDFKEDGNILGNKMEYNYNAHN VYIMTDKAKNGIKVNFKIRHNIEDGSVQLADHYQQNTPIGDGPVLLPDNH YLSTQSALSKDPNEKRDHMIYFGFVTAAAITHGMDELYKGSEETRELQSL AAAVVPSAQTLKITDFSFSDFELSDLETALCTIRMFTDLNLVQNFQMKHE VLCRWILSVKKNYRKNVAYHNWRHAFNTAQCMFAALKAGKIQNKLTD LGILALLIAALSHDLDHRGVNNSYIQRSEHPLAQLYCHSIMEHHHFDQCL MILNSPDNQILSGLSIEEYKTTLKIIKQAILATDLALYIKRRGEFFELIRKNQ FNLEDPHQKELFLAMLMTACDLSAITKPWPIQQRIAELVATEFFDQGDRE RKELNIEPTDLMNREKKNKTPSMQVGFIDAICLQLYEALTHVSEDCFPLL DGCRKNRQKWQALAEQQSR* | 8840 | 8860 |

Additional DDs generated by random mutagenesis are provided in Table 15.

**Table 15: hPDE5 mutants**

| **Construct ID/ Description** | **Sequence** | **AA SEQ ID NO** | **NA SEQ ID NO** |
|---|---|---|---|
| hPDE5 AA 535-860 of WT (M758T) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLATLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8861 | 8891 |
| hPDE5 AA 535-860 of WT (K752E) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQE ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8862 | 8892 |
| hPDE5 AA 535-860 of WT (C677Y, H685R, A722V) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYYHSIMEHHRFDQCLMILNS PGNQILSGLSIEEYKTTLKIIKQAILVTDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8863 | 8893 |
| hPDE5 AA 535-860 of WT (T639S, M816R) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLSD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSRQVGFIDAICLQLYEALTHVSEDCFPLLDGCRKNRQKWQALAEQQ | 8864 | 8894 |
| hPDE5 AA 535-860 of WT | EEARELQSLAAAVVPSAQTLKITGFSFSDFELSDLETALCTIRMFTDLNLVQNFQ MKHEVLCRWILSVKKNYRKNVAYHNWRHAFNTAQCMFAALKAGKIQNKLTD | 8865 | 8895 |
| (T537A, D558G, 1706T, F744L, D764N) | LEILALLIAALSHDLDHRGVNNSYIQRSEHPLAQLYCHSIMEHHHFDQCLMILNS PGNQILSGLSTEEYKTTLKIIKQAILATDLALYIKRRGEFFELIRKNQLNLEDPHQK ELFLAMLMTACNLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | | |
| hPDE5 AA 535-860 of WT (Q586R, D724G) | EETRELQSLAAAVVPSAQTLKITDFSFSDFELSDLETALCTIRMFTDLNLVRNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATGLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8866 | 8896 |
| hPDE5 AA 535-860 of WT (F686S) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHSDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8867 | 8897 |
| hPDE5 AA 535-860 of WT (E539G, L7381) | EETRGLQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFEIIRKNQFNLEDPHQKE LFLAMLMTACDLSAITKPWPIQQRIAELVATEFFDQGDRERKELNIEPTDLMNRE KKNKIPSMQVGFIDAICLQLYEALTHVSEDCFPLLDGCRKNRQKWQALAEQQ | 8868 | 8898 |
| hPDE5 AA 535-860 of WT (Q635R, E753K, I813T) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIRNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK KLFLAMLMTACDLSAITKPWPIQQRIAELVATEFFDQGDRERKELNIEPTDLMNR EKKNKTPSMQVGFIDAICLQLYEALTHVSEDCFPLLDGCRKNRQKWQALAEQQ | 8869 | 8899 |
| hPDE5 AA 535-860 of WT (L672P, S836L) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPPAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVLEDCFPLLDGCRKNRQKWQALAEQQ | 8870 | 8900 |
| hPDE5 AA 535-860 of WT (M691T, D764N) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLTILNSP GNQILSGLSIEEYKTTLKIIKQAILATDLALYIKRRGEFFELIRKNQFNLEDPHQKE LFLAMLMTACNLSAITKPWPIQQRIAELVATEFFDQGDRERKELNIEPTDLMNRE KKNKIPSMQVGFIDAICLQLYEALTHVSEDCFPLLDGCRKNRQKWQALAEQQ | 8871 | 8901 |
| hPDE5 AA 535-860 of WT (R807G) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNG EKKNKIPSMQVGFIDAICLQLYEALTHVSEDCFPLLDGCRKNRQKWQALAEQQ | 8872 | 8902 |
| hPDE5 AA 535-860 of WT (R577Q, C596R, V660A, 1715V, E785K, L856P) | EETRELQSLAAAVVPSAQTLKITDFSFSDFELSDLETALCTIQMFTDLNLVQNFQ MKHEVLRRWILSVKKNYRKNVAYHNWRHAFNTAQCMFAALKAGKIQNKLTD LEILALLIAALSHDLDHRGANNSYIQRSEHPLAQLYCHSIMEHHHFDQCLMILNS PGNQILSGLSIEEYKTTLKVIKQAILATDLALYIKRRGEFFELIRKNQFNLEDPHQK ELFLAMLMTACDLSAITKPWPIQQRIAELVATKFFDQGDRERKELNIEPTDLMNR EKKNKIPSMQVGFIDAICLQLYEALTHVSEDCFPLLDGCRKNRQKWQAPAEQQ | 8873 | 8903 |
| hPDE5 AA 535-860 of WT (I720V, F820S) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAVLATDLALYIKRRGEFFELIRKNQFNLEDPHQK ELFLAMLMTACDLSAITKPWPIQQRIAELVATEFFDQGDRERKELNIEPTDLMNR EKKNKIPSMQVGSIDAICLQLYEALTHVSEDCFPLLDGCRKNRQKWQALAEQQ | 8874 | 8904 |
| hPDE5 AA 535-860 of WT | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD | 8875 | 8905 |
| (S695G, E707K, I739M, C763R) | LEILALLIAALSHDLDHRGVNNSYIQRSEHPLAQLYCHSIMEHHHFDQCLMILNG PGNQILSGLSIKEYKTTLKIIKQAILATDLALYIKRRGEFFELMRKNQFNLEDPHQ KELFLAMLMTARDLSAITKPWPIQQRIAELVATEFFDQGDRERKELNIEPTDLMN REKKNKIPSMQVGFIDAICLQLYEALTHVSEDCFPLLDGCRKNRQKWQALAEQQ | | |
| hPDE5 AA 535-860 of WT (Y709H, K812R, L832P) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEHKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNRIPSMQVGFIDAICLQLYEAPTHVSEDCFPLLDGCRKNRQKWQALAEQQ | 8876 | 8906 |
| hPDE5 AA 535-860 of WT (N583S, K752E, C846S) | EETRELQSLAAAVVPSAQTLKITDFSFSDFELSDLETALCTIRMFTDLSLVQNFQM KHEVLCRWILSVKKNYRKNVAYHNWRHAFNTAQCMFAALKAGKIQNKLTDLE ILALLIAALSHDLDHRGVNNSYIQRSEHPLAQLYCHSIMEHHHFDQCLMILNSPG NQILSGLSIEEYKTTLKIIKQAILATDLALYIKRRGEFFELIRKNQFNLEDPHQEELF LAMLMTACDLSAITKPWPIQQRIAELVATEFFDQGDRERKELNIEPTDLMNREK KNKIPSMQVGFIDAICLQLYEALTHVSEDCFPLLDGSRKNRQKWQALAEQQ | 8877 | 8907 |
| hPDE5 AA 535-860 of WT (E682G, D748N) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMGHHHFDQCLMILNS PGNQILSGLSIEEYKTTLKIIKQAILATDLALYIKRRGEFFELIRKNQFNLENPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8878 | 8908 |
| hPDE5 AA 535-860 of WT (K591R, I643T, L856P) | EETRELQSLAAAVVPSAQTLKITDFSFSDFELSDLETALCTIRMFTDLNLVQNFQ MRHEVLCRWILSVKKNYRKNVAYHNWRHAFNTAQCMFAALKAGKIQNKLTD LETLALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQAPAEQQ | 8879 | 8909 |
| hPDE5 AA 535-860 of WT (F619S, V818A, Y829C) | EETRELQSLAAAVVPSAQTLKITDFSFSDFELSDLETALCTIRMFTDLNLVQNFQ MKHEVLCRWILSVKKNYRKNVAYHNWRHASNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQAGFIDAICLQLCEALTHVSEDCFPLLDGCRKNRQKWQALAEQQ | 8880 | 8910 |
| hPDE5 AA 535-860 of WT (V548E, Q589L, K6331, M681T, S702I, K752E, L781P, A857T) | EETRELQSLAAAVEPSAQTLKITDFSFSDFELSDLETALCTIRMFTDLNLVQNFLM KHEVLCRWILSVKKNYRKNVAYHNWRHAFNTAQCMFAALKAGIIQNKLTDLEI LALLIAALSHDLDHRGVNNSYIQRSEHPLAQLYCHSITEHHHFDQCLMILNSPGN QILIGLSIEEYKTTLKIIKQAILATDLALYIKRRGEFFELIRKNQFNLEDPHQEELFL AMLMTACDLSAITKPWPIQQRIAEPVATEFFDQGDRERKELNIEPTDLMNREKK NKIPSMQVGFIDAICLQLYEALTHVSEDCFPLLDGCRKNRQKWQALTEQQ | 8881 | 8911 |
| hPDE5 AA 535-860 of WT (S652G, Q688R) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALGHDLDHRGVNNSYIQRSEHPLAQLYCHSIMEHHHFDRCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8882 | 8912 |
| hPDE5 AA 535-860 of WT (E565G) | EETRELQSLAAAVVPSAQTLKITDFSFSDFGLSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8883 | 8913 |
| hPDE5 AA 535-860 of WT (I774V) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPVQQRIAELVATEFFDQGDRERKELNIEPTDLMN REKKNKIPSMQVGFIDAICLQLYEALTHVSEDCFPLLDGCRKNRQKWQALAEQQ | 8884 | 8914 |
| hPDE5 AA 535-860 of WT (K591R) | EETRELQSLAAAVVPSAQTLKITDFSFSDFELSDLETALCTIRMFTDLNLVQNFQ MRHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRKNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8885 | 8915 |
| hPDE5 AA 535-860 of WT (F559S, Y709C, M760T) | EETRELQSLAAAVVPSAQTLKITDSSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEECKTTLKIIKQAILATDLALYIKRRGEFFELIRKNQFNLEDPHQK ELFLAMLTTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8886 | 8916 |
| hPDE5 AA 535-860 of WT (A649V, A650T, K730E, E830K) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIVTLSHDLDHRGVNNSYIQRSEHPLAQLYCHSIMEHHHFDQCLMILNSP GNQILSGLSIEEYKTTLKIIKQAILATDLALYIERRGEFFELIRKNQFNLEDPHQKE LFLAMLMTACDLSAITKPWPIQQRIAELVATEFFDQGDRERKELNIEPTDLMNRE KKNKIPSMQVGFIDAICLQLYKALTHVSEDCFPLLDGCRKNRQKWQALAEQQ | 8887 | 8917 |
| hPDE5 AA 535-860 of WT (Y728C, Q817R) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALCIKRRGEFFELIRKNQFNLEDPHQK ELFLAMLMTACDLSAITKPWPIQQRIAELVATEFFDQGDRERKELNIEPTDLMNR EKKNKIPSMRVGFIDAICLQLYEALTHVSEDCFPLLDGCRKNRQKWQALAEQQ | 8888 | 8918 |
| hPDE5 AA 535-860 of WT (L595P, K741R) | EETRELQSLAAAVVPSAQTLKITDFSFSDFELSDLETALCTIRMFTDLNLVQNFQ MKHEVPCRWILSVKKNYRKNVAYHNWRHAFNTAQCMFAALKAGKIQNKLTD LEILALLIAALSHDLDHRGVNNSYIQRSEHPLAQLYCHSIMEHHHFDQCLMILNS PGNQILSGLSIEEYKTTLKIIKQAILATDLALYIKRRGEFFELIRRNQFNLEDPHQK ELFLAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAEQQ | 8889 | 8919 |
| hPDE5 AA 535-860 of WT (R577W, W615R, M805T, I821V) | EETRELQSLAAAVVPSAQTLKITDFSFSDFELSDLETALCTIWMFTDLNLVQNFQ MKHEVLCRWILSVKKNYRKNVAYHNRRHAFNTAQCMFAALKAGKIQNKLTDL EILALLIAALSHDLDHRGVNNSYIQRSEHPLAQLYCHSIMEHHHFDQCLMILNSP GNQILSGLSIEEYKTTLKIIKQAILATDLALYIKRRGEFFELIRKNQFNLEDPHQKE LFLAMLMTACDLSAITKPWPIQQRIAELVATEFFDQGDRERKELNIEPTDLTNRE KKNKIPSMQVGFVDAICLQLYEALTHVSEDCFPLLDGCRKNRQKWQALAEQQ | 8890 | 8920 |

### Stimulus

Biocircuits of the disclosure are triggered by one or more stimuli. Stimuli may be selected from a ligand, an externally added or endogenous metabolite, the presence or absence of a defined ligand, pH, temperature, light, ionic strength, radioactivity, cellular location, subject site, microenvironment, the presence or the concentration of one or more metal ions.

In some instances, the stimulus is a ligand. Ligands may be nucleic acid-based, protein-based, lipid based, organic, inorganic or any combination of the foregoing. In some instances, the ligand is selected from the group consisting of a protein, peptide, nucleic acid, lipid, lipid derivative, sterol, steroid, metabolite derivative and a small molecule. In some instances, the stimulus is a small molecule. In some instances, the small molecules are cell permeable. Ligands useful in the present disclosure include without limitation, any of those taught in Table 2 of co-owned U.S. Provisional Patent Application No. 62/320,864 filed April 11, 2016, 62/466,596 filed March 3, 2017 and the International Publication WO2017/180587. In some instances, the small molecules are FDA-approved, safe and orally administered.

In some instances, the ligand binds to phosphodiesterases. In some instances, the ligand binds to and inhibits phosphodiesterase function and is herein referred to as a phosphodiesterase inhibitor.

In some instances, the ligand is a small molecule that binds to phosphodiesterase 5. In one instance, the small molecule is a hPDE5 inhibitor. Examples of hPDE5 inhibitors include, but are not limited to, Sildenafil, Vardenafil, Tadalafil, Avanafil, Lodenafil, Mirodenafil, Udenafil, Benzamidenafil, Dasantafil, Beminafil, SLx-2101, LAS 34179, UK-343,664, UK-357903, UK-371800, and BMS-341400.

In some instances, ligands include sildenafil-derived ligands containing portions of the ligand known to mediate binding to hPDE5. Ligands may also be modified to reduce off-target binding to Phosphodiesterases and increase specific binding to hPDE5.

hPDE5 inhibitors cover a broad pharmacokinetic space with respect to the approved dose and their duration of action and is described in Table 16. In Table 16, PO stands for *per os* (i.e. by mouth); QD represents *quaque die* (i.e. every day); IV represents intravenous; TID represents *ter un die* (i.e. three times a day); and Cmax represents the peak serum concentration that a drug achieves after its administration.

**Table 16: Pharmacokinetics of hPDE5 inhibitors**

| **Drug** | **Approved Dose** | **Cmax** | **Duration of action** |
|---|---|---|---|
| Sildenafil | PO: 100 mg QD or 20 mg TID IV: 10 mg TID | PO: 1 µM IV: 1 µM | 4-8 hrs |
| Vardenafil | PO: 20 mg QD | 0.1 µM | 2-8 hrs |
| Tadalafil | PO: 40 QD | 1.5 µM | 24-36 hrs |

In some instances, the ligand selection is determined by the magnitude and duration of expression of the effector modules of the disclosure using the PK parameters described in Table 16. In some instances, high levels of expression of the payload for a short duration of time may be desired. In such instances, vardenafil may be selected as the ligand. In some instances, high levels of expression of the payload may be desired for a long duration. In such instances, the ligand, Tadalafil may be selected. In some instances, low levels of expression of the payload may be desired for a long duration of time. In such instances, Sildenafil may be selected as the ligand.

In some instances, additional hPDE5 inhibitors may be developed to show selectivity towards a specific phosphodiesterase protein; to reduce or increase the duration of treatment with the ligand; and to improve the rate of onset of the ligand.

In one instance, the ligand may not be capable of suppressing the immune system i.e. the ligand may be non-immunosuppressive.

Ligands may also be selected from the analysis of the dependence of a known hPDE5 ligand's activity on its molecular/ chemical structure, through Structure Activity Relationships (SAR) study. Any of the methods related to SAR, known in art may be utilized to identify stabilizing ligands of the disclosure. SAR may be utilized to improve properties of the ligand such as specificity, potency, pharmacokinetics, bioavailability, and safety. SAR analysis of known hPDE5 inhibitors may also be combined with high resolution X ray structures of hPDE5 complexed with ligands. The X ray structure of hPDE5 co-crystallized with Sildenafil, Tadalafil, and Vardenafil have been studied and the binding mode of the inhibitors has been identified (Zhang, K. Y. J. et al. (2004) Mol. Cell., 15, 279; Card, G.L. et al. (2004) Structure. 12, 2233). There are several classes of hPDE5 inhibitors described. These include aryl, beryl, heteroaryl or heterobiaryl classes with different scaffold structures. The aryl class includes substituted nitroanilines and the biaryl class includes substituted naphthalenes. The heterobiaryl and heterotriaryl are further sub classified based on its fused system into pyrazolopyrimidinones, triazolopyrimidinones, imidazotriazines, purines, pyrrolopyrimidinones, triazolotrizinones, isoxazolopyrimidinones, β-carbolines, pyrroloquinolones, isoquinolines, quinazolines, imidazoquinazolinones, pyrazolopyridines, pyrazolopyridopyrimidinones. These widely different chemical structures are suggested to have different orientation in the binding site of hPDE5 enzyme. Sildenafil has three main chemical groups, the pyrazolopyrimidinone ring, the ethoxyphenyl ring and the methylpiperazine ring. The pyrazolopyrimidinone group is responsible for the binding of the drug to its active binding site of hPDE5.

Many hPDE inhibitors act by competing with the substrate, cGMP, for the catalytic site of the enzyme. Sildenafil and Vardenafil differ in the heterocyclic ring system used to mimic the purine ring of cGMP. They also differ in the substituent (ethyl/methyl) of a piperazine side chain. Although these are the only structural differences, Vardenafil is more potent than Sildenafil. In some instances, the structural differences between different know inhibitors of hPDE may be utilized to design better hPDE inhibitor based ligands. For example, Corbin et al. synthesized an analog of sildenafil that contained the sildenafil ring system but with the appended ethyl group found in vardenafil; and an analog of vardenafil (dimethyl-vardenafil) that contained the vardenafil ring system but with the appended methyl group found in sildenafil was also generated. These studies identified that the ring systems play a critical role in higher potency of vardenafil over sildenafil (Corbin JD et al. (2004) Neurochem Int ;45(6):859-63). Based on the X ray crystal structure of hPDE complexed with sildenafil, the active site region was defined as a sphere within the 6.5 Angstrom from the reference ligand, sildenafil. Surface hydrophobicity (lipophilicity) potential physicochemical property map of the hPDE5 active site may be generated based on this information. The ethoxyphenyl group of sildenafil fits into the hydrophobic pocket formed by Phe 786, Ala783, Leu804, and Val782, and the pyrazolopyrimidinone ring also forms hydrophobic interaction with the side chains of Val782, Tyr612, and Phe820 in the binding pocket.

New hPDE5 inhibitors may also be developed using a series of analogs of known hPDE5 inhibitors. 3D-Quantitative Structure activity analysis (QSAR), CoMFA (comparative molecular field analysis) and CoMSIA (comparative molecular similarity indices analysis) may be implemented for this purpose. In one instance, the structure of hPDE5 inhibitor may preferentially incorporate an N ethyl group in the pyrrolopyrimidinone ring at N5 position; and a propoxyphenyl group, which bear a substituent; one methylene unit longer, respectively in comparison with the corresponding positions of sildenafil. Any of the modifications to Sildenafil structure taught by Koo J et al. may be useful in the present disclosure (Koo J et al. (2007) Bioorg. & Med. Chem Lett 17; 4271-4274).

In some instances, the stimulus may be a ligand that binds to more than one phosphodiesterase. In one instance, the stimulus is a pan phosphodiesterase inhibitor that may bind to two or more hPDEs such as Aminophyline, Paraxanthine, Pentoxifylline, Theobromine, Dipyridamole, Theophyline, Zaprinast, Icariin, CDP-840, Etazolate and Glaucine.

In some instances, the ligand is a hPDE1 inhibitor. Exemplary hPDE1 inhibitor, Vinpocetine may in some instances be used as the ligand in the present disclosure. In some instances, the ligand is a hPDE2 inhibitor. Exemplary hPDE2 inhibitors include EHNA (erythro-9-(2-hydroxy-3-nonyl) adenine), Oxindole, PDP and BAY 60-7550. Inhibitors that selectively inhibit a hPDE2 isoform may also be used, for example, substituted pyrido (2,3-b) pyrazines having a hPDE2A selective inhibitory action (See, e.g., US Pat. NO.: 9,527,841). In some instances, the ligand is a hPDE3 inhibitor. hPDE3 inhibitors useful in the disclosure include, but are not limited to Amrinone, Cilostazol, Milrinone, Enoximone, and Pimobendan. In some instances, the ligand is a hPDE4 inhibitor. Exemplary hPDE4 inhibitors include FDA approved small molecules such as, but not limited to AN2728 (4-[(1-hydroxy-1,3-dihydro-2,1-benzoxaborol-5-yl) oxy] benzonitrile), Apremilast/ CC10004 (N-{2-[(1S)-1-(3-Ethoxy-4-methoxyphenyl)-2-(methylsulfonyl)ethyl]-1,3-dioxo-2,3-dihydro-1H-isoindol-4-yl} acetamide), and Roflumilast. Other exemplary hPDE4 inhibitors include Other small molecules that inhibit hPDE4 also include E6005/RVT501, Cilomilast/SB-207,499, Ibudilast (AV-411 or MN-166), Mesembrenone, Piclamilast / RP 73401, Rolipram, Atizoram / CP-80633, Arofylline, CC-1088, Catramilast, CGH-2466, Cipamfylline, Drotaverine, Filaminast/WAY-PDA 641, HT-0712, DNS-001, ICI-63197, Indimilast, Irsogladine/MN 1695, Lirimilast/ BAY 19-8004, Oglemilast, Revamilast, Ro 20-1724, Ronomilast, GSK256066, DC-TA-46, AWD 12-281 and YM-976. Any of the hPDE4 inhibitors described in International Patent Publication No. WO2014078220A1 and US Patent Publication No. US20170129887A1 may be useful in the present disclosure. In some instances, the ligand is a hPDE6 inhibitor. In some instances, the ligand is a hPDE7 inhibitor. Exemplary hPDE7 inhibitors, include BRL-50481(N, N,2-Trimethyl-5-nitrobenzenesulfonamide) and ASB16165 (1H-Thieno(2,3-C) pyrazole-5-carboxamide, 1-cyclohexyl-N-(6-(4-hydroxy-1 -piperidinyl)-3-pyridinyl)-3-methyl). In some instances, the ligand is a hPDE8 inhibitor such as PF-04957325 (Pfizer). In some instances, the ligand is a hPDE9 inhibitor. Exemplary hPDE9 inhibitors include, but are not limited to BAY73-6691 (1-(2-chlorophenyl)-6-[(2R)-3,3,3-trifluoro-2-methylpropyl]-1,5-dihydro-4H-pyrazolo[3,4-d] pyrimidine-4-one), PF-04447943 (6-[(3S,4S)-4-methyl-1-(pyrimidin-2-ylmethyl) pyrrolidin-3-yl]-1-(oxan-4-yl)-2H-pyrazolo[3,4-d] pyrimidin-4-one) and WYQ-C28L. In some instances, the ligand is a hPDE10 inhibitor. Exemplary PDE10 inhibitors include, but are not limited to OMS 824, Papaverine and PF-2545920 (2-(4-(1-methyl-4-pyridin-4-yl-1H-pyrazol-3-yl) phenoxymethyl) quinolone), and GS-5759 (Gilead).

In one instance, the stimuli of the present disclosure may be FDA approved ligands capable of binding to the specific DDs or target regions within the DDs. In other instances, FDA approved ligands may be used to screen potential binders in the human protein. DDs may be designed based on the positive hits from the screen using the portion of the protein that binds to the ligand. In one instance, proteins that bind to FDA approved ligands as off target interactions may be used to design DDs of the present disclosure.

In some instances, ligands that do not affect the activity of the immune cell, and/or the chimeric antigen receptor, in the absence of the SREs may be preferably selected.

### Stabilizing Domains

In some instances, the stimulus response element may be stabilized in the absence of the stimulus but destabilized by the stimulus. In some instances, SREs may be derived from protein complexes that comprise at least one protein- protein interaction. In other aspects, the SRE may form a protein-protein interaction with a natural protein within the cell. Protein complexes reduce the exposure of the constituent proteins to the risk of undesired oligomerization by reducing the concentration of the free monomeric state. Payloads appended to such SREs may stabilized in the absence of the stimulus. In some aspects, the stimulus may be a small molecule that is capable of interrupting or disrupting the protein-protein interactions related to the SRE. In such instances, addition of the stimulus, results in the reduced expression and/or function of the payload. In some instances, stimuli that induce conformational change of the SRE may be utilized. In one aspect, the SRE may be stabilized by the conformational change. In another aspect, the SRE may be destabilized by the conformational change. The stimuli may also be small molecules that disrupt post translational modification of SREs which may result in the disruption of the protein-protein interaction related to the SRE. In some instances, SREs may be identified using protein interatomic techniques known in the art. Such methods may enable the identification of protein interactions that are therapeutically relevant. Any of the large-scale quantitative proteomics methods described in International Patent Publication NOs. WO2017210427A1, WO2016196994A9, and WO2014200987A3 may be useful in the present disclosure.

### Molecular Switches in Biocircuits, Genetic Circuits

### Molecular Switches

In some instances, the DDs described herein can include aspects of molecular switches. Alternatively or additionally, molecular switches can be constructed using DDs as described. The term "molecular switch" as used herein refers to any molecule that can be reversibly shifted between two or more stable states in response to a stimulus (e.g., a ligand). Molecular switches can be employed in biocircuits or genetic circuits, i.e., engineered inputresponsive biological circuits.

In some instances, molecular switches can be RNA-based switches. As a non-limiting example, a DD-regulated effector module can be provided in an RNA molecule that comprises a coding sequence for a destabilization domain fused to a translational repressor such as, for example, L7Ae, a kink-turn (K-turn) motif in the 5' UTR of a payload mRNA, and the coding sequence for the payload (e.g., peptide or protein) (see, e.g., International Patent Publication NO:WO2016040395). L7Ae is an archaeal protein that binds K-turn and K-loop motifs with high affinity. In the absence of a ligand that stabilizes the destabilization domain, fusion L7Ae protein is degraded and the payload peptide or protein is expressed (ON state), whereas in the presence of the ligand, the fusion L7Ae protein is not destabilized and binds to the K-tum motif and represses expression of the payload peptide or protein (OFF state). Other translational regulation systems can also be used, such as, but not limited to, MS2-tethered repressors, Tet repressors, and microRNAs. Other uses of tunable expression repressors are contemplated; for example, effector modules comprising DD-regulated repressor payloads can be used to control inducible expression of transgenes, including to block background or "leaky" expression of transgenes (see US Patent No. 9,388,425).

DD-regulated effector modules as described herein can be utilized to establish tunable bistable genetic toggles, wherein ligand-mediated stabilization and/or destabilization of a SRE appended, for example, to a payload of interest stably shifts expression of a gene of interest between on and off states (see US Patent No. 6,841,376). Trans-acting SREs can also be utilized to tunably transition cis-acting self-repressive expression cassettes (see US Patent No. 10,208,312).

RNA-based molecular switches can be synthetic RNA. In some cases, such RNA molecules can comprise modified bases (modRNA) or self-replicating RNAs (replicons). RNA-based molecular switches can be responsive to small molecules, for example, small molecule-responsive RNA binding proteins (RBPs) (e.g., Wagner et al., Nat. Chem. Bio. 2018 14:1043-50). In this way, effector modules as described herein can be RBPs useful in regulating RNA circuits.

In some instances, molecular switches can be switched on/off via dimerization. For example, a first effector module can comprise a DD-regulated SRE which is a first member of a dimerization pair and optionally a first payload, and a second effector module can comprise a DD-regulated SRE which is second member of a dimerization pair and optionally a second payload, wherein the two members of the dimerization pair dimerize upon addition of a dimerization ligand. In some instances, dimerization can restore stability of the members of the dimerization pair and/or the attached payloads. Dimerization induces interaction, whether direct or indirect, of the two payloads to create a desired effect. In some instances, dimerization can induce degradation of the dimerization pair and/or the attached payloads. For example, bivalent small molecules can dimerize two molecules of an E3 ubiquitin ligase to induce self-degradation (see, e.g., Maniaci et al., Nat Commun. 2017 Oct 10;8(1):830). A dimerization pair can comprise, or be derived from, for example, an antibody and its antigen, two fragments of an antibody, a ligand-binding domain and a cognate receptor (e.g., any of those described in International Patent Publication NO: WO2017120546), and an E3 ubiquitin ligase and a substrate (e.g., as described in Maniaci et al.).

In some instances, molecular switches can be conditionally active in specific cell types or under specific cellular conditions. For example, the stimulus required to stimulate the DD-regulated SREs of the effector modules may only be present in a particular cell type or under a particular cellular condition. This allows various applications, such as cell-type specific delivery of a payload, or detection of a particular cellular target. Based on this property, biocircuits or effector modules described herein can be developed as biosensors. For example, a DD can be fused to a reporter protein (e.g., GFP). Such DD, in the context of an SRE, can contain mutations such that the SRE is conditionally stable only in the presence of a stabilizing ligand which is also the target to be detected. When the target is present in the cell, the fusion protein is stabilized and the reporter activity can be detected (see, e.g., International Patent Publication NOs: WO2009137136, WO2017048316, and WO2017156238; and US patent Nos: US 8,329,889, and 9,766,255). In some instances, genetic circuits may include a conditional NF-kB (nuclear factor kappa-B) responsive promoter driving expression of the effector modules described herein. Such circuits are described in the International Patent Publication WO2018170390.

In some instances, engineered cell circuits that enable multifactorial modulation within and/or near a tumor (a tumor microenvironment (TME)) may be utilized. Such circuits are described in the International Patent Publication WO2018191619.

In some instances, the biocircuits described herein may include SynNotch switches that include a synthetic notch (SynNotch) receptors. SynNotch receptors contain the core regulatory domain of the juxtacrine signaling receptor Notch, linked to a chimeric extracellular recognition domain (such as but not limited to an scFv and a chimeric intracellular transcriptional domain. Upon interacting with the cognate ligand which may be present on a neighboring cell, the SynNotch receptor undergoes cleavage of the transmembrane region, releasing the intracellular transcriptional domain to enter the nucleus and drive the expression of user-specified target genes. SynNotch circuits may be generated by linking one or more transcriptional outputs to SynNotch receptors. Any of the SynNotch circuits described by Toda et al. may be used in the biocircuits described herein (Programming selforganizing multicellular structures with synthetic cell-cell signaling Science 13 Jul 2018: Vol. 361, Issue 6398, pp. 156-162).

In some instances, the biocircuits described herein may include a proteolytic switch. Such switches may include a set of split proteases with highly specific orthogonal cleavage motifs which may be combined with cleavage sites and orthogonal coiled coils dimerizing domains strategically positioned in payloads of interest. Proteolytic cleavage induces conformational rearrangements within and between the CC modules, thus leading to the reconstitution of downstream functional protein domains resulting in a cellular outcome. Split-protease-cleavable orthogonal-CC-based (SPOC) logic circuits described by Fink et al. may be utilized in the biocircuits described herein (Design of fast proteolysis-based signaling and logic circuits in mammalian cells, Nat. Chem. Bio 15, 115-122 (2019)).

In one instance, the molecular switches may be based on chemically induced proximity (CIP) systems, wherein two proteins are colocalized upon addition of a bridging small molecule. In some aspects, the molecular switches may be based on chemically disrupted proximity (CDP) systems, which may include systems that allow the interaction of two basally colocalized proteins to be rapidly disrupted with a small. As non-limiting example, the interaction between the antiapoptotic protein BCL-xL and a BH3 peptide may be used as a chemically disrupted proximity system for intramolecularly controlling the activities of various enzymes. Intermolecular CDP systems that allow a basally localized activity to be chemically disrupted may be used as off switches. In one instance, the CDP system may be based on the hepatitis C virus protease (HCVp) NS3a and its interaction with a peptide inhibitor. Clinically approved protease inhibitors that efficiently disrupt the NS3a/peptide interaction may be utilized as bioorthogonal inputs for this system.

In some instances, Zapalog, a small molecule dimerizer that undergoes photolysis when exposed to blue light. Zapalog dimerizes any two proteins tagged with the FKBP and DHFR domains until exposure to light causes its photolysis. Dimerization can be repeatedly restored with uncleaved Zapalog. In some instances, molecular switches described herein may be heterodimerization switches. Heterodimerization may include chemical induced dimerization domains (CIDs) with two orthogonal ligand moieties that exhibit high specificity and affinity for their binding domains and lack alternative endogenous binding partners in mammalian cells at useful concentrations. The CIDs may be membrane permeable. In some aspects, the linker may be long enough to allow both binding domains to be simultaneously engaged without steric interference. The linker may in some instances be a photocleavable linker. Linkers that respond to a wavelength that is neither cytotoxic nor interferes with imaging common fluorophores may be preferentially selected. In some instances, the heterodimerization switches may include FKBP and DHFR domains and Zapalog, a small molecule dimerizer that undergoes photolysis when exposed to blue light. Zapalog may dimerize any two proteins tagged with the FKBP and DHFR domains until exposure to light causes its photolysis. Dimerization may be restored with uncleaved Zapalog. In some instances, Zapalog may include the heterodimerizer trimethoprim- synthetic ligand of FK506-binding protein (TMP-SLF), wherein the SLF portion binds the FK506-binding protein (FKBP) domain of one chimeric partner; the TMP portion binds the Escherichia coli dihydrofolate reductase (DHFR) domain of another. To render dimerization reversible with light, the alkyl linker between TMP and SLF may be replaced with a dialkoxy nitrobenzyl (DANB) moiety. Two payloads described herein are tagged with DHFR and FKBP domains. Addition of Zapalog may induce dimerization of the tagged proteins. Exposure to 405 nm light may photocleave Zapalog, causing rapid dissociation of the dimer. Dimerization may be restored by the addition of uncleaved Zapalog which may outcompete the photolysed Zapalog moieties. Any of the ligands described herein may be utilized to build Zapalog-like ligands and any of the SREs described herein may be used to generate CIDs.

### Boolean Switches

DD-regulated effector modules described herein can also be incorporated into the design of cellular Boolean switches. As used herein, a Boolean switch refers to a circuit that is designed to perform a logical operation based on one or more inputs and which produces an output. Logical operations performed by Boolean switches include but are not limited to, AND, OR, NOR, NAND, NOT, IMPLY, NIMPLY, XOR, and XNOR. AND and OR gates represent the most fundament logical operations, where OR represents a scenario where any of the one or more inputs is required to produce an output, and AND represents a scenario where all of the inputs are required to generate an output. Compound Boolean switches that consist of multiple logical operations can also be generated using effector modules as described herein. In some instances, biocircuits and/or any of their components can represent one or more inputs in a Boolean switch. In other instances, DDs described herein can be combined with switches known in the art to generate Boolean switches. The output of a Boolean switch can depend on the payload of the effector module utilized. In one instance, the DDs and/or the biocircuits can be utilized in NOT gates. NOT gates are inverters whose function is to invert the input i.e. stimulus. In some instances, the NOT gates described herein can be used to convert an immunosuppressive stimulus into an immune potentiating output such as the expression and/or function of an immune potentiating (promoting) payload. In another non-limiting example, an AND based Boolean switch may be generated where a first input comprises a biocircuit with gene editing nuclease, Cas9, as the payload and a second input comprises a biocircuit with transcriptional activator, VPR, as the payload. In the presence of the target gene guide RNA, addition of the stimuli to both inputs is required for the transcriptional activation of the target gene (see Gao Y et al. (2016) Nat Methods.13(12): 1043-1049).

### Safety Switches

In some instances, effector module payloads can comprise DD-regulated safety switches that can eliminate adoptively transferred cells in the case of severe toxicity, thereby mitigating the adverse effects of T cell therapy. Adoptively transferred T cells in immunotherapy can attack normal cells in response to normal tissue expression of TAA. Even on-tumor target activity of adoptively transferred T cells can result in toxicities such as tumor lysis syndrome, cytokine release syndrome and the related macrophage activation syndrome. Safety switches can be utilized to eliminate inappropriately activated adoptively transferred cells by induction of apoptosis or by immunosurveillance.

In some instances, DD-regulated payloads can comprise inducible killer/suicide genes that act as a safety switch. The killer/suicide gene when introduced into adoptively transferred immune cells, could control their alloreactivity. The killer/suicide gene can be an apoptotic gene (e.g., any Caspase gene) which allows conditional apoptosis of the transduced cells by administration of a non-therapeutic ligand of the SRE (e.g., DD).

In some instances, the payload can be Caspase 9. In some instances, Caspase 9 can be modified to have low basal expression and lacking the caspase recruitment domain (CARD) (SEQ ID NO. 26 and SEQ ID NO.28 of US Patent No. US9434935B2). In some instances, the safety switches may also be referred to as genetic erasers. In one aspect, the genetic eraser may include a selection marker such as those described in International Patent Publication, WO2018022747.

In some instances, DDs, SREs and/or payloads of effector modules as described can include molecular safeguards for T-cell immunotherapies. Molecular safeguards can allow, in the event of side-effects, selective depletion of engineered T-cells (e.g., CAR T-cells). Molecular safeguards can respond to activating agents which trigger on-demand depletion of the engineered T-cells. Non-limiting examples of molecular safeguards to be used in combination with CAR expression include CD20, which respond to the antibody Rituximab; RQR8, which responds to the antibody Rituximab; huEGFRtr, which responds to the antibody Erbitux; HSV-TK, which responds to the small molecule Ganciclovir; ICasp9, which responds to the small molecule Rimudicid; and CubiCAR, which is a CAR architecture with built-in safeguard CD20 that responds to the antibody Rituximab (Valton et al., Scientific Reports volume 8, Article number: 8972 (2018)).

In one instance, the payload is a suicide gene system, iCasp9/Chemical induced dimerization (CID) system which consists of a polypeptide derived from the Caspase9 gene fused to a drug binding domain derived from the human FK506 protein. Administration of bioinert, small molecule AP1903(rimiducid), induces cross linking of the drug binding domains and dimerization of the fusion protein and in turn the dimerization of Caspase 9. This results in the activation of downstream effector Caspase 3 and subsequent induction of cellular apoptosis (Straathof et al., Blood, 2005, 105: 4247-4254). Preclinical trials using CART including an iCasp9 gene have shown effective elimination of CAR T cells in vivo in mouse models and demonstrate the potential efficacy of this approach. (Budde et al, Plos One, 2013, 8: e82742.10.1371; Hoyos et al., Leukemia, 2010; 24(6):1160-1170). In one instance, the payload can comprise Caspase 9. In one aspect, the effector module can be a DD-Caspase9 fusion polypeptide.

### Regulatory Switches

The utility of adoptive cell therapy (ACT) has been limited by the high incidence of graft versus host disease (GVHD). GVHD occurs when adoptively transferred T cells elicit an immune response resulting in host tissue damage. Recognition of host antigens by the graft cells triggers a proinflammatory cytokine storm cascade that signifies acute GVHD. GVHD is characterized as an imbalance between the effector and the regulatory arms of the immune system. In some instances, the payloads described herein can be used as regulatory switches. As used herein "regulatory switch" refers proteins, which when expressed in target cells increase tolerance to the graft by enhancing the regulatory arm of the immune system.

In one instance, regulatory switches can include DD-regulated payloads that preferentially promote the expansion of regulatory T (Treg cells). Tregs are a distinct population of cells that are positively selected on high affinity ligands in the thymus and play an important role in the tolerance to self-antigens. In addition, T regs have also been shown to play a role in peripheral tolerance to foreign antigens. Since Tregs promote immune tolerance, expansion of Tregs with compositions as described herein can be desirable to limit GVHD.

In some instances, the regulatory switch can include, but is not limited to T regs activation factors such NF-kappa B (NFKB or NEκB) FOXO, nuclear receptor Nr4a, Retinoic acid receptor alpha, NFAT, AP-1 and SMAD. Such factors can result in the expression of Fork headbox P3 (FOXP3) in T cells resulting in the activation of the regulatory T cell program and the expansion of T cells.

In some instances, the regulatory switch can be FOXP3, a transcriptional regulator in T cells. A function of FOXP3 is to suppress the function of NFAT, which leads to the suppression of expression of many genes including IL2 and effector T-cell cytokines. FOXP3 acts also as a transcription activator for genes such as CD2S, Cytotoxic T-Lymphocyte Antigen Cytotoxic T-Lymphocyte Antigen 4 (CTLA4), glucocorticoid-induced TNF receptor family gene (GITR) and folate receptor 4. FOXP3 also inhibits the differentiation of IL17 producing helper T-cells (Th17) by antagonizing RORC (RAR related orphan receptor C). Isoforms of FOXP3 lacking exon2 (FOXP3 delta 2) or exon 7 (FOXP3 delta 7) can also be used as regulatory switches. In one aspect, an effector module as described herein can be a DD-FOXP3 fusion polypeptide.

Regulatory switches can be designed using Boolean switches, e.g. NOT gates. For example, an immunosuppressive signal can be converted into an immune potentiating output such as expression and/or function of an immune potentiating (promoting) payload. The stimulus of a NOT gate can be, for example, phosphorylated SHP2, a signaling molecule produced by the activation of the immunosuppressive PD1 signaling pathway. Another non-limiting example for a NOT gate can be metabolite kynurenine, which is produced by the indoleamine 2,3-dioxygenase pathway.

### Epigenetic switches

In some instances, the DDs described herein can include epigenetic switches. Alternatively or additionally, epigenetic switches can be constructed using DDs described herein. Epigenetic switches utilize chemical modification of nucleic acids, the propagation, and the actuation of the chemical modifications. In some instances, the epigenetic switches may be built by utilizing chemical modifications of nucleic acids that are naturally found in bacterial such as 6-methyl-adenosine (m6A). m6A switches are described in Park et al. 2019 (Engineering Epigenetic Regulation Using Synthetic Read-Write Modules. Cell. Jan 10;176(1-2):227-238). The epigenetic circuits described herein may include a "writer" protein that adds m6A at specific DNA sequences. The writer module may be based on a methylase such as but not limited to dam methylase, which adds m6A to GATC sequences in Escherichia coli and is active when expressed in human cells. By fusing dam to a zinc finger proteins (ZF), allows targeting m6A methylation to specific loci. Specificity of the dam methylases to ZF targeted sequences may be achieved using by engineering mutations that reduce the catalytic activity of dam. In some instances, the epigenetic switch may include a "reader" protein that can recognize the m6A methylation marks. In one aspect, the reader domain may be the binding domain of the restriction enzyme DpnI. This reader domain (RD) may be fused to DAM to propagate m6A marks at nearby and complementary GATC sites in DNA, thereby spreading methylation across a locus and cell divisions. The RD may also be fused to proteins that activate or repress transcription (EDs), thus creating m6A-dependent transcriptional regulation.

### Payloads

In some instances, payloads can be any natural protein in an organism genome, a fusion polypeptide, an antibody, or variants, mutants and derivatives thereof. In some instances, the effector module of the present disclosure is a fusion construct comprising a DD of the disclosure operably linked to at least one payload. In one aspect, the payload may be any natural protein of interest (POI) or variants thereof, an antibody or fragments thereof, a therapeutic agent, or any artificial peptide or polypeptide.

In some instances, payloads of the present disclosure may be immunotherapeutic agents. As used herein, an immunotherapeutic agent is any agent that induces immune responses in an organism. An immunotherapeutic agent may be a natural protein in an organism or it may be an artificial protein such as a fusion protein or an antibody. The immunotherapeutic agent may be, but is not limited to, an antibody and fragments and variants thereof, a MHC molecule, an antigen and fragments thereof, a T cell receptor (TCR) such as a tumor specific TCR and variants thereof, a chimeric antigen receptor (CAR), a chimeric switch receptor, a co-stimulatory molecule, a co-inhibitory molecule, an inhibitor of a co-inhibitory receptor or ligand, an agonist of a co-stimulatory receptor and ligand, a cytokine, chemokine, a cytokine receptor, a chemokine receptor, a soluble growth factor, a metabolic factor, a homing receptor, a safety switch (e.g., a suicide gene), or any agent that induces an immune response. In one instance, the immunotherapeutic agent induces an anti-cancer immune response in a cell, or in a subject. In some aspects, the immunotherapeutic agent reduces the tumor burden in a subject.

The payload may be any immunotherapeutic agent used for cancer immunotherapy such as a T cell receptor (TCR), a chimeric agent receptor (CAR) such as CD 19 CAR that targets any molecule of tumor cells, an antibody, an antigen binding domain or combination of antigen binding domains, a cytokine such as IL2, IL12, IL15 or IL15/IL15Ra fusion, an antagonist of an immune checkpoint, an agonist of co-stimulatory molecule, a chimeric switch receptor, a safety switch, a metabolic factor, a growth factor, a chemokine, a chemokine receptor, a homing receptor, or any agent that can induce an immune response. The SRE and payload may be operably linked through one or more linkers and the positions of components may vary within the effector module.

In some instances, the payload may be a fusion protein comprising any of the immunotherapeutic agents described and ubiquitin. Within the fusion protein, the ubiquitin may be positioned at the N terminus and the immunotherapeutic agent may be positioned at the C terminus. In one aspect, the immunotherapeutic agent may itself be a fusion protein and the ubiquitin may be located in between the proteins that are fused. The payloads may include a single ubiquitin protein or a chain of ubiquitin proteins. The ubiquitin protein may be linked to the immunotherapeutic agent through a single amino acid. The selection of the single amino acid may depend on the desired half-life of the fusion protein. In one instance, the immunotherapeutic agent may be IL12.

### 1. Protein of interest

In some instances, payloads of the disclosure may be a natural protein in an organism genome, or variants, mutants, derivatives thereof. The natural protein may be from, for example, a mammalian organism, a bacterium, and a virus.

In one example, the payload may be a protein of interest, or a polypeptide from human genome.

### 2. Antibodies

In some instances, antibodies, fragments and variants thereof are payloads of the present disclosure. The antibody may be an intact antibody, an antibody light chain, antibody heavy chain, an antibody fragment, an antibody variant, or an antibody derivative.

In some instances, payloads of the disclosure may be an antibody or fragments thereof. Antibodies useful in this method include without limitation, any of those taught in co-owned U.S. Provisional Patent Application No. 62/320,864 filed April 11, 2016, 62/466,596 filed March 3, 2017 and the International Publication WO2017/180587.

### Antibody fragments and variants

In some instances, antibody fragments and variants may comprise antigen binding regions from intact antibodies. Examples of antibody fragments and variants may include, but are not limited to Fab, Fab', F(ab')2, and Fv fragments; diabodies; linear antibodies; single-chain antibody molecules such as single chain variable fragment (scFv); and multispecific antibodies formed from antibody fragments. Papain digestion of antibodies produces two identical antigen-binding fragments, called "Fab" fragments, each with a single antigen-binding site. Also produced is a residual "Fc" fragment, whose name reflects its ability to crystallize readily. Pepsin treatment yields an F(ab')2 fragment that has two antigen-binding sites and is still capable of cross-linking with the antigen. Pharmaceutical compositions, biocircuits, biocircuit components, effector modules including their SREs or payloads of the present disclosure may comprise one or more of these fragments.

For the purposes herein, an "antibody" may comprise a heavy and light variable domain as well as an Fc region. As used herein, the term "native antibody" usually refers to a heterotetrametric glycoprotein of about 150,000 Daltons, composed of two identical light (L) chains and two identical heavy (H) chains. Genes encoding antibody heavy and light chains are known and segments making up each have been well characterized and described (Matsuda et al., The Journal of Experimental Medicine. 1998, 188(11): 2151-62 and Li et al., Blood, 2004, 103(12): 4602-4609). Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain.

As used herein, the term "variable domain" refers to specific antibody domains found on both the antibody heavy and light chains that differ extensively in sequence among antibodies and are used in the binding and specificity of each particular antibody for its particular antigen. Variable domains comprise hypervariable regions. As used herein, the term "hypervariable region" refers to a region within a variable domain comprising amino acid residues responsible for antigen binding. The amino acids present within the hypervariable regions determine the structure of the complementarity determining regions (CDRs) that become part of the antigen-binding site of the antibody. As used herein, the term "CDR" refers to a region of an antibody comprising a structure that is complimentary to its target antigen or epitope. Other portions of the variable domain, not interacting with the antigen, are referred to as framework (FW) regions. The antigen-binding site (also known as the antigen combining site or paratope) comprises the amino acid residues necessary to interact with a particular antigen. The exact residues making up the antigen-binding site are typically elucidated by co-crystallography with bound antigen, however computational assessments based on comparisons with other antibodies can also be used (Strohl, W.R. Therapeutic Antibody Engineering. Woodhead Publishing, Philadelphia PA. 2012. Ch. 3, p47-54). Determining residues that make up CDRs may include the use of numbering schemes including, but not limited to, those taught by Kabat (Wu et al., JEM, 1970, 132(2):211-250 and Johnson et al., Nucleic Acids Res. 2000, 28(1): 214-218), Chothia (Chothia and Lesk, J. Mol. Biol. 1987, 196,901, Chothia et al., Nature, 1989, 342, 877, and Al-Lazikani et al., J. Mol. Biol. 1997, 273(4): 927-948), Lefranc (Lefranc et al., Immunome Res. 2005, 1:3) and Honegger (Honegger and Pluckthun, J. Mol. Biol. 2001, 309(3): 657-70).

VH and VL domains have three CDRs each. VL CDRs are referred to herein as CDR-L1, CDR-L2 and CDR-L3, in order of occurrence when moving from N- to C- terminus along the variable domain polypeptide. VH CDRs are referred to herein as CDR-H1, CDR-H2 and CDR-H3, in order of occurrence when moving from N- to C-terminus along the variable domain polypeptide. Each of CDRs has favored canonical structures with the exception of the CDR-H3, which comprises amino acid sequences that may be highly variable in sequence and length between antibodies resulting in a variety of three-dimensional structures in antigen-binding domains (Nikoloudis, et al., PeerJ. 2014, 2: e456). In some cases, CDR-H3s may be analyzed among a panel of related antibodies to assess antibody diversity. Various methods of determining CDR sequences are known in the art and may be applied to known antibody sequences (Strohl, W.R. Therapeutic Antibody Engineering. Woodhead Publishing, Philadelphia PA. 2012. Ch. 3, p47-54).

As used herein, the term "Fv" refers to an antibody fragment comprising the minimum fragment on an antibody needed to form a complete antigen-binding site. These regions consist of a dimer of one heavy chain and one light chain variable domain in tight, non-covalent association. Fv fragments can be generated by proteolytic cleavage, but are largely unstable. Recombinant methods are known in the art for generating stable Fv fragments, typically through insertion of a flexible linker between the light chain variable domain and the heavy chain variable domain (to form a single chain Fv (scFv)) or through the introduction of a disulfide bridge between heavy and light chain variable domains (Strohl, W.R. Therapeutic Antibody Engineering. Woodhead Publishing, Philadelphia PA. 2012. Ch. 3, p46-47).

As used herein, the term "light chain" refers to a component of an antibody from any vertebrate species assigned to one of two clearly distinct types, called kappa and lambda based on amino acid sequences of constant domains. Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2.

As used herein, the term "single chain Fv" or "scFv" refers to a fusion protein of VH and VL antibody domains, wherein these domains are linked together into a single polypeptide chain by a flexible peptide linker. In some instances, the Fv polypeptide linker enables the scFv to form the desired structure for antigen binding. In some instances, scFvs are utilized in conjunction with phage display, yeast display or other display methods where they may be expressed in association with a surface member (e.g. phage coat protein) and used in the identification of high affinity peptides for a given antigen.

Using molecular genetics, two scFvs can be engineered in tandem into a single polypeptide, separated by a linker domain, called a "tandem scFv" (tascFv). Construction of a tascFv with genes for two different scFvs yields a "bispecific single-chain variable fragments" (bis-scFvs). Only two tascFvs have been developed clinically by commercial firms; both are bispecific agents in active early phase development by Micromet for oncologic indications, and are described as "Bispecific T-cell Engagers (BiTE)." Blinatumomab is an anti-CD19/anti-CD3 bispecific tascFv that potentiates T-cell responses to B-cell non-Hodgkin lymphoma in Phase 2. MT110 is an anti-EP-CAM/anti-CD3 bispecific tascFv that potentiates T-cell responses to solid tumors in Phase 1. Bispecific, tetravalent "TandAbs" are also being researched by Affimed (Nelson, A. L., MAbs., 2010, Jan-Feb; 2(1):77-83). Maxibodies (bivalent scFv fused to the amino terminus of the Fc (CH2-CH3 domains) of IgG may also be included.

As used herein, the term "bispecific antibody" refers to an antibody capable of binding two different antigens. Such antibodies typically comprise regions from at least two different antibodies. Bispecific antibodies may include any of those described in Riethmuller, G. Cancer Immunity. 2012, 12:12-18, Marvin et al., 2005. Acta Pharmacologica Sinica. 2005, 26(6): 649-658 and Schaefer et al., PNAS. 2011, 108(27):11187-11192. In some aspects, bispecific antibodies may be trifunctional antibodies (3funct) and BiTE (bi-specific T cell engager).

As used herein, the term "diabody" refers to a small antibody fragment with two antigen-binding sites. Diabodies are functional bispecific single-chain antibodies (bscAb). Diabodies comprise a heavy chain variable domain VH connected to a light chain variable domain VL in the same polypeptide chain. By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al. (Hollinger, P. et al., "Diabodies": Small bivalent and bispecific antibody fragments. PNAS, 1993. 90: 6444-6448).

The term "intrabody" refers to a form of antibody that is not secreted from a cell in which it is produced, but instead targets one or more intracellular proteins. Intrabodies may be used to affect a multitude of cellular processes including, but not limited to intracellular trafficking, transcription, translation, metabolic processes, proliferative signaling and cell division. In some instances, methods of the present disclosure may include intrabody-based therapies. In some such instances, variable domain sequences and/or CDR sequences disclosed herein may be incorporated into one or more constructs for intrabody-based therapy.

As used herein, the term "monoclonal antibody" refers to an antibody obtained from a population of substantially homogeneous cells (or clones), i.e., the individual antibodies comprising the population are identical and/or bind the same epitope, except for possible variants that may arise during production of the monoclonal antibodies, such variants generally being present in minor amounts. In contrast to polyclonal antibody preparations that typically include different antibodies directed against different determinants (epitopes), each monoclonal antibody is directed against a single determinant on the antigen.

The modifier "monoclonal" indicates the character of the antibody as being obtained from a substantially homogeneous population of antibodies, and is not to be construed as requiring production of the antibody by any particular method. The monoclonal antibodies herein include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies.

As used herein, the term "humanized antibody" refers to a chimeric antibody comprising a minimal portion from one or more non-human (e.g., murine) antibody source(s) with the remainder derived from one or more human immunoglobulin sources. For the most part, humanized antibodies are human immunoglobulins (recipient antibody) in which residues from the hypervariable region from an antibody of the recipient are replaced by residues from the hypervariable region from an antibody of a non-human species (donor antibody) such as mouse, rat, rabbit or nonhuman primate having the desired specificity, affinity, and/or capacity. In one instance, the antibody may be a humanized full-length antibody. As a non-limiting example, the antibody may have been humanized using the methods taught in US Patent Publication NO. US20130303399.

As used herein, the term "antibody variant" refers to a modified antibody (in relation to a native or starting antibody) or a biomolecule resembling a native or starting antibody in structure and/or function (e.g., an antibody mimetic). Antibody variants may be altered in their amino acid sequence, composition or structure as compared to a native antibody. Antibody variants may include, but are not limited to, antibodies with altered isotypes (e.g., IgA, IgD, IgE, IgG1, IgG2, IgG3, IgG4, or IgM), humanized variants, optimized variants, multispecific antibody variants (e.g., bispecific variants), and antibody fragments.

In some instances, pharmaceutical compositions, biocircuits, biocircuit components, effector modules including their SREs or payloads of the present disclosure may be antibody mimetics. As used herein, the term "antibody mimetic" refers to any molecule which mimics the function or effect of an antibody and which binds specifically and with high affinity to their molecular targets. In some instances, antibody mimetics may be monobodies, designed to incorporate the fibronectin type III domain (Fn3) as a protein scaffold (US 6,673,901; US 6,348,584). In some instances, antibody mimetics may be those known in the art including, but are not limited to affibody molecules, affilins, affitins, anticalins, avimers, Centyrins, DARPINSTM, Fynomers and Kunitz and domain peptides. In other instances, antibody mimetics may include one or more non-peptide regions.

In one instance, the antibody may comprise a modified Fc region. As a non-limiting example, the modified Fc region may be made by the methods or may be any of the regions described in US Patent Publication NO. US20150065690.

In some instances, payloads of the disclosure may encode multispecific antibodies that bind more than one epitope. As used herein, the terms "multibody" or "multispecific antibody" refer to an antibody wherein two or more variable regions bind to different epitopes. The epitopes may be on the same or different targets. In one instance, the multispecific antibody may be generated and optimized by the methods described in International Patent Publication NO. WO2011109726 and US Patent Publication NO. US20150252119. These antibodies are able to bind to multiple antigens with high specificity and high affinity.

In certain instances, a multi-specific antibody is a "bispecific antibody" which recognizes two different epitopes on the same or different antigens. In one aspect, bispecific antibodies are capable of binding two different antigens. Such antibodies typically comprise antigen-binding regions from at least two different antibodies. For example, a bispecific monoclonal antibody (BsMAb, BsAb) is an artificial protein composed of fragments of two different monoclonal antibodies, thus allowing the BsAb to bind to two different types of antigen. Bispecific antibody frameworks may include any of those described in Riethmuller, G., 2012. Cancer Immunity, 2012,12:12-18; Marvin et al., Acta Pharmacologica Sinica. 2005, 26(6):649-658; and Schaefer et al., PNAS. 2011, 108(27): 11187-11192. New generations of BsMAb, called "trifunctional bispecific" antibodies, have been developed. These consist of two heavy and two light chains, one each from two different antibodies, where the two Fab regions (the arms) are directed against two antigens, and the Fc region (the foot) comprises the two heavy chains and forms the third binding site.

In some instances, payloads may encode antibodies comprising a single antigen-binding domain. These molecules are extremely small, with molecular weights approximately one-tenth of those observed for full-sized mAbs. Further antibodies may include "nanobodies" derived from the antigen-binding variable heavy chain regions (VHHs) of heavy chain antibodies found in camels and llamas, which lack light chains (Nelson, A. L., MAbs.2010. Jan-Feb; 2(1):77-83).

In some instances, the antibody may be "miniaturized". Among the best examples of mAb miniaturization are the small modular immunopharmaceuticals (SMIPs) from Trubion Pharmaceuticals. These molecules, which can be monovalent or bivalent, are recombinant single-chain molecules containing one VL, one VH antigen-binding domain, and one or two constant "effector" domains, all connected by linker domains. Presumably, such a molecule might offer the advantages of increased tissue or tumor penetration claimed by fragments while retaining the immune effector functions conferred by constant domains. At least three "miniaturized" SMIPs have entered clinical development. TRU-015, an anti-CD20 SMIP developed in collaboration with Wyeth, is the most advanced project, having progressed to Phase 2 for rheumatoid arthritis (RA). Earlier attempts in systemic lupus erythrematosus (SLE) and B cell lymphomas were ultimately discontinued. Trubion and Facet Biotechnology are collaborating in the development of TRU-016, an anti-CD37 SMIP, for the treatment of CLL and other lymphoid neoplasias, a project that has reached Phase 2. Wyeth has licensed the anti-CD20 SMIP SBI-087 for the treatment of autoimmune diseases, including RA, SLE and possibly multiple sclerosis, although these projects remain in the earliest stages of clinical testing. (Nelson, A. L., MAbs, 2010. Jan-Feb; 2(1):77-83).

On example of miniaturized antibodies is called "unibody" in which the hinge region has been removed from IgG4 molecules. While IgG4 molecules are unstable and can exchange light-heavy chain heterodimers with one another, deletion of the hinge region prevents heavy chain-heavy chain pairing entirely, leaving highly specific monovalent light/heavy heterodimers, while retaining the Fc region to ensure stability and half-life in vivo. This configuration may minimize the risk of immune activation or oncogenic growth, as IgG4 interacts poorly with FcRs and monovalent unibodies fail to promote intracellular signaling complex formation (see, e.g., Nelson, A. L., MAbs, 2010. Jan-Feb; 2(1):77-83).

In some instances, payloads of the disclosure may encode single-domain antibodies (sdAbs, or nanobodies) which are antibody fragment consisting of a single monomeric variable antibody domain. Like a whole antibody, it is able to bind selectively to a specific antigen. In one aspect, a sdAb may be a "Camel Ig or "camelid VHH". As used herein, the term "camel Ig" refers to the smallest known antigen-binding unit of a heavy chain antibody (Koch-No lte, et al, FASEB J., 2007, 21: 3490- 3498). A "heavy chain antibody" or a "camelid antibody" refers to an antibody that contains two VH domains and no light chains (Riechmann L. et al, J. Immunol. Methods, 1999, 231: 25-38; International patent publication NOs.: WO1994/04678 and WO1994/025591; and U.S. Patent No. 6,005,079). In another aspect, a sdAb may be a "immunoglobulin new antigen receptor" (IgNAR). As used herein, the term "immunoglobulin new antigen receptor" refers to class of antibodies from the shark immune repertoire that consist of homodimers of one variable new antigen receptor (VNAR) domain and five constant new antigen receptor (CNAR) domains. IgNARs represent some of the smallest known immunoglobulin-based protein scaffolds and are highly stable and possess efficient binding characteristics. The inherent stability can be attributed to both (i) the underlying Ig scaffold, which presents a considerable number of charged and hydrophilic surface exposed residues compared to the conventional antibody VH and VL domains found in murine antibodies; and (ii) stabilizing structural features in the complementary determining region (CDR) loops including inter-loop disulfide bridges, and patterns of intra-loop hydrogen bonds.

In some instances, payloads of the disclosure may encode intrabodies. Intrabodies are a form of antibody that is not secreted from a cell in which it is produced, but instead targets one or more intracellular proteins. Intrabodies are expressed and function intracellularly, and may be used to affect a multitude of cellular processes including, but not limited to intracellular trafficking, transcription, translation, metabolic processes, proliferative signaling and cell division. In some instances, methods described herein include intrabody-based therapies. In some such instances, variable domain sequences and/or CDR sequences disclosed herein are incorporated into one or more constructs for intrabody-based therapy. For example, intrabodies may target one or more glycated intracellular proteins or may modulate the interaction between one or more glycated intracellular proteins and an alternative protein.

The intracellular expression of intrabodies in different compartments of mammalian cells allows blocking or modulation of the function of endogenous molecules (Biocca, et al., EMBO J. 1990, 9: 101-108; Colby et al., Proc. Natl. Acad. Sci. U.S.A. 2004, 101: 17616-17621). Intrabodies can alter protein folding, protein-protein, protein-DNA, protein-RNA interactions and protein modification. They can induce a phenotypic knockout and work as neutralizing agents by direct binding to the target antigen, by diverting its intracellular trafficking or by inhibiting its association with binding partners. With high specificity and affinity to target antigens, intrabodies have advantages to block certain binding interactions of a particular target molecule, while sparing others.

Sequences from donor antibodies may be used to develop intrabodies. Intrabodies are often recombinantly expressed as single domain fragments such as isolated VH and VL domains or as a single chain variable fragment (scFv) antibody within the cell. For example, intrabodies are often expressed as a single polypeptide to form a single chain antibody comprising the variable domains of the heavy and light chains joined by a flexible linker polypeptide. Intrabodies typically lack disulfide bonds and are capable of modulating the expression or activity of target genes through their specific binding activity. Single chain intrabodies are often expressed from a recombinant nucleic acid molecule and engineered to be retained intracellularly (e.g., retained in the cytoplasm, endoplasmic reticulum, or periplasm). Intrabodies may be produced using methods known in the art, such as those disclosed and reviewed in: (Marasco et al., PNAS, 1993, 90: 7889-7893; Chen et al., Hum. Gene Ther. 1994, 5:595-601; Chen et al., 1994, PNAS, 91: 5932-5936; Maciejewski et al., 1995, Nature Med., 1: 667-673; Marasco, 1995, Immunotech, 1: 1-19; Mhashilkar, et al., 1995, EMBO J. 14: 1542-51; Chen et al., 1996, Hum. Gene Therap., 7: 1515-1525; Marasco, Gene Ther. 4:11-15, 1997; Rondon and Marasco, 1997, Annu. Rev. Microbiol. 51:257-283; Cohen, et al., 1998, Oncogene 17:2445-56; Proba et al., 1998, J. Mol. Biol. 275:245-253; Cohen et al., 1998, Oncogene 17:2445-2456; Hassanzadeh, et al., 1998, FEBS Lett. 437:81-6; Richardson et al., 1998, Gene Ther. 5:635-44; Ohage and Steipe, 1999, J. Mol. Biol. 291:1119-1128; Ohage et al., 1999, J. Mol. Biol. 291:1129-1134; Wirtz and Steipe, 1999, Protein Sci. 8:2245-2250; Zhu et al., 1999, J. Immunol. Methods 231:207-222; Arafat et al., 2000, Cancer Gene Ther. 7:1250-6; der Maur et al., 2002, J. Biol. Chem. 277:45075-85; Mhashilkar et al., 2002, Gene Ther. 9:307-19; and Wheeler et al., 2003, FASEB J. 17: 1733-5; and references cited therein).

In some instances, payloads of the disclosure may encode biosynthetic antibodies as described in U.S. Patent No. 5,091,513. Such antibody may include one or more sequences of amino acids constituting a region which behaves as a biosynthetic antibody binding site (BABS). The sites comprise 1) non-covalently associated or disulfide bonded synthetic VH and VL dimers, 2) VH-VL or VL-VH single chains wherein the VH and VL are attached by a polypeptide linker, or 3) individuals VH or VL domains. The binding domains comprise linked CDR and FR regions, which may be derived from separate immunoglobulins. The biosynthetic antibodies may also include other polypeptide sequences which function, e.g., as an enzyme, toxin, binding site, or site of attachment to an immobilization media or radioactive atom. Methods are disclosed for producing the biosynthetic antibodies, for designing BABS having any specificity that can be elicited by in vivo generation of antibody, and for producing analogs thereof.

In some instances, payloads may encode antibodies with antibody acceptor frameworks taught in U.S. Patent No. 8,399,625. Such antibody acceptor frameworks may be particularly well suited accepting CDRs from an antibody of interest.

In one instance, the antibody may be a conditionally active biologic protein. An antibody may be used to generate a conditionally active biologic protein which are reversibly or irreversibly inactivated at the wild type normal physiological conditions as well as to such conditionally active biologic proteins and uses of such conditional active biologic proteins are provided. Such methods and conditionally active proteins are taught in, for example, International Publication No. WO2015175375 and WO2016036916 and US Patent Publication No. US20140378660.

### Antibody preparations

The preparation of antibodies, whether monoclonal or polyclonal, is known in the art. Techniques for the production of antibodies are well known in the art and described, e.g. in Harlow and Lane "Antibodies, A Laboratory Manual", Cold Spring Harbor Laboratory Press, 1988; Harlow and Lane "Using Antibodies: A Laboratory Manual" Cold Spring Harbor Laboratory Press, 1999 and "Therapeutic Antibody Engineering: Current and Future Advances Driving the Strongest Growth Area in the Pharmaceutical Industry" Woodhead Publishing, 2012.

The antibodies and fragments and variants thereof as described herein can be produced using recombinant polynucleotides. In one instance, the polynucleotides have a modular design to encode at least one of the antibodies, fragments or variants thereof. As a non-limiting example, the polynucleotide construct may encode any of the following designs: (1) the heavy chain of an antibody, (2) the light chain of an antibody, (3) the heavy and light chain of the antibody, (4) the heavy chain and light chain separated by a linker, (5) the VH1, CH1, CH2, CH3 domains, a linker and the light chain or (6) the VH1, CH1, CH2, CH3 domains, VL region, and the light chain. Any of these designs may also comprise optional linkers between any domain and/or region. The polynucleotides of the present disclosure may be engineered to produce any standard class of immunoglobulins using an antibody described herein or any of its component parts as a starting molecule.

### Antibodies used for immunotherapy

In some instances, payloads of the present disclosure may be antibodies, fragments and variants thereof which are specific to tumor specific antigens (TSAs) and tumor associated antigens (TAAs). Antibodies circulate throughout the body until they find and attach to the TSA/TAA. Once attached, they recruit other parts of the immune system, increasing ADCC (antibody dependent cell-mediated cytotoxicity) and ADCP (antibody dependent cell-mediated phagocytosis) to destroy tumor cells. As used herein, the term "tumor specific antigen (TSA)" means an antigenic substance produced in tumor cells, which can trigger an anti-tumor immune response in a host organism. In one instance, a TSA may be a tumor neoantigen. The tumor antigen specific antibody mediates complementdependent cytotoxic response against tumor cells expressing the same antigen.

Of particular interest is a TSA that is a breast cancer antigen, an ovarian cancer antigen, a prostate cancer antigen, a cervical cancer antigen, a pancreatic carcinoma antigen, a lung cancer antigen, a bladder cancer antigen, a colon cancer antigen, a testicular cancer antigen, a glioblastoma cancer antigen, an antigen associated with a B cell malignancy, an antigen associated with multiple myeloma, an antigen associated with non-Hodgkin's lymphoma, or an antigen associated with chronic lymphocytic leukemia.

Suitable antibodies which can immunoactively bind to a TSA may include, but are not limited to, those specific to 5T4, 707-AP, A33, AFP (α-fetoprotein), AKAP-4 (A kinase anchor protein 4), ALK, α5β1-integrin, androgen receptor, annexin II, alpha- actinin-4, ART-4, B1, B7H3, B7H4, BAGE (B melanoma antigen), BCMA, BCR-ABL fusion protein, beta-catenin, BKT-antigen, BTAA, CA-I (carbonic anhydrase I), CA50 (cancer antigen 50), CA125, CA15-3, CA195, CA242, calretinin, CAIX (carbonic anhydrase), CAMEL (cytotoxic T-lymphocyte recognized antigen on melanoma), CAM43, CAP-1, Caspase-8/m, CD4, CD5, CD7, CD19, CD20, CD22, CD23, CD25, CD27/m, CD28, CD30, CD33, CD34, CD36, CD38, CD40/CD154, CD41, CD44v6, CD44v7/8, CD45,CD49f, CD56, CD68\KP1, CD74, CD79a/CD79b, CD103, CD123, CD133, CD138, CD171, cdc27/m, CDK4 (cyclin dependent kinase 4), CDKN2A, CDS, CEA (carcinoembryonic antigen), CEACAM5, CEACAM6, chromogranin, c-Met, c-Myc, coa-1, CSAp, CT7, CT10, cyclophilinB, cyclin B1, cytoplasmic tyrosine kinases, cytokeratin, DAM-10, DAM-6, dek-can fusion protein, desmin, DEPDC1 (DEP domain containing 1), E2A-PRL, EBNA, EGF-R (epidermal growth factor receptor), EGP-1 (epithelial glycoprotein -1) (TROP-2), EGP-2, EGP-40, EGFR (epidermal growth factor receptor), EGFRvIII, EF-2, ELF2M, EMMPRIN, EpCAM (epithelial cell adhesion molecule), EphA2, Epstein Barr virus antigens, Erb (ErbB1; ErbB3; ErbB4), ETA (epithelial tumor antigen), ETV6-AML1 fusion protein, FAP (fibroblast activation protein), FBP (folate-binding protein), FGF-5, folate receptor α, FOS related antigen 1, fucosyl GM1, G250, GAGE (GAGE-1; GAGE-2), galactin, GD2 (ganglioside), GD3, GFAP (glial fibrillary acidic protein), GM2 (oncofetal antigen- immunogenic-1; OFA-I-1), GnT-V, Gp100, H4-RET, HAGE (helicase antigen), HER-2/neu, HIFs (hypoxia inducible factors), HIF-1α, HIF-2α, HLA-A2, HLA-A*0201-R170I, HLA-A11, HMWMAA, Hom/Mel-40, HSP70-2M (Heat shock protein 70), HST-2, HTgp-175, hTERT (or hTRT), human papillomavirus-E6/human papillomavirus-E7 and E6, iCE (immune-capture EIA), IGF-1R, IGH-IGK, IL2R, IL5, ILK (integrin-linked kinase), IMP3 (insulin-like growth factor II mRNA-binding protein 3), IRF4 (interferon regulatory factor 4), KDR (kinase insert domain receptor), KIAA0205, KRAB-zinc finger protein (KID)-3; KID31, KSA (17-1A), K-ras, LAGE, LCK, LDLR/FUT (LDLR-fucosyltransferaseAS fusion protein), LeY (Lewis Y), MAD-CT-1, MAGE (tyrosinase, melanoma-associated antigen) (MAGE-1; MAGE-3), melan-A tumor antigen (MART), MART-2/Ski, MC1R (melanocortin 1 receptor), MDM2, mesothelin, MPHOSPH1, MSA(muscle-specific actin), mTOR (mammalian targets of rapamycin), MUC-1, MUC-2, MUM-1 (melanoma associated antigen (mutated) 1), MUM-2, MUM-3, Myosin/m, MYL-RAR, NA88-A, N-acetylglucosaminyltransferase, neo-PAP, NF-KB (nuclear factor-kappa B), neurofilament, NSE (neuron- specific enolase), Notch receptors, NuMa, N-Ras, NY-BR-1, NY- CO-1, NY-ESO-1, OncostatinM, OS-9, OY-TES1, p53 mutants, p190 minorbcr-abl, pl5(58), pl85erbB2, pl80erbB-3, PAGE (prostate associated gene), PAP (prostatic acid phosphatase), PAX3, PAX5, PDGFR (platelet derived growth factor receptor), cytochrome P450 involved in piperidine and pyrrolidine utilization (PIPA), Pml-RAR alpha fusion protein, PR-3 (proteinase 3), PSA (prostate specific antigen), PSM, PSMA (Prostate stem cell antigen), PRAME (preferentially expressed antigen of melanoma), PTPRK, RAGE (renal tumor antigen), Raf (A-Raf, B-Raf and C-Raf), Ras, receptor tyrosine kinases, RCAS1, RGSS, ROR1 (receptor tyrosine kinase-like orphan receptor 1), RU1, RU2, SAGE, SART-1, SART-3, SCP-1, SDCCAG16, SP-17 (sperm protein 17), src-family, SSX (synovial sarcoma X breakpoint)-1, SSX-2(HOM-MEL-40), SSX-3, SSX-4, SSX-5, STAT-3, STAT-5, STAT-6, STEAD, STn, survivin, syk-ZAP70, TA-90 (Mac-2 binding protein\cyclophilin C-associated protein), TAAL6, TACSTD1 (tumor associated calcium signal transducer 1), TACSTD2, TAG-72-4, TAGE, TARP (T cell receptor gamma alternate reading frame protein), TEL/AML1 fusion protein, TEM1, TEM8 (endosialin or CD248), TGFβ, TIE2, TLP, TMPRSS2 ETS fusion gene, TNF-receptor (TNF-α receptor, TNF-β receptor; or TNP-γ receptor), transferrin receptor, TPS, TRP-1 (tyrosine related protein 1), TRP-2, TRP-2/INT2, TSP-180, VEGF receptor, WNT, WT-1 (Wilm's tumor antigen) and XAGE.

In one instance, the payload of the present disclosure may be an anti-CD47 antibody. CD47 is a ubiquitously expressed immunoregulatory protein that prevents phagocytic removal of healthy cells by the immune system. CD47 is expressed on the surface of many types of cancer cells, thereby disrupting anti-cancer immune responses. CD 47 is also involved in various other important cellular processes, such as angiogenesis, cancer cell death and regulation of T-cell immunity. Anti-CD47 antibodies in several pre-clinical studies have shown therapeutic benefit in solid cancers and most notably B-cell malignancies.

In one instance, the payload of the present disclosure may be an anti-CD22 antibody. As a non-limiting example, the anti-CD22 antibody is any of the antibodies, fragments or variants thereof described in US Patent Publication No. US20150086562. The anti-CD22 antibody may comprise a heavy chain variable region having the amino acid sequences of SEQ ID NO: 49-64 in US20150086562, and/or a light chain variable region having the amino acid sequence of SEQ ID NO: 17-32 in US20150086562.

In some instances, payloads of the present disclosure may be antibodies, fragments and variants thereof which can specifically block an immunoinhibitory signal. These blocking antibodies (also referred to as antagonists) bind to co-inhibitory receptors, therefore blocking their signal transduction. As non-limiting examples, the blocking antibodies may be specific to CTLA-4, PD-1, and PD-L1/L2. In one instance, the anti-CTLA-4 antibody is Ipilimumab. In another instance, the anti-PD-1 antibody is Nivolumab. Antibodies that bind to PD-L1 and enhance T cell immune response may include antibodies taught in US patent publication NO.: 2016/0108123. Other inhibitory immunomodulatory targets may include B7-H3, which can increase cancer cell metabolism such as glucose uptakes and lactate production (Lim et al., Cancer Res., 2016, 76(8): 1-12). Antibodies that block B7-H3 are disclosed in US Pat. NO.: 9,150,656.

In one aspect, payloads of the present disclosure may be antagonistic antibodies specific to VSIG8 (v-set and immunoglobulin domain containing 8) comprising the amino acid sequences of SEQ ID NOs: 1, 2 and 3 in US patent publication NO.: US2016/0159927. Antagonistic antibodies may also include a chimeric IL2 receptor (CD25) antibody (Basiliximab) (US Patent Publication NO.: 20080171017), and antagonizing antibodies which bind to human TIM-3 (US patent publication NO.: US2015/0218274), BTLA, VISTA and LAG-3 (See, e.g., US patent publication NO.: US2015/0259420).

In one aspect, the payload of the present disclosure may be an anti-CSF-IR antibody, which is characterized in binding to the dimerization domains D4 to D5 of the extracellular domain of human CSF-IR. This antibody inhibitor can inhibit cell proliferation and survival in CSF-IR ligand-dependent and CSF-1 ligandindependent CSF-IR expressing tumor cells, monocytes and infiltrating macrophages (See, e.g., International Patent Publication NO.: WO2013/132044).

In another aspect, the payload of the present disclosure may be an antagonistic antibody against CXCL12. The anti-CXCL12 antibody blocks the interaction of CXCL12 with its receptor CXCR4, thereby inhibiting CXCR4 signaling. The CXCR4 signaling inhibitor increases the proximity or the frequency of T-cells among cancer cells in the tumor tissue (See, International Patent Publication NO.: WO 2015/019284).

In some instances, payloads of the present disclosure may be agonistic antibodies, fragments and variants thereof, which trigger immune responses, including antibodies specific to co-stimulatory molecules, including but not limited to 4-1BB (CD137), OX40 (CD134), CD40, GITR and CD27.

In one instance, the payload of the present disclosure may be an agonistic CD40 antibody. In another instance, the agonistic antibody specific to 4-1BB (CD137) may be Uremab, a fully human IgG4 monoclonal antibody which specifically binds to and activates 4-1BB (CD137) expressing immune cells, stimulating an immune response, in particular a cytotoxic T cell response, against tumor cells; or Utomilumab, a fully human IgG2 monoclonal antibody; or anti-CD 137 antibody described in International Patent Publication NO.: WO2006/088447.

In some instances, the payload of the present disclosure may be an antagonistic antibody against Amphiregulin. Amphiregulin (AREG) is an EGF-like growth factor which binds to the EGFR receptor and enhances T regulatory cell function. AREG is produced in a phenotypically and functionally distinct subtype of CD4⁺ regulatory T cells (Tregs) which have a distinct T cell receptor (TCR) repertoire and express the IL33R. AREG promotes immune suppression in the tumor environment. The anti-Amphiregulin antibody may comprise a heavy chain variable region having the amino acid sequences of SEQ ID NO:: 2,4, and 12 in U.S. Patent No. 7,223,393, and/or a light chain variable region having the amino acid sequence of SEQ ID NO:: 3, 5, and 14 in U.S. Patent No. 7,223,393.

In some instances, antibodies specific to co-inhibitory molecules and co-stimulatory molecules may be secreted scFv antibodies.

In some instances, antibody payloads of the present disclosure may be T-cell bispecific antibodies (e.g. T cell-engaging BiTE^{™} antibodies CDS- CD 19, CD3-EpCam, and CD3-EGFR). Other bispecific antibodies used for immunotherapy may also be included as payloads of the present disclosure, for example, bispecific anti-TNF-α and anti- IL6 antibody (EP3062818), bispecific antibodies to an immune cell antigen and TAG-72 (WO2016/089610), anti-ovarian and D3 bispecific antibodies in US. Pat. No. 7,262,276; bispecific antibodies against CD133 and CD3 in WO2014/128185; bispecific antibodies against CTLA-4 and PD-1 discussed in US2016/0145355, bispecific antibodies against CD3 and CD19 disclosed in WO2015/006749, and US. Pat. NOs. 7, 635,472; 7, 112, 324; bispecific antibodies against Her2 and CD3 in US2014/0170149; bispecific antibodies against CD19 and CD16 in US2005/0089519.

In some instances, antibodies with decreased affinity may be selected over antibodies with high affinity for the same antigen. Such low affinity antibodies are more effective in discriminating tumors which express high levels of the antigen and normal tissues that express the same antigen at lower levels, while maintaining similar antitumor response.

In some instances, the targeting moieties of the present disclosure may include variable heavy chain and variable light chain comprising the amino acid sequences selected from those in Table 10 of co-owned U.S. Provisional Patent Application No. 62/683,972, filed June 12,2018, and the International Patent Application No. PCT/US18/37005 filed on June 12, 2018. Heavy chain amino acid sequences are provided as SEQ ID NO: 539 to SEQ ID NO: 3094; and light chain amino acid sequences are provided as SEQ ID NO: 3095 to SEQ ID NO: 5473.

### Intrabodies

In some instances, payloads of the present disclosure may be intrabodies against players in regulating immune cells. An intrabody is an antibody that is designed to be expressed intracellularly and can be directed to a specific target antigen present in various subcellular locations including the cytosol, nucleus, endoplasmic reticulum (ER), mitochondria, peroxisomes, plasma membrane and trans-Golgi network (TGN) through in frame fusion with intracellular trafficking/localization peptide sequences. The most commonly used format is a single chain antibody (scFv) created by joining the antigen-binding variable domains of heavy and light chain with a linker, most often the 15-amino acid linker (e.g., (GGGGS)₃ (SEQ ID NO: 307)) between the variable heavy (VH) and variable light (VL) chains. The intracellular intrabodies are being developed to bind to, neutralize, or modify the function or localization of cancer-related targets and thereby affect the malignant phenotype.

Players that modulate immune cells (e.g., T cells) may be any intracellular signaling checkpoint. Exemplary players may include a co-inhibitory ligand, Casitas B-linage lymphoma proto-oncogene-b (Cbl-b) (a E3 ligase), a protein tyrosine phosphatase (PTP) such as Src homology 2 domain-containing protein tyrosine phosphatase 1 (SHP-1), and Ras. For example, an intrabody may be an intrabody against oncogenic form of RAS disclosed in PCT patent publication NO.: WO2004/046186.

### Therapeutic Antibodies

In some instances, antibody payloads of the present disclosure may be therapeutic antibodies. As non-limiting examples, antibodies and fragments and variants thereof may be specific to tumor associated antigens, or tumor specific antigens, or pathogen antigens. In some aspects, antibodies may be blocking antibodies (also referred to as antagonistic antibodies), for example, blocking antibodies against PD-1, PD-L1, PD-L2, CTLA-4 and other inhibitory molecules. In other aspects, antibodies may be agonist antibodies such as agonistic antibodies specific to stimulatory molecules, e g., 4-1BB (CD137), OX40 (CD134), CD40, GITR and CD27.

Other exemplary therapeutic antibodies may include, but are not limited to, Abagovomab, Abcxmab, Abituzumab, Abrilumab, Actoxumab, Adalimumab, Adecatumumab, Afasevikumab, Afelimomab, Afutuzumab, Alacizumab, Alemtuzumab, Alirocumab, Altumomab, Amatuximab, Anetumab, Anifrolumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab, Bleselumab, Blinatumomab, Blinatumomab, Blosozumab, Bococizumab, Brentuximab, Briaknumab, Brodalumab, Brolucizumab, Brontictuzumab, Cabiralizumab, Canakinumab, Cantuzumab, Caplacizumab, Capromab, Carlumab, Carotuximab, Catumaxomab, cBR96-doxorubicin immunoconjugate, Cedelizumab, Cergutuzumab, Certolizumab pegol, Cetuximab, Citatuzumab, Cixutumumab, Clazakizumab, Clenoliximab, Clivatuzumab, Codrituzumab, Coltuximab, Contatumumab, Concizumab, Crenezumab, Crotedumab, CR6261, Dacetumab, Daclizumab, Dalotuzumab, Dapirolizumab pegol, Daratumumab, Dectrekumab, Demcizumab, Denintuzumab, Denosumab, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, Enfortumab vedotin, Enlimomab pegol, Enoblituzumab, Enokizumab, Enoticumab, Ensituximab, Epitumomab cituxetan, Epratuzumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tituxetan, icrucumab, Idarucizumab, Igovomab, IMAB362, Imalumab, Imciromab, Imgatuzumab, Inclacumab, Indatuximab, Indusatumab, Inebilizumab, Infliximab, Intetumumab, Inolimomab, Inotuzumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab, Lebrikizumab, Lemalesomab, Lendalizumab, Lenzilumab, Lerdelimumab, Lexatumumab, Libivirumab, Lifastuzumab, Ligelizumab, Lilotomab, Lintuzumab, Lirilumab, Lodelcizumab, Lokivetmab, Lorvotuzumab, Lucatumumab, Lulizumab pegol, Lumiliximab, Lumretuzumab, Mapatumumab, Margetuximab, Maslimomab, Mavrilimumab, Matuzumab, Mepolizumab, Metelimumab, Milatuzumab, Minretumomab, Mirvetuximab, Mitumomab, Mogamulizumab, Monalizumab, Morolimumab, Motavizumab, Moxetumomab pasudotox, Muromonab-CD3, nacolomab tafenatox, Namilumab, naptumomab, naratuximab, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab, Obiltoxaximab, Obinutuzumab, Ocaratuzumab, Ocrelizumab, Odulimomab, Ofatumumab, Olaratumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab, Pintumomab, Placulumab, Plozalizumab, Pogalizumab, Polatuzumab, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab, Rovelizumab, Ruplizumab, Sacituzumab, Samalizumab, Sapelizumab, Sarilumab, Satumomab pendetide, Secukinumab, Seribantumab, Setoxaximab, Sevirumab, Sibrotuzumab, SGN-CD19A, SGN-CD33A, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, tabalumab, Tacatuzumab, Tadocizumab, Talizumab, Tamtuvetmab, Tanezumab, Taplitumomab, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tildrakizumab, Tigatuzumab, Timolumab, Tisotumab vedotin, TNX-650, Tocilizumab, Toralizumab, Tosatoxumab, Tositumomab, Tovetumab, Tralokinumab, Trastuzumab, TRBS07, Tregalizumab, Tremelimumab, Trevogrumab, Tucotuzumab, Tuvirumab, Ublituximab, Ulcocuplumab, Urelumab, Urtoxazumab, Ustekinumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab, Votumumab, Xentuzumab, Zalutumumab, Zanolimumab, Zatuximab, Ziralimumab and Zolimomab aritox.

### Bicistronic and/or Pseudo-bicistronic antibody payloads

According to the present disclosure, a bicistronic payload is a polynucleotide encoding a two-protein chain antibody on a single polynucleotide strand. A pseudo-bicistronic payload is a polynucleotide encoding a single chain antibody discontinuously on a single polynucleotide strand. For bicistronic payloads, the encoded two strands or two portions/regions and/or domains (as is the case with pseudo-bicistronic) are separated by at least one nucleotide not encoding the strands or domains. More often the separation comprises a cleavage signal or site or a non-coding region of nucleotides. Such cleavage sites include, for example, furin cleavage sites encoded as an "RKR" site, or a modified furin cleavage site in the resultant polypeptide or any of those taught herein.

According to the present disclosure, a single domain payload comprises one or two polynucleotides encoding a single monomeric variable antibody domain. Typically, single domain antibodies comprise one variable domain (VH) of a heavy-chain antibody.

According to the present disclosure, a single chain Fv payloads is a polynucleotide encoding at least two coding regions and a linker region. The scFv payload may encode a fusion protein of the variable regions of the heavy (VH) and light chains (VL) of immunoglobulins, connected with a short linker peptide of ten to about 25 amino acids. The linker is usually rich in glycine for flexibility, as well as serine or threonine for solubility, and can either connect the N-terminus of the VH with the C-terminus of the VL, or vice versa. Other linkers include those known in the art and disclosed herein.

According to the present disclosure, a bispecific payload is a polynucleotide encoding portions or regions of two different antibodies. Bispecific payloads encode polypeptides which may bind two different antigens. Polynucleotides of the present disclosure may also encode trispecific antibodies having an affinity for three antigens.

### 3. Tumor and pathogen specific antigens

In some instances, payloads of the present disclosure may be tumor specific antigens (TSAs), tumor associated antigens (TAAs), pathogen associated antigens, or fragments thereof. The antigen can be expressed as a peptide or as an intact protein or portion thereof. The intact protein or a portion thereof can be native or mutagenized. Antigens associated with cancers or virus-induced cancers as described herein are well-known in the art. Such a TSA or TAA may be previously associated with a cancer or may be identified by any method known in the art.

A tumor specific antigen (TSA) may be a tumor neoantigen. A neoantigen is a mutated antigen that is only expressed by tumor cells because of genetic mutations or alterations in transcription which alter protein coding sequences, therefore creating novel, foreign antigens. The genetic changes result from genetic substitution, insertion, deletion or any other genetic changes of a native cognate protein (i.e. a molecule that is expressed in normal cells).

As non-limiting examples, neoantigens may include mutated new peptides derived from alpha-actinin-4, ARTC1, BCR-ABL fusion protein (b3a2), B-RAF, CASP-5, CASP-8, beta-catenin, Cdc27, CDK4, CDKN2A, CLPP, CML-66, COA-1, connexin 37, dek-can fusion protein, EFTUD2, Elongation factor 2, ETV6-AML1 fusion protein, fibronectin, FLT3-ITD, FN1, GPNM8, LDLR-fucosyltransferase AS fusion protein, HLA-A2, HLA-A11, Hsp-70-1B, MART-2, ME1, MUM-1, MUM-2, MUM-3, Myosin class I, NFYC, neo-PAP, OGT, OS-9, p53, pml-RARalpha fusion protein, PRDX5, PTPRK, K-Ras, N-Ras, RBAF600, sirtuin-2, SNRPD1, SYT-SSX1/SSX2 fusion protein, TGF-beta receptor II, etc. Additional neoantigen peptides may include SF3B1 peptides, MYD peptides, TP53 peptides, Abl peptides, FBXW7 peptides, MAPK peptides, and GNB1 peptides disclosed in US patent publication NO.: 20110293637.

New neoantigens identified through large-scale sequencing and algorithm calculation may also be included. See, e.g., International Patent Publication NO.: WO2014/168874; Nishimura et al., Cancer Sci. 2015, 106(5): 505-511; and Linnemann et al., Nat. Med., 2015, 21(1): 81-85.

A tumor associated antigen (TAA) may be an overexpressed or accumulated antigen that is expressed by both normal and neoplastic tissue, with the level of expression highly elevated in cancer tissues. Numerous proteins (e.g. oncogenes) are up-regulated in tumor tissues, including but not limited to adipophilin, AIM-2, ALDH1A1, BCLX(L), BING-4, CALCA, CD45, CD274, CPSF, cyclinD1, DKK1, ENAH, epCAM, ephA3, EZH2, FGF5, G250, HER-2/neu, HLA-DOB, Hepsin, IDO1, IGFB3, IL13Ralpha2, Intestinal carboxyl esterase, kallikrein 4, KIF20A, lengsin, M-CSF, MCSP, mdm-2, Meloe, Midkine, MMP-2, MMP-7, MUC-1, MUC5AC, p53, Pax5, PBF, PRAME, PSMA, RAGE-1, RGSS, RhoC, RNF43, RU2A5, SECERNIN 1, SOX10, STEAP1, survivin, Telomerase, TPBG, VEGF, and WT1.

A TAA may be an oncofetal antigen that is typically only expressed at different stages during the development of the fetus and in cancerous somatic cells. Many proteins are normally expressed during fetal development but are transcriptionally repressed after birth or at early stage of infancy, therefore are not present, or are expressed in significantly lower levels in the corresponding normal adult tissue. Some of these developmental proteins are re-expressed in certain tumor cells and become oncofetal antigens. Examples of oncofetal antigens may include, but are not limited to CEA (carcinoembryonic antigen) in colorectal carcinoma, iLRP/OFA (immature laminin receptor protein/oncofetal antigen) in renal cell carcinoma (RCC), TAG-72 (tumor associated glycoprotein-72) in prostate carcinoma, AFP (alpha-fetoprotein) in hepatocellular carcinoma (HCC), ROR1 (a receptor tyrosine kinase) in many malignant cells such as brain tumors, sperm protein 17, HMGA2 (high mobility group A2) in ovarian carcinoma, oncofetal H19, CR-1 (Cripto-1, a member of epidermal growth factor (EGF)-CFC family), trophoblast glycoprotein precursor and GPC-3 (Glypican-3, a member of heparan sulphate proteoglycans) in HCC.

A TAA may be a cancer-testis antigen that is expressed only by cancer cells and adult reproductive tissues such as testis and placenta, including, but limited to antigens from BAGE family, CAGE family, HAGE family, GAGE family, MAGE family (e.g., MAGE-A1, MAGE-A2, MAGE-A3, MAGE-A6 and MAGE-A13), SAGE family, XAGE family, MCAK, NA88-A (cancer/testis antigen 88), PSAD1, SSX-2, and SLLP-1.

A TAA may be a lineage restricted antigen that is expressed largely by a single cancer histotype, such as Melan-A/MART-1, Gp10/pmel17, Tyrosinase, TRP-1/-2, P. polypeptide, MC1R in melanoma; and prostate specific antigen (PSA) in prostate cancer.

A TAA may be an oncoviral antigen that is encoded by tumorigenic transforming viruses (also called oncogenic viruses). Oncogenic viruses, when they infect host cells, can insert their own DNA (or RNA) into that of the host cells. When the viral DNA or RNA affects the host cell's genes, it can push the cell toward becoming cancer. Oncogenic viruses include, but are not limited to, RNA viruses, and DNA viruses. Some examples of commonly known oncoviruses include human papilloma viruses (HPVs) which are main causes of cervical cancer, Epstein-Barr virus (EBV) which may cause nasopharyngeal cancer, certain types of fast-growing lymphomas (e.g., Burkitt lymphoma) and stomach cancer, hepatitis B, C and D viruses (HBV, HCV and HDV) in hepatocellular carcinoma (HCC), human immunodeficiency virus (HIV) which increases the risk of getting many types of cancer (e.g., liver cancer, anal cancer and Hodgkin cancer), Kaposi sarcoma herpes virus (KSHV; also known as human herpes virus 8 (HHV8)) which is linked to lymphoma, human T-lymphotropic virus (HTLV-1) and Merkle cell polyomavirus (MCV). A viral antigen can be any defined antigen of a virus that is associated with a cancer in a human. For example, antigens from EBV may include but are not limited to, Epstein-Barr nuclear antigen-1 (EBNA1), latent membrane protein 1 (LMP1), or latent membrane protein 2 (LMP2).

A TAA may be an idiotypic antigen that is generated from highly polymorphic genes where a tumor cell expresses a specific "clonotype", i.e., as in B cell, T cell lymphoma/leukemia resulting from clonal aberrancies, such as Immunoglobulin and T cell receptors (TCRs). Idiotypic antigens are a class of non-pathogen-associated neoantigens. For example, the malignant B cells express rearranged and multiply mutated surface immunoglobulins (Ig). Tumor specific idiotypes (e.g., immunoglobulin idiotypes) are regarded as particularly attractive tumor-specific antigens that can be successfully targeted by immunotherapy (e.g., Alejandro et al., Front Oncol., 2012, 2: 159).

### 4. T cell receptors (TCRs)

In some instances, payloads of the present disclosure may be T cell receptors (TCRs). The TCR may be specific to a cancer antigen, having a specific α chain and β chain which together form a TCRαβ heterodimer, or having a specific γ chain and δ chain which together form a TCRγδ heterodimer. The TCR may be a recombinant antigen specific T cell receptor.

The variable regions of α chain and β chain determine T cell specificity to an antigenic peptide presented by the major histocompatibility complex (MHC) class I and II molecules. The TCR recognition of the tumor antigen on the surface of a tumor cell presented by MHC molecules by TCR triggers T cell activation. The use of TCR gene therapy can equip a subject's own T cells with desired specificities and generate sufficient numbers of T cells to eradicate tumor cells. In some instances, a biocircuit or an effector module comprising a tumor specific TCR may be transduced into T cells and TCR-engineered T cells will be infused into cancer patients who have lymphocytopenia or lymphopenia by chemotherapy or irradiation, allowing efficient engraftment but inhibiting immune suppression.

Sequences encoding tumor antigen recognizing TCR chains can be obtained from tumor-reactive T cells (e.g., tumor-infiltrating lymphocytes isolated from the tumor of a patient).

According to the present disclosure, a TCR specific to tumor cells can be produced by methods described in International Patent Publication NO.: WO2014/083173. A host organism expressing a transgene of a human leucocyte antigen (HLA) type which is known or suspected to be able to present a mutated tumor specific antigen (TSA) is transduced to express the un-rearranged human TCR loci. Preferably these loci encode TCR α and β chains, and preferably comprise a plurality, ideally all, of human TCR V, D, J, and/or C genes. The host organism is immunized with a cancer specific TSA or a peptide epitope derived from the TSA and T cells expressing rearranged TCRs specifically against the TSA are isolated and cloned. The TCR from the cloned T cells are sequenced (International Patent Publication NO.: WO2014/083173).

In some instances, payloads of the present disclosure may be TCRs that specifically recognize TSAs, TAAs, or epitopes thereof, complexed with MHC molecules.

Exemplary tumor antigens that can be recognized by a TCR may include at least the following: 5T4, 707-AP, A33, AFP (α-fetoprotein), AKAP-4 (A kinase anchor protein 4), ALK, α5β1-integrin, androgen receptor, annexin II, alpha- actinin-4, ART-4, B1, B7H3, B7H4, BAGE (B melanoma antigen), BCMA, BCR-ABL fusion protein, beta-catenin, BKT-antigen, BTAA, CA-I (carbonic anhydrase I), CA50 (cancer antigen 50), CA125, CA15-3, CA195, CA242, calretinin, CAIX (carbonic anhydrase), CAMEL (cytotoxic T-lymphocyte recognized antigen on melanoma), CAM43, CAP-1, Caspase-8/m, CD4, CD5, CD7, CD19, CD20, CD22, CD23, CD25, CD27/m, CD28, CD30, CD33, CD34, CD36, CD38, CD40/CD154, CD41, CD44v6, CD44v7/8, CD45,CD49f, CD56, CD68\KP1, CD74, CD79a/CD79b, CD103, CD123, CD133, CD138, CD171, cdc27/m, CDK4 (cyclin dependent kinase 4), CDKN2A, CDS, CEA (carcinoembryonic antigen), CEACAM5, CEACAM6, chromogranin, c-Met, c-Myc, coa-1, CSAp, CT7, CT10, cyclophilinB, cyclinB1, cytoplasmic tyrosine kinases, cytokeratin, DAM-10, DAM-6, dek-can fusion protein, desmin, DEPDC1 (DEP domain containing 1), E2A-PRL, EBNA, EGF-R (epidermal growth factor receptor), EGP-1 (epithelial glycoprotein -1) (TROP-2), EGP-2, EGP-40, EGFR (epidermal growth factor receptor), EGFRvIII, EF-2, ELF2M, EMMPRIN, EpCAM (epithelial cell adhesion molecule), EphA2, Epstein Barr virus antigens, Erb (ErbB1; ErbB3; ErbB4), ETA (epithelial tumor antigen), ETV6-AML1 fusion protein, FAP (fibroblast activation protein), FBP (folate-binding protein), FGF-5, folate receptor α, FOS related antigen 1, fucosyl GM1, G250, GAGE (GAGE-1; GAGE-2), galactin, GD2 (ganglioside), GD3, GFAP (glial fibrillary acidic protein), GM2 (oncofetal antigen- immunogenic-1; OFA-I-1), GnT-V, Gp100, H4-RET, HAGE (helicase antigen), HER-2/neu, HIFs (hypoxia inducible factors), HIF-1α, HIF-2α, HLA-A2, HLA-A*0201-R170I, HLA-All, HMWMAA, Hom/Mel-40, HSP70-2M (Heat shock protein 70), HST-2, HTgp-175, hTERT (or hTRT), human papillomavirus-E6/human papillomavirus-E7 and E6, iCE (immune-capture EIA), IGF-1R, IGH-IGK, IL2R, IL5, ILK (integrin-linked kinase), IMP3 (insulin-like growth factor II mRNA-binding protein 3), IRF4 (interferon regulatory factor 4), KDR (kinase insert domain receptor), KIAA0205, KRAB-zinc finger protein (KID)-3; KID31, KSA (17-1A), K-ras, LAGE, LCK, LDLR/FUT (LDLR-fucosyltransferaseAS fusion protein), LeY (Lewis Y), MAD-CT-1, MAGE (tyrosinase, melanoma-associated antigen) (MAGE-1; MAGE-3), melan-A tumor antigen (MART), MART-2/Ski, MC1R (melanocortin 1 receptor), MDM2, mesothelin, MPHOSPH1, MSA(muscle-specific actin), mTOR (mammalian targets of rapamycin), MUC-1, MUC-2, MUM-1 (melanoma associated antigen (mutated) 1), MUM-2, MUM-3, Myosin/m, MYL-RAR, NA88-A, N-acetylglucosaminyltransferase, neo-PAP, NF-KB (nuclear factor-kappa B), neurofilament, NSE (neuron- specific enolase), Notch receptors, NuMa, N-Ras, NY-BR-1, NY- CO-1, NY-ESO-1, OncostatinM, OS-9, OY-TES1, p53 mutants, p190 minorbcr-abl, pl5(58), pl85erbB2, pl80erbB-3, PAGE (prostate associated gene), PAP (prostatic acid phosphatase), PAX3, PAX5, PDGFR (platelet derived growth factor receptor), cytochrome P450 involved in piperidine and pyrrolidine utilization (PIPA), Pml-RAR alpha fusion protein, PR-3 (proteinase 3), PSA (prostate specific antigen), PSM, PSMA (Prostate stem cell antigen), PRAME (preferentially expressed antigen of melanoma), PTPRK, RAGE (renal tumor antigen), Raf (A-Raf, B-Raf and C-Raf), Ras, receptor tyrosine kinases, RCAS1, RGSS, ROR1 (receptor tyrosine kinase-like orphan receptor 1), RU1, RU2, SAGE, SART-1, SART-3, SCP-1, SDCCAG16, SP-17 (sperm protein 17), src-family, SSX (synovial sarcoma X breakpoint)-1, SSX-2(HOM-MEL-40), SSX-3, SSX-4, SSX-5, STAT-3, STAT-5, STAT-6, STEAD, STn, survivin, syk-ZAP70, TA-90 (Mac-2 binding protein\cyclophilin C-associated protein), TAAL6, TACSTD1 (tumor associated calcium signal transducer 1), TACSTD2, TAG-72-4, TAGE, TARP (T cell receptor gamma alternate reading frame protein), TEL/AML1 fusion protein, TEM1, TEM8 (endosialin or CD248), TGFβ, TIE2, TLP, TMPRSS2 ETS fusion gene, TNF-receptor (TNF-α receptor, TNF-β receptor; or TNF-γ receptor), transferrin receptor, TPS, TRP-1 (tyrosine related protein 1), TRP-2, TRP-2/INT2, TSP-180, VEGF receptor, WNT, WT-1 (Wilm's tumor antigen) and XAGE.

In one aspect, the payload of the present disclosure may be a TCR specifically recognizing Her2/neu epitope which has nucleic acid sequences of α chain and β chain disclosed in U.S. Patent Publication NO.: US20110280894 and International Patent Publication NO. WO2016133779A1. In another aspect, the payload of the present disclosure may be a TCR specific to TSA tyrosinase (See US Pat. NO.: 8, 697,854).

In other aspects, payloads of the present disclosure may be TCRs having polypeptide sequences specific to synovial sarcoma X Breakpoint (SSX)-2 antigen (U.S. Pat. NO.: 9,345,748); human papillomavirus (HPV) 16 E6 antigen (International Patent Publication NO.: WO2015/009606); cytomegalovirus (CMV) phosphoprotein pp65 (US Pat. NO.: 8,722,048); and WT-1 specific TCR comprising a TCR α-chain having an amino acid sequence as set forth in any one of SEQ ID NOs.: 5-8, and comprising a TCR β-chain having an amino acid sequence as set forth in SEQ ID NO:: 12 or 13, as disclosed in US patent publication NO.: US2016/0083449.

In some instances, the TCR specific to a TSA may be modified to possess a sequence encoding an affinity weakening motif which imparts a reduction in non-specific binding to a TSA. In some instances, an affinity weakening motif having a modification to a TCR CDR1 or CDR2 region may be used to weaken the interaction between TCR and HLA proteins (see, International Patent Publication NO.: WO2016/014725).

In some instances, the TCR specific to a TSA may be modified to possess an affinity enhancing motif which imparts an enhancement of binding specificity and affinity for a target antigen. In some instances, such high affinity TCRs may be generated by using TCR α-chain to select *de novo* generated TCR β-chains that pair with an antigen specific TCR α-chain during T cell development *in vitro* to form enhanced TCRs (see, International Patent Publication NO.: WO2013/ 166321). In other instances, the modified TCR may also possess a sequence encoding an affinity enhancing modification CDR3 region which strengthens the interaction between the TCR and the TSA.

In one instance, the TCR specific to a TSA may be modified using zinc finger nucleases (ZFNs), TALENs or a CRISPR/Cas system. The TCR α chain and β chain may contain target sites of a nuclease. The nuclease can cleave the TCR sequence causing a certain degree of disruption of the TCR (see US Patent Publication NO.: US2014/0301990).

In one instance, the TCR specific to a TSA may be a soluble single-chain TCR having the structure: Vα2-L-Vβ or Vβ-L-Vα2, wherein L is a linker peptide that links a TCR variable β region (Vβ) with a TCR variable α region of the family 2 (Vα2), as discussed in International Patent Publication NO.: WO2011/044186.

In one instance, the TCR specific to a TSA may be maturated to increase its affinity to the TSA according to methods described in US Patent Publication NO.: US2014/0065111.

In some instances, the TCRs may be specific to the Fc domain of an antibody (e.g. FcgRla) and utilized to enhance efficacy of antibody mediated therapy, as discussed in International Patent Publication NO.: WO2015/179833.

In some instances, payloads of the present disclosure may be recombinant constructs which act as trifunctional T-cell signaling couplers (TriTACs) mimicking the naturally signaling through T cell receptors. The TriTACs enhance chimeric receptor activity while retaining MHC unrestricted targeting. In some aspects, the recombinant construct may comprise a target specific binding ligand such as a scFV specific to a TSA or a designed ankyrin repeat (DARPin), a ligand that binds a protein associated with the TCR complex and a TCR signaling domain polypeptide, e.g. as described in the International Patent Application NO.: WO2015/117229. The TCR associated proteins may be selected from CD3, ZAP70 9zeta-chain associated protein kinase 70), TYN and CD247. In some aspects, the ligand of a TriTAC that binds the TCR associated protein may be an antibody or an antibody fragment (e.g., scFv). In other aspects, the TCR signaling domain polypeptide of a TriTAC may comprise the transmembrane and cytoplasmic domain of CD4.

According to the present disclosure, the α chain and β chain of the TCR of the present disclosure may be included in separate constructs, for example as payloads of two effector modules. In other instances, the α chain and β chain of the TCR of the present disclosure may be included in a single effector module as two payloads of the same effector module, for example as illustrated in Figures 3-6.

### 5. Chimeric antigen receptors (CARs)

In some instances, payloads of the present disclosure may be a chimeric antigen receptors (CARs) which when transduced into immune cells (e.g., T cells and NK cells), can re-direct the immune cells against the target (e.g., a tumor cell) which expresses a molecule recognized by the extracellular target moiety of the CAR.

As used herein, the term "chimeric antigen receptor (CAR)" refers to a synthetic receptor that mimics TCR on the surface of T cells. In general, a CAR is composed of an extracellular targeting domain, a transmembrane domain/region and an intracellular signaling/activation domain. In a standard CAR receptor, the components: the extracellular targeting domain, transmembrane domain and intracellular signaling/activation domain, are linearly constructed as a single fusion protein. The extracellular region comprises a targeting domain/moiety (e.g., a scFv) that recognizes a specific tumor antigen or other tumor cell-surface molecules. The intracellular region may contain a signaling domain of TCR complex (e.g., the signal region of CD3ζ), and/or one or more costimulatory signaling domains, such as those from CD28, 4-1BB (CD137) and OX-40 (CD134). For example, a "first-generation CAR" only has the CD3ζ signaling domain, whereas in an effort to augment T-cell persistence and proliferation, costimulatory intracellular domains are added, giving rise to second generation CARs having a CD3ζsignal domain plus one costimulatory signaling domain, and third generation CARs having CD3ζ signal domain plus two or more costimulatory signaling domains. A CAR, when expressed by a T cell, endows the T cell with antigen specificity determined by the extracellular targeting moiety of the CAR. Recently, it is also desirable to add one or more elements such as homing and suicide genes to develop a more competent and safer architecture of CAR, so called the fourth-generation CAR.

Cells such as T cells engineered to express a CAR can be redirected to attack target cells that express a molecule which can be recognized by the targeting moiety of the CAR.

In some instances, the extracellular targeting domain is joined through the hinge (also called space domain or spacer) and transmembrane regions to an intracellular signaling domain. The hinge connects the extracellular targeting domain to the transmembrane domain which transverses the cell membrane and connects to the intracellular signaling domain. The hinge may need to be varied to optimize the potency of CAR transformed cells toward cancer cells due to the size of the target protein where the targeting moiety binds, and the size and affinity of the targeting domain itself. Upon recognition and binding of the targeting moiety to the target cell, the intracellular signaling domain leads to an activation signal to the CAR T cell, which is further amplified by the "second signal" from one or more intracellular costimulatory domains. The CAR T cell, once activated, can destroy the target cell.

In some instances, the CAR of the present disclosure may be split into two parts, each part is linked a dimerizing domain, such that an input that triggers the dimerization promotes assembly of the intact functional receptor. Wu and Lim recently reported a split CAR in which the extracellular CD 19 binding domain and the intracellular signaling element are separated and linked to the FKBP domain and the FRB* (T2089L mutant of FKBP-rapamycin binding) domain that heterodimerize in the presence of the rapamycin analog AP21967. The split receptor is assembled in the presence of AP21967 and together with the specific antigen binding, activates T cells (Wu et al., Science, 2015, 625(6258): aab4077).

In some instances, the CAR of the present disclosure may be designed as an inducible CAR. Sakemura et al recently reported the incorporation of a Tet-On inducible system to the CD19 CAR construct. The CD19 CAR is activated only in the presence of doxycycline (Dox). Sakemura reported that Tet-CD19CAR T cells in the presence of Dox were equivalently cytotoxic against CD19⁺ cell lines and had equivalent cytokine production and proliferation upon CD19 stimulation, compared with conventional CD19CAR T cells (Sakemura et al., Cancer Immuno. Res., 2016, Jun 21, Epub ahead of print). In one example, this Tet-CAR may be the payload of the effector module under the control of SREs (e.g., DDs) of the disclosure. The dual systems provide more flexibility to turn-on and off the CAR expression in transduced T cells.

According to the present disclosure, the payload of the present disclosure may be a first-generation CAR, or a second-generation CAR, or a third-generation CAR, or a fourth-generation CAR. Representative effector module instances comprising CAR constructs are illustrated in Figures 13-18. In some instances, the payload of the present disclosure may be a full CAR construct composed of the extracellular domain, the hinge and transmembrane domain and the intracellular signaling region. In other instances, the payload of the present disclosure may be a component of the full CAR construct including an extracellular targeting moiety, a hinge region, a transmembrane domain, an intracellular signaling domain, one or more co-stimulatory domain, and other additional elements that improve CAR architecture and functionality including but not limited to a leader sequence, a homing element and a safety switch, or the combination of such components.

CARs regulated by biocircuits and compositions of the present disclosure are tunable and thereby offer several advantages. The reversible on-off switch mechanism allows management of acute toxicity caused by excessive CAR-T cell expansion. Pulsatile CAR expression using SREs of the present disclosure may be achieved by cycling ligand level. The ligand conferred regulation of the CAR may be effective in offsetting tumor escape induced by antigen loss, avoiding functional exhaustion caused by tonic signaling due to chronic antigen exposure and improving the persistence of CAR expressing cells *in vivo.*

In some instances, biocircuits and compositions of the disclosure may be utilized to down regulate CAR expression to limit on target on tissue toxicity caused by tumor lysis syndrome. Down regulating the expression of the CARs of the present disclosure following anti-tumor efficacy may prevent (1) On target off tumor toxicity caused by antigen expression in normal tissue. (2) antigen independent activation *in vivo.*

In some instances, the payload may be a cytokine-based CAR, wherein the antigen binding domain of the CAR is substituted by a cytokine which may bind to its cognate receptor. In one instance, the payload is humanized interleukin-13 receptor α2 (IL-13Rα2) chimeric antigen receptors (CARs), Hu07BBz and Hu08BBz, that recognized human IL-13Rα2, but not IL-13Rα1.The efficacy of such CAR may further be improved by PD-1 and TIM-3 blockade. Any of the CARs described in Yin et al. Mol Ther Oncolytics. 2018 Aug 28; 11:20-38. may be useful in the present disclosure.

In some instances, the CD19 CAR tertiary structure may be modified to improve safety profile of CD19 CAR T cell therapy using tertiary-structure-prediction program (Phrye2) (Kelley et al. 2009. Nat. Protoc. 4, 363-371. CAR with improved safety profile may include but are not limited to those with reduced ability to produce cytokines. In some instances, the CARs with improved safety profile may include the CD19-BBz CAR variant contains an 86-amino-acid fragment from human CD8α, comprising a longer extracellular-domain fragment (55 amino acids versus 45 amino acids in the CD19-BBz(prototype) and a longer intracellular sequence (7 amino acids versus 3 amino acids in CD19-BBz(prototype/ Kyrmirah TM)) as described by Ying et al. 2019 Nat Med. doi: 10.1038/s41591-019-0421-7. In another aspect, the payloads of the present disclosure may be the CD19-BBz (86) variant CAR co-expressed with truncated, nonfunctional epidermal growth factor receptor (tEGFR) as described by Ying et al. 2019.

### Extracellular targeting domain/moiety

In accordance with the disclosure, the extracellular target moiety of a CAR may be any agent that recognizes and binds to a given target molecule, for example, a neoantigen on tumor cells, with high specificity and affinity. The target moiety may be an antibody and variants thereof that specifically binds to a target molecule on tumor cells, or a peptide aptamer selected from a random sequence pool based on its ability to bind to the target molecule on tumor cells, or a variant or fragment thereof that can bind to the target molecule on tumor cells, or an antigen recognition domain from native T- cell receptor (TCR) (e.g. CD4 extracellular domain to recognize HIV infected cells), or exotic recognition components such as a linked cytokine that leads to recognition of target cells bearing the cytokine receptor, or a natural ligand of a receptor.

In some instances, the targeting domain of a CAR may be a Ig NAR, a Fab fragment, a Fab' fragment, a F(ab)'2 fragment, a F(ab)'3 fragment, Fv, a single chain variable fragment (scFv), a bis-scFv, a (scFv)2, a minibody, a diabody, a tribody, a tetrabody, a disulfide stabilized Fv protein (dsFv), a unibody, a nanobody, or an antigen binding region derived from an antibody that specifically recognizes a target molecule, for example a tumor specific antigen (TSA). In one instance, the targeting moiety is a scFv antibody. The scFv domain, when it is expressed on the surface of a CAR T cell and subsequently binds to a target protein on a cancer cell, is able to maintain the CAR T cell in proximity to the cancer cell and to trigger the activation of the T cell. A scFv can be generated using routine recombinant DNA technology techniques and is discussed in the present disclosure.

In one instance, the targeting moiety of a CAR construct may be an aptamer such as a peptide aptamer that specifically binds to a target molecule of interest. The peptide aptamer may be selected from a random sequence pool based on its ability to bind to the target molecule of interest.

In some instances, the targeting moiety of a CAR construct may be a natural ligand of the target molecule, or a variant and/or fragment thereof capable of binding the target molecule. In some aspects, the targeting moiety of a CAR may be a receptor of the target molecule, for example, a full-length human CD27, as a CD70 receptor, may be fused in frame to the signaling domain of CD3 ζ forming a CD27 chimeric receptor as an immunotherapeutic agent for CD70-positive malignancies (See, e.g., US patent publication NO.: US20130323214).

In some instances, the targeting moiety of a CAR may recognize a tumor specific antigen (TSA), for example a cancer neoantigen which is restrictedly expressed on tumor cells.

As non-limiting examples, the CAR of the present disclosure may comprise the extracellular targeting domain capable of binding to a tumor specific antigen selected from 5T4, 707-AP, A33, AFP (α-fetoprotein), AKAP-4 (A kinase anchor protein 4), ALK, α5β1-integrin, androgen receptor, annexin II, alpha- actinin-4, ART-4, B1, B7H3, B7H4, BAGE (B melanoma antigen), BCMA, BCR-ABL fusion protein, beta-catenin, BKT-antigen, BTAA, CA-I (carbonic anhydrase I), CA50 (cancer antigen 50), CA125, CA15-3, CA195, CA242, calretinin, CAIX (carbonic anhydrase), CAMEL (cytotoxic T-lymphocyte recognized antigen on melanoma), CAM43, CAP-1, Caspase-8/m, CD4, CD5, CD7, CD19, CD20, CD22, CD23, CD25, CD27/m, CD28, CD30, CD33, CD34, CD36, CD38, CD40/CD154, CD41, CD44v6, CD44v7/8, CD45,CD49f, CD56, CD68\KP1, CD74, CD79a/CD79b, CD103, CD123, CD133, CD138, CD171, cdc27/m, CDK4 (cyclin dependent kinase 4), CDKN2A, CDS, CEA (carcinoembryonic antigen), CEACAM5, CEACAM6, chromogranin, c-Met, c-Myc, coa-1, CSAp, CT7, CT10, cyclophilin B, cyclin B1, cytoplasmic tyrosine kinases, cytokeratin, DAM-10, DAM-6, dek-can fusion protein, desmin, DEPDC1 (DEP domain containing 1), E2A-PRL, EBNA, EGF-R (epidermal growth factor receptor), EGP-1 (epithelial glycoprotein -1) (TROP-2), EGP-2, EGP-40, EGFR (epidermal growth factor receptor), EGFRvIII, EF-2, ELF2M, EMMPRIN, EpCAM (epithelial cell adhesion molecule), EphA2, Epstein Barr virus antigens, Erb (ErbB 1; ErbB3; ErbB4), ETA (epithelial tumor antigen), ETV6-AML1 fusion protein, FAP (fibroblast activation protein), FBP (folate-binding protein), FGF-5, folate receptor α, FOS related antigen 1, fucosyl GM1, G250, GAGE (GAGE-1; GAGE-2), galactin, GD2 (ganglioside), GD3, GFAP (glial fibrillary acidic protein), GM2 (oncofetal antigenimmunogenic-1; OFA-I-1), GnT-V, Gp100, H4-RET, HAGE (helicase antigen), HER-2/neu, HIFs (hypoxia inducible factors), HIF-1α, HIF-2α, HLA-A2, HLA-A*0201-R170I, HLA-All, HMWMAA, Hom/Mel-40, HSP70-2M (Heat shock protein 70), HST-2, HTgp-175, hTERT (or hTRT), human papillomavirus-E6/human papillomavirus-E7 and E6, iCE (immune-capture EIA), IGF-1R, IGH-IGK, IL2R, IL5, ILK (integrin-linked kinase), IMP3 (insulin-like growth factor II mRNA-binding protein 3), IRF4 (interferon regulatory factor 4), KDR (kinase insert domain receptor), KIAA0205, KRAB-zinc finger protein (KID)-3; KID31, KSA (17-1A), K-ras, LAGE, LCK, LDLR/FUT (LDLR-fucosyltransferaseAS fusion protein), LeY (Lewis Y), MAD-CT-1, MAGE (tyrosinase, melanoma-associated antigen) (MAGE-1; MAGE-3), melan-A tumor antigen (MART), MART-2/Ski, MC1R (melanocortin 1 receptor), MDM2, mesothelin, MPHOSPH1, MSA(muscle-specific actin), mTOR (mammalian targets of rapamycin), MUC-1, MUC-2, MUM-1 (melanoma associated antigen (mutated) 1), MUM-2, MUM-3, Myosin/m, MYL-RAR, NA88-A, N-acetylglucosaminyltransferase, neo-PAP, NF-KB (nuclear factor-kappa B), neurofilament, NSE (neuron- specific enolase), Notch receptors, NuMa, N-Ras, NY-BR-1, NY- CO-1, NY-ESO-1, OncostatinM, OS-9, OY-TES1, p53 mutants, p190 minor bcr-abl, pl5(58), pl85erbB2, pl80erbB-3, PAGE (prostate associated gene), PAP (prostatic acid phosphatase), PAX3, PAX5, PDGFR (platelet derived growth factor receptor), cytochrome P450 involved in piperidine and pyrrolidine utilization (PIPA), Pml-RAR alpha fusion protein, PR-3 (proteinase 3), PSA (prostate specific antigen), PSM, PSMA (Prostate stem cell antigen), PRAME (preferentially expressed antigen of melanoma), PTPRK, RAGE (renal tumor antigen), Raf (A-Raf, B-Raf and C-Raf), Ras, receptor tyrosine kinases, RCAS1, RGSS, ROR1 (receptor tyrosine kinase-like orphan receptor 1), RU1, RU2, SAGE, SART-1, SART-3, SCP-1, SDCCAG16, SP-17 (sperm protein 17), src-family, SSX (synovial sarcoma X breakpoint)-1, SSX-2(HOM-MEL-40), SSX-3, SSX-4, SSX-5, STAT-3, STAT-5, STAT-6, STEAD, STn, survivin, syk-ZAP70, TA-90 (Mac-2 binding protein\cyclophilin C-associated protein), TAAL6, TACSTD1 (tumor associated calcium signal transducer 1), TACSTD2, TAG-72-4, TAGE, TARP (T cell receptor gamma alternate reading frame protein), TEL/AML1 fusion protein, TEM1, TEM8 (endosialin or CD248), TGFβ, TIE2, TLP, TMPRSS2 ETS fusion gene, TNF-receptor (TNF-α receptor, TNF-β receptor; or TNF-γ receptor), transferrin receptor, TPS, TRP-1 (tyrosine related protein 1), TRP-2, TRP-2/INT2, TSP-180, VEGF receptor, WNT, WT-1 (Wilm's tumor antigen) and XAGE.

In some instances, the CAR of the present disclosure may comprise a universal immune receptor which has a targeting moiety capable of binding to a labelled antigen. Methods of generating universal immune receptor CAR are discussed in International Patent Publication NO.: WO2013044225A1.

In some instances, the CAR of the present disclosure may comprise the targeting moiety capable of binding to a pathogen antigen.

In some instances, the CAR of the present disclosure may comprise the targeting moiety capable of binding to non-protein molecules such as tumor-associated glycolipids and carbohydrates. TSAs may also be lipid molecules, polysaccharides, saccharides, nucleic acids, haptens, carbohydrate, or the combinations thereof.

In some instances, the CAR of the present disclosure may comprise the targeting moiety capable of binding to a component within the tumor microenvironment including proteins expressed in various tumor stroma cells including tumor associated macrophages (TAMs), immature monocytes, immature dendritic cells, immunosuppressive CD4⁺CD25⁺ regulatory T cells (Treg) and MDSCs. A recent study using an animal model, demonstrated that after systemic transplantation, T cells expressing VEGFR-2 CAR and IL12 infiltrated the tumors, expanded and persisted within tumor mass leading to tumor regression. The anti-tumor effect was dependent on targeting of IL12-responsive host cells via activation of VEGFR-2 CAR-T cells and release of IL12 (Chinnasamy et al., Clinical Cancer Research, 2012, 18: 1672-1683).

In some instances, the CAR of the present disclosure may comprise the targeting moiety capable of binding to a cell surface adhesion molecule, a surface molecule of an inflammatory cell that appears in an autoimmune disease, or a TCR causing autoimmunity.

As non-limiting examples, the targeting moiety of the present disclosure may be a scFv antibody that recognizes a tumor specific antigen (TSA), for example scFvs of antibodies SS, SS1 and HN1 that specifically recognize and bind to human mesothelin (US Pat. NO.: 9,359, 447), scFv of antibody of GD2 (US Pat. NO.: 9,315,585), a CD19 antigen binding domain (U.S. Pat. NO.: 9,328,156); a NKG2D ligand binding domain (U.S. Pat. NO.: 9, 273,283; US patent publication NO.: US20160311906A1); human anti-mesothelin scFvs comprising the amino acid sequences of SEQ ID Nos.: 11 and 12 of US Pat. 9,272,002; an anti-CS1 binding agent (US patent publication NO.: US20160075784); an anti-BCMA binding domain (International Patent Publication NO.: WO2016/014565); anti-CD19 scFv antibody of SEQ ID NO:: 20 in US Pat. NO.: 9,102,761; GFR alpha 4 antigen binding fragments having the amino acid sequences of SEQ ID NOs: 59 and 79 of International patent publication NO.: 2016/025880; anti-CLL-1 (C-type lectin-like molecule 1) binding domains having the amino acid sequences of SEQ ID N.O:47, 44, 48, 49, 50, 39, 40, 41, 42, 43, 45, 46, 51, 73, 70, 74, 75, 76, 65, 66, 67, 68, 69, 71, 72, 77,195, 86, 83, 87, 88, 89, 78, 79, 80, 81, 82, 84, 85, 90 and 196 of International Patent Publication NO.: WO2016014535); CD33 binding domains having the amino acid sequences of SEQ ID NOs: 39-46 of International patent publication NO.: WO2016014576; a GPC3 (glypican-3) binding domain (SEQ ID NO:: 2 and SEQ ID NO:: 4 of International patent publication NO.: WO2016036973); a GFR alpha4 (Glycosyl-phosphatidylinositol (GPI)-linked GDNF family α -receptor 4 cell-surface receptor) binding domain (International Patent Publication NO.: WO2016025880); CD123 binding domains having the amino acid sequences of SEQ ID NOs: 480, 483, 485, 478, 158, 159, 160, 157, 217, 218, 219, 216, 276, 277, 278, and 275 of International patent publication NO.: WO20160258896; an anti-ROR1 antibody or fragments thereof (International patent publication NO.: WO2016016344); scFvs specific to GPC-3 (SEQ ID NOs: 1 and 24 of International patent publication NO.: WO2016049459); scFv for CSPG4 (SEQ ID NO:: 2 of International patent publication NO.: WO2015080981; scFv for folate receptor alpha (US Patent Publication NO.: US20170002072A1).

In some instances, natural ligands may be used as the targeting moieties of the CARs of the present disclosure. Such natural ligands may be capable of binding to the antigens with affinity in the range of the scFvs and can redirect T cells specificity and effector functions to target cells expressing the complementary receptor. In some instances, the targeting moiety of the CAR may be neuregulin-1 (NRG1) which is a natural ligand for HER3 and HER4; VEGF which is a natural ligand of VEGFR; IL13 wildtype protein or IL13 mutein e.g., E13Y which binds to IL13Ra2; NKG2D ligand, which is a natural ligand of NKG2D receptor; CD70 which is ligand of CD27; and a proliferation-inducing ligand (APRIL) which is a natural high affinity ligand for BCMA8 and transmembrane activator and CAML interactor (TACI). Any of the ligand based BCMA CARs taught in the US Patent Publication No. US20160362467A1.

In some instances, the targeting moieties of the present disclosure may be scFv comprising the amino acid sequences in Table 11 of co-owned U.S. Provisional Patent Application No. 62/683,972, filed June 12, 2018, and the International Patent Application No. PCT/US18/37005 filed on June 12, 2018. SEQ ID NO: 5474 to SEQ ID NO: 6571 describe scFv that may be utilized in the payloads described herein.

In one instance, the targeting moiety of the CAR may recognize CD19. CD19 is a well-known B cell surface molecule, which upon B cell receptor activation enhances B-cell antigen receptor induced signaling and expansion of B cell populations. CD 19 is broadly expressed in both normal and neoplastic B cells. Malignancies derived from B cells such as chronic lymphocytic leukemia, acute lymphocytic leukemia and many non-Hodgkin lymphomas frequently retain CD19 expression. This near universal expression and specificity for a single cell lineage has made CD19 an attractive target for immunotherapies. Human CD19 has 14 exons wherein exon 1-4 encode the extracellular portion of the CD19, exon 5 encodes the transmembrane portion of CD19 and exons 6-14 encode the cytoplasmic tail. In one instance, the targeting moiety may comprise scFvs derived from the variable regions of the FMC63 antibody. FMC63 is an IgG2a mouse monoclonal antibody clone specific to the CD19 antigen that reacts with CD19 antigen on cells of the B lineage. The epitope of CD19 recognized by the FMC63 antibody is in exon 2 (Sotillo et al (2015) Cancer Discov ;5(12): 1282-95). In some instances, the targeting moiety of the CAR may be derived from the variable regions of other CD19 monoclonal antibody clones including but not limited to 4G7, SJ25C1, CVID3/429, CVID3/155, HIB19, and J3-119.

In some instances, the targeting moiety of a CAR may recognize a tumor specific antigen (TSA), for example a cancer neoantigen that is only expressed by tumor cells because of genetic mutations or alterations in transcription which alter protein coding sequences, therefore creating novel, foreign antigens. The genetic changes result from genetic substitution, insertion, deletion or any other genetic changes of a native cognate protein (i.e. a molecule that is expressed in normal cells). In the context of CD 19, TSAs may include a transcript variant of human CD19 lacking exon 2 or lacking exon 5-6 or both (see International patent publication No. WO2016061368). Since FMC63 binding epitope is in exon 2, CD19 lacking exon 2 is not recognized by FMC63 antibody. Thus, in some instances, the targeting moiety of the CAR may be an FMC63-distinct scFV. As used herein "FMC63-distinct" refers, to an antibody, scFv or a fragment thereof that is immunologically specific and binds to an epitope of the CD19 antigen that is different or unlike the epitope of CD19 antigen that is bound by FMC63. In some instances, targeting moiety may recognize a CD19 antigen lacking exon2. In one instance, the targeting moiety recognizes a fragment of CD19 encoded by exon 1, 3 and/or 4. In one example, the targeting moiety recognizes the epitope that bridges the portion of CD19 encoded by exon 1 and the portion of CD19 encoded by exon 3.

### Intracellular signaling domains

The intracellular domain of a CAR fusion polypeptide, after binding to its target molecule, transmits a signal to the immune effector cell, activating at least one of the normal effector functions of immune effector cells, including cytolytic activity (e.g., cytokine secretion) or helper activity. Therefore, the intracellular domain comprises an "intracellular signaling domain" of a T cell receptor (TCR).

In some aspects, the entire intracellular signaling domain can be employed. In other aspects, a truncated portion of the intracellular signaling domain may be used in place of the intact chain as long as it transduces the effector function signal.

In some instances, the intracellular signaling domain of the present disclosure may contain signaling motifs which are known as immunoreceptor tyrosine-based activation motifs (ITAMs). Examples of ITAM containing cytoplasmic signaling sequences include those derived from TCR CD3zeta, FcR gamma, FcR beta, CD3 gamma, CD3 delta, CD3 epsilon, CD5, CD22, CD79a, CD79b, and CD66d. In one example, the intracellular signaling domain is a CD3 zeta (CD3ζ) signaling domain.

In some instances, the intracellular region of the present disclosure further comprises one or more costimulatory signaling domains which provide additional signals to the immune effector cells. These costimulatory signaling domains, in combination with the signaling domain can further improve expansion, activation, memory, persistence, and tumor-eradicating efficiency of CAR engineered immune cells (e.g., CAR T cells). In some cases, the costimulatory signaling region contains 1, 2, 3, or 4 cytoplasmic domains of one or more intracellular signaling and /or costimulatory molecules. The costimulatory signaling domain may be the intracellular/cytoplasmic domain of a costimulatory molecule, including but not limited to CD2, CD7, CD27, CD28, 4-1BB (CD137), OX40 (CD134), CD30, CD40, ICOS (CD278), GITR (glucocorticoid-induced tumor necrosis factor receptor), LFA-1 (lymphocyte function-associated antigen-1), LIGHT, NKG2C, B7-H3. In one example, the costimulatory signaling domain is derived from the cytoplasmic domain of CD28. In another example, the costimulatory signaling domain is derived from the cytoplasmic domain of 4-1BB (CD137). In another example, the co-stimulatory signaling domain may be an intracellular domain of GITR as taught in U.S. Pat. NO.: 9, 175, 308.

In some instances, the intracellular region of the present disclosure may comprise a functional signaling domain from a protein selected from the group consisting of an MHC class I molecule, a TNF receptor protein, an immunoglobulin-like protein, a cytokine receptor, an integrin, a signaling lymphocytic activation protein (SLAM) such as CD48, CD229, 2B4, CD84, NTB-A, CRACC, BLAME,CD2F-10, SLAMF6, SLAMF7, an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), SLAMF7, NKp80 (KLRF1), NKp44, NKp30, NKp46, CD19, CD4, CD8alpha, CD8beta, IL2R beta, IL2R gamma, IL7R alpha, IL15Ra, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NKD2C SLP76, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, CD270 (HVEM), GADS, SLP-76, PAG/Cbp, CD19a, a ligand that specifically binds with CD83, DAP 10, TRIM, ZAP70, Killer immunoglobulin receptors (KIRs) such as KIR2DL1, KIR2DL2/L3, KIR2DL4, KIR2DL5A, KIR2DL5B, KIR2DS1, KIR2DS2, KIR2DS3, KIR2DS4, KIR2DS5, KIR3DL1/S1, KIR3DL2, KIR3DL3, and KIR2DP1; lectin related NK cell receptors such as Ly49, Ly49A, and Ly49C.

In some instances, the intracellular signaling domain of the present disclosure may contain signaling domains derived from JAK-STAT. In other instances, the intracellular signaling domain of the present disclosure may contain signaling domains derived from DAP-12 (Death associated protein 12) (Topfer et al., Immunol., 2015, 194: 3201-3212; and Wang et al., Cancer Immunol., 2015, 3: 815-826). DAP-12 is a key signal transduction receptor in NK cells. The activating signals mediated by DAP-12 play important roles in triggering NK cell cytotoxicity responses toward certain tumor cells and virally infected cells. The cytoplasmic domain of DAP12 contains an Immunoreceptor Tyrosine-based Activation Motif (ITAM). Accordingly, a CAR containing a DAP12-derived signaling domain may be used for adoptive transfer of NK cells.

In some instances, T cells engineered with two or more CARs incorporating distinct co-stimulatory domains and regulated by distinct DD may be used to provide kinetic control of downstream signaling.

In some instances, the payload of the disclosure may be any of the co-stimulatory molecules and/or intracellular domains described herein. In some instances, one or more co-stimulatory molecules, each under the control of different SRE may be used in the present disclosure. SRE regulated co- stimulatory molecules may also be expressed in conjunction with a first-generation CAR, a second-generation CAR, a third-generation CAR, a fourth-generation, or any other CAR design described herein.

In some instances, the intracellular domain of the present disclosure may comprise amino acid sequences of Table 12 of co-owned U.S. Provisional Patent Application No. 62/683,972, filed June 12, 2018, and the International Patent Application No. PCT/US18/37005 filed on June 12, 2018. Exemplary intracellular signaling domains and costimulatory domains are described as SEQ ID NO: 6572 to SEQ ID NO: 6678.

### Transmembrane domains

In some instances, the CAR of the present disclosure may comprise a transmembrane domain. As used herein, the term "Transmembrane domain (TM)" refers broadly to an amino acid sequence of about 15 residues in length which spans the plasma membrane. More preferably, a transmembrane domain includes at least 20, 21, 22, 23, 24, 25, 26, 27, 28, 29, 30, 31, 32, 33, 34, 35, 36, 37, 38, 39, 40, 41, 42, 43, 44, or 45 amino acid residues and spans the plasma membrane. In some instances, the transmembrane domain of the present disclosure may be derived either from a natural or from a synthetic source. The transmembrane domain of a CAR may be derived from any naturally membrane-bound or transmembrane protein. For example, the transmembrane region may be derived from (i.e. comprise at least the transmembrane region(s) of) the alpha, beta or zeta chain of the T-cell receptor, CD3 epsilon, CD4, CD5, CD8, CD8α, CD9, CD16, CD22, CD33, CD28, CD37, CD45, CD64, CD80, CD86, CD134, CD137, CD152, or CD154.

Alternatively, the transmembrane domain of the present disclosure may be synthetic. In some aspects, the synthetic sequence may comprise predominantly hydrophobic residues such as leucine and valine.

In some instances, the transmembrane domain of the present disclosure may be selected from the group consisting of a CD8a transmembrane domain, a CD4 transmembrane domain, a CD 28 transmembrane domain, a CTLA-4 transmembrane domain, a PD-1 transmembrane domain, and a human Ig_{G4} Fc region. As non-limiting examples, the transmembrane domain may be a CTLA-4 transmembrane domain comprising the amino acid sequences of SEQ ID NOs.: 1-5 of International Patent Publication NO.: WO2014/100385; and a PD-1 transmembrane domain comprising the amino acid sequences of SEQ ID NOs.: 6-8 of International Patent Publication NO.: WO2014100385.

In some instances, the CAR of the present disclosure may comprise an optional hinge region (also called spacer). A hinge sequence is a short sequence of amino acids that facilitates flexibility of the extracellular targeting domain that moves the target binding domain away from the effector cell surface to enable proper cell/cell contact, target binding and effector cell activation (Patel et al., Gene Therapy, 1999; 6: 412-419). The hinge sequence may be positioned between the targeting moiety and the transmembrane domain. The hinge sequence can be any suitable sequence derived or obtained from any suitable molecule. The hinge sequence may be derived from all or part of an immunoglobulin (e.g., IgG1, IgG2, IgG3, IgG4) hinge region, i.e., the sequence that falls between the CHI and CH2 domains of an immunoglobulin, e.g., an IgG4 Fc hinge, the extracellular regions of type 1 membrane proteins such as CD8a CD4, CD28 and CD7, which may be a wild type sequence or a derivative. Some hinge regions include an immunoglobulin CH3 domain or both a CH3 domain and a CH2 domain. In certain instances, the hinge region may be modified from an IgG1, IgG2, IgG3, or IgG4 that includes one or more amino acid residues, for example, 1, 2, 3, 4 or 5 residues, substituted with an amino acid residue different from that present in an unmodified hinge. Table 13 of co-owned U.S. Provisional Patent Application No. 62/683,972 filed June 12, 2018, and the International Patent Application No. PCT/US18/37005 filed on June 12, 2018. and SEQ ID NO: 6679 to SEQ ID NO: 6811 provide various transmembrane regions that can be used in the CARs described herein.

Hinge region sequences useful in the present disclosure are provided in Table 14 of co-owned U.S. Provisional Patent Application No. 62/683,972, filed June 12,2018, and the International Patent Application No. PCT/US18/37005 filed on June 12, 2018 and SEQ ID NO: 6730 to SEQ ID NO: 6806.

In some instances, hinge domains may be present at the N terminus of the transmembrane domain, at C terminus of the transmembrane domain or within the transmembrane domain. Hinge and transmembrane region sequences which may be useful in the present disclosure are provided in Table 15 of co-owned U.S. Provisional Patent Application No. 62/683,972, filed June 12,2018, and the International Patent Application No. PCT/US18/37005 filed on June 12, 2018 and as SEQ ID NO: 6807 to SEQ ID NO:6811.

In some instances, the CAR of the present disclosure may comprise one or more linkers between any of the domains of the CAR. The linker may be between 1-30 amino acids long. In this regard, the linker may be 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids in length. In other instances, the linker may be flexible.

In some instances, the components including the targeting moiety, transmembrane domain and intracellular signaling domains of the present disclosure may be constructed in a single fusion polypeptide. The fusion polypeptide may be the payload of an effector module of the disclosure. In some instances, more than one CAR fusion polypeptides may be included in an effector module, for example, two, three or more CARs may be included in the effector module under the control of a single SRE (e.g., a DD). Representative effector modules comprising the CAR payload are illustrated in Figures 2-6.

In some instances, payloads of the present disclosure may comprise the CAR constructs including the extracellular targeting domain, transmembrane domain and intracellular signaling domains that are taught in the art, for example, a CAR targeting mesothelin (US Pat. NOs: 9,272, 002 and 9, 359,447); EGFRvIII specific CARs in US Pat. NO.: 9,266,960; anti-TAG CARs in US Pat. NO.: 9,233,125; CD19 CARs in US Patent publication NO.: 2016014533; CD19 CAR having the amino acid sequence of SEQ ID NO:: 24 of US Pat. NO.: 9, 328, 156; CD19 CARs in US Pat NOs: 8,911,993; 8,975,071; 9,101,584; 9,102,760; and 9,102,761; BCMA (CD269) specific CARs disclosed in International patent publication NOs: WO2016/014565 (SEQ ID Nos.: 109-113 and 213 to 233) and WO2016/014789; CLL-1(C-type lectin-like molecule 1) CARs comprising the amino acid sequences of SEQ ID NOs: 99, 96, 100, 101, 102, 91, 92, 93, 94, 95, 97, 98, 103, and 197 disclosed in International patent publication NO.: WO2016014535; CD33 specific CARs comprising the amino acid sequences of SEQ ID NOs: 48-56 in International patent publication NO.: WO2016014576; CD33 specific CARs comprising the amino acid sequences of SEQ ID NOs: 19-22, 27-30 and 35-38 in International patent publication NO.: WO2015150526; CD37 specific CARs encoded by the nucleic acids of SEQ ID NOs: 1-5 in US patent publication NO.: US20150329640; GPC3 CAR (International patent publication NO.: WO2016036973), GFR alpha 4 CARs having the amino acid sequences of SEQ ID NOs: 85, 86, 90, 92, 94, 96, 98,100,102, and 104 in International patent publication NO.: WO2016025880; CD123 CARs comprising the amino acid sequences of SEQ ID NO: 98, 99, 100 and 101 in International patent publication NOs: WO2016028896; CD123 specific multi-chain CARs in International patent publication NO: WO2015193406; CD123 CARS comprising the amino acid sequences of SEQ ID NO:: 160,171, 188-197 in International patent publication NO: WO2016/120220; ROR-1 specific CARs comprising the amino acid sequences of SEQ ID NOs: 93, 95 and 117 in International patent publication NO.: WO2016/016344; ROR-1 specific multi-chain CARs in International patent publication NO.: WO2016/016343; trophoblast glycoprotein (5T4, TPBG) specific CARs comprising the amino acid sequences of SEQ ID NOs: 21,27,33,39,23,29,34,41,19,25, 31, 37, 20, 26, 32, 38, 22, 28, 34, 40, 24, 30, 36 and 42 in International patent publication NO.: WO2016034666; EGFRvIII specific CARs comprising the amino acid sequences of SEQ ID NOs: 15, 17, 24, 25, 26 and 27 in International patent publication NO.: WO2016016341; a TEM 8 CAR comprising the amino acid sequence of SEQ ID NO: 1 in International patent publication NO.: WO2014164544, a TEMl CAR comprising the amino acid sequence of SEQ ID NO:2 in International patent publication NO.: WO2014164544; GPC-3 CAR having the amino acid sequences of SEQ ID NOs: 3 and 26 in International patent publication NO.: WO2016/049459; a chondroitin sulfate proteoglycan-4 (CSPG4) CAR in International patent publication NO.: WO2015/080981; Kappa/lambda CARs in International patent publication NO.: WO2015/164739; GD2 CAR in International patent publication NO.: WO2016/134284; CLL1 CARs in International patent publication NO.: WO2016120218; CLL1 multi-subunit CARs in International patent publication NO.: WO2016120219; Hsp70 CARs in International patent publication NO.: WO2016120217; mAb-driven CARs in International patent publication NO.: WO2016120216.

In some instances, the CAR constructs of the present disclosure may include CAIX (carboxyanhydrase-IX (CAIX) specific CAR (Lamers et al., Biochem Soc Trans, 2016, 44(3): 951-959), HIV-1 specific CAR (Ali et al., J Virol., 2016, May 25, pii: JVI.00805-16), CD20 specific CAR (Rufener et al., Cancer Immunol. Res., 2016, 4(6): 509-519), a CD20/CD19 bispecific CAR (Zah et al., Cancer Immunol Res., 2016, 4(6): 498-508), a CD22/CD19 CAR (International Publication No: WO2016/149578), a CD138/BCMA bi-specific CAR (International Publication No: WO2016/130598) an EGFR specific CARs and anti EGFR viii specific CAR.

In some instances, the chimeric antigen receptors described herein may include CD3 zeta domains altered to tune CAR activity. The CD3 zeta domains may include one or more mutations in the immunoreceptor tyrosine-based activation motifs (ITAMs). In one aspect, the tyrosine residues within the ITAMs may be mutated resulting in reduced phosphorylation and limited downstream signaling. In some instances, one or more of the ITAMs may be deleted from the CD3 zeta domain. In one aspect, the CD3 zeta may include one ITAM. Any of the CARs and CD3 zeta domains described by Feucht et al. 2019 may be used herein (Calibration of CAR activation potential directs alternative T cell fates and therapeutic potency. Nature Medicine 25, 82-88 (2019)).

In some instances, the CAR sequences may be selected from Table 16 of co-owned U.S. Provisional Patent Application No. 62/683,972, filed June 12,2018, and the International Patent Application No. PCT/US18/37005 filed on June 12,2018 or from SEQ ID NO:6812 to SEQ ID NO: 8232.

In one instance of the present disclosure, the payload of the disclosure is a CD19 specific CAR targeting different B cell malignancies and HER2-specific CAR targeting sarcoma, glioblastoma, and advanced Her2-positive lung malignancy.

In some instances, the CAR is a CD19 CAR. The amino acid sequences of CD19 CAR components and CD19 CAR constructs are presented in Table 17 and Table 18.

**Table 17: CD19 CAR construct components**

| **Description** | **Amino Acid Sequence** | **AA SEQ ID NO** | **NA SEQ ID NO** |
|---|---|---|---|
| CD19 scFv | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEITGG GGSGGGGSGGGGSEVKLQESGPGLVAPSQSLSVTCTVSGVSLPDYGVSWIRQP PRKGLEWLGVIWGSETTYYNSALKSRLTIIKDNSKSQVFLKMNSLQTDDTAIYY CAKHYYYGGSYAMDYWGQGTSVTVSS | 8233 | 8241-8246 |
| CD8α hinge | TTTPAPRPPTPAPTIASQPLSLRPEACRPAAGGAVHTRGLDFACD | 8234 | 8247-8251 |
| CD8α hinge-TM (hinge and transmembrane) | TTTPAPRPPTPAPTIASQPLSLRPEACRPAAGGAVHTRGLDFACDIYIWAPLAGT CGVLLLSLVITLYC | 8235 | 8252-8254 |
| CD3 zeta signaling domain | RVKFSRSADAPAYKQGQNQLYNELNLGRREEYDVLDKRRGRDPEMGGKPRR KNPQEGLYNELQKDKMAEAYSEIGMKGERRRGKGHDGLYQGLSTATKDTYD ALHMQALPPR | 8236 | 8255-8261 |
| 4-1BB intracellular signaling domain; CD28 costimulatory domain; | KRGRKKLLYIFKQPFMRPVQTTQEEDGCSCRFPEEEEGGCEL | 8237 | 8262-8267 |
| CD8α leader | MALPVTALLLPLALLLHAARP | 278 | ₂₇₉-₂₈₃ |
| hPDE5 (Amino acid 535-860 of WT) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSHDLDHRGVNNSYIQRSEHPLAQLYCHSIMEHHHFDQCLMIL NSPGNQILSGLSIEEYKTTLKIIKQAILATDLALYIKRRGEFFELIRKNQFNLEDPH QKELFLAMLMTACDLSAITKPWPIQQRIAELVATEFFDQGDRERKELNIEPTDL | 3 | 339 |
| | MNREKKNKIPSMQVGFIDAICLQLYEALTHVSEDCFPLLDGCRKNRQKWQALA EQQ | | |
| hPDE5 (Amino acid 535-860 of WT, R732L) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSHDLDHRGVNNSYIQRSEHPLAQLYCHSIMEHHHFDQCLMIL NSPGNQILSGLSIEEYKTTLKIIKQAILATDLALYIKRLGEFFELIRKNQFNLEDPH QKELFLAMLMTACDLSAITKPWPIQQRIAELVATEFFDQGDRERKELNIEPTDL MNREKKNKIPSMQVGFIDAICLQLYEALTHVSEDCFPLLDGCRKNRQKWQALA EQQ | 12 | 359 |
| hPDE5 (Amino acid 535-860 of WT, R732L, D764N) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSHDLDHRGVNNSYIQRSEHPLAQLYCHSIMEHHHFDQCLMIL NSPGNQILSGLSIEEYKTTLKIIKQAILATDLALYIKRLGEFFELIRKNQFNLEDPH QKELFLAMLMTACNLSAITKPWPIQQRIAELVATEFFDQGDRERKELNIEPTDL MNREKKNKIPSMQVGFIDAICLQLYEALTHVSEDCFPLLDGCRKNRQKWQALA EQQ | 505 | 520 |
| hPDE5 (Amino acid 535-860 of WT, R732L, F736A) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSHDLDHRGVNNSYIQRSEHPLAQLYCHSIMEHHHFDQCLMIL NSPGNQILSGLSIEEYKTTLKIIKQAILATDLALYIKRLGEFAELIRKNQFNLEDP HQKELFLAMLMTACDLSAITKPWPIQQRIAELVATEFFDQGDRERKELNIEPTD LMNREKKNKIPSMQVGFIDAICLQLYEALTHVSEDCFPLLDGCRKNRQKWQAL AEQQ | 227 | 233 |
| hPDE5 (Amino acid 535-860 of WT, H653A) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSADLDHRGVNNSYIQRSEHPLAQLYCHSIMEHHHFDQCLMIL NSPGNQILSGLSIEEYKTTLKIIKQAILATDLALYIKRRGEFFELIRKNQFNLEDPH QKELFLAMLMTACDLSAITKPWPIQQRIAELVATEFFDQGDRERKELNIEPTDL MNREKKNKIPSMQVGFIDAICLQLYEALTHVSEDCFPLLDGCRKNRQKWQALA EQQ | 348 | 361 |
| hPDE5 (Amino acid 535-860 of WT, R732L, H653A) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSADLDHRGVNNSYIQRSEHPLAQLYCHSIMEHHHFDQCLMIL NSPGNQILSGLSIEEYKTTLKIIKQAILATDLALYIKRLGEFFELIRKNQFNLEDPH QKELFLAMLMTACDLSAITKPWPIQQRIAELVATEFFDQGDRERKELNIEPTDL MNREKKNKIPSMQVGFIDAICLQLYEALTHVSEDCFPLLDGCRKNRQKWQALA EQQ | 509 | 524 |
| hPDE5 (Amino acid 535-860 of WT, R732L, D764A) | EETRELQSLAAAVVPSAQTLKITDFSFSDFELSDLETALCTIRMFTDLNLVQNFQ MKHEVLCRWILSVKKNYRKNVAYHNWRHAFNTAQCMFAALKAGKIQNKLT DLEILALLIAALSHDLDHRGVNNSYIQRSEHPLAQLYCHSIMEHHHFDQCLMIL NSPGNQILSGLSIEEYKTTLKIIKQAILATDLALYIKRLGEFFELIRKNQFNLEDPH QKELFLAMLMTACALSAITKPWPIQQRIAELVATEFFDQGDRERKELNIEPTDL MNREKKNKIPSMQVGFIDAICLQLYEALTHVSEDCFPLLDGCRKNRQKWQALA EQQ | 510 | 525 |
| HA Tag | YPYDVPDYA | 8238 | 8268 |
| P2A Cleavable peptide | GATNFSLLKQAGDVEENPGP | 8239 | 8270 |
| Modified Furin Cleavage Site | ESRRVRRNKRSK | 288 | 291 |
| P2A Cleavage Site | ATNFSLLKQAGDVEENPGP | 8742 | 8817 |
| eGFP (A206K) | MVSKGEELFTGVVPILVELDGDVNGHKFSVSGEGEGDATYGKLTLKFICTTGK LPVPWPTLVTTLTYGVQCFSRYPDHMKQHDFFKSAMPEGYVQERTIFFKDDGN YKTRAEVKFEGDTLVNRIELKGIDFKEDGNILGHKLEYNYNSHNVYIMADKQK NGIKVNFKIRHNIEDGSVQLADHYQQNTPIGDGPVLLPDNHYLSTQSKLSKDPN EKRDHMVLLEFVTAAGITLGMDELYK | 8921 | 8923 |
| eGFP (M1del) | VSKGEELFTGVVPILVELDGDVNGHKFSVSGEGEGDATYGKLTLKFICTTGKLP VPWPTLVTTLTYGVQCFSRYPDHMKQHDFFKSAMPEGYVQERTIFFKDDGNY KTRAEVKFEGDTLVNRIELKGIDFKEDGNILGHKLEYNYNSHNVYIMADKQKN | 8922 | 8924 |
| | GIKVNFKIRHNIEDGSVQLADHYQQNTPIGDGPVLLPDNHYLSTQSALSKDPNE KRDHMVLLEFVTAAGITLGMDELYK | | |
| Firefly Luciferase (N50D, N119G, S548I, K549A, L550V) | MEDAKNIKKGPAPFYPLEDGTAGEQLHKAMKRYALVPGTIAFTDAHIEVDITY AEYFEMSVRLAEAMKRYGLNTNHRIVVCSENSLQFFMPVLGALFIGVAVAPAN DIYNERELLNSMGISQPTVVFVSKKGLQKILNVQKKLPIIQKIIIMDSKTDYQGF QSMYTFVTSHLPPGFNEYDFVPESFDRDKTIALIMNSSGSTGLPKGVALPHRTA CVRFSHARDPIFGNQIIPDTAILSVVPFHHGFGMFTTLGYLICGFRVVLMYRFEE ELFLRSLQDYKIQSALLVPTLFSFFAKSTLIDKYDLSNLHEIASGGAPLSKEVGE AVAKRFHLPGIRQGYGLTETTSAILITPEGDDKPGAVGKVVPFFEAKVVDLDTG KTLGVNQRGELCVRGPMIMSGYVNNPEATNALIDKDGWLHSGDIAYWDEDEH FFIVDRLKSLIKYKGYQVAPAELESILLQHPNIFDAGVAGLPDDDAGELPAAVV VLEHGKTMTEKEIVDYVASQVTTAKKLRGGVVFVDEVPKGLTGKLDARKIREI LIKAKKGGKIAV | 223 | 224 |
| mCherry (M1L) | LSKGEEDNMAIIKEFMRFKVHMEGSVNGHEFEIEGEGEGRPYEGTQTAKLKVT KGGPLPFAWDILSPQFMYGSKAYVKHPADIPDYLKLSFPEGFKWERVMNFEDG GVVTVTQDSSLQDGEFIYKVKLRGTNFPSDGPVMQKKTMGWEASSERMYPED GALKGEIKQRLKLKDGGHYDAEVKTTYKAKKPVQLPGAYNVNIKLDITSHNE DYTIVEQYERAEGRHSTGGMDELYK | 8240 | 8269 |
| SG LINKER | SG | - | AGTGGT |
| Linker (GSG) (BamH1-Gly) | GSG | - | GGATCC GGA |
| Flexible G/S rich linker; BamH1 Site | GS | - | GGATCC |
| Lys-Asp Acid Linker | LD | - | CTAGAT |

**Table 18: CD19 CARs constructs**

| **Description** | **Amino Acid Sequence** | **Amino Acid SEQ ID NO** | **Nucleic Acid SEQ ID NO** |
|---|---|---|---|
| OT-001407 (CD8a leader; CD19 scFv; CD8a Hinge and Transmembrane Domain; 4-1BB intracellular domain; CD3 zeta signaling domain; stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPR* | 8271 | 8285 |
| OT-001258 (CD8a leader; CD19 scFV; CD8a-Tm; (4-1BB intracellular domain); CD3zeta signaling domain; Linker (GSG), hPDE5 (Amino acid 535-860 of WT, R732L, D764N), stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSGEETRELQSLAAA VVPSAQTLKITDFSFSDFELSDLETALCTIRMFTDLNLVQNFQMKHE VLCRWILSVKKNYRKNVAYHNWRHAFNTAQCMFAALKAGKIQN KLTDLEILALLIAALSHDLDHRGVNNSYIQRSEHPLAQLYCHSIMEH HHFDQCLMILNSPGNQILSGLSIEEYKTTLKIIKQAILATDLALYIKR | 8272 | 8286 |
| | LGEFFELIRKNQFNLEDPHQKELFLAMLMTACNLSAITKPWPIQQRI AELVATEFFDQGDRERKELNIEPTDLMNREKKNKIPSMQVGFIDAI CLQLYEALTHVSEDCFPLLDGCRKNRQKWQALAEQQ* | | |
| OT-001298 (CD8a leader; CD19 scFV; CD8a-Tm; (4-1BB intracellular domain); CD3zeta signaling domain; Linker (GSG); hPDE5 (Amino acid 535-860 of WT, R732L, F736A); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSGEETRELQSLAAA VVPSAQTLKITDFSFSDFELSDLETALCTIRMFTDLNLVQNFQMKHE VLCRWILSVKKNYRKNVAYHNWRHAFNTAQCMFAALKAGKIQN KLTDLEILALLIAALSHDLDHRGVNNSYIQRSEHPLAQLYCHSIMEH HHFDQCLMILNSPGNQILSGLSIEEYKTTLKIIKQAILATDLALYIKR LGEFAELIRKNQFNLEDPHQKELFLAMLMTACDLSAITKPWPIQQRI AELVATEFFDQGDRERKELNIEPTDLMNREKKNKIPSMQVGFIDAI CLQLYEALTHVSEDCFPLLDGCRKNRQKWQALAEQQ* | 8273 | 8287 |
| OT-001299 (CD8a leader; CD19 scFV; CD8a-Tm; (4-1BB intracellular domain); CD3zeta signaling domain; Linker (GSG); hPDE5(Amino acid 535-860 of WT); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSGEETRELQSLAAA VVPSAQTLKITDFSFSDFELSDLETALCTIRMFTDLNLVQNFQMKHE VLCRWILSVKKNYRKNVAYHNWRHAFNTAQCMFAALKAGKIQN KLTDLEILALLIAALSHDLDHRGVNNSYIQRSEHPLAQLYCHSIMEH HHFDQCLMILNSPGNQILSGLSIEEYKTTLKIIKQAILATDLALYIKR RGEFFELIRKNQFNLEDPHQKELFLAMLMTACDLSAITKPWPIQQRI AELVATEFFDQGDRERKELNIEPTDLMNREKKNKIPSMQVGFIDAI CLQLYEALTHVSEDCFPLLDGCRKNRQKWQALAEQQ* | 8274 | 8288 |
| OT-001300 (CD8a leader; HA Tag; CD19 scFV; CD8a-Tm; (4-1BB intracellular domain); CD3zeta signaling domain; Linker (SG); hPDE5 (Amino acid 535-860 of WT); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8275 | 8289 |
| OT-001301 (CD8a leader; HA Tag; CD19 scFV; CD8a-Tm; (4-1BB intracellular domain); CD3zeta signaling domain; linker (SG); hPDE5 (Amino acid 535-860 of WT, R732L); stop) | MALPVTALLLPLALLLHAARPYPYDVPDYADIQMTQTTSSLSASLG DRVTISCRASQDISKYLNWYQQKPDGTVKLLIYHTSRLHSGVPSRF | 8276 | 8290 |
| | SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRLGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | | |
| OT-001302 (CD8a leader; CD19 scFV; CD8a-Tm; (4-1BB intracellular domain); CD3zeta signaling domain; linker (GS); hPDE5 (Amino acid 535-860 of WT, R732L, F736A); linker (GS); mcherry (M1L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRL GEFAELIRKNQFNLEDPHQKELFLAMLMTACDLSAITKPWPIQQRI AELVATEFFDQGDRERKELNIEPTDLMNREKKNKIPSMQVGFIDAI CLQLYEALTHVSEDCFPLLDGCRKNRQKWQALAEQQGSLSKGEED NMAIIKEFMRFKVHMEGSVNGHEFEIEGEGEGRPYEGTQTAKLKV TKGGPLPFAWDILSPQFMYGSKAYVKHPADIPDYLKLSFPEGFKWE RVMNFEDGGVVTVTQDSSLQDGEFIYKVKLRGTNFPSDGPVMQK KTMGWEASSERMYPEDGALKGEIKQRLKLKDGGHYDAEVKTTYK AKKPVQLPGAYNVNIKLDITSHNEDYTIVEQYERAEGRHSTGGMD ELYK* | 8277 | 8291 |
| OT-001303 (CD8a leader; CD19 scFV; CD8a-Tm; (4-1BB intracellular domain); CD3zeta signaling domain; linker (GS); hPDE5 (Amino acid 535-860 of WT, R732L, F736A); linker (GSG); P2A linker; mcherry (M1L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRL GEFAELIRKNQFNLEDPHQKELFLAMLMTACDLSAITKPWPIQQRI AELVATEFFDQGDRERKELNIEPTDLMNREKKNKIPSMQVGFIDAI CLQLYEALTHVSEDCFPLLDGCRKNRQKWQALAEQQGSGGATNF SLLKQAGDVEENPGPLSKGEEDNMAIIKEFMRFKVHMEGSVNGHE | 8278 | 8292 |
| | FEIEGEGEGRPYEGTQTAKLKVTKGGPLPFAWDILSPQFMYGSKAY VKHPADIPDYLKLSFPEGFKWERVMNFEDGGVVTVTQDSSLQDGE FIYKVKLRGTNFPSDGPVMQKKTMGWEASSERMYPEDGALKGEIK QRLKLKDGGHYDAEVKTTYKAKKPVQLPGAYNVNIKLDITSHNED YTIVEQYERAEGRHSTGGMDELYK* | | |
| OT-001304 (CD8a leader; HA Tag; CD19 scFV; CD8a-Tm, (4-1BB intracellular domain); CD3zeta signaling domain; linker (GS); hPDE5(Amino acid 535-860 of WT); linker (GS), Spacer (LD); P2A linker; mcherry (M1L); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRGSEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ QGSLDGATNFSLLKQAGDVEENPGPLSKGEEDNMAIIKEFMRFKV HMEGSVNGHEFEIEGEGEGRPYEGTQTAKLKVTKGGPLPFAWDILS PQFMYGSKAYVKHPADIPDYLKLSFPEGFKWERVMNFEDGGVVT VTQDSSLQDGEFIYKVKLRGTNFPSDGPVMQKKTMGWEASSERM YPEDGALKGEIKQRLKLKDGGHYDAEVKTTYKAKKPVQLPGAYN VNIKLDITSHNEDYTIVEQYERAEGRHSTGGMDELYK* | 8279 | 8293 |
| OT-001305 (CD8a leader; HA Tag, CD19 scFV; CD8a-Tm; (4-1BB intracellular domain); CD3zeta signaling domain; Linker (GS); hPDE5(Amino acid 535-860 of WT, R732L); Linker (GS), Spacer (LD); P2A linker; mcherry (M1L); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRGSEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRLGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ QGSLDGATNFSLLKQAGDVEENPGPLSKGEEDNMAIIKEFMRFKV HMEGSVNGHEFEIEGEGEGRPYEGTQTAKLKVTKGGPLPFAWDILS PQFMYGSKAYVKHPADIPDYLKLSFPEGFKWERVMNFEDGGVVT VTQDSSLQDGEFIYKVKLRGTNFPSDGPVMQKKTMGWEASSERM YPEDGALKGEIKQRLKLKDGGHYDAEVKTTYKAKKPVQLPGAYN VNIKLDITSHNEDYTIVEQYERAEGRHSTGGMDELYK* | 8280 | 8294 |
| OT-001454 (CD8a leader; CD19 scFV; CD8a-Tm; (4-1BB intracellular domain); CD3zeta signaling domain; Linker (GS); hPDE5(Amino acid 535-860 of WT, R732L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL | 8281 | 8295 |
| | LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | | |
| OT-001455 (CD8a leader; CD19 scFV; CD8a-Tm; (4-1BB intracellular domain); CD3zeta signaling domain; Linker (GS); hPDE5(Amino acid 535-860 of WT, H653A); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSADLDHRGVNNSYIQRSEHPLAQLYCHSIMEHH HFDQCLMILNSPGNQILSGLSIEEYKTTLKIIKQAILATDLALYIKRR GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | 8282 | 8296 |
| OT-001456 (CD8a leader; CD19 scFV; CD8a-Tm; (4-1BB intracellular domain); CD3zeta signaling domain; Linker (GS); hPDE5(Amino acid 535-860 of WT, H653A, R732L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSADLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | 8283 | 8297 |
| OT-001457 (CD8a leader; CD19 scFV; CD8a-Tm; (4-1BB intracellular domain); CD3zeta signaling domain; Linker (GS); hPDE5(Amino acid 535-860 of WT, R732L, D764A); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK | 8284 | 8298 |
| | LTDLEILALLIAALSHDLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACALSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | | |
| OT-001897 (CD8a leader; CD19 scFv; CD8a Hinge and Transmembrane Domain; 4-1BB intracellular domain (CD28 co-stimulatory domain); CD3 zeta intracellular domain; Linker (GS); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGS* | 8925 | 8976 |
| OT-001896 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 AA 535-860 of WT (H653A, R732L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSADLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | 8926 | 8977 |
| OT-001907 (CD8a leader; HA Tag; CD19 scFv; CD8a Hinge and Transmembrane Domain; 4-1BB intracellular domain (CD28 co-stimulatory domain); CD3 zeta intracellular domain; Linker (GS); hPDE5 AA 535-860 of WT (R732L, F736A); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRGSEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRLGEFAELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8927 | 8978 |
| OT-001903 (CD8a leader; HA Tag; CD19 scFv; CD8a Hinge and Transmembrane Domain; 4-1BB intracellular domain (CD28 co-stimulatory domain); CD3 zeta intracellular domain; Linker | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW | 8928 | 8979 |
| (GS); hPDE5 AA 535-860 of WT (H653A, R732L); stop) | APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRGSEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSADLDHRGVNNSYIQRSEHPLA QLYCHSIMEHHHFDQCLMILNSPGNQILSGLSIEEYKTTLKIIKQAIL ATDLALYIKRLGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | | |
| OT-001906 (CD8a leader; HA Tag; CD19 scFv; CD8a Hinge and Transmembrane Domain; 4-1BB intracellular domain (CD28 co-stimulatory domain); CD3 zeta intracellular domain; Linker (GS); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRGS* | 8929 | 8980 |
| OT-001905 (CD8a leader; CD19 scFv; CD8a Hinge and Transmembrane Domain; 4-1BB intracellular domain (CD28 co-stimulatory domain); CD3 zeta intracellular domain; Linker (GS); enhanced Green Fluorescent Protein (A206K); Linker (GS); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSMVSKGEELFTGVV PILVELDGDVNGHKFSVSGEGEGDATYGKLTLKFICTTGKLPVPWP TLVTTLTYGVQCFSRYPDHMKQHDFFKSAMPEGYVQERTIFFKDD GNYKTRAEVKFEGDTLVNRIELKGIDFKEDGNILGHKLEYNYNSH NVYIMADKQKNGIKVNFKIRHNIEDGSVQLADHYQQNTPIGDGPV LLPDNHYLSTQSKLSKDPNEKRDHMVLLEFVTAAGITLGMDELYK GS* | 8930 | 8981 |
| OT-001904 (CD8a leader; CD19 scFv; CD8a Hinge and Transmembrane Domain; 4-1BB intracellular domain (CD28 co-stimulatory domain); CD3 zeta intracellular domain; Linker (GS); enhanced Green Fluorescent Protein (A206K); Linker (GS); hPDE5 AA 535-860 of WT (H653A, R732L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSMVSKGEELFTGVV PILVELDGDVNGHKFSVSGEGEGDATYGKLTLKFICTTGKLPVPWP TLVTTLTYGVQCFSRYPDHMKQHDFFKSAMPEGYVQERTIFFKDD GNYKTRAEVKFEGDTLVNRIELKGIDFKEDGNILGHKLEYNYNSH NVYIMADKQKNGIKVNFKIRHNIEDGSVQLADHYQQNTPIGDGPV LLPDNHYLSTQSKLSKDPNEKRDHMVLLEFVTAAGITLGMDELYK GSEETRELQSLAAAVVPSAQTLKITDFSFSDFELSDLETALCTIRMFT DLNLVQNFQMKHEVLCRWILSVKKNYRKNVAYHNWRHAFNTAQ CMFAALKAGKIQNKLTDLEILALLIAALSADLDHRGVNNSYIQRSE HPLAQLYCHSIMEHHHFDQCLMILNSPGNQILSGLSIEEYKTTLKIIK | 8931 | 8982 |
| | QAILATDLALYIKRLGEFFELIRKNQFNLEDPHQKELFLAMLMTAC DLSAITKPWPIQQRIAELVATEFFDQGDRERKELNIEPTDLMNREKK NKIPSMQVGFIDAICLQLYEALTHVSEDCFPLLDGCRKNRQKWQAL AEQQ* | | |
| OT-001902 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 AA 535-860 of WT (H653A, R732L); Linker (GSG); P2A Cleavage peptide; Firefly Luciferase (N50D, N119G, S5481, K549A, L550V); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSADLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQGSGATNFSLLK QAGDVEENPGPMEDAKNIKKGPAPFYPLEDGTAGEQLHKAMKRY ALVPGTIAFTDAHIEVDITYAEYFEMSVRLAEAMKRYGLNTNHRIV VCSENSLQFFMPVLGALFIGVAVAPANDIYNERELLNSMGISQPTV VFVSKKGLQKILNVQKKLPIIQKIIIMDSKTDYQGFQSMYTFVTSHL PPGFNEYDFVPESFDRDKTIALIMNSSGSTGLPKGVALPHRTACVRF SHARDPIFGNQIIPDTAILSVVPFHHGFGMFTTLGYLICGFRVVLMY RFEEELFLRSLQDYKIQSALLVPTLFSFFAKSTLIDKYDLSNLHEIAS GGAPLSKEVGEAVAKRFHLPGIRQGYGLTETTSAILITPEGDDKPGA VGKVVPFFEAKVVDLDTGKTLGVNQRGELCVRGPMIMSGYVNNP EATNALIDKDGWLHSGDIAYWDEDEHFFIVDRLKSLIKYKGYQVA PAELESILLQHPNIFDAGVAGLPDDDAGELPAAVVVLEHGKTMTEK EIVDYVASQVTTAKKLRGGVVFVDEVPKGLTGKLDARKIREILIKA KKGGKIAV* | 8932 | 8983 |
| OT-001901 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 (535-860 of WT); Linker (GSG); P2A Cleavage Site; Firefly Luciferase (N50D, N119G, S5481, K549A, L550V); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRR GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQGSGATNFSLLK QAGDVEENPGPMEDAKNIKKGPAPFYPLEDGTAGEQLHKAMKRY ALVPGTIAFTDAHIEVDITYAEYFEMSVRLAEAMKRYGLNTNHRIV VCSENSLQFFMPVLGALFIGVAVAPANDIYNERELLNSMGISQPTV VFVSKKGLQKILNVQKKLPIIQKIIIMDSKTDYQGFQSMYTFVTSHL PPGFNEYDFVPESFDRDKTIALIMNSSGSTGLPKGVALPHRTACVRF SHARDPIFGNQIIPDTAILSVVPFHHGFGMFTTLGYLICGFRVVLMY RFEEELFLRSLQDYKIQSALLVPTLFSFFAKSTLIDKYDLSNLHEIAS | 8933 | 8984 |
| | GGAPLSKEVGEAVAKRFHLPGIRQGYGLTETTSAILITPEGDDKPGA VGKVVPFFEAKVVDLDTGKTLGVNQRGELCVRGPMIMSGYVNNP EATNALIDKDGWLHSGDIAYWDEDEHFFIVDRLKSLIKYKGYQVA PAELESILLQHPNIFDAGVAGLPDDDAGELPAAVVVLEHGKTMTEK EIVDYVASQVTTAKKLRGGVVFVDEVPKGLTGKLDARKIREILIKA KKGGKIAV* | | |
| OT-001900 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 AA 535-860 of WT (H653A, R732L); Linker (GSG); P2A Cleavage Site; enhanced Green Fluorescent Protein (M1del); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSADLDHRGVNNSYIQRSEHPLAQLYCHSIMEHH HFDQCLMILNSPGNQILSGLSIEEYKTTLKIIKQAILATDLALYIKRL GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQGSGATNFSLLK QAGDVEENPGPVSKGEELFTGVVPILVELDGDVNGHKFSVSGEGE GDATYGKLTLKFICTTGKLPVPWPTLVTTLTYGVQCFSRYPDHMK QHDFFKSAMPEGYVQERTIFFKDDGNYKTRAEVKFEGDTLVNRIEL KGIDFKEDGNILGHKLEYNYNSHNVYIMADKQKNGIKVNFKIRHNI EDGSVQLADHYQQNTPIGDGPVLLPDNHYLSTQSALSKDPNEKRD HMVLLEFVTAAGITLGMDELYK* | 8934 | 8985 |
| OT-001899 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 (535-860 of WT); Linker (GSG); P2A Cleavage Site; enhanced Green Fluorescent Protein (M1del); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRR GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQGSGATNFSLLK QAGDVEENPGPVSKGEELFTGVVPILVELDGDVNGHKFSVSGEGE GDATYGKLTLKFICTTGKLPVPWPTLVTTLTYGVQCFSRYPDHMK QHDFFKSAMPEGYVQERTIFFKDDGNYKTRAEVKFEGDTLVNRIEL KGIDFKEDGNILGHKLEYNYNSHNVYIMADKQKNGIKVNFKIRHNI EDGSVQLADHYQQNTPIGDGPVLLPDNHYLSTQSALSKDPNEKRD HMVLLEFVTAAGITLGMDELYK* | 8935 | 8986 |
| OT-001898 (CD8a leader; Linker (GS); hPDE5 AA 535-860 of WT (H653A, R732L); Linker (GS); CD19 scFv; CD8a Hinge and Transmembrane Domain; 4-1BB intracellular domain (CD28 co-stimulatory domain); CD3 zeta intracellular domain; stop) | MALPVTALLLPLALLLHAARPGSEETRELQSLAAAVVPSAQTLKIT DFSFSDFELSDLETALCTIRMFTDLNLVQNFQMKHEVLCRWILSVK KNYRKNVAYHNWRHAFNTAQCMFAALKAGKIQNKLTDLEILALL IAALSADLDHRGVNNSYIQRSEHPLAQLYCHSIMEHHHFDQCLMIL NSPGNQILSGLSIEEYKTTLKIIKQAILATDLALYIKRLGEFFELIRKN QFNLEDPHQKELFLAMLMTACDLSAITKPWPIQQRIAELVATEFFD | 8936 | 8987 |
| | QGDRERKELNIEPTDLMNREKKNKIPSMQVGFIDAICLQLYEALTH VSEDCFPLLDGCRKNRQKWQALAEQQGSDIQMTQTTSSLSASLGD RVTISCRASQDISKYLNWYQQKPDGTVKLLIYHTSRLHSGVPSRFS GSGSGTDYSLTISNLEQEDIATYFCQQGNTLPYTFGGGTKLEITGGG GSGGGGSGGGGSEVKLQESGPGLVAPSQSLSVTCTVSGVSLPDYG VSWIRQPPRKGLEWLGVIWGSETTYYNSALKSRLTIIKDNSKSQVF LKMNSLQTDDTAIYYCAKHYYYGGSYAMDYWGQGTSVTVSSTTT PAPRPPTPAPTIASQPLSLRPEACRPAAGGAVHTRGLDFACDIYIWA PLAGTCGVLLLSLVITLYCKRGRKKLLYIFKQPFMRPVQTTQEEDG CSCRFPEEEEGGCELRVKFSRSADAPAYKQGQNQLYNELNLGRRE EYDVLDKRRGRDPEMGGKPRRKNPQEGLYNELQKDKMAEAYSEI GMKGERRRGKGHDGLYQGLSTATKDTYDALHMQALPPR* | | |
| OT-001908 (CD8a leader; Linker (GS); hPDE5 AA 535-860 of WT (H653A, R732L); Linker (GS); Modified Furin Cleavage Site; CD19 scFv; CD8a Hinge and Transmembrane Domain; 4-1BB intracellular domain (CD28 co-stimulatory domain); CD3 zeta intracellular domain; stop) | MALPVTALLLPLALLLHAARPGSEETRELQSLAAAVVPSAQTLKIT DFSFSDFELSDLETALCTIRMFTDLNLVQNFQMKHEVLCRWILSVK KNYRKNVAYHNWRHAFNTAQCMFAALKAGKIQNKLTDLEILALL IAALSADLDHRGVNNSYIQRSEHPLAQLYCHSIMEHHHFDQCLMIL NSPGNQILSGLSIEEYKTTLKIIKQAILATDLALYIKRLGEFFELIRKN QFNLEDPHQKELFLAMLMTACDLSAITKPWPIQQRIAELVATEFFD QGDRERKELNIEPTDLMNREKKNKIPSMQVGFIDAICLQLYEALTH VSEDCFPLLDGCRKNRQKWQALAEQQGSESRRVRRNKRSKDIQM TQTTSSLSASLGDRVTISCRASQDISKYLNWYQQKPDGTVKLLIYH TSRLHSGVPSRFSGSGSGTDYSLTISNLEQEDIATYFCQQGNTLPYT FGGGTKLEITGGGGSGGGGSGGGGSEVKLQESGPGLVAPSQSLSVT CTVSGVSLPDYGVSWIRQPPRKGLEWLGVIWGSETTYYNSALKSR LTIIKDNSKSQVFLKMNSLQTDDTAIYYCAKHYYYGGSYAMDYW GQGTSVTVSSTTTPAPRPPTPAPTIASQPLSLRPEACRPAAGGAVHT RGLDFACDIYIWAPLAGTCGVLLLSLVITLYCKRGRKKLLYIFKQPF MRPVQTTQEEDGCSCRFPEEEEGGCELRVKFSRSADAPAYKQGQN QLYNELNLGRREEYDVLDKRRGRDPEMGGKPRRKNPQEGLYNEL QKDKMAEAYSEIGMKGERRRGKGHDGLYQGLSTATKDTYDALH MQALPPR* | 8937 | 8988 |
| OT-001508 (CD8a leader; CD19 scFv; Linker (ASFE); CD28 transmembrane and signaling domain; CD3 zeta intracellular domain; Linker (GS); hPDE5 AA 535-860 of WT (H653A, R732L); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSASFEIEVMYPPPY LDNEKSNGTIIHVKGKHLCPSPLFPGPSKPFWVLVVVGGVLACYSL LVTVAFIIFWVRSKRSRLLHSDYMNMTPRRPGPTRKHYQPYAPPRD FAAYRSRVKFSRSADAPAYKQGQNQLYNELNLGRREEYDVLDKR RGRDPEMGGKPRRKNPQEGLYNELQKDKMAEAYSEIGMKGERRR GKGHDGLYQGLSTATKDTYDALHMQALPPRGSEETRELQSLAAA VVPSAQTLKITDFSFSDFELSDLETALCTIRMFTDLNLVQNFQMKHE VLCRWILSVKKNYRKNVAYHNWRHAFNTAQCMFAALKAGKIQN KLTDLEILALLIAALSADLDHRGVNNSYIQRSEHPLAQLYCHSIMEH HHFDQCLMILNSPGNQILSGLSIEEYKTTLKIIKQAILATDLALYIKR LGEFFELIRKNQFNLEDPHQKELFLAMLMTACDLSAITKPWPIQQRI AELVATEFFDQGDRERKELNIEPTDLMNREKKNKIPSMQVGFIDAI CLQLYEALTHVSEDCFPLLDGCRKNRQKWQALAEQQ* | 8938 | 8989 |
| OT-001511 (CD8a leader; CD19 scFv; CD8a Hinge and Transmembrane Domain; 4-1BB intracellular domain (CD28 co-stimulatory domain); Linker (GS); hPDE5 AA 535-860 of WT (H653A, R732L); Linker (GS); CD3 zeta intracellular domain; stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELGSEETRELQSLAAAVVPSAQTLKITDFSFSDFELSDLETALC TIRMFTDLNLVQNFQMKHEVLCRWILSVKKNYRKNVAYHNWRH AFNTAQCMFAALKAGKIQNKLTDLEILALLIAALSADLDHRGVNN | 8939 | 8990 |
| | SYIQRSEHPLAQLYCHSIMEHHHFDQCLMILNSPGNQILSGLSIEEY KTTLKIIKQAILATDLALYIKRLGEFFELIRKNQFNLEDPHQKELFLA MLMTACDLSAITKPWPIQQRIAELVATEFFDQGDRERKELNIEPTDL MNREKKNKIPSMQVGFIDAICLQLYEALTHVSEDCFPLLDGCRKNR QKWQALAEQQGSRVKFSRSADAPAYKQGQNQLYNELNLGRREEY DVLDKRRGRDPEMGGKPRRKNPQEGLYNELQKDKMAEAYSEIGM KGERRRGKGHDGLYQGLSTATKDTYDALHMQALPPR* | | |
| OT-001510 (CD8a leader; CD19 scFv; CD8a Hinge and Transmembrane Domain; 4-1BB intracellular domain (CD28 co-stimulatory domain); Linker (GS); hPDE5 (535-860 of WT); Linker (GS); CD3 zeta intracellular domain; stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELGSEETRELQSLAAAVVPSAQTLKITDFSFSDFELSDLETALC TIRMFTDLNLVQNFQMKHEVLCRWILSVKKNYRKNVAYHNWRH AFNTAQCMFAALKAGKIQNKLTDLEILALLIAALSHDLDHRGVNN SYIQRSEHPLAQLYCHSIMEHHHFDQCLMILNSPGNQILSGLSIEEY KTTLKIIKQAILATDLALYIKRRGEFFELIRKNQFNLEDPHQKELFLA MLMTACDLSAITKPWPIQQRIAELVATEFFDQGDRERKELNIEPTDL MNREKKNKIPSMQVGFIDAICLQLYEALTHVSEDCFPLLDGCRKNR QKWQALAEQQGSRVKFSRSADAPAYKQGQNQLYNELNLGRREEY DVLDKRRGRDPEMGGKPRRKNPQEGLYNELQKDKMAEAYSEIGM KGERRRGKGHDGLYQGLSTATKDTYDALHMQALPPR* | 8940 | 8991 |
| OT-001512 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 (535-860 of WT); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRR GEFFELIRKNQFNLEDPHQKELFLAMLMTACDLSAITKPWPIQQRIA ELVATEFFDQGDRERKELNIEPTDLMNREKKNKIPSMQVGFIDAICL QLYEALTHVSEDCFPLLDGCRKNRQKWQALAEQQ* | 8941 | 8992 |
| OT-001501 (CD8a leader; CD19 scFv; CD8a Hinge and Transmembrane Domain; 4-1BB intracellular domain (CD28 co-stimulatory domain); CD3 zeta intracellular domain; Linker (GS); hPDE5 AA 535-860 of WT (R732L, F736A); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSEETRELQSLAAAV VPSAQTLKITDFSFSDFELSDLETALCTIRMFTDLNLVQNFQMKHEV LCRWILSVKKNYRKNVAYHNWRHAFNTAQCMFAALKAGKIQNK LTDLEILALLIAALSHDLDHRGVNNSYIQRSEHPLAQLYCHSIMEHH HFDQCLMILNSPGNQILSGLSIEEYKTTLKIIKQAILATDLALYIKRL GEFAELIRKNQFNLEDPHQKELFLAMLMTACDLSAITKPWPIQQRI | 8942 | 8993 |
| | AELVATEFFDQGDRERKELNIEPTDLMNREKKNKIPSMQVGFIDAI CLQLYEALTHVSEDCFPLLDGCRKNRQKWQALAEQQ* | | |
| OT-001509 (CD8a leader; CD19 scFv; CD8a Hinge and Transmembrane Domain; 4-1BB intracellular domain (CD28 co-stimulatory domain); Linker (GS); hPDE5 AA 535-860 of WT (R732L, F736A); Linker (GS); CD3 zeta intracellular domain; stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELGSEETRELQSLAAAVVPSAQTLKITDFSFSDFELSDLETALC TIRMFTDLNLVQNFQMKHEVLCRWILSVKKNYRKNVAYHNWRH AFNTAQCMFAALKAGKIQNKLTDLEILALLIAALSHDLDHRGVNN SYIQRSEHPLAQLYCHSIMEHHHFDQCLMILNSPGNQILSGLSIEEY KTTLKIIKQAILATDLALYIKRLGEFAELIRKNQFNLEDPHQKELFLA MLMTACDLSAITKPWPIQQRIAELVATEFFDQGDRERKELNIEPTDL MNREKKNKIPSMQVGFIDAICLQLYEALTHVSEDCFPLLDGCRKNR QKWQALAEQQGSRVKFSRSADAPAYKQGQNQLYNELNLGRREEY DVLDKRRGRDPEMGGKPRRKNPQEGLYNELQKDKMAEAYSEIGM KGERRRGKGHDGLYQGLSTATKDTYDALHMQALPPR* | 8943 | 8994 |
| OT-001602 (CD8a leader; CD19 scFv; CD8a Hinge and Transmembrane Domain; 4-1BB intracellular domain (CD28 co-stimulatory domain); CD3 zeta intracellular domain; Linker (GSG); P2A Cleavage Site; Linker (GSG); enhanced Green Fluorescent Protein (M1del); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSGATNFSLLKQAGD VEENPGPGSGVSKGEELFTGVVPILVELDGDVNGHKFSVSGEGEGD ATYGKLTLKFICTTGKLPVPWPTLVTTLTYGVQCFSRYPDHMKQH DFFKSAMPEGYVQERTIFFKDDGNYKTRAEVKFEGDTLVNRIELKG IDFKEDGNILGHKLEYNYNSHNVYIMADKQKNGIKVNFKIRHNIED GSVQLADHYQQNTPIGDGPVLLPDNHYLSTQSALSKDPNEKRDHM VLLEFVTAAGITLGMDELYK* | 8944 | 8995 |
| OT-001598 (CD8a leader; CD19 scFv; CD8a Hinge and Transmembrane Domain; 4-1BB intracellular domain (CD28 co-stimulatory domain); CD3 zeta intracellular domain; Linker (GSG); P2A Cleavage Site; Firefly Luciferase (N50D, N119G, S5481, K549A, L550V); stop) | MALPVTALLLPLALLLHAARPDIQMTQTTSSLSASLGDRVTISCRAS QDISKYLNWYQQKPDGTVKLLIYHTSRLHSGVPSRFSGSGSGTDYS LTISNLEQEDIATYFCQQGNTLPYTFGGGTKLEITGGGGSGGGGSG GGGSEVKLQESGPGLVAPSQSLSVTCTVSGVSLPDYGVSWIRQPPR KGLEWLGVIWGSETTYYNSALKSRLTIIKDNSKSQVFLKMNSLQTD DTAIYYCAKHYYYGGSYAMDYWGQGTSVTVSSTTTPAPRPPTPAP TIASQPLSLRPEACRPAAGGAVHTRGLDFACDIYIWAPLAGTCGVL LLSLVITLYCKRGRKKLLYIFKQPFMRPVQTTQEEDGCSCRFPEEEE GGCELRVKFSRSADAPAYKQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPRGSGATNFSLLKQAGD VEENPGPMEDAKNIKKGPAPFYPLEDGTAGEQLHKAMKRYALVP GTIAFTDAHIEVDITYAEYFEMSVRLAEAMKRYGLNTNHRIVVCSE NSLQFFMPVLGALFIGVAVAPANDIYNERELLNSMGISQPTVVFVS KKGLQKILNVQKKLPIIQKIIIMDSKTDYQGFQSMYTFVTSHLPPGF NEYDFVPESFDRDKTIALIMNSSGSTGLPKGVALPHRTACVRFSHA RDPIFGNQIIPDTAILSVVPFHHGFGMFTTLGYLICGFRVVLMYRFEE ELFLRSLQDYKIQSALLVPTLFSFFAKSTLIDKYDLSNLHEIASGGAP LSKEVGEAVAKRFHLPGIRQGYGLTETTSAILITPEGDDKPGAVGK VVPFFEAKVVDLDTGKTLGVNQRGELCVRGPMIMSGYVNNPEAT NALIDKDGWLHSGDIAYWDEDEHFFIVDRLKSLIKYKGYQVAPAE LESILLQHPNIFDAGVAGLPDDDAGELPAAVVVLEHGKTMTEKEIV | 8945 | 8996 |
| | DYVASQVTTAKKLRGGVVFVDEVPKGLTGKLDARKIREILIKAKK GGKIAV* | | |
| OT-001912 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (M758T); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLATLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ * | 8946 | 8997 |
| OT-001911 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (K752E); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQEELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8947 | 8998 |
| OT-001918 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (C677Y, H685R, A722V); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYYHSIMEHHRFDQCLMILNSPGNQILSGLSIEEYKTTLKIIKQAIL VTDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8948 | 8999 |
| OT-001920 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (T639S, M816R); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLSDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SRQVGFIDAICLQLYEALTHVSEDCFPLLDGCRKNRQKWQALAEQ Q* | 8949 | 9000 |
| OT-001910 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (T537A, D558G, 1706T, F744L, D764N); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEEA RELQSLAAAVVPSAQTLKITGFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSTEEYKTTLKIIKQAIL ATDLALYIKRRGEFFELIRKNQLNLEDPHQKELFLAMLMTACNLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8950 | 9001 |
| OT-001913 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (Q586R, D724G); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VRNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATGLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8951 | 9002 |
| OT-001919 (CD8a leader; HA Tag; CD19 scFv; CD8a Hinge and Transmembrane | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG | 8952 | 9003 |
| Domain; 4-1BB intracellular domain (CD28 co-stimulatory domain); CD3 zeta intracellular domain; Linker (SG); hPDE5 AA 535-860 of WT (F686S); stop) | GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHSDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | | |
| OT-001914 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (E539G, L7381); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RGLQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFEIIRKNQFNLEDPHQKELFLAMLMTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ * | 8953 | 9004 |
| OT-001916 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (Q635R, E753K, 1813T); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIRNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKKLFLAMLMTACDLSA ITKPWPIQQRIAELVATEFFDQGDRERKELNIEPTDLMNREKKNKTP SMQVGFIDAICLQLYEALTHVSEDCFPLLDGCRKNRQKWQALAEQ Q* | 8954 | 9005 |
| OT-001915 (CD8a leader; HA Tag; CD19 scFv; CD8a Hinge and Transmembrane Domain; 4-1BB intracellular domain (CD28 co-stimulatory domain); CD3 zeta | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT | 8955 | 9006 |
| intracellular domain; Linker (SG); hPDE5 AA 535-860 of WT (L672P, S836L); stop) | TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPPA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVLEDCFPLLDGCRKNRQKWQALAEQ Q* | | |
| OT-001917 (CD8a leader; HA Tag; CD19 scFv; CD8a Hinge and Transmembrane Domain; 4-1BB intracellular domain (CD28 co-stimulatory domain); CD3 zeta intracellular domain; hPDE5 AA 535-860 of WT (M691T, D764N); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLTILNSPGNQILSGLSIEEYKTTLKIIKQAIL ATDLALYIKRRGEFFELIRKNQFNLEDPHQKELFLAMLMTACNLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8956 | 9007 |
| OT-001923 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (R807G); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNGEKKNKIP SMQVGFIDAICLQLYEALTHVSEDCFPLLDGCRKNRQKWQALAEQ Q* | 8957 | 9008 |
| OT-001922 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR | 8958 | 9009 |
| WT (R577Q, C596R, V660A, 1715V, E785K, L856P); stop) | EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIQMFTDLNL VQNFQMKHEVLRRWILSVKKNYRKNVAYHNWRHAFNTAQCMFA ALKAGKIQNKLTDLEILALLIAALSHDLDHRGANNSYIQRSEHPLA QLYCHSIMEHHHFDQCLMILNSPGNQILSGLSIEEYKTTLKVIKQAI LATDLALYIKRRGEFFELIRKNQFNLEDPHQKELFLAMLMTACDLS AITKPWPIQQRIAELVATKFFDQGDRERKELNIEPTDLMNREKKNKI PSMQVGFIDAICLQLYEALTHVSEDCFPLLDGCRKNRQKWQAPAE QQ* | | |
| OT-001921 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (1720V, F820S); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAV LATDLALYIKRRGEFFELIRKNQFNLEDPHQKELFLAMLMTACDLS AITKPWPIQQRIAELVATEFFDQGDRERKELNIEPTDLMNREKKNKI PSMQVGSIDAICLQLYEALTHVSEDCFPLLDGCRKNRQKWQALAE QQ* | 8959 | 9010 |
| OT-001926 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (S695G, E707K, 1739M, C763R); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNGPGNQILSGLSIKEYKTTLKIIKQAIL ATDLALYIKRRGEFFELMRKNQFNLEDPHQKELFLAMLMTARDLS AITKPWPIQQRIAELVATEFFDQGDRERKELNIEPTDLMNREKKNKI PSMQVGFIDAICLQLYEALTHVSEDCFPLLDGCRKNRQKWQALAE QQ* | 8960 | 9011 |
| OT-001924 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (Y709H, K812R, L832P); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL | 8961 | 9012 |
| | VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEHKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNRIP SMQVGFIDAICLQLYEAPTHVSEDCFPLLDGCRKNRQKWQALAEQ Q* | | |
| OT-001925 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (N583S, K752E, C846S); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLSL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQEELFLAMLMTACDLSA ITKPWPIQQRIAELVATEFFDQGDRERKELNIEPTDLMNREKKNKIP SMQVGFIDAICLQLYEALTHVSEDCFPLLDGSRKNRQKWQALAEQ Q* | 8962 | 9013 |
| OT-001929 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (E682G, D748N); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMGHHHFDQCLMILNSPGNQILSGLSIEEYKTTLKIIKQAIL ATDLALYIKRRGEFFELIRKNQFNLENPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8963 | 9014 |
| OT-001928 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (K591R, 1643T, L856P); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMRHEVLCRWILSVKKNYRKNVAYHNWRHAFNTAQCMFA ALKAGKIQNKLTDLETLALLIAALSHDLDHRGVNNSYIQRSEHPLA QLYCHSIMEHHHFDQCLMILNSPGNQILSGLSIEEYKTTLKIIKQAIL | 8964 | 9015 |
| | ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQAPAEQ Q* | | |
| OT-001930 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (F619S, V818A, Y829C); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHASNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQAGFIDAICLQLCEALTHVSEDCFPLLDGCRKNRQKWQALAEQ Q* | 8965 | 9016 |
| OT-001927 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (V548E, Q589L, K6331, M681T, S7021, K752E, L781P, A857T); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVEPSAQTLKITDFSFSDFELSDLETALCTIRMFTDLNL VQNFLMKHEVLCRWILSVKKNYRKNVAYHNWRHAFNTAQCMFA ALKAGIIQNKLTDLEILALLIAALSHDLDHRGVNNSYIQRSEHPLAQ LYCHSITEHHHFDQCLMILNSPGNQILIGLSIEEYKTTLKIIKQAILAT DLALYIKRRGEFFELIRKNQFNLEDPHQEELFLAMLMTACDLSAIT KPWPIQQRIAEPVATEFFDQGDRERKELNIEPTDLMNREKKNKIPS MQVGFIDAICLQLYEALTHVSEDCFPLLDGCRKNRQKWQALTEQQ * | 8966 | 9017 |
| OT-001933 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (S652G, Q688R); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALGHDLDHRGVNNSYIQRSEHPLA QLYCHSIMEHHHFDRCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP | 8967 | 9018 |
| | SMQVGFIDAICLQLYEALTHVSEDCFPLLDGCRKNRQKWQALAEQ Q* | | |
| OT-001931 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (E565G); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFGLSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8968 | 9019 |
| OT-001932 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (1774V); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPVQQRIAELVATEFFDQGDRERKELNIEPTDLMNREKKNKI PSMQVGFIDAICLQLYEALTHVSEDCFPLLDGCRKNRQKWQALAE QQ* | 8969 | 9020 |
| OT-001935 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (K591R); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMRHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | 8970 | 9021 |
| OT-001934 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (F559S, Y709C, M760T); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDSSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEECKTTLKIIKQAIL ATDLALYIKRRGEFFELIRKNQFNLEDPHQKELFLAMLTTACDLSAI TKPWPIQQRIAELVATEFFDQGDRERKELNIEPTDLMNREKKNKIPS MQVGFIDAICLQLYEALTHVSEDCFPLLDGCRKNRQKWQALAEQQ * | 8971 | 9022 |
| OT-001937 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (A649V, A650T, K730E, E830K); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIVTLSHDLDHRGVNNSYIQRSEHPLAQ LYCHSIMEHHHFDQCLMILNSPGNQILSGLSIEEYKTTLKIIKQAILA TDLALYIERRGEFFELIRKNQFNLEDPHQKELFLAMLMTACDLSAIT KPWPIQQRIAELVATEFFDQGDRERKELNIEPTDLMNREKKNKIPS MQVGFIDAICLQLYKALTHVSEDCFPLLDGCRKNRQKWQALAEQQ * | 8972 | 9023 |
| OT-001936 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (Y728C, Q817R); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVLCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALCIKRRGEFFELIRKNQFNLEDPHQKELFLAMLMTACDLSA ITKPWPIQQRIAELVATEFFDQGDRERKELNIEPTDLMNREKKNKIP SMRVGFIDAICLQLYEALTHVSEDCFPLLDGCRKNRQKWQALAEQ Q* | 8973 | 9024 |
| OT-001938 (CD8a leader; HA Tag; CD19 scFv; CD8a Hinge and Transmembrane | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG | 8974 | 9025 |
| Domain; 4-1BB intracellular domain (CD28 co-stimulatory domain); CD3 zeta intracellular domain; Linker (SG); hPDE5 AA 535-860 of WT (L595P, K741R); stop) | GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIRMFTDLNL VQNFQMKHEVPCRWILSVKKNYRKNVAYHNWRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRRNQFNLEDPHQKELFLAMLMTACDLSA ITKPWPIQQRIAELVATEFFDQGDRERKELNIEPTDLMNREKKNKIP SMQVGFIDAICLQLYEALTHVSEDCFPLLDGCRKNRQKWQALAEQ Q* | | |
| OT-001909 (CD8a leader; HA Tag; CD19 scFv; CD8a Hinge and Transmembrane Domain; 4-1BB intracellular domain (CD28 co-stimulatory domain); CD3 zeta intracellular domain; Linker (SG); hPDE5 AA 535-860 of WT (R577W, W615R, M805T, 1821V); stop) | MALPVTALLLPLALLLHAARPYPYDVPDYADIQMTQTTSSLSASLG DRVTISCRASQDISKYLNWYQQKPDGTVKLLIYHTSRLHSGVPSRF SGSGSGTDYSLTISNLEQEDIATYFCQQGNTLPYTFGGGTKLEITGG GGSGGGGSGGGGSEVKLQESGPGLVAPSQSLSVTCTVSGVSLPDY GVSWIRQPPRKGLEWLGVIWGSETTYYNSALKSRLTIIKDNSKSQV FLKMNSLQTDDTAIYYCAKHYYYGGSYAMDYWGQGTSVTVSSTT TPAPRPPTPAPTIASQPLSLRPEACRPAAGGAVHTRGLDFACDIYIW APLAGTCGVLLLSLVITLYCKRGRKKLLYIFKQPFMRPVQTTQEED GCSCRFPEEEEGGCELRVKFSRSADAPAYKQGQNQLYNELNLGRR EEYDVLDKRRGRDPEMGGKPRRKNPQEGLYNELQKDKMAEAYSE IGMKGERRRGKGHDGLYQGLSTATKDTYDALHMQALPPRSGEET RELQSLAAAVVPSAQTLKITDFSFSDFELSDLETALCTIWMFTDLNL VQNFQMKHEVLCRWILSVKKNYRKNVAYHNRRHAFNTAQCMFA ALKAGKIQNKLTDLEILALLIAALSHDLDHRGVNNSYIQRSEHPLA QLYCHSIMEHHHFDQCLMILNSPGNQILSGLSIEEYKTTLKIIKQAIL ATDLALYIKRRGEFFELIRKNQFNLEDPHQKELFLAMLMTACDLSA ITKPWPIQQRIAELVATEFFDQGDRERKELNIEPTDLTNREKKNKIPS MQVGFVDAICLQLYEALTHVSEDCFPLLDGCRKNRQKWQALAEQ Q* | 8975 | 9026 |

### Tandem CAR (TanCAR)

In some instances, the CAR of the present disclosure may be a tandem chimeric antigen receptor (TanCAR) which is able to target two, three, four, or more tumor specific antigens. In some aspects, the CAR is a bispecific TanCAR including two targeting domains which recognize two different TSAs on tumor cells. The bispecific CAR may be further defined as comprising an extracellular region comprising a targeting domain (e.g., an antigen recognition domain) specific for a first tumor antigen and a targeting domain (e.g., an antigen recognition domain) specific for a second tumor antigen. In other aspects, the CAR is a multispecific TanCAR that includes three or more targeting domains configured in a tandem arrangement. The space between the targeting domains in the TanCAR may be between about 5 and about 30 amino acids in length, for example, 6, 7, 8, 9, 10, 11, 12, 13, 14, 15, 16, 17, 18, 19, 20, 21, 22, 23, 24, 25, 26, 27, 28, 29 and 30 amino acids.

### Split CAR

In some instances, the components including the targeting moiety, transmembrane domain and intracellular signaling domains of the present disclosure may be split into two or more parts such that it is dependent on multiple inputs that promote assembly of the intact functional receptor. In one instance, the split synthetic CAR system can be constructed in which the assembly of an activated CAR receptor is dependent on the binding of a ligand to the SRE (e.g. a small molecule) and a specific antigen to the targeting moiety. As a non-limiting example, the split CAR consists of two parts that assemble in a small molecule-dependent manner; one part of the receptor features an extracellular antigen binding domain (e.g. scFv) and the other part has the intracellular signaling domains, such as the CD3ζ intracellular domain.

In other aspects, the split parts of the CAR system can be further modified to increase signal. In one example, the second part of cytoplasmic fragment may be anchored to the plasma membrane by incorporating a transmembrane domain (e.g., CD8α transmembrane domain) to the construct. An additional extracellular domain may also be added to the second part of the CAR system, for instance an extracellular domain that mediates homo-dimerization. These modifications may increase receptor output activity, i.e., T cell activation.

In some aspects, the two parts of the split CAR system contain heterodimerization domains that conditionally interact upon binding of a heterodimerizing small molecule. As such, the receptor components are assembled in the presence of the small molecule, to form an intact system which can then be activated by antigen engagement. Any known heterodimerizing components can be incorporated into a split CAR system. Other small molecule dependent heterodimerization domains may also be used, including, but not limited to, gibberellin-induced dimerization system (GID1-GAI), trimethoprim-SLF induced ecDHFR and FKBP dimerization (Czlapinski et al., J Am Chem Soc., 2008, 130(40): 13186-13187) and ABA (abscisic acid) induced dimerization of PP2C and PYL domains (Cutler et al., Annu Rev Plant Biol. 2010, 61: 651-679). The dual regulation using inducible assembly (e.g., ligand dependent dimerization) and degradation (e.g., destabilizing domain induced CAR degradation) of the split CAR system may provide more flexibility to control the activity of the CAR modified T cells.

### Switchable CAR

In some instances, the CAR of the disclosure may be a switchable CAR. Juilerat et al (Juilerat et al., Sci. Rep., 2016, 6: 18950) recently reported controllable CARs that can be transiently switched on in response to a stimulus (e.g. a small molecule). In this CAR design, a system is directly integrated in the hinge domain that separate the scFv domain from the cell membrane domain in the CAR. Such system is possible to split or combine different key functions of a CAR such as activation and co-stimulation within different chains of a receptor complex, mimicking the complexity of the TCR native architecture. This integrated system can switch the scFv and antigen interaction between on/off states controlled by the absence/presence of the stimulus.

### Reversible CAR

In other instances, the CAR of the disclosure may be a reversible CAR system. In this CAR architecture, a LID domain (ligand-induced degradation) is incorporated into the CAR system. The CAR can be temporarily down-regulated by adding a ligand of the LID domain. The combination of LID and DD mediated regulation provides tunable control of continuingly activated CAR T cells, thereby reducing CAR mediated tissue toxicity.

### Inhibitory CAR (iCAR)

In some instances, payloads of the present disclosure may be inhibitory CARs. Inhibitory CAR (iCAR) refers to a bispecific CAR design wherein a negative signal is used to enhance the tumor specificity and limit normal tissue toxicity. This design incorporates a second CAR having a surface antigen recognition domain combined with an inhibitory signal domain to limit T cell responsiveness even with concurrent engagement of an activating receptor. This antigen recognition domain is directed towards a normal tissue specific antigen such that the T cell can be activated in the presence of first target protein, but if the second protein that binds to the iCAR is present, the T cell activation is inhibited.

As a non-limiting example, iCARs against Prostate specific membrane antigen (PMSA) based on CTLA4 and PD1 inhibitory domains demonstrated the ability to selectively limit cytokine secretion, cytotoxicity and proliferation induced by T cell activation (Fedorov V.D, et al., 2013, Sci Transl Med, 11;5(215):215ra172).

### Chimeric switch receptor

In some instances, payloads of the disclosure may be chimeric switch receptors which can switch a negative signal to a positive signal. As used herein, the term "chimeric switch receptor" refers to a fusion protein comprising a first extracellular domain and a second transmembrane and intracellular domain, wherein the first domain includes a negative signal region and the second domain includes a positive intracellular signaling region. In some aspects, the fusion protein is a chimeric switch receptor that contains the extracellular domain of an inhibitory receptor on T cell fused to the transmembrane and cytoplasmic domain of a co-stimulatory receptor. This chimeric switch receptor may convert a T cell inhibitory signal into a T cell stimulatory signal.

As a non-limiting example, the chimeric switch receptor may comprise the extracellular domain of PD-1 fused to the transmembrane and cytoplasmic domain of CD28 as taught by Liu et al. (Liu et al., Cancer Res., 2016, 76(6): 1578-1590). In some aspects, extracellular domains of other inhibitory receptors such as CTLA-4, LAG-3, TIM-3, KIRs and BTLA may also be fused to the transmembrane and cytoplasmic domain derived from costimulatory receptors such as CD28, 4-1BB, CD27, OX40, CD40, GTIR and ICOS. In the context of the present disclosure, the SRE domain (e.g., DD) may be inserted at the N- or C- terminus of the chimeric switch receptor.

In some instances, chimeric switch receptors of the present disclosure may include recombinant receptors comprising the extracellular cytokine-binding domain of an inhibitory cytokine receptor (e.g., IL13 receptor α (IL13Rα1), IL10R, and IL4Rα) fused to an intracellular signaling domain of a stimulatory cytokine receptor such as IL2R (IL2Rα, IL2Rβ and IL2Rgamma) and IL7Rα. One example of such chimeric cytokine receptor is a recombinant receptor containing the cytokine-binding extracellular domain of IL4Rα linked to the intracellular signaling domain of IL7Rα (see, US patent publication NO: 2014/0050709).

In one instance, the chimeric switch receptor of the present disclosure may be a chimeric TGFβ receptor. The chimeric TGFβ receptor may comprise an extracellular domain derived from a TGFβ receptor such as TGFβ receptor 1, TGFβ receptor 2, TGFβ receptor 3, or any other TGFβ receptor or variant thereof; and a non-TGFβ receptor intracellular domain. The non- TGFβ receptor intracellular domain may be the intracellular domain or fragment thereof derived from TLR1, TLR2, TLR3, TLR4, TLR5, TLR6, TLR7, TLR8, TLR9, TLR10, CD28, 4-1BB (CD137), OX40 (CD134), CD3zeta, CD40, CD27, or a combination thereof. One example of such chimeric TGFβ receptor is discussed in US patent publication NO.: US2016/0075755.

In some instances, payloads of the present disclosure may be bipartite fusion receptors. In one aspect, the bipartite fusion receptor may comprise an antigen-binding domain that binds to a tumor antigen and an activation domain that binds to one or more cell surface molecules. In some aspects, the antigen binding domain of a bipartite receptor is a scFv antibody. In other aspects, the activation domain that binds to a T cell surface stimulatory molecule may be selected from CD3, CD27, CD 28, CD40, OX40(CD134) and 4-1BB(CD137), or a NK cell surface stimulatory molecule selected from CD 16, NKG2D and NKp30. The activation domain may also be a scFv specific to a stimulatory molecule on the surface of immune cells (e.g., T cells and NK cells). Immune cells can be genetically modified to express a bipartite molecule comprising at least one antigen binding domain and an activation domain. The antigen binding domain binds to one or more molecules present on target cells such as cancer cells. Immune cells that express the molecule recognized by the activation domain will be activated and attack the recognized cancer cells. As a non-limiting example, a bipartite molecule may be an engager molecule comprising an antigen recognition scFv specific to CD19 or EphA2, as described in US patent publication NO: US2016/0015749.

### Activation-conditional CAR

In some instances, payloads of the disclosure may be an activation-conditional chimeric antigen receptor, which is only expressed in an activated immune cell. The expression of the CAR may be coupled to activation conditional control region which refers to one or more nucleic acid sequences that induce the transcription and/or expression of a sequence e.g. a CAR under its control. Such activation conditional control regions may be promoters of genes that are upregulated during the activation of the immune effector cell e.g. IL2 promoter or NFAT binding sites. In some instances, activation of the immune cell may be achieved by a constitutively expressed CAR (International Publication No: WO 2016126608).

### CAR targeting to tumor cells with specific proteoglycan markers

In some instances, payloads of the present disclosure may be a CAR that targets specific types of cancer cells. Human cancer cells and metastasis may express unique and otherwise abnormal proteoglycans, such as polysaccharide chains (e.g., chondroitin sulfate (CS), dermatan sulfate (DS or CSB), heparan sulfate (HS) and heparin). Accordingly, the CAR may be fused with a binding moiety that recognizes cancer associated proteoglycans. In one example, a CAR may be fused with VAR2CSA polypeptide (VAR2-CAR) that binds with high affinity to a specific type of chondroitin sulfate A (CSA) attached to proteoglycans. The extracellular ScFv portion of the CAR may be substituted with VAR2CSA variants comprising at least the minimal CSA binding domain, generating CARs specific to chondroitin sulfate A (CSA) modifications. Alternatively, the CAR may be fused with a split-protein binding system to generate a spy-CAR, in which the scFv portion of the CAR is substituted with one portion of a split-protein binding system such as Spy Tag and Spy-catcher and the cancer-recognition molecules (e.g. scFv and or VAR2-CSA) are attached to the CAR through the split-protein binding system (See, e.g., PCT publication No.: WO2016/135291).

### SUPRA CAR

In some instances, the payload of the present disclosure may be a Split Universal Programmable (SUPRA) CAR. A SUPRA CAR may be a two-component receptor system comprising of a universal receptor (zip CAR) expressed on T cells and a tumor-targeting scFv adaptor. The zip CAR universal receptor may be generated by the fusion of intracellular signaling domains and a leucine zipper as the extracellular domain. The tumor-targeting scFv adaptor molecule or zipFv, may be generated by the fusion of a cognate leucine zipper and an scFv. The scFv of the zipFv may bind to a tumor antigen, and the leucine zipper may bind and activate the zip CAR on the T cells. Unlike the conventional fixed CAR design, the SUPRA CAR modular design allows targeting of multiple antigens without further genetic manipulations of the immune cells. Any of the CAR designs disclosed by Cho et al., 2018, Cell 173, 1-13, may be useful in the present disclosure.

### 6. Cytokines, chemokines and other soluble factors

In accordance with the present disclosure, payloads of the present disclosure may be cytokines, chemokines, growth factors, and soluble proteins produced by immune cells, cancer cells and other cell types, which act as chemical communicators between cells and tissues within the body. These proteins mediate a wide range of physiological functions, from effects on cell growth, differentiation, migration and survival, to a number of effector activities. For example, activated T cells produce a variety of cytokines for cytotoxic function to eliminate tumor cells.

In some instances, payloads of the present disclosure may be cytokines, and fragments, variants, analogs and derivatives thereof, including but not limited to interleukins, tumor necrosis factors (TNFs), interferons (IFNs), TGF beta and chemokines. In some instances, payloads of the present disclosure may be cytokines that stimulate immune responses. In other instances, payloads of the disclosure may be antagonists of cytokines that negatively impact anti-cancer immune responses.

In some instances, payloads of the present disclosure may be cytokine receptors, recombinant receptors, variants, analogs and derivatives thereof; or signal components of cytokines.

In some instances, cytokines of the present disclosure may be utilized to improve expansion, survival, persistence, and potency of immune cells such as CD8+T_{EM}, natural killer cells and tumor infiltrating lymphocytes (TIL) cells used for immunotherapy. In other instances, T cells engineered with two or more DD regulated cytokines are utilized to provide kinetic control of T cell activation and tumor microenvironment remodeling. In one aspect, the present disclosure provides biocircuits and compositions to minimize toxicity related to cytokine therapy. Despite its success in mitigating tumor burden, systemic cytokine therapy often results in the development of severe dose limiting side effects. Two factors contribute to the observed toxicity (a) Pleiotropism, wherein cytokines affect different cells types and sometimes produce opposing effects on the same cells depending on the context (b) Cytokines have short serum half-life and thus need to be administered at high doses to achieve therapeutic effects, which exacerbates the pleiotropic effects. In one aspect, cytokines of the present disclosure may be utilized to modulate cytokine expression in the event of adverse effects. In some instances, cytokines of the present disclosure may be designed to have prolonged life span or enhanced specificity to minimize toxicity.

In some instances, the payload of the present disclosure may be an interleukin (IL) cytokine. Interleukins (ILs) are a class of glycoproteins produced by leukocytes for regulating immune responses. As used herein, the term "interleukin (IL)" refers to an interleukin polypeptide from any species or source and includes the full-length protein as well as fragments or portions of the protein. In some aspects, the interleukin payload is selected from IL1, ILlalpha (also called hematopoietin-1), ILlbeta (catabolic), IL1 delta, ILlepsilon, ILleta, IL1 zeta, interleukin-1 family member 1 to 11 (IL1F1 to IL1F11), interleukin-1 homolog 1 to 4 (IL1H1 to IL1H4), IL1 related protein 1 to 3 (IL1RP1 to IL1RP3), IL2, IL3, IL4, IL5, IL6, IL7, IL8, IL9, IL10, IL10C, IL10D, IL11, IL11a, IL11b, IL12, IL13, IL14, IL15, IL16, IL17, IL17A, I117B, IL17C, IL17E, IL17F, IL18, IL19, IL20, IL20 like (IL20L), Il21, IL22, IL23, IL23A, IL23-pl9, IL23-p40, IL24, 1125, IL26, IL27, IL28A, IL28B, IL29, IL30, IL31, IL32, IL33, IL34, IL35, IL36 alpha, IL36 beta, IL36 gamma, IL36RN, IL37, IL37a, IL37b, IL37c, IL37d, IL37e and IL38. In other aspects, the payload of the present disclosure may be an interleukin receptor selected from CD121a, CDw121b, IL2Ra/CD25, IL2Rβ/CD122, IL2Ry/CD132, CDw131, CD124, CD131, CDw125, CD126, CD130, CD127, CDw210, IL8RA, IL11Rα, CD212, CD213α1, CD213α2, IL14R, IL15Rα, CDw217, IL18Rα, IL18Rβ, IL20Rα, and IL20Rβ.

In certain instances, a cytokine may be a type I interferons (IFN) including IFN alpha subtypes (IFN α1, IFN α1b, IFN α1c), IFN beta, IFN delta subtypes (IFN delta 1, IFN delta 2, IFN delta 8), IFN gamma, IFN kappa, and IFN epsilon, IFN lambda, IFN omega, IFN tau and IFN zeta. In certain instances, a cytokine may be a member of tumor necrosis factor (TNF) superfamily, including TNF-alpha, TNF-beta (also known as lymphotoxin-alpha (LT-α)), lymphotoxin-beta (LT-β), CD40L(CD154), CD27L (CD70), CD30L(CD153), FASL(CD178), 4-1BBL (CD137L), OX40L, TRAIL (TNF-related apoptosis inducing ligand), APRIL (a proliferation-inducing ligand), TWEAK, TRANCE, TALL-1, GITRL, LIGHT and TNFSF1 to TNFSF20 (TNF ligand superfamily member 1 to 20).

In one instance, the payload of the disclosure may comprise IL2 (SEQ ID NO: 8299, encoded by SEQ ID NO: 8300 and 8301. In one aspect, the effector module of the disclosure may be a DD-IL2 fusion polypeptide.

In some aspects of the disclosure, an IL2 mutein may be used as a payload. As used herein, the term "mutein" is a construct, molecule or sequence of a mutation, change or alteration in a protein and hence is also known as a mutant, e.g., a protein mutant, mutein. Consequently, an "IL2 mutein" is an IL2 mutant. In some instances an IL2 mutein is a variant of wild type IL2 protein, where the wildtype IL2 consists of the amino acid sequence of SEQ ID NO: 8299. In some respects, it refers to an IL2 variant which binds to and activates only cells expressing IL2Rαβγ, but does not significantly bind to or activate cell expressing only IL2Rβγ. In some examples, an IL2 mutein may be an IL2 protein in which residues of IL2 responsible for binding to either IL2Rβ or IL2Rγ are substituted and abolish their interaction. In other examples, an IL2 mutein may be an IL2 protein comprising mutations conferring high affinity for IL2Rα. An IL2 mutein may be an IL2 selective agonist (IL2_{SA}) which can preferentially activate the high affinity IL2 receptor (i.e., IL2Rαβγ) which is necessary to selectively activate T cells with respect to NK cells. In some instances, the IL2 mutein may be IL2 protein which preferentially binds to the lower affinity IL2Rβγ but with reduced affinity to CD25.

In some instances, IL2 muteins may be used to preferentially expand or stimulate Treg cells. As used herein "preferentially expand or stimulate Treg cells" means the IL2 muteins promote the proliferation, survival, activation and /or function of T regulatory cells.

Exemplary IL2 muteins may include, but are not limited to, N88R substitution (Shanafelt et al., Nature Biotech., 2000, 18:1197-1202), an IL2 with a V91K substitution (e.g., US Patent publication NO. US20140286898); V91K substitution, C125A substitution, an IL2 with three mutations: V69A, N71R, Q74P; an IL2 mutein with high affinity for IL2Rα (N29S, Y31H, K35R, T37A, K48E, V69A, N71R, Q74P); an IL2 mutein with high affinity for IL2Rα and reduced signaling activity (N29S, Y31H, K35R, T37A, K48E, V69A, N71R, Q74P, N88D), and D20H, D20I, N88G, N88I, N88R, and Q126L substitutions as described in PCT application NO. 1999060128. In other aspects, IL2 muteins may include those described in US Pat.NO.: 4,518,584; 5,116,943*;* 5,206,344*;* 6,955,807; 7,105,653; 7,371,371; 7,803,361*;* 8,124,066*;* 8,349,311*;* 8,759,486; and 9,206,243; PCT patent publication NOs: WO2005086751 and WO2012088446; EP Pat. NOs: EP0234599 and EP0200280 and Sim, G.C. et al. (2016) Cancer Immunol Res; 4(11):983-994.

In some aspects, the IL2 mutein may be fused to a polypeptide that extends the serum half-life of the IL2 mutein, such as an IgG Fc fragment. Preferred Fc regions are derived from human IgG, which includes IgG1, IgG2, IgG3, and IgG4. In other aspects, the payload of the disclosure may be an IL2 fusion protein comparing a second functional polypeptide. In a non-limiting example, an IL2 fusion protein may comprise an IL2 or IL2 mutein polypeptide fused with a pro-apoptotic Bcl-2 family polypeptide (such as Bad, Bik/Nbk, Bid, Bim/Bod, Hrk, Bak or Bax); such fusion protein may be capable of inhibiting cell survival, inhibiting cell proliferation, or enhancing cell death or apoptosis of a target cell expressing an IL2 receptor. Alternatively, an IL2 or IL2 mutein polypeptide may be fused with an anti-apoptotic Bcl-2 family polypeptide (such as Bcl-x_{L}, Bcl-w or Bcl-2). The fusion protein may be capable of enhancing cell survival, enhancing cell proliferation, or inhibiting cell death or apoptosis of a target cell expressing an IL2 receptor. See, e.g., US patent publication NO.: US2016/0229901.

In addition, the IL2 fusion protein may be a IL2-GMCSF fusion protein which can promote cell-cell interaction; therefore, enhances anti-cancer immune responses (Wen et al., J. Translational Med., 2016, 14: 41).

In one instance, the payload of the disclosure may comprise IL12. IL12 is a heterodimeric protein of two subunits (p35, p40) that is secreted by antigen presenting cells, such as macrophages and dendritic cells. IL12 is type 1 cytokine that acts on natural killer (NK) cells, macrophages, CD8⁺ Cytotoxic T cells, and CD4⁺ T helper cells through STAT4 pathway to induce IFN γ production in these effector immune cells (reviewed by Trinchieri G, Nat Rev Immunol. 2003; 3(2): 133-146). IL12 can promote the cytotoxic activity of NK cells and CD8⁺ T cells, therefore has anti-tumor function. Intravenous injection of recombinant IL12 exhibited modest clinical efficacy in a handful of patients with advanced melanoma and renal cell carcinoma (Gollob et al., Clin. Cancer Res. 2000; 6(5):1678-1692). IL 12 has been used as an adjuvant to enhance cytotoxic immunity using a melanoma antigen vaccine, or using peptide pulsed peripheral blood mononuclear cells; and to promote NK cell activity in breast cancer with trastuzumab treatment. Local delivery of IL12 to the tumor microenvironment promotes tumor regression in several tumor models. These studies all indicate that locally increased IL12 level can promote anti-tumor immunity. One major obstacle of systemic or local administration of recombinant IL 12 protein, or through oncolytic viral vectors is the severe side effects when IL12 is presented at high level. Developing a system that tightly controls IL12 level may provide a safe use of IL12 in cancer treatment.

In one aspect, the effector module of the disclosure may be a DD-IL12 fusion polypeptide. This regulatable DD-IL 12 fusion polypeptide may be directly used as an immunotherapeutic agent or be transduced into an immune effector cell (T cells and TIL cells) to generate modified T cells with greater *in vivo* expansion and survival capabilities for adoptive cell transfer. The need for harsh preconditioning regimens in current adoptive cell therapies may be minimized using regulated IL12. DD-IL12 may be utilized to modify tumor microenvironment and increase persistence in solid tumors that are currently refractory to tumor antigen targeted therapy. In some instances, CAR expressing T cells may be armored with DD regulated IL12 to relieve immunosuppression without systemic toxicity.

In some instances, the IL12 may be a Flexi IL12, wherein both p35 and p40 subunits, are encoded by a single cDNA that produces a single chain polypeptide. In one instance, the IL12 may comprise p40 subunit, which includes amino acids 23-328 of wildtype IL12B and comprise the amino acid sequence of SEQ ID NO: 8302 (encoded by SEQ ID NO: 8303-8312) and a p35 subunit, which includes amino acids 57-253 of wildtype IL12A and comprise the amino acid sequence of SEQ ID NO: 8313 (encoded by SEQ ID NO: 8314-8323). Any portion of IL12 that retains one or more functions of full length or mature IL 12 may be useful in the present disclosure.

In some instances, DD regulated IL12 compositions of the disclosure may be utilized to minimize the cytotoxicity associated with systemic IL12 administration. Treatment with IL12 has been associated with systemic flu-like symptoms (fever, chills, fatigue, erythromelalgia, headache), toxic effects on the bone marrow, and liver. Hematologic toxicity observed most commonly included neutropenia and thrombocytopenia; hepatic dysfunction manifested in transient (dose dependent) increase in transaminases, hyperbilirubinemia and hypoalbuminemia. In some instances, toxicity is also associated with inflammation of the mucus membranes (oral mucositis, stomatitis or colitis). These toxic effects of IL12 were related to the secondary production of IFNγ, TNF alpha, and chemokines such as IP10, and MIG. In certain aspects of the disclosure, DD regulated IL12 may be utilized to prevent the toxic effects associated with elevated production of secondary messengers. In some instances, DD regulated Flexi-IL12 constructs may be used to improve the efficacy of the CARs, especially in solid tumor settings, by providing a controlled local signal for tumor microenvironment remodeling and epitope spreading. DD regulation also provides rapid, dose dependent, and local production of Flexi IL12.

The format of the IL12 constructs utilized as payload of the present disclosure may be optimized. In one instance, the payload of the disclosure may be a bicistronic IL12 containing p40 and p35 subunits separated by an internal ribosome entry site or a cleavage site such as P2A or Furin to allow independent expression of both subunits from a single vector. This results in a configuration of secreted IL12 that is more akin to the naturally occurring IL12 than the flexi IL12 construct, the payload of the disclosure may be the p40 subunit of the IL12. DD regulated p40 may be co-expressed with constitutive p35 construct to generate "regulatable IL12" expression. Alternatively, the DD regulated p40 may heterodimerize with the endogenous p35. p40 has been shown to stabilize p35 expression and stimulate the export of p35 (JalahR, et al. (2013). Journal of Biol. Chem. 288, 6763-6776.

In some instances, modified forms of IL12 may be utilized as the payload. These modified forms of IL12 may be engineered to have shortened half-life in vivo compared to the non-modified form of especially when used in combination with tunable systems described herein.

Human flexi IL12 has a reported half-life of 5-19 hours which, when administered as a therapeutic compound, can result in systemic cytotoxicity (Car et al. (1999) The Toxicology of Interleukin-12: A Review" Toxicologic Path.27 A, 58-63; Robertson et al. (1999) "Immunological Effects of Interleukin 12 Administered by Bolus Intravenous Injection to Patients with Cancer" Clin. Cancer Res. 5:9-16; Atkins et al. (1997)"Phase I Evaluation of Intravenous Recombinant Human Interleukin 12 in Patients with Advance Malignancies" Clin. Cancer Res. 3:409-417). The ligand inducible control of IL12 can regulate production in a dose dependent fashion, the time from cessation of ligand dosing to cessation of protein synthesis and IL12 clearance may be insufficient to prevent toxic accumulation of IL12 in plasma.

In one instance, the modified form of IL12 utilized as the payload may be a Topo-sc IL12 which have the configuration as follows from N to C terminus (i) a first IL12 p40 domain (p40N), (ii) an optional first peptide linker, (iii) an IL12 p35 domain, (iv) an optional second peptide linker, and (v) a second IL12 p40 domain (p40C). In one instance, modified topo-sc IL12 polypeptides exhibit increased susceptibility to proteolysis. Topo-sc IL12 is described in International Patent Publication No. WO2016048903.

IL12 polypeptide may also be modified (e.g. genetically, synthetically, or recombinantly engineered) to increase susceptibility to proteinases to reduce the biologically active half-life of the IL12 complex, compared to a corresponding IL12 lacking proteinases susceptibility. Proteinase susceptible forms of IL12 are described in International Patent Publication No. WO2017062953.

IL12 systemic toxicity may also be limited or tightly controlled via mechanisms involving tethering IL12 to the cell surface to limit its therapeutic efficacy to the tumor site. Membrane tethered IL12 forms have been described previously using Glycosyl phosphatidylinositol (GPI) signal peptide or using CD80 transmembrane domain (Nagarajan S, et al. (2011) J Biomed Mater Res A. 99(3):410-7; Bozeman EN, et al. (2013) Vaccine. 7;31(20):2449-56; Wen-Yu Pan et al. (2012), Mol. Ther.20:5, 927-937). In some instances, transmembrane domains may be selected from any of those described in Table 13, Table 14, and Table 15 of co-owned U.S. Provisional Patent Application No. 62/683,972, filed June 12,2018, and the International Patent Application No. PCT/US18/37005 filed on June 12, 2018.

In some instances, the IL12 levels secreted by the immune cells of the disclosure may approximately be comparable to the IL12 levels secreted by human myeloid dendritic cells (mDC1), when activated with TLR agonists. In one instance, the TLR agonist may be the combination of lipopolysaccharide administered with R848.

In some instances, the IFN gamma secreted by IL12 induced activation of the immune cells is at least 5-fold greater in the presence of ligand, compared to the levels in the absence of ligand.

In some instances, regulation of IL12 provides the necessary safety switch. In some instances, IL-12 secretion recruit and/or activates effector cells in the tumor microenvironment. In some instances, the IL12 regulation provides a benefit to CAR T function without causing toxicity.

In one instance, the payload of the disclosure may comprise IL15. Interleukin 15 is a potent immune stimulatory cytokine and an essential survival factor for T cells, and Natural Killer cells. Preclinical studies comparing IL2 and IL15, have shown than IL15 is associated with less toxicity than IL2. In some instances, the effector module of the disclosure may be a DD-IL15 fusion polypeptide. IL15 polypeptide may also be modified to increase its binding affinity for the IL15 receptor. For example, the asparagine may be replaced by aspartic acid at position 72 of IL15 (SEQ ID NO: 2 of US patent publication US20140134128A1). In some aspects, the IL15 comprises amino acid sequence of SEQ ID NO: 8234 (encoded by SEQ ID NO: 8236), which include amino acids 49-162 of wildtype IL15. In some instances the IL15 sequence may include a stop codon and may be encoded by the nucleotide sequence of SEQ ID NO: 8235, 8237-8238.

A unique feature of IL15 mediated activation is the mechanism of trans-presentation in which IL15 is presented as a complex with the alpha subunit of IL15 receptor (IL15Ra) that binds to and activates membrane bound IL15 beta/gamma receptor, either on the same cell or a different cell. The IL15/IL15Ra complex is more effective in activating IL15 signaling, than IL15 by itself. Thus, in some instances, the effector module of the disclosure may include a DD-IL15/IL 15Ra fusion polypeptide. In one instance, the payload may be IL15/IL15Ra fusion polypeptide described in US Patent Publication NO.: US20160158285A1. The IL15 receptor alpha comprises an extracellular domain called the sushi domain which contains most of the structural elements necessary for binding to IL15. Thus, in some instances, payload may be the IL15/IL15Ra sushi domain fusion polypeptide described in US Patent Publication NO.: US20090238791A1.

Regulated IL15/IL15Ra may be used to promote expansion, survival and potency of CD8T_{EM} cell populations without impacting regulatory T cells, NK cells and TIL cells. In one instance, DD-IL15/IL15Ra may be utilized to enhance CD 19 directed T cell therapies in B cell leukemia and lymphomas. In one aspect, IL15/IL15Ra may be used as payload of the disclosure to reduce the need for pre-conditioning regimens in current CAR-T treatment paradigms.

The effector modules containing DD-IL15, DD-IL15/IL15Ra and/or DD-IL15/IL15Ra sushi domain may be designed to be secreted (using e.g. IL2 signal sequence) or membrane bound (using e.g. IgE or CD8a signal sequence).

In some instances, the IFN gamma secreted by IL15 induced activation of the immune cells is at least 10-fold greater in the presence of ligand, compared to the levels in the absence of ligand.

In some instances, regulation of IL15-IL15Ra fusion proteins provides a safety switch as compared to constitutively expressed IL15-IL15Ra. In some instances, IL-15-IL15Ra leads to better expansion, and/or persistence of CAR T cells.

In one instance, regulated cytokines may enable CAR-T in solid tumors to overcome stromal barriers by improving tumor homing, reducing immunosuppression, and reducing tumor promoting conditions. In one instance, regulated cytokines may enable CAR-T in solid tumors to overcome Antigen negative escape by promoting epitope spreading, antigen presenting cell trafficking, activation and licensing. In one instance, regulated cytokines may enable CAR-T in solid tumors to overcome Antigen positive escape by improving expansion, increasing persistence, reducing exhaustion of T cells. In one instance, regulated cytokines enable local, on demand production of cytokines can safely improve efficacy. In one instance, regulated cytokines enable pulsatile production that can reduce feedback inhibition of cytokine signaling. In one instance, regulated cytokines can reduce senescence or exhaustion. In one instance, regulated cytokines enable on demand expression in patient which may reduce any effect of cytokine on cell phenotype during product manufacturing.

In some instances, the payloads may proteins that induce the expression of IL 15 such as proteins in the STING pathway and/or Type I interferons. In one aspect, the payload may be STING agonist. In one aspect, such expressing IL15 directly as a payload or using payloads that induce IL15 expression may use to regulate the tumor-infiltrating lymphocyte numbers in the tumor microenvironment. Any of the payloads described by Carrero et al. to induce IL15 expression may be used herein (Carrero et al. 2019 PNAS Jan 2019, 116 (2) 599-608; DOI: 10. 1073/pnas. 18 14642116).

In some aspects, the DD-IL115/IL15Ra comprises the amino acid sequences provided in Table 19. In some instances, the linker utilized in Table 19 or Table 20 may be SG linker. In Table 19, asterisk indicates the translation of the stop codon. In some instances, the DDs described in Table 19 may contain an additional stop codon. As used herein the wildtype (WT) of IL15 refers to Uniprot ID: P40933 and wildtype (WT) of IL15Ra refers to UniProt ID: Q13261.

**Table 19: IL15/IL15Ra and IL15 constructs**

| **Description/ Construct ID** | **Amino Acid Sequence** | **AA SEQ ID NO** | **NA SEQ ID NO** |
|---|---|---|---|
| IL15 (49-162 of WT) | NWVNVISDLKKIEDLIQSMHIDATLYTESDVHPSCKVTAMKCFLLELQVISLE SGDASIHDTVENLIILANNSLSSNGNVTESGCKECEELEEKNIKEFLQSFVHIV QMFINTS | 8324 | 8326; 8344 |
| IL15Ra (31-267 of WT) | ITCPPPMSVEHADIWVKSYSLYSRERYICNSGFKRKAGTSSLTECVLNKATN VAHWTTPSLKCIRDPALVHQRPAPPSTVTTAGVTPQPESLSPSGKEPAASSPS SNNTAATTAAIVPGSQLMPSKSPSTGTTEISSHESSHGTPSQTTAKNWELTAS ASHQPPGVYPQGHSDTTVAISTSTVLLCGLSAVSLLACYLKSRQTPPLASVE MEAMEALPVTWGTSSRDEDLENCSHHL | 8329 | 8345; 8346 |
| IgE leader | MDWTWILFLVAAATRVHS | 276 | 277 |
| linker (GS) | GS | - | GGATCC; GGTTCC |
| Linker (GSG) (BamH1-Gly) | GSG | - | GGATCCG GT |
| SG3-(SG4)3-SG3-SLQ linker | SGGGSGGGGSGGGGSGGGGSGGGSLQ | 323 | 324; 8665 |
| SG Linker | SG | - | AGTGGT |
| GSGSGS linker | GSGSGS | 8330 | 8347 |
| GSGSGSGS linker | GSGSGSGS | 8331 | 8348 |
| GSGSGGGSGS linker | GSGSGGGSGS | 8332 | 8349 |
| hPDE5 (Amino acid 535-860 of WT) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRRGEFFELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 3 | 339 |
| hPDE5 (Amino acid 535-860 of WT, R732L) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRLGEFFELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 12 | 359 |
| hPDE5 (Amino acid 535-860 of WT, R732L, F736A) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRLGEFAELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 227 | 233 |
| hPDE5 (Amino acid 535-860 of WT, R732L, F736A) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRLGEFAELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 227 | 233 |
| hPDE5 (Amino acid 535-860 of WT, F736A) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRRGEFAELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 9 | 355 |
| hPDE5 (Amino acid 535-860 of WT, R7321) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRIGEFFELIRKNQF NLEDPHQKELFLAMLMTACDLSAITKPWPIQQRIAELVATEFFDQGDRERKE LNIEPTDLMNREKKNKIPSMQVGFIDAICLQLYEALTHVSEDCFPLLDGCRK NRQKWQALAEQQ | 386 | 8551 |
| hPDE5 (Amino acid 535-860 of WT, R732G) | EETRELQSLAAAVVPSAQTLKITDFSFSDFELSDLETALCTIRMFTDLNLVQN FQMKHEVLCRWILSVKKNYRKNVAYHNWRHAFNTAQCMFAALKAGKIQN KLTDLEILALLIAALSHDLDHRGVNNSYIQRSEHPLAQLYCHSIMEHHHFDQ CLMILNSPGNQILSGLSIEEYKTTLKIIKQAILATDLALYIKRGGEFFELIRKNQ FNLEDPHQKELFLAMLMTACDLSAITKPWPIQQRIAELVATEFFDQGDRERK ELNIEPTDLMNREKKNKIPSMQVGFIDAICLQLYEALTHVSEDCFPLLDGCRK NRQKWQALAEQQ | 383 | 402 |
| AcGFP (Amino acid 2-239 of WT) | VSKGAELFTGIVPILIELNGDVNGHKFSVSGEGEGDATYGKLTLKFICTTGKL PVPWPTLVTTLSYGVQCFSRYPDHMKQHDFFKSAMPEGYIQERTIFFEDDGN YKSRAEVKFEGDTLVNRIELTGTDFKEDGNILGNKMEYNYNAHNVYIMTDK AKNGIKVNFKIRHNIEDGSVQLADHYQQNTPIGDGPVLLPDNHYLSTQSALS KDPNEKRDHMIYFGFVTAAAITHGMDELYK | 79 | 372 |
| mCherry | MSKGEEDNMAIIKEFMRFKVHMEGSVNGHEFEIEGEGEGRPYEGTQTAKLK VTKGGPLPFAWDILSPQFMYGSKAYVKHPADIPDYLKLSFPEGFKWERVMN FEDGGVVTVTQDSSLQDGEFIYKVKLRGTNFPSDGPVMQKKTMGWEASSER MYPEDGALKGEIKQRLKLKDGGHYDAEVKTTYKAKKPVQLPGAYNVNIKL DITSHNEDYTIVEQYERAEGRHSTGGMDELYK | 8648 | 8666 |
| mCherry (M1L) | LSKGEEDNMAIIKEFMRFKVHMEGSVNGHEFEIEGEGEGRPYEGTQTAKLK VTKGGPLPFAWDILSPQFMYGSKAYVKHPADIPDYLKLSFPEGFKWERVMN FEDGGVVTVTQDSSLQDGEFIYKVKLRGTNFPSDGPVMQKKTMGWEASSER MYPEDGALKGEIKQRLKLKDGGHYDAEVKTTYKAKKPVQLPGAYNVNIKL DITSHNEDYTIVEQYERAEGRHSTGGMDELYK | 8240 | 8269 |
| FLAG-tag | DYKDDDDK | 8333 | 8350 |
| HA Tag | YPYDVPDYA | 8238 | 8351 |
| Modified Furin | ESRRVRRNKRSK | 288 | 291 |
| P2A Cleavable peptide | GATNFSLLKQAGDVEENPGP | 8239 | 8270 |
| IRES | - | - | 8667 |
| Spacer | - | - | 8668 |
| Spacer | - | - | 8669 |
| OT-001254 (IgE signal sequence; IL15 (Amino acid 49-162 of WT); linker (SG3-(SG4)3-SG3-SLQ (SEQ ID NO: 323)); IL15Ra (Amino acid 31-267 of WT); linker (SG); hPDE5 (Amino acid 535-860 of WT, R732L, F736A); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGEETRELQSLAAAVVPSAQTLKI TDFSFSDFELSDLETALCTIRMFTDLNLVQNFQMKHEVLCRWILSVKKNYRK NVAYHNWRHAFNTAQCMFAALKAGKIQNKLTDLEILALLIAALSHDLDHR GVNNSYIQRSEHPLAQLYCHSIMEHHHFDQCLMILNSPGNQILSGLSIEEYKT TLKIIKQAILATDLALYIKRLGEFAELIRKNQFNLEDPHQKELFLAMLMTACD | 8334 | 8352 |
| | LSAITKPWPIQQRIAELVATEFFDQGDRERKELNIEPTDLMNREKKNKIPSMQ VGFIDAICLQLYEALTHVSEDCFPLLDGCRKNRQKWQALAEQQ* | | |
| OT-001469 (IgE signal sequence; IL15 (Amino acid 49-162 of WT); linker (SG3-(SG4)3-SG3-SLQ (SEQ ID NO: 323)); IL15Ra (Amino acid 31-267 of WT); linker (SG); AcGFP (Amino acid 2-239 of WT); linker (SG); hPDE5 (Amino acid 535-860 of WT, R732L, F736A); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGVSKGAELFTGIVPILIELNGDVN GHKFSVSGEGEGDATYGKLTLKFICTTGKLPVPWPTLVTTLSYGVQCFSRYP DHMKQHDFFKSAMPEGYIQERTIFFEDDGNYKSRAEVKFEGDTLVNRIELTG TDFKEDGNILGNKMEYNYNAHNVYIMTDKAKNGIKVNFKIRHNIEDGSVQL ADHYQQNTPIGDGPVLLPDNHYLSTQSALSKDPNEKRDHMIYFGFVTAAAIT HGMDELYKSGEETRELQSLAAAVVPSAQTLKITDFSFSDFELSDLETALCTIR MFTDLNLVQNFQMKHEVLCRWILSVKKNYRKNVAYHNWRHAFNTAQCMF AALKAGKIQNKLTDLEILALLIAALSHDLDHRGVNNSYIQRSEHPLAQLYCH SIMEHHHFDQCLMILNSPGNQILSGLSIEEYKTTLKIIKQAILATDLALYIKRL GEFAELIRKNQFNLEDPHQKELFLAMLMTACDLSAITKPWPIQQRIAELVAT EFFDQGDRERKELNIEPTDLMNREKKNKIPSMQVGFIDAICLQLYEALTHVSE DCFPLLDGCRKNRQKWQALAEQQ* | 8335 | 8353 |
| OT-001470 (IgE signal sequence; IL15 (Amino acid 49-162 of WT); linker (SG3-(SG4)3-SG3-SLQ (SEQ ID NO: 323)); IL15Ra (Amino acid 31-267 of WT); linker (SG); hPDE5 (Amino acid 535-860 of WT, R732L, F736A); linker (SG); AcGFP (Amino acid 2-239 of WT); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGEETRELQSLAAAVVPSAQTLKI TDFSFSDFELSDLETALCTIRMFTDLNLVQNFQMKHEVLCRWILSVKKNYRK NVAYHNWRHAFNTAQCMFAALKAGKIQNKLTDLEILALLIAALSHDLDHR GVNNSYIQRSEHPLAQLYCHSIMEHHHFDQCLMILNSPGNQILSGLSIEEYKT TLKIIKQAILATDLALYIKRLGEFAELIRKNQFNLEDPHQKELFLAMLMTACD LSAITKPWPIQQRIAELVATEFFDQGDRERKELNIEPTDLMNREKKNKIPSMQ VGFIDAICLQLYEALTHVSEDCFPLLDGCRKNRQKWQALAEQQSGVSKGAE LFTGIVPILIELNGDVNGHKFSVSGEGEGDATYGKLTLKFICTTGKLPVPWPT LVTTLSYGVQCFSRYPDHMKQHDFFKSAMPEGYIQERTIFFEDDGNYKSRAE VKFEGDTLVNRIELTGTDFKEDGNILGNKMEYNYNAHNVYIMTDKAKNGIK VNFKIRHNIEDGSVQLADHYQQNTPIGDGPVLLPDNHYLSTQSALSKDPNEK RDHMIYFGFVTAAAITHGMDELYK* | 8336 | 8354 |
| OT-001315 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (SG); Furin cleavage site (ESRRVRRNKRSK); hPDE5 (Amino acid 535-860 of WT, R732L); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGESRRVRRNKRSKEETRELQSLA AAVVPSAQTLKITDFSFSDFELSDLETALCTIRMFTDLNLVQNFQMKHEVLC RWILSVKKNYRKNVAYHNWRHAFNTAQCMFAALKAGKIQNKLTDLEILAL LIAALSHDLDHRGVNNSYIQRSEHPLAQLYCHSIMEHHHFDQCLMILNSPGN QILSGLSIEEYKTTLKIIKQAILATDLALYIKRLGEFFELIRKNQFNLEDPHQKE LFLAMLMTACDLSAITKPWPIQQRIAELVATEFFDQGDRERKELNIEPTDLM NREKKNKIPSMQVGFIDAICLQLYEALTHVSEDCFPLLDGCRKNRQKWQAL AEQQ* | 8337 | 8355 |
| OT-001316 (IgE signal sequence; IL15 (Amino acid 49-162 of WT); Linker (SG3-(SG4)3-SG3-SLQ | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP | 8338 | 8356 |
| (SEQ ID NO: 323)); IL15Ra (Amino acid 31-267 of WT); Linker (SG); hPDE5 (Amino acid 535-860 of WT, R732L); stop) | SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGEETRELQSLAAAVVPSAQTLKI TDFSFSDFELSDLETALCTIRMFTDLNLVQNFQMKHEVLCRWILSVKKNYRK NVAYHNWRHAFNTAQCMFAALKAGKIQNKLTDLEILALLIAALSHDLDHR GVNNSYIQRSEHPLAQLYCHSIMEHHHFDQCLMILNSPGNQILSGLSIEEYKT TLKIIKQAILATDLALYIKRLGEFFELIRKNQFNLEDPHQKELFLAMLMTACD LSAITKPWPIQQRIAELVATEFFDQGDRERKELNIEPTDLMNREKKNKIPSMQ VGFIDAICLQLYEALTHVSEDCFPLLDGCRKNRQKWQALAEQQ* | | |
| OT-001317 (IgE signal sequence; IL15 (Amino acid 49-162 of WT); Linker (SG3-(SG4)3-SG3-SLQ (SEQ ID NO: 323)); IL15Ra (Amino acid 31-267 of WT); linker (SG); hPDE5 (Amino acid 535-860 of WT); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGEETRELQSLAAAVVPSAQTLKI TDFSFSDFELSDLETALCTIRMFTDLNLVQNFQMKHEVLCRWILSVKKNYRK NVAYHNWRHAFNTAQCMFAALKAGKIQNKLTDLEILALLIAALSHDLDHR GVNNSYIQRSEHPLAQLYCHSIMEHHHFDQCLMILNSPGNQILSGLSIEEYKT TLKIIKQAILATDLALYIKRRGEFFELIRKNQFNLEDPHQKELFLAMLMTACD LSAITKPWPIQQRIAELVATEFFDQGDRERKELNIEPTDLMNREKKNKIPSMQ VGFIDAICLQLYEALTHVSEDCFPLLDGCRKNRQKWQALAEQQ* | 8339 | 8357 |
| OT-001499 (IgE signal sequence; IL15 (Amino acid 49-162 of WT); Linker (SG3-(SG4)3-SG3-SLQ (SEQ ID NO: 323)); IL15Ra (Amino acid 31-267 of WT); linker (SG); hPDE5 (Amino acid 535-860 of WT); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLSGEETRELQSLAAAVVPSAQTLKI TDFSFSDFELSDLETALCTIRMFTDLNLVQNFQMKHEVLCRWILSVKKNYRK NVAYHNWRHAFNTAQCMFAALKAGKIQNKLTDLEILALLIAALSHDLDHR GVNNSYIQRSEHPLAQLYCHSIMEHHHFDQCLMILNSPGNQILSGLSIEEYKT TLKIIKQAILATDLALYIKRRGEFFELIRKNQFNLEDPHQKELFLAMLMTACD LSAITKPWPIQQRIAELVATEFFDQGDRERKELNIEPTDLMNREKKNKIPSMQ VGFIDAICLQLYEALTHVSEDCFPLLDGCRKNRQKWQALAEQQ* | 8340 | 8358 |
| OT-001344 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); linker (GSGSGS (SEQ ID NO: 8330)); hPDE5 (Amino acid 535-860 of WT, R732L); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGS GSEETRELQSLAAAVVPSAQTLKITDFSFSDFELSDLETALCTIRMFTDLNLV QNFQMKHEVLCRWILSVKKNYRKNVAYHNWRHAFNTAQCMFAALKAGKI QNKLTDLEILALLIAALSHDLDHRGVNNSYIQRSEHPLAQLYCHSIMEHHHF DQCLMILNSPGNQILSGLSIEEYKTTLKIIKQAILATDLALYIKRLGEFFELIRK NQFNLEDPHQKELFLAMLMTACDLSAITKPWPIQQRIAELVATEFFDQGDRE RKELNIEPTDLMNREKKNKIPSMQVGFIDAICLQLYEALTHVSEDCFPLLDGC RKNRQKWQALAEQQ* | 8341 | 8359 |
| OT-001345 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGS | 8342 | 8360 |
| WT); linker (GSGSGSGS (SEQ ID NO: 8331)); hPDE5 (Amino acid 535-860 of WT, R732L); stop) | GSGSEETRELQSLAAAVVPSAQTLKITDFSFSDFELSDLETALCTIRMFTDLNL VQNFQMKHEVLCRWILSVKKNYRKNVAYHNWRHAFNTAQCMFAALKAG KIQNKLTDLEILALLIAALSHDLDHRGVNNSYIQRSEHPLAQLYCHSIMEHHH FDQCLMILNSPGNQILSGLSIEEYKTTLKIIKQAILATDLALYIKRLGEFFELIR KNQFNLEDPHQKELFLAMLMTACDLSAITKPWPIQQRIAELVATEFFDQGDR ERKELNIEPTDLMNREKKNKIPSMQVGFIDAICLQLYEALTHVSEDCFPLLDG CRKNRQKWQALAEQQ* | | |
| OT-001346 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); linker (GSGSGGGSGS (SEQ ID NO: 8332)); hPDE5 (Amino acid 535-860 of WT, R732L); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGS GGGSGSEETRELQSLAAAVVPSAQTLKITDFSFSDFELSDLETALCTIRMFTD LNLVQNFQMKHEVLCRWILSVKKNYRKNVAYHNWRHAFNTAQCMFAALK AGKIQNKLTDLEILALLIAALSHDLDHRGVNNSYIQRSEHPLAQLYCHSIMEH HHFDQCLMILNSPGNQILSGLSIEEYKTTLKIIKQAILATDLALYIKRLGEFFEL IRKNQFNLEDPHQKELFLAMLMTACDLSAITKPWPIQQRIAELVATEFFDQG DRERKELNIEPTDLMNREKKNKIPSMQVGFIDAICLQLYEALTHVSEDCFPLL DGCRKNRQKWQALAEQQ* | 8343 | 8361 |
| OT-001318 (IgE signal sequence; HA Tag; IL15 (Amino acid 49-162 of WT); Linker (SG3-(SG4)3-SG3-SLQ (SEQ ID NO: 323)); IL15Ra (Amino acid 31-267 of WT); Linker (SG); hPDE5 (Amino acid 535-860 of WT); stop) | MDWTWILFLVAAATRVHSYPYDVPDYANWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQITCPPPMSVEHADIWVKSYSLYSRERYICNSGFKRKAGTSS LTECVLNKATNVAHWTTPSLKCIRDPALVHQRPAPPSTVTTAGVTPQPESLS PSGKEPAASSPSSNNTAATTAAIVPGSQLMPSKSPSTGTTEISSHESSHGTPSQ TTAKNWELTASASHQPPGVYPQGHSDTTVAISTSTVLLCGLSAVSLLACYLK SRQTPPLASVEMEAMEALPVTWGTSSRDEDLENCSHHLSGEETRELQSLAA AVVPSAQTLKITDFSFSDFELSDLETALCTIRMFTDLNLVQNFQMKHEVLCR WILSVKKNYRKNVAYHNWRHAFNTAQCMFAALKAGKIQNKLTDLEILALLI AALSHDLDHRGVNNSYIQRSEHPLAQLYCHSIMEHHHFDQCLMILNSPGNQI LSGLSIEEYKTTLKIIKQAILATDLALYIKRRGEFFELIRKNQFNLEDPHQKELF LAMLMTACDLSAITKPWPIQQRIAELVATEFFDQGDRERKELNIEPTDLMNR EKKNKIPSMQVGFIDAICLQLYEALTHVSEDCFPLLDGCRKNRQKWQALAE QQ* | 8649 | 8670 |
| OT-001314 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GSG), hPDE5 (Amino acid 535-860 of WT, F736A), stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRRGEFAELIRKNQFNLEDPHQKELFLAMLMTAC DLSAITKPWPIQQRIAELVATEFFDQGDRERKELNIEPTDLMNREKKNKIPSM QVGFIDAICLQLYEALTHVSEDCFPLLDGCRKNRQKWQALAEQQ* | 8650 | 8671 |
| OT-001319 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GSG); hPDE5 | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR | 8651 | 8672 |
| (Amino acid 535-860 of WT, R732L, F736A); stop) | KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRLGEFAELIRKNQFNLEDPHQKELFLAMLMTAC DLSAITKPWPIQQRIAELVATEFFDQGDRERKELNIEPTDLMNREKKNKIPSM QVGFIDAICLQLYEALTHVSEDCFPLLDGCRKNRQKWQALAEQQ* | | |
| OT-001320 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GSG); hPDE5 (Amino acid 535-860 of WT); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRRGEFFELIRKNQFNLEDPHQKELFLAMLMTAC DLSAITKPWPIQQRIAELVATEFFDQGDRERKELNIEPTDLMNREKKNKIPSM QVGFIDAICLQLYEALTHVSEDCFPLLDGCRKNRQKWQALAEQQ* | 8652 | 8673 |
| OT-001388 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GS); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGS* | 8653 | 8674 |
| OT-001321 (IgE signal sequence; Flag tag, 1L15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGE ETRELQSLAAAVVPSAQTLKITDFSFSDFELSDLETALCTIRMFTDLNLVQNF QMKHEVLCRWILSVKKNYRKNVAYHNWRHAFNTAQCMFAALKAGKIQNK LTDLEILALLIAALSHDLDHRGVNNSYIQRSEHPLAQLYCHSIMEHHHFDQCL MILNSPGNQILSGLSIEEYKTTLKIIKQAILATDLALYIKRRGEFFELIRKNQFN LEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKN RQKWQALAEQQ* | 8654 | 8675 |
| OT-001323 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, F736A); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGE ETRELQSLAAAVVPSAQTLKITDFSFSDFELSDLETALCTIRMFTDLNLVQNF QMKHEVLCRWILSVKKNYRKNVAYHNWRHAFNTAQCMFAALKAGKIQNK LTDLEILALLIAALSHDLDHRGVNNSYIQRSEHPLAQLYCHSIMEHHHFDQCL MILNSPGNQILSGLSIEEYKTTLKIIKQAILATDLALYIKRRGEFAELIRKNQFN LEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKN RQKWQALAEQQ* | 8655 | 8676 |
| OT-001324 (IgE signal sequence; Flag | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN | 8656 | 8677 |
| tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732L), stop) | GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGE ETRELQSLAAAVVPSAQTLKITDFSFSDFELSDLETALCTIRMFTDLNLVQNF QMKHEVLCRWILSVKKNYRKNVAYHNWRHAFNTAQCMFAALKAGKIQNK LTDLEILALLIAALSHDLDHRGVNNSYIQRSEHPLAQLYCHSIMEHHHFDQCL MILNSPGNQILSGLSIEEYKTTLKIIKQAILATDLALYIKRLGEFFELIRKNQFN LEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKN RQKWQALAEQQ* | | |
| OT-001325 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732L, F736A); stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGE ETRELQSLAAAVVPSAQTLKITDFSFSDFELSDLETALCTIRMFTDLNLVQNF QMKHEVLCRWILSVKKNYRKNVAYHNWRHAFNTAQCMFAALKAGKIQNK LTDLEILALLIAALSHDLDHRGVNNSYIQRSEHPLAQLYCHSIMEHHHFDQCL MILNSPGNQILSGLSIEEYKTTLKIIKQAILATDLALYIKRLGEFAELIRKNQFN LEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKN RQKWQALAEQQ* | 8657 | 8678 |
| OT-001349 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732L); V5 epitope; stop) | MDWTWILFLVAAATRVHSDYKDDDDKNWVNVISDLKKIEDLIQSMHIDAT LYTESDVHPSCKVTAMKCFLLELQVISLESGDASIHDTVENLIILANNSLSSN GNVTESGCKECEELEEKNIKEFLQSFVHIVQMFINTSSGGGSGGGGSGGGGS GGGGSGGGSLQYPYDVPDYAITCPPPMSVEHADIWVKSYSLYSRERYICNSG FKRKAGTSSLTECVLNKATNVAHWTTPSLKCIRDPALVHQRPAPPSTVTTAG VTPQPESLSPSGKEPAASSPSSNNTAATTAAIVPGSQLMPSKSPSTGTTEISSH ESSHGTPSQTTAKNWELTASASHQPPGVYPQGHSDTTVAISTSTVLLCGLSA VSLLACYLKSRQTPPLASVEMEAMEALPVTWGTSSRDEDLENCSHHLGSGE ETRELQSLAAAVVPSAQTLKITDFSFSDFELSDLETALCTIRMFTDLNLVQNF QMKHEVLCRWILSVKKNYRKNVAYHNWRHAFNTAQCMFAALKAGKIQNK LTDLEILALLIAALSHDLDHRGVNNSYIQRSEHPLAQLYCHSIMEHHHFDQCL MILNSPGNQILSGLSIEEYKTTLKIIKQAILATDLALYIKRLGEFFELIRKNQFN LEDPHQKELFLAMLMTACDLSAITKPWPIQQRIAELVATEFFDQGDRERKEL NIEPTDLMNREKKNKIPSMQVGFIDAICLQLYEALTHVSEDCFPLLDGCRKN RQKWQALAEQQGKPIPNPLLGLDST* | 8658 | 8679 |
| OT-001350 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GSG); hPDE5 (Amino acid 535-860 of WT, R732L); Linker (GS), stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRLGEFFELIRKNQFNLEDPHQKELFLAMLMTAC DLSAITKPWPIQQRIAELVATEFFDQGDRERKELNIEPTDLMNREKKNKIPSM QVGFIDAICLQLYEALTHVSEDCFPLLDGCRKNRQKWQALAEQQGS* | 8659 | 8680 |
| OT-001351 (IgE signal sequence; IL15 (Amino acid 49-162 of | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS | 8660 | 8681 |
| WT); Linker (SG3-(SG4)3-SG3-SLQ (SEQ ID NO: 323)); IL15Ra (Amino acid 31-267 of WT); Linker (GSG); hPDE5 (Amino acid 535-860 of WT, R7321); Linker (GS); stop) | LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRIGEFFELIRKNQFNLEDPHQKELFLAMLMTACD LSAITKPWPIQQRIAELVATEFFDQGDRERKELNIEPTDLMNREKKNKIPSMQ VGFIDAICLQLYEALTHVSEDCFPLLDGCRKNRQKWQALAEQQGS* | | |
| OT-001352 (IgE signal sequence; IL15 (Amino acid 49-162 of WT); Linker (SG3-(SG4)3-SG3-SLQ (SEQ ID NO: 323)); IL15Ra (Amino acid 31-267 of WT); Linker (GSG); hPDE5 (Amino acid 535-860 of WT, R732G); Linker (GS); stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSSGGGSGGGGSGGGGSGGGGSGGGS LQITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTA SASHQPPGVYPQGHSDTTVAISTSTVLLCGLSAVSLLACYLKSRQTPPLASVE MEAMEALPVTWGTSSRDEDLENCSHHLGSGEETRELQSLAAAVVPSAQTLK ITDFSFSDFELSDLETALCTIRMFTDLNLVQNFQMKHEVLCRWILSVKKNYR KNVAYHNWRHAFNTAQCMFAALKAGKIQNKLTDLEILALLIAALSHDLDH RGVNNSYIQRSEHPLAQLYCHSIMEHHHFDQCLMILNSPGNQILSGLSIEEYK TTLKIIKQAILATDLALYIKRGGEFFELIRKNQFNLEDPHQKELFLAMLMTAC DLSAITKPWPIQQRIAELVATEFFDQGDRERKELNIEPTDLMNREKKNKIPSM QVGFIDAICLQLYEALTHVSEDCFPLLDGCRKNRQKWQALAEQQGS* | 8661 | 8682 |
| OT-001347 (IgE signal sequence; IL15 (Amino acid 49-162 of WT); Linker (GS); hPDE5 (Amino acid 535-860 of WT, R732L, F736A); stop; spacer; IRES; Spacer; mcherry; stop) | ORF1: MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSGSEETRELQSLAAAVVPSAQTLKITD FSFSDFELSDLETALCTIRMFTDLNLVQNFQMKHEVLCRWILSVKKNYRKNV AYHNWRHAFNTAQCMFAALKAGKIQNKLTDLEILALLIAALSHDLDHRGV NNSYIQRSEHPLAQLYCHSIMEHHHFDQCLMILNSPGNQILSGLSIEEYKTTL KIIKQAILATDLALYIKRLGEFAELIRKNQFNLEDPHQKELFLAMLMTACDLS AITKPWPIQQRIAELVATEFFDQGDRERKELNIEPTDLMNREKKNKIPSMQV GFIDAICLQLYEALTHVSEDCFPLLDGCRKNRQKWQALAEQQ* ORF2: MSKGEEDNMAIIKEFMRFKVHMEGSVNGHEFEIEGEGEGRPYEGTQTAKLK VTKGGPLPFAWDILSPQFMYGSKAYVKHPADIPDYLKLSFPEGFKWERVMN FEDGGVVTVTQDSSLQDGEFIYKVKLRGTNFPSDGPVMQKKTMGWEASSER MYPEDGALKGEIKQRLKLKDGGHYDAEVKTTYKAKKPVQLPGAYNVNIKL DITSHNEDYTIVEQYERAEGRHSTGGMDELYK* | 8662 8663 | 8683 |
| OT-001348 (IgE signal sequence; IL15 (Amino acid 49-162 of WT); Linker (GS); hPDE5 (Amino acid 535-860 of WT, R732L, F736A); Linker (GS); P2A peptide; mcherry; stop) | MDWTWILFLVAAATRVHSNWVNVISDLKKIEDLIQSMHIDATLYTESDVHP SCKVTAMKCFLLELQVISLESGDASIHDTVENLIILANNSLSSNGNVTESGCK ECEELEEKNIKEFLQSFVHIVQMFINTSGSEETRELQSLAAAVVPSAQTLKITD FSFSDFELSDLETALCTIRMFTDLNLVQNFQMKHEVLCRWILSVKKNYRKNV AYHNWRHAFNTAQCMFAALKAGKIQNKLTDLEILALLIAALSHDLDHRGV NNSYIQRSEHPLAQLYCHSIMEHHHFDQCLMILNSPGNQILSGLSIEEYKTTL KIIKQAILATDLALYIKRLGEFAELIRKNQFNLEDPHQKELFLAMLMTACDLS AITKPWPIQQRIAELVATEFFDQGDRERKELNIEPTDLMNREKKNKIPSMQV GFIDAICLQLYEALTHVSEDCFPLLDGCRKNRQKWQALAEQQGSGATNFSLL KQAGDVEENPGPLSKGEEDNMAIIKEFMRFKVHMEGSVNGHEFEIEGEGEG RPYEGTQTAKLKVTKGGPLPFAWDILSPQFMYGSKAYVKHPADIPDYLKLSF PEGFKWERVMNFEDGGVVTVTQDSSLQDGEFIYKVKLRGTNFPSDGPVMQ KKTMGWEASSERMYPEDGALKGEIKQRLKLKDGGHYDAEVKTTYKAKKP VQLPGAYNVNIKLDITSHNEDYTIVEQYERAEGRHSTGGMDELYK* | 8664 | 8684 |

In some instances, any of the hPDE5 R732 mutants described in Table 6 may be appended to immunotherapeutic payloads such as IL15, IL15Ra or cytokine- cytokine receptor fusion polypeptides such IL15 fused to IL15Ra. Any of the linkers, cleavage peptides, and tags described in Table 19 may be used to build the generate described in Table 20.

**Table 20: IL15/IL15Ra-hPDE5 R732 constructs**

| **Construct ID (Description)** | **Amino Acid Sequence** | **AA SEQ ID NO** | **NA SEQ ID NO** |
|---|---|---|---|
| OT-001327 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R7321); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRIGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8685 | 8702 |
| OT-001328 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732M); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRMGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8686 | 8703 |
| OT-001329 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732V; stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRVGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8687 | 8704 |
| OT-001330 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732S); stop) | MDWTWILFLVAAATRVHSDYKDDDDKNWVNVISDLKKIEDLIQSM HIDATLYTESDVHPSCKVTAMKCFLLELQVISLESGDASIHDTVENLI | 8688 | 8705 |
| | ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRSGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | | |
| OT-001331 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732P); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRPGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8689 | 8706 |
| OT-001332 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732A); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRAGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8690 | 8707 |
| OT-001333 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732Y); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRYGEFFELIRKNQFNLEDPHQKELFLAMLMTACDLSAITKPWPIQQ | 8691 | 8708 |
| | RIAELVATEFFDQGDRERKELNIEPTDLMNREKKNKIPSMQVGFIDAI CLQLYEALTHVSEDCFPLLDGCRKNRQKWQALAEQQ* | | |
| OT-001334 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732H); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRHGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8692 | 8709 |
| OT-001335 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732Q); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRQGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8693 | 8710 |
| OT-001336 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732N); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRNGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8694 | 8711 |
| OT-001337 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732K); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ | 8695 | 8712 |
| | MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRKGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | | |
| OT-001338 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732D); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRDGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8696 | 8713 |
| OT-001339 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732E); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KREGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8697 | 8714 |
| OT-001340 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732C); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRCGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8698 | 8715 |
| OT-001341 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS | 8699 | 8716 |
| (GSG); hPDE5 (Amino acid 535-860 of WT, R732G); stop) | NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRGGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | | |
| OT-001342 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732W); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRWGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8700 | 8717 |
| OT-001343 (IgE signal sequence; Flag tag; IL15 (Amino acid 49-162 of WT); Linker (SG3-(SG4)3-SG3-SLQ (SEQ ID NO: 323)); HA tag; IL15Ra (Amino acid 31-267 of WT); Linker (GSG); hPDE5 (Amino acid 535-860 of WT, R732T); stop) | MDWTWILFLVAAATRVHSDYKDDDDKNWVNVISDLKKIEDLIQSM HIDATLYTESDVHPSCKVTAMKCFLLELQVISLESGDASIHDTVENLI ILANNSLSSNGNVTESGCKECEELEEKNIKEFLQSFVHIVQMFINTSS GGGSGGGGSGGGGSGGGGSGGGSLQYPYDVPDYAITCPPPMSVEH ADIWVKSYSLYSRERYICNSGFKRKAGTSSLTECVLNKATNVAHWT TPSLKCIRDPALVHQRPAPPSTVTTAGVTPQPESLSPSGKEPAASSPSS NNTAATTAAIVPGSQLMPSKSPSTGTTEISSHESSHGTPSQTTAKNWE LTASASHQPPGVYPQGHSDTTVAISTSTVLLCGLSAVSLLACYLKSR QTPPLASVEMEAMEALPVTWGTSSRDEDLENCSHHLGSGEETRELQ SLAAAVVPSAQTLKITDFSFSDFELSDLETALCTIRMFTDLNLVQNFQ MKHEVLCRWILSVKKNYRKNVAYHNWRHAFNTAQCMFAALKAG KIQNKLTDLEILALLIAALSHDLDHRGVNNSYIQRSEHPLAQLYCHSI MEHHHFDQCLMILNSPGNQILSGLSIEEYKTTLKIIKQAILATDLALYI KRTGEFFELIRKNQFNLEDPHQKELFLAMLMTACDLSAITKPWPIQQ RIAELVATEFFDQGDRERKELNIEPTDLMNREKKNKIPSMQVGFIDAI CLQLYEALTHVSEDCFPLLDGCRKNRQKWQALAEQQ* | 8701 | 8718 |

In some instances, the sequences described in Table 19, may contain an additional stop codon. For example, the construct "hPDE5 (Amino acid 535-860 of WT, R732L); stop" may be encoded by the nucleotide sequence of SEQ ID NO: 8362, and the construct "hPDE5 (Amino acid 535-860 of WT, R732L, F736A); stop" may be encoded by the nucleotide sequence of SEQ ID NO: 8363.

In one instance, the payload of the present disclosure may comprise IL18. IL18 is a proinflammatory and immune regulatory cytokine that promotes IFN γ production by T and NK cells. IL18 belongs to the IL1 family. Secreted IL 18 binds to a heterodimer receptor complex, consisting of IL18Rα and *β*-chains and initiates signal transduction. IL18 acts in concert with other cytokines to modulate immune system functions, including induction of IFN *γ* production, Th1 responses, and NK cell activation in response to pathogen products. IL18 showed anti-cancer effects in several tumors. Administration of recombinant IL18 protein or IL18 transgene induces melanoma or sarcoma regression through the activation of CD4⁺T and/or NK cell-mediated responses (reviewed by Srivastava et al., Curr. Med. Chem., 2010,17: 3353-3357). The combination of IL18 with other cytokines, such as IL12 or co-stimulatory molecules (e.g., CD80) increases IL18 anti-tumor effects. For example, IL18 and IL12A/B or CD80 genes have been integrated successfully in the genome of oncolytic viruses, with the aim to trigger synergistically T cell-mediated anti-tumor immune responses (Choi et al., Gene Ther., 2011, 18: 898-909). IL2/IL18 fusion proteins also display enhanced anti-tumor properties relative to either cytokine alone and low toxicity in preclinical models (Acres et al., Cancer Res., 2005, 65:9536-9546).

IL18 alone, or in combination of IL12 and IL15, activates NK cells. Preclinical studies have demonstrated that adoptively transferred IL12, IL15 and IL18 pre-activated NK cells display enhanced effector function against established tumors *in vivo* (Ni et al., J Exp Med. 2012, 209: 2351-2365; and Romee et al., Blood. 2012,120:4751-4760). Human IL12/IL15/IL18 activated NK cells also display memory-like features and secrete more IFN γ in response to cytokines (e.g., low concentration of IL2). In one instance, the effector module of the present disclosure may be a DD-IL18 fusion polypeptide.

In one instance, the payload of the present disclosure may comprise IL21. IL21 is another pleiotropic type I cytokine that is produced mainly by T cells and natural killer T (NKT) cells. IL21 has diverse effects on a variety of cell types including but not limited to CD4⁺ and CD8⁺ T cells, B cells, macrophages, monocytes, and dendritic cells (DCs). The functional receptor for IL21 is composed of IL21 receptor (IL21R) and the common cytokine receptor gamma chain, which is also a subunit of the receptors for IL2, IL4, IL7, IL9 and IL15. Studies provide compelling evidence that IL21 is a promising immunotherapeutic agent for cancer immunotherapy. IL21 promotes maturation, enhances cytotoxicity, and induces production of IFN *γ* and perforin by NK cells. These effector functions inhibit the growth of B16 melanoma (Kasaian et al., Immunity. 2002, 16(4):559-569; and Brady et al., J Immunol. 2004, 172(4):2048-2058). IL21 together with IL15 expands antigen-specific CD8⁺ T-cell numbers and their effector function, resulting in tumor regression (Zeng et al., J Exp Med.2005, 201(1):139-148). IL21 may also be used to rejuvenate multiple immune effector cells in the tumor microenvironment. IL21 may also directly induce apoptosis in certain types of lymphoma such as diffuse large B-cell lymphoma, mantle cell lymphoma, and chronic lymphocytic leukemia cells, via activation of STAT3 or STAT1 signal pathway. IL21, alone or in combination with anti-CD20 mAb (rituximab) can activate NK cell-dependent cytotoxic effects. Interestingly, discovery of the immunosuppressive actions of IL21 suggests that this cytokine is a "double-edged sword"- IL21 stimulation may lead to either the induction or suppression of immune responses. Both stimulatory and suppressive effects of IL21 must be considered when using IL21-related immunotherapeutic agents. The level of IL21 needs to be tightly controlled by regulatory elements. In one aspect, the effector module of the present disclosure may be a DD-IL21 fusion polypeptide.

In some instances, payloads of the present disclosure may comprise type I interferons. Type I interferons (IFNs-I) are soluble proteins important for fighting viral infection in humans. IFNs-I include IFN alpha subtypes (IFN α1, IFN α1b, IFN α1c), IFN beta, IFN delta subtypes (IFN delta 1, IFN delta 2, IFN delta 8), IFN gamma, IFN kappa, and IFN epsilon, IFN lambda, IFN omega, IFN tau and IFN zeta. IFN α and IFN β are the main IFN I subtypes in immune responses. All subtypes of IFN I signal through a unique heterodimeric receptor, interferon alpha receptor (IFNAR), composed of 2 subunits, IFNAR1 and IFNAR2. IFNR activation regulates the host response to viral infections and in adaptive immunity. Several signaling cascades can be activated by IFNR, including the Janus activated kinase-signal transducer and activation of transcription (JAK-STAT) pathway, the mitogen activated protein kinase (MAPK) pathway, the phosphoinositide 3-kinase (PI3K) pathway, the v-crk sarcoma virus CT10 oncogene homolog (avian)-like (CRKL) pathway, and NF-κB cascade. It has long been established that type I IFNs directly inhibit the proliferation of tumor cells and virus-infected cells, and increase MHC class I expression, enhancing antigen recognition. IFNs-I have also proven to be involved in immune system regulation. IFNs can either directly, through interferon receptor (IFNR), or indirectly by the induction of chemokines and cytokines, regulate the immune system. Type I IFNs enhance NK cell functions and promote survival of NK cells. Type I IFNs also affect monocytes, supporting the differentiation of monocytes into DC with high capacity for antigen presentation, and stimulate macrophage function and differentiation. Several studies also demonstrate that IFNs-I promote CD8⁺ T cell survival and functions. In some instances, it may be desirable to tune the expression of Type I IFNs using biocircuits of the present disclosure to avoid immunosuppression caused by long-term treatment with IFNs.

New anticancer immunotherapies are being developed that use recombinant type I IFN proteins, type I IFN transgene, type I IFN encoding vectors and type I IFN expressing cells. For example, IFN α has received approval for treatment of several neoplastic diseases, such as melanoma, RCC and multiple myeloma. Though type I IFNs are powerful tools to directly and indirectly modulate the functions of the immune system, side effects of systemic long-term treatments and lack of sufficiently high efficacy have dampened the interest of IFN α for clinical use in oncology. It is believed that if IFN levels are tightly regulated at the malignant tissues, type I IFNs are likely more efficacious. Approaches for intermittent delivery are proposed according to the observation that intermittency at an optimized pace may help to avoid signaling desensitizing mechanisms (negative feedback mechanisms) induced by IFNs-I (i.e., because of SOCS1 induction) in the responding immune cells. In accordance with the present disclosure, the effector module may comprise a DD-IFN fusion polypeptide. The DD and its ligand control the expression of IFN to induce an antiviral and antitumor immune responses and in the meantime, to minimize the side effects caused by long-term exposure of IFN.

In some instances, payloads of the present disclosure may comprise members of tumor necrosis factor (TNF) superfamily. The term "TNF superfamily" as used herein refers to a group of cytokines that can induce apoptosis. Members of TNF family include TNF-alpha, TNF-beta (also known as lymphotoxin-alpha (LT-α)), lymphotoxin-beta (LT-β), CD40L(CD154), CD27L (CD70), CD30L(CD153), FASL(CD178), 4-1BBL (CD137L), OX40L, TRAIL (TNF-related apoptosis inducing ligand), APRIL (a proliferation-inducing ligand), TWEAK, TRANCE, TALL-1, GITRL, LIGHT and TNFSF1 to TNFSF20 (TNF ligand superfamily member 1 to 20). In one instance, the payload of the disclosure may be TNF-alpha. TNF-alpha can cause cytolysis of tumor cells, and induce cell proliferation differentiation as well. In one aspect, the effector module of the present disclosure may comprise a DD-TNF alpha fusion polypeptide.

In one instance, the payloads of the present disclosure may be cytokines fused to TNF alpha ectodomain. Such payloads are produced as membrane associated cytokines fused to the TNF ectodomain. In one instance, the cytokine may be shed from the cell surface by the action of membrane associated proteases, and/or proteases in the extracellular space e.g. MMP9. Any of the cytokines described herein may be useful in the present disclosure. Such cytokine-TNF scaffold constructs may be used to preserve the native sequence of the processed cytokine while preserving regulation.

In some instances, payloads of the present disclosure may comprise inhibitory molecules that block inhibitory cytokines. The inhibitors may be blocking antibodies specific to an inhibitory cytokine, and antagonists against an inhibitory cytokine, or the like.

In some aspects, payloads of the present disclosure may comprise an inhibitor of a secondary cytokine IL35. IL35 belongs to the interleukin-12 (IL12) cytokine family, and is a heterodimer composed of the IL27 β chain Ebi3 and the IL 12 α chain p35. Secretion of bioactive IL35 has been described only in forkhead box protein 3 (Foxp3) ⁺ regulatory T cells (Tregs) (resting and activated Tregs). Unlike other membranes in the family, IL35 appears to function solely in an anti-inflammatory fashion by inhibiting effector T cell proliferation and perhaps other parameters (Collison et al., Nature, 2007, 450(7169): 566-569).

In some instances, payloads of the present disclosure may comprise inhibitors that block the transforming growth factor beta (TGF-β) subtypes (TGF-β1, TGF-β2 and TGF-β3). TGF-β is secreted by many cell types, including macrophages and is often complexed with two proteins LTBP and LAP. Serum proteinases such as plasmin catalyze the release of active TGF-β from the complex from the activated macrophages. It has been shown that an increase in expression of TGF-β correlates with the malignancy of many cancers. The immunosuppressive activity of TGF-β in the tumor microenvironment contributes to oncogenesis.

In some instances, payloads of the present disclosure may comprise inhibitors of IDO enzyme. In some instances, payloads fused to the DDs of the disclosure may be an inhibitor of an immunosuppressive molecule such as TGF-beta and IDO.

In some instances, payloads of the present disclosure may comprise chemokines and chemokine receptors. Chemokines are a family of secreted small cytokines, or signaling proteins that can induce directed chemotaxis in nearby responsive cells. The chemokine may be a SCY (small cytokine) selected from the group consisting of SCYA1-28 (CCL1-28), SCYB1-16 (CXCL1-16), SCYC1-2 (XCL1-2), SCYD-1 and SCYE-1; or a C chemokine selected from XCL1 and XCL2; or a CC chemokine selected from CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27 and CCL28; or a CXC chemokine selected from CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16 and CXCL17; or a CX3C chemokine CX3CL1. In some aspects, the chemokine receptor may be a receptor for the C chemokines including XCR1; or a receptor for the CC chemokines including CCR1, CCR2, CCR3, CCR4, CCR5, CCR6, CCR7, CCR8, CCR9 and CCR10; or a receptor for the CXC chemokines including CXCR1, CXCR2, CXCR3, CXCR4 and CXCR5; or a CX3C chemokine receptor CX3CR1.

In some instances, payloads of the present disclosure may comprise other immunomodulators that play a critical role in immunotherapy, such as GM-CSF (Granulocyte-macrophage colony stimulating factor), erythropoietin (EPO), MIP3a, monocyte chemotactic protein (MCP)-1, intracellular adhesion molecule (ICAM), macrophage colony stimulating factor (M-CSF), Interleukin-1 receptor activating kinase (iRAK-1), lactotransferrin, and granulocyte colony stimulating factor (G-CSF).

In some instances, the payload of the present disclosure may comprise Amphiregulin. Amphiregulin (AREG) is an EGF-like growth factor which binds to the EGFR receptor and enhances CD4⁺ regulatory T cells (Tregs) function. AREG promotes immune suppression in the tumor environment. Thus, in some instance, the payloads of the present disclosure may comprise Amphiregulin to dampen immune response during immunotherapy.

In some instances, payloads of the present disclosure may comprise fusion proteins wherein a cytokine, chemokine and/or other soluble factor may be fused to other biological molecules such as antibodies and or ligands for a receptor. Such fusion molecules may increase the half-life of the cytokines, reduce systemic toxicity, and increase local concentration of the cytokines at the tumor site. Fusion proteins containing two or more cytokines, chemokines and or other soluble factors may be utilized to obtain synergistic therapeutic benefits. In one instance, payload may be a GM-CSF/IL2 fusion protein.

In some instances, any of the hinge and transmembrane domains described herein may be used as a scaffold for soluble cytokine presentation. The cytokine may be operably linked to the CD8 hinge and transmembrane domain by a protease cleavage site. Cleavage at the cleavage site releases the cytokine from the cell surface membrane. In some aspects, the cytokine may be in a precursor form. Generation of the active form of the cytokine from the precursor form occurs via cleavage at the cleavage site. Any of the cytokines described herein may be engineered using the any of the hinge and transmembrane domains described herein as a scaffold.

### 7. Immune regulators

In some instances, payloads of the present disclosure may comprise inhibitors (antagonists) of co-inhibitory molecules (e.g., immune checkpoint), including without limitation, PD-1, PD-L1, PD-L2, CTLA-4, TIM-3, VISTA, BTLA, TIGIT, LAIRA, CD160, 2B4 and TGFR. In some aspects, the inhibitor may be a blocking/antagonistic antibody or fragment thereof, as discussed previously, or a ligand of the co-inhibitory receptor.

In some instances, payloads of the present disclosure may comprise agonists of co-stimulatory molecules, including without limitation, CD27, CD28, CD30, CD40, OX40 (CD134), 4-1BB (CD137), CDS, ICAM-1, LFA-1 (CD11a/CD18), ICOS (CD278), GITR, BAFFR, HVEM, CD7, LIGHT, NKG2C, SLAMF7, NKp80, CD160, B7-H3 and CD83. As a non-limiting example, agonists of co-stimulatory molecule ICOS (CD278) may be an ICOS binding protein comprising an amino acid sequence of SEQ ID NOs: 1, 2, 3, 4, 5 and 6 disclosed in International Patent Publication NO: WO2016120789.

In some aspects, the agonist of a co-stimulatory molecule may be an agonistic antibody or fragment thereof, as discussed previously; or a ligand of the co-stimulatory receptor; or any binding molecules that can enhance the biological activity of its target. For example, an agonistic ligand of OX40 may be OX40L (CD252). The OX40 ligand as used herein, includes the intact OX40 ligand, soluble OX40 ligand, fusion proteins including a functionally active portion of OX40 ligand covalently linked to a second moiety, e.g., a protein domain, and variants may be which vary in amino acid sequence from naturally occurring OX4L but which retain the ability to specifically bind to the OX40 receptor or even enhance the biological activity of OX40L.

In general, an agonist of a co-stimulatory molecule substantially enhances the biological activity of its target molecule, such as T cell activation. Desirably, the biological activity is enhanced by 10, 20, 30, 50, 70, 80, 90, 95, or even 100.

In some instances, payloads of the present disclosure may comprise immunomodulators including stress proteins and heat shock proteins (HSPs) that can integrate both innate and adaptive immune responses. They may also be other chaperones and adaptors that stimulate immune responses. As a non-limiting example, the payload of the present disclosure may be a fusion protein comprising an NF-KB-activating domain of Flagellin fused with an ATP-binding domain truncated glucose regulated protein 170 (Grp170) (See. US patent publication NO.: US2015/0315255). The fusion construct forms a secretable Grp170-Flagellin hybrid chaperone (Flagrp170) which can be used to stimulate anti-cancer immune responses.

In other instances, the payload of the present disclosure may comprise a STING (stimulator of interferon gene) protein, an adaptor molecule in the cytoplasm which, as a component of the host cytosolic surveillance pathway, activates the TANK binding kinase (TBK1)-IRF3 signaling axis, resulting in the induction of IFN β and other IRF-3 dependent gene products that strongly activate innate immunity, leading to the development of an adaptive immune response consisting of both antigen-specific CD4⁺ and CD8⁺ T cells as well as pathogen-specific antibodies.

Demaria et al. reported that enforced activation of a STING protein by intratumoral injection of cyclic dinucleotide GMP-AMP (cGAMP), an agonist of STING, can enhance antitumor CD8⁺ T cell responses leading to growth control of injected and contralateral tumors in mouse models of melanoma and colon cancer. The STING-dependent antitumor immunity was dependent on type I IFNs produced by endothelial cells in the tumor microenvironment (Demaria et al., Proc. Natl. Acad. Sci. USA, 2015, 112(5): 15408-15413). These studies demonstrate that STING contributes to anti-tumor immune responses via enhancement of type I IFN signaling in the tumor microenvironment. Biocircuits, effector modules comprising STING may be applied to the tumor microenvironment to enhance anti-tumor responses either alone or in combination with other immunotherapeutic agents of the disclosure.

In addition to STING proteins, payloads of the present disclosure may comprise PRRs (pattern recognition receptors) that are involved in sensing the infection of cells by viruses and microorganisms to activate innate immune inflammatory responses. Such PRRs include Toll like receptors (TLRs), RIG-I-like receptors (RLRs), NOD-like receptors (NLRs), and C-type lectin receptors (CLRs). The RLR family is a RNA sensing system that is comprised of retinoic acid inducible gene-like-I (RIG-1), melanoma differentiation-associated gene 5 (MDA5), and laboratory of genetics and physiology 2 (LGP2). RIG-1 recognizes relatively short dsRNA (up to 1 kb) whereas MDA5 detects long dsRNA (more than 2 kb) to activate synthesis of type I IFNs, including IFN α and IFN β (Wilkins et al., Curr Opin Immunol., 2010, 22: 41-47). RLRs activate downstream signaling proteins evoking type I IFN production. TLRs recognize distinct structures in microbes; often referred to as "PAMPs" (pathogen associated molecular patterns). Ligand binding to TLRs invokes a cascade of intra-cellular signaling pathways that induce the production of factors involved in inflammation and immunity such as pro-inflammatory cytokines, and chemokines, as well as CD4⁺ and CD8⁺ T cell activation. Among ten TLRs identified in human, TLRs-1, 2, 4, 5 and 6 are expressed in the cell surface, while TLR-3, -7/8, and -9 are expressed with the ER compartment. In some instances, payloads of the disclosure may be one of the PRRs, or agonists of PRRs.

### 8. Metabolic factors/metabolic checkpoint

In some instances, immune cells such as T cells used for immunotherapy may be metabolically reprogrammed to enhance anti-tumor T cell responses. Metabolic activities are necessary to support immune cells, specifically T cells growth, expansion, differentiation and effector functions, upon activation through T cell receptor or CAR and co-stimulatory signals. Metabolic competition between cancer cells and infiltrating immune effector cells leads to T cell energy and dysfunction.

In some instances, payloads of the present disclosure may be modulators of glycolysis. The Warburg effect in cancer cells leads to the massive generation of lactic acid that can suppress T cell cytotoxic and effector functions. As a non-limiting example, immune cells for adoptive transfer may be modified to overexpress phosphoenolpyruvate carboxykinase 1 (PCK1), which increases PEP production in T cells. Increased production of the glycolytic metabolite phosphoenolpyruvate (PEP) can repress sarco/ER Ca (2+)-ATPase (SERCA) activity, therefore sustaining T cell receptor-mediated Ca (2+)-NFAT signaling and effector functions (Ho et al, Cell, 2015, 162(6): 1217-1228).

In some instances, the payloads of the present disclosure may comprise of proteins involved in the OXPHOS pathway. For example, LEM (lymphocyte expansion molecule), a protein that can promote cytotoxic CD8⁺ T cell proliferation and effector function, and memory T cell generation in response to infection with lymphocyte choriomeningitis (CMV). LEM is part of a complex of CRIF1 (CR6 interacting factor 1) that mediates the translation and insertion of OXPHOS (Oxidative phosphorylation) proteins into mitochondrial inner membrane, thereby regulates OXPHOS activity. Thus, LEM is a positive modulator of T cell metabolism (mitochondria respiratory levels) and expansion (Okoye et al., Science, 2015, 348(6238): 995-1001).

In some instances, the payloads of the present disclosure may be inhibitors of metabolic enzymes involved in amino acid regulation. Metabolic competition for between immune cells and tumor cells can lead to establishing and maintaining an immunosuppressive tumor microenvironment due to T cell energy. Non-limiting examples include inhibitors of nitric oxide synthase and arginase I that can degrade extracellular arginine, or Indoleamine 2,3- dioxygenase (IDO) that degrades tryptophan. Abrogation of these enzymes secreted by tumor cells and immune suppressive cells can promote antitumor immunity.

In other instances, payloads of the present disclosure may comprise proteins critical for *de novo* fatty acid and cholesterol biosynthesis. This may include proteins such as SREBP1 (also known as SREBF1), SREBP2 (SREBF2), HMGCR, HMGCS, FASN, ACACA and SQLE, and transport pathways, such as LDLR. Modulating cholesterol metabolism of cytotoxic CD8⁺ T cells may potentiate their anti-tumor effector function and proliferation. Cholesterol is a key component of membrane lipids, and has been shown that the increase in the plasma membrane cholesterol level of CD8⁺ T cells, enhances T-cell receptor clustering and signaling as well as more efficient formation of the immunological synapse (Molnar et al., J Biol Chem. 2012, 287:42664-42674). T cells used for adoptive transfer (e.g., anti-tumor CAR T cells) may be further engineered to express a protein that enhances cholesterol biosynthesis and/or transportation.

### 9. Safety switch

In some instances, payloads of the present disclosure may comprise SRE regulated safety switches that can eliminate adoptively transferred cells in the case of severe toxicity, thereby mitigating the adverse effects of T cell therapy. Adoptively transferred T cells in immunotherapy may attack normal cells in response to normal tissue expression of TAA. Even on-tumor target activity of adoptively transferred T cells can result in toxicities such as tumor lysis syndrome, cytokine release syndrome and the related macrophage activation syndrome.

In one instance, the payloads of the present disclosure may eliminate the inappropriately activated cells by induction of apoptosis or by immunosurveillance have been developed in the art.

In some instances, payloads of the present disclosure may comprise inducible killer/suicide genes that acts as a safety switch. The killer/suicide gene when introduced into adoptively transferred immune cells, could control their alloreactivity. The killer/suicide gene may be an apoptotic gene (e.g., a caspase) which allows conditional apoptosis of the transduced cells by administration of a non-therapeutic ligand of the SRE (e.g., DD).

In some instances, the payload of the present disclosure may include Caspase 9. In some instances, Caspase 9 may be modified to have low basal expression and lacking the caspase recruitment domain (CARD) (SEQ ID NO:: 26 and SEQ ID NO:: 28 of US Patent No. US9434935B2).

In one instance, the payload of the present disclosure is a suicide gene system, iCasp9/Chemical induced dimerization (CID) system which consists of a polypeptide derived from the Caspase9 gene fused to a drug binding domain derived from the human FK506 protein. Administration of bioinert, small molecule AP 1903 (rimiducid), induces cross linking of the drug binding domains and dimerization of the fusion protein and in turn the dimerization of Caspase 9. This results in the activation of downstream effector Caspase 3 and subsequent induction of cellular apoptosis (Straathof et al., Blood, 2005, 105: 4247-4254). Preclinical trials using CART including an iCasp9 gene have shown effective elimination of CAR T cells *in vivo* in mouse models and demonstrate the potential efficacy of this approach. (Budde et al, Plos One, 2013, 8: e82742.10.1371; Hoyos et al., Leukemia, 2010; 24(6):1160-1170). In one instance, the payload of the disclosure may comprise Caspase9. In one aspect, the effector module of the disclosure may be a DD-Caspase9 fusion polypeptide. In some instances, the payload of the disclosure may be full length Caspase 9 (SEQ ID NO: 8364, encoded by SEQ ID No. 8365, 8366) or caspase 9 delta CD (SEQ ID NO: 8367), encoded by SEQ ID No. 8368.The Caspases 9 sequences described herein may optionally include a stop codon at the C terminal of the sequence.

In some instances, the iCasp9/CID system has been shown to have a basal rate of dimerization even in the absence of rimiducid, resulting in unintended cell death. Regulating the expression levels of iCasp9/CID is critical for maximizing the efficacy of iCasp9/CID system. Biocircuits of the present disclosure and/or any of their components may be utilized in regulating or tuning the iCasp9/CID system in order to optimize its utility. Other examples of proteins used in dimerization-induced apoptosis paradigm may include, but are not limited to Fas receptor, the death effector domain of Fas-associated protein, FADD, Caspase 1, Caspase 3, Caspase 7 and Caspase 8. (Belshaw P.J. et al, Chem Biol., 996,3:731-738; MacCorkle R.A. et al, Proc Natl Acad Sci, 1998, 95:3655-3660; Spencer, D.M. et al., Curr Biol. 1996; 6:839-847).

In some instances, the safety switch of the present disclosure may comprise a metabolic enzyme, such as herpes simplex virus thymidine kinase (HSV-TK) and cytosine deaminase (CD). HSV-TK phosphorylates nucleoside analogs, including acyclovir and ganciclovir (GCV) to generate triphosphate form of nucleosides. When incorporated into DNA, it leads to chain termination and cell death. Unlike the mammalian thymidine kinase, HSV-TK is characterized by 1000-fold higher affinity to nucleoside analogs such as GCV, making it suitable for use as a suicide gene in mammalian cells. Cytosine deaminase (CD) can converts 5-fluorocytosine (5-FC) into the cytotoxic 5-fluorouracil (5-FU) (Tiraby et al., FEMS Lett., 1998, 167: 41-49).

In some instances, the safety switch of the present disclosure may comprise a CYP4B1 mutant (as suicide gene), which may be co-expressed in a CAR engineered T cells (Roellecker et al., Gen Ther., 2016, May 19, doi: 10.1038/gt.2016.38.).

In some instances, the payload of the present disclosure may comprise a fusion construct that can induce cell death, for example, a polypeptide with the formula of S1-R1-S1-Q-S2-R2, wherein the St is a stalk sequence, R1/2 and Q are different epitopes; and S1/2 are optional spacer sequences (See, International patent publication NO.: WO2013/153391).

In some instances, safety switch may be mediated by therapeutic antibodies which specifically bind to an antigen that is expressed in the plasma membrane of adoptively transferred cells. The antigen-antibody interaction allows cell removal after administration of a specific monoclonal antibody against the antigen. As non-limiting examples, payloads of the present disclosure may comprise the antigen and antibody pair used to mediate safety switch such as CD20 and anti-CD20 antibody (Griffioen et al., Haematologica, 2009, 94:1316-1320), a protein tag and anti-tag antibody (Kieback et al., Natl. Acad. Sci. U.S.A., 2008, 105: 623-628), a compact suicide gene (RQR8) combining epitopes from CD34 (as a marker moiety) and CD20 (as a suicide moiety) which enables CD34 selection, cell tracking, as well as cell deletion after anti-CD20 monoclonal antibody administration (Philip et al., Blood, 2014, 124: 1277-1287); truncated human EGFR polypeptide and anti-EGFR monoclonal antibody (Wang et al., Blood, 2011, 118:1255-1263); and a compact polypeptide safety switch having a structural formula as discussed in U.S Patent Application Publication NO: 2015/0093401.

### 10. Regulatory switch

The utility of adoptive cell therapy (ACT) has been limited by the high incidence of graft versus host disease (GVHD). GVHD occurs when adoptively transferred T cells elicit an immune response resulting in host tissue damage. Recognition of host antigens by the graft cells triggers a proinflammatory cytokine storm cascade that signifies acute GVHD. GVHD is characterized as an imbalance between the effector and the regulatory arms of the immune system. In some instances, the payloads of the present disclosure may be used as regulatory switches. As used herein "regulatory switch" refers proteins, which when expressed in target cells increase tolerance to the graft by enhancing the regulatory arm of the immune system.

In one instance, regulatory switches may include payloads that preferentially promote the expansion of regulatory T (Treg cells). Tregs are a distinct population of cells that are positively selected on high affinity ligands in the thymus and play an important role in the tolerance to self-antigens. In addition, T regs have also been shown to play a role in peripheral tolerance to foreign antigens. Since Tregs promote immune tolerance, expansion of Tregs with the compositions of the disclosure may be desirable to limit GVHD.

In some instances, the regulatory switch may include, but is not limited to T regs activation factors such NPκB, FOXO, nuclear receptor Nr4a, Retinoic acid receptor alpha, NFAT, AP-1 and SMAD. Such factors can result in the expression of Fork headbox P3 (FOXP3) in T cells resulting in the activation of the regulatory T cell program and the expansion of T cells.

In one instance, the regulatory switch may be FOXP3, a transcriptional regulator in T cells. A function of FOXP3 is to suppress the function of NFAT, which leads to the suppression of expression of many genes including IL2 and effector T-cell cytokines. FOXP3 acts also as a transcription activator for genes such as CD2S, Cytotoxic T-Lymphocyte Antigen Cytotoxic T-Lymphocyte Antigen 4 (CTLA4), glucocorticoid-induced TNF receptor family gene (GITR) and folate receptor 4. FOXP3 also inhibits the differentiation of IL 17 producing helper T-cells (Th17) by antagonizing RORC (RAR related orphan receptor C). Isoforms of FOXP3 lacking exon2 (FOXP3 delta 2), or exon 7 (FOXP3 delta 7) may also be used as regulatory switches. In one aspect, the effector module of the disclosure may be a DD-FOXP3 fusion polypeptide. FOXP3 may be a full length FOXP3 (SEQ ID NO: 8369, encoded by SEQ ID NO: 8370); or FOXP3 (amino acid 2-431 of WT) (SEQ ID NO: 8371, encoded by SEQ ID NO: 8372), a delta 2 FOXP3 (SEQ ID NO: 8373, encoded by SEQ ID NO:8374); or FOXP3 delta (amino acid 2-396 of WT) (SEQ ID NO: 8375, encoded by SEQ ID NO:8376).

### 11. Homing receptors

In some instances, payloads of the present disclosure may comprise homing receptors that guide immunotherapeutic cells to different anatomical compartments, such as designated tumor sites. For example, T cells expressing a chimeric antigen receptor may be further modified to express a homing receptor that is not normally expressed by the T cell. As used herein, the term "homing receptor" is a receptor that guides a cell expressing the receptor to a designated organ, a particular tissue, or a particular type of cell. Such trafficking receptors favor T cell accumulation in certain target organs. In some instances, the homing receptors of the present disclosure may be adhesion molecules. In other instances, the homing receptors of the disclosure may be chemokine receptors which mediate chemotaxis to chemokines. As non-limiting examples, a homing receptor may be a B cell zone homing receptor such as CXCR5; T cell zone homing receptor such as CXCR7; a gastrointestinal homing receptor such as CCR9 and integrin α4β7 (also known as lymphocyte Peyer patch adhesion molecule); a skin homing receptor such as CLA (cutaneous lymphocyte-associated antigen receptor), CCR4, CCR8 and CCR10 (See, e.g., International Patent Publication NO.: WO2016025454). Other homing receptors include, without limitation, CXCR2 and CXCR1 which redirect chemokine receptor modified tumor-infiltrating lymphocytes to melanoma tumor (Idorn et al., Methods Mol. Biol., 2016, 1428: 261-276; and Sapoznik et al., Cancer Immunol Immunother., 2012, 61(10): 1833-1847); CCR2 which, when expressed by CD8⁺ T cells, can home modified CD8⁺ T cells to the site of prostate cancer in which the CCL2 (a CCR2 ligand) expression is increased (Garetto et al., Oncotarget, 2016, May 10. doi: 10.18632/oncotarget.9280); and CD103 as an intestinal homing receptor.

### 12. Immune signaling

Treatment with immunotherapeutic agents may induce immune cell signaling, leading to the activation of cell- type specific immune activities, ultimately resulting in an immune response. In some instances, payloads of the present disclosure may be immune signaling biomolecules used to achieve exogenous control of signaling pathways. Exemplary immune signaling biomolecules include transcription factors such as Nuclear factor of activated T-cells (NFAT) (e.g., NFAT, NFAT2, NFAT3 and NFAT 4), Nuclear Factor Kappa B (NEκB), Signal transducer and activator of transcription (STAT), Activator protein-1 (AP-1), Rel, Fos, and Jun; kinases such as Janus Kinase (JAK), Extracellular signal-regulated kinases (ERK), Mitogen- Activated Protein Kinases (MAPK), Mammalian target of rapamycin (mTOR), Phsophoinositide-dependent kinase (PDK), Protein kinase B (PKB), IkB kinase (IKK), Calcium/Calmodulin dependent kinase(CaMK); and other signaling molecules such as Ras, Cbl, Calmodulin (CaM), Calpain, and IkkB kinase.

In one instance, the payloads of the present disclosure may be administered in conjunction with inhibitors of SHP-1 and/or SHP-2. The tyrosine-protein phosphatase SHP1 (also known as PTPN6) and SHP2 (also known as PTPN11) are involved in the Programmed Cell Death (PD1) inhibitory signaling pathway. The intracellular domain of PD 1 contains an immunoreceptor tyrosine-based inhibitory motif (ITIM) and an immunoreceptor tyrosine-based switch motif (ITSM). ITSM has been shown to recruit SHP-1 and 2. This generates negative costimulatory micro clusters that induce the dephosphorylation of the proximal TCR signaling molecules, thereby resulting in suppression of T cell activation, which can lead to T cell exhaustion. In one instance, inhibitors of SHP-1 and 2 may include expressing dominant negative versions of the proteins in T cells, TILs or other cell types to relieve exhaustion. Such mutants can bind to the endogenous, catalytically active proteins, and inhibit their function. In one instance, the dominant negative mutant of SHP-1 and/ or SHP-2 lack the phosphatase domain required for catalytic activity. In some instances, any of the dominant negative SHP-1 mutants taught Bergeron S et al. (2011). Endocrinology. 2011 Dec;152(12):4581-8.; Dustin JB et al. (1999) J Immunol. Mar 1;162(5):2717-24.; Berchtold S (1998) Mol Endocrinol. Apr;12(4):556-67 and Schram et al. (2012) Am J Physiol Heart Circ Physiol. 1;302(1):H231-43.; may be useful in the disclosure.

### 13. Oncolytic viruses

In some instances, payloads of the present disclosure may comprise oncolytic viruses or any components of oncolytic viruses. In some instances, the payload may be oncolytic viruses or components that have been genetically modified oncolytic viruses for use in oncolytic virotherapy. As used herein, the term "virotherapy" refers to a therapeutic use of oncolytic viruses (replication competent viruses) to attack and destroy cancer cells. Oncolytic viruses refer to those viruses that are able to eliminate malignancies by direct targeting and killing of cancer cells within the tumor, without causing harm to normal tissues. Exemplary oncolytic viruses and genetically engineered oncolytic viruses with cancer specific tropism may include Arvoviruses, Adenoviruses, Coxsackie viruses, Herpes Simplex Viruses (HSVs), Measles, Mumps viruses, Moloney leukemia viruses, Myxovirus, Newcastle Disease Viruses, Reoviruses, Rhabdovirus, Vesticular Stomatic Viruses, and Vaccinia Viruses (VV). It may also be chimeric viruses with increased oncolytic potential such as an adeno-parvovirus chimera in US. Pat. NO.: 9,441,246. The oncolytic viruses may be modified to be less susceptible to immune suppression while more specifically targeting particular classes of cancer cells, or be modified to insert and express cancer-suppressing transgenes. Modifications to the oncolytic virus may also be made to improve replicative potential of the virus, increase viral titers, and/ or enhance the range cancer cells that can be infected by the virus. Examples of modified oncolytic viruses that may be used as payload include US Patent NO.: US8282917B2, International Patent Publication NO.: WO2011070440, WO2004078206A1, WO2016144564, WO2016119052, WO2009111892.

In some instances, payloads of the present disclosure may be one or more coat proteins of the viruses, inserted transgenes, other factors that can increase intratumoral virus replication and the combinations.

In some instance, two or more oncolytic viruses may also be used as payload within the same SRE or in two or more SREs to achieve synergistic killing of target cancer cells as described in International Patent Publication NO.: WO2010020056.

### 14. Genomic editing systems

In some instances, payloads of the present disclosure may be components of gene editing systems including a CRISPR (Clustered Regularly Interspaced Short Palindromic Repeats), CRISPR enzyme (Cas9), CRISPR-Cas9 or CRISPR system and CRISPR-CAS9 complex. It may also be other genomic editing systems, such as Zinc finger nucleases, TALEN (Transcription activator-like effector-based nucleases) and meganucleases.

### Additional features

The effector module of the present disclosure may further comprise a signal sequence which regulates the distribution of the payload, a cleavage and/or processing feature which facilitate cleavage of the payload from the effector module construct, a targeting and/or penetrating signal which can regulate the cellular localization of the effector module, and/or one or more linker sequences which link different components (e.g. a DD and a payload) of the effector module. In some instances, the effector module may further comprise of one or more additional features such as linker sequences (with specific sequences and lengths), cleavage sites, regulatory elements (that regulate expression of the protein of interest such as microRNA targeting sites), signal sequences that lead the effector module to a specific cellular or subcellular location, penetrating sequences, or tags and biomarkers for tracking the effector module.

### 1. Signal sequences

In addition to the SRE (e.g., DD) and payload region, effector modules of the disclosure may further comprise one or more signal sequences. Signal sequences (sometimes referred to as signal peptides, targeting signals, target peptides, localization sequences, transit peptides, leader sequences or leader peptides) direct proteins (e.g., the effector module of the present disclosure) to their designated cellular and/or extracellular locations. Protein signal sequences play a central role in the targeting and translocation of nearly all secreted proteins and many integral membrane proteins.

A signal sequence is a short (5-30 amino acids long) peptide present at the N-terminus of the majority of newly synthesized proteins that are destined towards a particular location. Signal sequences can be recognized by signal recognition particles (SRPs) and cleaved using type I and type II signal peptide peptidases. Signal sequences derived from human proteins can be incorporated as a regulatory module of the effector module to direct the effector module to a particular cellular and/or extracellular location. These signal sequences are experimentally verified and can be cleaved (Zhang et al., Protein Sci. 2004, 13:2819-2824).

In some instances, a signal sequence may be, although not necessarily, located at the N-terminus or C-terminus of the effector module, and may be, although not necessarily, cleaved off the desired effector module to yield a "mature" payload, i.e., an immunotherapeutic agent as discussed herein.

In some examples, a signal sequence may be a secreted signal sequence derived from a naturally secreted protein, and its variant thereof. In some instances, the secreted signal sequences may be cytokine signal sequences such as, but not limited to, IL2 signal sequence comprising amino acid of SEQ ID NO: 261(encoded by the nucleotide sequence of SEQ ID NO: 262-265), and/or p40 signal sequence comprising the amino acid sequence of SEQ ID NO: 266, (encoded by the nucleotide sequence of SEQ ID NO: 267-275).

In some instances, signal sequences directing the payload to the surface membrane of the target cell may be used. Expression of the payload on the surface of the target cell may be useful to limit the diffusion of the payload to non-target *in vivo* environments, thereby potentially improving the safety profile of the payloads. Additionally, the membrane presentation of the payload may allow for physiologically and qualitative signaling as well as stabilization and recycling of the payload for a longer half-life. Membrane sequences may be the endogenous signal sequence of the N terminal component of the payload. Optionally, it may be desirable to exchange this sequence for a different signal sequence. Signal sequences may be selected based on their compatibility with the secretory pathway of the cell type of interest so that the payload is presented on the surface of the T cell. In some instances, the signal sequence may be IgE signal sequence comprising amino acid of SEQ ID NO: 276 (encoded by the nucleotide sequence of SEQ ID NO: 277) or CD8a signal sequence comprising amino acid SEQ ID NO: 278 (encoded by the nucleotide sequence of SEQ ID NO: 279-283).

Other examples of signal sequences include, a variant may be a modified signal sequence discussed in U.S. Pat. NOs. 8,148,494, 8,258,102, 9,133,265, 9,279,007, and U.S. patent application publication NO. 2007/0141666; and International patent application publication NO. WO1993/018181. In other examples, a signal sequence may be a heterogeneous signal sequence from other organisms such as virus, yeast and bacteria, which can direct an effector module to a particular cellular site, such as a nucleus (e.g., EP 1209450). Other examples may include Aspartic Protease (NSP24) signal sequences from *Trichoderma* that can increase secretion of fused protein such as enzymes (e.g., U. S. Pat. NO. 8,093,016 to Cervin and Kim), bacterial lipoprotein signal sequences (e.g., PCT application publication NO. 1991/09952 to Lau and Rioux), *E.coli* enterotoxin II signal peptides (e.g., U.S. Pat. NO. 6,605,697 to Kwon et al.), *E.coli* secretion signal sequence (e.g., U.S. patent publication NO. 2016/090404 to Malley et al.), a lipase signal sequence from a methylotrophic yeast (e.g., U.S. Pat. NO. 8,975,041), and signal peptides for DNases derived from *Coryneform bacteria* (e.g., U.S. Pat. NO. 4,965,197).

Signal sequences may also include nuclear localization signals (NLSs), nuclear export signals (NESs), polarized cell tubulo-vesicular structure localization signals (See, e.g., U.S. Pat. NO.: 8,993,742; Cour et al., Nucleic Acids Res. 2003, 31(1): 393-396),extracellular localization signals, signals to subcellular locations (e.g. lysosome, endoplasmic reticulum, golgi, mitochondria, plasma membrane and peroxisomes, etc.) (See, e.g., U.S. Pat. NO. 7,396,811; and Negi et al., Database, 2015, 1-7).

### 2. Cleavage sites

In some instances, the effector module comprises a cleavage and/or processing feature. The effector module of the present disclosure may include at least one protein cleavage signal/site. The protein cleavage signal/site may be located at the N-terminus, the C-terminus, at any space between the N- and the C- termini such as, but not limited to, half-way between the N- and C-termini, between the N-terminus and the half-way point, between the half-way point and the C-terminus, and combinations thereof.

The effector module may include one or more cleavage signal(s)/site(s) of any proteinases. The proteinases may be a serine proteinase, a cysteine proteinase, an endopeptidase, a dipeptidase, a metalloproteinase, a glutamic proteinase, a threonine proteinase and an aspartic proteinase. In some aspects, the cleavage site may be a signal sequence of furin, actinidain, calpain-1, carboxypeptidase A, carboxypeptidase P, carboxypeptidase Y, caspase-1, caspase-2, caspase-3, caspase-4, caspase-5, caspase-6, caspase-7, caspase-8, caspase-9, caspase-10, cathepsin B, cathepsin C, cathepsin G, cathepsin H, cathepsin K, cathepsin L, cathepsin S, cathepsin V, clostripain, chymase, chymotrypsin, elastase, endoproteinase, enterokinase, factor Xa, formic acid, granzyme B, Matrix metallopeptidase-2, Matrix metallopeptidase-3, pepsin, proteinase K, SUMO protease, subtilisin, TEV protease, thermolysin, thrombin, trypsin and TAGZyme.

In one instance, the cleavage site is a furin cleavage site comprising the amino acid sequence SARNRQKRS (SEQ ID NO: 284, encoded by the nucleotide sequence of SEQ ID NO: 285), or a revised furin cleavage site comprising the amino acid sequence ARNRQKRS (SEQ ID NO: 286, encoded by the nucleotide sequence of SEQ ID NO: 287), or a modified furin site comprising the amino acid sequence ESRRVRRNKRSK (SEQ ID NO: 288, encoded by the nucleotide sequence of SEQ ID NO: 289-291).

### 3. Protein tags

In some instances, the effector module of the disclosure may comprise a protein tag. The protein tag may be used for detecting and monitoring the process of the effector module. The effector module may include one or more tags such as an epitope tag (e.g., a FLAG or hemagglutinin (HA) tag). A large number of protein tags may be used for the present effector modules. They include, but are not limited to, self-labeling polypeptide tags (e.g., haloalkane dehalogenase (halotag2 or halotag7), ACP tag, clip tag, MCP tag, snap tag), epitope tags (e.g., FLAG, HA, His, and Myc), fluorescent tags (e.g., green fluorescent protein (GFP), red fluorescent protein (RFP), yellow fluorescent protein (YFP), and its variants), bioluminescent tags (e.g. luciferase and its variants), affinity tags (e.g., maltose-binding protein (MBP) tag, glutathione-S-transferase (GST) tag), immunogenic affinity tags (e.g., protein A/G, IRS, AU1, AU5, glu-glu, KT3, S-tag, HSV, VSV-G, Xpress and V5), and other tags (e.g., biotin (small molecule), StrepTag (StrepII), SBP, biotin carboxyl carrier protein (BCCP), eXact, CBP, CYD, HPC, CBD intein-chitin binding domain, Trx, NorpA, and NusA.

In other instances, a tag may also be selected from those disclosed in U.S. Pat. NOs.: 8,999,897; 8,357,511; 7,094,568; 5,011,912; 4,851,341; and 4,703,004; U.S patent application publication NOs.: 2013/115635 and 2013/012687; and International application publication NO.: WO2013/091661.

In some aspects, a multiplicity of protein tags, either the same or different tags, may be used; each of the tags may be located at the same N- or C-terminus, whereas in other cases these tags may be located at each terminus.

### 4. Targeting peptides

In some instances, the effector module of the disclosure may further comprise a targeting and/or penetrating peptide. Small targeting and/or penetrating peptides that selectively recognize cell surface markers (e.g. receptors, trans-membrane proteins, and extra-cellular matrix molecules) can be employed to target the effector module to the desired organs, tissues or cells. Short peptides (5-50 amino acid residues) synthesized *in vitro* and naturally occurring peptides, or analogs, variants, derivatives thereof, may be incorporated into the effector module for homing the effector module to the desired organs, tissues and cells, and/or subcellular locations inside the cells.

In some instances, a targeting sequence and/or penetrating peptide may be included in the effector module to drive the effector module to a target organ, or a tissue, or a cell (e.g., a cancer cell). In other instances, a targeting and/or penetrating peptide may direct the effector module to a specific subcellular location inside a cell.

A targeting peptide has any number of amino acids from about 6 to about 30 inclusive. The peptide may have 6, 7, 8, 9, 10, 11, 12, 13, 14, 15, 16, 17, 18, 19, 20, 21, 22, 23, 24, 25, 26, 27, 28, 29 or 30 amino acids. Generally, a targeting peptide may have 25 or fewer amino acids, for example, 20 or fewer, for example 15 or fewer.

Exemplary targeting peptides may include, but are not limited to, those disclosed in the art, e.g., U.S. Pat. NOs. 9,206,231, 9,110,059, 8,706,219; and 8,772,449, and U.S. application publication NOs. 2016/089447, 2016/060296, 2016/060314, 2016/060312, 2016/060311, 2016/009772, 2016/002613, 2015/314011 and 2015/166621. and International application publication NOs. WO2015/179691 and WO2015/183044.

### 5. Linkers

In some instances, the effector module of the disclosure may further comprise a linker sequence. The linker region serves primarily as a spacer between two or more polypeptides within the effector module. A "linker" or "spacer", as used herein, refers to a molecule or group of molecules that connects two molecules, or two parts of a molecule such as two domains of a recombinant protein.

In some instances, "Linker" (L) or "linker domain" or "linker region" or "linker module" or "peptide linker" as used herein refers to an oligo- or polypeptide region of from about 1 to 100 amino acids in length, which links together any of the domains/regions of the effector module (also called peptide linker). The peptide linker may be 1-40 amino acids in length, or 2-30 amino acids in length, or 20-80 amino acids in length, or 50-100 amino acids in length. Linker length may also be optimized depending on the type of payload utilized and based on the crystal structure of the payload. In some instances, a shorter linker length may be preferably selected. In some aspects, the peptide linker is made up of amino acids linked together by peptide bonds, preferably from 1 to 20 amino acids linked by peptide bonds, wherein the amino acids are selected from the 20 naturally occurring amino acids: Glycine (G), Alanine (A), Valine (V), Leucine (L), Isoleucine (I), Serine (S), Cysteine (C), Threonine (T), Methionine (M), Proline (P), Phenylalanine (F), Tyrosine (Y), Tryptophan (W), Histidine (H), Lysine (K), Arginine (R), Aspartate (D), Glutamic acid (E), Asparagine (N), and Glutamine (Q). One or more of these amino acids may be glycosylated, as is understood by those in the art. In some aspects, amino acids of a peptide linker may be selected from Alanine (A), Glycine (G), Proline (P), Asparagine (R), Serine (S), Glutamine (Q) and Lysine (K).

In one example, an artificially designed peptide linker may preferably be composed of a polymer of flexible residues like Glycine (G) and Serine (S) so that the adjacent protein domains are free to move relative to one another. Longer linkers may be used when it is desirable to ensure that two adjacent domains do not interfere with one another. The choice of a particular linker sequence may concern if it affects biological activity, stability, folding, targeting and/or pharmacokinetic features of the fusion construct. Examples of peptide linkers include, but are not limited to: SG, MH, GGSG (SEQ ID NO: 292, encoded by the nucleotide sequence of SEQ ID NO: 293; GGSGG (SEQ ID NO: 294), encoded by the nucleotide sequence of SEQ ID NOs. 295-299; GGSGGG (SEQ ID NO: 78), encoded by the nucleotide sequence of SEQ ID NO: 93 and 300; SGGGS (SEQ ID NO: 301), encoded by the nucleotide sequence of SEQ ID NO: 302-303; GGSGGGSGG (SEQ ID NO: 77), encoded by the nucleotide sequence of SEQ ID NO: 92; GGGGG (SEQ ID NO: 304), GGGGS (SEQ ID NO: 305) or (GGGGS)n (n= 2 (SEQ ID NO: 306), 3 (SEQ ID NO: 307; encoded by SEQ ID NO: 308-313), 4 (SEQ ID NO: 314), 5 (SEQ ID NO: 315), or 6 (SEQ ID NO: 316)), SSSSG (SEQ ID NO: 317) or (SSSSG)n (n= 2 (SEQ ID NO: 318), 3 (SEQ ID NO: 319), 4 (SEQ ID NO: 320), 5 (SEQ ID NO: 321), or 6 (SEQ ID NO: 322)), SGGGSGGGGSGGGGSGGGGSGGGSLQ (SEQ ID NO: 323), encoded by the nucleotide sequence of SEQ ID NO: 324 or SEQ ID NO: 8665 ; EFSTEF (SEQ ID NO: 325), encoded by the nucleotide sequence of SEQ ID NOs. 326-327; GKSSGSGSESKS (SEQ ID NO: 328), GGSTSGSGKSSEGKG (SEQ ID NO: 329), GSTSGSGKSSSEGSGSTKG (SEQ ID NO: 330), GSTSGSGKPGSGEGSTKG (SEQ ID NO: 331), VDYPYDVPDYALD (SEQ ID NO: 332), encoded by the nucleotide sequence of SEQ ID NO: 333; or EGKSSGSGSESKEF (SEQ ID NO: 334) ; or GSGSGS (SEQ ID NO: 8330), encoded by the nucleotide sequence of SEQ ID NO: 8347; or GSGSGSGS (SEQ ID NO: 8331), encoded by the nucleotide sequence of SEQ ID NO: 8348; or GSGSGGGSGS (SEQ ID NO: 8332), encoded by the nucleotide sequence of SEQ ID NO: 8349; SGSGSGS linker (SEQ ID NO: 8382), or SG linker, encoded by AGTGGT; or an LD Linker comprising Lysine Aspartic acid, encoded by CTAGAT; GGSGGG (SEQ ID NO: 8630), encoded by the nucleotide sequence of SEQ ID NO: 8631. Linkers may also be DNA restriction enzyme recognition sites or modifications thereof such as flexible GS or G/S rich linker; BamH1 Site encoded by GGATCC; flexible G/S rich linker or BamH1 Site; SR/Xba I site, encoded by TCTAGA; or a GSG linker (BamH1-Gly) linker, encoded by GGATCCGGA.

In other examples, a peptide linker may be made up of a majority of amino acids that are sterically unhindered, such as Glycine (G) and Alanine (A). Exemplary linkers are polyglycines (such as (G)₄ (SEQ ID NO: 8378), (G)s (SEQ ID NO: 8379), (G)s (SEQ ID NO: 8380)), poly(GA), and polyalanines. The linkers described herein are exemplary, and linkers that are much longer and which include other residues are contemplated by the present disclosure.

In some instances, the linker may be a spacer comprising the nucleotide sequence of SEQ ID NO: 8668-8669. In one instance, the spacer is a nucleotide sequence that is not translated into an amino acid sequence.

A linker sequence may be a natural linker derived from a multi-domain protein. A natural linker is a short peptide sequence that separates two different domains or motifs within a protein.

In some aspects, linkers may be flexible or rigid. In other aspects, linkers may be cleavable or non-cleavable. As used herein, the terms "cleavable linker domain or region" or "cleavable peptide linker" are used interchangeably. In some instances, the linker sequence may be cleaved enzymatically and/or chemically. Examples of enzymes (e.g., proteinase/peptidase) useful for cleaving the peptide linker include, but are not limited, to Arg-C proteinase, Asp-N endopeptidase, chymotrypsin, clostripain, enterokinase, Factor Xa, glutamyl endopeptidase, Granzyme B, Achromobacter proteinase I, pepsin, proline endopeptidase, proteinase K, Staphylococcal peptidase I, thermolysin, thrombin, trypsin, and members of the Caspase family of proteolytic enzymes (e.g. Caspases 1-10). Chemical sensitive cleavage sites may also be included in a linker sequence. Examples of chemical cleavage reagents include, but are not limited to, cyanogen bromide, which cleaves methionine residues; N-chloro succinimide, iodobenzoic acid or BNPS-skatole [2-(2-nitrophenylsulfenyl)-3-methylindole], which cleaves tryptophan residues; dilute acids, which cleave at aspartyl-prolyl bonds; and e aspartic acid-proline acid cleavable recognition sites (i.e., a cleavable peptide linker comprising one or more D-P dipeptide moieties). The fusion module may include multiple regions encoding peptides of interest separated by one or more cleavable peptide linkers.

In other instances, a cleavable linker may be a "self-cleaving" linker peptide, such as 2A linkers (for example T2A), 2A-like linkers or functional equivalents thereof and combinations thereof. In some instances, the linkers include the picornaviral 2A-like linker, CHYSEL sequences of porcine teschovirus (P2A), Thosea asigna virus (T2A) or combinations, variants and functional equivalents thereof. In some instances, the biocircuits of the present disclosure may include 2A peptides. The 2A peptide is a sequence of about 20 amino acid residues from a virus that is recognized by a protease (2A peptidases) endogenous to the cell. The 2A peptide was identified among picornaviruses, a typical example of which is the Foot-and Mouth disease virus (Robertson BH, et. al., J Virol 1985, 54:651-660). 2A-like sequences have also been found in Picornaviridae like equine rhinitis A virus, as well as unrelated viruses such as porcine teschovirus-1 and the insect Thosea asigna virus (TaV). In such viruses, multiple proteins are derived from a large polyprotein encoded by an open reading frame. The 2A peptide mediates the co-translational cleavage of this polyprotein at a single site that forms the junction between the virus capsid and replication polyprotein domains. The 2A sequences contain the consensus motif D-V/I-E-X-N-P-G-P (SEQ ID NO: 8381). These sequences are thought to act co-translationally, preventing the formation of a normal peptide bond between the glycine and last proline, resulting in the ribosome skipping of the next codon (Donnelly ML et al. (2001). J Gen Virol, 82:1013-1025). After cleavage, the short peptide remains fused to the C -terminus of the protein upstream of the cleavage site, while the proline is added to the N-terminus of the protein downstream of the cleavage site. Of the 2A peptides identified, four have been widely used namely FMDV 2A (abbreviated herein as F2A); equine rhinitis A virus (ERAV) 2A (E2A); porcine teschovirus-1 2A (P2A) and Thoseaasigna virus 2A (T2A). In some instances, the 2A peptide sequences useful in the present disclosure are selected from SEQ ID NO: 8-11 of International Patent Publication WO2010042490. In some instances, the cleavage site may be a P2A cleavable peptide (SEQ ID NO: 8239), encoded by the nucleotide sequence of SEQ ID NO: 8269.

Other linkers will be apparent to those skilled in the art and may be used in connection with alternate instances of the disclosure.

The linkers of the present disclosure may also be non-peptide linkers. For example, alkyl linkers such as -NH-(CH₂) a-C(O)-, wherein a=2-20 can be used. These alkyl linkers may further be substituted by any non-sterically hindering group such as lower alkyl (e.g., C₁-C₆) lower acyl, halogen (e.g., Cl, Br), CN, NH₂, phenyl, etc.

In some aspects, the linker may be an artificial linker from U.S. Pat. NOs. 4,946,778, 5,525,491, 5,856,456, and International patent publication NO. WO2012/083424.

### 6. Embedded stimulus, signals and other regulatory features

microRNAs (or miRNA) are 19-25 nucleotide long noncoding RNAs that bind to the 3'UTR of nucleic acid molecules and down-regulate gene expression either by reducing nucleic acid molecule stability or by inhibiting translation. The polynucleotides of the disclosure may comprise one or more microRNA target sequences, microRNA sequences, or microRNA seeds. Such sequences may correspond to any known microRNA such as those taught in US Publication No. US2005/0261218 and US Publication No. US2005/0059005. As a non-limiting instance, known microRNAs, their sequences and their binding site sequences in the human genome are listed Table 14 of the co-owned U.S. Provisional Patent Application No. 62/320,864 filed April 11, 2016, 62/466,596 filed March 3, 2017 and the International Publication WO2017/180587 .

A microRNA sequence comprises a "seed" region, i.e., a sequence in the region of positions 2-8 of the mature microRNA, which sequence has perfect Watson-Crick complementarity to the miRNA target sequence. A microRNA seed may comprise positions 2-8 or 2-7 of the mature microRNA. In some instances, a microRNA seed may comprise 7 nucleotides (e.g., nucleotides 2-8 of the mature microRNA), wherein the seed-complementary site in the corresponding miRNA target is flanked by an adenine (A) opposed to microRNA position 1. In some instances, a microRNA seed may comprise 6 nucleotides (e.g., nucleotides 2-7 of the mature microRNA), wherein the seed-complementary site in the corresponding miRNA target is flanked by an adenine (A) opposed to microRNA position 1. See for example, Grimson A, Farh KK, Johnston WK, Garrett-Engele P, Lim LP, Bartel DP; Mol Cell. 2007 Jul 6;27(1):91-105. The bases of the microRNA seed have complete complementarity with the target sequence. By engineering microRNA target sequences into the polynucleotides encoding the biocircuit components, effector modules, SREs or payloads of the disclosure one can target the molecule for degradation or reduced translation, provided the microRNA in question is available. This process will reduce the hazard of off target effects upon nucleic acid molecule delivery.

Identification of microRNA, microRNA target regions, and their expression patterns and role in biology have been reported (Bonauer et al., Curr Drug Targets 2010 11:943-949; Anand and Cheresh Curr Opin Hematol 2011 18:171-176; Contreras and Rao Leukemia 2012 26:404-413 (2011 Dec 20. doi: 10.1038/leu.2011.356); Bartel Cell 2009 136:215-233; Landgraf et al, Cell, 2007 129:1401-1414; Gentner and Naldini, Tissue Antigens. 2012 80:393-403 and all references therein).

For example, if the polynucleotide is not intended to be delivered to the liver but ends up there, then miR-122, a microRNA abundant in liver, can inhibit the expression of the polynucleotide if one or multiple target sites of miR-122 are engineered into the polynucleotide. Introduction of one or multiple binding sites for different microRNA can be engineered to further decrease the longevity, stability, and protein translation of a polynucleotide hence providing an additional layer of tenability beyond the stimulus selection, SRE design and payload variation.

As used herein, the term "microRNA site" refers to a microRNA target site or a microRNA recognition site, or any nucleotide sequence to which a microRNA binds or associates. It should be understood that "binding" may follow traditional Watson-Crick hybridization rules or may reflect any stable association of the microRNA with the target sequence at or adjacent to the microRNA site.

Conversely, for the purposes of the polynucleotides of the present disclosure, microRNA binding sites can be engineered out of (i.e. removed from) sequences in which they naturally occur in order to increase protein expression in specific tissues. For example, miR-122 binding sites may be removed to improve protein expression in the liver.

Regulation of expression in multiple tissues can be accomplished through introduction or removal or one or several microRNA binding sites.

Specifically, microRNAs are known to be differentially expressed in immune cells (also called hematopoietic cells), such as antigen presenting cells (APCs) (e.g. dendritic cells and macrophages), macrophages, monocytes, B lymphocytes, T lymphocytes, granulocytes, natural killer cells, etc. Immune cell specific microRNAs are involved in immunogenicity, autoimmunity, the immune -response to infection, inflammation, as well as unwanted immune response after gene therapy and tissue/organ transplantation. Immune cells specific microRNAs also regulate many aspects of development, proliferation, differentiation and apoptosis of hematopoietic cells (immune cells). For example, miR-142 and miR-146 are exclusively expressed in the immune cells, particularly abundant in myeloid dendritic cells. Introducing the miR-142 binding site into the 3'-UTR of a polypeptide of the present disclosure can selectively suppress the gene expression in the antigen presenting cells through miR-142 mediated mRNA degradation, limiting antigen presentation in professional APCs (e.g. dendritic cells) and thereby preventing antigen-mediated immune response after gene delivery (see, Annoni A et al., blood, 2009, 114, 5152-5161.)

In one instance, microRNAs binding sites that are known to be expressed in immune cells, in particular, the antigen presenting cells, can be engineered into the polynucleotides to suppress the expression of the polynucleotide in APCs through microRNA mediated RNA degradation, subduing the antigen-mediated immune response, while the expression of the polynucleotide is maintained in non-immune cells where the immune cell specific microRNAs are not expressed.

Many microRNA expression studies have been conducted, and are described in the art, to profile the differential expression of microRNAs in various cancer cells /tissues and other diseases. Some microRNAs are abnormally over-expressed in certain cancer cells and others are under-expressed. For example, microRNAs are differentially expressed in cancer cells (WO2008/154098, US2013/0059015, US2013/0042333, WO2011/157294); cancer stem cells (US2012/0053224); pancreatic cancers and diseases (US2009/0131348, US2011/0171646, US2010/0286232, US8389210); asthma and inflammation (US8415096); prostate cancer (US2013/0053264); hepatocellular carcinoma (WO2012/151212, US2012/0329672, WO2008/054828, US8252538); lung cancer cells (WO2011/076143, WO2013/033640, WO2009/070653, US2010/0323357); cutaneous T cell lymphoma (WO2013/011378); colorectal cancer cells (WO2011/0281756, WO2011/076142); cancer positive lymph nodes (WO2009/100430, US2009/0263803); nasopharyngeal carcinoma (EP2112235); chronic obstructive pulmonary disease (US2012/0264626, US2013/0053263); thyroid cancer (WO2013/066678); ovarian cancer cells ( US2012/0309645, WO2011/095623); breast cancer cells (WO2008/154098, WO2007/081740, US2012/0214699), leukemia and lymphoma (WO2008/073915, US2009/0092974, US2012/0316081, US2012/0283310, WO2010/018563).

In one instance, microRNA may be used as described herein in support of the creation of tunable biocircuits.

In some instances, effector modules may be designed to encode (as a DNA or RNA or mRNA) one or more payloads, SREs and/or regulatory sequence such as a microRNA or microRNA binding site. In some instances, any of the encoded payloads or SREs may be stabilized or de-stabilized by mutation and then combined with one or more regulatory sequences to generate a dual or multi-tuned effector module or biocircuit system.

Each aspect or tuned modality may bring to the effector module or biocircuit a differentially tuned feature. For example, an SRE may represent a destabilizing domain, while mutations in the protein payload may alter its cleavage sites or dimerization properties or half-life and the inclusion of one or more microRNA or microRNA binding site may impart cellular detargeting or trafficking features. Consequently, the present disclosure embraces biocircuits which are multifactorial in their tenability.

In some instances, compositions of the disclosure may include optional proteasome adaptors. As used herein, the term "proteasome adaptor" refers to any nucleotide/ amino acid sequence that targets the appended payload for degradation. In some aspects, the adaptors target the payload for degradation directly thereby circumventing the need for ubiquitination reactions. Proteasome adaptors may be used in conjunction with destabilizing domains to reduce the basal expression of the payload. Exemplary proteasome adaptors include the UbL domain of Rad23 or hHR23b, HPV E7 which binds to both the target protein Rb and the S4 subunit of the proteasome with high affinity, which allows direct proteasome targeting, bypassing the ubiquitination machinery; the protein gankyrin which binds to Rb and the proteasome subunit S6.

Such biocircuits may be engineered to contain one, two, three, four or more tuned features.

### Polynucleotides

The present disclosure provides polynucleotides encoding novel hPDE5 DDs, effector modules comprising payloads and associated DDs, biocircuit systems comprising DDs and effector modules, and other components of the present disclosure.

The disclosure provides isolated biocircuit polypeptides, effector modules, stimulus response elements (SREs) and payloads, as well as polynucleotides encoding any of the foregoing; vectors comprising polynucleotides of the disclosure; and cells expressing polypeptides, polynucleotides and vectors of the disclosure. The polypeptides, polynucleotides, viral vectors and cells are useful for inducing anti-tumor immune responses in a subject.

The term "polynucleotide" or "nucleic acid molecule" in its broadest sense, includes any compound and/or substance that comprise a polymer of nucleotides, e.g., linked nucleosides. These polymers are often referred to as polynucleotides. Exemplary nucleic acids or polynucleotides of the disclosure include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, α-LNA having an α-L-ribo configuration (a diastereomer of LNA), 2'-amino-LNA having a 2'-amino functionalization, and 2'-amino- α-LNA having a 2'-amino functionalization) or hybrids thereof.

In some instances, polynucleotides of the disclosure may be a messenger RNA (mRNA) or any nucleic acid molecule and may or may not be chemically modified. In one aspect, the nucleic acid molecule is a mRNA. As used herein, the term "messenger RNA (mRNA)" refers to any polynucleotide which encodes a polypeptide of interest and which is capable of being translated to produce the encoded polypeptide of interest *in vitro, in vivo, in situ* or *ex vivo.*

Traditionally, the basic components of an mRNA molecule include at least a coding region, a 5'UTR, a 3'UTR, a 5' cap and a poly-A tail. Building on this wild type modular structure, the present disclosure expands the scope of functionality of traditional mRNA molecules by providing payload constructs which maintain a modular organization, but which comprise one or more structural and/or chemical modifications or alterations which impart useful properties to the polynucleotide, for example tenability of function. As used herein, a "structural" feature or modification is one in which two or more linked nucleosides are inserted, deleted, duplicated, inverted or randomized in a polynucleotide without significant chemical modification to the nucleosides themselves. Because chemical bonds will necessarily be broken and reformed to effect a structural modification, structural modifications are of a chemical nature and hence are chemical modifications. However, structural modifications will result in a different sequence of nucleotides. For example, the polynucleotide "ATCG" may be chemically modified to "AT-5meC-G". The same polynucleotide may be structurally modified from "ATCG" to "ATCCCG". Here, the dinucleotide "CC" has been inserted, resulting in a structural modification to the polynucleotide.

In some instances, polynucleotides of the present disclosure may harbor 5'UTR sequences which play a role in translation initiation. 5'UTR sequences may include features such as Kozak sequences which are commonly known to be involved in the process by which the ribosome initiates translation of genes, Kozak sequences have the consensus XCCR(A/G) CCAUG, where R is a purine (adenine or guanine) three bases upstream of the start codon (AUG) and X is any nucleotide. In one instance, the Kozak sequence is ACCGCC. By engineering the features that are typically found in abundantly expressed genes of target cells or tissues, the stability and protein production of the polynucleotides of the disclosure can be enhanced.

Further provided are polynucleotides, which may contain an internal ribosome entry site (IRES) which play an important role in initiating protein synthesis in the absence of 5' cap structure in the polynucleotide. An IRES may act as the sole ribosome binding site, or may serve as one of the multiple binding sites. Polynucleotides of the disclosure containing more than one functional ribosome binding site may encode several peptides or polypeptides that are translated independently by the ribosomes giving rise to bicistronic and/or multicistronic nucleic acid molecules. In one instance, the IRES may be SEQ ID NO: 8667.

In some instances, polynucleotides encoding biocircuits, effector modules, DDs and payloads may include from about 30 to about 100,000 nucleotides (e.g., from 30 to 50, from 30 to 100, from 30 to 250, from 30 to 500, from 30 to 1,000, from 30 to 1,500, from 30 to 3,000, from 30 to 5,000, from 30 to 7,000, from 30 to 10,000, from 30 to 25,000, from 30 to 50,000, from 30 to 70,000, from 100 to 250, from 100 to 500, from 100 to 1,000, from 100 to 1,500, from 100 to 3,000, from 100 to 5,000, from 100 to 7,000, from 100 to 10,000, from 100 to 25,000, from 100 to 50,000, from 100 to 70,000, from 100 to 100,000, from 500 to 1,000, from 500 to 1,500, from 500 to 2,000, from 500 to 3,000, from 500 to 5,000, from 500 to 7,000, from 500 to 10,000, from 500 to 25,000, from 500 to 50,000, from 500 to 70,000, from 500 to 100,000, from 1,000 to 1,500, from 1,000 to 2,000, from 1,000 to 3,000, from 1,000 to 5,000, from 1,000 to 7,000, from 1,000 to 10,000, from 1,000 to 25,000, from 1,000 to 50,000, from 1,000 to 70,000, from 1,000 to 100,000, from 1,500 to 3,000, from 1,500 to 5,000, from 1,500 to 7,000, from 1,500 to 10,000, from 1,500 to 25,000, from 1,500 to 50,000, from 1,500 to 70,000, from 1,500 to 100,000, from 2,000 to 3,000, from 2,000 to 5,000, from 2,000 to 7,000, from 2,000 to 10,000, from 2,000 to 25,000, from 2,000 to 50,000, from 2,000 to 70,000, and from 2,000 to 100,000 nucleotides). In some aspects, polynucleotides of the disclosure may include more than 10,000 nucleotides.

Regions of the polynucleotides which encode certain features such as cleavage sites, linkers, trafficking signals, tags or other features may range independently from 10-1,000 nucleotides in length (e.g., greater than 20, 30, 40, 45, 50, 55, 60, 70, 80, 90, 100, 120, 140, 160, 180, 200, 250, 300, 350, 400, 450, 500, 600, 700, 800, and 900 nucleotides or at least 10, 15, 20, 25, 30, 35, 40, 45, 50, 55, 60, 70, 80, 90, 100, 120, 140, 160, 180, 200, 250, 300, 350, 400, 450, 500, 600, 700, 800, 900, and 1,000 nucleotides).

In some instances, polynucleotides of the present disclosure may further comprise embedded regulatory moieties such as microRNA binding sites within the 3'UTR of nucleic acid molecules which when bind to microRNA molecules, down-regulate gene expression either by reducing nucleic acid molecule stability or by inhibiting translation. Conversely, for the purposes of the polynucleotides of the present disclosure, microRNA binding sites can be engineered out of (i.e. removed from) sequences in which they naturally occur in order to increase protein expression in specific tissues. For example, miR-142 and miR-146 binding sites may be removed to improve protein expression in the immune cells. In some instances, any of the encoded payloads may be regulated by an SRE and then combined with one or more regulatory sequences to generate a dual or multi-tuned effector module or biocircuit system.

In some instances, polynucleotides of the present disclosure may encode fragments, variants, derivatives of polypeptides of the disclosures. In some aspects, the variant sequence may keep the same or a similar activity. Alternatively, the variant may have an altered activity (e.g., increased or decreased) relative to the start sequence. Generally, variants of a particular polynucleotide or polypeptide of the disclosure will have at least about 40%, 45%, 50%, 55%, 60%, 65%, 70%, 75%, 80%, 85%, 90%, 91%, 92%, 93%, 94%, 95%, 96%, 97%, 98%, 99% but less than 100% sequence identity to that particular reference polynucleotide or polypeptide as determined by sequence alignment programs and parameters described herein and known to those skilled in the art. Such tools for alignment include those of the BLAST suite (Stephen et al., Gapped BLAST and PSI-BLAST: a new generation of protein database search programs, Nucleic Acids Res., 1997, 25:3389-3402.)

In some instances, polynucleotides of the present disclosure may be modified. As used herein, the terms "modified", or as appropriate, "modification" refers to chemical modification with respect to A, G, U (T in DNA) or C nucleotides. Modifications may be on the nucleoside base and/or sugar portion of the nucleosides which comprise the polynucleotide. In some instances, multiple modifications are included in the modified nucleic acid or in one or more individual nucleoside or nucleotide. For example, modifications to a nucleoside may include one or more modifications to the nucleobase and the sugar. Modifications to the polynucleotides of the present disclosure may include any of those taught in, for example, International Publication NO. WO2013/052523y.

As described herein "nucleoside" is defined as a compound containing a sugar molecule (e.g., a pentose or ribose) or a derivative thereof in combination with an organic base (e.g., a purine or pyrimidine) or a derivative thereof (also referred to herein as "nucleobase"). As described herein, "nucleotide" is defined as a nucleoside including a phosphate group.

In some instances, the modification may be on the internucleoside linkage (e.g., phosphate backbone). Herein, in the context of the polynucleotide backbone, the phrases "phosphate" and "phosphodiester" are used interchangeably. Backbone phosphate groups can be modified by replacing one or more of the oxygen atoms with a different substituent. Further, the modified nucleosides and nucleotides can include the wholesale replacement of an unmodified phosphate moiety with another internucleoside linkage. Examples of modified phosphate groups include, but are not limited to, phosphorothioate, phosphonoselenoates, boranophosphates, boranophosphate esters, hydrogen phosphonates, phosphoramidates, phosphonodiamidites, alkyl or aryl phosphonates, and phosphotriesters. Phosphorodithioates have both non-linking oxygens replaced by sulfur. The phosphate linker can also be modified by the replacement of a linking oxygen with nitrogen (bridged phosphoramidates), sulfur (bridged phosphorothioates), and carbon (bridged methylene-phosphonates). Other modifications which may be used are taught in, for example, International Application NO.: WO2013/052523.

Chemical modifications and/or substitution of the nucleotides or nucleobases of the polynucleotides of the disclosure which are useful in the present disclosure include any modified substitutes known in the art, for example, (±)1-(2-Hydroxypropyl)pseudouridine TP, (2R)-1-(2-Hydroxypropyl)pseudouridine TP, 1-(4-Methoxyphenyl)pseudo-UTP, ,2'-O-dimethyladenosine, 1,2'-O-dimethylguanosine, 1,2'-O-dimethylinosine, 1-Hexyl-pseudo-UTP , 1-Homoallylpseudouridine TP, 1-Hydroxymethylpseudouridine TP, 1-iso-propyl-pseudo-UTP , 1-Me-2-thio-pseudo-UTP, 1-Me-4-thio-pseudo-UTP, 1-Me-alpha-thio-pseudo-UTP, 1-Me-GTP, 2'-Amino-2'-deoxy-ATP, 2'-Amino-2'-deoxy-CTP, 2'-Amino-2'-deoxy-GTP, 2'-Amino-2'-deoxy-UTP, 2'-Azido-2'-deoxy-ATP, tubercidine, undermodified hydroxywybutosine, uridine 5-oxyacetic acid, uridine 5-oxyacetic acid methyl ester, wybutosine, wyosine, xanthine, Xanthosine-5'-TP, xylo-adenosine, zebularine, α-thio-adenosine, α-thio-cytidine, α-thio-guanosine, and/or α-thio-uridine.

Polynucleotides of the present disclosure may comprise one or more of the modifications taught herein. Different sugar modifications, base modifications, nucleotide modifications, and/or internucleoside linkages (e.g., backbone structures) may exist at various positions in the polynucleotide of the disclosure. One of ordinary skill in the art will appreciate that the nucleotide analogs or other modification(s) may be located at any position(s) of a polynucleotide such that the function of the polynucleotide is not substantially decreased. A modification may also be a 5' or 3' terminal modification. The polynucleotide may contain from about 1% to about 100% modified nucleotides (either in relation to overall nucleotide content, or in relation to one or more types of nucleotide, i.e. any one or more of A, G, U or C) or any intervening percentage (e.g., from 1% to 20%, from 1% to 25%, from 1% to 50%, from 1% to 60%, from 1% to 70%, from 1% to 80%, from 1% to 90%, from 1% to 95%, from 10% to 20%, from 10% to 25%, from 10% to 50%, from 10% to 60%, from 10% to 70%, from 10% to 80%, from 10% to 90%, from 10% to 95%, from 10% to 100%, from 20% to 25%, from 20% to 50%, from 20% to 60%, from 20% to 70%, from 20% to 80%, from 20% to 90%, from 20% to 95%, from 20% to 100%, from 50% to 60%, from 50% to 70%, from 50% to 80%, from 50% to 90%, from 50% to 95%, from 50% to 100%, from 70% to 80%, from 70% to 90%, from 70% to 95%, from 70% to 100%, from 80% to 90%, from 80% to 95%, from 80% to 100%, from 90% to 95%, from 90% to 100%, and from 95% to 100%).

In some instances, one or more codons of the polynucleotides of the present disclosure may be replaced with other codons encoding the native amino acid sequence to tune the expression of the SREs, through a process referred to as codon selection. Since mRNA codon, and tRNA anticodon pools tend to vary among organisms, cell types, sub cellular locations and over time, the codon selection described herein is a spatiotemporal (ST) codon selection.

In some instances of the disclosure, certain polynucleotide features may be codon optimized. Codon optimization refers to a process of modifying a nucleic acid sequence for enhanced expression in the host cell by replacing at least 1, 2, 3, 4, 5, 10, 15, 20, 25, 50 or more codons of the native sequence with codons that are most frequently used in the genes of that host cell while maintaining the native amino acid sequence. Codon usage may be measured using the Codon Adaptation Index (CAI) which measures the deviation of a coding polynucleotide sequence from a reference gene set. Codon usage tables are available at the Codon Usage Database (www.kazusa.or.jp/codon/) and the CAI can be calculated by EMBOSS CAI program (emboss.sourceforge.net/). Codon optimization methods are known in the art and may be useful in efforts to achieve one or more of several goals. These goals include to match codon frequencies in target and host organisms to ensure proper folding, bias nucleotide content to alter stability or reduce secondary structures, minimize tandem repeat codons or base runs that may impair gene construction or expression, customize transcriptional and translational control regions, insert or remove protein signaling sequences, remove/add post translation modification sites in encoded protein (e.g. glycosylation sites), add, remove or shuffle protein domains, insert or delete restriction sites, modify ribosome binding sites and degradation sites, to adjust translational rates to allow the various domains of the protein to fold properly, or to reduce or eliminate problem secondary structures within the polynucleotide. Codon optimization tools, algorithms and services are known in the art, and non-limiting examples include services from GeneArt (Life Technologies), DNA2.0 (Menlo Park CA), OptimumGene (GenScript, Piscataway, NJ), algorithms such as but not limited to, DNAWorks v3.2.3, and/or proprietary methods. In one instance, a polynucleotide sequence or portion thereof is codon optimized using optimization algorithms. Codon options for each amino acid are well-known in the art as are various species table for optimizing for expression in that particular species.

In some instances of the disclosure, certain polynucleotide features may be codon optimized. For example, a preferred region for codon optimization may be upstream (5') or downstream (3') to a region which encodes a polypeptide. These regions may be incorporated into the polynucleotide before and/or after codon optimization of the payload encoding region or open reading frame (ORF).

After optimization (if desired), the polynucleotide components are reconstituted and transformed into a vector such as, but not limited to, plasmids, viruses, cosmids, and artificial chromosomes.

Spatiotemporal codon selection may impact the expression of the polynucleotides of the disclosure, since codon composition determines the rate of translation of the mRNA species and its stability. For example, tRNA anticodons to optimized codons are abundant, and thus translation may be enhanced. In contrast, tRNA anticodons to less common codons are fewer and thus translation may proceed at a slower rate. Presnyak et al. have shown that the stability of an mRNA species is dependent on the codon content, and higher stability and thus higher protein expression may be achieved by utilizing optimized codons (Presnyak et al. (2015) Cell 160, 1111-1124). Thus, in some instances, ST codon selection may include the selection of optimized codons to enhance the expression of the SRES, effector modules and biocircuits of the disclosure. In other instances, spatiotemporal codon selection may involve the selection of codons that are less commonly used in the genes of the host cell to decrease the expression of the compositions of the disclosure. The ratio of optimized codons to codons less commonly used in the genes of the host cell may also be varied to tune expression.

In some instances, certain regions of the polynucleotide may be modified using codon selection methods. For example, a preferred region for codon selection may be upstream (5') or downstream (3') to a region which encodes a polypeptide. These regions may be incorporated into the polynucleotide before and/or after codon selection of the payload encoding region or open reading frame (ORF).

The stop codon of the polynucleotides of the present disclosure may be modified to include sequences and motifs to alter the expression levels of the SREs, payloads and effector modules of the present disclosure. Such sequences may be incorporated to induce stop codon readthrough, wherein the stop codon may specify amino acids e.g. selenocysteine or pyrrolysine. In other instances, stop codons may be skipped altogether to resume translation through an alternate open reading frame. Stop codon read through may be utilized to tune the expression of components of the effector modules at a specific ratio (e.g.as dictated by the stop codon context). Examples of preferred stop codon motifs include UGAN, UAAN, and UAGN, where N is either C or U.

Suppression of termination occurs during translation of many viral mRNAs as a means of generating a second protein with extended carboxy terminus. In retroviruses, gag and pol genes are encoded by a single mRNA and separated by an amber termination codon UAG. Translational suppression of the amber codon allows synthesis of the gag pol precursor. Translation suppression is mediated by suppressor tRNAs that can recognize termination codons and insert a specific amino acid. In some instances, effector modules described herein may incorporate amber termination codons. Such codons may be used in lieu of or in addition to IRES and p2A sequences in bicistronic constructs. Stop codon read through may be combined with P2A to obtain low level expression of downstream gene (e.g. IL12). In some instances, the amber stop codons may be combined with tRNA expression or amino-acyl tRNA synthetase for further control. In one aspect, the payload may be a regulated tRNA synthetase.

Polynucleotide modifications and manipulations can be accomplished by methods known in the art such as, but not limited to, site directed mutagenesis and recombinant technology. The resulting modified molecules may then be tested for activity using *in vitro* or *in vivo* assays such as those described herein or any other suitable screening assay known in the art.

In some instances, polynucleotides of the disclosure may comprise two or more effector module sequences, or two or more payload sequences, which are in a pattern such as ABABAB or AABBAABBAABB or ABCABCABC or variants thereof repeated once, twice, or more than three times. In these patterns, each letter, A, B, or C represent a different effector module component.

In yet another instance, polynucleotides of the disclosure may comprise two or more effector module component sequences with each component having one or more SRE sequences (DD sequences), or two or more payload sequences. As a non-limiting example, the sequences may be in a pattern such as ABABAB or AABBAABBAABB or ABCABCABC or variants thereof repeated once, twice, or more than three times in each of the regions. As another non-limiting example, the sequences may be in a pattern such as ABABAB or AABBAABBAABB or ABCABCABC or variants thereof repeated once, twice, or more than three times across the entire polynucleotide. In these patterns, each letter, A, B, or C represent a different sequence or component.

According to the present disclosure, polynucleotides encoding distinct biocircuits, effector modules, SREs and payload constructs may be linked together through the 3'-end using nucleotides which are modified at the 3'-terminus. Chemical conjugation may be used to control the stoichiometry of delivery into cells. Polynucleotides can be designed to be conjugated to other polynucleotides, dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), alkylating agents, phosphate, amino, mercapto, PEG (e.g., PEG-40K), MPEG, [MPEG]₂, polyamino, alkyl, substituted alkyl, radiolabeled markers, enzymes, haptens (e.g. biotin), transport/absorption facilitators (e.g., aspirin, vitamin E, folic acid), synthetic ribonucleases, proteins, e.g., glycoproteins, or peptides, e.g., molecules having a specific affinity for a co-ligand, or antibodies e.g., an antibody, that binds to a specified cell type such as a cancer cell, endothelial cell, or bone cell, hormones and hormone receptors, non-peptidic species, such as lipids, lectins, carbohydrates, vitamins, cofactors, or a drug. As non-limiting examples, they may be conjugates with other immune conjugates.

In some instances, the compositions of the polynucleotides of the disclosure may generated by combining the various components of the effector modules using the Gibson assembly method. The Gibson assembly reaction consists of three isothermal reactions, each relying on a different enzymatic activity including a 5' exonuclease which generates long overhangs, a polymerase which fills in the gaps of the annealed single strand regions and a DNA ligase which seals the nicks of the annealed and filled-in gaps. Polymerase chain reactions are performed prior to Gibson assembly which may be used to generate PCR products with overlapping sequence. These methods can be repeated sequentially, to assemble larger and larger molecules. For example, the method can comprise repeating a method as above to join a second set of two or more DNA molecules of interest to one another, and then repeating the method again to join the first and second set DNA molecules of interest, and so on. At any stage during these multiple rounds of assembly, the assembled DNA can be amplified by transforming it into a suitable microorganism, or it can be amplified in vitro (e.g., with PCR).

In some instances, polynucleotides of the disclosure may encode effector modules comprising a destabilizing domain (DD) and at least one payload taught herein. The DD domain may be a hPDE5 mutant comprising one, two, three, four, five or more mutations

In some instances, the effector module may be a PDE5-GFP fusion encoded by SEQ ID NO: 95-106; 205-222; 234-236; 256-260; 378-379; 469-503; 526-533; 8521- 8550; 8644-8647; and 8611-8629.In some instances, the effector module may be hPDE5-CAR constructs, encoded by SEQ ID NO: 8285-8298 or a hPDE5-IL15-IL15Ra constructs, encoded by SEQ ID NO: 8352- 8361; 8670-8684 or 8702-8718.

### Cells

In accordance with the present disclosure, cells genetically modified to express at least one biocircuit, SRE (e.g, DD), effector module and immunotherapeutic agent of the disclosure, are provided. Cells of the disclosure may include, without limitation, immune cells, stem cells and tumor cells. In some instances, immune cells are immune effector cells, including, but not limiting to, T cells such as CD8⁺ T cells and CD4⁺ T cells (e.g., Th1, Th2, Th17 , Foxp3+ cells), memory T cells such as T memory stem cells, central T memory cells, and effector memory T cells, terminally differentiated effector T cells, natural killer (NK) cells, NK T cells, tumor infiltrating lymphocytes (TILs), cytotoxic T lymphocytes (CTLs), regulatory T cells (Tregs), and dendritic cells (DCs), other immune cells that can elicit an effector function, or the mixture thereof. T cells may be Tαβ cells and Tγδ cells. In some instances, stem cells may be from human embryonic stem cells, mesenchymal stem cells, and neural stem cells. In some instances, T cells may be depleted endogenous T cell receptors (See US Pat. NOs.: 9,273,283; 9,181,527; and 9,028,812).

In some instances, cells of the disclosure may be autologous, allogeneic, syngeneic, or xenogeneic in relation to a particular individual subject.

In some instances, cells of the disclosure may be mammalian cells, particularly human cells. Cells of the disclosure may be primary cells or immortalized cell lines.

In some instances, cells of the disclosure may include expansion factors as payload to trigger proliferation and expansion of the cells. Exemplary payloads include RAS such as KRAS, NRAS, RRAS, RRAS2, MRAS, ERAS, and HRAS, DIRAS such as DIRAS1, DIRAS2, and DIRAS3, NKIRAS such as NKIRAS1, and NKIRAS2, RAL such as RALA, and RALB, RAP such as RAP1A, RAP1B, RAP2A, RAP2B, and RAP2C, RASD such as RASD1, and RASD2, RASL such as RASL10A, RASL10B, RASL11A, RASL11B, and RASL12, REM such as REM1, and REM2, GEM, RERG, RERGL, and RRAD.

Engineered immune cells can be accomplished by transducing a cell with a polypeptide of a biocircuit, an effector module, a SRE and/or a payload of interest (i.e., immunotherapeutic agent), or a polynucleotide encoding said polypeptide, or a vector comprising said polynucleotide. The vector may be a viral vector such as a lentiviral vector, a gamma-retroviral vector, a recombinant AAV, an adenoviral vector and an oncolytic viral vector. In other aspects, non-viral vectors for example, nanoparticles and liposomes may also be used. In some instances, immune cells of the disclosure are genetically modified to express at least one immunotherapeutic agent of the disclosure which is tunable using a stimulus. In some examples, two, three or more immunotherapeutic agents constructed in the same biocircuit and effector module are introduced into a cell. In other examples, two, three, or more biocircuits, effector modules, each of which comprises an immunotherapeutic agent, may be introduced into a cell.

In some instances, immune cells of the disclosure may be T cells modified to express an antigen-specific T cell receptor (TCR), or an antigen specific chimeric antigen receptor (CAR) taught herein (known as CAR T cells). Accordingly, at least one polynucleotide encoding a CAR system (or a TCR) described herein, or a vector comprising the polynucleotide is introduced into a T cell. The T cell expressing the CAR or TCR binds to a specific antigen via the extracellular targeting moiety of the CAR or TCR, thereby a signal via the intracellular signaling domain (s) is transmitted into the T cell, and as a result, the T cell is activated. The activated CAR T cell changes its behavior including release of a cytotoxic cytokine (e.g., a tumor necrosis factor, and lymphotoxin, etc.), improvement of a cell proliferation rate, change in a cell surface molecule, or the like. Such changes cause destruction of a target cell expressing the antigen recognized by the CAR or TCR. In addition, release of a cytokine or change in a cell surface molecule stimulates other immune cells, for example, a B cell, a dendritic cell, a NK cell, and a macrophage.

The CAR introduced into a T cell may be a first-generation CAR including only the intracellular signaling domain from TCR CD3zeta, or a second-generation CAR including the intracellular signaling domain from TCR CD3zeta and a costimulatory signaling domain, or a third-generation CAR including the intracellular signaling domain from TCR CD3zeta and two or more costimulatory signaling domains, or a split CAR system, or an on/off switch CAR system. In one example, the expression of the CAR or TCR is controlled by a destabilizing domain (DD) such as a hDHFR mutant, in the effector module of the disclosure. The presence or absence of hDHFR binding ligand such as TMP is used to tune the CAR or TCR expression in transduced T cells or NK cells.

In some instances, CAR T cells of the disclosure may be further modified to express another one, two, three or more immunotherapeutic agents. The immunotherapeutic agents may be another CAR or TCR specific to a different target molecule; a cytokine such as IL2, IL12, IL15 and IL18, or a cytokine receptor such as IL15Ra; a chimeric switch receptor that converts an inhibitory signal to a stimulatory signal; a homing receptor that guides adoptively transferred cells to a target site such as the tumor tissue; an agent that optimizes the metabolism of the immune cell; or a safety switch gene (e.g., a suicide gene) that kills activated T cells when a severe event is observed after adoptive cell transfer or when the transferred immune cells are no-longer needed. These molecules may be included in the same effector module or in separate effector modules.

In one instance, the CAR T cell (including TCR T cell) of the disclosure may be an "armed" CAR T cell which is transformed with an effector module comprising a CAR and an effector module comprising a cytokine. The inducible or constitutively secrete active cytokines further armor CAR T cells to improve efficacy and persistence. In this context, such CAR T cell is also referred to as "armored CAR T cell". The "armor" molecule may be selected based on the tumor microenvironment and other elements of the innate and adaptive immune systems. In some instances, the molecule may be a stimulatory factor such as IL2, IL12, IL15, IL18, type I IFN, CD40L and 4-1BBL which have been shown to further enhance CAR T cell efficacy and persistence in the face of a hostile tumor microenvironment via different mechanisms (Yeku et al., Biochem Soc Trans., 2016, 44(2): 412-418).

In some aspects, the armed CAR T cell of the disclosure is modified to express a CD19 CAR and IL12. Such T cells, after CAR mediated activation in the tumor, release inducible IL12 which augments T-cell activation and attracts and activates innate immune cells to eliminate CD19-negative cancer cells.

In one instance, T cells of the disclosure may be modified to express an effector module comprising a CAR and an effector module comprising a suicide gene.

In one instance, the CAR T cell (including TCR T cell) of the disclosure may be transformed with effector modules comprising a cytokine and a safety switch gene (e.g., suicide gene). The suicide gene may be an inducible caspase such as caspase 9 which induces apoptosis, when activated by an extracellular stimulus of a biocircuit system. Such induced apoptosis eliminates transferred cell as required to decrease the risk of direct toxicity and uncontrolled cell proliferation.

In some instances, immune cells of the disclosure may be NK cells modified to express an antigen-specific T cell receptor (TCR), or an antigen specific chimeric antigen receptor (CAR) taught herein.

Natural killer (NK) cells are members of the innate lymphoid cell family and characterized in humans by expression of the phenotypic marker CD56 (neural cell adhesion molecule) in the absence of CD3 (T-cell co-receptor). NK cells are potent effector cells of the innate immune system which mediate cytotoxic attack without the requirement of prior antigen priming, forming the first line of defense against diseases including cancer malignancies and viral infection.

Several pre-clinical and clinical trials have demonstrated that adoptive transfer of NK cells is a promising treatment approach against cancers such as acute myeloid leukemia (Ruggeri et al., Science; 2002, 295: 2097-2100; and Geller et al., Immunotherapy, 2011, 3: 1445-1459). Adoptive transfer of NK cells expressing CAR such as DAP12-Based Activating CAR revealed improved eradication of tumor cells (Topfer et al., J Immunol. 2015; 194:3201-3212). NK cell engineered to express a CS-1 specific CAR also displayed enhanced cytolysis and interferon-γ (IFN γ) production in multiple myeloma (Chu et al., Leukemia, 2014, 28(4): 917-927).

NK cell activation is characterized by an array of receptors with activating and inhibitory functions. The important activation receptors on NK cells include CD94/NKG2C and NKG2D (the C-type lectin-like receptors), and the natural cytotoxicity receptors (NCR) NKp30, NKp44 and NKp46, which recognize ligands on tumor cells or virally infected cells. NK cell inhibition is essentially mediated by interactions of the polymorphic inhibitory killer cell immunoglobulin-like receptors (KIRs) with their cognate human-leukocyte-antigen (HLA) ligands via the alpha-1 helix of the HLA molecule. The balance between signals that are generated from activating receptors and inhibitory receptors mainly determines the immediate cytotoxic activation.

NK cells may be isolated from peripheral blood mononuclear cells (PBMCs), or derived from human embryonic stem (ES) cells and induced pluripotent stem cells (iPSCs). The primary NK cells isolated from PBMCs may be further expanded for adoptive immunotherapy. Strategies and protocols useful for the expansion of NK cells may include interleukin 2 (IL2) stimulation and the use of autologous feeder cells, or the use of genetically modified allogeneic feeder cells. In some aspects, NK cells can be selectively expanded with a combination of stimulating ligands including IL15, IL21, IL2, 4-1BBL, IL12, IL18, MICA, 2B4, LFA-1, and BCM1/SLAMF2 (e.g., US patent publication NO: US20150190471).

Immune cells expressing effector modules comprising a CAR and/or other immunotherapeutic agents can be used as cancer immunotherapy. The immunotherapy comprises the cells expressing a CAR and/or other immunotherapeutic agents as an active ingredient, and may further comprise a suitable excipient. Examples of the excipient may include the pharmaceutically acceptable excipients, including various cell culture media, and isotonic sodium chloride.

In some instances, cells of the present disclosure may be dendritic cells that are genetically modified to express the compositions of the disclosure. Such cells may be used as cancer vaccines.

### III. PHARMACEUTICAL COMPOSITIONS AND FORMULATIONS

The present disclosure further provides pharmaceutical compositions comprising one or more biocircuits, effector modules, SREs (e.g., DDs), stimuli and payloads of interest (i.e., immunotherapeutic agents), vectors, cells and other components of the disclosure, and optionally at least one pharmaceutically acceptable excipient or inert ingredient.

As used herein the term "pharmaceutical composition" refers to a preparation of biocircuits, SREs, stimuli and payloads of interest (i.e., immunotherapeutic agents), other components, vectors, cells and described herein, or pharmaceutically acceptable salts thereof, optionally with other chemical components such as physiologically suitable carriers and excipients. The pharmaceutical compositions of the disclosure comprise an effective amount of one or more active compositions of the disclosure. The preparation of a pharmaceutical composition that contains at least one composition of the present disclosure and/or an additional active ingredient will be known to those skilled in the art considering the present disclosure, as exemplified by Remington's Pharmaceutical Sciences, 18th Ed. Mack Printing Company, 1990.

The term "excipient" or "inert ingredient" refers to an inactive substance added to a pharmaceutical composition and formulation to further facilitate administration of an active ingredient. For the purposes of the present disclosure, the phrase "active ingredient" generally refers to any one or more biocircuits, effector modules, SREs, stimuli and payloads of interest (i.e., immunotherapeutic agents), other components, vectors, and cells to be delivered as described herein. The phrases "pharmaceutically acceptable" refers to molecular entities and compositions that do not produce an adverse, allergic or other untoward reaction when administered to an animal, such as, for example, a human, as appropriate.

In some instances, pharmaceutical compositions and formulations are administered to humans, human patients or subjects. Although the descriptions of pharmaceutical compositions provided herein are principally directed to pharmaceutical compositions which are suitable for administration to humans, it will be understood by the skilled artisan that such compositions are generally suitable for administration to any other animal, e.g., to non-human animals, e.g. non-human mammals. Subjects to which administration of the pharmaceutical compositions is contemplated include, but are not limited to, non-human mammals, including agricultural animals such as cattle, horses, chickens and pigs, domestic animals such as cats, dogs, or research animals such as mice, rats, rabbits, dogs and non-human primates. It will be understood that, for human administration, preparations should meet sterility, pyrogenicity, general safety and purity standards as required by FDA Office of Biological Standards.

A pharmaceutical composition and formulation in accordance with the disclosure may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

The compositions of the present disclosure may be formulated in any manner suitable for delivery. The formulation may be, but is not limited to, nanoparticles, poly (lactic-co-glycolic acid) (PLGA) microspheres, lipidoids, lipoplex, liposome, polymers, carbohydrates (including simple sugars), cationic lipids and combinations thereof.

In one instance, the formulation is a nanoparticle which may comprise at least one lipid. The lipid may be selected from, but is not limited to, DLin-DMA, DLin-K-DMA, 98N12-5, C12-200, DLin-MC3-DMA, DLin-KC2-DMA, DODMA, PLGA, PEG, PEG-DMG and PEGylated lipids. In another aspect, the lipid may be a cationic lipid such as, but not limited to, DLin-DMA, DLin-D-DMA, DLin-MC3-DMA, DLin-KC2-DMA and DODMA.

For polynucleotides of the disclosure, the formulation may be selected from any of those taught, for example, in International Application PCT/US2012/069610.

Relative amounts of the active ingredient, the pharmaceutically acceptable excipient or inert ingredient, and/or any additional ingredients in a pharmaceutical composition in accordance with the disclosure will vary, depending upon the identity, size, and/or condition of the subject treated and further depending upon the route by which the composition is to be administered. By way of example, the composition may comprise between 0.1 and 100, e.g., between 0.5 and 50, between 1-30, between 5-80, at least 80 (w/w) active ingredient.

Efficacy of treatment or amelioration of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters. In connection with the administration of compositions of the present disclosure, "effective against" for example a cancer, indicates that administration in a clinically appropriate manner results in a beneficial effect for at least a statistically significant fraction of patients, such as an improvement of symptoms, a cure, a reduction in disease load, reduction in tumor mass or cell numbers, extension of life, improvement in quality of life, or other effect generally recognized as positive by medical doctors familiar with treating the particular type of cancer.

A treatment or preventive effect is evident when there is a statistically significant improvement in one or more parameters of disease status, or by a failure to worsen or to develop symptoms where they would otherwise be anticipated. As an example, a favorable change of at least 10 in a measurable parameter of disease, and preferably at least 20, 30, 40, 50 or more can be indicative of effective treatment. Efficacy for a given composition or formulation of the present disclosure can also be judged using an experimental animal model for the given disease as known in the art. When using an experimental animal model, efficacy of treatment is evidenced when a statistically significant change is observed.

### IV. DELIVERY MODALITIES AND/OR VECTORS

### Vectors

The present disclosure also provides vectors that package polynucleotides of the disclosure encoding biocircuits, effector modules, SREs (DDs) and payload constructs, and combinations thereof. In some instances, polynucleotides encoding destabilizing domains, effector modules and biocircuit systems, are provided. Vectors comprising polynucleotides of the disclosure are provided. In some aspects, the vector may be a non-viral vector, or a viral vector. In some instances, the vector of the disclosure is a viral vector. The viral vector may include, but is not limited to a retroviral vector, an adenoviral vector, an adeno-associated viral vector, or a lentiviral vector. In some instances, the vector of the disclosure may be a non-viral vector, such as a nanoparticles and liposomes.

Vectors of the present disclosure may also be used to deliver the packaged polynucleotides to a cell, a local tissue site or a subject. These vectors may be of any kind, including DNA vectors, RNA vectors, plasmids, viral vectors and particles. Viral vector technology is well known and described in Sambrook et al. (2001, Molecular Cloning: A Laboratory Manual, Cold Spring Harbor Laboratory, New York). Viruses, which are useful as vectors include, but are not limited to lentiviral vectors, adenoviral vectors, adeno-associated viral (AAV) vectors, herpes simplex viral vectors, retroviral vectors, oncolytic viruses, and the like.

In general, vectors contain an origin of replication functional in at least one organism, a promoter sequence and convenient restriction endonuclease site, and one or more selectable markers e.g. a drug resistance gene.

As used herein a promoter is defined as a DNA sequence recognized by transcription machinery of the cell, required to initiate specific transcription of the polynucleotide sequence of the present disclosure. Vectors can comprise native or non-native promoters operably linked to the polynucleotides of the disclosure. The promoters selected may be strong, weak, constitutive, inducible, tissue specific, development stage-specific, and/or organism specific. One example of a suitable promoter is the immediate early cytomegalovirus (CMV) promoter sequence. This promoter sequence is a strong constitutive promoter sequence capable of driving high levels of expression of polynucleotide sequence that is operatively linked to it. Another example of a preferred promoter is Elongation Growth Factor-1. Alpha (EF-1. alpha). Other constitutive promoters may also be used, including, but not limited to simian virus 40 (SV40), mouse mammary tumor virus (MMTV), human immunodeficiency virus (HIV), long terminal repeat (LTR), promoter, an avian leukemia virus promoter, an Epstein-Barr virus immediate early promoter, a Rous sarcoma virus promoter as well as human gene promoters including, but not limited to the phosphoglycerate kinase (PGK) promoter, actin promoter, the myosin promoter, the hemoglobin promoter, the Ubiquitin C (Ubc) promoter, the human U6 small nuclear protein promoter and the creatine kinase promoter. In some instances, inducible promoters such as but not limited to metallothionine promoter, glucocorticoid promoter, a progesterone promoter, and a tetracycline promoter may be used. In some instances, the promoter may be selected from the following a CMV promoter, comprising a nucleotide sequence of SEQ ID NO: 335, an EFla promoter, comprising a nucleotide sequence of SEQ ID NOs. 336-337, and a PGK promoter, comprising a nucleotide sequence of SEQ ID NO: 338.

In some instances, the optimal promoter may be selected based on its ability to achieve minimal expression of the SREs and payloads of the disclosure in the absence of the ligand and detectable expression in the presence of the ligand.

Additional promoter elements e.g. enhancers may be used to regulate the frequency of transcriptional initiation. Such regions may be located 10-100 base pairs upstream or downstream of the start site. In some instances, two or more promoter elements may be used to cooperatively or independently activate transcription.

In some instances, the recombinant expression vector may comprise regulatory sequences, such as transcription and translation initiation and termination codons, which are specific to the type of host cell into which the vector is to be introduced.

### 1. Lentiviral vectors

In some instances, lentiviral vectors/particles may be used as vehicles and delivery modalities. Lentiviruses are subgroup of the *Retroviridae* family of viruses, named because reverse transcription of viral RNA genomes to DNA is required before integration into the host genome. As such, the most important features of lentiviral vehicles/particles are the integration of their genetic material into the genome of a target/host cell. Some examples of lentivirus include the Human Immunodeficiency Viruses: HIV-1 and HIV-2, the Simian Immunodeficiency Virus (SIV), feline immunodeficiency virus (FIV), bovine immunodeficiency virus (BIV), Jembrana Disease Virus (JDV), equine infectious anemia virus (EIAV), equine infectious anemia virus, visna-maedi and caprine arthritis encephalitis virus (CAEV).

Typically, lentiviral particles making up the gene delivery vehicle are replication defective on their own (also referred to as "self-inactivating"). Lentiviruses can infect both dividing and non-dividing cells by virtue of the entry mechanism through the intact host nuclear envelope (Naldini L et al., Curr. Opin. Biotechnol, 1998, 9: 457-463). Recombinant lentiviral vehicles/particles have been generated by multiply attenuating the HIV virulence genes, for example, the genes Env, Vif, Vpr, Vpu, Nef and Tat are deleted making the vector biologically safe. Correspondingly, lentiviral vehicles, for example, derived from HIV-1/HIV-2 can mediate the efficient delivery, integration and long-term expression of transgenes into non-dividing cells. As used herein, the term "recombinant" refers to a vector or other nucleic acid containing both lentiviral sequences and non-lentiviral retroviral sequences.

Lentiviral particles may be generated by co-expressing the virus packaging elements and the vector genome itself in a producer cell such as human HEK293T cells. These elements are usually provided in three (in second generation lentiviral systems) or four separate plasmids (in third generation lentiviral systems). The producer cells are co-transfected with plasmids that encode lentiviral components including the core (i.e. structural proteins) and enzymatic components of the virus, and the envelope protein(s) (referred to as the packaging systems), and a plasmid that encodes the genome including a foreign transgene, to be transferred to the target cell, the vehicle itself (also referred to as the transfer vector). In general, the plasmids or vectors are included in a producer cell line. The plasmids/vectors are introduced via transfection, transduction or infection into the producer cell line. Methods for transfection, transduction or infection are well known by those of skill in the art. As non-limiting example, the packaging and transfer constructs can be introduced into producer cell lines by calcium phosphate transfection, lipofection or electroporation, generally together with a dominant selectable marker, such as neo, DHFR, Gln synthetase or ADA, followed by selection in the presence of the appropriate drug and isolation of clones.

The producer cell produces recombinant viral particles that contain the foreign gene, for example, the effector module of the present disclosure. The recombinant viral particles are recovered from the culture media and titrated by standard methods used by those of skill in the art. The recombinant lentiviral vehicles can be used to infect target cells.

Cells that can be used to produce high-titer lentiviral particles may include, but are not limited to, HEK293T cells, 293G cells, STAR cells (Relander et al., Mol. Ther., 2005, 11: 452-459), FreeStyle^{™} 293 Expression System (ThermoFisher, Waltham, MA), and other HEK293T-based producer cell lines (e.g., Stewart et al., Hum Gene Ther._2011, 22(3):357-369; Lee et al., Biotechnol Bioeng, 2012, 10996): 1551-1560; Throm et al., Blood. 2009, 113(21): 5104-5110).

In some aspects, the envelope proteins may be heterologous envelop proteins from other viruses, such as the G protein of vesicular stomatitis virus (VSV G) or baculoviral gp64 envelop proteins. The VSV-G glycoprotein may especially be chosen among species classified in the vesiculovirus genus: *Carajas virus* (CJSV), *Chandipura virus* (CHPV), *Cocal virus* (COCV), *Isfahan virus* (ISFV), *Maraba virus* (MARAV), *Piry virus* (PIRYV), *Vesicular stomatitis Alagoas virus* (VSAV), *Vesicular stomatitis Indiana virus* (VSIV) *and Vesicular stomatitis New Jersey virus* (VSNJV) and/or stains provisionally classified in the vesiculovirus genus as *Grass carp rhabdovirus, BeAn 157575 virus* (BeAn 157575), *Boteke virus* (BTKV), *Calchaqui virus* (CQIV), *Eel virus American* (EVA), *Gray Lodge virus* (GLOV), *Jurona virus* (JURY), *Klamath virus* (KLAV), *Kwatta virus* (KW A V), *La Joya virus* (LJV), *Malpais Spring virus* (MSPV), *Mount Elgon bat virus* (MEBV), *Perinet virus* (PERV), *Pike fry rhabdovirus* (PFRV), *Porton virus* (PORV), *Radi virus* (RADIV), *Spring viremia of carp virus* (SVCV), *Tupaia virus* (TUPV), *Ulcerative disease rhabdovirus* (UDRV) and *Yug Bogdanovac virus* (YBV). The gp64 or other baculoviral env protein can be derived from *Autographa californica* nucleopolyhedrovirus (AcMNPV), *Anagrapha falcifera* nuclear polyhedrosis virus, *Bombyx mori* nuclear polyhedrosis virus, *Choristoneurafumiferana* nucleopolyhedrovirus, *Orgyia pseudotsugata* single capsid nuclear polyhedrosis virus, *Epiphyas postvittana* nucleopolyhedrovirus, *Hyphantria cunea* nucleopolyhedrovirus, *Galleria mellonella* nuclear polyhedrosis virus, Dhori virus, Thogoto virus, *Antheraea pemyi* nucleopolyhedrovirus or Batken virus.

Additional elements provided in lentiviral particles may comprise retroviral LTR (long-terminal repeat) at either 5' or 3' terminus, a retroviral export element, optionally a lentiviral reverse response element (RRE), a promoter or active portion thereof, and a locus control region (LCR) or active portion thereof. Other elements include central polypurine tract (cPPT) sequence to improve transduction efficiency in non-dividing cells, Woodchuck Hepatitis Virus (WHP) Posttranscriptional Regulatory Element (WPRE) which enhances the expression of the transgene, and increases titer. The effector module is linked to the vector.

Methods for generating recombinant lentiviral particles are discussed in the art, for example, U.S. Pat. NOs.: 8, 846, 385; 7,745, 179; 7,629,153; 7,575,924; 7,179, 903; and 6, 808, 905.

Lentivirus vectors used may be selected from, but are not limited to pLVX, pLenti, pLenti6, pLJM1, FUGW, pWPXL, pWPI, pLenti CMV puro DEST, pLJM1-EGFP, pULTRA, pInducer20, pHIV-EGFP, pCW57.1, pTRPE, pELPS, pRRL, and pLionII.

Lentiviral vehicles known in the art may also be used (See, U.S. Pat. NOs. 9,260,725; 9,068,199; 9,023,646; 8,900,858; 8,748,169; 8,709,799; 8,420,104; 8,329,462; 8,076,106; 6,013,516; and 5,994,136; International Patent Publication NO.: WO2012079000).

### Lentiviral vectors and Cell Engineering

Lentiviral vectors are used for introducing transgenes into T cells (e.g., primary human T cells or Jurkat cells) for preclinical research and clinical applications, including recently approved products such as Tisagenlecleucel (KYMRIAH^{®}) for relapsed/refractory B-cell lymphoma. VSV-G pseudotyped 3rd generation lentiviral vectors offer high titers, high transduction efficiency and safety, and have become the vectors of choice for T cell engineering. While not wishing to be bound by theory, T cell engineering usually involves T cell activation by CD3/CD28 antibodies, followed by lentivirus transduction, and then cell expansion which can last from 5 to 30 days (e.g., 9 to 14 days or 9 to 15 days). In general, lentivirus transgene integration may take over 7 days to fully stabilize in T cells (e.g., primary human T cells or Jurkat cells). While longer cultures can increase the cell numbers, the longer cultures can also change the T cell phenotype to a more differentiated state. Therefore, the duration of *ex vivo* culture can impact the persistence and efficacy of CAR T cells. For example, cells cultured for shorter duration may display a less differentiated phenotype and can be highly efficacious in preclinical models.

While not wishing to be bound by theory, the state of T cell differentiation may influence the engraftment and persistence of T cells following adoptive transfer. Ghassemi et al (Reducing Ex Vivo Culture Improves the Antileukemic Activity of Chimeric Antigen Receptor (CAR) T Cells. Cancer Immunol Res; 6(9) Sept. 2018) describe primary human T cell differentiation over time and saw that early harvested CAR T cells exhibited enhanced effector function and proliferation, as well as enhanced potency and persistence *in vivo.*

Lentivirus dynamics such as transduction, integration and/or expression kinetics of lentivirally introduced transgenes in T cells (e.g., primary human T cells or Jurkat cells) *ex vivo* may impact the efficacy and durability of *in vivo* anti-tumor responses. Some type of T cells may produce different results. For example, the Jurkat cell line may not provide the dynamic range of expression as primary human T cells. Methods to evaluate these lentivirus dynamics are known in the art and are described herein.

In some instance, to determine the transgene expression kinetics CD3/CD28 activated primary human T cells can be transduced with lentivirus carrying a transgene (e.g., a regulated transgene or constitutive transgene such as CD19 CAR, IL12, fluorescent protein or any transgene (e.g., payload) described herein). The cells may be analyzed by methods described herein and/or known in the art for viability, viral genomic integration (e.g., by using quantitative PCR), transcript levels (e.g., by using quantitative RT-PCR), and cell surface expression of the transgene if applicable (e.g., if the transgene is or includes CD19 CAR then the surface expression of the CD19 CAR can be evaluated). The cells may be analyzed prior to transduction and/or after transduction such as 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 or more than 30 days after transduction. As a non-limiting example, the cells may be analyzed at various time points between 3 to 14 days after transduction (e.g., 3 days, 4 days, 5 days, 6 days, 7 days, 8 days, 9 days, 10 days, 11 days, 12 days, 13 days, and/or 14 days). As a non-limiting example, the cells may be analyzed 3 to 15 days after transduction. As a non-limiting example, the cells may be analyzed 9 to 15 days after transduction.

In some instances, the CD3/CD28 activated primary human T cells can be reactivated with CD3/CD28 beads after transduction. The cells may be reactivated 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 or more than 30 days after transduction. The cells may be analyzed by methods described herein and/or known in the art for viability, viral genomic integration (e.g., by using quantitative PCR), transcript levels (e.g., by using quantitative RT-PCR), cell surface expression of the transgene if applicable (e.g., if the transgene is or includes CD19 CAR then the surface expression of the CD19 CAR can be evaluated), copy number, and/or mRNA levels.

In some instances, the cell viability of activated primary human T cells transduced with lentivirus carrying a transgene is greater than 65%, 70%, 75%, 80%, 85%, 90%, 95%, 97%, or 99%. As a non-limiting example, the cell viability is greater than 90%. As a non-limiting example, the cell viability is greater than 85%.

In some instances, the cell viability of Jurkat cells transduced with lentivirus carrying a transgene is greater than 65%, 70%, 75%, 80%, 85%, 90%, 95%, 97%, or 99%. As a non-limiting example, the cell viability is greater than 90%. As a non-limiting example, the cell viability is greater than 85%.

In some instances, the integration of the transgene into the genome of the cell may be at or above the saturation point. As a non-limiting example, the saturation point may be 3 copies per cell.

In some instances, the integration of the transgene into the genome may be high in the initial timepoints evaluated and then decline to a lower integration value before becoming stable for the remainder of the culture. As a non-limiting example, the integration may be up to 20 copies per cell of the transgene into the genome during the early timepoints before declining to 2 copies per cell and being stable throughout the remainder of the culture.

In some instances, the transduction of ability of T cells may be evaluated. T cells from at least one donor may be transduced with a lentivirus containing a transgene at a dose that is predicted to reach the saturating levels (e.g., enough virus that each cell should contain a copy if a Poisson distribution is expected) and a higher lentivirus dose that exceeds saturation 5 times. Copies per cell, percentage and MFI of cells (or concentration in media of transgene) may be detected in order to determine if all cells are expressing transgene. As a non-limiting example, T cells from two distinct donors may be transduced with lentivirus which includes a transgene. The transduction may be at two doses, saturation and 5x saturation, and show that 5-10 days after transduction that all groups may reach or exceed a predicted saturating level of integrated transgene and similar expression intensity across groups but not all cells are expressing the transgene. Not all T cells may have equal transduction susceptibility, even when sourced from the same donor. The fraction of total cells that express GFP (above the detection threshold) may vary between donors, lots and/or viral dose. The percent of total cells that express GFP from a single donor may be between 70% and 95%, such as, but not limited to, 70%, 70.1%, 70.2%, 70.3%, 70.4%, 70.5%, 70.6%, 70.7%, 70.8%, 70.9%, 71%, 71.1%, 71.2%, 71.3%, 71.4%, 71.5%, 71.6%, 71.7%, 71.8%, 71.9%, 72%, 72.1%, 72.2%, 72.3%, 72.4%, 72.5%, 72.6%, 72.7%, 72.8%, 72.9%, 73%, 73.1%, 73.2%, 73.3%, 73.4%, 73.5%, 73.6%, 73.7%, 73.8%, 73.9%, 74%, 74.1%, 74.2%, 74.3%, 74.4%, 74.5%, 74.6%, 74.7%, 74.8%, 74.9%, 75%, 75.1%, 75.2%, 75.3%, 75.4%, 75.5%, 75.6%, 75.7%, 75.8%, 75.9%, 76%, 76.1%, 76.2%, 76.3%, 76.4%, 76.5%, 76.6%, 76.7%, 76.8%, 76.9%, 77%, 77.1%, 77.2%, 77.3%, 77.4%, 77.5%, 77.6%, 77.7%, 77.8%, 77.9%, 78%, 78.1%, 78.2%, 78.3%, 78.4%, 78.5%, 78.6%, 78.7%, 78.8%, 78.9%, 79%, 79.1%, 79.2%, 79.3%, 79.4%, 79.5%, 79.6%, 79.7%, 79.8%, 79.9%, 80%, 80.1%, 80.2%, 80.3%, 80.4%, 80.5%, 80.6%, 80.7%, 80.8%, 80.9%, 81%, 81.1%, 81.2%, 81.3%, 81.4%, 81.5%, 81.6%, 81.7%, 81.8%, 81.9%, 82%, 82.1%, 82.2%, 82.3%, 82.4%, 82.5%, 82.6%, 82.7%, 82.8%, 82.9%, 83%, 83.1%, 83.2%, 83.3%, 83.4%, 83.5%, 83.6%, 83.7%, 83.8%, 83.9%, 84%, 84.1%, 84.2%, 84.3%, 84.4%, 84.5%, 84.6%, 84.7%, 84.8%, 84.9%, 85%, 85.1%, 85.2%, 85.3%, 85.4%, 85.5%, 85.6%, 85.7%, 85.8%, 85.9%, 86%, 86.1%, 86.2%, 86.3%, 86.4%, 86.5%, 86.6%, 86.7%, 86.8%, 86.9%, 87%, 87.1%, 87.2%, 87.3%, 87.4%, 87.5%, 87.6%, 87.7%, 87.8%, 87.9%, 88%, 88.1%, 88.2%, 88.3%, 88.4%, 88.5%, 88.6%, 88.7%, 88.8%, 88.9%, 89%, 89.1%, 89.2%, 89.3%, 89.4%, 89.5%, 89.6%, 89.7%, 89.8%, 89.9%, 90%, 90.1%, 90.2%, 90.3%, 90.4%, 90.5%, 90.6%, 90.7%, 90.8%, 90.9%, 91%, 91.1%, 91.2%, 91.3%, 91.4%, 91.5%, 91.6%, 91.7%, 91.8%, 91.9%, 92%, 92.1%, 92.2%, 92.3%, 92.4%, 92.5%, 92.6%, 92.7%, 92.8%, 92.9%, 93%, 93.1%, 93.2%, 93.3%, 93.4%, 93.5%, 93.6%, 93.7%, 93.8%, 93.9%, 94%, 94.1%, 94.2%, 94.3%, 94.4%, 94.5%, 94.6%, 94.7%, 94.8%, 94.9%, or 95%. As a non-limiting example, the percent of total cells expressing GFP in cells from one donor may be 83.8% for a dose of 1 uL and 83.7% or 78.8% for a dose of 5 uL. As another non-limiting example, the percent of total cells expressing GFP in cells from one donor may be 80.6%, 89.1%, or 91.2% for a dose of 1 uL and 75.1%, 89.6% or 91.7% for a dose of 5 uL.

In some instances, a percentage of the cultured T cells (e.g., primary human T cells and/or Jurkat cells) may express the transgene. The percentage of culture T cells expressing the transgene may be, but is not limited to, 5%, 10%, 15%, 20%, 25%, 30%, 35%, 40%, 45%, 50%, 55%, 60%, 65%, 70%, 75%, 80%, 85%, 90%, 95%, 97%, 99% or greater than 99%. As a non-limiting example, the percentage may be greater than 70%. As a non-limiting example, the percentage may be greater than 75%. As a non-limiting example, the percentage may be greater than 80%. As a non-limiting example, the percentage may be greater than 85%. As a non-limiting example, the percentage may be greater than 90%. As a non-limiting example, the percentage may be greater than 95%.

In some instances, the mRNA levels from the culture may decline over the duration of the study. The decline may not be limited to a specific transgene and the trend may be seen across multiple classes of expressed proteins. In order to increase the mRNA levels, the cells may be reactivated after the mRNA levels decrease from the initial levels. The cells may be reactivated 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 or more than 30 days after transduction. As a non-limiting example, in order to increase mRNA levels in the culture, the cells may be reactivated with CD3/CD28 beads 13 days after transduction. As a non-limiting example, in order to increase mRNA levels in the culture, the cells may be reactivated with CD3/CD28 beads 14 days after transduction. As a non-limiting example, in order to increase mRNA levels in the culture, the cells may be reactivated with CD3/CD28 beads 15 days after transduction

In some instances, the surface expression from the culture may decline over the duration of the study. For example, the surface expression may decline between days 3 to 13 days, 3 to 14 days, or 3 to 15 days after transduction. In order to increase the surface expression, the cells may be reactivated after the surface expression decrease from the initial levels. The cells may be reactivated 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 or more than 30 days after transduction. As a non-limiting example, in order to increase surface expression in the culture, the cells may be reactivated with CD3/CD28 beads 13 days after transduction. As a non-limiting example, in order to increase surface expression in the culture, the cells may be reactivated with CD3/CD28 beads 14 days after transduction. As a non-limiting example, in order to increase surface expression in the culture, the cells may be reactivated with CD3/CD28 beads 15 days after transduction.

In some instances, the transgene is a CAR such as, but not limited to, CD19 CAR. As a non-limiting example, the CAR is CD 19 CAR. The cell viability may be greater than 90% in cells transduced with a CD 19 CAR. The cell viability may be greater than 85% in cells transduced with a CD 19 CAR. If the cells are primary T cells transduced with a CD 19 CAR, then number of viable cells may increase over the initial timepoints before decreasing. If the cells are Jurkat cells transduced with a CD 19 CAR, then the number of viable cells may increase for at least 10 days. The number of copies per cell for CD19 CAR transduced cells may be higher for the initial timepoints before decreasing by 50% or more for the later timepoints. The cell surface expression of CD19 CAR may decrease during the course of the study from about 20000 CAR MFI to less than 5000 CAR MFI over a period of 10 days (e.g., day 3 to day 13). After restimulation on day 15 the MFI may increase to above 5000 CAR MFI. The percentage of primary human T cells expressing CAR may be between 40% and 60% for 3-13 days after transduction. The percentage of Jurkat cells expressing CAR may be between 30% and 70% for 3-13 days after transduction. An initial decline of about 20% may be seen between days 3 and 6 after transduction. Restimulation of the T cells may increase the percent of CAR positive cells back to initial percentage levels (e.g., around 60%).

In some instances, the transgene is an interleukin such as, but not limited to, IL12 (e.g., membrane bound IL12 or secreted cytokine IL12), IL15 (e.g., membrane bound IL15), and IL15Ra. As a non-limiting example, the interleukin is IL12. The cell viability may be greater than 90% in cells transduced with IL12. The cell viability may be greater than 85% in cells transduced with IL12. If the cells are primary T cells transduced with IL12, the number of viable cells may increase over the initial timepoints before decreasing. If the cells are Jurkat cells transduced with IL 12, the number of viable cells may increase for at least 10 days. The number of copies per cell for IL12 transduced cells may be higher for the initial timepoints before decreasing by 50% or more for the later timepoints. For IL12 transduced primary human T cells, the level of soluble IL12 in the media may drop steadily over the time course of the study with a slight increase visible in the restimulated group. For IL12 transduced Jurkat cells, the level of soluble IL12 in the media may have a drop in IL12 secretion in the first half of the culture with the levels remaining low through the second half of the culture time.

In some instances, the transgene encodes a fluorescent protein such as, but not limited to cytosolic green fluorescence protein (GFP), luciferase, and mCherry. As a non-limiting example, the fluorescent protein is GFP. The cell viability may be greater than 90% in cells transduced with GFP. The cell viability may be greater than 85% in cells transduced with GFP. If the cells are primary T cells transduced with GFP, then the number of viable cells may increase over the initial timepoints before decreasing. If the cells are Jurkat cells transduced with GFP, then the number of viable cells may increase for at least 10 days. The number of copies per cell for GFP transduced cells may be higher for the initial timepoints before decreasing by 50% or more for the later timepoints. The surface expression of the cells may have a steady and rapid decline bottoming out at day 10 with a slight increase if restimulated. The highest level of cell surface expression of GFP in Jurkat cells may be at day 10 (about 35000 GFP MFI) before decreasing for the rest of the study. The percentage of primary human T cells expressing GFP may be around 80% for 3-13 days after transduction. The percentage of Jurkat cells expressing GFP may be around 90% for 3-13 days after transduction.

In some instances, lentivirally engineered cells described herein have genomic DNA integration that stabilizes after an initial decline of copy number, decreasing RNA and surface expression levels over time, and an increase in RNA and surface expression after re-stimulation.

In some instances, lentivirally engineering cells may be evaluated using the following 14-day method where samples are collected 5 times throughout the culture. On day -1 the T cells (e.g., primary human T cells or Jurkat cells) may be thawed and the CD3/CD28 beads are added. On day 0, the lentivirus for each of the conditions is added (e.g., 4 mL of cells at 0.5e6/mL) and there is a control of non-transduced cells. Double media to 8 mL on day 1 and then double the media to 16 mL on day 2. On day 3, harvest 4 mL and then double media to 24 mL on day 4. Harvest 4 mL on day 6 before doubling media to 40 mL. The cells can be split (e.g., 14 mL 0.5e6 cells/mL) on day 8 and then on day 6 harvest 4 mL before doubling media to 40 mL. 4mL may be harvested on day 10 before the media is doubled to 20 mL. On day 13, 4 mL are harvested before doubling the media to 32 mL. The culture is split in half and half of the culture is activated (CD3/CD28 activation beads 1:1) and stimulated overnight. On day 14, 4 mL of each stimulated and non-stimulated cells are harvested and the culture is ended. Transgene copy number per cell are assayed by harvesting cells and extracting genomic DNA then quantifying with standard curve qPCR against the endogenous genome and against the transgene sequence, then converting the detected quantities to a ratio. Mean Fluorescence Intensity (MFI) is assayed by FLO on an Attune with appropriate staining for each group. Percent expressing may also be assayed by FLO on an attune quantifying the percent of cells fluorescing above threshold. Soluble payloads can be quantified by harvesting culture supernatant at each marked timepoint and running MesoScale Discovery plate assay (MSD) then normalizing for cell density.

### 2. Retroviral vectors (γ-retroviral vectors)

In some instances, retroviral vectors may be used to package and deliver the biocircuits, biocircuit components, effector modules, SREs or payload constructs of the present disclosure. Retroviral vectors (RVs) allow the permanent integration of a transgene in target cells. In addition to lentiviral vectors based on complex HIV-1/2, retroviral vectors based on simple gamma-retroviruses have been widely used to deliver therapeutic genes and demonstrated clinically as one of the most efficient and powerful gene delivery systems capable of transducing a broad range of cell types. Example species of Gamma retroviruses include the murine leukemia viruses (MLVs) and the feline leukemia viruses (FeLV).

In some instances, gamma-retroviral vectors derived from a mammalian gamma-retrovirus such as murine leukemia viruses (MLVs), are recombinant. The MLV families of gamma retroviruses include the ecotropic, amphotropic, xenotropic and polytropic subfamilies. Ecotropic viruses are able to infect only murine cells using mCAT-1 receptor. Examples of ecotropic viruses are Moloney MLV and AKV. Amphotropic viruses infect murine, human and other species through the Pit-2 receptor. One example of an amphotropic virus is the 4070A virus. Xenotropic and polytropic viruses utilize the same (Xpr1) receptor, but differ in their species tropism. Xenotropic viruses such as NZB-9-1 infect human and other species but not murine species, whereas polytropic viruses such as focus-forming viruses (MCF) infect murine, human and other species.

Gamma-retroviral vectors may be produced in packaging cells by co-transfecting the cells with several plasmids including one encoding the retroviral structural and enzymatic (gag-pol) polyprotein, one encoding the envelope (env) protein, and one encoding the vector mRNA comprising polynucleotide encoding the compositions of the present disclosure that is to be packaged in newly formed viral particles.

In some aspects, the recombinant gamma-retroviral vectors are pseudotyped with envelope proteins from other viruses. Envelope glycoproteins are incorporated in the outer lipid layer of the viral particles which can increase/alter the cell tropism. Exemplary envelop proteins include the gibbon ape leukemia virus envelope protein (GALV) or vesicular stomatitis virus G protein (VSV-G), or Simian endogenous retrovirus envelop protein, or Measles Virus H and F proteins, or Human immunodeficiency virus gp120 envelope protein, or cocal vesiculovirus envelop protein (See, e.g., U.S. application publication NO.: 2012/164118). In other aspects, envelope glycoproteins may be genetically modified to incorporate targeting/binding ligands into gamma-retroviral vectors, binding ligands including, but not limited to, peptide ligands, single chain antibodies and growth factors (Waehler et al., Nat. Rev. Genet. 2007, 8(8):573-587). These engineered glycoproteins can retarget vectors to cells expressing their corresponding target moieties. In other aspects, a "molecular bridge" may be introduced to direct vectors to specific cells. The molecular bridge has dual specificities: one end can recognize viral glycoproteins, and the other end can bind to the molecular determinant on the target cell. Such molecular bridges, for example ligand-receptor, avidin-biotin, and chemical conjugations, monoclonal antibodies and engineered fusogenic proteins, can direct the attachment of viral vectors to target cells for transduction (Yang et al., Biotechnol. Bioeng., 2008, 101(2): 357-368; and Maetzig et al., Viruses, 2011, 3, 677-713).

In some instances, the recombinant gamma-retroviral vectors are self-inactivating (SIN) gammaretroviral vectors. The vectors are replication incompetent. SIN vectors may harbor a deletion within the 3' U3 region initially comprising enhancer/promoter activity. Furthermore, the 5' U3 region may be replaced with strong promoters (needed in the packaging cell line) derived from Cytomegalovirus or RSV, or an internal promoter of choice, and/or an enhancer element. The choice of the internal promoters may be made according to specific requirements of gene expression needed for a particular purpose of the disclosure.

In some instances, polynucleotides encoding the biocircuit, biocircuit components, effector module, SRE are inserted within the recombinant viral genome. The other components of the viral mRNA of a recombinant gamma-retroviral vector may be modified by insertion or removal of naturally occurring sequences (e.g., insertion of an IRES, insertion of a heterologous polynucleotide encoding a polypeptide or inhibitory nucleic acid of interest, shuffling of a more effective promoter from a different retrovirus or virus in place of the wild-type promoter and the like). In some examples, the recombinant gamma-retroviral vectors may comprise modified packaging signal, and/or primer binding site (PBS), and/or 5'-enhancer/promoter elements in the U3-region of the 5'- long terminal repeat (LTR), and/or 3'-SIN elements modified in the U3-region of the 3'-LTR. These modifications may increase the titers and the ability of infection.

Gamma retroviral vectors suitable for delivering biocircuit components, effector modules, SREs or payload constructs of the present disclosure may be selected from those disclosed in U.S. Pat. NOs.: 8,828,718; 7,585,676; 7,351,585; U.S. application publication NO.: 2007/048285; PCT application publication NOs.: WO2010/113037; WO2014/121005; WO2015/056014; and EP Pat. NOs.: EP1757702; EP1757703.

### 3. Adeno-associated viral vectors (AAV)

In some instances, polynucleotides of present disclosure may be packaged into recombinant adeno-associated viral (rAAV) vectors. Such vectors or viral particles may be designed to utilize any of the known serotype capsids or combinations of serotype capsids. The serotype capsids may include capsids from any identified AAV serotypes and variants thereof, for example, AAV1, AAV2, AAV2G9, AAV3, AAV4, AAV4-4, AAV5, AAV6, AAV7, AAV8, AAV9, AAV10, AAV11, AAV12 and AAVrh10.

In one instance, the AAV serotype may be or have a sequence as described in United States Publication No. US20030138772, such as, but not limited to, AAV1 (SEQ ID NO: 6 and 64 of US20030138772), AAV2 (SEQ ID NO: 7 and 70 of US20030138772), AAV3 (SEQ ID NO: 8 and 71 of US20030138772), AAV4 (SEQ ID NO: 63 of US20030138772), AAV5 (SEQ ID NO: 114 of US20030138772), AAV6 (SEQ ID NO: 65 of US20030138772), AAV7 (SEQ ID NO: 1-3 of US20030138772), AAV8 (SEQ ID NO: 4 and 95 of US20030138772), AAV9 (SEQ ID NO: 5 and 100 of US20030138772), AAV10 (SEQ ID NO: 117 of US20030138772), AAV11 (SEQ ID NO: 118 of US20030138772), AAV12 (SEQ ID NO: 119 of US20030138772), AAVrh10 (amino acids 1 to 738 of SEQ ID NO: 81 of US20030138772) or variants thereof. Non-limiting examples of variants include SEQ ID NOs: 9, 27-45, 47-62, 66-69, 73-81, 84-94, 96, 97, 99, 101-113 of US20030138772.

In one instance, the AAV serotype may have a sequence as described in Pulicherla et al. (Molecular Therapy, 2011,19(6):1070-1078), U.S. Pat. NOs.: 6,156,303; 7,198,951; U.S. Patent Publication NOs.: US2015/0159173 and US2014/0359799; and International Patent Publication NOs.: WO1998/011244, WO2005/033321 and WO2014/14422.

AAV vectors include not only single stranded vectors but self-complementary AAV vectors (scAAVs). scAAV vectors contain DNA which anneals together to form double stranded vector genome. By skipping second strand synthesis, scAAVs allow for rapid expression in the cell.

The rAAV vectors may be manufactured by standard methods in the art such as by triple transfection, in sf9 insect cells or in suspension cell cultures of human cells such as HEK293 cells.

The biocircuits, biocircuit components, effector modules, SREs or payload constructs may be encoded in one or more viral genomes to be packaged in the AAV capsids taught herein.

Such vectors or viral genomes may also include, in addition to at least one or two ITRs (inverted terminal repeats), certain regulatory elements necessary for expression from the vector or viral genome. Such regulatory elements are well known in the art and include for example promoters, introns, spacers, stuffer sequences, and the like.

In some instances, more than one effector module or SRE (e.g. DD) may be encoded in a viral genome.

### 4. Oncolytic viral vector

In some instances, polynucleotides of present disclosure may be packaged into oncolytic viruses, such as vaccine viruses. Oncolytic vaccine viruses may include viral particles of a thymidine kinase (TK)-deficient, granulocyte macrophage (GM)-colony stimulating factor (CSF)-expressing, replication-competent vaccinia virus vector sufficient to induce oncolysis of cells in the tumor (e.g., US Pat. NO.: 9,226,977).

In some instances, the viral vector of the disclosure may comprise two or more immunotherapeutic agents taught herein, wherein the two or more immunotherapeutic agents may be included in one effector module under the regulation of the same DD. In this case, the two or more immunotherapeutic agents are tuned by the same stimulus simultaneously. In other instances, the viral vector of the disclosure may comprise two or more effector modules, wherein each effector module comprises a different immunotherapeutic agent. In this case, the two or more effector modules and immunotherapeutic agents are tuned by different stimuli, providing separately independent regulation of the two or more components.

### 5. Messenger RNA (mRNA)

In some instances, the effector modules of the disclosure may be designed as a messenger RNA (mRNA). As used herein, the term "messenger RNA" (mRNA) refers to any polynucleotide which encodes a polypeptide of interest and which is capable of being translated to produce the encoded polypeptide of interest *in vitro, in vivo, in situ* or *ex vivo.* Such mRNA molecules may have the structural components or features of any of those taught in International Application number PCT/US2013/030062.

Polynucleotides of the disclosure may also be designed as taught in, for example, Ribostem Limited in United Kingdom patent application serial number 0316089.2 filed on July 9, 2003 now abandoned, PCT application number PCT/GB2004/002981 filed on July 9, 2004 published as WO2005005622, United States patent application national phase entry serial number 10/563,897 filed on June 8, 2006 published as US20060247195 now abandoned, and European patent application national phase entry serial number EP2004743322 filed on July 9, 2004 published as EP 1646714 now withdrawn; Novozymes, Inc. in PCT application number PCT/US2007/88060 filed on December 19, 2007 published as WO2008140615, United States patent application national phase entry serial number 12/520,072 filed on July 2, 2009 published as US20100028943 and European patent application national phase entry serial number EP2007874376 filed on July 7, 2009 published as EP2104739; University of Rochester in PCT application number PCT/US2006/46120 filed on December 4, 2006 published as WO2007064952 and United States patent application serial number 11/606,995 filed on December 1, 2006 published as US20070141030; BioNTech AG in European patent application serial number EP2007024312 filed December 14, 2007 now abandoned, PCT application number PCT/EP2008/01059 filed on December 12, 2008 published as WO2009077134, European patent application national phase entry serial number EP2008861423 filed on June 2, 2010 published as EP2240572, United States patent application national phase entry serial number 12/,735,060 filed November 24,2010 published as US20110065103, German patent application serial number DE 10 2005 046 490 filed September 28, 2005, PCT application PCT/EP2006/0448 filed September 28, 2006 published as WO2007036366, national phase European patent EP1934345 published March, 21, 2012 and national phase US patent application serial number 11/992,638 filed August 14, 2009 published as 20100129877; Immune Disease Institute Inc. in United States patent application serial number 13/088,009 filed April 15, 2011 published as US20120046346 and PCT application PCT/US2011/32679 filed April 15, 2011 published as WO20110130624; Shire Human Genetic Therapeutics in United States patent application serial number 12/957,340 filed on November 20, 2010 published as US20110244026; Sequitur Inc. in PCT application PCT/US1998/019492 filed on September 18, 1998 published as WO1999014346; The Scripps Research Institute in PCT application number PCT/US2010/00567 filed on February 24, 2010 published as WO2010098861, and United States patent application national phase entry serial number 13/203,229 filed November 3, 2011 published as US20120053333; Ludwig-Maximillians University in PCT application number PCT/EP2010/004681 filed on July 30, 2010 published as WO2011012316; Cellscript Inc. in United States patent number 8,039,214 filed June 30, 2008 and granted October 18, 2011, United States patent application serial numbers 12/962,498 filed on December 7, 2010 published as US20110143436, 12/962,468 filed on December 7, 2010 published as US20110143397, 13/237,451 filed on September 20, 2011 published as US20120009649, and PCT applications PCT/US2010/59305 filed December 7, 2010 published as WO2011071931 and PCT/US2010/59317 filed on December 7, 2010 published as WO2011071936; The Trustees of the University of Pennsylvania in PCT application number PCT/US2006/32372 filed on August 21, 2006 published as WO2007024708, and United States patent application national phase entry serial number 11/990,646 filed on March 27, 2009 published as US20090286852; Curevac GMBH in German patent application serial numbers DE10 2001 027 283.9 filed June 5, 2001, DE10 2001062 480.8 filed December 19, 2001, and DE 20 2006 051 516 filed October 31, 2006 all abandoned, European patent numbers EP1392341 granted March 30, 2005 and EP1458410 granted January 2, 2008, PCT application numbers PCT/EP2002/06180 filed June 5, 2002 published as WO2002098443, PCT/EP2002/14577 filed on December 19, 2002 published as WO2003051401, PCT/EP2007/09469 filed on December 31, 2007 published as WO2008052770, PCT/EP2008/03033 filed on April 16, 2008 published as WO2009127230, PCT/EP2006/004784 filed on May 19, 2005 published as WO2006122828, PCT/EP2008/00081 filed on January 9, 2007 published as WO2008083949, and United States patent application serial numbers 10/729,830 filed on December 5, 2003 published as US20050032730, 10/870,110 filed on June 18, 2004 published as US20050059624, 11/914,945 filed on July 7, 2008 published as US20080267873, 12/446,912 filed on October 27, 2009 published as US2010047261 now abandoned, 12/522,214 filed on January 4, 2010 published as US20100189729, 12/787,566 filed on May 26, 2010 published as US20110077287, 12/787,755 filed on May 26, 2010 published as US20100239608, 13/185,119 filed on July 18, 2011 published as US20110269950, and 13/106,548 filed on May 12, 2011 published as US20110311472.

In some instances, the effector modules may be designed as self-amplifying RNA. "Self-amplifying RNA" as used herein refers to RNA molecules that can replicate in the host resulting in the increase in the amount of the RNA and the protein encoded by the RNA. Such self-amplifying RNA may have structural features or components of any of those taught in International Patent Application Publication No. WO2011005799.

### V. DOSING, DELIVERY AND ADMINISTRATIONS

The compositions of the disclosure may be delivered to a cell or a subject through one or more routes and modalities. The viral vectors containing one or more effector modules, SREs, immunotherapeutic agents and other components described herein may be used to deliver them to a cell and/or a subject. Other modalities may also be used such as mRNAs, plasmids, and as recombinant proteins.

### 1. Delivery to cells

In another aspect of the disclosure, polynucleotides encoding biocircuits, effector modules, SREs (e.g., DDs), payloads of interest (immunotherapeutic agents) and compositions of the disclosure and vectors comprising said polynucleotides may be introduced into cells such as immune effector cells.

In one aspect of the disclosure, polynucleotides encoding biocircuits, effector modules, SREs (e.g., DDs), payloads of interest (immunotherapeutic agents) and compositions of the disclosure, may be packaged into viral vectors or integrated into viral genomes allowing transient or stable expression of the polynucleotides. Preferable viral vectors are retroviral vectors including lentiviral vectors. In order to construct a retroviral vector, a polynucleotide molecule encoding a biocircuit, an effector module, a DD or a payload of interest (i.e. an immunotherapeutic agent) is inserted into the viral genome in the place of certain viral sequences to produce a virus that is replication-defective. The recombinant viral vector is then introduced into a packaging cell line containing the gag, pol, and env genes, but without the LTR and packaging components. The recombinant retroviral particles are secreted into the culture media, then collected, optionally concentrated, and used for gene transfer. Lentiviral vectors are especially preferred as they are capable of infecting both dividing and non-dividing cells.

Vectors may also be transferred to cells by non-viral methods by physical methods such as needles, electroporation, sonoporation, hyrdoporation; chemical carriers such as inorganic particles (e.g. calcium phosphate, silica, gold) and/or chemical methods. In some instances, synthetic or natural biodegradable agents may be used for delivery such as cationic lipids, lipid nano emulsions, nanoparticles, peptide based vectors, or polymer based vectors.

In some instances, the polypeptides of the disclosure may be delivered to the cell directly. In one instance, the polypeptides of the disclosure may be delivered using synthetic peptides comprising an endosomal leakage domain (ELD) fused to a cell penetration domain (CLD). The polypeptides of the disclosure are co introduced into the cell with the ELD-CLD-synthetic peptide. ELDs facilitate the escape of proteins that are trapped in the endosome, into the cytosol. Such domains are derived proteins of microbial and viral origin and have been described in the art. CPDs allow the transport of proteins across the plasma membrane and have also been described in the art. The ELD-CLD fusion proteins synergistically increase the transduction efficiency when compared to the co-transduction with either domain alone. In some instances, a histidine rich domain may optionally be added to the shuttle construct as an additional method of allowing the escape of the cargo from the endosome into the cytosol. The shuttle may also include a cysteine residue at the N or C terminus to generate multimers of the fusion peptide. Multimers of the ELD-CLD fusion peptides generated by the addition of cysteine residue to the terminus of the peptide show even greater transduction efficiency when compared to the single fusion peptide constructs. The polypeptides of the disclosure may also be appended to appropriate localization signals to direct the cargo to the appropriate sub-cellular location e.g. nucleus. In some instances any of the ELDs, CLDs or the fusion ELD-CLD synthetic peptides taught in the International Patent Publication, WO2016161516 and WO2017175072 may be useful in the present disclosure .

### 2. Dosing

The present disclosure provides methods comprising administering any one or more compositions for immunotherapy to a subject in need thereof. These may be administered to a subject using any amount and any route of administration effective for preventing or treating a clinical condition such as cancer, infection diseases and other immunodeficient diseases.

Compositions in accordance with the disclosure are typically formulated in dosage unit form for ease of administration and uniformity of dosage. It will be understood, however, that the total daily usage of the compositions of the present disclosure may be decided by the attending physician within the scope of sound medical judgment. The specific therapeutically effective, or prophylactically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, previous or concurrent therapeutic interventions and rate of excretion of the specific compound employed; the duration of the treatment; drugs used in combination or coincidental with the specific compound employed; and like factors well known in the medical arts.

Compositions of the disclosure may be used in varying doses to avoid T cell anergy, prevent cytokine release syndrome and minimize toxicity associated with immunotherapy. For example, low doses of the compositions of the present disclosure may be used to initially treat patients with high tumor burden, while patients with low tumor burden may be treated with high and repeated doses of the compositions of the disclosure to ensure recognition of a minimal tumor antigen load. In another instance, the compositions of the present disclosure may be delivered in a pulsatile fashion to reduce tonic T cell signaling and enhance persistence *in vivo.* In some aspects, toxicity may be minimized by initially using low doses of the compositions of the disclosure, prior to administering high doses. Dosing may be modified if serum markers such as ferritin, serum C-reactive protein, IL6, IFN γ , and TNF- a are elevated.

In one instance, polypeptides and/or polynucleotides expressing the compositions described herein e.g. effector modules, are administered to a subject in need thereof to treat cancer. In one instance, a population of cells comprising the biocircuits, effector modules and/or the SREs described herein is administered to a subject in need thereof to treat cancer.

In one instance, the cells expressing the compositions described herein is administered at a dose and/or dosing schedule described herein.

In one instance, compositions are introduced into immune cells (e.g., T cells, NK cells). The compositions may be introduced into the immune cells by methods including but not limited to viral transduction, transfection and/or in vitro transcription. In some instances, the immune cells are transduced with retroviruses. In one aspect, the retrovirus may be a lentivirus. In some aspects, the titer of the lentiviruses may be used to tune the expression of the payload. In some instances, the titer of the lentivirus may have a multiplicity of infection (MOI) ranging from 0.001- 0.01, 0.01- 0.1, 0.1-1, or 1-10. In some instances, the MOI may be 1, 2, 3, 4, 5, 6, 7, 8, 9, or 10.

In some instances, the subject (e.g., human) receives an initial administration of immune cells comprising the compositions described herein e.g. SREs, and one or more subsequent administrations of cells. In some instances, a therapeutically effective amount of the compositions and/or cells described herein may be administered to the subject. In some instances, the therapeutically effective amount may indicate the precise amount required to tumor inhibition, tumor prevalence and/or tumor burden. The therapeutically effective amount may be determined with consideration of the subject's age, weight, tumor size, sex, extent of infection or metastasis. In some instances, the cells expressing the compositions described herein may be administered at a dosage of 10⁴ to 10⁹ cells/kg body weight of the subject. The cells can be administered by using infusion techniques that are commonly known in immunotherapy (see, e.g., Rosenberg et al., New Eng. J. of Med. 319: 1676, 1988).

In some aspects, subjects may undergo leukapheresis, wherein leukocytes are collected, enriched, or depleted ex vivo to select and/or isolate the cells of interest, e.g., T cells. These T cell isolates may be expanded by methods known in the art and treated such that the compositions of the disclosure may be introduced into the cell. Subjects in need thereof may subsequently undergo standard treatment with high dose chemotherapy followed by peripheral blood stem cell transplantation. In certain aspects, following or concurrent with the transplant, subjects receive an infusion of the immune cells of the present disclosure. In an additional aspect, expanded cells are administered before or following surgery.

The dosage of the above treatments to be administered to a patient will vary with the precise nature of the condition being treated and the recipient of the treatment. The scaling of dosages for human administration can be performed according to art- accepted practices.

In one instance, the compositions are introduced into immune cells (e.g., T cells, NK cells), e.g., using in vitro transcription, and the subject (e.g., human) receives an initial administration of immune cells (e.g., T cells, NK cells) of the disclosure, and one or more subsequent administrations of the immune cells (e.g., T cells, NK cells) of the disclosure, wherein the one or more subsequent administrations are administered less than 15 days, e.g., 14, 13, 12, 11, 10, 9, 8, 7, 6, 5, 4, 3, or 2 days after the previous administration. In one instance, more than one administration of the immune cells (e.g., T cells, NK cells) of the disclosure are administered to the subject (e.g., human) per week, e.g., 2, 3, or 4 administrations of the immune cells (e.g., T cells, NK cells) of the disclosure are administered per week. In one instance, the subject (e.g., human subject) receives more than one administration of the immune cells (e.g., T cells, NK cells) per week (e.g., 2, 3 or 4 administrations per week) (also referred to herein as a cycle), followed by a week of no immune cells (e.g., T cells, NK cells) administrations, and then one or more additional administration of the immune cells (e.g., T cells, NK cells) (e.g., more than one administration of the immune cells (e.g., T cells, NK cells) per week) is administered to the subject. In another instance, the subject (e.g., human subject) receives more than one cycle of immune cells (e.g., T cells, NK cells), and the time between each cycle is less than 10, 9, 8, 7, 6, 5, 4, or 3 days. In one instance, the immune cells (e.g., T cells, NK cells) are administered every other day for 3 administrations per week. In one instance, the immune cells (e.g., T cells, NK cells) of the disclosure are administered for at least two, three, four, five, six, seven, eight or more weeks. In some instances, a dose of immune cells expressing compositions of the disclosure described herein comprises about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, 3 x 10⁸, or 5 x 10⁸ cells/kg. In some instances, a dose of immune cells comprises at least about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, 3 x 10⁸, or 5 x 10⁸ cells/kg. In some instances, a dose of immune cells comprises up to about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, 3 x 10⁸, or 5 x 10⁸ cells/kg. In some instances, a dose of immune cells comprises about 1.1 x 10⁶- 1.8 x 10⁷ cells/kg. In some instances, a dose of immune cells comprises about 1 x 10⁷, 2 x 10⁷, 5 x 10⁷, 1 x 10⁸, 2 x 10⁸, 3 x 10⁸, 5 x 10⁸, 1 x 10⁹, 2 x 10⁹, or 5 x 10⁹cells. In some instances, a dose of the immune cells comprises at least about 1 x 10⁷, 2x 10⁷, 5 x 10⁷, 1 x 10⁸, 2 x 10⁸, 3 x 10⁸, 5 x 10⁸, 1 x 10⁹, 2 x 10⁹, or 5 x 10⁹cells. In some instances, a dose of immune cells comprises up to about 1 x 10⁷, 2 x 10⁷, 5 x 10⁷, 1 x 10⁸, 2 x 10⁸, 3 x 10⁸, 5 x 10⁸, 1 x 10⁹, 2 x 10⁹, or 5 x 10⁹ cells. In some instances, a dose of the immune cells comprises up to about 1 x 10⁷, 1.5 x 10⁷, 2 x 10⁷, 2.5 x 10⁷, 3 x 10⁷, 3.5 x 10⁷, 4 x 10⁷, 5 x 10⁷, 1 x 10⁸, 1.5 x 10⁸, 2 x 10⁸, 2.5 x 10⁸, 3 x 10⁸, 3.5 x 10⁸, 4 x 10⁸, 5 x 10⁸, 1 x 10⁹, 2 x 10⁹, or 5 x 10⁹ cells. In some instances, a dose of immune cells comprises up to about 1-3 x 10⁷ to 1-3 x 10⁸ cells.

In one instance, the cells expressing the compositions described herein, are administered as a first line treatment for the disease, e.g., the cancer, e.g., the cancer described herein. In another instance, the cells expressing the compositions described herein, are administered as a second, third, fourth line treatment for the disease, e.g., the cancer, e.g., the cancer described herein. In some instances, the subject may undergo preconditioning prior to the administration of the cells.

Also provided herein are methods of administering ligands in accordance with the disclosure to a subject in need thereof. The ligand may be administered to a subject or to cells, using any amount and any route of administration effective for tuning the biocircuits of the disclosure. The exact amount required will vary from subject to subject, depending on the species, age, and general condition of the subject, the severity of the disease, the particular composition, its mode of administration, its mode of activity, and the like. The subject may be a human, a mammal, or an animal. Compositions in accordance with the disclosure are typically formulated in unit dosage form for ease of administration and uniformity of dosage. It will be understood, however, that the total daily usage of the compositions of the present disclosure may be decided by the attending physician within the scope of sound medical judgment. In certain instances, the ligands in accordance with the present disclosure may be administered at dosage levels sufficient to deliver from about 0.0001 mg/kg to about 100 mg/kg, from about 0.001 mg/kg to about 0.05 mg/kg, from about 0.005 mg/kg to about 0.05 mg/kg, from about 0.001 mg/kg to about 0.005 mg/kg, from about 0.05 mg/kg to about 0.5 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, from about 10 mg/kg to about 100 mg/kg, from about 1 mg/kg to about 1000 mg/kg, from about 50 mg/kg to about 500 mg/kg, from about 100 mg/kg to about 1000 mg/kg, of subject body weight per day, one or more times a day, to obtain the desired effect. In some instances, the dosage levels may be 1mg/kg, 5 mg/kg, 10mg/kg, 20mg/kg, 30 mg/kg, 40 mg/kg, 50 mg/kg, 60 mg/kg, 70 mg/kg, 80 mg/kg, 90 mg/kg, 100 mg/kg, 100 mg/kg, 110 mg/kg, 120 mg/kg, 130 mg/kg,140 mg/kg, 150 mg/kg, 160 mg/kg, 170 mg/kg, 180 mg/kg, 190 mg/kg or mg/kg of subject body weight per day, or more times a day, to obtain the desired effect. In one instance, the dose of the ligand may be 10 mg/kg. In one instance, the dose of the ligand may be 30 mg/kg. In one aspect, the dose of the ligand may be 100 mg/kg.

The present disclosure provides methods for delivering to a cell or tissue any of the ligands described herein, comprising contacting the cell or tissue with said ligand and can be accomplished in vitro, ex vivo, or in vivo. In certain instances, the ligands in accordance with the present disclosure may be administered to cells at dosage levels sufficient to deliver from about 1 nM to about 10 nM, from about 5 nM to about 50 nM, from about 10 nM to about 100 nM, from about 50 nM to about 500 nM, from about 100 nM to about 1000 nM, from about 1µM to about 10µM, from about 5µM to about 50µM, from about 10µM to about 100µM from about 25µM to about 250µM, from about 50µM to about 500µM. In some instances, the ligand may be administered to cells at doses selected from but not limited to 0.0064µM, 0.0032µM, 0.016µM, 0.08µM, 0.4µM, 1µM, 2µM, 5µM, 10µM,25µM, 50µM, 75µM, 100µM, 150µM, 200µM, 250µM.

The desired dosage of the ligands of the present disclosure may be delivered only once, three times a day, two times a day, once a day, every other day, every third day, every week, every two weeks, every three weeks, or every four weeks. In certain instances, the desired dosage may be delivered using multiple administrations (e.g., two, three, four, five, six, seven, eight, nine, ten, eleven, twelve, thirteen, fourteen, or more administrations). When multiple administrations are employed, split dosing regimens such as those described herein may be used. As used herein, a "split dose" is the division of "single unit dose" or total daily dose into two or more doses, e.g., two or more administrations of the "single unit dose". As used herein, a "single unit dose" is a dose of any therapeutic administered in one dose/at one time/single route/single point of contact, i.e., single administration event. The desired dosage of the ligand of the present disclosure may be administered as a "pulse dose" or as a "continuous flow". As used herein, a "pulse dose" is a series of single unit doses of any therapeutic administered with a set frequency over a period of time. As used herein, a "continuous flow" is a dose of therapeutic administered continuously for a period of time in a single route/single point of contact, i.e., continuous administration event. A total daily dose, an amount given or prescribed in 24-hour period, may be administered by any of these methods, or as a combination of these methods, or by any other methods suitable for a pharmaceutical administration.

### 3. Administration

In some instances, the compositions for immunotherapy may be administered to cells *ex vivo* and subsequently administered to the subject. Immune cells can be isolated and expanded *ex vivo* using a variety of methods known in the art. For example, methods of isolating cytotoxic T cells are described in U.S. Pat. NOs. 6,805,861 and 6,531,451. Isolation of NK cells is described in U.S. Pat. NO.: 7,435, 596.

In some instances, depending upon the nature of the cells, the cells may be introduced into a host organism e.g. a mammal, in a wide variety of ways including by injection, transfusion, infusion, local instillation or implantation. In some aspects, the cells of the disclosure may be introduced at the site of the tumor. The number of cells that are employed will depend upon a number of circumstances, the purpose for the introduction, the lifetime of the cells, the protocol to be used, for example, the number of administrations, the ability of the cells to multiply, or the like. The cells may be in a physiologically-acceptable medium.

In some instances, the cells of the disclosure may be administrated in multiple doses to subjects having a disease or condition. The administrations generally effect an improvement in one or more symptoms of cancer or a clinical condition and/or treat or prevent cancer or clinical condition or symptom thereof.

In some instances, the compositions for immunotherapy may be administered *in vivo.* In some instances, polypeptides of the present disclosure comprising biocircuits, effector molecules, SREs, payloads of interest (immunotherapeutic agents) and compositions of the disclosure may be delivered in vivo to the subject. In vivo delivery of immunotherapeutic agents is well described in the art. For example, methods of delivery of cytokines are described in the E.P. Pat. No.: EP0930892 A1.

### Routes of delivery

The pharmaceutical compositions, biocircuits, biocircuit components, effector modules including their SREs (e.g., DDs), payloads (i.e. immunotherapeutic agents), vectors and cells of the present disclosure may be administered by any route to achieve a therapeutically effective outcome.

These include, but are not limited to enteral (into the intestine), gastroenteral, epidural (into the dura matter), oral (by way of the mouth), transdermal, peridural, intracerebral (into the cerebrum), intracerebroventricular (into the cerebral ventricles), epicutaneous (application onto the skin), intradermal, (into the skin itself), subcutaneous (under the skin), nasal administration (through the nose), intravenous (into a vein), intravenous bolus, intravenous drip, intra-arterial (into an artery), intramuscular (into a muscle), intracranial (into the heart), intraosseous infusion (into the bone marrow), intrathecal (into the spinal canal), intraperitoneal, (infusion or injection into the peritoneum), intrasinal infusion, intravitreal, (through the eye), intravenous injection (into a pathologic cavity) intracavitary (into the base of the penis), intravaginal administration, intrauterine, extra-amniotic administration, transdermal (diffusion through the intact skin for systemic distribution), transmucosal (diffusion through a mucous membrane), transvaginal, insufflation (snorting), sublingual, sublabial, enema, eye drops (onto the conjunctiva), in ear drops, auricular (in or by way of the ear), buccal (directed toward the cheek), conjunctival, cutaneous, dental (to a tooth or teeth), electro-osmosis, endocervical, endosinusial, endotracheal, extracorporeal, hemodialysis, infiltration, interstitial, intra-abdominal, intra-amniotic, intra-articular, intrabiliary, intrabronchial, intrabursal, intracartilaginous (within a cartilage), intracaudal (within the cauda equine), intracisternal (within the cisterna magna cerebellomedularis), intracorneal (within the cornea), dental intracomal, intracoronary (within the coronary arteries), intracorporus cavernosum (within the dilatable spaces of the corporus cavernosa of the penis), intradiscal (within a disc), intraductal (within a duct of a gland), intraduodenal (within the duodenum), intradural (within or beneath the dura), intraepidermal (to the epidermis), intraesophageal (to the esophagus), intragastric (within the stomach), intragingival (within the gingivae), intraileal (within the distal portion of the small intestine), intralesional (within or introduced directly to a localized lesion), intraluminal (within a lumen of a tube), intralymphatic (within the lymph), intramedullary (within the marrow cavity of a bone), intrameningeal (within the meninges), intramyocardial (within the myocardium), intraocular (within the eye), intraovarian (within the ovary), intrapericardial (within the pericardium), intrapleural (within the pleura), intraprostatic (within the prostate gland), intrapulmonary (within the lungs or its bronchi), intrasinal (within the nasal or periorbital sinuses), intraspinal (within the vertebral column), intrasynovial (within the synovial cavity of a joint), intratendinous (within a tendon), intratesticular (within the testicle), intrathecal (within the cerebrospinal fluid at any level of the cerebrospinal axis), intrathoracic (within the thorax), intratubular (within the tubules of an organ), intratumor (within a tumor), intratympanic (within the aurus media), intravascular (within a vessel or vessels), intraventricular (within a ventricle), iontophoresis (by means of electric current where ions of soluble salts migrate into the tissues of the body), irrigation (to bathe or flush open wounds or body cavities), laryngeal (directly upon the larynx), nasogastric (through the nose and into the stomach), occlusive dressing technique (topical route administration which is then covered by a dressing which occludes the area), ophthalmic (to the external eye), oropharyngeal (directly to the mouth and pharynx), parenteral, percutaneous, periarticular, peridural, perineural, periodontal, rectal, respiratory (within the respiratory tract by inhaling orally or nasally for local or systemic effect), retrobulbar (behind the pons or behind the eyeball), intramyocardial (entering the myocardium), soft tissue, subarachnoid, subconjunctival, submucosal, topical, transplacental (through or across the placenta), transtracheal (through the wall of the trachea), transtympanic (across or through the tympanic cavity), ureteral (to the ureter), urethral (to the urethra), vaginal, caudal block, diagnostic, nerve block, biliary perfusion, cardiac perfusion, photopheresis or spinal.

### Kits

The also provides a kit comprising any of the polynucleotides or expression vectors described herein.

The present disclosure includes a variety of kits for conveniently and/or effectively carrying out methods of the present disclosure. Typically, kits will comprise sufficient amounts and/or numbers of components to allow a user to perform one or multiple treatments of a subject(s) and/or to perform one or multiple experiments.

In one instance, the present disclosure provides kits for inhibiting genes in vitro or in vivo, comprising a biocircuit of the present disclosure or a combination of biocircuits of the present disclosure, optionally in combination with any other suitable active agents.

The kit may further comprise packaging and instructions and/or a delivery agent to form a formulation composition. The delivery agent may comprise, for example, saline, a buffered solution.

In additional instances, assay screening kits are provided. The kit includes a container for the screening assay. An instruction for the use of the assay and the information about the screening method are to be included in the kit.

In some instances, the DDs, effector modules and biocircuit system and compositions of the disclosure may be used as research tools to investigate protein activity in a biological system such a cell and a subject. In other instances, the DDs, effector modules and biocircuit system and compositions of the disclosure may be used for treating a disease such as a cancer and a genetic disorder.

### VI. APPLICATIONS

In one aspect of the present disclosure, methods for reducing a tumor volume or burden are provided. The methods comprise administering a pharmaceutically effective amount of a pharmaceutical composition comprising at least one biocircuit system, effector module, DD, and/or payload of interest (i.e., an immunotherapeutic agent), at least one vector, or cells to a subject having a tumor. The biocircuit system and effector module having any immunotherapeutic agent as described herein may be in forms of a polypeptide, or a polynucleotide such as mRNA, or a viral vector comprising the polynucleotide, or a cell modified to express the biocircuit, effector module, DD, and payload of interest (i.e., immunotherapeutic agent).

In another aspect of the present disclosure, methods for inducing an anti-tumor immune response in a subject are provided. The methods comprise administering a pharmaceutically effective amount of a pharmaceutical composition comprising at least one biocircuit system, effector module, DD, and/or payload of interest (i.e., an immunotherapeutic agent), at least one vector, or cells to a subject having a tumor. The biocircuit and effector module having any immunotherapeutic agent as described herein may be in forms of a polypeptide, or a polynucleotide such as mRNA, or a viral vector comprising the polynucleotide, or a cell modified to express the biocircuit, effector module, DD, and payload of interest (i.e., immunotherapeutic agent).

The methods, per the present disclosure, may be adoptive cell transfer (ACT) using genetically engineered cells such as immune effector cells of the disclosure, cancer vaccines comprising biocircuit systems, effector modules, DDs, payloads of interest (i.e., immunotherapeutic agents) of the disclosure, or compositions that manipulate the tumor immunosuppressive microenvironment, or the combination thereof. These treatments may be further employed with other cancer treatment such as chemotherapy and radiotherapy.

### 1. Adoptive cell transfer (adoptive immunotherapy)

Recent progress in the field of cancer immunology has allowed the development of several approaches to help the immune system keep the cancer at bay. Such immunotherapy approaches include the targeting of cancer antigens through monoclonal antibodies or through adoptive transfer of ex vivo engineered T cells (e.g., which contain chimeric antigen receptors or engineered T cell receptors). The present disclosure also provides methods for inducing immune responses in a subject using the compositions of the disclosure. Also provided are methods for reducing a tumor burden in a subject using the compositions of the disclosure. The present disclosure also provides immune cells engineered to include one or more polypeptides, polynucleotides, or vectors of the present disclosure. The cells may be immune effector cells, including T cells such as cytotoxic T cells, helper T cells, memory T cells, regulatory T cells, natural killer (NK) cells, NK T cells, cytokine-induced killer (CIK) cells, cytotoxic T lymphocytes (CTLs), and tumor infiltrating lymphocytes (TILs). The engineered cell may be used for adoptive cell transfer for treating a disease (e.g., a cancer).

In some instances, pharmaceutical compositions, biocircuits, biocircuit components, effector modules including their SREs or payloads of the present disclosure may be used in the modulation or alteration or exploitation of the immune system to target one or more cancers. This approach may also be considered with other such biological approaches, e.g., immune response modifying therapies such as the administration of interferons, interleukins, colony-stimulating factors, other monoclonal antibodies, vaccines, gene therapy, and nonspecific immunomodulating agents are also envisioned as anti-cancer therapies to be combined with the pharmaceutical compositions, biocircuits, biocircuit components, effector modules including their SREs or payloads of the present disclosure.

Cancer immunotherapy refers to a diverse set of therapeutic strategies designed to induce the patient's own immune system to fight the cancer. In some instances, pharmaceutical compositions, biocircuits, biocircuit components, effector modules including their SREs or payloads of the present disclosure are designed as immune-oncology therapeutics.

In some instances, cells which are genetically modified to express at least one biocircuit system, effector module, DD, and/or payload of interest (immunotherapeutic agent) may be used for adoptive cell therapy (ACT). As used herein, Adoptive cell transfer refers to the administration of immune cells (from autologous, allogenic or genetically modified hosts) with direct anticancer activity. ACT has shown promise in clinical application against malignant and infectious disease. For example, T cells genetically engineered to recognize CD19 have been used to treat follicular B cell lymphoma (Kochenderfer et al., Blood, 2010, 116:4099-4102; and Kochenderfer and Rosenberg, Nat Rev Clin Oncol., 2013, 10(5): 267-276) and ACT using autologous lymphocytes genetically-modified to express anti-tumor T cell receptors has been used to treat metastatic melanoma (Rosenberg and Dudley, Curr. Opin. Immunol. 2009, 21: 233-240).

Immune cells for adoptive cell therapy may be selected from but not limited to selected from a CD8+ T cell, a CD4+ T cell, a helper T cell, a natural killer (NK) cell, a NKT cell, a cytotoxic T lymphocyte (CTL), a tumor infiltrating lymphocyte (TIL), a memory T cell, a regulatory T (Treg) cell, a cytokine-induced killer (CIK) cell, a dendritic cell, a human embryonic stem cell, a mesenchymal stem cell, a hematopoietic stem cell, or a mixture thereof.

There are several types of cellular immunotherapies, including tumor infiltrating lymphocyte (TIL) therapy, genetically engineered T cells bearing chimeric antigen receptors (CARs), and recombinant TCR technology. In some instances, the compositions of the present disclosure may be utilized to alter TIL (tumor infiltrating lymphocyte) populations in a subject. In one instance, any of the payloads described herein may be utilized to change the ratio of CD4 positive cells to CD8 positive populations. In some instances, TILs may be sorted ex vivo and engineered to express any of the cytokines described herein. Payloads of the disclosure may be used to expand CD4 and/or CD8 populations of TILs to enhance TIL mediated immune response.

In one instance, the chimeric antigen receptor (CAR) of the present disclosure may be a conditionally active CAR. A wild type protein or domain thereof, such as those described herein may be used to generate a conditionally active biologic protein which are reversibly or irreversibly inactivated at the wild type normal physiological conditions as well as to such conditionally active biologic proteins and domains and uses of such conditional active biologic proteins and domains are provided. Such methods and conditionally active proteins are taught in, for example, International Publication No. WO2016033331. As a non-limiting example, the CAR comprises at least one antigen specific targeting region evolved from a wild type protein or a domain thereof and one or more of a decrease in activity in the assay at the normal physiological condition compared to the antigen specific targeting region of the wild-type protein or a domain thereof, and an increase in activity in the assay under the aberrant condition compared to the antigen specific targeting region of the wild-type protein or a domain thereof.

According to the present disclosure, the biocircuits and systems may be used in the development and implementation of cell therapies such as adoptive cell therapy. Certain effector modules useful in cell therapy are given in Figures 7-12. The biocircuits, their components, effector modules and their SREs and payloads may be used in cell therapies to effect TCR removal -TCR gene disruption, TCR engineering, to regulate epitope tagged receptors, in APC platforms for stimulating T cells, as a tool to enhance *ex vivo* APC stimulation, to improve methods of T cell expansion, in *ex vivo* stimulation with antigen, in TCR/CAR combinations, in the manipulation or regulation of TILs, in allogeneic cell therapy, in combination T cell therapy with other treatment lines (e.g. radiation, cytokines), to encode engineered TCRs, or modified TCRs, or to enhance T cells other than TCRs (e.g. by introducing cytokine genes, genes for the checkpoint inhibitors PD1, CTLA4).

In some instances, improved response rates are obtained in support of cell therapies.

Expansion and persistence of cell populations may be achieved through regulation or fine tuning of the payloads, e.g., the receptors or pathway components in T cells, NK cells or other immune-related cells. In some instances, biocircuits, their components, SREs or effector modules are designed to spatially and/or temporally control the expression of proteins which enhance T-cell or NK cell response. In some instances, biocircuits, their components, SREs or effector modules are designed to spatially and/or temporally control the expression of proteins which inhibit T-cell or NK cell response.

Provided herein are methods for use in adoptive cell therapy. The methods involve preconditioning a subject in need thereof, modulating immune cells with SRE, biocircuits and compositions of the present disclosure, administering to a subject, engineered immune cells expressing compositions of the disclosure and the successful engraftment of engineered cells within the subject.

In some instances, SREs, biocircuits and compositions of the present disclosure may be used to minimize preconditioning regimens associated with adoptive cell therapy. As used herein "preconditioning" refers to any therapeutic regimen administered to a subject in order to improve the outcome of adoptive cell therapy. Preconditioning strategies include, but are not limited to total body irradiation and/or lymphodepleting chemotherapy. Adoptive therapy clinical trials without preconditioning have failed to demonstrate any clinical benefit, indicating its importance in ACT. Yet, preconditioning is associated with significant toxicity and limits the subject cohort that is suitable for ACT. In some instances, immune cells for ACT may be engineered to express cytokines such as IL12 and IL15 as payload using SREs of the present disclosure to reduce the need for preconditioning (Pengram et al. (2012) Blood 119 (18): 4133-41).

In some instances, the neurotoxicity may be associated with CAR or TIL therapy. Such neurotoxicity may be associated CD19-CARs. Toxicity may be due to excessive T cell infiltration into the brain. In some instances, neurotoxicity may be alleviated by preventing the passage of T cells through the blood brain barrier. This can be achieved by the targeted gene deletion of the endogenous alpha-4 integrin inhibitors such as tysabri/natalizumab may also be useful in the present disclosure.

In some instances, the effector modules may encode one or more cytokines. In some instances, the cytokine is IL15. Effector modules encoding IL15 may be designed to induce proliferation in cytotoxic populations and avoid stimulation of T regs. In other cases, the effector modules which induce proliferation in cytotoxic populations may also stimulate NK and NKT cells.

In some instances, effector modules may encode, or be tuned or induced to produce, one or more cytokines for expansion of cells in the biocircuits of the disclosure. In such cases the cells may be tested for actual expansion. Expansion may be at least 5%, 10%, 20%, 30%, 40%, 50%, 60%, 70%, 80%, 90% or more.

In some instances, the tumor microenvironment may be remodeled using a biocircuit containing an effector module encoding IL17.

In some instances, biocircuits, their components, SREs or effector modules are designed to modulate Tregs to attenuate autoimmune disorders. In such a case, IL2 may be regulated using a singly tuned module or one having multiple tuned features as described herein.

In some instances, immune cells for ACT may be dendritic cells, T cells such as CD8⁺ T cells and CD4⁺ T cells, natural killer (NK) cells, NK T cells, Cytotoxic T lymphocytes (CTLs), tumor infiltrating lymphocytes (TILs), lymphokine activated killer (LAK) cells, memory T cells, regulatory T cells (Tregs), helper T cells, cytokine-induced killer (CIK) cells, and any combination thereof. In other instances, immune stimulatory cells for ACT may be generated from embryonic stem cell (ESC) and induced pluripotent stem cell (iPSC). In some instances, autologous or allogeneic immune cells are used for ACT.

In some instances, cells used for ACT may be T cells engineered to express T cell receptors (TCRs) with new specificities or CARs comprising an antigen-binding domain specific to an antigen on tumor cells of interest. In other instances, cells used for ACT may be NK cells engineered to express CARs comprising an antigen-binding domain specific to an antigen on tumor cells of interest. In addition to adoptive transfer of genetically modified T cells (e.g., CAR T cells) for immunotherapy, alternate types of CAR-expressing leukocytes, either alone, or in combination with CAR T cells may be used for adoptive immunotherapy. In one example, a mixture of T cells and NK cells may be used for ACT. The expression level of CARs in T cells and NK cells, according to the present disclosure, is tuned and controlled by a small molecule that binds to the DD(s) operably linked to the CAR in the effector module.

In some instances, NK cells engineered to express the present compositions may be used for ACT. NK cell activation induces perforin/granzyme-dependent apoptosis in target cells. NK cell activation also induces cytokine secretion such as IFN γ, TNF-α and GM-CSF. These cytokines enhance the phagocytic function of macrophages and their antimicrobial activity, and augment the adaptive immune response via up-regulation of antigen presentation by antigen presenting cells such as dendritic cells (DCs) (Reviewed by Vivier et al., Nat. Immunol., 2008, 9(5): 503-510).

Other examples of genetic modification may include the introduction of chimeric antigen receptors (CARs) and the down-regulation of inhibitory NK cell receptors such as NKG2A. Examples of CARs include, but are not limited to, CD19 and CD20 specific CARs against B cell malignancies, CARs targeting CD33 on leukemia cells, CS1 CAR and CD138 CAR on myeloma cells, GD2 CAR on neuroblastoma cells, Her2/Neu and erbB2 on breast cancer cells, carcinoembryonic antigen (CEA) on colon cancers, EpCAM on epithelial tumors, GPA7 on melanoma, NKG2D ligand on leukemia and solid tumors, and TRAIL R1 on various tumor targets. The CARs may be POIs of the effector modules and regulated by the binding of the DD with its corresponding ligand.

In another example, NK cells for ACT may be modified to express an effector module comprising the high-affinity CD16-158V polymorphism (HA-CD16) which augments NK cell mediated antibody dependent cell cytotoxicity (ADCC) against tumors. Infusion of NK cells genetically modified to express HA-CD16 could be used as a strategy to improve the efficacy of antibody-based therapies for cancer patients.

NK cells may also be genetically reprogrammed to circumvent NK cell inhibitory signals upon interaction with tumor cells. For example, using CRISPR, ZFN, or TALEN to genetically modify NK cells to silence their inhibitory receptors may enhance the anti-tumor capacity of NK cells.

Immune cells can be isolated and expanded *ex vivo* using a variety of methods known in the art. For example, methods of isolating and expanding cytotoxic T cells are described in U.S. Pat. NOs. 6,805,861 and 6,531, 451; US Patent Publication No.: US20160348072A1 and International Patent Publication NO: WO2016168595A1. Isolation and expansion of NK cells is described in US Patent Publication NO.: US20150152387A1, U.S. Patent NO.: 7,435, 596; and Oyer, J.L. (2016). Cytotherapy.18(5):653-63. Specifically, human primary NK cells may be expanded in the presence of feeder cells e.g. a myeloid cell line that has been genetically modified to express membrane bound IL15, IL21, IL12 and 4-1BBL.

In some instances, sub populations of immune cells may be enriched for ACT. Methods for immune cell enrichment are taught in International Patent Publication NO.: WO2015039100A1. In another example, T cells positive for B and T lymphocyte attenuator marker BTLA) may be used to enrich for T cells that are anti-cancer reactive as described in U.S. Pat. NO.: 9,512,401.

In some instances, immune cells for ACT may be depleted of select sub populations to enhance T cell expansion. For example, immune cells may be depleted of Foxp3+ T lymphocytes to minimize the ant-tumor immune response using methods taught in US Patent Publication NO.: US 20160298081A1.

In some instances, activation and expansion of T cells for ACT is achieved antigenic stimulation of a transiently expressed Chimeric Antigen Receptor (CAR) on the cell surface. Such activation methods are taught in International Patent NO.: WO2017015427.

In some instances, immune cells may be activated by antigens associated with antigen presenting cells (APCs). In some instances, the APCs may be dendritic cells, macrophages or B cells that antigen specific or nonspecific. The APCs may autologous or homologous in their organ. In some instances the APCs may be artificial antigen presenting cells (aAPCs) such as cell based aAPCs or acellular aAPCs. Cell based aAPCs are may be selected from either genetically modified allogeneic cells such as human erythroleukemia cells or xenogeneic cells such as murine fibroblasts and Drosophila cells. Alternatively, the APCs maybe be acellular wherein the antigens or costimulatory domains are presented on synthetic surfaces such as latex beads, polystyrene beads, lipid vesicles or exosomes.

In some instances, adoptive cell therapy is carried out by autologous transfer, wherein the cells are derived from a subject in need of a treatment and the cells, following isolation and processing are administered to the same subject. In other instances, ACT may involve allogenic transfer wherein the cells are isolated and/or prepared from a donor subject other than the recipient subject who ultimately receives cell therapy. The donor and recipient subject may be genetically identical, or similar or may express the same HLA class or subtype.

In some instances, the multiple immunotherapeutic agents introduced into the immune cells for ACT (e.g., T cells and NK cells) may be controlled by the same biocircuit system. In one example, a cytokine such as IL12 and a CAR construct such as CD 19 CAR are linked to the same hDHFR destabilizing domain. The expression of IL12 and CD19 CAR is tuned using TMP simultaneously. In other instances, the multiple immunotherapeutic agents introduced into the immune cells for ACT (e.g., T cells and NK cells) may be controlled by different biocircuit systems. In one example, a cytokine such as IL12 and a CAR construct such as CD19 CAR are linked to different DDs in two separate effector modules, thereby can be tuned separately using different stimuli. In another example, a suicide gene and a CAR construct may be linked to two separate effector modules.

Following genetic modulation using SREs, biocircuits and compositions of the disclosure, cells are administered to the subject in need thereof. Methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In some instances, immune cells for ACT may be modified to express one or more immunotherapeutic agents which facilitate immune cells activation, infiltration, expansion, survival and anti-tumor functions. The immunotherapeutic agents may be a second CAR or TCR specific to a different target molecule; a cytokine or a cytokine receptor; a chimeric switch receptor that converts an inhibitory signal to a stimulatory signal; a homing receptor that guides adoptively transferred cells to a target site such as the tumor tissue; an agent that optimizes the metabolism of the immune cell; or a safety switch gene (e.g., a suicide gene) that kills activated T cells when a severe event is observed after adoptive cell transfer or when the transferred immune cells are no-longer needed.

In some instances, immune cells used for adoptive cell transfer can be genetically manipulated to improve their persistence, cytotoxicity, tumor targeting capacity, and ability to home to disease sites *in vivo,* with the overall aim of further improving upon their capacity to kill tumors in cancer patients. One example is to introduce effector modules of the disclosure comprising cytokines such as gamma-cytokines (IL2 and IL15) into immune cells to promote immune cell proliferation and survival. Transduction of cytokine genes (e.g., gamma-cytokines IL2 and IL15) into cells will be able to propagate immune cells without addition of exogenous cytokines and cytokine expressing NK cells have enhanced tumor cytotoxicity.

Another example includes the use of genetic modification for directing the infused NK cells to proper tumor tissues. NK cells may be genetically engineered with an effector module that encodes the CCR7 receptor to improve their migration toward one of its ligands CCL19. Other strategies may involve utilizing chemokine receptors, such as CXCR3 to improve NK cell migration to inflamed tissues, such as those infiltrated with metastatic tumors.

NK cells may be modified to become insensitive to suppressive cytokines such as TGF-β, thereby preserving their cytotoxicity. For example, NK cells can be genetically modified to express the dominant negative mutant form of TGF-β type II receptor (DNTβRII) on their surface that render NK cells resistant to the suppressive effects of TGF-β.

In some instances, biocircuits, their components, SREs or effector modules are designed to be significantly less immunogenic than other biocircuits or switches in the art.

As used herein, "significantly less immunogenic" refers to a detectable decrease in immunogenicity. In another instance, the term refers to a fold decrease in immunogenicity. In another instance, the term refers to a decrease such that an effective amount of the biocircuits, their components, SREs or effector modules which can be administered without triggering a detectable immune response. In another instance, the term refers to a decrease such that the biocircuits, their components, SREs or effector modules can be repeatedly administered without eliciting an immune response. In another instance, the decrease is such that the biocircuits, their components, SREs or effector modules can be repeatedly administered without eliciting an immune response.

In another instance, the biocircuits, their components, SREs or effector modules is 2-fold less immunogenic than its unmodified counterpart or reference compound. In another instance, immunogenicity is reduced by a 3-fold factor. In another instance, immunogenicity is reduced by a 5-fold factor. In another instance, immunogenicity is reduced by a 7-fold factor. In another instance, immunogenicity is reduced by a 10-fold factor. In another instance, immunogenicity is reduced by a 15-fold factor. In another instance, immunogenicity is reduced by a fold factor. In another instance, immunogenicity is reduced by a 50-fold factor. In another instance, immunogenicity is reduced by a 100-fold factor. In another instance, immunogenicity is reduced by a 200-fold factor. In another instance, immunogenicity is reduced by a 500-fold factor. In another instance, immunogenicity is reduced by a 1000-fold factor. In another instance, immunogenicity is reduced by a 2000-fold factor. In another instance, immunogenicity is reduced by another fold difference.

Methods of determining immunogenicity are well known in the art, and include, e.g. measuring secretion of cytokines (e.g. IL12, IFNalpha, TNF-alpha, RANTES, MIP-1alpha or beta, IL6, IFN beta, or IL8), measuring expression of DC activation markers (e.g. CD83, HLA-DR, CD80 and CD86), or measuring ability to act as an adjuvant for an adaptive immune response.

The present disclosure provides method of inducing an immune response in a cell. As used herein the term "immune response" refers to the activity of the cells of the immune system in response to stimulus, or an antigen. In some instances, the antigen may be a cancer antigen. In some aspects, the methods of inducing an immune response may involve administering to the cell, a therapeutically effective amount of any of the compositions described herein. In one aspect, the method may involve administering the pharmaceutical compositions described herein. In one aspect, the method may involve administering the polynucleotides, vectors. In some instances, induction of the immune response occurs due to the expression and or function of the immunotherapeutic agents described herein. Methods of inducing immune response further may involve administering to the cell, an effective amount of the stimulus to tune the expression of the immunotherapeutic agent. In some instances, the immunotherapeutic agent is capable of inducing an immune response in response to the stimulus. The induction of the immune response may occur in a cell within a subject i.e. *in vivo, ex vivo* or *in vitro.* The induction of an immune response may be evaluated by measuring the release of cytokine such as IL2 and IFNγ from the cells. In some instances, the induction of an immune response may be measured by evaluating the cell surface markers such as but not limited to CD3, CD4, CD8, CD 14, CD20, CD11b, CD16, CD45 and HLA-DR, CD 69, CD28, CD44, IFNgamma. Examples of cell surface markers for antigen presenting cells include, but are not limited to, MHC class I, MHC Class II, CD40, CD45, B7-1, B7-2, IFN y receptor and IL2 receptor, ICAM-1 and/or Fcy receptor. Examples of cell surface markers for dendritic cells include, but are not limited to, MHC class I, MHC Class II, B7-2, CD18, CD29, CD31, CD43, CD44, CD45, CD54, CD58, CD83, CD86, CMRF-44, CMRF-56, DCIR and/or Dectin-1 and the like; while in some cases also having the absence of CD2, CD3, CD4, CD8, CD14, CD15, CD16, CD 19, CD20, CD56, and/or CD57. Examples of cell surface markers for NK cells include, but are not limited to, CCL3, CCL4, CCL5, Granulysin, Granzyme B, Granzyme K, IL10, IL22, IFNg, LAP, Perforin, and TNFa.

Parameters for improving CAR-T therapy outcome are described in Finney et al. JCI. 2019;129(5):2123-2132. The levels of biomarker LAG3 (high)/TNF-α (low) in peripheral blood CD8+ T cells at the time of apheresis may also predict a subsequent dysfunctional response in subjects with high antigen load who do not achieve complete response that is durable for more than a few weeks. T cell-intrinsic features that are a consequence of the starting T cell repertoire and the effects of the manufacturing process converge with CD 19 antigen-induced activation following adoptive transfer may also play a role in the outcome of CAR-T therapy. The starting T cell repertoire may in part be affected by the timing of the apheresis. In one instance, the apheresis may be performed prior to chemotherapy. Cumulative burden of CD19 expressing leukemic and normal B cells, as evaluated in the bone marrow prior to lymphodepleting chemotherapy may be important for determining CAR-T therapy outcome. According to Finney et al., increase antigen burden improves CAR-T therapy outcome. To increase CD19 antigen burden *in vivo,* subjects may also be infused with expanded subject derived T cells genetically modified to express CD19 (also referred to as T-APCs).

### 2. Cancer vaccines

In some instances, biocircuits, effector modules, payloads of interest (immunotherapeutic agents), vectors, cells and compositions of the present disclosure may be used for cancer vaccines. In one aspect, dendritic cells are modified to express the compositions of the disclosure and used as cancer vaccines.

In some instances, cancer vaccine may comprise peptides and/or proteins derived from tumor associated antigen (TAA). Such strategies may be utilized to evoke an immune response in a subject, which in some instances may be a cytotoxic T lymphocyte (CTL) response. Peptides used for cancer vaccines may also modified to match the mutation profile of a subject. For example, EGFR derived peptides with mutations matched to the mutations found in the subject in need of therapy have been successfully used in patients with lung cancer (Li F et al. (2016) Oncoimmunology. Oct 7;5(12): e1238539).

In one instance, cancer vaccines of the present disclosure may superagonist altered peptide ligands (APL) derived from TAAs. These are mutant peptide ligands deviate from the native peptide sequence by one or more amino acids, which activate specific CTL clones more effectively than native epitopes. These alterations may allow the peptide to bind better to the restricting Class I MHC molecule or interact more favorably with the TCR of a given tumor-specific CTL subset. APLs may be selected using methods taught in US Patent Publication NO.: US20160317633A1.

### 3. Tumor microenvironment (TME)

In some instances, biocircuits, their components, SREs or effector modules are designed to reshape the tumor microenvironment in order to extend utility of the biocircuit or a pharmaceutical composition beyond direct cell killing.

In some instances, biocircuits, their components, SREs or effector modules are designed to reduce, mitigate or eliminate the CAR cytokine storm. In some instances, such reduction, mitigation and/or elimination occurs in solid tumors or tumor microenvironments.

In some instances, biocircuits, effector modules, payloads of interest (immunotherapeutic agents), vectors, cells and pharmaceutical compositions of the present disclosure may be used to convert the immunosuppressive microenvironment to increase immune responses.

In some instances, the disclosure provides methods for converting the inhibitory immunoregulatory signals from immunosuppressive cytokines secreted by cancer cells or the surrounding tumor stroma into stimulatory signals using compositions of the disclosure. Immunosuppressive cytokines include without limitation, IL13, IL4, TGF-beta, IL6, IL8, and IL10. In one aspect, the genetically modified tumor-specific T cells (e.g., CAR T cells) or T cells with native tumor specificity may be further engineered to express an effector module comprising a chimeric switch receptor that binds to inhibitory/suppressive cytokines and converts their intracellular consequences to an immunostimulatory/activating signal, thus improving the efficacy of tumor-specific T cells. The chimeric switch receptor is composed of the extracellular domains of an inhibitory cytokine receptor (e.g., IL13R, IL4R, IL10R, TGFβR1/ALK5, TGFβR2, and TGFβR3/β-glycan) fused with the intracellular signal transducing domains derived from stimulatory cytokine receptors such as IL2R (i.e., IL2Rα/CD25, IL2Rβ/CD122, and common y chain receptor/CD 132 which is shared by various cytokine receptors) and/or IL7R (IL7Rα/CD127, common y chain receptor/CD132). These manipulations render tumor specific T cells or CAR T cells resistant to the suppressive tumor microenvironment.

In some instances, the present disclosure provides methods to abrogate the immunosuppressive effects produced by myeloid derived suppressor cells (MDSCs). Tumor cells secrete indoleamine 2,3-dioxygenase (IDO) which promotes immunosuppression through the recruitment of MDSCs. The immunosuppressive environment is further promoted by the MDSCs through nitric oxide synthase (NOS) and arginase 1 (ARG1) which can degrade extracellular arginine. Amino acid deprivation within the tumor microenvironment suppresses T cell anti-tumor activity. In some instances, payloads of the present disclosure may comprise inhibitors of NOS, ARG1 and tryptophan metabolism pathway such as IDO. In one instance, the payload may include inhibitors of colony stimulating factor receptor 1 (CSFR1) which required for the proliferation and function of MDSCs.

In some instances, dominant negative mutants of inhibitory co receptor such as PD-1, CTLA-4, LAG-3, TIM-3, KIRs, or BTLA may be utilized as payloads of the disclosure to overcome inhibitory signals in the tumor microenvironment.

### 4. Combination treatments

In some instances, it is desirable to combine compositions, vectors and cells of the disclosure for administration to a subject. Compositions of the disclosure comprising different immunotherapeutic agents may be used in combination for enhancement of immunotherapy.

### Immunotherapeutic Agents

In some instances, it is desirable to combine compositions of the disclosure with adjuvants, that can enhance the potency and longevity of antigen-specific immune responses. Adjuvants used as immunostimulants in combination therapy include biological molecules or delivery carriers that deliver antigens. As non-limiting examples, the compositions of the disclosure may be combined with biological adjuvants such as cytokines, Toll Like Receptors, bacterial toxins, and/or saponins. In other instances, the compositions of the present disclosure may be combined with delivery carriers. Exemplary delivery carriers include, polymer microspheres, immune stimulating complexes, emulsions (oil-in-water or water-in-oil), aluminum salts, liposomes or virosomes.

In some instances, immune effector cells modified to express biocircuits, effector modules, DDs and payloads of the disclosure may be combined with the biological adjuvants described herein. Dual regulation of CAR and cytokines and ligands to segregate the kinetic control of target-mediated activation from intrinsic cell T cell expansion. Such dual regulation also minimizes the need for pre-conditioning regimens in patients. As a non-limiting example, DD regulated CAR e.g. CD19 CAR may be combined with cytokines e.g. IL12 to enhance the anti-tumor efficacy of the CAR (Pegram H.J., et al. Tumor-targeted T cells modified to secrete IL12 eradicate systemic tumors without need for prior conditioning. Blood.2012;119:4133-41). As another non-limiting example, Merchant et al. combined dendritic cell- based vaccinations with recombinant human IL7 to improve outcome in high-risk pediatric sarcomas patients (Merchant, M.S. et. al. Adjuvant immunotherapy to Improve Outcome in High-Risk Pediatric Sarcomas. Clin Cancer Res. 2016. 22(13):3182-91).

In some instances, immune effector cells modified to express one or more antigen-specific TCRs or CARs may be combined with compositions of the disclosure comprising immunotherapeutic agents that convert the immunosuppressive tumor microenvironment.

In one aspect, effector immune cells modified to express CARs specific to different target molecules on the same cell may be combined. In another aspect, different immune cells modified to express the same CAR construct such as NK cells and T cells may be used in combination for a tumor treatment, for instance, a T cell modified to express a CD19 CAR may be combined with a NK cell modified to express the same CD19 CAR to treat B cell malignancy.

In other instances, immune cells modified to express CARs may be combined with checkpoint blockade agents.

In some instances, immune effector cells modified to expressed biocircuits, effector modules, DDs and payloads of the disclosure may be combined with cancer vaccines of the disclosure.

In some instances, an effector module comprising a CAR may be used in combination with an effector module comprising a cytokine, or an effector module comprising a safety switch, or an effector module comprising a metabolic factor, or an effector module comprising a homing receptor.

In one instance, an effector module comprising a CD 19 CAR may be used in combination with amino pyrimidine derivatives such as the Burkit's tyrosine receptor kinase (BTK) inhibitor using methods taught in International Patent Application NO.: WO2016164580.

### Cancer

In some instances, methods of the disclosure may include combination of the compositions of the disclosure with other agents effective in the treatment of cancers, infection diseases and other immunodeficient disorders, such as anti-cancer agents. As used herein, the term "anti-cancer agent" refers to any agent which is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer.

In some instances, anti-cancer agent or therapy may be a chemotherapeutic agent, or radiotherapy, immunotherapeutic agent, surgery, or any other therapeutic agent which, in combination with the present disclosure, improves the therapeutic efficacy of treatment.

In some instances, compositions of the present disclosure may be used in combination with immunotherapeutics other than the inventive therapy described herein, such as antibodies specific to some target molecules on the surface of a tumor cell.

Exemplary chemotherapies include, without limitation, Acivicin; Aclarubicin; Acodazole hydrochloride; Acronine; Adozelesin; Aldesleukin; Altretamine; Ambomycin; Ametantrone acetate; Amsacrine; Anastrozole; Anthramycin; Asparaginase; Asperrin, Sulindac, Curcumin, alkylating agents including: Nitrogen mustards such as mechlor-ethamine, cyclophosphamide, ifosfamide, melphalan and chlorambucil; nitrosoureas such as carmustine (BC U), lomustine (CCNU), and semustine (methyl-CC U); thylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrrolidine analogs such as 5- fluorouracil, fluorodeoxyuridine, gemcitabine, cytosine arabinoside (AraC, cytarabine), 5-azacytidine, 2,2'- difluorodeoxycytidine, purine analogs such as 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2- CdA); natural products including antimitotic drugs such as paclitaxel, vinca alkaloids including vinblastine (VLB), vincristine, and vinorelbine, taxotere, estramustine, and estramustine phosphate; epipodophylotoxins such as etoposide and teniposide; antibiotics, such as actimomycin D, daunomycin (rubidomycin), doxorubicin, mitoxantrone, idarubicin, bleomycins, plicamycin (mithramycin), mitomycinC, and actinomycin; enzymes such as L-asparaginase, cytokines such as interferon (IFN)-gamma, tumor necrosis factor (TNF)-alpha, TNF-beta and GM-CSF, anti-angiogenic factors, such as angiostatin and endostatin, inhibitors of FGF or VEGF such as soluble forms of receptors for angiogenic factors, including soluble VGF/VEGF receptors, platinum coordination complexes such as cisplatin and carboplatin, anthracenediones such as mitoxantrone, substituted urea such as hydroxyurea, methylhydrazine derivatives including N- methylhydrazine (MIFf) and procarbazine, adrenocortical suppressants such as mitotane (ο,ρ'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; non-steroidal antiandrogens such as flutamide; kinase inhibitors, histone deacetylase inhibitors, methylation inhibitors, proteasome inhibitors, monoclonal antibodies, oxidants, anti-oxidants, telomerase inhibitors, BH3 mimetics, ubiquitin ligase inhibitors, stat inhibitors and receptor tyrosin kinase inhibitors such as imatinib mesylate (marketed as Gleevac or Glivac) and erlotinib (an EGF receptor inhibitor) now marketed as Tarveca; anti-virals such as oseltamivir phosphate, Amphotericin B, and palivizumab; Sdi 1 mimetics; Semustine; Senescence derived inhibitor 1; Sparfosic acid; Spicamycin D; Spiromustine; Splenopentin; Spongistatin 1; Squalamine; Stipiamide; Stromelysin inhibitors; Sulfinosine; Superactive vasoactive intestinal peptide antagonist; Velaresol; Veramine; Verdins; Verteporfin; Vinorelbine; Vinxaltine; Vitaxin; Vorozole; Zanoterone; Zeniplatin; Zilascorb; and Zinostatin stimalamer; PI3Kβ small-molecule inhibitor, GSK2636771; pan-PI3K inhibitor (BKM120); BRAF inhibitors. Vemurafenib (Zelboraf) and dabrafenib (Tafinlar); or any analog or derivative and variant of the foregoing.

In one instance, the disclosure further relates to the use of pharmaceutical compositions, biocircuits, biocircuit components, effector modules including their SREs or payloads of the present disclosure for treating one or more forms of cancer, in combination with other pharmaceuticals and/or other therapeutic methods, e.g., with known pharmaceuticals and/or known therapeutic methods, such as, for example, those which are currently employed for treating these disorders. For example, the pharmaceutical compositions, biocircuits, biocircuit components, effector modules including their SREs or payloads of the present disclosure can also be administered in conjunction with one or more additional anti-cancer treatments, such as biological, chemotherapy and radiotherapy. Accordingly, a treatment can include, for example, imatinib (Gleevac), all-trans-retinoic acid, a monoclonal antibody treatment (gemtuzumab, ozogamicin), chemotherapy (for example, chlorambucil, prednisone, prednisolone, vincristine, cytarabine, clofarabine, farnesyl transferase inhibitors, decitabine, inhibitors of MDR1), rituximab, interferon-α, anthracycline drugs (such as daunorubicin or idarubicin), L-asparaginase, doxorubicin, cyclophosphamide, doxorubicin, bleomycin, fludarabine, etoposide, pentostatin, or cladribine), bone marrow transplant, stem cell transplant, radiation therapy, anti-metabolite drugs (methotrexate and 6-mercaptopurine), or any of the antibodies taught herein such as those in Table 6 of the co-owned U.S. Provisional Patent Application No. 62/320,864 filed April 11, 2016, 62/466,596 filed March 3, 2017 and the International Publication WO2017/180587 , or combinations thereof.

The present disclosure provides methods for increasing the survival of a subject with cancer. Such methods may include (a) administering to the subject, an effective amount of an immune cell. The immune cell may express an effector module which includes a stimulus response element (SRE) derived from a region or a portion of PDE5 (SEQ ID NO:1) operably linked to a payload. In one instance, the payload may be a chimeric antigen receptor (CAR). The SRE described herein may be responsive to and/or interact with at least one stimulus. The methods for increasing survival may also include administering the stimulus to the subject. The stimulus may cause the expression of the CAR to be modulated. The modulation of CAR expression may induce an anti-cancer immune response, which may increase the survival of the subject. The stimulus useful in the methods for promoting survival may be a small molecule. The small molecule may be Tadalafil, Vardenafil or Vardenafil. In one instance, the small molecule is Tadalafil. The small molecule may be administered to the subject at a dose of from about 1 mg/kg to about 1000 mg/kg body weight of the subject. In one instance, the small molecule may be administered at a dose of 100 mg/kg. In one aspect, the small molecule is administered at a dose of 30 mg/kg. The method of increasing the survival in the subject with cancer may involve administering the small molecule to the subject at least once a day. In one instance, the survival in the subject may be increased by a survival ratio of at least 1.3. The SRE described herein may be derived from a region or portion of PDE5 (SEQ ID NO:1) may include at least one mutation selected from the group comprising S542L, V548M, P549S, S550F, L554R, I556S, S562G, F564I, L569M, T571S, T571I, L573M, C574Y, V585M, Q586L, Q586P, Q589E, H592R, V594I, K604R, N605D, N605Y, R607W, R607Q, N609Y, A611T, Y612F, Y612W, Y612A, H613L, H613R, W615R, H617L, N620S, M625I, K633E, H653A, D656L, R658H, G659A, N662Y, S663Y, Q666H, L675P, I680S, E682A, D687A, H685L, M691I, L693P, I700F, I706N, E708V, Y709H, K710N, T712M, I715K, A722V, T723S, T723R, D724Y, D724A, D724G, L727P, Y728L, K730E, R732L, R732G, R732A, R732V, R732I, R732P, R732F, R732W, R732Y, R732H, R732S, R732T, R732D, R732E, R732Q, R732N, R732M, R732C, R732K, F735L, F736A, F736G, F736L, F736M, F736R, F736W, F736K, F736Q, F736E, F736S, F736P, F736V, F736C, F736Y, F736H, F736I, F736N, F736D, F736T, Q743L, F755Y, M758I, M760I, A762T, D764A, A767E, I768N, K770Q, W772C, A779T, L781P, T784I, F787V, F787A, E795R, K795N, E796G, E796D, I799L, D803N, E808G, M816A, F820I, I821A, A823T, Y829A, T833S, R847G, R847T, K848N, W853F, E858V, and Q859R.

Also provided herein are methods of reducing tumor burden in a subject. Such methods may include (a) administering to the subject, an effective amount of an immune cell. The immune cell may express an effector module which includes a stimulus response element (SRE) derived from a region or a portion of PDE5 (SEQ ID NO:1) operably linked to a payload. In one instance, the payload may be a chimeric antigen receptor (CAR). The SRE described herein may be responsive to and/or interact with at least one stimulus. The methods for increasing survival may also include administering the stimulus to the subject. The stimulus may cause the expression of the CAR to be modulated. The modulation of CAR expression may induce an anti-tumor immune response in the subject. The stimulus useful in the methods for reducing tumor burden may be a small molecule. The small molecule may be Tadalafil, Vardenafil or Vardenafil. In one instance, the small molecule is Tadalafil. The small molecule may be administered to the subject at a dose of from about 1 mg/kg to about 1000 mg/kg body weight of the subject. In one instance, the small molecule may be administered at a dose of 100 mg/kg. In one aspect, the small molecule is administered at a dose of 30 mg/kg. The methods reducing tumor burden in the subject may involve administering the small molecule to the subject at least once a day. In one instance, the small molecule may be administered twice a day. The SRE described herein may be derived from a region or portion of PDE5 (SEQ ID NO:1) may include at least one mutation selected from the group comprising S542L, V548M, P549S, S550F, L554R, I556S, S562G, F564I, L569M, T571S, T571I, L573M, C574Y, V585M, Q586L, Q586P, Q589E, H592R, V594I, K604R, N605D, N605Y, R607W, R607Q, N609Y, A611T, Y612F, Y612W, Y612A, H613L, H613R, W615R, H617L, N620S, M625I, K633E, H653A, D656L, R658H, G659A, N662Y, S663Y, Q666H, L675P, I680S, E682A, D687A, H685L, M691I, L693P, I700F, I706N, E708V, Y709H, K710N, T712M, I715K, A722V, T723S, T723R, D724Y, D724A, D724G, L727P, Y728L, K730E, R732L, R732G, R732A, R732V, R732I, R732P, R732F, R732W, R732Y, R732H, R732S, R732T, R732D, R732E, R732Q, R732N, R732M, R732C, R732K, F735L, F736A, F736G, F736L, F736M, F736R, F736W, F736K, F736Q, F736E, F736S, F736P, F736V, F736C, F736Y, F736H, F736I, F736N, F736D, F736T, Q743L, F755Y, M758I, M760I, A762T, D764A, A767E, I768N, K770Q, W772C, A779T, L781P, T784I, F787V, F787A, E795R, K795N, E796G, E796D, I799L, D803N, E808G, M816A, F820I, I821A, A823T, Y829A, T833S, R847G, R847T, K848N, W853F, E858V, and Q859R.

Radiotherapeutic agents and factors include radiation and waves that induce DNA damage for example, γ-irradiation, X-rays, UV-irradiation, microwaves, electronic emissions, radioisotopes, and the like. Therapy may be achieved by irradiating the localized tumor site with the above described forms of radiations. It is most likely that all of these factors effect a broad range of damage DNA, on the precursors of DNA, the replication and repair of DNA, and the assembly and maintenance of chromosomes. Dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

Radiation therapy (also called radiotherapy, X-ray therapy, or irradiation) is the use of ionizing radiation to kill cancer cells and shrink tumors. Radiation therapy can be administered externally via external beam radiotherapy (EBRT) or internally via brachytherapy. The effects of radiation therapy are localized and confined to the region being treated. Radiation therapy may be used to treat almost every type of solid tumor, including cancers of the brain, breast, cervix, larynx, lung, pancreas, prostate, skin, stomach, uterus, or soft tissue sarcomas. Radiation is also used to treat leukemia and lymphoma.

In some instances, the chemotherapeutic agent may be an immunomodulatory agent such as lenalidomide (LEN). Recent studies have demonstrated that lenalidomide can enhance antitumor functions of CAR modified T cells (Otahal et al., Oncoimmunology, 2015, 5(4): e1115940). Some examples of anti-tumor antibodies include tocilizumab, siltuximab.

Chemotherapy is the treatment of cancer with drugs that can destroy cancer cells. In current usage, the term "chemotherapy" usually refers to cytotoxic drugs which affect rapidly dividing cells in general, in contrast with targeted therapy. Chemotherapy drugs interfere with cell division in various possible ways, e.g. with the duplication of DNA or the separation of newly formed chromosomes. Most forms of chemotherapy target all rapidly dividing cells and are not specific to cancer cells, although some degree of specificity may come from the inability of many cancer cells to repair DNA damage, while normal cells generally can.

Most chemotherapy regimens are given in combination. Exemplary chemotherapeutic agents include, but are not limited to, 5-FU Enhancer, 9-AC, AG2037, AG3340, Aggrecanase Inhibitor, Aminoglutethimide, Amsacrine (m-AMSA), Asparaginase, Azacitidine, Batimastat (BB94), BAY 12-9566, BCH-4556, Bis-Naphthalimide, Busulfan, Capecitabine, Carboplatin, Carmustaine+Polifepr Osan, cdk4/cdk2 inhibitors, Chlorambucil, CI-994, Cisplatin, Cladribine, CS-682, Cytarabine HCl, D2163, Dactinomycin, Daunorubicin HCl, DepoCyt, Dexifosamide, Docetaxel, Dolastain, Doxifluridine, Doxorubicin, DX8951f, E 7070, EGFR, Epirubicin, Erythropoietin, Estramustine phosphate sodium, Etoposide (VP16-213), Farnesyl Transferase Inhibitor, FK 317, Flavopiridol, Floxuridine, Fludarabine, Fluorouracil (5-FU), Flutamide, Fragyline, Gemcitabine, Hexamethylmelamine (HMM), Hydroxyurea (hydroxycarbamide), Ifosfamide, Interferon Alfa-2a, Interferon Alfa-2b, Interleukin-2, Irinotecan, ISI 641, Krestin, Lemonal DP 2202, Leuprolide acetate (LHRH-releasing factor analogue), Levamisole, LiGLA (lithium-gamma linolenate), Lodine Seeds, Lometexol, Lomustine (CCNU), Marimistat, Mechlorethamine HCl (nitrogen mustard), Megestrol acetate, Meglamine GLA, Mercaptopurine, Mesna, Mitoguazone (methyl-GAG; methyl glyoxal bis-guanylhydrazone; MGBG), Mitotane (o.p'-DDD), Mitoxantrone, Mitoxantrone HCl, MMI 270, MMP, MTA/LY 231514, Octreotide, ODN 698, OK-432, Oral Platinum, Oral Taxoid, Paclitaxel (TAXOL.RTM.), PARP Inhibitors, PD 183805, Pentostatin (2' deoxycoformycin), PKC 412, Plicamycin, Procarbazine HCl, PSC 833, Ralitrexed, RAS Farnesyl Transferase Inhibitor, RAS Oncogene Inhibitor, Semustine (methyl-CCNU), Streptozocin, Suramin, Tamoxifen citrate, Taxane Analog, Temozolomide, Teniposide (VM-26), Thioguanine, Thiotepa, Topotecan, Tyrosine Kinase, UFT (Tegafur/Uracil), Valrubicin, Vinblastine sulfate, Vindesine sulfate, VX-710, VX-853, YM 116, ZD 0101, ZD 0473/Anormed, ZD 1839, ZD 9331.

Other agents may be used in combination with compositions of the disclosure may also include, but not limited to, agents that affect the upregulation of cell surface receptors and their ligands such as Fas/Fas ligand, DR4 or DR5/TRAIL and GAP junctions, cytostatic and differentiation agents, inhibitors of cell adhesion such as focal adhesion kinase (FAKs) inhibitors and Lovastatin, or agents that increase the sensitivity of the hyper proliferative cells to apoptotic inducers such as the antibody C225.

The combinations may include administering the compositions of the disclosure and other agents at the same time or separately. Alternatively, the present immunotherapy may precede or follow the other agent/therapy by intervals ranging from minutes, days, weeks to months.

### 5. Therapeutic Uses

Provided in the present disclosure is a method of reducing a tumor volume or burden in a subject in need, the method comprising introducing into the subject a composition of the disclosure.

Also provided herein are methods of increasing the survival in a subject with cancer. The methods may comprise introducing into the subject, any of the compositions described herein. The subject may be administered at least one stimulus described herein. In one instance the stimulus may be a ligand. In one instance, administration of the ligand may result in the modulation of the payload. Payloads such as but not limited to CARs when expressed in response to the ligand, may trigger an anti-cancer immune response. Such anti-cancer immune responses may result in reduction of tumor burden which in turn may increase the survival of the subject with cancer. Ligand may be administered prior to, during or after the introduction of the compositions into the subject. Survival may be measured and quantified using any of the methods known in the art. In some instance, survival may be quantified using Kaplan Meier analysis and/or the log rank test. In some instances, survival may be quantified using the survival ratio, which may be defined as the ratio of the median survival observed in a group to the median survival of the untransduced-vehicle treatment group. In some instances, the survival ratio is at least 1. In some instances, the survival ratio is at least 1.1, 1.2, 1.3, 1.4, 1.5, 1.6, 1.7 1.8, 1.9, 2, 3, 4, 5, 6, 7, 8, 9, or 10.

The present disclosure also provides methods for treating a cancer in a subject, comprising administering to the subject an effective amount of an immune effector cell genetically modified to express at least one effector module of the disclosure.

### Cancer

Various cancers may be treated with pharmaceutical compositions, biocircuits, biocircuit components, effector modules including their SREs or payloads of the present disclosure. As used herein, the term "cancer" refers to any of various malignant neoplasms characterized by the proliferation of anaplastic cells that tend to invade surrounding tissue and metastasize to new body sites and refers to the pathological condition characterized by such malignant neoplastic growths. Cancers may be tumors or hematological malignancies, and include but are not limited to, all types of lymphomas/leukemias, carcinomas and sarcomas, such as those cancers or tumors found in the anus, bladder, bile duct, bone, brain, breast, cervix, colon/rectum, endometrium, esophagus, eye, gallbladder, head and neck, liver, kidney, larynx, lung, mediastinum (chest), mouth, ovaries, pancreas, penis, prostate, skin, small intestine, stomach, spinal marrow, tailbone, testicles, thyroid and uterus.

Types of carcinomas which may be treated with the compositions of the present disclosure include, but are not limited to, papilloma/carcinoma, choriocarcinoma, endodermal sinus tumor, teratoma, adenoma/adenocarcinoma, melanoma, fibroma, lipoma, leiomyoma, rhabdomyoma, mesothelioma, angioma, osteoma, chondroma, glioma, lymphoma/leukemia, squamous cell carcinoma, small cell carcinoma, large cell undifferentiated carcinomas, basal cell carcinoma and sinonasal undifferentiated carcinoma.

Types of carcinomas which may be treated with the compositions of the present disclosure include, but are not limited to, soft tissue sarcoma such as alveolar soft part sarcoma, angiosarcoma, dermatofibrosarcoma, desmoid tumor, desmoplastic small round cell tumor, extraskeletal chondrosarcoma, extraskeletal osteosarcoma, fibrosarcoma, hemangiopericytoma, hemangiosarcoma, Kaposi's sarcoma, leiomyosarcoma, liposarcoma, lymphangiosarcoma, lymphosarcoma, malignant fibrous histiocytoma, neurofibrosarcoma, rhabdomyosarcoma, synovial sarcoma, and Askin's tumor, Ewing's sarcoma (primitive neuroectodermal tumor), malignant hemangioendothelioma, malignant schwannoma, osteosarcoma, and chondrosarcoma.

As a non-limiting example, the carcinoma which may be treated may be Acute granulocytic leukemia, Acute lymphocytic leukemia, Acute myelogenous leukemia, Adenocarcinoma, Adenosarcoma, Adrenal cancer, Adrenocortical carcinoma, Anal cancer, Anaplastic astrocytoma, Angiosarcoma, Appendix cancer, Astrocytoma, Basal cell carcinoma, B-Cell lymphoma), Bile duct cancer, Bladder cancer, Bone cancer, Bowel cancer, Brain cancer, Brain stem glioma, Brain tumor, Breast cancer, Carcinoid tumors, Cervical cancer, Cholangiocarcinoma, Chondrosarcoma, Chronic lymphocytic leukemia, Chronic myelogenous leukemia, Colon cancer, Colorectal cancer, Craniopharyngioma, Cutaneous lymphoma, Cutaneous melanoma, Diffuse astrocytoma, Ductal carcinoma in situ, Endometrial cancer, Ependymoma, Epithelioid sarcoma, Esophageal cancer, Ewing sarcoma, Extrahepatic bile duct cancer, Eye cancer, Fallopian tube cancer, Fibrosarcoma, Gallbladder cancer, Gastric cancer, Gastrointestinal cancer, Gastrointestinal carcinoid cancer, Gastrointestinal stromal tumors, General, Germ cell tumor, Glioblastoma multiforme, Glioma, Hairy cell leukemia, Head and neck cancer, Hemangioendothelioma, Hodgkin lymphoma, Hodgkin's disease, Hodgkin's lymphoma, Hypopharyngeal cancer, Infiltrating ductal carcinoma, Infiltrating lobular carcinoma, Inflammatory breast cancer, Intestinal Cancer, Intrahepatic bile duct cancer, Invasive / infiltrating breast cancer, Islet cell cancer, Jaw cancer, Kaposi sarcoma, Kidney cancer, Laryngeal cancer, Leiomyosarcoma, Leptomeningeal metastases, Leukemia, Lip cancer, Liposarcoma, Liver cancer, Lobular carcinoma in situ, Low-grade astrocytoma, Lung cancer, Lymph node cancer, Lymphoma, Male breast cancer, Medullary carcinoma, Medulloblastoma, Melanoma, Meningioma, Merkel cell carcinoma, Mesenchymal chondrosarcoma, Mesenchymous, Mesothelioma, Metastatic breast cancer, Metastatic melanoma, Metastatic squamous neck cancer, Mixed gliomas, Mouth cancer, Mucinous carcinoma, Mucosal melanoma, Multiple myeloma, Nasal cavity cancer, Nasopharyngeal cancer, Neck cancer, Neuroblastoma, Neuroendocrine tumors, Non-Hodgkin lymphoma, Non-Hodgkin's lymphoma, Non-small cell lung cancer, Oat cell cancer, Ocular cancer, Ocular melanoma, Oligodendroglioma, Oral cancer, Oral cavity cancer, Oropharyngeal cancer, Osteogenic sarcoma, Osteosarcoma, Ovarian cancer, Ovarian epithelial cancer, Ovarian germ cell tumor, Ovarian primary peritoneal carcinoma, Ovarian sex cord stromal tumor, Paget's disease, Pancreatic cancer, Papillary carcinoma, Paranasal sinus cancer, Parathyroid cancer, Pelvic cancer, Penile cancer, Peripheral nerve cancer, Peritoneal cancer, Pharyngeal cancer, Pheochromocytoma, Pilocytic astrocytoma, Pineal region tumor, Pineoblastoma, Pituitary gland cancer, Primary central nervous system lymphoma, Prostate cancer, Rectal cancer, Renal cell cancer, Renal pelvis cancer, Rhabdomyosarcoma, Salivary gland cancer, Sarcoma, Sarcoma, bone, Sarcoma, soft tissue, Sarcoma, uterine, Sinus cancer, Skin cancer, Small cell lung cancer, Small intestine cancer, Soft tissue sarcoma, Spinal cancer, Spinal column cancer, Spinal cord cancer, Spinal tumor, Squamous cell carcinoma, Stomach cancer, Synovial sarcoma, T-cell lymphoma), Testicular cancer, Throat cancer, Thymoma / thymic carcinoma, Thyroid cancer, Tongue cancer, Tonsil cancer, Transitional cell cancer, Transitional cell cancer, Transitional cell cancer, Triple-negative breast cancer, Tubal cancer, Tubular carcinoma, Ureteral cancer, Ureteral cancer, Urethral cancer, Uterine adenocarcinoma, Uterine cancer, Uterine sarcoma, Vaginal cancer, and Vulvar cancer.

In some instances, the CARs of the present disclosure may be a CAR useful in the treatment of multiple myeloma such as a CS1 CAR, a CD38 CAR, a CD138 CAR, and a BCMA CAR. In some instances, the CARs of the present disclosure may be a CAR useful in the treatment of acute myeloid leukemia such as a CD33 CAR, a CD123 CAR, and a CLL1 CAR. In some instances, the CARs of the present disclosure may be a CAR useful in the treatment of T cell leukemia such as a CD5 CAR, and a CD7 CAR. In some instances, the CARs of the present disclosure may be a CAR useful in the treatment of solid tumors such a mesothelin CAR, a GD2 CAR, a GPC3 CAR, a Her2 CAR, an EGFR CAR, a Muc1 CAR, an EpCAM CAR, a PD-L1 CAR, a CEA CAR, a Muc16 CAR, a CD133 CAR, a CD171 CAR, a CD70 CAR, a CLD18 CAR, a cMET CAR, a EphA2 CAR, a FAP CAR, a Folate Receptor CAR, an IL13Ra2 CAR, an MG7 CAR, a PSMA CAR, a ROR1 CAR, and a VEGFR2 CAR.

The present disclosure also provides methods of reducing tumor burden in a subject. In some instances. As used herein, "tumor burden" refers to the number of cancer cells, or the amount of cancer in a subject. In some aspects tumor burden also refers to tumor load. In some instances, the tumor may be disseminated throughout the body of the subject. In one aspect, the tumor may be a liquid tumor such as leukemia or a lymphoma. The methods of reducing tumor burden may involve administering to the subject, a therapeutically effective amount of the immune cells. Immune cells may be engineered to express the compositions described herein. In some instances, the immune cells expressing the compositions of the disclosure may be administered to the subject via any of the routes of delivery described herein. Also provided herein are dosing regimens for administering the immune cells. In some instances, the subject may also be administered a therapeutically effective amount of the stimulus to tune the expression of the immunotherapeutic agent. In some aspects, the immunotherapeutic agents may be capable of reducing the tumor burden. Regimens for ligand/ stimulus dosing are also provided. Reduction in tumor burden may be measured by any of the methods known in the art including tumor imaging, and measurement of marker proteins. In some aspects, bioluminescent imaging may be used to measure tumor burden. Bioluminescence imaging utilizes native light emission from bioluminescent proteins such as luciferase. Such bioluminescent proteins can participate in chemical reactions that release photons by the addition of suitable substrates. The release of photons can be captured by sensitive detection methods and quantified. Tumor cells may be engineered to express luciferase and the efficacy of the compositions described herein to reduce tumor burden may quantified by imaging. In some aspects, the tumor burden may be measured by the flux of photons (photons per sec). In some instances, photon flux positively correlates with tumor burden.

### Diseases and Toxins

Various infectious diseases may be treated with pharmaceutical compositions, biocircuits, biocircuit components, effector modules including their SREs or payloads of the present disclosure. As used herein, the term "infectious disease" refers to any disorders caused by organisms such as bacteria, viruses, fungi or parasites. As a non-limiting example, the infectious disease may be Acute bacterial rhinosinusitis, 14-day measles, Acne, Acrodermatitis chronica atrophicans (ACA)-(late skin manifestation of latent Lyme disease), Acute hemorrhagic conjunctivitis, Acute hemorrhagic cystitis, Acute rhinosinusitis, Adult T-cell Leukemia-Lymphoma (ATLL), African Sleeping Sickness, AIDS (Acquired Immunodeficiency Syndrome), Alveolar hydatid, Amebiasis, Amebic meningoencephalitis, Anaplasmosis, Anthrax, Arboviral or parainfectious, Ascariasis -(Roundworm infections), Aseptic meningitis, Athlete's foot (Tinea pedis), Australian tick typhus, Avian Influenza, Babesiosis, Bacillary angiomatosis, Bacterial meningitis, Bacterial vaginosis, Balanitis, Balantidiasis, Bang's disease, Barmah Forest virus infection, Bartonellosis (Verruga peruana; Carrion's disease; Oroya fever), Bat Lyssavirus Infection, Bay sore (Chiclero's ulcer), Baylisascaris infection (Racoon roundworm infection), Beaver fever, Beef tapeworm, Bejel (endemic syphilis), Biphasic meningoencephalitis, Black Bane, Black death, Black piedra, Blackwater Fever, Blastomycosis, Blennorrhea of the newborn, Blepharitis, Boils, Bornholm disease (pleurodynia), Borrelia miyamotoi Disease, Botulism, Boutonneuse fever, Brazilian purpuric fever, Break Bone fever, Brill, Bronchiolitis, Bronchitis, Brucellosis (Bang's disease), Bubonic plague, Bullous impetigo, Burkholderia mallei (Glanders), Burkholderia pseudomallei (Melioidosis), Buruli ulcers (also Mycoburuli ulcers), Busse, Busse-Buschke disease (Cryptococcosis), California group encephalitis, Campylobacteriosis, Candidiasis, Canefield fever (Canicola fever; 7-day fever; Weil's disease; leptospirosis; canefield fever), Canicola fever, Capillariasis, Carate, Carbapenem-resistant Enterobacteriaceae (CRE), Carbuncle, Carrion's disease, Cat Scratch fever, Cave disease, Central Asian hemorrhagic fever, Central European tick, Cervical cancer, Chagas disease, Chancroid (Soft chancre), Chicago disease, Chickenpox (Varicella), Chiclero's ulcer, Chikungunya fever, Chlamydial infection, Cholera, Chromoblastomycosis, Ciguatera, Clap, Clonorchiasis (Liver fluke infection), Clostridium Difficile Infection, Clostridium Perfringens (Epsilon Toxin), Coccidioidomycosis fungal infection (Valley fever; desert rheumatism), Coenurosis, Colorado tick fever, Condyloma accuminata, Condyloma accuminata( Warts), Condyloma lata, Congo fever, Congo hemorrhagic fever virus, Conjunctivitis, cowpox, Crabs, Crimean, Croup, Cryptococcosis, Cryptosporidiosis (Crypto), Cutaneous Larval Migrans, Cyclosporiasis, Cystic hydatid, Cysticercosis, Cystitis, Czechoslovak tick, D68 (EV-D68), Dacryocytitis, Dandy fever, Darling's Disease, Deer fly fever, Dengue fever (1, 2, 3 and 4), Desert rheumatism, Devil's grip, Diphasic milk fever, Diphtheria, Disseminated Intravascular Coagulation, Dog tapeworm, Donovanosis, Donovanosis (Granuloma inguinale), Dracontiasis, Dracunculosis, Duke's disease, Dum Dum Disease, Durand-Nicholas-Favre disease, Dwarf tapeworm, E. Coli infection (*E.coli*)*,* Eastern equine encephalitis, Ebola Hemorrhagic Fever (Ebola virus disease EVD), Ectothrix, Ehrlichiosis (Sennetsu fever), Encephalitis, Endemic Relapsing fever, Endemic syphilis, Endophthalmitis, Endothrix, Enterobiasis (Pinworm infection), Enterotoxin - B Poisoning (Staph Food Poisoning), Enterovirus Infection, Epidemic Keratoconjunctivitis, Epidemic Relapsing fever, Epidemic typhus, Epiglottitis, Ery sipelis, Erysipeloid (Erysipelothricosis), Erythema chronicum migrans, Erythema infectiosum, Erythema marginatum, Erythema multiforme, Erythema nodosum, Erythema nodosum leprosum, Erythrasma, Espundia, Eumycotic mycetoma, European blastomycosis, Exanthem subitum (Sixth disease), Eyeworm, Far Eastern tick, Fascioliasis, Fievre boutonneuse( Tick typhus), Fifth Disease (erythema infectiosum), Filatow-Dukes' Disease (Scalded Skin Syndrome; Ritter's Disease), Fish tapeworm, Fitz-Hugh-Curtis syndrome - Perihepatitis, Flinders Island Spotted Fever, Flu (Influenza), Folliculitis, Four Corners Disease, Four Corners Disease (Human Pulmonary Syndrome (HPS)), Frambesia, Francis disease, Furunculosis, Gas gangrene, Gastroenteritis, Genital Herpes, Genital Warts, German measles, Gerstmann-Straussler-Scheinker (GSS), Giardiasis, Gilchrist's disease, Gingivitis, Gingivostomatitis, Glanders, Glandular fever (infectious mononucleosis), Gnathostomiasis, Gonococcal Infection (Gonorrhea), Gonorrhea, Granuloma inguinale (Donovanosis), Guinea Worm, Haemophilus Influenza disease, Hamburger disease, Hansen's disease - leprosy, Hantaan disease, Hantaan-Korean hemorrhagic fever, Hantavirus Pulmonary Syndrome, Hantavirus Pulmonary Syndrome (HPS), Hard chancre, Hard measles, Haverhill fever - Rat bite fever, Head and Body Lice, Heartland fever, Helicobacterosis, Hemolytic Uremic Syndrome (HUS), Hepatitis A, Hepatitis B, Hepatitis C, Hepatitis D, Hepatitis E, Herpangina, Herpes- genital, Herpes labialis, Herpes- neonatal, Hidradenitis, Histoplasmosis, Histoplasmosis infection (Histoplasmosis), His-Werner disease, HIV infection, Hookworm infections, Hordeola, Hordeola (Stye), HTLV, HTLV- associated myelopathy (HAM), Human granulocytic ehrlichiosis, Human monocytic ehrlichiosis, Human Papillomavirus (HPV), Human Pulmonary Syndrome, Hydatid cyst, Hydrophobia, Impetigo, Including congenital (German Measles), Inclusion conjunctivitis, Inclusion conjunctivitis - Swimming Pool conjunctivitis- Pannus, Infantile diarrhea, Infectious Mononucleosis, Infectious myocarditis, Infectious pericarditis, Influenza, Isosporiasis, Israeli spotted fever, Japanese Encephalitis, Jock itch, Jorge Lobo disease - lobomycosis, Jungle yellow fever, Junin Argentinian hemorrhagic fever, Kala Azar, Kaposi's sarcoma, Keloidal blastomycosis, Keratoconjunctivitis, Kuru, Kyasanur forest disease, LaCrosse encephalitis, Lassa hemorrhagic fever, Legionellosis (Legionnaires Disease), Legionnaire's pneumonia, Lemierre's Syndrome (Postanginal septicemia), Lemming fever, Leprosy, Leptospirosis (Nanukayami fever; Weil's disease), Listeriosis (Listeria), Liver fluke infection, Lobo's mycosis, Lockjaw, Loiasis, Louping Ill, Ludwig's angina, Lung fluke infection, Lung fluke infection (Paragonimiasis), Lyme disease, Lymphogranuloma venereum infection (LGV), Machupo Bolivian hemorrhagic fever, Madura foot, Mal del pinto, Malaria, Malignant pustule, Malta fever, Marburg hemorrhagic fever, Masters disease, Maternal Sepsis (Puerperal fever), Measles, Mediterranean spotted fever, Melioidosis (Whitmore's disease), Meningitis, Meningococcal Disease, MERS, Milker's nodule, Molluscum contagiosum, Moniliasis, monkeypox, Mononucleosis, Mononucleosis-like syndrome, Montezuma's Revenge, Morbilli, MRSA (methicillin-resistant *Staphylococcus aureus*) infection, Mucormycosis- Zygomycosis, Multiple Organ Dysfunction Syndrome or MODS, Multiple-system atrophy (MSA), Mumps, Murine typhus, Murray Valley Encephalitis(MVE), Mycoburuli ulcers, Mycoburuli ulcers- Buruli ulcers, Mycotic vulvovaginitis, Myositis, Nanukayami fever, Necrotizing fasciitis, Necrotizing fasciitis- Type 1, Necrotizing fasciitis- Type 2, Negishi, New world spotted fever, Nocardiosis, Nongonococcal urethritis, Non-Polio (Non-Polio Enterovirus), Norovirus infection, North American blastomycosis, North Asian tick typhus, Norwalk virus infection, Norwegian itch, O'Hara disease, Omsk hemorrhagic fever, Onchoceriasis, Onychomycosis, Opisthorchiasis, Opthalmia neonatorium, Oral hairy leukoplakia, Orf, Oriental Sore, Oriental Spotted Fever, Ornithosis (Parrot fever; Psittacosis), Oroya fever, Otitis externa, Otitis media, Pannus, Paracoccidioidomycosis, Paragonimiasis, Paralytic Shellfish Poisoning (Paralytic Shellfish Poisoning), Paronychia (Whitlow), Parotitis, PCP pneumonia, Pediculosis, Peliosis hepatica, Pelvic Inflammatory Disease, Pertussis (also called Whooping cough), Phaeohyphomycosis, Pharyngoconjunctival fever, Piedra (White Piedra), Piedra(Black Piedra), Pigbel, Pink eye conjunctivitis, Pinta, Pinworm infection, Pitted Keratolysis, Pityriasis versicolor (Tinea versicolor), Plague; Bubonic, Pleurodynia, Pneumococcal Disease, Pneumocystosis, Pneumonia, Pneumonic (Plague), Polio or Poliomyelitis, Polycystic hydatid, Pontiac fever, Pork tapeworm, Posada-Wernicke disease, Postanginal septicemia, Powassan, Progressive multifocal leukoencephalopathy, Progressive Rubella Panencephalitis, Prostatitis, Pseudomembranous colitis, Psittacosis, Puerperal fever, Pustular Rash diseases (Small pox), Pyelonephritis, Pylephlebitis, Q-Fever, Quinsy, Quintana fever (5-day fever), Rabbit fever, Rabies, Racoon roundworm infection, Rat bite fever, Rat tapeworm, Reiter Syndrome, Relapsing fever, Respiratory syncytial virus (RSV) infection, Rheumatic fever, Rhodotorulosis, Ricin Poisoning, Rickettsialpox, Rickettsiosis, Rift Valley Fever, Ringworm, Ritter's Disease, River Blindness, Rocky Mountain spotted fever, Rose Handler's disease (Sporotrichosis), Rose rash of infants, Roseola, Ross River fever, Rotavirus infection, Roundworm infections, Rubella, Rubeola, Russian spring, Salmonellosis gastroenteritis, San Joaquin Valley fever, Sao Paulo Encephalitis, Sao Paulo fever, SARS, Scabies Infestation (Scabies) (Norwegian itch), Scalded Skin Syndrome, Scarlet fever (Scarlatina), Schistosomiasis, Scombroid, Scrub typhus, Sennetsu fever, Sepsis (Septic shock), Severe Acute Respiratory Syndrome, Severe Acute Respiratory Syndrome (SARS), Shiga Toxigenic Escherichia coli (STEC/VTEC), Shigellosis gastroenteritis (Shigella), Shinbone fever, Shingles, Shipping fever, Siberian tick typhus, Sinusitis, Sixth disease, Slapped cheek disease, Sleeping sickness, Smallpox (Variola), Snail Fever, Soft chancre, Southern tick associated rash illness, Sparganosis, Spelunker's disease, Sporadic typhus, Sporotrichosis, Spotted fever, Spring, St. Louis encephalitis, Staphylococcal Food Poisoning, Staphylococcal Infection, Strep. throat, Streptococcal Disease, Streptococcal Toxic-Shock Syndrome, Strongyloiciasis, Stye, Subacute Sclerosing Panencephalitis, Subacute Sclerosing Panencephalitis (SSPE), Sudden Acute Respiratory Syndrome, Sudden Rash, Swimmer's ear, Swimmer's Itch, Swimming Pool conjunctivitis, Sylvatic yellow fever, Syphilis, Systemic Inflammatory Response Syndrome (SIRS), Tabes dorsalis (tertiary syphilis), Taeniasis, Taiga encephalitis, Tanner's disease, Tapeworm infections, Temporal lobe encephalitis, Temporal lobe encephalitis, tetani (Lock Jaw), Tetanus Infection, Threadworm infections, Thrush, Tick, Tick typhus, *Tinea barbae, Tinea capitis, Tinea corporis, Tinea cruris, Tinea manuum, Tinea nigra, Tinea pedis, Tinea unguium, Tinea versicolor,* Torulopsosis, Torulosis, Toxic Shock Syndrome, Toxoplasmosis, transmissible spongioform (CJD), Traveler's diarrhea, Trench fever 5, Trichinellosis, Trichomoniasis, Trichomycosis axillaris, Trichuriasis, Tropical Spastic Paraparesis (TSP), Trypanosomiasis, Tuberculosis (TB), Tuberculosis, Tularemia, Typhoid Fever, Typhus fever, Ulcus molle, Undulant fever, Urban yellow fever, Urethritis, Vaginitis, Vaginosis, Vancomycin Intermediate (VISA), Vancomycin Resistant (VRSA), Varicella, Venezuelan Equine encephalitis, *Verruga pervana, Vibrio cholerae* (Cholera), Vibriosis (Vibrio), Vincent's disease or Trench mouth, Viral conjunctivitis, Viral Meningitis, Viral meningoencephalitis, Viral rash, Visceral Larval Migrans, Vomito negro, Vulvovaginitis, Warts, Waterhouse, Weil's disease, West Nile Fever, Western equine encephalitis, Whipple's disease, Whipworm infection, White Piedra, Whitlow, Whitmore's disease, Winter diarrhea, Wolhynia fever, Wool sorters' disease, Yaws, Yellow Fever, Yersinosis, Yersinosis (Yersinia), Zahorsky's disease, Zika virus disease, Zoster, Zygomycosis, John Cunningham Virus (JCV), Human immunodeficiency virus (HIV), Influenza virus, Hepatitis B, Hepatitis C, Hepatitis D, Respiratory syncytial virus (RSV), Herpes simplex virus 1 and 2, Human Cytomegalovirus, Epstein-Barr virus , Varicella zoster virus, Coronaviruses , Poxviruses, Enterovirus 71, Rubella virus, Human papilloma virus, *Streptococcus pneumoniae, Streptococcus viridans, Staphylococcus aureus (S. aureus),* Methicillin-resistant *Staphylococcus aureus* (MRSA), Vancomycin-intermediate *Staphylococcus aureus* (VISA), Vancomycin-resistant *Staphylococcus aureus* (VRSA), *Staphylococcus epidermidis (S. epidermidis), Clostridium Tetani, Bordetella pertussis, Bordetella paratussis, Mycobacterium, Francisella tularensis, Toxoplasma gondii,* Candida (*C. albicans, C. glabrata, C. parapsilosis, C. tropicalis, C. krusei and C. lusitaniae*) and/or any other infectious diseases, disorders or syndromes.

Various toxins may be treated with pharmaceutical compositions, biocircuits, biocircuit components, effector modules including their SREs or payloads of the present disclosure. Non-limited examples of toxins include Ricin, Bacillus anthracis, Shiga toxin and Shiga-like toxin, Botulinum toxins.

Various tropical diseases may be treated with pharmaceutical compositions, biocircuits, biocircuit components, effector modules including their SREs or payloads of the present disclosure. Non-limited examples of tropical diseases include Chikungunya fever, Dengue fever, Chagas disease, Rabies, Malaria, Ebola virus, Marburg virus, West Nile Virus, Yellow Fever, Japanese encephalitis virus, St. Louis encephalitis virus.

Various foodborne illnesses and gastroenteritis may be treated with pharmaceutical compositions, biocircuits, biocircuit components, effector modules including their SREs or payloads of the present disclosure. Non-limited examples of foodborne illnesses and gastroenteritis include Rotavirus, Norwalk virus (Norovirus), *Campylobacter jejuni, Clostridium difficile, Entamoeba histolytica, Helicobacter pylori,* Enterotoxin B of Staphylococcus aureus, Hepatitis A virus (HAV), Hepatitis E, Listeria monocytogenes, Salmonella, Clostridium perfringens, and Salmonella.

Various infectious agents may be treated with pharmaceutical compositions, biocircuits, biocircuit components, effector modules including their SREs or payloads of the present disclosure. Non-limited examples of infectious agents include adenoviruses, *Anaplasma phagocytophilium, Ascaris lumbricoides, Bacillus anthracis, Bacillus cereus, Bacteriodes* sp, Barmah Forest virus, *Bartonella bacilliformis, Bartonella henselae, Bartonella quintana,* beta-toxin of *Clostridium perfringens, Bordetella pertussis, Bordetella parapertussis, Borrelia burgdorferi, Borrelia miyamotoi, Borrelia recurrentis, Borrelia* sp., *Botulinum* toxin, *Brucella* sp., *Burkholderia pseudomallei,* California encephalitis virus, *Campylobacter, Candida albicans,* chikungunya virus, *Chlamydia psittaci, Chlamydia trachomatis, Clonorchis sinensis, Clostridium difficile bacteria, Clostridium tetani,* Colorado tick fever virus, *Corynebacterium diphtheriae, Corynebacterium minutissimum, Coxiella burnetii,* coxsackie A, coxsackie B, Crimean-Congo hemorrhagic fever virus, cytomegalovirus, dengue virus, Eastern Equine encephalitis virus, Ebola viruses, echovirus, *Ehrlichia chaffeensis, Ehrlichia equi, Ehrlichia* sp., *Entamoeba histolytica, Enterobacter sp., Enterococcus faecalis,* Enterovirus 71, Epstein-Barr virus (EBV), *Erysipelothrix rhusiopathiae, Escherichia coli,* Flavivirus, *Fusobacterium necrophorum, Gardnerella vaginalis,* Group B *streptococcus, Haemophilus aegyptius, Haemophilus ducreyi, Haemophilus influenzae,* hantavirus, *Helicobacter pylori,* Hepatitis A, Hepatitis B, Hepatitis C, Hepatitis D, Hepatitis E, herpes simplex virus 1 and 2,, human herpes virus 6, human herpes Virus 8, human immunodeficiency virus 1 and 2, human T-cell leukemia viruses I and II, influenza viruses (A, B, C), Jamestown Canyon virus, Japanese encephalitis antigenic, Japanese encephalitis virus, John Cunninham virus, juninvirus, Kaposi's Sarcoma-associated Herpes Virus (KSHV), *Klebsiella granulomatis, Klebsiella sp.,* Kyasanur Forest Disease virus, La Crosse virus, Lassavirus, *Legionella pneumophila, Leptospira interrogans, Listeria monocytogenes,* lymphocytic choriomeningitis virus, lyssavirus, Machupovirus, Marburg virus, measles virus, MERS coronavirus (MERS-CoV), *Micrococcus sedentarius, Mobiluncus sp.,* Molluscipoxvirus, *Moraxella catarrhalis,* Morbilli- Rubeola virus, Mumps virus, *Mycobacterium leprae, Mycobacterium tuberculosis, Mycobacterium ulcerans, Mycoplasma genitalium, Mycoplasma* sp, Nairovirus, *Neisseria gonorrhoeae, Neisseria meningitidis,* Nocardia, Norwalk virus, norovirus, Omsk hemorrhagic fever virus, papilloma virus, parainfluenza viruses 1-3, parapoxvirus, parvovirus B19, *Peptostreptococccus* sp., *Plasmodium* sp., polioviruses types I, II, and III, *Proteus* sp., *Pseudomonas aeruginosa, Pseudomonas pseudomallei, Pseudomonas* sp., rabies virus, respiratory syncytial virus, ricin toxin, *Rickettsia australis, Rickettsia conori, Rickettsia honei, Rickettsia prowazekii,* Ross River Virus, rotavirus, rubellavirus, Saint Louis encephalitis, *Salmonella typhi, Sarcoptes scabiei,* SARS-associated coronavirus (SARS-CoV), *Serratia sp.,* Shiga toxin and Shiga-like toxin, *Shigella* sp., Sin Nombre Virus, Snowshoe hare virus, *Staphylococcus aureus, Staphylococcus epidermidis, Streptobacillus moniliformis, Streptococcus pneumoniae, Streptococcus agalactiae, Streptococcus agalactiae, Streptococcus* group A-H, *Streptococcus pneumoniae, Streptococcus pyogenes, Treponema pallidum subsp. Pallidum, Treponema pallidum var. carateum, Treponema pallidum var. endemicum, Tropheryma whippelii, Ureaplasma urealyticum,* Varicella-Zoster virus, variola virus, *Vibrio cholerae,* West Nile virus, yellow fever virus, *Yersinia enterocolitica, Yersinia pestis,* and Zika virus.

Various rare diseases may be treated with pharmaceutical compositions, biocircuits, biocircuit components, effector modules including their SREs or payloads of the present disclosure. As used herein, the term "rare disease" refers to any disease that affects a small percentage of the population. As a non-limiting example, the rare disease may be Acrocephalosyndactylia, Acrodermatitis, Addison Disease, Adie Syndrome, Alagille Syndrome, Amylose, Amyotrophic Lateral Sclerosis, Angelman Syndrome, Angiolymphoid Hyperplasia with Eosinophilia, Arnold-Chiari Malformation, Arthritis, Juvenile Rheumatoid, Asperger Syndrome, Bardet-Biedl Syndrome, Barrett Esophagus, Beckwith-Wiedemann Syndrome, Behcet Syndrome, Bloom Syndrome, Bowen's Disease, Brachial Plexus Neuropathies, Brown-Sequard Syndrome, Budd-Chiari Syndrome, Burkitt Lymphoma, Carcinoma 256, Walker, Caroli Disease, Charcot-Marie-Tooth Disease, Chediak-Higashi Syndrome, Chiari-Frommel Syndrome, Chondrodysplasia Punctata, Colonic Pseudo-Obstruction, Colorectal Neoplasms, Hereditary Nonpolyposis, Craniofacial Dysostosis, Creutzfeldt-Jakob Syndrome, Crohn Disease, Cushing Syndrome, Cystic Fibrosis, Dandy-Walker Syndrome, De Lange Syndrome, Dementia, Vascular, Dermatitis Herpetiformis, DiGeorge Syndrome, Diffuse Cerebral Sclerosis of Schilder, Duane Retraction Syndrome, Dupuytren Contracture, Ebstein Anomaly, Eisenmenger Complex, Ellis-Van Creveld Syndrome, Encephalitis, Enchondromatosis, Epidermal Necrolysis, Toxic, Facial Hemiatrophy, Factor XII Deficiency, Fanconi Anemia, Felty's Syndrome, Fibrous Dysplasia, Polyostotic, Fox-Fordyce Disease, Friedreich Ataxia, Fusobacterium, Gardner Syndrome, Gaucher Disease, Gerstmann Syndrome, Giant Lymph Node Hyperplasia, Glycogen Storage Disease Type I, Glycogen Storage Disease Type II, Glycogen Storage Disease Type IV, Glycogen Storage Disease Type V, Glycogen Storage Disease Type VII, Goldenhar Syndrome, Guillain-Barre Syndrome, Hallermann's Syndrome, Hamartoma Syndrome, Multiple, Hartnup Disease, Hepatolenticular Degeneration, Hepatolenticular Degeneration, Hereditary Sensory and Motor Neuropathy, Hirschsprung Disease, Histiocytic Necrotizing Lymphadenitis, Histiocytosis, Langerhans-Cell, Hodgkin Disease, Horner Syndrome, Huntington Disease, Hyperaldosteronism, Hyperhidrosis, Hyperostosis, Diffuse Idiopathic Skeletal, Hypopituitarism, Inappropriate ADH Syndrome, Intestinal Polyps, Isaacs Syndrome, Kartagener Syndrome, Kearns-Sayre Syndrome, Klippel-Feil Syndrome, Klippel-Trenaunay-Weber Syndrome, Kluver-Bucy Syndrome, Korsakoff Syndrome, Lafora Disease, Lambert-Eaton Myasthenic Syndrome, Landau-Kleffner Syndrome, Langer-Giedion Syndrome, Leigh Disease, Lesch-Nyhan Syndrome, Leukodystrophy, Globoid Cell, Li-Fraumeni Syndrome, Long QT Syndrome, Machado-Joseph Disease, Mallory-Weiss Syndrome, Marek Disease, Marfan Syndrome, Meckel Diverticulum, Meige Syndrome, Melkersson-Rosenthal Syndrome, Meniere Disease, Mikulicz's Disease, Miller Fisher Syndrome, Mobius Syndrome, Moyamoya Disease, Mucocutaneous Lymph Node Syndrome, Mucopolysaccharidosis I, Mucopolysaccharidosis II, Mucopolysaccharidosis III, Mucopolysaccharidosis IV, Mucopolysaccharidosis VI, Multiple Endocrine Neoplasia Type 1, Munchausen Syndrome by Proxy, Muscular Atrophy, Spinal, Narcolepsy, Neuroaxonal Dystrophies, Neuromyelitis Optica, Neuronal Ceroid-Lipofuscinoses, Niemann-Pick Diseases, Noonan Syndrome, Optic Atrophies, Hereditary, Osteitis Deformans, Osteochondritis, Osteochondrodysplasias, Osteolysis, Essential, Paget Disease Extramammary, Paget's Disease, Mammary, Panniculitis, Nodular Nonsuppurative, Papillon-Lefevre Disease, Paralysis, Pelizaeus-Merzbacher Disease, Pemphigus, Benign Familial, Penile Induration, Pericarditis, Constrictive, Peroxisomal Disorders, Peutz-Jeghers Syndrome, Pick Disease of the Brain, Pierre Robin Syndrome, Pigmentation Disorders, Pityriasis Lichenoides, Polycystic Ovary Syndrome, Polyendocrinopathies, Autoimmune, Prader-Willi Syndrome, Pupil Disorders, Rett Syndrome, Reye Syndrome, Rubinstein-Taybi Syndrome, Sandhoff Disease, Sarcoma, Ewing's, Schnitzler Syndrome, Sjogren's Syndrome, Sjogren-Larsson Syndrome, Smith-Lemli-Opitz Syndrome, Spinal Muscular Atrophies of Childhood, Sturge-Weber Syndrome, Sweating, Gustatory, Takayasu Arteritis, Tangier Disease, Tay-Sachs Disease, Thromboangiitis Obliterans, Thyroiditis, Autoimmune, Tietze's Syndrome, Togaviridae Infections, Tolosa-Hunt Syndrome, Tourette Syndrome, Uveomeningoencephalitic Syndrome, Waardenburg's Syndrome, Wegener Granulomatosis, Weil Disease, Werner Syndrome, Williams Syndrome, Wilms Tumor, Wolff-Parkinson-White Syndrome, Wolfram Syndrome, Wolman Disease, Zellweger Syndrome, Zollinger-Ellison Syndrome, and von Willebrand Diseases.

Various autoimmune diseases and autoimmune-related diseases may be treated with pharmaceutical compositions, biocircuits, biocircuit components, effector modules including their SREs or payloads of the present disclosure. As used herein, the term "autoimmune disease" refers to a disease in which the body produces antibodies that attack its own tissues. As a non-limiting example, the autoimmune disease may be Acute Disseminated Encephalomyelitis (ADEM), Acute necrotizing hemorrhagic leukoencephalitis, Addison's disease, Agammaglobulinemia, Alopecia areata, Amyloidosis, Ankylosing spondylitis, Anti-GBM/Anti-TBM nephritis, Antiphospholipid syndrome (APS), Autoimmune angioedema, Autoimmune aplastic anemia, Autoimmune dysautonomia, Autoimmune hepatitis, Autoimmune hyperlipidemia, Autoimmune immunodeficiency, Autoimmune inner ear disease (AIED), Autoimmune myocarditis, Autoimmune oophoritis, Autoimmune pancreatitis, Autoimmune retinopathy, Autoimmune thrombocytopenic purpura (ATP), Autoimmune thyroid disease, Autoimmune urticaria, Axonal & neuronal neuropathies, Balo disease, Behcet's disease, Bullous pemphigoid, Cardiomyopathy, Castleman disease, Celiac disease, Chagas disease, Chronic fatigue syndrome, Chronic inflammatory demyelinating polyneuropathy (CIDP), Chronic recurrent multifocal ostomyelitis (CRMO), Churg-Strauss syndrome, Cicatricial pemphigoid/benign mucosal pemphigoid, Crohn's disease, Cogans syndrome, Cold agglutinin disease, Congenital heart block, Coxsackie myocarditis, CREST disease, Essential mixed cryoglobulinemia, Demyelinating neuropathies, Dermatitis herpetiformis, Dermatomyositis, Devic's disease (neuromyelitis optica), Discoid lupus, Dressler's syndrome, Endometriosis, Eosinophilic esophagitis, Eosinophilic fasciitis, Erythema nodosum, Experimental allergic encephalomyelitis, Evans syndrome, Fibromyalgia**, Fibrosing alveolitis, Giant cell arteritis (temporal arteritis), Giant cell myocarditis, Glomerulonephritis, Goodpasture's syndrome, Granulomatosis with Polyangiitis (GPA) (formerly called Wegener's Granulomatosis), Graves' disease, Guillain-Barre syndrome, Hashimoto's encephalitis, Hashimoto's thyroiditis, Hemolytic anemia, Henoch-Schonlein purpura, Herpes gestationis, Hypogammaglobulinemia, Idiopathic thrombocytopenic purpura (ITP), IgA nephropathy, IgG4-related sclerosing disease, Immunoregulatory lipoproteins, Inclusion body myositis, Interstitial cystitis, Juvenile arthritis, Juvenile diabetes (Type 1 diabetes), Juvenile myositis, Kawasaki syndrome, Lambert-Eaton syndrome, Leukocytoclastic vasculitis, Lichen planus, Lichen sclerosis, Ligneous conjunctivitis, Linear IgA disease (LAD), Lupus (SLE), Lyme disease, chronic, Meniere's disease, Microscopic polyangiitis, Mixed connective tissue disease (MCTD), Mooren's ulcer, Mucha-Habermann disease, Multiple sclerosis, Myasthenia gravis, Myositis, Narcolepsy, Neuromyelitis optica (Devic's), Neutropenia, Ocular cicatricial pemphigoid, Optic neuritis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria (PNH), Parry Romberg syndrome, Parsonnage-Turner syndrome, Pars planitis (peripheral uveitis), Pemphigus, Peripheral neuropathy, Perivenous encephalomyelitis, Pernicious anemia, POEMS syndrome, Polyarteritis nodosa, Type I, II, & III autoimmune polyglandular syndromes, Polymyalgia rheumatica, Polymyositis, Postmyocardial infarction syndrome, Postpericardiotomy syndrome, Progesterone dermatitis, Primary biliary cirrhosis, Primary sclerosing cholangitis, Psoriasis, Psoriatic arthritis, Idiopathic pulmonary fibrosis, Pyoderma gangrenosum, Pure red cell aplasia, Raynauds phenomenon, Reactive Arthritis, Reflex sympathetic dystrophy, Reiter's syndrome, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Sjogren's syndrome, Sperm & testicular autoimmunity, Stiff person syndrome, Subacute bacterial endocarditis (SBE), Susac's syndrome, Sympathetic ophthalmia, Takayasu's arteritis, Temporal arteritis/Giant cell arteritis, Thrombocytopenic purpura (TTP), Tolosa-Hunt syndrome, Transverse myelitis, Ulcerative colitis, Undifferentiated connective tissue disease (UCTD), Uveitis, Vasculitis, Vesiculobullous dermatosis, Vitiligo, and Wegener's granulomatosis (now termed Granulomatosis with Polyangiitis (GPA).

Various kidney diseases may be treated with pharmaceutical compositions, biocircuits, biocircuit components, effector modules including their SREs or payloads of the present disclosure. As a non-limiting example, the kidney disease Abderhalden-Kaufmann-Lignac syndrome (Nephropathic Cystinosis), Abdominal Compartment Syndrome, Acute Kidney Failure/Acute Kidney Injury, Acute Lobar Nephronia, Acute Phosphate Nephropathy, Acute Tubular Necrosis, Adenine Phosphoribosyltransferase Deficiency, Adenovirus Nephritis, Alport Syndrome, Amyloidosis, ANCA Vasculitis Related to Endocarditis and Other Infections, Angiomyolipoma, Analgesic Nephropathy, Anorexia Nervosa and Kidney Disease, Angiotensin Antibodies and Focal Segmental Glomerulosclerosis, Antiphospholipid Syndrome, Anti-TNF-α Therapy-related Glomerulonephritis, APOL1 Mutations, Apparent Mineralocorticoid Excess Syndrome, Aristolochic Acid Nephropathy, Chinese Herbal Nephropathy, Balkan Endemic Nephropathy, Bartter Syndrome, Beeturia, β-Thalassemia Renal Disease, Bile Cast Nephropathy, BK Polyoma Virus Nephropathy in the Native Kidney, Bladder Rupture, Bladder Sphincter Dyssynergia, Bladder Tamponade, Border-Crossers' Nephropathy, Bourbon Virus and Acute Kidney Injury, Burnt Sugarcane Harvesting and Acute Renal Dysfunction, Byetta and Renal Failure, C1q Nephropathy, Cannabinoid Hyperemesis Acute Renal Failure, Cardiorenal syndrome, Carfilzomib-Indiced Renal Injury, CFHR5 nephropathy, Charcot-Marie-Tooth Disease with Glomerulopathy, Cherry Concentrate and Acute Kidney Injury, Cholesterol Emboli, Churg-Strauss syndrome, Chyluria, Colistin Nephrotoxicity, Collagenofibrotic Glomerulopathy, Collapsing Glomerulopathy, Collapsing Glomerulopathy Related to CMV, Congenital Nephrotic Syndrome, Conorenal syndrome (Mainzer-Saldino Syndrome or Saldino-Mainzer Disease), Contrast Nephropathy, Copper Sulfate Intoxication, Cortical Necrosis, Crizotinib-related Acute Kidney Injury, Cryoglobuinemia, Crystalglobulin-Induced Nephropathy, Crystal-Induced Acute Kidney injury, Cystic Kidney Disease, Acquired, Cystinuria, Dasatinib-Induced Nephrotic-Range Proteinuria, Dense Deposit Disease (MPGN Type 2), Dent Disease (X-linked Recessive Nephrolithiasis), Dialysis Disequilibrium Syndrome, Diabetes and Diabetic Kidney Disease, Diabetes Insipidus, Dietary Supplements and Renal Failure, Drugs of Abuse and Kidney Disease, Duplicated Ureter, EAST syndrome, Ebola and the Kidney, Ectopic Kidney, Ectopic Ureter, Edema, Swelling, Erdheim-Chester Disease, Fabry's Disease, Familial Hypocalciuric Hypercalcemia, Fanconi Syndrome, Fraser syndrome, Fibronectin Glomerulopathy, Fibrillary Glomerulonephritis and Immunotactoid Glomerulopathy, Fraley syndrome, Focal Segmental Glomerulosclerosis, Focal Sclerosis, Focal Glomerulosclerosis, Galloway Mowat syndrome, Giant Cell (Temporal) Arteritis with Kidney Involvement, Gestational Hypertension, Gitelman Syndrome, Glomerular Diseases, Glomerular Tubular Reflux, Glycosuria, Goodpasture Syndrome, Hair Dye Ingestion and Acute Kidney Injury, Hantavirus Infection Podocytopathy, Hematuria (Blood in Urine), Hemolytic Uremic Syndrome (HUS), Atypical Hemolytic Uremic Syndrome (aHUS), Hemophagocytic Syndrome, Hemorrhagic Cystitis, Hemorrhagic Fever with Renal Syndrome (HFRS, Hantavirus Renal Disease, Korean Hemorrhagic Fever, Epidemic Hemorrhagic Fever, Nephropathis Epidemica), Hemosiderosis related to Paroxysmal Nocturnal Hemoglobinuria and Hemolytic Anemia, Hepatic Glomerulopathy, Hepatic Veno-Occlusive Disease, Sinusoidal Obstruction Syndrome, Hepatitis C-Associated Renal Disease, Hepatorenal Syndrome, Herbal Supplements and Kidney Disease, High Blood Pressure and Kidney Disease, HIV-Associated Nephropathy (HIVAN), Horseshoe Kidney (Renal Fusion), Hunner's Ulcer, Hyperaldosteronism, Hypercalcemia, Hyperkalemia, Hypermagnesemia, Hypernatremia, Hyperoxaluria, Hyperphosphatemia, Hypocalcemia, Hypokalemia, Hypokalemia-induced renal dysfunction, Hypokalemic Periodic Paralysis, Hypomagnesemia, Hyponatremia, Hypophosphatemia, IgA Nephropathy, IgG4 Nephropathy, Interstitial Cystitis, Painful Bladder Syndrome (Questionnaire), Interstitial Nephritis, Ivemark's syndrome, Ketamine-Associated Bladder Dysfunction, Kidney Stones, Nephrolithiasis, Kombucha Tea Toxicity, Lead Nephropathy and Lead-Related Nephrotoxicity, Leptospirosis Renal Disease, Light Chain Deposition Disease, Monoclonal Immunoglobulin Deposition Disease, Liddle Syndrome, Lightwood-Albright Syndrome, Lipoprotein Glomerulopathy, Lithium Nephrotoxicity, LMX1B Mutations Cause Hereditary FSGS, Loin Pain Hematuria, Lupus, Systemic Lupus Erythematosis, Lupus Kidney Disease, Lupus Nephritis, Lupus Nephritis with Antineutrophil Cytoplasmic Antibody Seropositivity, Lyme Disease-Associated Glomerulonephritis, Malarial Nephropathy, Malignancy-Associated Renal Disease, Malignant Hypertension, Malakoplakia, Meatal Stenosis, Medullary Cystic Kidney Disease, Medullary Sponge Kidney, Megaureter, Melamine Toxicity and the Kidney, Membranoproliferative Glomerulonephritis, Membranous Nephropathy, MesoAmerican Nephropathy, Metabolic Acidosis, Metabolic Alkalosis, Methotrexate-related Renal Failure, Microscopic Polyangiitis, Milk-alkalai syndrome, Minimal Change Disease, MDMA (Molly; Ecstacy; 3,4-Methylenedioxymethamphetamine) and Kidney Failure, Multicystic dysplastic kidney, Multiple Myeloma, Myeloproliferative Neoplasms and Glomerulopathy, Nail-patella Syndrome, Nephrocalcinosis, Nephrogenic Systemic Fibrosis, Nephroptosis (Floating Kidney, Renal Ptosis), Nephrotic Syndrome, Neurogenic Bladder, Nodular Glomerulosclerosis, Non-Gonococcal Urethritis, Nutcracker syndrome, Orofaciodigital Syndrome, Orotic Aciduria, Orthostatic Hypotension, Orthostatic Proteinuria, Osmotic Diuresis, Ovarian Hyperstimulation Syndrome, Page Kidney, Papillary Necrosis, Papillorenal Syndrome (Renal-Coloboma Syndrome, Isolated Renal Hypoplasia), Parvovirus B19 and the Kidney, The Peritoneal-Renal Syndrome, Posterior Urethral Valve, Post-infectious Glomerulonephritis, Post-streptococcal Glomerulonephritis, Polyarteritis Nodosa, Polycystic Kidney Disease, Posterior Urethral Valves, Preeclampsia, Propofol infusion syndrome, Proliferative Glomerulonephritis with Monoclonal IgG Deposits (Nasr Disease), Propolis (Honeybee Resin) Related Renal Failure, Proteinuria (Protein in Urine), Pseudohyperaldosteronism, Pseudohypobicarbonatemia, Pseudohypoparathyroidism, Pulmonary-Renal Syndrome, Pyelonephritis (Kidney Infection), Pyonephrosis, Radiation Nephropathy, Ranolazine and the Kidney, Refeeding syndrome, Reflux Nephropathy, Rapidly Progressive Glomerulonephritis, Renal Abscess, Perinephric Abscess, Renal Agenesis, Renal Arcuate Vein Microthrombi-Associated Acute Kidney Injury, Renal Artery Aneurysm, Renal Artery Stenosis, Renal Cell Cancer, Renal Cyst, Renal Hypouricemia with Exercise-induced Acute Renal Failure, Renal Infarction, Renal Osteodystrophy, Renal Tubular Acidosis, Renin Secreting Tumors (Juxtaglomerular Cell Tumor), Reset Osmostat, Retrocaval Ureter, Retroperitoneal Fibrosis, Rhabdomyolysis, Rhabdomyolysis related to Bariatric Surgery, Rheumatoid Arthritis-Associated Renal Disease, Sarcoidosis Renal Disease, Salt Wasting, Renal and Cerebral, Schistosomiasis and Glomerular Disease, Schimke immuno-osseous dysplasia, Scleroderma Renal Crisis, Serpentine Fibula-Polycystic Kidney Syndrome, Exner Syndrome, Sickle Cell Nephropathy, Silica Exposure and Chronic Kidney Disease, Sri Lankan Farmers' Kidney Disease, Sjögren's Syndrome and Renal Disease, Synthetic Cannabinoid Use and Acute Kidney Injury, Kidney Disease Following Hematopoietic Cell Transplantation, Kidney Disease Related to Stem Cell Transplantation, Thin Basement Membrane Disease, Benign Familial Hematuria, Trigonitis, Tuberculosis, Genitourinary, Tuberous Sclerosis, Tubular Dysgenesis, Immune Complex Tubulointerstitial Nephritis Due to Autoantibodies to the Proximal Tubule Brush Border, Tumor Lysis Syndrome, Uremia, Uremic Optic Neuropathy, Ureteritis Cystica, Ureterocele, Urethral Caruncle, Urethral Stricture, Urinary Incontinence, Urinary Tract Infection, Urinary Tract Obstruction, Vesicointestinal Fistula, Vesicoureteral Reflux, Volatile Anesthetics and Acute Kidney Injury, Von Hippel-Lindau Disease, Waldenstrom's Macroglobulinemic Glomerulonephritis, Warfarin-Related Nephropathy, Wasp Stings and Acute Kidney Injury, Wegener's Granulomatosis, Granulomatosis with Polyangiitis, West Nile Virus and Chronic Kidney Disease, and Wunderlich syndrome.

Various cardiovascular diseases may be treated with pharmaceutical compositions, biocircuits, biocircuit components, effector modules including their SREs or payloads of the present disclosure. As a non-limiting example, the cardiovascular disease may be Ischemic heart disease also known as coronary artery disease, Cerebrovascular disease (Stroke), Peripheral vascular disease, Heart failure, Rheumatic heart disease, and Congenital heart disease.

Various antibody deficiencies may be treated with pharmaceutical compositions, biocircuits, biocircuit components, effector modules including their SREs or payloads of the present disclosure. As a non-limiting example, the antibody deficiencies may be X-Linked Agammaglobulinemia (XLA), Autosomal Recessive Agammaglobulinemia (ARA), Common Variable Immune Deficiency (CVID), IgG (IgG1, IgG2, IgG3 and IgG4) Subclass Deficiency, Selective IgA Deficiency, Specific Antibody Deficiency (SAD), Transient Hypogammaglobulinemia of Infancy, Antibody Deficiency with Normal or Elevated Immunoglobulins, Selective IgM Deficiency, Immunodeficiency with Thymoma (Good's Syndrome), Transcobalamin II Deficiency, Warts, Hypogammaglobulinemia, Infection, Myelokathexis (WHIM) Syndrome, Drug-Induced Antibody Deficiency, Kappa Chain Deficiency, Heavy Chain Deficiencies, Post-Meiotic Segregation (PMS2) Disorder, and Unspecified Hypogammaglobulinemia.

Various ocular diseases may be treated with pharmaceutical compositions, biocircuits, biocircuit components, effector modules including their SREs or payloads of the present disclosure. As a non-limiting example, the ocular disease may be thyroid eye disease (TED), Graves' disease (GD) and orbitopathy, Retina Degeneration, Cataract, optic atrophy, macular degeneration, Leber congenital amaurosis, retinal degeneration, cone-rod dystrophy, Usher syndrome, leopard syndrome, photophobia, and photoaversion.

Various neurological diseases may be treated with pharmaceutical compositions, biocircuits, biocircuit components, effector modules including their SREs or payloads of the present disclosure. As a non-limiting example, the neurological disease may be Absence of the Septum Pellucidum, Acid Lipase Disease, Acid Maltase Deficiency, Acquired Epileptiform Aphasia, Acute Disseminated Encephalomyelitis, Attention Deficit-Hyperactivity Disorder (ADHD), Adie's Pupil, Adie's Syndrome, Adrenoleukodystrophy, Agenesis of the Corpus Callosum, Agnosia, Aicardi Syndrome, Aicardi-Goutieres Syndrome Disorder, AIDS - Neurological Complications, Alexander Disease, Alpers' Disease, Alternating Hemiplegia, Alzheimer's Disease, Amyotrophic Lateral Sclerosis (ALS), Anencephaly, Aneurysm, Angelman Syndrome, Angiomatosis, Anoxia, Antiphospholipid Syndrome, Aphasia, Apraxia, Arachnoid Cysts, Arachnoiditis, Arnold-Chiari Malformation, Arteriovenous Malformation, Asperger Syndrome, Ataxia, Ataxia Telangiectasia, Ataxias and Cerebellar or Spinocerebellar Degeneration, Atrial Fibrillation and Stroke, Attention Deficit-Hyperactivity Disorder, Autism Spectrum Disorder, Autonomic Dysfunction, Back Pain, Barth Syndrome, Batten Disease, Becker's Myotonia, Behcet's Disease, Bell's Palsy, Benign Essential Blepharospasm, Benign Focal Amyotrophy, Benign Intracranial Hypertension, Bernhardt-Roth Syndrome, Binswanger's Disease, Blepharospasm, Bloch-Sulzberger Syndrome, Brachial Plexus Birth Injuries, Brachial Plexus Injuries, Bradbury-Eggleston Syndrome, Brain and Spinal Tumors, Brain Aneurysm, Brain Injury, Brown-Sequard Syndrome, Bulbospinal Muscular Atrophy, Cerebral Autosomal Dominant Arteriopathy with Sub-cortical Infarcts and Leukoencephalopathy (CADASIL), Canavan Disease, Carpal Tunnel Syndrome, Causalgia, Cavernomas, Cavernous Angioma, Cavernous Malformation, Central Cervical Cord Syndrome, Central Cord Syndrome, Central Pain Syndrome, Central Pontine Myelinolysis, Cephalic Disorders, Ceramidase Deficiency, Cerebellar Degeneration, Cerebellar Hypoplasia, Cerebral Aneurysms, Cerebral Arteriosclerosis, Cerebral Atrophy, Cerebral Beriberi, Cerebral Cavernous Malformation, Cerebral Gigantism, Cerebral Hypoxia, Cerebral Palsy, Cerebro-Oculo-Facio-Skeletal Syndrome (COFS), Charcot-Marie-Tooth Disease, Chiari Malformation, Cholesterol Ester Storage Disease, Chorea, Choreoacanthocytosis, Chronic Inflammatory Demyelinating Polyneuropathy (CIDP), Chronic Orthostatic Intolerance, Chronic Pain, Cockayne Syndrome Type II, Coffin Lowry Syndrome, Colpocephaly, Coma, Complex Regional Pain Syndrome, Congenital Facial Diplegia, Congenital Myasthenia, Congenital Myopathy, Congenital Vascular Cavernous Malformations, Corticobasal Degeneration, Cranial Arteritis, Craniosynostosis, Cree encephalitis, Creutzfeldt-Jakob Disease, Cumulative Trauma Disorders, Cushing's Syndrome, Cytomegalic Inclusion Body Disease, Cytomegalovirus Infection, Dancing Eyes-Dancing Feet Syndrome, Dandy-Walker Syndrome, Dawson Disease, De Morsier's Syndrome, Dejerine-Klumpke Palsy, Dementia, Dementia -Multi-Infarct, Dementia - Semantic, Dementia - Subcortical, Dementia With Lewy Bodies, Dentate Cerebellar Ataxia, Dentatorubral Atrophy, Dermatomyositis, Developmental Dyspraxia, Devic's Syndrome, Diabetic Neuropathy, Diffuse Sclerosis, Dravet Syndrome, Dysautonomia, Dysgraphia, Dyslexia, Dysphagia, Dyspraxia, Dyssynergia Cerebellaris Myoclonica, Dyssynergia Cerebellaris Progressiva, Dystonias, Early Infantile Epileptic Encephalopathy, Empty Sella Syndrome, Encephalitis, Encephalitis Lethargica, Encephaloceles, Encephalopathy, Encephalopathy (familial infantile), Encephalotrigeminal Angiomatosis, Epilepsy, Epileptic Hemiplegia, Erb's Palsy, Erb-Duchenne and Dejerine-Klumpke Palsies, Essential Tremor, Extrapontine Myelinolysis, Fabry Disease, Fahr's Syndrome, Fainting, Familial Dysautonomia, Familial Hemangioma, Familial Idiopathic Basal Ganglia Calcification, Familial Periodic Paralyses, Familial Spastic Paralysis, Farber's Disease, Febrile Seizures, Fibromuscular Dysplasia, Fisher Syndrome, Floppy Infant Syndrome, Foot Drop, Friedreich's Ataxia, Frontotemporal Dementia, Gaucher Disease, Generalized Gangliosidoses, Gerstmann's Syndrome, Gerstmann-Straussler-Scheinker Disease, Giant Axonal Neuropathy, Giant Cell Arteritis, Giant Cell Inclusion Disease, Globoid Cell Leukodystrophy, Glossopharyngeal Neuralgia, Glycogen Storage Disease, Guillain-Barré Syndrome, Hallervorden-Spatz Disease, Head Injury, Headache, Hemicrania Continua, Hemifacial Spasm, Hemiplegia Alterans, Hereditary Neuropathies, Hereditary Spastic Paraplegia, Heredopathia Atactica Polyneuritiformis, Herpes Zoster, Herpes Zoster Oticus, Hirayama Syndrome, Holmes-Adie syndrome, Holoprosencephaly, HTLV-1 Associated Myelopathy, Hughes Syndrome, Huntington's Disease, Hydranencephaly, Hydrocephalus, Hydrocephalus - Normal Pressure, Hydromyelia, Hypercortisolism, Hypersomnia, Hypertonia, Hypotonia, Hypoxia, Immune-Mediated Encephalomyelitis, Inclusion Body Myositis, Incontinentia Pigmenti, Infantile Hypotonia, Infantile Neuroaxonal Dystrophy, Infantile Phytanic Acid Storage Disease, Infantile Refsum Disease, Infantile Spasms, Inflammatory Myopathies, Iniencephaly, Intestinal Lipodystrophy, Intracranial Cysts, Intracranial Hypertension, Isaacs' Syndrome, Joubert Syndrome, Kearns-Sayre Syndrome, Kennedy's Disease, Kinsbourne syndrome, Kleine-Levin Syndrome, Klippel-Feil Syndrome, Klippel-Trenaunay Syndrome (KTS), Khiver-Bucy Syndrome, Korsakoffs Amnesic Syndrome, Krabbe Disease, Kugelberg-Welander Disease, Kuru, Lambert-Eaton Myasthenic Syndrome, Landau-Kleffner Syndrome, Lateral Femoral Cutaneous Nerve Entrapment, Lateral Medullary Syndrome, Learning Disabilities, Leigh's Disease, Lennox-Gastaut Syndrome, Lesch-Nyhan Syndrome, Leukodystrophy, Levine-Critchley Syndrome, Lewy Body Dementia, Lipid Storage Diseases, Lipoid Proteinosis, Lissencephaly, Locked-In Syndrome, Lou Gehrig's Disease, Lupus - Neurological Sequelae, Lyme Disease - Neurological Complications, Machado-Joseph Disease, Macrocephaly, Megalocephaly, Melkersson-Rosenthal Syndrome, Meningitis, Meningitis and Encephalitis, Menkes Disease, Meralgia Paresthetica, Metachromatic Leukodystrophy, Microcephaly, Migraine, Miller Fisher Syndrome, Mini Stroke, Mitochondrial Myopathy, Moebius Syndrome, Monomelic Amyotrophy, Motor Neuron Diseases, Moyamoya Disease, Mucolipidoses, Mucopolysaccharidosis, Multi-Infarct Dementia, Multifocal Motor Neuropathy, Multiple Sclerosis, Multiple System Atrophy, Multiple System Atrophy with Orthostatic Hypotension, Muscular Dystrophy, Myasthenia - Congenital, Myasthenia Gravis, Myelinoclastic Diffuse Sclerosis, Myoclonic Encephalopathy of Infants, Myoclonus, Myopathy, Myopathy- Congenital, Myopathy -Thyrotoxic, Myotonia, Myotonia Congenita, Narcolepsy, Neuroacanthocytosis, Neurodegeneration with Brain Iron Accumulation, Neurofibromatosis, Neuroleptic Malignant Syndrome, Neurological Complications of AIDS, Neurological Complications of Lyme Disease, Neurological Consequences of Cytomegalovirus Infection, Neurological Manifestations of Pompe Disease, Neurological Sequelae Of Lupus, Neuromyelitis Optica, Neuromyotonia, Neuronal Ceroid Lipofuscinosis, Neuronal Migration Disorders, Neuropathy- Hereditary, Neurosarcoidosis, Neurosyphilis, Neurotoxicity, Nevus Cavernosus, Niemann-Pick Disease, O'Sullivan-McLeod Syndrome, Occipital Neuralgia, Ohtahara Syndrome, Olivopontocerebellar Atrophy, Opsoclonus Myoclonus, Orthostatic Hypotension, Overuse Syndrome, Pain -Chronic, Pantothenate Kinase-Associated Neurodegeneration, Paraneoplastic Syndromes, Paresthesia, Parkinson's Disease, Paroxysmal Choreoathetosis, Paroxysmal Hemicrania, Parry-Romberg, Pelizaeus-Merzbacher Disease, Pena Shokeir II Syndrome, Perineural Cysts, Periodic Paralyses, Peripheral Neuropathy, Periventricular Leukomalacia, Persistent Vegetative State, Pervasive Developmental Disorders, Phytanic Acid Storage Disease, Pick's Disease, Pinched Nerve, Piriformis Syndrome, Pituitary Tumors, Polymyositis, Pompe Disease, Porencephaly, Post-Polio Syndrome, Postherpetic Neuralgia, Post infectious Encephalomyelitis, Postural Hypotension, Postural Orthostatic Tachycardia Syndrome, Postural Tachycardia Syndrome, Primary Dentatum Atrophy, Primary Lateral Sclerosis, Primary Progressive Aphasia, Prion Diseases, Progressive Hemifacial Atrophy, Progressive Locomotor Ataxia, Progressive Multifocal Leukoencephalopathy, Progressive Sclerosing Poliodystrophy, Progressive Supranuclear Palsy, Prosopagnosia, Pseudo-Torch syndrome, Pseudotoxoplasmosis syndrome, Pseudotumor Cerebri, Psychogenic Movement, Ramsay Hunt Syndrome I, Ramsay Hunt Syndrome II, Rasmussen's Encephalitis, Reflex Sympathetic Dystrophy Syndrome, Refsum Disease, Refsum Disease - Infantile, Repetitive Motion Disorders, Repetitive Stress Injuries, Restless Legs Syndrome, Retrovirus-Associated Myelopathy, Rett Syndrome, Reye's Syndrome, Rheumatic Encephalitis, Riley-Day Syndrome, Sacral Nerve Root Cysts, Saint Vitus Dance, Salivary Gland Disease, Sandhoff Disease, Schilder's Disease, Schizencephaly, Seitelberger Disease, Seizure Disorder, Semantic Dementia, Septo-Optic Dysplasia, Severe Myoclonic Epilepsy of Infancy (SMEI), Shaken Baby Syndrome, Shingles, Shy-Drager Syndrome, Sjögren's Syndrome, Sleep Apnea, Sleeping Sickness, Sotos Syndrome, Spasticity, Spina Bifida, Spinal Cord Infarction, Spinal Cord Injury, Spinal Cord Tumors, Spinal Muscular Atrophy, Spinocerebellar Atrophy, Spinocerebellar Degeneration, Steele-Richardson-Olszewski Syndrome, Stiff-Person Syndrome, Striatonigral Degeneration, Stroke, Sturge-Weber Syndrome, Subacute Sclerosing Panencephalitis, Subcortical Arteriosclerotic Encephalopathy, Short-lasting, Unilateral, Neuralgiform (SUNCT) Headache, Swallowing Disorders, Sydenham Chorea, Syncope, Syphilitic Spinal Sclerosis, Syringohydromyelia, Syringomyelia, Systemic Lupus Erythematosus, Tabes Dorsalis, Tardive Dyskinesia, Tarlov Cysts, Tay-Sachs Disease, Temporal Arteritis, Tethered Spinal Cord Syndrome, Thomsen's Myotonia, Thoracic Outlet Syndrome, Thyrotoxic Myopathy, Tic Douloureux, Todd's Paralysis, Tourette Syndrome, Transient Ischemic Attack, Transmissible Spongiform Encephalopathies, Transverse Myelitis, Traumatic Brain Injury, Tremor, Trigeminal Neuralgia, Tropical Spastic Paraparesis, Troyer Syndrome, Tuberous Sclerosis, Vascular Erectile Tumor, Vasculitis Syndromes of the Central and Peripheral Nervous Systems, Von Economo's Disease, Von Hippel-Lindau Disease (VHL), Von Recklinghausen's Disease, Wallenberg's Syndrome, Werdnig-Hoffman Disease, Wernicke-Korsakoff Syndrome, West Syndrome, Whiplash, Whipple's Disease, Williams Syndrome, Wilson Disease, Wolman's Disease, X-Linked Spinal and Bulbar Muscular Atrophy.

Various psychological disorders may be treated with pharmaceutical compositions, biocircuits, biocircuit components, effector modules including their SREs or payloads of the present disclosure. As a non-limiting example, the psychological disorders may be Aboulia, Absence epilepsy, Acute stress Disorder, Adjustment Disorders, Adverse effects of medication NOS, Age related cognitive decline, Agoraphobia, Alcohol Addiction, Alzheimer's Disease, Amnesia (also known as Amnestic Disorder), Amphetamine Addiction, Anorexia Nervosa, Anterograde amnesia, Antisocial personality disorder (also known as Sociopathy), Anxiety Disorder (Also known as Generalized Anxiety Disorder), Anxiolytic related disorders, Asperger's Syndrome (now part of Autism Spectrum Disorder), Attention Deficit Disorder (Also known as ADD), Attention Deficit Hyperactivity Disorder (Also known as ADHD), Autism Spectrum Disorder (also known as Autism), Autophagia, Avoidant Personality Disorder, Barbiturate related disorders, Benzodiazepine related disorders, Bereavement, Bibliomania, Binge Eating Disorder, Bipolar disorder (also known as Manic Depression, includes Bipolar I and Bipolar II), Body Dysmorphic Disorder, Borderline intellectual functioning, Borderline Personality Disorder, Breathing-Related Sleep Disorder, Brief Psychotic Disorder, Bruxism, Bulimia Nervosa, Caffeine Addiction, Cannabis Addiction, Catatonic disorder, Catatonic schizophrenia, Childhood amnesia, Childhood Disintegrative Disorder (now part of Autism Spectrum Disorder), Childhood Onset Fluency Disorder (formerly known as Stuttering), Circadian Rhythm Disorders, Claustrophobia, Cocaine related disorders, Communication disorder, Conduct Disorder, Conversion Disorder, Cotard delusion, Cyclothymia (also known as Cyclothymic Disorder), Delerium, Delusional Disorder, dementia, Dependent Personality Disorder (also known as Asthenic Personality Disorder), Depersonalization disorder (now known as Depersonalization / Derealization Disorder), Depression (also known as Major Depressive Disorder), Depressive personality disorder, Derealization disorder (now known as Depersonalization / Derealization Disorder), Dermotillomania, Desynchronosis, Developmental coordination disorder, Diogenes Syndrome, Disorder of written expression, Dispareunia, Dissocial Personality Disorder, Dissociative Amnesia, Dissociative Fugue, Dissociative Identity Disorder (formerly known as Multiple Personality Disorder), Down syndrome, Dyslexia, Dyspareunia, Dysthymia (now known as Persistent Depressive Disorder), Eating disorder NOS, Ekbom's Syndrome (Delusional Parasitosis), Emotionally unstable personality disorder, Encopresis, Enuresis (bedwetting), Erotomania, Exhibitionistic Disorder, Expressive language disorder, Factitious Disorder, Female Sexual Disorders, Fetishistic Disorder, Folie a deux, Fregoli delusion, Frotteuristic Disorder, Fugue State, Ganser syndrome, Gambling Addiction, Gender Dysphoria (formerly known as Gender Identity Disorder), Generalized Anxiety Disorder, General adaptation syndrome, Grandiose delusions, Hallucinogen Addiction, Haltlose personality disorder, Histrionic Personality Disorder, Primary hypersomnia, Huntington's Disease, Hypoactive sexual desire disorder, Hypochondriasis, Hypomania, Hyperkinetic syndrome, Hypersomnia, Hysteria, Impulse control disorder, Impulse control disorder NOS, Inhalant Addiction, Insomnia, Intellectual Development Disorder, Intermittent Explosive Disorder, Joubert syndrome, Kleptomania, Korsakoff's syndrome, Lacunar amnesia, Language Disorder, Learning Disorders, Major Depression (also known as Major Depressive Disorder), major depressive disorder, Male Sexual Disorders, Malingering, Mathematics disorder, Medication-related disorder, Melancholia, Mental Retardation (now known as Intellectual Development Disorder), Misophonia, Morbid jealousy, Multiple Personality Disorder (now known as Dissociative Identity Disorder), Munchausen Syndrome, Munchausen by Proxy, Narcissistic Personality Disorder, Narcolepsy, Neglect of child, Neurocognitive Disorder (formerly known as Dementia), Neuroleptic-related disorder, Nightmare Disorder, Non Rapid Eye Movement, Obsessive-Compulsive Disorder, Obsessive-Compulsive Personality Disorder (also known as Anankastic Personality Disorder), Oneirophrenia, Onychophagia, Opioid Addiction, Oppositional Defiant Disorder, Orthorexia (ON), Pain disorder, Panic attacks, Panic Disorder, Paranoid Personality Disorder, Parkinson's Disease, Partner relational problem, Passive-aggressive personality disorder, Pathological gambling, Pedophilic Disorder, Perfectionism, Persecutory delusion, Persistent Depressive Disorder (also known as Dysthymia), Personality change due to a general medical condition, Personality disorder, Pervasive developmental disorder (PDD), Phencyclidine related disorder, Phobic disorder, Phonological disorder, Physical abuse, Pica, Polysubstance related disorder, Postpartum Depression, Post-traumatic embitterment disorder (PTED), Post-Traumatic Stress Disorder, Premature ejaculation, Premenstrual Dysphoric Disorder, Psychogenic amnesia, Psychological factor affecting medical condition, Psychoneurotic personality disorder, Psychotic disorder, not otherwise specified, Pyromania, Reactive Attachment Disorder, Reading disorder, Recurrent brief depression, Relational disorder, REM Sleep Behavior Disorder, Restless Leg Syndrome, Retrograde amnesia, Retts Disorder (now part of Autism Spectrum Disorder), Rumination syndrome, Sadistic personality disorder, Schizoaffective Disorder, Schizoid Personality Disorder, Schizophrenia, Schizophreniform disorder, Schizotypal Personality Disorder, Seasonal Affective Disorder, Sedative, Hypnotic, or Anxiolytic Addiction, Selective Mutism, Self-defeating personality disorder, Separation Anxiety Disorder, Sexual Disorders Female, Sexual Disorders Male, Sexual Addiction, Sexual Masochism Disorder, Sexual Sadism Disorder, Shared Psychotic Disorder, Sleep Arousal Disorders, Sleep Paralysis, Sleep Terror Disorder (now part of Nightmare Disorder, Social Anxiety Disorder, Somatization Disorder, Specific Phobias, Stendhal syndrome, Stereotypic movement disorder, Stimulant Addiction, Stuttering (now known as Childhood Onset Fluency Disorder), Substance related disorder, Tardive dyskinesia, Tobacco Addiction, Tourettes Syndrome, Transient tic disorder, Transient global amnesia, Transvestic Disorder, Trichotillomania, Undifferentiated Somatoform Disorder, Vaginismus, and Voyeuristic Disorder.

Various lung diseases may be treated with pharmaceutical compositions, biocircuits, biocircuit components, effector modules including their SREs or payloads of the present disclosure. As a non-limiting example, the lung diseases may be Asbestosis, Asthma, Bronchiectasis, Bronchitis, Chronic Cough, Chronic Obstructive Pulmonary Disease (COPD), Croup, Cystic Fibrosis, Hantavirus, Idiopathic Pulmonary Fibrosis, Pertussis, Pleurisy, Pneumonia, Pulmonary Embolism, Pulmonary Hypertension, Sarcoidosis, Sleep Apnea, Spirometry, Sudden Infant Death Syndrome (SIDS), Tuberculosis, Alagille Syndrome, Autoimmune Hepatitis, Biliary Atresia, Cirrhosis, ERCP (Endoscopic Retrograde Cholangiopancreatography), and Hemochromatosis. Nonalcoholic Steatohepatitis, Porphyria, Primary Biliary Cirrhosis, Primary Sclerosing Cholangitis.

Various bone diseases may be treated with pharmaceutical compositions, biocircuits, biocircuit components, effector modules including their SREs or payloads of the present disclosure. As a non-limiting example, the bone diseases may be osteoporosis, neurofibromatosis, osteogenesis imperfecta (OI), rickets, osteosarcoma, achondroplasia, fracture, osteomyelitis, Ewing tumor of bone, osteomalacia, hip dysplasia, Paget disease of bone, marble bone disease, osteochondroma, bone cancer, bone disease, osteochondrosis, osteoma, fibrous dysplasia, cleidocranial dysostosis, osteoclastoma, bone cyst, metabolic bone disease, melorheostosis, callus, Caffey syndrome, and mandibulofacial dysostosis.

Various blood diseases may be treated with pharmaceutical compositions, biocircuits, biocircuit components, effector modules including their SREs or payloads of the present disclosure. As a non-limiting example, the blood diseases may be Anemia and CKD (for health care professionals), Aplastic Anemia and Myelodysplastic Syndromes, Deep Vein Thrombosis, Hemochromatosis, Hemophilia, Henoch-Schönlein Purpura, Idiopathic Thrombocytopenic Purpura, Iron-Deficiency Anemia, Pernicious Anemia, Pulmonary Embolism, Sickle Cell Anemia, Sickle Cell Trait and Other Hemoglobinopathies, Thalassemia, Thrombotic Thrombocytopenic Purpura, and Von Willebrand Disease.

In some instance, biocircuits of the disclosure may be used for the treatment of infectious diseases. Biocircuits of the disclosure may be introduced in cells suitable for adoptive cell transfer such as macrophages, dendritic cells, natural killer cells, and or T cells. Infectious diseases treated by the biocircuits of the disclosure may be diseases caused by viruses, bacteria, fungi, and/or parasites. IL15-IL15Ra payloads of the disclosure may be used to increase immune cell proliferation and/or persistence of the immune cells useful in treating infectious diseases.

### 6. Microbiome

Alterations in the composition of the microbiome may impact the action of anti-cancer therapies. A diverse community of symbiotic, commensal and pathogenic microorganisms exist in all environmentally exposed sites in the body and is herein referred to as the "Microbiome." Environmentally exposed sites of the body that may be inhabited by a microbiome include the skin, nasopharynx, the oral cavity, respiratory tract, gastrointestinal tract, and the reproductive tract.

In some instances, microbiome engineered with the biocircuits of the present disclosure may be used to improve the efficacy of the anti-cancer immunotherapies. Sivan et al., found that mice with *Bifidobacterium* in their gut microbiome were more responsive to immune check point blockage e.g. anti PD-L1 immunotherapy in subcutaneous melanoma tumor model (Sivan A., et al. Commensal Bifidobacterium promotes antitumor immunity and facilitates anti-PD-Ll efficacy. Science 2015; 350:1084-9). In one instance, protein, RNA and/or other biomolecules derived from the microbiome may be used as a payload to influence the efficacy of the anti-cancer immunotherapies. In other instances, the microorganisms may be delivered along with immunotherapeutic compositions of the present disclosure to improve the efficacy of immunotherapy.

### 7. Tools and agents for making therapeutics

Provided in the present disclosure are tools and agents that may be used in generating immunotherapeutics for reducing a tumor volume or burden in a subject in need. A considerable number of variables are involved in producing a therapeutic agent, such as structure of the payload, type of cells, method of gene transfers, method and time of ex vivo expansion, pre- conditioning and the amount and type of tumor burden in the subject. Such parameters may be optimized using tools and agents described herein.

### Cell lines

The present disclosure provides a mammalian cell that has been genetically modified with the compositions of the disclosure. Suitable mammalian cells include primary cells and immortalized cell lines. Suitable mammalian cell lines include, but are not limited to Human embryonic kidney cell line 293, fibroblast cell line NIH 3T3, human colorectal carcinoma cell line HCT116, ovarian carcinoma cell line SKOV-3, immortalized T cell lines Jurkat cells, lymphoma cell line Raji cells, NALM-6 cells, K562 cells, HeLa cells, PC12 cells, HL-60 cells, NK cell lines (e.g. NKL. NK962, and YTS), and the like. In some instances, the cell is not an immortalized cell line, but instead a cell obtained from an individual and is herein referred to as a primary cell. For example, the cell is a T lymphocyte obtained from an individual. Other examples include, but are not limited to cytotoxic cells, stem cells, peripheral blood mononuclear cells or progenitor cells obtained from an individual.

### Tracking SREs, biocircuits and cell lines

In some instances, it may be desirable to track the compositions of the disclosure or the cells modified by the compositions of the disclosure. Tracking may be achieved by using payloads such as reporter moieties, which, as used herein, refers to any protein capable of creating a detectable signal, in response to an input. Examples include alkaline phosphatase, β-galactosidase, chloramphenicol acetyltransferase, β-glucuronidase, peroxidase, β-lactamase, catalytic antibodies, bioluminescent proteins e.g. luciferase, and fluorescent proteins such as Green fluorescent protein (GFP).

Reporter moieties may be used to monitor the response of the DD upon addition of the ligand corresponding to the DD. In other instances, reporter moieties may be used to track cell survival, persistence, cell growth, and/or localization *in vitro, in vivo, or ex vivo.*

In some instances, the preferred reporter moiety may be luciferase proteins. In one instance, the reporter moiety is the Renilla luciferase, or a firefly luciferase.

### Animal models

The utility and efficacy of the compositions of the present disclosure may be tested *in vivo* animal models, preferably mouse models. Mouse models used to may be syngeneic mouse models wherein mouse cells are modified with compositions of the disclosure and tested in mice of the same genetic background. Examples include pMEL-1 and 4T1 mouse models. Alternatively, xenograft models where human cells such as tumor cells and immune cells are introduced into immunodeficient mice may also be utilized in such studies. Immunodeficient mice used may be CByJ.Cg-Foxn1^{nu}/J, B6;129S7-*Rag1^{tm1Mom}*/J, B6.129S7-*Rag1*^{*tm1*Mom}/J, B6. CB17-*Prkdc^{scid}*/SzJ*,* NOD.129S7(B6)-*Rag1^{tm1Mom}*/J, NOD.Cg-*Rag1^{tm1Mom}Prf1^{tm1Sd}*z/Sz*,* NOD.CB17-*Prkdc^{scid}*/SzJ, NOD.Cg-*Prkdc^{scid}B2m^{tm1Unc}*/J*, NOD-scid* IL2Rg^{null}, Nude (nu) mice, SCID mice, NOD mice, RAG1/RAG2 mice, NOD-Scid mice, IL2rg*null* mice, b2m*null* mice, *NOD-scid* IL2rγnull mice, NOD*-scid-*B2m*null* mice, and HLA transgenic mice.

### Cellular assays

In some instances, the effectiveness of the compositions of the disclosures as immunotherapeutic agents may be evaluated using cellular assays. Levels of expression and/or identity of the compositions of the disclosure may be determined according to any methods known in the art for identifying proteins and/or quantitating proteins levels. In some instances, such methods may include Western Blotting, flow cytometry, and immunoprecipitation.

Provided herein are methods for functionally characterizing cells expressing SRE, biocircuits and compositions of the disclosure. In some instances, functional characterization is carried out in primary immune cells or immortalized immune cell lines and may be determined by expression of cell surface markers. Examples of cell surface markers for T cells include, but are not limited to, CD3, CD4, CD8, CD 14, CD20, CD11b, CD16, CD45 and HLA-DR, CD 69, CD28, CD44, IFNgamma. Examples of cell surface markers for antigen presenting cells include, but are not limited to, MHC class I, MHC Class II, CD40, CD45, B7-1, B7-2, IFN γ receptor and IL2 receptor, ICAM-1 and/or Fcy receptor. Examples of cell surface markers for dendritic cells include, but are not limited to, MHC class I, MHC Class II, B7-2, CD18, CD29, CD31, CD43, CD44, CD45, CD54, CD58, CD83, CD86, CMRF-44, CMRF-56, DCIR and/or Dectin-1 and the like; while in some cases also having the absence of CD2, CD3, CD4, CD8, CD14, CD15, CD16, CD 19, CD20, CD56, and/or CD57. Examples of cell surface markers for NK cells include, but are not limited to, CCL3, CCL4, CCL5, Granulysin, Granzyme B, Granzyme K, IL10, IL22, IFNg, LAP, Perforin, and TNFa.

### 8. Gene editing

The CRISPR-Cas9 system is a novel genome editing system which has been rapidly developed and implemented in a multitude of model organisms and cell types, and supplants other genome editing technologies, such as TALENs and ZFNs. CRISPRs are sequence motifs are present in bacterial and archaeal genomes, and are composed of short (about 24-48 nucleotide) direct repeats separated by similarly sized, unique spacers (Grissa et al. BMC Bioinformatics 8, 172 (2007). They are generally flanked by a set of CRISPR-associated (Cas) protein-coding genes that are required for CRISPR maintenance and function (Barrangou et al., Science 315, 1709 (2007), Brouns et al., Science 321, 960 (2008), Haft et al. PLoS Comput Biol 1, e60 (2005)). CRISPR-Cas systems provide adaptive immunity against invasive genetic elements (e.g., viruses, phages and plasmids) (Horvath and Barrangou, Science, 2010, 327: 167-170; Bhaya et al., Annu. Rev. Genet., 2011, 45: 273-297; and Brrangou R, RNA, 2013, 4: 267-278). Three different types of CRISPR-Cas systems have been classified in bacteria and the type II CRISPR-Cas system is most studied. In the bacterial Type II CRISPR-Cas system, small CRISPR RNAs (crRNAs) processed from the pre-repeat-spacer transcript (pre-crRNA) in the presence of a trans-activating RNA (tracrRNA)/ Cas9 can form a duplex with the tracrRNA/Cas9 complex. The mature complex is recruited to a target double strand DNA sequence that is complementary to the spacer sequence in the tracrRNA: crRNA duplex to cleave the target DNA by Cas9 endonuclease (Garneau et al., Nature, 2010, 468: 67-71; Jinek et al., Science, 2012, 337: 816-821; Gasiunas et al., Proc. Natl Acad. Sci. USA., 109: E2579-2586; and Haurwitz et al., Science, 2010, 329: 1355-1358). Target recognition and cleavage by the crRNA: tracrRNA/Cas9 complex in the type II CRISPR-CAS system not only requires a sequence in the tracrRNA: crRNA duplex that is a complementary to the target sequence (also called "protospacer" sequence) but also requires a protospacer adjacent motif (PAM) sequence located 3'end of the protospacer sequence of a target polynucleotide. The PAM motif can vary between different CRISPR-Cas systems.

CRISPR-Cas9 systems have been developed and modified for use in genetic editing and prove to be a high effective and specific technology for editing a nucleic acid sequence even in eukaryotic cells. Many researchers disclosed various modifications to the bacterial CRISPR-Cas systems and demonstrated that CRISPR-Cas systems can be used to manipulate a nucleic acid in a cell, such as in a mammalian cell and in a plant cell. Representative references include US Pat. NOs.: 8,993,233; 8,999,641; 8,945,839; 8, 932,814; 8,906, 616; 8,895,308; 8,889,418; 8,889,356; 8,871,445; 8,865,406; 8,771,945; and 8,697,359; US patent publication NOs.: 20150031134; 20150203872; 20150218253; 20150176013; 20150191744; 20150071889; 20150067922; and 20150167000.

However, controlling the effects and activity of the CRISPR-Cas system (e.g., guide RNA and nuclease) has been challenging and often can be problematic.

The biocircuits of the present disclosure and/or any of their components may be utilized in regulating or tuning the CRISPR/Cas9 system to optimize its utility.

In some instances, the payloads of the effector modules of the disclosure may include alternative isoforms or orthologs of the Cas9 enzyme.

The most commonly used Cas9 is derived from *Streptococcus pyogenes* and the RuvC domain can be inactivated by a D10A mutation and the HNH domain can be inactivated by an H840A mutation.

In addition to Cas9 derived from *S. pyogenes,* other RNA guided endonucleases (RGEN) may also be used for programmable genome editing. Cas9 sequences have been identified in more than 600 bacterial strains. Though Cas9 family shows high diversity of amino acid sequences and protein sizes, All Cas9 proteins share a common architecture with a central HNH nuclease domain and a split RuvC/RHase H domain. Examples of Cas9 orthologs from other bacterial strains including but not limited to, Cas proteins identified *inAcaryochloris marina* MBIC11017; *Acetohalobium arabaticum* DSM 5501; *Acidithiobacillus caldus; Acidithiobacillusferrooxidans* ATCC 23270; *Alicyclobacillus acidocaldarius* LAA1; *Alicyclobacillus acidocaldarius subsp. acidocaldarius* DSM 446; *Allochromatium vinosum* DSM 180; *Ammonifex degensii* KC4; *Anabaena variabilis* ATCC 29413; *Arthrospira maxima* CS-328; *Arthrospira platensis str. Paraca; Arthrospira sp.* PCC 8005; *Bacillus pseudomycoides* DSM 12442; *Bacillus selenitireducens* MLS10; *Burkholderiales bacterium* 1_1_47; *Caldicelulosiruptor becscii* DSM 6725; *Candidates Desulforudis audaxviator* MP104C; *Caldicellulosiruptor hydrothermalis_108; Clostridium phage* c-st; *Clostridium botulinum A3 str. Loch Maree; Clostridium botulinum* Ba4 str. 657; *Clostridium difficile* QCD-63q42; *Crocosphaera watsonii* WH 8501; *Cyanothece sp.* ATCC 51142; *Cyanothece sp.* CCY0110; *Cyanothece sp.* PCC 7424; *Cyanothece sp.* PCC 7822; *Exiguobacterium sibiricum* 255-15; *Finegoldia magna* ATCC 29328; *Ktedonobacter racemifer* DSM 44963; *Lactobacillus delbrueckii subsp. bulgaricus* PB2003/044-T3-4; *Lactobacillus salivarius* ATCC 11741; *Listeria innocua; Lyngbya sp.* PCC 8106; *Marinobacter sp.* ELB17; *Methanohalobium evestigatum* Z-7303; *Microcystis phage* Ma-LMM01; *Microcystis aeruginosa* NIES-843; *Microscilla marina* ATCC 23134; *Microcoleus chthonoplastes* PCC 7420; *Neisseria meningitidis; Nitrosococcus halophilus* Nc4; *Nocardiopsis dassonvillei subsp. dassonvillei* DSM 43111; *Nodularia spumigena* CCY9414; *Nostoc sp.* PCC 7120; *Oscillatoria sp.* PCC 6506; *Pelotomaculum thermopropionicum* SI; *Petrotoga mobilis* SJ95; *Polaromonas naphthalenivorans* CJ2; *Polaromonas sp.* JS666; *Pseudoalteromonas haloplanktis* TAC125; *Streptomyces pristinaespiralis* ATCC 25486; *Streptomyces pristinaespiralis* ATCC 25486; *Streptococcus thermophilus; Streptomyces viridochromogenes* DSM 40736; *Streptosporangium roseum* DSM 43021; *Synechococcus sp.* PCC 7335; and *Thermosipho africanus* TCF52B (Chylinski et al., RNA Biol., 2013; 10(5): 726-737).

In addition to Cas9 orthologs, other Cas9 variants such as fusion proteins of inactive dCas9 and effector domains with different functions may be served as a platform for genetic modulation. Any of the foregoing enzymes may be useful in the present disclosure.

### 9. Stem cell applications

The biocircuits of the present disclosure and/or any of their components may be utilized in the regulated reprogramming of cells, stem cell engraftment or other application where controlled or tunable expression of such reprogramming factors are useful.

The biocircuits of the present disclosure may be used in reprogramming cells including stem cells or induced stem cells. Induction of induced pluripotent stem cells (iPSC) was first achieved by Takahashi and Yamanaka (Cell, 2006. 126(4):663-76) using viral vectors to express KLF4, c-MYC, OCT4 and SOX2 otherwise collectively known as KMOS.

Excisable lentiviral and transposon vectors, repeated application of transient plasmid, episomal and adenovirus vectors have also been used to try to derive iPSC (Chang, C.-W., et al., Stem Cells, 2009. 27(5):1042-1049; Kaji, K., et al., Nature, 2009. 458(7239):771-5; Okita, K., et al., Science, 2008. 322(5903):949-53; Stadtfeld, M., et al., Science, 2008. 322(5903):945-9; Woltjen, K., et al., Nature, 2009; Yu, J., et al., Science, 2009:1172482; Fusaki, N., et al., Proc Jpn Acad Ser B Phys Biol Sci, 2009. 85(8):348-62).

DNA-free methods to generate human iPSC has also been derived using serial protein transduction with recombinant proteins incorporating cell-penetrating peptide moieties (Kim, D., et al., Cell Stem Cell, 2009. 4(6): 472-476; Zhou, H., et al., Cell Stem Cell, 2009. 4(5):381-4), and infectious transgene delivery using the Sendai virus (Fusaki, N., et al., Proc Jpn Acad Ser B Phys Biol Sci, 2009. 85(8): p. 348-62).

The effector modules of the present disclosure may include a payload comprising any of the genes including, but not limited to, OCT such as OCT4, SOX such as SOX1, SOX2, SOX3, SOX15 and SOX18, NANOG, KLF such as KLF1, KLF2, KLF4 and KLF5, MYC such as c-MYC and n-MYC, REM2, TERT and LIN28 and variants thereof in support of reprogramming cells. Sequences of such reprogramming factors are taught in for example International Application PCT/US2013/074560.

In some instances, the payload of the present disclosure may be cardiac lineage specification factors such as eomesodermin (EOMES), a T-box transcription factor; WNT signaling pathway components such as WNT3 and WNT 3A. EOMES is crucially required for the development of the heart. Cardiomyocyte programming by EOMES involves autocrine activation of the canonical WNT signaling pathway and vice versa. Under conditions that are conducive to promoting cardiac lineage, WNT signaling activates EOMES and EOMES in turn promotes WNT signaling creating a self-sustaining loop that promotes the cardiac lineage. An activation loop that is too weak or too strong promotes non-cardiac fates such as endodermal and other mesodermal fates respectively. The DDs of the present disclosure may be used to tune EOMES and WNT payload levels to generate an activation loop that initiate and/or sustain cardiac specification during gastrulation.

### VII. DEFINITIONS

At various places in the present specification, features or functions of the compositions of the present disclosure are disclosed in groups or in ranges. It is specifically intended that the present disclosure include each and every individual sub-combination of the members of such groups and ranges. The following is a non-limiting list of term definitions.

*Activity:* As used herein, the term "activity" refers to the condition in which things are happening or being done. Compositions of the disclosure may have activity and this activity may involve one or more biological events. In some instances, biological events may include cell signaling events. In some instances, biological events may include cell signaling events associated protein interactions with one or more corresponding proteins, receptors, small molecules or any of the biocircuit components described herein.

*Adoptive cell therapy (ACT)*: The terms "Adoptive cell therapy" or "Adoptive cell transfer", as used herein, refer to a cell therapy involving in the transfer of cells into a patient, wherein cells may have originated from the patient, or from another individual, and are engineered (altered) before being transferred back into the patient. The therapeutic cells may be derived from the immune system, such as Immune effector cells: CD4+ T cell; CD8+ T cell, Natural Killer cell (NK cell); and B cells and tumor infiltrating lymphocytes (TILs) derived from the resected tumors. Most commonly transferred cells are autologous anti-tumor T cells after ex vivo expansion or manipulation. For example, autologous peripheral blood lymphocytes can be genetically engineered to recognize specific tumor antigens by expressing T-cell receptors (TCR) or chimeric antigen receptor (CAR).

*Agent*: As used herein, the term "agent" refers to a biological, pharmaceutical, or chemical compound. Non-limiting examples include simple or complex organic or inorganic molecule, a peptide, a protein, an oligonucleotide, an antibody, an antibody derivative, antibody fragment, a receptor, and soluble factor.

*Agonist*: the term "agonist" as used herein, refers to a compound that, in combination with a receptor, can produce a cellular response. An agonist may be a ligand that directly binds to the receptor. Alternatively, an agonist may combine with a receptor indirectly by, for example, (a) forming a complex with another molecule that directly binds to the receptor, or (b) otherwise resulting in the modification of another compound so that the other compound directly binds to the receptor. An agonist may be referred to as an agonist of a particular receptor or family of receptors, e.g., agonist of a co-stimulatory receptor.

*Antagonist*: the term "antagonist" as used herein refers to any agent that inhibits or reduces the biological activity of the target(s) it binds.

*Antigen*: the term "antigen" as used herein is defined as a molecule that provokes an immune response when it is introduced into a subject or produced by a subject such as tumor antigens which arise by the cancer development itself. This immune response may involve either antibody production, or the activation of specific immunologically-competent cells such as cytotoxic T lymphocytes and T helper cells, or both. An antigen can be derived from organisms, subunits of proteins/antigens, killed or inactivated whole cells or lysates. In the context of the disclosure, the terms "antigens of interest" or "desired antigens" refers to those proteins and/or other biomolecules provided herein that are immunospecifically bound or interact with antibodies of the present disclosure and/or fragments, mutants, variants, and/or alterations thereof described herein. In some instances, antigens of interest may comprise any of the polypeptides or payloads or proteins described herein, or fragments or portions thereof.

*Approximately*: As used herein, the term "approximately" or "about," as applied to one or more values of interest, refers to a value that is similar to a stated reference value. In certain instances, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Alkyl:* The terms "alkyl", "alkoxy", "hydroxyalkyl", "alkoxyalkyl", and "alkoxycarbonyl", as used herein, include both straight and branched chains containing one to twelve carbon atoms, and/or which may or may not be substituted.

*Alkenyl:* The terms "alkenyl" and "alkynyl" as used herein alone or as part of a larger moiety shall include both straight and branched chains containing two to twelve carbon atoms.

*Aryl:* The term "aryl" as used herein alone or as part of a larger moiety as in "aralkyl", "aralkoxy", or "aryloxyalkyl", refers to monocyclic, bicyclic and tricyclic carbocyclic ring systems having a total of five to fourteen ring members, wherein at least one ring is aromatic and wherein each ring in the system contains 3 to 8 ring members. The term "aryl" may be used interchangeably with the term "aryl ring."

*Aromatic:* The term "aromatic" as used herein, refers to an unsaturated hydrocarbon ring structure with delocalized pi electrons. As used herein "aromatic" may refer to monocyclic, bicyclic or polycyclic aromatic compounds.

*Aliphatic:* The term "aliphatic" or "aliphatic group" as used herein, refers to a straight or branched C1-C8 hydrocarbon chain or a monocyclic C3-C8hydrocarbon or bicyclic C8- C12 hydrocarbon which are fully saturated or that contains one or more units of unsaturation, that is completely saturated or that contains one or more units of unsaturation, but which is not aromatic (also referred to herein as "carbocycle" or "cycloalkyl"), and that has a single point of attachment to the rest of the molecule wherein any individual ring in said bicyclic ring system has 3-7 members.

*Associated with*: As used herein, the terms "associated with," "conjugated," "linked," "attached," and "tethered," when used with respect to two or more moieties, mean that the moieties are physically associated or connected with one another, either directly or via one or more additional moieties that serve as linking agents, to form a structure that is sufficiently stable so that the moieties remain physically associated under the conditions in which the structure is used, e.g., physiological conditions. An "association" need not be strictly through direct covalent chemical bonding. It may also suggest ionic or hydrogen bonding or a hybridization based connectivity sufficiently stable such that the "associated" entities remain physically associated.

*Autologous*: the term "autologous" as used herein is meant to refer to any material derived from the same individual to which it is later to be re-introduced into the individual.

*Barcode*: the term "barcode" as used herein refers to polynucleotide or amino acid sequence that distinguishes one polynucleotide or amino acid from another.

*Biocircuit system*: As used herein, a "biocircuit" or "biocircuit system" is defined as a circuit within or useful in biologic systems comprising a stimulus and at least one effector module responsive to a stimulus, where the response to the stimulus produces at least one signal or outcome within, between, as an indicator of, or on a biologic system. Biologic systems are generally understood to be any cell, tissue, organ, organ system or organism, whether animal, plant, fungi, bacterial, or viral. It is also understood that biocircuits may be artificial circuits which employ the stimuli or effector modules taught by the present disclosure and effect signals or outcomes in acellular environments such as with diagnostic, reporter systems, devices, assays or kits. The artificial circuits may be associated with one or more electronic, magnetic, or radioactive components or parts. In the context of the present disclosure, a biocircuit includes a destabilizing domain (DD) biocircuit system.

*Checkpoint factor*: As used herein, a checkpoint factor is any moiety or molecule whose function acts at the junction of a process. For example, a checkpoint protein, ligand or receptor may function to stall or accelerate the cell cycle.

*Co-stimulatory molecule*: As used herein, in accordance with its meaning in immune T cell activation, refers to a group of immune cell surface receptor/ligands which engage between T cells and APCs and generate a stimulatory signal in T cells which combines with the stimulatory signal in T cells that results from T cell receptor (TCR) recognition of antigen/MHC complex (pMHC) on APCs

*Cytokines*: the term "cytokines", as used herein, refers to a family of small soluble factors with pleiotropic functions that are produced by many cell types that can influence and regulate the function of the immune system.

*Delivery*: the term "delivery" as used herein refers to the act or manner of delivering a compound, substance, entity, moiety, cargo or payload. A "delivery agent" refers to any agent which facilitates, at least in part, the in vivo delivery of one or more substances (including, but not limited to compounds and/or compositions of the present disclosure) to a cell, subject or other biological system cells.

*Destabilized*: As used herein, the term "destable," "destabilize," "destabilizing region" or "destabilizing domain" means a region or molecule that is less stable than a starting, reference, wild-type or native form of the same region or molecule.

*Engineered*: As used herein, instances of the disclosure are "engineered" when they are designed to have a feature or property, whether structural or chemical, that varies from a starting point, wild type or native molecule.

*Expression*: As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end processing); (3) translation of an RNA into a polypeptide or protein; (4) folding of a polypeptide or protein; and (5) post-translational modification of a polypeptide or protein.

*Feature*: As used herein, a "feature" refers to a characteristic, a property, or a distinctive element.

*Formulation*: As used herein, a "formulation" includes at least a compound and/or composition of the present disclosure and a delivery agent.

*Fragment*: A "fragment," as used herein, refers to a portion. For example, fragments of proteins may comprise polypeptides obtained by digesting full-length protein. In some instances, a fragment of a protein includes at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250 or more amino acids. In some instances, fragments of an antibody include portions of an antibody.

*Functional*: As used herein, a "functional" biological molecule is a biological entity with a structure and in a form in which it exhibits a property and/or activity by which it is characterized.

*Heterocycle*: The term "heterocycle", "heterocyclyl", or "heterocyclic" as used herein refers to monocyclic, bicyclic or tricyclic ring systems having three to fourteen ring members in which one or more ring members is a heteroatom, wherein each ring in the system contains 3 to 7 ring members and is non-aromatic.

*Hotspot*: As used herein, a "hotspot" or a "mutational hotspot" refers to an amino acid position in a protein coding gene that is mutated (by substitutions) more frequently relative to elsewhere within the same gene.

*Hydrophilic*: As used herein, "hydrophilic" refers to a molecule that interacts with or has affinity for water.

*Hydrophobic*: As used herein, "hydrophobic" refers to a molecule that does not interact or have affinity for water.

*Immune cells*: the term "an immune cell", as used herein, refers to any cell of the immune system that originates from a hematopoietic stem cell in the bone marrow, which gives rise to two major lineages, a myeloid progenitor cell (which give rise to myeloid cells such as monocytes, macrophages, dendritic cells, megakaryocytes and granulocytes) and a lymphoid progenitor cell (which give rise to lymphoid cells such as T cells, B cells and natural killer (NK) cells). Exemplary immune system cells include a CD4+ T cell, a CD8+ T cell, a CD4- CD8-double negative T cell, a T γδ cell, a Tαβ cell, a regulatory T cell, a natural killer cell, and a dendritic cell. Macrophages and dendritic cells may be referred to as "antigen presenting cells" or "APCs," which are specialized cells that can activate T cells when a major histocompatibility complex (MHC) receptor on the surface of the APC complexed with a peptide interacts with a TCR on the surface of a T cell.

*Immunotherapy:* the term "immunotherapy" as used herein, refers to a type of treatment of a disease that uses immunological tools, such as monoclonal antibodies, receptor-immunoglobulin fusion proteins, vaccines and/or immune cells.

*In vitro:* As used herein, the term "in vitro" refers to events that occur in an artificial environment, e.g., in a test tube or reaction vessel, in cell culture, in a Petri dish, etc., rather than within an organism (e.g., animal, plant, or microbe).

*In vivo*: As used herein, the term "in vivo" refers to events that occur within an organism (e.g., animal, plant, or microbe or cell or tissue thereof).

*Linker*: As used herein, a linker refers to a moiety that connects two or more domains, moieties or entities. In one instance, a linker may comprise 10 or more atoms. In a further instance, a linker may comprise a group of atoms, e.g., 10-1,000 atoms, and can be comprised of the atoms or groups such as, but not limited to, carbon, amino, alkylamino, oxygen, sulfur, sulfoxide, sulfonyl, carbonyl, and imine. In some instances, a linker may comprise one or more nucleic acids comprising one or more nucleotides. In some instances, the linker may comprise an amino acid, peptide, polypeptide or protein. In some instances, a moiety bound by a linker may include, but is not limited to an atom, a chemical group, a nucleoside, a nucleotide, a nucleobase, a sugar, a nucleic acid, an amino acid, a peptide, a polypeptide, a protein, a protein complex, a payload (e.g., a therapeutic agent), or a marker (including, but not limited to a chemical, fluorescent, radioactive or bioluminescent marker). The linker can be used for any useful purpose, such as to form multimers or conjugates, as well as to administer a payload, as described herein. Examples of chemical groups that can be incorporated into the linker include, but are not limited to, alkyl, alkenyl, alkynyl, amido, amino, ether, thioether, ester, alkylene, heteroalkylene, aryl, or heterocyclyl, each of which can be optionally substituted, as described herein. Examples of linkers include, but are not limited to, unsaturated alkanes, polyethylene glycols (e.g., ethylene or propylene glycol monomeric units, e.g., diethylene glycol, dipropylene glycol, triethylene glycol, tripropylene glycol, tetraethylene glycol, or tetraethylene glycol), and dextran polymers, Other examples include, but are not limited to, cleavable moieties within the linker, such as, for example, a disulfide bond (-S-S-) or an azo bond (-N=N-), which can be cleaved using a reducing agent or photolysis. Non-limiting examples of a selectively cleavable bonds include an amido bond which may be cleaved for example by the use of tris(2-carboxyethyl) phosphine (TCEP), or other reducing agents, and/or photolysis, as well as an ester bond which may be cleaved for example by acidic or basic hydrolysis.

*Lipophilic*: As used herein, the term "lipophilic" refers to an affinity for lipids or fats.

*Metabolite*: Metabolites are the intermediate products of metabolic reactions catalyzed by enzymes that naturally occur within cells. This term is usually used to describe small molecules, fragments of larger biomolecules or processed products.

*Modified*: As used herein, the term "modified" refers to a changed state or structure of a molecule or entity as compared with a parent or reference molecule or entity. Molecules may be modified in many ways including chemically, structurally, and functionally. In some instances, compounds and/or compositions of the present disclosure are modified by the introduction of non-natural amino acids.

*Mutation*: As used herein, the term "mutation" refers to a change and/or alteration. In some instances, mutations may be changes and/or alterations to proteins (including peptides and polypeptides) and/or nucleic acids (including polynucleic acids). In some instances, mutations comprise changes and/or alterations to a protein and/or nucleic acid sequence. Such changes and/or alterations may comprise the addition, substitution and or deletion of one or more amino acids (in the case of proteins and/or peptides) and/or nucleotides (in the case of nucleic acids and or polynucleic acids). In some instances, wherein mutations comprise the addition and/or substitution of amino acids and/or nucleotides, such additions and/or substitutions may comprise 1 or more amino acid and/or nucleotide residues and may include modified amino acids and/or nucleotides. The resulting construct, molecule or sequence of a mutation, change or alteration may be referred to herein as a mutant.

*Neoantigen*: the term "neoantigen", as used herein, refers to a tumor antigen that is present in tumor cells but not normal cells and do not induce deletion of their cognate antigen specific T cells in thymus (i.e., central tolerance). These tumor neoantigens may provide a "foreign" signal, similar to pathogens, to induce an effective immune response needed for cancer immunotherapy. A neoantigen may be restricted to a specific tumor. A neoantigen be a peptide/protein with a missense mutation (missense neoantigen), or a new peptide with long, completely novel stretches of amino acids from novel open reading frames (neoORFs). The neoORFs can be generated in some tumors by out-of-frame insertions or deletions (due to defects in DNA mismatch repair causing microsatellite instability), gene-fusion, read-through mutations in stop codons, or translation of improperly spliced RNA (e.g., Saeterdal et al., Proc Natl Acad Sci USA, 2001, 98: 13255-13260).

*Off-target*: As used herein, "off target" refers to any unintended effect on any one or more target, gene, cellular transcript, cell, and/or tissue.

*Operably linked*: As used herein, the phrase "operably linked" refers to a functional connection between two or more molecules, constructs, transcripts, entities, moieties or the like.

*Phenyl*: As used herein, "phenyl" refers to a cyclic group of atoms with a formula C₆H₅.

*Payload or payload of interest (POI):* the terms "payload" and "payload of interest (POI)", as used herein, are used interchangeable. A payload of interest (POI) refers to any protein or compound whose function is to be altered. In the context of the present disclosure, the POI is a component in the immune system, including both innate and adaptive immune systems. Payloads of interest may be a protein, a fusion construct encoding a fusion protein, or non-coding gene, or variant and fragment thereof. Payload of interest may, when amino acid based, may be referred to as a protein of interest.

*Pharmaceutically acceptable excipients:* the term "pharmaceutically acceptable excipient," as used herein, refers to any ingredient other than active agents (e.g., as described herein) present in pharmaceutical compositions and having the properties of being substantially nontoxic and non-inflammatory in subjects. In some instances, pharmaceutically acceptable excipients are vehicles capable of suspending and/or dissolving active agents. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

*Pharmaceutically acceptable salts:* Pharmaceutically acceptable salts of the compounds described herein are forms of the disclosed compounds wherein the acid or base moiety is in its salt form (e.g., as generated by reacting a free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Pharmaceutically acceptable salts include the conventional non-toxic salts, for example, from non-toxic inorganic or organic acids. In some instances, a pharmaceutically acceptable salt is prepared from a parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al., Journal of Pharmaceutical Science, 66, 1-19 (1977). Pharmaceutically acceptable solvate: The term "pharmaceutically acceptable solvate," as used herein, refers to a crystalline form of a compound wherein molecules of a suitable solvent are incorporated in the crystal lattice. For example, solvates may be prepared by crystallization, recrystallization, or precipitation from a solution that includes organic solvents, water, or a mixture thereof. Examples of suitable solvents are ethanol, water (for example, mono-, di-, and tri-hydrates), N-methylpyrrolidinone (NMP), dimethyl sulfoxide (DMSO), N, N'-dimethylformamide (DMF), N, N'-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate." In some instances, the solvent incorporated into a solvate is of a type or at a level that is physiologically tolerable to an organism to which the solvate is administered (e.g., in a unit dosage form of a pharmaceutical composition).

*Piperazine:* As used herein, "piperazine" refers to a six-membered ring containing two nitrogen atoms at opposite positions in the ring.

*Protein of interest:* As used herein, the terms "proteins of interest" or "desired proteins" include those provided herein and fragments, mutants, variants, and alterations thereof.

*Purine:* As used herein, "purine" refers to an aromatic heterocyclic structure, wherein one of the heterocycles is an imidazole ring and one of the heterocycles is a pyrimidine ring.

*Pyrimidine:* As used herein, "pyrimidine" refers to an aromatic heterocyclic structure similar to benzene, but wherein two of the carbon atoms are replaced by nitrogen atoms.

*Pyridopyrimidine:* As used herein, "Pyridopyrimidine" refers to an aromatic heterocyclic structure, wherein one of the heterocycles is a purine ring and one of the heterocycles is a pyrimidine ring.

*Quinazoline:* As used herein, the term, "Quinazoline" refers to an aromatic heterocyclic structure, wherein one of the heterocycles is a benzene ring and one of the heterocycles is a pyrimidine ring.

*Stable:* As used herein "stable" refers to a compound or entity that is sufficiently robust to survive isolation to a useful degree of purity from a reaction mixture, and preferably capable of formulation into an efficacious therapeutic agent.

*Stabilized:* As used herein, the term "stabilize", "stabilized," "stabilized region" means to make or become stable. In some instances, stability is measured relative to an absolute value. In some instances, stability is measured relative to a secondary status or state or to a reference compound or entity.

*Standard CAR*: As used herein, the term "standard CAR" refers to the standard design of a chimeric antigen receptor. The components of a CAR fusion protein including the extracellular scFv fragment, transmembrane domain and one or more intracellular domains are linearly constructed as a single fusion protein.

*Stimulus response element (SRE)*: the term "stimulus response element (SRE), as used herein, is a component of an effector module which is joined, attached, linked to or associated with one or more payloads of the effector module and in some instances, is responsible for the responsive nature of the effector module to one or more stimuli. As used herein, the "responsive" nature of an SRE to a stimulus may be characterized by a covalent or noncovalent interaction, a direct or indirect association or a structural or chemical reaction to the stimulus. Further, the response of any SRE to a stimulus may be a matter of degree or kind. The response may be a partial response. The response may be a reversible response. The response may ultimately lead to a regulated signal or output. Such output signal may be of a relative nature to the stimulus, e.g., producing a modulatory effect of between 1 and 100 or a factored increase or decrease such as 2-fold, 3-fold, 4-fold, 5-fold, 10-fold or more. One non-limiting example of an SRE is a destabilizing domain (DD).

*Subject:* As used herein, the term "subject" or "patient" refers to any organism to which a composition in accordance with the disclosure may be administered, e.g., for experimental, diagnostic, prophylactic, and/or therapeutic purposes. Typical subjects include animals (e.g., mammals such as mice, rats, rabbits, non-human primates, and humans) and/or plants.

*T cell*: A T cell is an immune cell that produces T cell receptors (TCRs). T cells can be naïve (not exposed to antigen; increased expression of CD62L, CCR7, CD28, CD3, CD127, and CD45RA, and decreased expression of CD45RO as compared to TCM), memory T cells (TM) (antigen-experienced and long-lived), and effector cells (antigen-experienced, cytotoxic). TM can be further divided into subsets of central memory T cells (TCM, increased expression of CD62L, CCR7, CD28, CD127, CD45RO, and CD95, and decreased expression of CD54RA as compared to naive T cell and effector memory T cells (TEM, decreased expression of CD62L, CCR7, CD28, CD45RA, and increased expression of CD127 as compared to naive T cells or TCM). Effector T cells (TE) refers to antigen-experienced CD8+ cytotoxic T lymphocytes that have decreased expression of CD62L, CCR7, CD28, and are positive for granzyme and perforin as compared to TCM. Other exemplary T cells include regulatory T cells, such as CD4+ CD25+ (Foxp3+) regulatory T cells and Treg17 cells, as well as Tr1, Th3, CD8+CD28-, and Qa-1 restricted T cells.

*T cell receptor*: T cell receptor (TCR) refers to an immunoglobulin superfamily member having a variable antigen binding domain, a constant domain, a transmembrane region, and a short cytoplasmic tail, which is capable of specifically binding to an antigen peptide bound to a MHC receptor. A TCR can be found on the surface of a cell or in soluble form and generally is comprised of a heterodimer having α and β chains (also known as TCRα and TCRβ, respectively), or y and δ chains (also known as TCRγ and TCRδ, respectively). The extracellular portion of TCR chains (e.g., α-chain, β-chain) contains two immunoglobulin domains, a variable domain (e.g., α-chain variable domain or Vα, β-chain variable domain or Vβ) at the N-terminus, and one constant domain (e.g., α-chain constant domain or Cα and β-chain constant domain or Cβ,) adjacent to the cell membrane. Similar to immunoglobulin, the variable domains contain complementary determining regions (CDRs) separated by framework regions (FRs). A TCR is usually associated with the CD3 complex to form a TCR complex. As used herein, the term "TCR complex" refers to a complex formed by the association of CD3 with TCR. For example, a TCR complex can be composed of a CD3γ chain, a CD3δ chain, two CD3ε chains, a homodimer of CD3ζ chains, a TCRα chain, and a TCRβ chain. Alternatively, a TCR complex can be composed of a CD3γ chain, a CD3δ chain, two CD3ε chains, a homodimer of CD3ζ chains, a TCRγ chain, and a TCRδ chain. A "component of a TCR complex," as used herein, refers to a TCR chain (i.e., TCRα, TCRβ, TCRγ or TCRδ), a CD3 chain (i.e., CD3γ, CD3δ, CD3ε or CD3ζ), or a complex formed by two or more TCR chains or CD3 chains (e.g., a complex of TCRα and TCRβ, a complex of TCRγ and TCRδ, a complex of CD3ε and CD3δ, a complex of CD3v and CD3ε, or a sub-TCR complex of TCRα, TCRβ, CD3γ, CD3δ, and two CD3ε chains.

*Therapeutic Agent:* The term "therapeutic agent" refers to any agent that, when administered to a subject, has a therapeutic, diagnostic, and/or prophylactic effect and/or elicits a desired biological and/or pharmacological effect. Therapeutic agents of the present disclosure include any of the biocircuit components taught herein either alone or in combination with other therapeutic agents.

*Therapeutically effective amount*: As used herein, the term "therapeutically effective amount" means an amount of an agent to be delivered (e.g., nucleic acid, drug, therapeutic agent, diagnostic agent, prophylactic agent, etc.) that is sufficient, when administered to a subject suffering from or susceptible to an infection, disease, disorder, and/or condition, to treat, improve symptoms of, diagnose, prevent, and/or delay the onset of the infection, disease, disorder, and/or condition. In some instances, a therapeutically effective amount is provided in a single dose. In some instances, a therapeutically effective amount is administered in a dosage regimen comprising a plurality of doses. Those skilled in the art will appreciate that in some instances, a unit dosage form may be considered to comprise a therapeutically effective amount of a particular agent or entity if it comprises an amount that is effective when administered as part of such a dosage regimen.

*Triazine*: As used herein, "triazine" is a class of nitrogen containing heterocycles with a structure similar to benzene, but wherein three carbon atoms are replaced by nitrogen atoms.

*Treatment or treating*: As used herein, the terms "treatment" or "treating" denote an approach for obtaining a beneficial or desired result including and preferably a beneficial or desired clinical result. Such beneficial or desired clinical results include, but are not limited to, one or more of the following: reducing the proliferation of (or destroying) cancerous cells or other diseased, reducing metastasis of cancerous cells found in cancers, shrinking the size of the tumor, decreasing symptoms resulting from the disease, increasing the quality of life of those suffering from the disease, decreasing the dose of other medications required to treat the disease, delaying the progression of the disease, and/or prolonging survival of individuals.

*Survival*: As used herein, the term "survival" may be used to denote that a subject is alive at a particular point in time. In some instances, the subject may have been diagnosed with cancer. In one aspect, the subject may be an animal model, in which case the subject may be injected with cancer prior to the analysis of survival. Survival may be denoted as median survival which refers to how long patients survive with a disease in general or after a certain treatment. Survival may also be measured using survival ratio.

*Tune:* As used herein, the term "tune" means to adjust, balance or adapt one thing in response to a stimulus or toward a particular outcome. In one non-limiting example, the SREs and/or DDs of the present disclosure adjust, balance or adapt the function or structure of compositions to which they are appended, attached or associated with in response to particular stimuli and/or environments.

*Variant:* As used herein, the term "variant" refers to a first composition (e.g., a first DD or payload), that is related to a second composition (e.g., a second DD or payload, also termed a "parent" molecule). The variant molecule can be derived from, isolated from, based on or homologous to the parent molecule. The term variant can be used to describe either polynucleotides or polypeptides.

### VIII. EQUIVALENTS AND SCOPE

Those skilled in the art will recognize, or be able to ascertain using no more than routine experimentation, many equivalents to the specific embodiments in accordance with the disclosure described herein. The scope of the present disclosure is not intended to be limited to the above Description, but rather is as set forth in the appended claims.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or the entire group members are present in, employed in, or otherwise relevant to a given product or process.

It is also noted that the term "comprising" is intended to be open and permits but does not require the inclusion of additional elements or steps. When the term "comprising" is used herein, the term "consisting of" is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the disclosure, to the tenth of the unit of the lower limit of the range, unless the context clearly dictates otherwise.

In addition, it is to be understood that any particular embodiment of the present disclosure that falls within the prior art may be explicitly excluded from any one or more of the claims. Since such embodiments are deemed to be known to one of ordinary skill in the art, they may be excluded even if the exclusion is not set forth explicitly herein. Any particular embodiment of the compositions of the disclosure (e.g., any antibiotic, therapeutic or active ingredient; any method of production; any method of use; etc.) can be excluded from any one or more claims, for any reason, whether or not related to the existence of prior art.

It is to be understood that the words which have been used are words of description rather than limitation.

While the present disclosure has been described at some length and with some particularity with respect to the several described embodiments, it is not intended that it should be limited to any such particulars or embodiments or any particular embodiment, but it is to be construed with references to the appended claims. The present disclosure is further illustrated by the following nonlimiting examples.

### EXAMPLES

In the examples below, constructs and DDs are referred to by their identifiers (e.g., OT-001075). Additional information regarding the constructs and DDs may be found throughout the specification.

### Example 1. Generation of novel ligand responsive DDs by mutagenesis screening

### Study design

To engineer constructs that display ligand dependent stability, a candidate ligand binding domain (LBD) is selected and a cell-based screen using yellow fluorescent protein (YFP) as a reporter for protein stability is designed to identify mutants of the candidate LBD possessing the desired characteristics of a destabilizing domain: low protein levels in the absence of a ligand of the LBD, (i.e., low basal stability), large dynamic range, robust and predictable dose-response behavior, and rapid kinetics of degradation (Banaszynski, et al., (2006) Cell; 126(5): 995-1004). The candidate LBD binds to a desired ligand but not endogenous signaling molecules.

The candidate LBD sequence (as a template) is first mutated using a combination of nucleotide analog mutagenesis and error-prone PCR, to generate libraries of mutants based on the template candidate domain sequence. The libraries generated are cloned in-frame at either the 5'- or 3'-ends of the YFP gene, and a retroviral expression system is used to stably transduce the libraries of YFP fusions into NIH3T3 fibroblasts.

The transduced NIH3T3 cells are subjected to three to four rounds of sorting using fluorescence-activated cell sorting (FACS) to screen the libraries of candidate DDs. Transduced NIH3T3 cells are cultured in the absence of the high affinity ligand of the ligand binding domain (LBD), and cells that exhibit low levels of YFP expression are selected through FACS.

### Screening Strategy I

The selected cell population is cultured in the presence of the high affinity ligand of the ligand binding domain for a period of time (e.g., 24 hours), at which point cells are sorted again by FACS. Cells that exhibit high levels of YFP expression are selected through FACS and the selected cell population is split into two groups and treated again with the high affinity ligand of the ligand binding domain at different concentrations; one group is treated with the lower concentration of the ligand and the other is treated with a high concentration of the ligand, for a period of time (e.g., 24 hours), at which point cells are sorted again by FACS. Cells expressing mutants that are responsive to lower concentrations of the ligand are isolated.

The isolated cells responsive to the lower concentration of the ligand are treated with the ligand again and cells exhibiting low fluorescence levels are collected 4 hours following removal of the ligand from the media. This fourth sorting is designed to enrich cells that exhibit fast kinetics of degradation (Iwamoto et al., Chem Biol. 2010 Sep 24; 17(9): 981-988).

### Screening Strategy II

The selected cell population is subject to additional one or more sorts by FACS in the absence of high affinity ligand of LBD and cells that exhibit low levels of YFP expression are selected for further analysis. Cells are treated with high affinity ligand of the ligand binding domain, for a period of time (e.g. 24 hours), and sorted again by FACS. Cells expressing high levels of YFP are selected for through FACS. Cells with high expression of YFP are treated with ligand again and cells exhibiting low fluorescence levels are collected 4 hours following removal of the ligand from the media to enrich cells that exhibit fast kinetics of degradation. Any of the sorting steps may be repeated to identify DDs with ligand dependent stability.

The cells are recovered after sorting. The identified candidate cells are harvested and the genomic DNA is extracted. The candidate DDs are amplified by PCR and isolated. The candidate DDs are sequenced and compared to the LBD template to identify the mutations in candidate DDs.

### Example 2. Novel DDs derived from human PDE5 by site directed mutagenesis

To identify novel destabilizing domain mutations, mutagenic PCR was performed on the open reading frame of human PDE5 catalytic domain (SEQ ID NO: 3) using non-natural nucleotides. The mutant library was ligated in frame with an AcGFP reporter at C-terminus and cloned into pLVX-IRES- Puro vectors. The lentivirus library was then used to infect HEK 293T cells. Cells were selected with puromycin and the library was screened using screening strategies described in Example 1. DNA was extracted from the cell pool, cloned into vectors, and transformed into *E. coli.* Individual clones were sequenced and cloned in frame with a linker GGSGGGSGG (SEQ ID NO: 77) and AcGFP at C terminus into pLVX.IRES puro. The catalytic domain of wildtype hPDE5 was also cloned into pLVX.IRES. Puro and used as a control. HEK293 cells were transduced with individual clones and selected with puromycin.

HEK293 cells expressing OT-hPDE5 N terminus constructs were incubated with 10µM Sildenafil or vehicle control, DMSO for 48 hours and the stability of hPDE5 mutants was evaluated at the protein level. Cell lysates obtained from hPDE5N construct expressing cells were immunoblotted using the AcGFP antibody (Clonetech, Mountain View, CA). Samples were also immunoblotted with the GAPDH antibody to ensure uniform protein loading. The immunoblot demonstrated that OT-001078, OT-001080, OT-001076, OT-001083, OT-001084, OT-001070 showed an increase in PDE5-GFP protein levels with Sildenafil treatment when compared to DMSO treatment suggesting a Sildenafil dependent stabilization of the construct. Further, the hPDE5-GFP levels with DMSO treatment in OT-001078, OT-001080, and OT-001076 was lower than in the wildtype hPDE5 construct, OT-001075, indicating that these constructs are destabilized in the absence of ligand.

The GFP expression in cells expressing hPDE5 constructs was measured by FACS. HEK293 cells expressing OT-001075 to OT-001070 were incubated with 10µM Sildenafil or vehicle control, DMSO for 48 hours and the mean fluorescence intensity (MFI) was calculated. The stabilization ratio was calculated as the fold change in GFP intensity in Sildenafil treated samples compared to treatment with DMSO (i.e. in the absence of ligand) with the same construct. The destabilizing mutation co-efficient was calculated as the fold change in GFP intensity in the hPDE5 mutant constructs (OT-001078 to OT-001070) compared to the hPDE5 wildtype construct (OT-001075) in the absence of the ligand. Destabilizing mutation co-efficient less than 1 and stabilization ratios greater than 1 are desired in DDs. The results and ratios are presented in Table 21.

**Table 21: GFP expression in hPDE5 derived DDs**

| **Constructs** | **MFI** | | **Stabilization ratio** | **Destabilizing mutation co-efficient** |
|---|---|---|---|---|
| | **DMSO** | **Sildenafil** | | |
| 01-001075 | 11064 | 25789 | 2.33 | |
| 01-001078 | 1649 | 12285 | 7.45 | 0.15 |
| 01-001080 | 1184 | 7303 | 6.17 | 0.11 |
| OT-001081 | 795 | 1065 | 1.34 | 0.07 |
| 01-001074 | 6924 | 29491 | 4.26 | 0.63 |
| OT-001076 | 1542 | 19211 | 12.46 | 0.14 |
| 01-001082 | 3038 | 7078 | 2.33 | 0.27 |
| 01-001083 | 1134 | 13189 | 11.63 | 0.10 |
| OT-001084 | 744 | 15587 | 20.95 | 0.07 |
| 01-001070 | 5053 | 21755 | 4.31 | 0.46 |

As shown in Table 21, all constructs demonstrated a stabilization ratio greater than one, indicating that all constructs show ligand (Sildenafil) dependent stabilization. The stabilization ratios of OT-001076, OT-001083, and OT-001084 was greater than 10 indicating strong ligand dependent stabilization. Constructs OT-001078, OT-001080, OT-001074, and OT-001070 showed stabilization ratio in the range of 2 to 10 indicating modest ligand dependent stabilization. The destabilizing mutation coefficient observed with all constructs was less than 1 indicating that all hPDE5 mutants are destabilized as compared to the wildtype hPDE5. Notably, constructs OT-001078, OT-001080, OT-001081, OT-001076, OT-001083, and OT-001084 demonstrated destabilizing mutation coefficients less than 0.2 indicating strong destabilizing in the absence of ligand. OT-001076, OT-001083, and OT-001084 were identified as mutants with desirable characteristics of a DD i.e., low basal expression in the absence of ligand and high expression in the presence of ligand.

### Example 3. Sildenafil dependent stabilization of hPDE5 mutants

HEK293 cells expressing OT-001076, OT-001083, or OT-001084 constructs were incubated with varying concentrations of Sildenafil ranging from 0.005 µM to 10µM, or vehicle control (DMSO) for 48 hours. The stability of hPDE5 mutants was measured using FACS and mean fluorescence intensity (MFI) of GFP was calculated. The stabilization ratio was calculated as the fold change in GFP intensity in Sildenafil treated samples compared to treatment with DMSO (i.e. absence of ligand) with the same construct. Stabilization ratio greater than 1 is desired in DDs. The MFI and stabilization ratios are presented in Table 22.

**Table 22: Sildenafil Dose titration**

| **Sildenafil dose (µM)** | **OT-001076** | | **OT-001083** | | **OT-001084** | |
|---|---|---|---|---|---|---|
| | MFI | Stabilization ratio | MFI | Stabilization ratio | MFI | Stabilization ratio |
| Untreated | 7036 | | 4388 | | 3506 | |
| 0.0005 | 6848 | 0.97 | 4422 | 1.01 | 3513 | 1.00 |
| 0.0015 | 7239 | 1.03 | 4557 | 1.04 | 3473 | 0.99 |
| 0.0046 | 7218 | 1.03 | 4397 | 1.00 | 3539 | 1.01 |
| 0.0137 | 7127 | 1.01 | 4274 | 0.97 | 3683 | 1.05 |
| 0.041 | 8097 | 1.15 | 4395 | 1.00 | 4171 | 1.19 |
| 0.123 | 12248 | 1.74 | 4875 | 1.11 | 6463 | 1.84 |
| 0.37 | 22018 | 3.13 | 6753 | 1.54 | 13076 | 3.73 |
| 1.11 | 34171 | 4.86 | 10495 | 2.39 | 23468 | 6.69 |
| 3.33 | 49220 | 7.00 | 17960 | 4.09 | 34615 | 9.87 |
| 10 | 67084 | 9.53 | 32480 | 7.40 | 47793 | 13.63 |

As shown in Table 22, all three constructs showed an increase in stabilization ratio with increasing doses of Sildenafil indicating a ligand dose-dependent stabilization of hPDE5 mutants. The half maximal effective concentration or EC₅₀ was approximately 1µM.

Sildenafil dependent stabilization of hPDE5 mutants was also measured over a period of time. HEK293 cells expressing OT-001076, OT-001083, and OT-001084 were treated with 10µM of Sildenafil for 2, 4, 6, 16, 24, and 48 hours. The stability of hPDE5 mutants was measured using FACS and mean fluorescence intensity (MFI) of GFP was calculated. The stabilization ratio was calculated as the fold change in GFP intensity in Sildenafil treated samples compared to treatment with DMSO (i.e. absence of ligand) with the same construct. Stabilization ratio greater than 1 is desired in DDs. The MFI and stabilization ratios are presented in Table 23.

**Table 23: Time course of Sildenafil treatment**

| **Time (h)** | **OT-001076** | | **OT-001083** | | **OT-001084** | |
|---|---|---|---|---|---|---|
| | MFI | Stabilization ratio | MFI | Stabilization ratio | MFI | Stabilization ratio |
| Parental | 675 | - | 667 | - | 673 | - |
| 0 | 7921 | - | 5304 | - | 4195 | - |
| 2 | 12088 | 1.54 | 7290 | 1.37 | 6337 | 1.51 |
| 4 | 14046 | 1.77 | 8675 | 1.64 | 8159 | 1.95 |
| 6 | 16710 | 2.11 | 10929 | 2.06 | 10203 | 2.43 |
| 16 | 39029 | 4.93 | 19034 | 3.59 | 26279 | 6.26 |
| 24 | 51534 | 6.51 | 27246 | 5.14 | 36516 | 8.71 |
| 48 | 70811 | 8.94 | 34796 | 6.56 | 47407 | 11.30 |

As shown in Table 23, all three constructs showed an increase in stabilization ratio with increasing duration of treatment with Sildenafil indicating a time-dependent increase in stabilization of hPDE5 mutants.

### Example 4. Vardenafil dependent stabilization of hPDE5 DDs

Vardenafil has an IC ₅₀ of 0.7 nM and is a more potent inhibitor of hPDE5 than Sildenafil which has an IC ₅₀ of 3.9 nM (Doggrell S, et al. (2007) Int J Impot. Res 19(3):281-95). To test if Vardenafil is capable of stabilizing hPDE5 DDs, HEK293 cells expressing OT-001076, OT-001083, and OT-001084 were treated with Vardenafil at concentrations ranging from 0.0005µM to 10µM for 48 hours. The stability of hPDE5 mutants was measured using FACS and mean fluorescence intensity (MFI) of GFP was calculated. The stabilization ratio was calculated as the fold change in GFP intensity in Vardenafil treated samples compared to treatment with DMSO (i.e. absence of ligand) with the same construct. Stabilization ratio greater than 1 is desired in DDs. The MFI and stabilization ratios are presented in Table 24.

**Table 24: Vardenafil Dose Titration**

| **Vardenafil dose (µM)** | **OT-001076** | | **OT-001083** | | **OT-001084** | |
|---|---|---|---|---|---|---|
| | MFI | Stabilization ratio | MFI | Stabilization ratio | MFI | Stabilization ratio |
| Parental | 988 | - | 999 | - | 980 | - |
| 0 | 11287 | - | 7971 | - | 5841 | - |
| 0.0005 | 11770 | 1.04 | 7894 | 0.99 | 5954 | 1.02 |
| 0.0015 | 12965 | 1.15 | 8099 | 1.02 | 6601 | 1.13 |
| 0.0046 | 18739 | 1.66 | 8897 | 1.12 | 9946 | 1.70 |
| 0.0137 | 36940 | 3.27 | 12440 | 1.56 | 21894 | 3.75 |
| 0.041 | 58755 | 5.21 | 18527 | 2.32 | 39580 | 6.78 |
| 0.123 | 91994 | 8.15 | 33614 | 4.22 | 63622 | 10.89 |
| 0.37 | 125140 | 11.09 | 61010 | 7.65 | 86481 | 14.81 |
| 1.11 | 144927 | 12.84 | 95149 | 11.94 | 105930 | 18.14 |
| 3.33 | 172990 | 15.33 | 134306 | 16.85 | 123757 | 21.19 |
| 10 | 188068 | 16.66 | 166236 | 20.86 | 139165 | 23.83 |

As shown in Table 24, all three constructs showed an increase in stabilization ratio with increasing doses of Vardenafil indicating a Vardenafil dose-dependent stabilization of hPDE5 DDs. The half maximal effective concentration or EC₅₀ was approximately 0.1-0.3µM which was less than the EC₅₀ observed with sildenafil (~1µM) indicating that Vardenafil may stabilize hPDE5 DDs more potently than sildenafil.

The ability of Vardenafil to stabilize hPDE5 derived DDs was measured in cell lines stably transduced with hPDE5 DDs e.g. HEK293 cells, HCT-116 cells, and SKOV-3 cells. Cells were transduced with OT-001076, OT-001083, and OT-001084 constructs and incubated with 1µM or 10 µM Vardenafil or with DMSO for 48 hours. The stability of hPDE5 mutants was measured using FACS and mean fluorescence intensity (MFI) of GFP was calculated. The stabilization ratio was calculated as the fold change in GFP intensity in Vardenafil treated samples compared to treatment with DMSO (i.e. absence of ligand) with the same construct. Stabilization ratio greater than 1 is desired in DDs. The MFI and stabilization ratios are presented in Table 25.

**Table 25: Vardenafil Dose Titration**

| **Dose** | **OT-001076 (hPDE5-F736A)** | **OT-001083 (hPDE5-Y728L)** | **OT-001084 (hPDE5-R732L)** | | | |
|---|---|---|---|---|---|---|
| | MFI | Stabilization ratio | MFI | Stabilization ratio | MFI | Stabilization ratio |
| DMSO | 9604 | - | 5191 | - | 3605 | - |
| Vardenafil (1µM) | 110603 | 11.52 | 68816 | 13.26 | 81484 | 22.60 |
| Vardenafil (10µM) | 143351 | 14.93 | 123150 | 23.72 | 106403 | 29.52 |

As shown in Table 25, all three constructs showed an increase in stabilization ratio with both doses of Vardenafil indicating a ligand dose-dependent stabilization of hPDE5 DDs. These data are consistent with the results observed with the dose response of Vardenafil observed in Table 24.

### Example 5. Characterization of hPDE5 combination mutants

Single destabilizing mutants identified were combined to test if the combining two or more mutations generates domains with greater destabilizing potential either additively or synergistically. Desirable qualities of a DD include, low expression of the SRE in the absence of ligand and ligand dependent stabilization of the SRE. Constructs were generated using DDs linked to GFP using an SG linker. HCT-116 cells were stably transduced with the constructs described above and treated with 10µM stabilizing ligands, Sildenafil, or Vardenafil or DMSO for 48 hours. As a control, parental untransduced cells and cells expressing OT-001075, or OT-001224 were also included in the analysis. The mean fluorescence intensity (MFI) of GFP was analyzed by FACS. The stabilization ratio was calculated as the fold change in GFP intensity in Sildenafil treated samples compared to treatment with DMSO (i.e. in the absence of ligand) with the same construct. The destabilizing mutation coefficient was calculated as the fold change in GFP intensity in the hPDE5 mutant constructs compared to the hPDE5 wildtype construct (OT-001216) in the absence of the ligand. Destabilizing mutation coefficient less than 1 are desirable. Stabilization ratios greater than 1 are desired in DDs. The results and ratios are presented in Table 26.

**Table 26: Expression of hPDE5 combination mutants**

| **Construct** | **MFI** | | | **Sildenafil Stabilization ratio** | **Vardenafil Stabilization ratio** | **Destabilizing mutation coefficient** |
|---|---|---|---|---|---|---|
| | **DMSO** | **Sildenafil** | **Vardenafil** | | | |
| Parental | 2093 | 3735 | 3200 | 1.78 | 1.53 | - |
| OT-001075 | 42973 | 53736 | 49344 | 1.25 | 1.15 | - |
| OT-001224 | 150356 | 187845 | 170129 | 1.25 | 1.13 | - |
| OT-001216 | 64942 | 66860 | 67388 | 1.03 | 1.04 | - |
| OT-001222 | 12633 | 13862 | 53848 | 1.10 | 4.26 | 0.19 |
| OT-001223 | 8625 | 48592 | 109667 | 5.63 | 12.72 | 0.13 |

As shown in Table 26, only the OT-001223 construct with R732L and D764N mutations showed Sildenafil dependent stabilization with a ratio of 5.63. Similar results were obtained with OT-001223 upon treatment with Vardenafil. OT-001222 however only showed Vardenafil dependent stabilization with a ratio of 4.26. Both constructs were destabilized in the absence of ligand. As expected, hPDE5 wildtype constructs did not show any significant ligand dependent stabilization. These data suggest that the strategy of combining mutations may be used to identify improved DDs as well as DDs that are stabilized by specific ligands.

Protein expression analysis of the combination mutants was performed in parallel via western blot. HCT 116 cells expressing OT-001222, and OT-001223 were treated with 10µM Vardenafil for 24 hours and immunoblotted for AcGFP using anti AcGFP antibodies (catalog no. 63277, Clonetech, Mountain View, California). GAPDH levels were also analyzed to ensure even protein loading. OT-001222 with hPDE5 (F736A, D764N) and OT-001223 with hPDE5 (R732L, D764N) constructs consisting showed Vardenafil dependent stabilization of AcGFP protein levels as measured via western blotting. This result is consistent with the FACS analysis.

The response of hPDE5 combination mutants to increasing doses of sildenafil was tested. HCT116 cells transduced with hPDE5 constructs were treated with Sildenafil for 24 hours at doses ranging from 0.04µM to 30 µM. Mean fluorescence intensity (MFI) was measured by FACS. Parental HCT116 and cells expressing OT-001216 wildtype construct were also included as controls. The response of the combination mutants to Sildenafil was compared to the response of the single mutant construct OT-001217. The stabilization ratio was calculated as the fold change in GFP intensity in Sildenafil treated samples compared to treatment with DMSO (i.e. in the absence of ligand) with the same construct. The destabilizing mutation coefficient ratio was calculated as the fold change in GFP intensity in the hPDE5 mutant constructs compared to the hPDE5 wildtype construct (OT-001216) in the absence of the ligand. Destabilizing mutation coefficient ratios less than 1 are desirable. Stabilization ratios greater than 1 are desired in DDs. The results and stabilization ratios are presented in Table 27.

**Table 27: Sildenafil dose response of combination mutants**

| **Dose (µM Sildenafil)** | **OT-001216** | | **OT-001217** | | **OT-001222** | | **OT-001223** | |
|---|---|---|---|---|---|---|---|---|
| | MFI | Stabilization ratio | MFI | Stabilization ratio | MFI | Stabilization ratio | MFI | Stabilization ratio |
| DMSO | 62116 | - | 6172 | - | 10736 | - | 7093 | - |
| 30 | 75478 | 1.22 | 47992 | 7.78 | 26837 | 2.50 | 69864 | 9.85 |
| 10 | - | - | 27693 | 4.49 | 13263 | 1.24 | 34587 | 4.88 |
| 3.33 | - | - | 23549 | 3.82 | 10384 | 0.97 | 22392 | 3.16 |
| 1.11 | - | - | 15952 | 2.58 | 9636 | 0.90 | 10353 | 1.46 |
| 0.37 | - | - | 12739 | 2.06 | 9802 | 0.91 | 6961 | 0.98 |
| 0.12 | - | - | 6441 | 1.04 | 8619 | 0.80 | 6603 | 0.93 |
| 0.04 | - | - | 5299 | 0.86 | 8688 | 0.81 | 5948 | 0.84 |

As shown in Table 27, the stabilization ratios for OT-001223 construct obtained across multiple doses of sildenafil were higher than the single mutant OT-001217 construct. The combination mutant 025, achieved stabilization ratios much lower than the OT-001223 construct for any given ligand dose. The destabilizing mutation co-efficient ratios obtained for the OT-001217 (ratio= 0.1), OT-001222 (ratio= 0.17), and OT-001223 (ratio =0.1) indicate that all constructs are destabilized in the absence of the ligand. These data indicate that the combination mutant OT-001223 is strong DD candidate when the stabilizing ligand is Sildenafil.

The response of hPDE5 combination mutants to increasing doses of Vardenafil was tested. HCT116 cells transduced with hPDE5 constructs were treated with Vardenafil for 24 hours at doses ranging from 0.04 µM to 30 µM. Mean fluorescence intensity (MFI) was measured by FACS. Parental HCT116 and cells expressing OT-001216 comprising hPDE5 wildtype construct were also included as controls. The response of the combination mutants to Vardenafil was compared to the response of the single mutant construct OT-001217. The stabilization ratio was calculated as the fold change in GFP intensity in Vardenafil treated samples compared to treatment with DMSO (i.e. in the absence of ligand) with the same construct. The destabilizing mutation coefficient ratio was calculated as the fold change in GFP intensity in the hPDE5 mutant constructs compared to the hPDE5 wildtype construct (OT-001216) in the absence of the ligand. Destabilizing mutation co-efficient ratios less than 1 are desirable. Stabilization ratios greater than 1 are desired in DDs. The results and stabilization ratios are presented in Table 28.

**Table 28: Vardenafil dose response of combination mutants**

| **Dose (µM Vardenafil)** | **OT-001216** | | **OT-001217** | | **OT-001222** | | **OT-001223** | |
|---|---|---|---|---|---|---|---|---|
| | MFI | Stabilization ratio | MFI | Stabilization ratio | MFI | Stabilization ratio | MFI | Stabilization ratio |
| **DMSO** | 62116 | - | 7829 | - | 7694 | - | 5156 | |
| 30 | 70898 | 1.14 | 75323 | 9.62 | 46109 | 5.99 | 55843 | 10.83 |
| 10 | - | - | 39089 | 4.99 | 29971 | 3.90 | 35191 | 6.83 |
| 3.33 | - | - | 35564 | 4.54 | 29520 | 3.84 | 40408 | 7.84 |
| 1.11 | - | - | 30879 | 3.94 | 12634 | 1.64 | 32868 | 6.37 |
| 0.37 | - | - | 24847 | 3.17 | 6667 | 0.87 | 14543 | 2.82 |
| 0.12 | - | - | 16866 | 2.15 | 7314 | 0.95 | 10676 | 2.07 |
| 0.04 | - | - | 14745 | 1.88 | 6174 | 0.80 | 5497 | 1.07 |

As shown in Table 28, the stabilization ratios for OT-001223 construct obtained across multiple doses of Vardenafil were higher than the single mutant OT-001217 construct. The combination mutant 025, achieved stabilization ratios much lower than the OT-001223 construct for any given ligand dose. The destabilizing mutation coefficient obtained for the OT-001217 (ratio= 0.13), OT-001222 (ratio= 0.12), and OT-001223 (ratio =0.08) indicate that all constructs are destabilized in the absence of the ligand. These data indicate that the combination mutant OT-001223 is strong DD candidate when the stabilizing ligand is Vardenafil.

The response of hPDE5 combination mutants to increasing doses of Tadalafil was tested. HCT116 cells transduced with hPDE5 constructs were treated with Tadalafil for 24 hours at doses ranging from 0.14 to 100 µM. Mean fluorescence intensity (MFI) was measured by FACS. Parental HCT116 and cells expressing OT-001216 comprising hPDE5 wildtype construct were also included as controls. The response of the combination mutants to Tadalafil was compared to the response of the single mutant construct OT-001217. The stabilization ratio was calculated as the fold change in GFP intensity in Tadalafil treated samples compared to treatment with DMSO (i.e. in the absence of ligand) with the same construct. The destabilizing mutation coefficient was calculated as the fold change in GFP intensity in the hPDE5 mutant constructs compared to the hPDE5 wildtype construct (OT-001216) in the absence of the ligand. Destabilizing mutation coefficient less than 1 are desirable. Stabilization ratios greater than 1 are desired in DDs. The results and stabilization ratios are presented in Table 29.

**Table 29: Tadalafil dose response of combination mutants**

| **Dose (µM Vardenafil)** | **OT-001216** | | **OT-001217** | | **OT-001222** | | **OT-001223** | |
|---|---|---|---|---|---|---|---|---|
| | MFI | Stabilization ratio | MFI | Stabilization ratio | MFI | Stabilization ratio | MFI | Stabilization ratio |
| DMSO | 36453 | - | 3806 | - | 6043 | - | 3746 | - |
| 100 | 58468 | 1.60 | 34041 | 8.94 | 23542 | 3.90 | 30029 | 8.02 |
| 33.33 | - | - | 44713 | 11.75 | 20464 | 3.39 | 30028 | 8.02 |
| 11.11 | - | - | 30641 | 8.05 | 13789 | 2.28 | 24499 | 6.54 |
| 3.7 | - | - | 26480 | 6.96 | 9515 | 1.57 | 17317 | 4.62 |
| 1.23 | - | - | 20458 | 5.38 | 8634 | 1.43 | 9283 | 2.48 |
| 0.41 | - | - | 13206 | 3.47 | 6141 | 1.02 | 6044 | 1.61 |
| 0.14 | - | - | 8512 | 2.24 | 5768 | 0.95 | 4682 | 1.25 |

As shown in Table 29, the stabilization ratios for OT-001223 construct obtained across multiple doses of Tadalafil were higher than the single mutant OT-001217 construct, except at the 100 µM concentration of the drug. The combination mutant OT-001222, achieved stabilization ratios much lower than the OT-001223 construct for any given ligand dose. The destabilizing mutation coefficient obtained for the OT-001217 (ratio= 0.1), OT-001222 (ratio= 0.17), and OT-001223 (ratio =0.1) indicate that all constructs are destabilized in the absence of the ligand. These data indicate that the combination mutant OT-001223 is strong DD candidate when the stabilizing ligand is Tadalafil.

The stabilization ratios obtained for the highest concentrations of all three ligands, Sildenafil, Vardenafil and Tadalafil for each of the constructs were compared and are shown Table 30.

**Table 30: Comparative analysis of hPDE5 ligands**

| Ligand | Stabilization ratio | | | |
|---|---|---|---|---|
| | OT-001216 | OT-001217 | OT-001222 | OT-001223 |
| Sildenafil (30 µM) | 1.22 | 7.78 | 2.5 | 9.85 |
| Vardenafil (30 µM) | 1.14 | 9.62 | 5.99 | 10.83 |
| Tadalafil (100 µM) | 1.60 | 8.94 | 3.9 | 8.02 |

As shown in Table 30, based on the stabilization ratios it was evident that OT-001223 was effectively stabilized by all three ligands, in comparison to both the single mutant construct as well as the other combination mutant construct (OT-001222). Both combination mutants were most effectively stabilized by Vardenafil, which is the most potent inhibitor of hPDE5. Construct OT-001222 was stabilized more effectively by Tadalafil than Sildenafil, although only by a small margin-this observation is noteworthy as Sildenafil is a much stronger inhibitor of hPDE5 than Tadalafil

The response of hPDE5 combination mutants to increasing duration treatment with sildenafil was tested. HCT116 cells transduced with hPDE5 constructs were treated with Sildenafil at 0.5 or 5 µM for 0-72 hours. Mean fluorescence intensity (MFI) was measured by FACS. The response of the combination mutants to Sildenafil was compared to the response of the single mutant construct OT-001217. The stabilization ratio was calculated as the fold change in GFP intensity in Sildenafil treated samples compared to treatment with DMSO (i.e. in the absence of ligand) with the same construct. The destabilizing mutation coefficient was calculated as the fold change in GFP intensity in the hPDE5 mutant constructs compared to the hPDE5 wildtype construct (OT-001216) in the absence of the ligand. Destabilizing mutation coefficient less than 1 are desirable. Stabilization ratios greater than 1 are desired in DDs. The results and stabilization ratios for time course experiments with 5 or 0.5 µM are presented in Table 31 and Table 32 respectively.

**Table 31: Response to Sildenafil (5 µM) over time**

| **Time (hours)** | **OT-001217** | | **OT-001222** | | **OT-001223** | |
|---|---|---|---|---|---|---|
| | MFI | Stabilization ratio | MFI | Stabilization ratio | MFI | Stabilization ratio |
| 0 | 2668 | - | 3364 | - | 2769 | - |
| 72 | 58479 | 21.92 | 26995 | 8.02 | 90413 | 32.65 |
| 48 | 46604 | 17.47 | 22061 | 6.56 | 65233 | 23.56 |
| 24 | 20955 | 7.85 | 16705 | 4.97 | 27962 | 10.10 |
| 20 | 15529 | 5.82 | 13280 | 3.95 | 21603 | 7.80 |
| 16 | 14855 | 5.57 | 12280 | 3.65 | 17600 | 6.36 |
| 12 | 9097 | 3.41 | 9199 | 2.73 | 10451 | 3.77 |
| 8 | 5672 | 2.13 | 7021 | 2.09 | 6179 | 2.23 |
| 4 | 4236 | 1.59 | 6314 | 1.88 | 4266 | 1.54 |

**Table 32: Response to Sildenafil (0.5 µM) over time**

| Time (hours) | OT-001217 | | OT-001222 | | OT-001223 | |
|---|---|---|---|---|---|---|
| | MFI | Stabilization ratio | MFI | Stabilization ratio | MFI | Stabilization ratio |
| 0 | 2727 | - | 3245 | - | 2611 | - |
| 72 | 58786 | 21.56 | 6906 | 2.13 | 31835 | 12.19 |
| 48 | 45754 | 16.78 | 6682 | 2.06 | 27231 | 10.43 |
| 24 | 20524 | 7.53 | 6144 | 1.89 | 17848 | 6.84 |
| 20 | 16142 | 5.92 | 6003 | 1.85 | 13318 | 5.10 |
| 16 | 14469 | 5.31 | 5600 | 1.73 | 11390 | 4.36 |
| 12 | 9058 | 3.32 | 5122 | 1.58 | 8126 | 3.11 |
| 8 | 5461 | 2.00 | 5083 | 1.57 | 5742 | 2.20 |
| 4 | 4127 | 1.51 | 5322 | 1.64 | 3870 | 1.48 |

As shown in Table 31 and Table 32, the stabilization ratios for OT-001223 construct obtained over time at 5 µM dose of Sildenafil were higher than the single mutant OT-001217 construct. However, this pattern was reversed at lower dose of 0.5 µM where the single mutant construct showed greater stabilization. The combination mutant OT-001222, achieved stabilization ratios much lower than the OT-001223 construct for both ligand doses at any given time. These data indicate that the choice of a suitable DD may sometime depend on the duration of the ligand treatment desired as well as the concentration of the ligand that is available for use in a system.

### Example 6. Vardenafil dose response curve in hPDE5 mutants

Combination mutants were generated as described in Example 9. The response of hPDE5 combination mutants to increasing doses of Vardenafil was tested in HCT116 cells transduced with hPDE5 constructs were treated with Vardenafil for 48 hours at doses ranging from 0.1 to 10 µM or vehicle control (DMSO). Mean fluorescence intensity (MFI) of GFP was measured by FACS. The response of the combination mutants to Vardenafil was compared to the response of the single mutant construct OT-001217. The fold change in MFI over DMSO is presented in Table 33.

**Table 33: Vardenafil dose response**

| **Sildenafil (µM)** | **OT-001217** | **OT-001222** | **OT-001223** |
|---|---|---|---|
| 0.01 | 4026 | 4444 | 3815 |
| 0.04 | 6970 | 4934 | 3839 |
| 0.12 | 14832 | 5133 | 4870 |
| 0.37 | 26429 | 5007 | 8107 |
| 1.11 | 34932 | 5751 | 21857 |
| 3.33 | 49739 | 10516 | 49105 |
| 10.00 | 51306 | 24927 | 64734 |

As shown in Table 33, the ligand dependent stabilization obtained for OT-001217 construct was observed at lower doses i.e. 0.1 and 0.3 micro molar concentrations of Vardenafil compared to the other two constructs, indicating that very low doses of the ligand are sufficient to stabilize this hPDE5 mutant. The OT-001223 construct did not show ligand dependent stabilization at similar doses. However, at the highest dose of Vardenafil the GFP expression obtained was much higher than OT-001217. In contrast, OT-001222 showed very little expression of GFP at lower doses and only modest GFP expression compared to the other two constructs at the higher doses. These experiments indicate that combination of single hPDE5 DD mutations produces synergistic effects not predicted by the properties of the single mutants. Thus, based on the properties of the payload of interest and the expression levels desired, suitable hPDE5 DDs may be selected.

### Example 7. Sildenafil and Tadalafil dose response curve in hPDE5 mutants

Combination mutations were generated as previously described in Example 9. The response of HCT 116 cells expressing hPDE5 combination mutants to increasing doses ranging from 0.1 µM to 100 µM of Tadalafil and Sildenafil for 48 hours or vehicle control (DMSO) was tested. Mean fluorescence intensity (MFI) was measured by FACS. The response of the combination mutants to ligand treatment was compared to the single mutant construct, OT-001217. The MFI with ligand treatment is shown in Table 34 and Table 35.

**Table 34: Tadalafil dose response**

| **Tadalafil (µM)** | **OT-001217** | **OT-001222** | **OT-001223** |
|---|---|---|---|
| 0.14 | 3616 | 4328 | 3720 |
| 0.41 | 5811 | 5002 | 3804 |
| 1.23 | 11694 | 5503 | 4810 |
| 3.70 | 24185 | 5744 | 7893 |
| 11.11 | 40380 | 8299 | 24617 |
| 33.33 | 57233 | 15831 | 52927 |
| 100.00 | 72587 | 31020 | 84334 |

**Table 35: Sildenafil dose response**

| **Sildenafil (µM)** | **OT-001217** | **OT-001222** | **OT-001223** |
|---|---|---|---|
| 0.14 | 3771 | 4398 | 3401 |
| 0.41 | 5463 | 4832 | 3681 |
| 1.23 | 10479 | 5031 | 4477 |
| 3.70 | 22441 | 4974 | 7625 |
| 11.11 | 35561 | 5675 | 24104 |
| 33.33 | 52029 | 9677 | 51416 |
| 100.00 | 101226 | 37494 | 136010 |

As shown in Table 34 and 35, ligand dose dependent stabilization was obtained with all three constructs. The dose dependent stabilization at lower doses (e.g., up to 11 µM of Sildenafil and Tadalafil) is more evident in the single mutant construct, OT-001217, compared to the other two constructs. At higher concentrations of ligand, both OT-001222 and OT-001223 showed ligand dependent stabilization. In fact, OT-001223 construct demonstrated much higher MFI values at 33 and 100 µM, compared to both OT-001217 and OT-001222. These experiments indicate that combination of single hPDE5 DD mutations produces synergistic effects not predicted by the properties of the single mutants. Thus, suitable hPDE5 DDs may be selected based on the properties of the payload of interest and the expression levels desired,

### Example 8. Kinetics of hPDE5 mutants

The off kinetics of hPDE5 mutants, OT-001217 and OT-001223 was tested by treating HCT 116 cells expressing these constructs or the wild type control construct OT-001216. Cells were treated with ligand for 48 hours. The media containing the ligand was removed and fresh media without the ligand was added. MFI was analyzed by FACS at 0 hours (i.e., prior to the beginning of the experiment), 48 hours after ligand treatment, as well as at several time points after the ligand washout, up until 96 hours. The stabilization ratio was calculated as the fold change in GFP intensity in ligand treated samples compared to the GFP intensity in the absence of ligand with the same construct. The results are shown in Table 36.

**Table 36: Off-Kinetics**

| **Time (hrs)** | **OT-001217** | | **OT-001223** | | **OT-001216 (WT)** | |
|---|---|---|---|---|---|---|
| | MFI | Stabilization Ratio | MFI | Stabilization Ratio | MFI | Stabilization Ratio |
| 0 | 3675 | - | 3648 | - | 45512 | - |
| 48 | 71538 | 19.47 | 57105 | 15.65 | 102137 | 2.24 |
| 50 | 63832 | 17.37 | 41074 | 11.26 | 97225 | 2.14 |
| 52 | 51544 | 14.03 | 26926 | 7.38 | 96264 | 2.12 |
| 54 | 41800 | 11.37 | 16391 | 4.49 | 95410 | 2.10 |
| 56 | 25521 | 6.94 | 7700 | 2.11 | 81733 | 1.80 |
| 60 | 19598 | 5.33 | 5635 | 1.54 | 84323 | 1.85 |
| 72 | 8062 | 2.19 | 4062 | 1.11 | 70151 | 1.54 |
| 80 | 6427 | 1.75 | 3587 | 0.98 | 63208 | 1.39 |
| 96 | 4319 | 1.18 | 3443 | 0.94 | 55918 | 1.23 |

As shown in Table 36, the stabilization ratio of hPDE5 mutants decreased following removal of the ligand. The stabilization ratio values decreased more quickly in the combination mutant OT-001223 compared to OT-001217 construct suggesting the destabilization of the DD is achieved more quickly in the OT-001223 construct. The ligand dependent stabilization as indicated by the stabilization ratio, achieved with OT-001223 construct is lower than OT-001217 construct, which may also contribute to the superior off kinetics seen with the OT-001223 construct.

### Example 9. Behavior of hPDE5 C terminal mutants

The ability of hPDE5 mutants to tune the expression of payloads when appended to the C terminal of the payload was tested. OT-001226 was compared to OT-001232. NIH 3T3 cells were incubated with varying doses of Sildenafil or Vardenafil for 48 hours and MFIs were analyzed by FACS. The stabilization ratio was calculated as the fold change in GFP intensity in ligand treated samples compared to the GFP intensity in the absence of ligand with the same construct. The results are shown in Table 37.

**Table 37: hPDE5 C-terminal mutant's response to Sildenafil and Vardenafil**

| **Ligand concentration (nM)** | **OT-001226** | | | | **OT-001232** | | | |
|---|---|---|---|---|---|---|---|---|
| | Sildenafil | | Vardenafil | | Sildenafil | | Vardenafil | |
| | MFI | Stabilization Ratio | MFI | Stabilization Ratio | MFI | Stabilization Ratio | MFI | Stabilization Ratio |
| 0 | 102 | - | | - | 1476 | - | 1410 | - |
| 100 | 278 | 2.73 | 971 | 9.34 | 1639 | 1.11 | 1614 | 1.14 |
| 300 | 562 | 5.51 | 1165 | 11.20 | 1648 | 1.12 | 1656 | 1.03 |
| 1000 | 1039 | 10.19 | 1235 | 11.88 | 1780 | 1.21 | 1686 | 1.02 |
| 3000 | 1276 | 12.51 | 1299 | 12.49 | 1784 | 1.21 | 1721 | 1.02 |
| 10000 | 1442 | 14.14 | 1309 | 12.59 | 1888 | 1.28 | 1721 | 1.00 |

As shown in Table 37, OT-001226 construct showed stabilization ratios greater than one, indicating that both Vardenafil and Tadalafil ligand could stabilize GFP expression. As expected, the wildtype construct, OT-001232 did not stabilization much greater than one, indicating that virtually no stabilization was achieved with the wildtype construct.

GFP expression was also measured at 24 and 48 hours to monitor the dependence of hPDE5-DD GFP constructs on the duration of ligand exposure. The results are shown in Table 38 and Table 39.

**Table 38: Dose response of hPDE5 C-terminal mutants with Sildenafil treatment**

| **Sildenafil concentration (nM)** | **OT-001226** | | | | **OT-001232** | | | |
|---|---|---|---|---|---|---|---|---|
| | 24 hours | | 48 hours | | 24 hours | | 48 hours | |
| | MFI | Stabilization Ratio | MFI | Stabilization Ratio | MFI | Stabilization Ratio | MFI | Stabilization Ratio |
| 0 | 108 | | 102 | - | 1385 | - | 1476 | - |
| 100 | 243 | 2.25 | 278 | 2.73 | 1495 | 1.08 | 1639 | 1.11 |
| 300 | 380 | 3.52 | 562 | 5.51 | 1565 | 1.13 | 1648 | 1.12 |
| 1000 | 575 | 5.32 | 1039 | 10.19 | 1669 | 1.21 | 1780 | 1.21 |
| 3000 | 666 | 6.17 | 1276 | 12.51 | 1831 | 1.32 | 1784 | 1.21 |
| 10000 | - | - | 1442 | 14.14 | - | - | 1888 | 1.28 |

**Table 39: Dose response of hPDE5 C-terminal mutants with Vardenafil treatment**

| **Vardenafil concentration (nM)** | **OT-001226** | | | | **OT-001232** | | | |
|---|---|---|---|---|---|---|---|---|
| | 24 hours | | 48 hours | | 24 hours | | 48 hours | |
| | MFI | Stabilization Ratio | MFI | Stabilization Ratio | MFI | Stabilization Ratio | MFI | Stabilization Ratio |
| 0 | 108 | | 104 | - | 1360 | - | 1410 | - |
| 100 | 684 | 6.33 | 971 | 9.34 | 1439 | 1.06 | 1614 | 1.14 |
| 300 | 761 | 7.05 | 1165 | 11.20 | 1730 | 1.27 | 1656 | 1.17 |
| 1000 | 863 | 7.99 | 1235 | 11.88 | 1721 | 1.27 | 1666 | 1.18 |
| 3000 | 856 | 7.93 | 1299 | 12.49 | 1606 | 1.18 | 1721 | 1.22 |
| 10000 | 889 | 8.23 | 1309 | 12.59 | 1678 | 1.23 | 1721 | 1.22 |

Consistent with the responses measured at 48 hours, the responses measured at 24 hours showed that the hPDE5 mutant construct stabilized GFP expression as evidenced by the stabilization ratios greater than one, while the wildtype construct, OT-001232 showed stabilization ratios around 1, indicating no ligand dependent stabilization. Results were consistent between Sildenafil and Vardenafil treatment. It was noted that the stabilization ratios obtained at 48 hours was much greater than the values obtained at 24 hours suggesting, a time dependent stabilization of GFP expression. Similar results were obtained by western blot analysis using a GFP antibody. At 0.1µM sildenafil, modest stabilization of GFP protein levels was observed via western blot. The stabilization of GFP increased when the cells were treated with 1µM sildenafil Both 0.1µM and 1µM dose of vardenafil showed stabilization of GFP levels. These trends were observed both at 24 hours and 48 hours. All comparisons were made against cells that were not treated with either ligand.

The response of hPDE5C terminal mutants to lower doses of Vardenafil was also tested and is shown in Table 40.

**Table 40: Low dose response of hPDE5 C-terminal mutants with Vardenafil treatment**

| **Vardenafil (nM)** | **MFI** | **Stabilization ratio** |
|---|---|---|
| 0 | 137 | - |
| 1 | 163 | 1.19 |
| 3 | 230 | 1.68 |
| 10 | 477 | 3.48 |
| 30 | 909 | 6.64 |
| 100 | 1417 | 10.34 |
| 1000 | 1789 | 13.06 |

As shown in Table 40, the hPDE5C terminal mutant showed a dose dependent stabilization even at lower doses of Vardenafil as indicated by the increase in the stabilization ratio. Thus, even low nano molar doses of Vardenafil are sufficient to induce ligand dependent stabilization of hPDE5 DD.

### Example 10. Behavior of hPDE5 luciferase constructs

PDE5 derived DDs were appended to reporter payload such as luciferase to generate constructs OT-001227, OT-001228, OT-001229, and OT-001231 constructs. Such constructs may be useful to study in the dynamics of destabilization and stabilization of DDs *in vivo.* These constructs were transduced into HCT116 cells. Cells were seeded into 96 well plates at 2000 cells per well and incubated with 1 µM or DMSO Vardenafil for 48 hours. The luciferase activity was then measured using a plate reader assay. Parental untransduced cells were used as a control to measure the background fluorescence levels. The results are shown in Table 41. In Table 41, the stabilization ratio was calculated as the fold change in luciferase signal in Vardenafil treated samples compared to treatment with DMSO (i.e. in the absence of ligand) with the same construct. The destabilizing mutation coefficient was calculated as the fold change in GFP intensity in the hPDE5 mutant constructs compared to the hPDE5 wildtype construct (OT-001227) in the absence of the ligand. Destabilizing mutation coefficients less than 1 are desirable. Stabilization ratios greater than 1 are desired in DDs.

**Table 41: Stability of Luciferase constructs**

| **Construct** | **Control (DMSO)** | **Vardenafil** | **Destabilizing mutation co-efficient** | **Stabilization ratio** |
|---|---|---|---|---|
| Parental cells | 1367.47 | 3824.04 | - | - |
| OT-001227 | 85429.26 | 127499.78 | - | 1.49 |
| OT-001228 | 88881.46 | 174814.64 | 1.04 | 1.97 |
| OT-001229 | 53239.88 | 88918.04 | 0.62 | 1.67 |
| OT-001231 | 96460.45 | 269718.66 | 1.13 | 2.80 |

The luciferase signal intensity observed in parental cells that did not express the construct indicated that some of the signal was likely background noise. Construct, OT-001229 showed the lowest destabilizing mutation coefficient indicating the construct is destabilized. Increase in stabilization ratios was observed with all constructs including the wildtype hPDE5 construct, OT-001227. Construct OT-001231 showed the highest stabilization ratio indicating the strongest ligand dependent stabilization. Among the constructs tested, OT-001229 showed destabilization in the absence of ligand and stabilization in the presence of ligand.

### Example 11. Mutagenesis of the amino acid 732 of hPDE5

The analysis of mutants identified by site directed mutagenesis identified amino acid hotspots whose mutation confers destabilization and ligand dependent stabilization properties to hPDE5. The amino acid at position 732 was chosen for the further analysis since the hPDE5 (R732L) mutation possessed the properties described above. To improve the DD characteristics of this DD, the amino acid at the position 732 was mutated to any of the known amino acids, including, but not limited to, aspartic acid, glutamic acid, glutamine, asparagine, histidine, serine, threonine, tyrosine, cysteine, methionine, tryptophan, alanine, isoleucine, leucine, phenylalanine, valine, proline, and glycine. The library of mutations was generated by site directed mutagenesis and each of the mutants in the library was fused to a reporter protein e.g. AcGFP via a linker and transduced into HCT116 cells. The properties of the DDs were analyzed in the presence and absence of ligands via FACS as previously described. Ligands that were evaluated included, but not limited Sildenafil, and Vardenafil. The results are shown in Table 42 and Table 43.

**Table 42: Ligand responsive behavior of hotspot constructs**

| **Construct** | **Mutation** | **Vardenafil** | | **Sildenafil** | |
|---|---|---|---|---|---|
| | | **0 µM (DMSO)** | **1 µM** | **0 µM (DMSO)** | **10 µM** |
| OT-001075 | - | 161 | 294 | 989 | 219 |
| OT-001084 | R732L | 182 | 173 | 143 | 122 |
| OT-001186 | R732G | 176 | 854 | 298 | 288 |
| OT-001187 | R732A | 175 | 853 | 288 | 348 |
| OT-001188 | R732V | 150 | 507 | 189 | 113 |
| OT-001189 | R732I | 151 | 587 | 201 | 170 |
| OT-001190 | R732P | 355 | 310 | 379 | 361 |
| OT-001191 | R732F | 189 | 943 | 233 | 351 |
| OT-001192 | R732W | 301 | 179 | 248 | 158 |
| OT-001193 | R732Y | 302 | 345 | 397 | 648 |
| OT-001195 | R732H | 308 | 328 | 352 | 710 |
| OT-001196 | R732S | 566 | 399 | 760 | 746 |
| OT-001197 | R732T | 235 | 270 | 276 | 546 |
| OT-001198 | R732D | 294 | 422 | 298 | 394 |
| OT-001199 | R732E | 276 | 212 | 295 | 316 |
| OT-001200 | R732Q | 305 | 289 | 353 | 553 |
| OT-001201 | R732N | 313 | 290 | 315 | 516 |
| OT-001202 | R732M | 316 | 337 | 285 | 503 |
| OT-001203 | R732C | 526 | 349 | 499 | 707 |
| OT-001204 | R732K | 113 | 423 | 165 | 765 |

**Table 43: Stabilization ratio and Destabilizing mutation coefficient of hotspot constructs**

| **Construct** | **Mutation** | **Vardenafil** | | **Sildenafil** | |
|---|---|---|---|---|---|
| | | **Destabilizing mutation coefficient** | **Stabilization ratio** | **Destabilizing mutation coefficient** | **Stabilization ratio** |
| OT-001075 | - | - | 1.83 | - | 0.22 |
| OT-001084 | R732L | 1.13 | 0.95 | 0.14 | 0.85 |
| OT-001231 | R732G | 1.09 | 4.85 | 0.3 | 0.97 |
| OT-001187 | R732A | 1.09 | 4.87 | 0.29 | 1.21 |
| OT-001188 | R732V | 0.93 | 3.38 | 0.19 | 0.6 |
| OT-001189 | R732I | 0.94 | 3.89 | 0.2 | 0.85 |
| OT-001190 | R732P | 2.2 | 0.87 | 0.38 | 0.95 |
| OT-001191 | R732F | 1.17 | 4.99 | 0.24 | 1.51 |
| OT-001192 | R732W | 1.87 | 0.59 | 0.25 | 0.64 |
| OT-001193 | R732Y | 1.88 | 1.14 | 0.4 | 1.63 |
| OT-001195 | R732H | 1.91 | 1.06 | 0.36 | 2.02 |
| OT-001196 | R732S | 3.52 | 0.7 | 0.77 | 0.98 |
| OT-001197 | R732T | 1.46 | 1.15 | 0.28 | 1.98 |
| OT-001198 | R732D | 1.83 | 1.44 | 0.3 | 1.32 |
| OT-001199 | R732E | 1.71 | 0.77 | 0.3 | 1.07 |
| OT-001200 | R732Q | 1.89 | 0.95 | 0.36 | 1.57 |
| OT-001201 | R732N | 1.94 | 0.93 | 0.32 | 1.64 |
| OT-001202 | R732M | 1.96 | 1.07 | 0.29 | 1.76 |
| OT-001203 | R732C | 3.27 | 0.66 | 0.5 | 1.42 |
| OT-001204 | R732K | 0.7 | 3.74 | 0.17 | 4.64 |

The destabilizing mutation coefficients and stabilization ratios obtained for each construct with the indicated ligands were analyzed to identify constructs which possessed the lowest destabilizing mutation coefficient ratio and a second sort of the data was then performed to identify constructs with the highest stabilization ratio. This analysis allowed the identification of constructs with both the lowest destabilizing mutation coefficient that also possessed the highest stabilization ratios. Based on this analysis, it was identified that virtually all mutants tested showed destabilizing mutation coefficients less than one for the DMSO control of Sildenafil treatment, indicating that all constructs are destabilized in the absence of sildenafil. In the presence of Sildenafil, OT-001204 construct with arginine to lysine substitution showed high sildenafil dependent stabilization ratio. Similar analysis of the constructs with Vardenafil identified OT-001204, OT-001188, OT-001189, and OT-001187 as having a low destabilizing mutation coefficient and high-ligand dependent stabilization ratios. Other constructs with low destabilizing mutation coefficients and high stabilization ratios include OT-001189, OT-001187, OT-001191, OT-001197, and OT-001198.

The response of select mutants to increasing doses of Sildenafil, Vardenafil and Tadalafil was also tested in HCT 116 cells treated with the ligand for 48 hours. The MFI values obtained with each ligand treatment are shown in Table 44, Table 45 and Table 46.

**Table 44: Vardenafil Dose Response**

| **Vardenafil (µM)** | **OT-001187** | **OT-001189** | **OT-001191** | **OT-001197** | **OT-001198** |
|---|---|---|---|---|---|
| 0 | 2551 | 1810 | 2188 | 2854 | 4336 |
| 0.01 | 2551 | 1810 | 2188 | 2854 | 4336 |
| 0.04 | 8345 | 3356 | 8468 | 6862 | 7350 |
| 0.12 | 10946 | 4874 | 10941 | 13187 | 15637 |
| 0.37 | 13850 | 7855 | 12987 | 28895 | 25047 |
| 1.11 | 15202 | 11777 | 13754 | 33075 | 37828 |
| 3.33 | 16783 | 14531 | 14705 | 41788 | 44499 |
| 10 | 18945 | 17645 | 17021 | 48946 | 50822 |
| 30 | 22548 | 24322 | 20693 | 67364 | 70645 |
| 1000 | 22548 | 24322 | 20693 | 67364 | 70645 |

**Table 45: Sildenafil Dose Response**

| **Sildenafil (µM)** | **OT-001187** | **OT-001189** | **OT-001191** | **OT-001197** | **OT-001198** |
|---|---|---|---|---|---|
| 0.01 | 808 | 579 | 688 | 856 | 1192 |
| 0.04 | 1025 | 612 | 916 | 1041 | 1270 |
| 0.12 | 1270 | 592 | 1205 | 1113 | 1337 |
| 0.37 | 1931 | 771 | 2002 | 1835 | 1867 |
| 1.11 | 2655 | 1040 | 2711 | 3355 | 3108 |
| 3.33 | 3769 | 1709 | 3555 | 6896 | 6171 |
| 10 | 4716 | 2733 | 4427 | 12407 | 13971 |
| 30 | 5694 | 4197 | 5449 | 21258 | 21120 |
| 1000 | 5694 | 4197 | 5449 | 21258 | 21120 |

**Table 46: Tadalafil Dose Response**

| **Tadalafil (µM)** | **OT-001187** | **OT-001189** | **OT-001191** | **OT-001197** | **OT-001198** |
|---|---|---|---|---|---|
| 0.01 | 752 | 498 | 631 | 825 | 1163 |
| 0.14 | 1259 | 540 | 1193 | 1068 | 1361 |
| 0.41 | 2066 | 712 | 2075 | 1655 | 1548 |
| 1.23 | 3412 | 1041 | 3134 | 3433 | 2504 |
| 3.7 | 4690 | 1745 | 4294 | 6697 | 4821 |
| 11.11 | 5126 | 2746 | 4734 | 12843 | 10583 |
| 33.33 | 6264 | 3970 | 5293 | 17164 | 16956 |
| 100 | 6301 | 5487 | 5867 | 21113 | 22630 |
| 1000 | 6301 | 5487 | 5867 | 21113 | 22630 |

OT-001197 with an arginine to threonine substitution showed the strongest increase in GFP expression at the highest dose, with all three ligands tested. At doses lower than 1 µM of Vardenafil, all constructs showed ligand dependent stabilization. At doses of Sildenafil lower than 1 µM, OT-001191, OT-001197 and OT-001198 showed ligand dependent stabilization. With doses of Tadalafil lower than 1 µM, OT-001187, OT-001191 and OT-001197 showed ligand dependent stabilization. By mutagenesis of the R732 locus, DDs with a variety of properties were obtained. The selection of a particular DD may be made based on the payload utilized and the extent of expression desired with the payload. The EC50 values for each of the constructs with each of the ligands is shown in Table 47.

**Table 47: EC₅₀ for Hotspot Mutants**

| **Ligand** | **OT-001187** | **OT-001189** | **OT-001191** | **OT-001197** | **OT-001198** |
|---|---|---|---|---|---|
| Vardenafil | 0.30 | 2.20 | 0.28 | 1.47 | 1.44 |
| Sildenafil | 2.08 | 6.90 | 1.76 | 7.50 | 7.04 |
| Tadalafil | 1.45 | 14.28 | 1.33 | 8.64 | 14.54 |

These results are consistent with the western blot analysis of GFP protein levels in HCT 116 cells stably transduced with these mutants. The following constructs showed a strong stabilization of GFP levels in the presence of vardenafil and when compared to DMSO controls: OT-001084, OT-001231, OT-001187, OT-001190, OT-001192, OT-001193, OT-001195, OT-001196, OT-001197, OT-001198, OT-001199, OT-001200, OT-001201, OT-001202, and OT-001203. Modest ligand dependent stabilization of GFP levels was observed with OT-001188, OT-001189, OT-001191, and OT-001204. All samples showed equal protein loading as measured by GAPDH protein levels.

### Example 12: Dynamic range of hPDE5 regulation

HCT116 cells were transduced with hPDE5-GFP constructs, OT-001205, OT-001206, OT-001207, and OT-001253 and incubated with the indicated concentrations of ligand for 48 hours. GFP fluorescence was measured by FACS. The fold change in GFP expression over untreated DMSO control is shown in Table 48, Table 49 and Table 50 for vardenafil, sildenafil and tadalafil respectively.

**Table 48: Vardenafil dose response**

| **Vardenafil (log)** | **OT-001205** | **OT-001206** | **OT-001207** | **OT-001253** |
|---|---|---|---|---|
| 1.477 | 20.664 | 13.612 | 4.507 | 16.171 |
| 1.000 | 18.207 | 12.030 | 2.178 | 13.462 |
| 0.523 | 18.327 | 10.260 | 1.277 | 13.230 |
| 0.046 | 17.229 | 7.465 | 1.078 | 12.550 |
| -0.431 | 16.143 | 4.374 | 1.022 | 11.992 |
| -0.908 | 13.764 | 2.258 | 1.025 | 9.770 |
| -1.386 | 10.108 | 1.323 | 1.015 | 5.477 |
| -1.863 | 4.673 | 0.834 | 1.030 | 2.303 |
| -2.340 | 2.575 | 0.741 | 1.034 | 1.412 |
| -2.817 | 1.744 | 0.725 | 1.047 | 1.105 |

**Table 49: Sildenafil dose response**

| **Sildenafil (log)** | **OT-001205** | **OT-001206** | **OT-001207** | **OT-001253** |
|---|---|---|---|---|
| 2.000 | 11.370 | 14.212 | 1.909 | 11.290 |
| 1.523 | 12.925 | 15.247 | 1.509 | 13.091 |
| 1.046 | 12.066 | 10.028 | 1.212 | 12.344 |
| 0.569 | 10.587 | 5.408 | 1.091 | 10.615 |
| 0.092 | 9.190 | 2.822 | 0.995 | 8.165 |
| -0.386 | 6.849 | 1.640 | 1.040 | 5.536 |
| -0.863 | 4.225 | 1.249 | 0.993 | 2.540 |
| -1.340 | 2.295 | 1.106 | 1.007 | 1.397 |
| -1.817 | 1.434 | 1.025 | 1.013 | 1.134 |
| -2.294 | 1.136 | 1.024 | 0.966 | 0.997 |

**Table 50: Tadalafil dose response**

| **Tadalafil (log)** | **OT-001205** | **OT-001206** | **OT-001207** | **OT-001253** |
|---|---|---|---|---|
| 2.000 | 12.074 | 14.919 | 1.498 | 12.606 |
| 1.523 | 11.481 | 13.740 | 1.224 | 11.910 |
| 1.046 | 9.897 | 8.083 | 1.011 | 11.144 |
| 0.569 | 8.403 | 4.360 | 0.945 | 9.902 |
| 0.092 | 5.445 | 2.312 | 0.973 | 7.992 |
| -0.386 | 2.725 | 1.472 | 0.907 | 5.049 |
| -0.863 | 1.563 | 1.225 | 0.946 | 2.887 |
| -1.340 | 1.121 | 1.034 | 0.938 | 1.402 |
| -1.817 | 1.095 | 1.013 | 0.976 | 1.077 |
| -2.294 | 1.045 | 0.985 | 0.942 | 1.064 |

As shown in Table 48, Table 49 and Table 50, dynamic regulation of hPDE5 DDs was observed with OT-001205, OT-001206, and OT-001207. The hPDE5 wildtype construct shown little to no ligand dependent stabilization with all three ligands. The stabilization concentration 50 or (SC₅₀), which is the concentration of ligand required to achieve 50% stabilization, was also calculated for all four constructs with each of the ligands and the results are shown Table 51.

**Table 51: Stabilization Concentration 50 (SC₅₀) of hPDE5 DDs**

| **Ligand** | **OT-001205** | **OT-001206** | **OT-001207** | **OT-001253** |
|---|---|---|---|---|
| Vardenafil | 0.04 | 1.15 | >30 | 0.07 |
| Sildenafil | 0.38 | 6.82 | >30 | 0.67 |
| Tadalafil | 1.97 | 9.54 | >100 | 0.77 |

Taken together these results indicate that structure-guided mutagenesis of ligand-binding site in PDE5 generates 23 nM ligand-DD interaction. OT-001205 with Y612F, R732L, was able to bind to Vardenafil with SC₅₀, of 23 nM suggesting potent DD-ligand interaction.

### Example 13. DD regulated CD19 CAR expression and function

Immunotherapeutic payloads of the disclosure such as CD19 CAR were fused to any of the DDs described herein and cloned into expression vectors pLVX and pELNS vectors. In this manner, OT-001300 (hPDE5 (WT)) and OT-001301 (hPDE5(R732L)). The HA tag was added to enable the easy detection of the chimeric proteins.

To test ligand dependent CD19 CAR production, NIH 3T3 cells were transiently transfected with the constructs. Cells were then plated in a growth media e.g. DMEM with 10% FBS and incubated overnight at 37°C, 5% CO2. The growth media was exchanged for media containing ligand, at various concentrations of Vardenafil ranging from 30 nM to 10,000 nM. Following 48-hour incubation with ligand, cells were analyzed by FACS using the HA antibody and the mean fluorescence intensity (MFI) was calculated. The results are shown in Table 52, where the stabilization ratio was defined as the ratio of expression, function or level of a protein of interest, i.e. CD19 CAR in response to the stimulus, i.e. Vardenafil; to the expression, of the CD19 CAR in the absence of the stimulus, i.e. DMSO control. Stabilization ratios greater than one are desired.

**Table 52: CD19 CAR expression**

| **Vardenafil (nM)** | **OT-001300 (WT)** | **OT-001301 (R732L)** | |
|---|---|---|---|
| | **MFI** | **MFI** | **Stabilization ratio** |
| 0 | 0 | 1336 | - |
| 30 | 9125 | 3066 | 2.29 |
| 100 | 9592 | 4674 | 3.50 |
| 300 | 10509 | 5732 | 4.29 |
| 1000 | 10328 | 6738 | 5.04 |
| 10000 | 9285 | 3931 | 2.94 |

As shown in Table 52, a stabilization ration greater than 1 was observed with all doses of Vardenafil, and even with the lowest concentration of Vardenafil (30nM) suggesting that low doses of ligand may be sufficient to achieve ligand dependent stabilization. It was also noted that the stabilization ratio obtained at the highest concentration of ligand i.e. 10,000 nM was lower than the ratio obtained with 1000nM ligand suggesting a bimodal pattern of stabilization.

The dose dependent regulation of OT-001300 and OT-001301 was measured in HEK 293 cells transfected with 2ug DNA and stably selected using 2ug/ml puromycin. CAR surface expression was detected using Protein L staining following 24 hours of ligand treatment at the indicated concentrations. The median Protein L fluorescence is shown in Table 53.

**Table 53: Ligand dose dependent CAR expression**

| **Ligand (nM)** | **Tadalafil** | | | **Sildenafil** | | | **Vardenafil** | | |
|---|---|---|---|---|---|---|---|---|---|
| | **OT-001300** | **OT-001301** | **Empty Vector (pLVX)** | **OT-001300** | **OT-001301** | **Empty Vector (pLVX)** | **OT-001300** | **OT-001301** | **Empty Vector (pLVX)** |
| 0.15 | 21122 | 4064 | 77 | 14703 | 2848 | 89.8 | 17946 | 4202 | 87.9 |
| 0.46 | 15335 | 3501 | 75.8 | 15335 | 3304 | 87 | 17746 | 4064 | 92 |
| 1.39 | 14662 | 3501 | 78.8 | 13143 | 3250 | 83.9 | 16175 | 4604 | 89.8 |
| 4.22 | 14377 | 3341 | 77 | 13901 | 2810 | 85.7 | 16450 | 5977 | 93.9 |
| 12.79 | 10773 | 3599 | 78.8 | 9419 | 2976 | 81.8 | 17646 | 9210 | 92 |
| 38.74 | 14098 | 4042 | 80 | 12083 | 3268 | 84.8 | 16175 | 13329 | 94.8 |
| 117.41 | 18354 | 6302 | 77 | 15037 | 3589 | 83 | 19856 | 20594 | 93.9 |
| 355.78 | 13785 | 11235 | 72.9 | 13901 | 5274 | 83.9 | 22406 | 23175 | 95.8 |
| 1078.11 | 18200 | 15640 | 80 | 13708 | 8054 | 88.8 | 22343 | 31138 | 97.7 |
| 3267.00 | 15950 | 21301 | 75.8 | 14337 | 13291 | 84.8 | 24933 | 31226 | 100 |
| 9900.00 | 21421 | 24654 | 75.8 | 17746 | 23371 | 84.8 | 26824 | 36249 | 110 |
| 30000.00 | 27902 | 33691 | 77 | 25003 | 25215 | 84.8 | 30617 | 35945 | 140 |

As shown in Table 54, Table 55 and Table 56, only OT-001301 responded to increasing doses of ligand with increase surface CAR expression, but not the WT construct OT-001300. This was observed with all three ligands. However, the highest levels of CAR expression were observed with increasing doses of vardenafil. The EC50 for vardenafil, tadalafil and sildenafil were determined to 25 nM, 380 nM, and 1500 nM respectively.

Cells were also treated with ligand for varying durations of time with ligand concentrations shown in Table 54 and Protein L expression was measured using FACS. The results are shown in Table 55 and Table 56. Stabilization ratios are shown in Table 58.

**Table 54: Time dependent increase in CAR expression with Vardenafil**

| **Hours** | **Vardenafil 10 µM** | | | **Vardenafil 1 uM** | **DMSO** | | |
|---|---|---|---|---|---|---|---|
| | **Empty Vector (pLVX)** | **OT-001300** | **OT-001301** | **OT-001301** | **OT-001300** | **OT-001301** | **Empty Vector (pLVX)** |
| 0 | 1412 | 5188 | 1486 | 65.9 | 5060 | 1386 | 71.9 |
| 2 | 2863 | 11643 | 2918 | 78.7 | 5545 | 1270 | 71 |
| 4 | 3654 | 11258 | 3526 | 76.8 | 5408 | 1274 | 71.9 |
| 24 | 6624 | 10439 | 8005 | 83.7 | 5845 | 1241 | 71 |
| 48 | 7764 | 8118 | 8489 | 93.8 | 6128 | 1287 | 71 |
| 72 | 9282 | 7961 | 9788 | 92.8 | 6442 | 1329 | 65.9 |

**Table 55: Time dependent increase in CAR expression with Tadalafil and Sildenafil**

| **Hours** | **Tadalafil 10 µM** | | | **Tadalafil 1 µM** | **Sildenafil 10 µM** | | | **Sildenafil 1 µM** |
|---|---|---|---|---|---|---|---|---|
| | **Empty Vector (pLVX)** | **OT-001300** | **OT-001301** | **OT-001301** | **Empty Vector (pLVX)** | **OT-001300** | **OT-001301** | **OT-001301** |
| 0 | 65 | 4909 | 1336 | 1368 | 62.8 | 5469 | 1534 | 1372 |
| 2 | 65 | 5911 | 2778 | 2451 | 65 | 8536 | 2801 | 2278 |
| 4 | 65 | 6196 | 3032 | 2950 | 63.7 | 8560 | 3256 | 2617 |
| 24 | 65 | 8608 | 6606 | 4346 | 62.8 | 8118 | 5499 | 3159 |
| 48 | 62.8 | 8827 | 7384 | 4683 | 62.8 | 7404 | 5927 | 3283 |
| 72 | 58.7 | 8118 | 8050 | 6027 | 58.7 | 6111 | 5188 | 3506 |

**Table 56: Time dependent increase in CAR expression**

| **Time (hrs)** | **Tadalafil 10 µM** | | **Tadalafil 1 µM** | **Sildenafil 10 µM** | | **Sildenafil 1 µM** | **Vardenafil 10 µM** | **Vardenafil 1 µM** | |
|---|---|---|---|---|---|---|---|---|---|
| | **OT-001300** | **OT-001301** | **OT-001301** | **OT-001300** | **OT-001301** | **OT-001301** | **OT-001300** | **OT-001301** | **OT-001301** |
| 0 | 0.97 | 0.96 | 0.99 | 1.08 | 1.11 | 0.99 | 1.03 | 1.07 | 0.05 |
| 2 | 1.07 | 2.19 | 1.93 | 1.54 | 2.21 | 1.79 | 2.10 | 2.30 | 0.06 |
| 4 | 1.15 | 2.38 | 2.32 | 1.58 | 2.56 | 2.05 | 2.08 | 2.77 | 0.06 |
| 24 | 1.47 | 5.32 | 3.50 | 1.39 | 4.43 | 2.55 | 1.79 | 6.45 | 0.07 |
| 48 | 1.44 | 5.74 | 3.64 | 1.21 | 4.61 | 2.55 | 1.32 | 6.60 | 0.07 |
| 72 | 1.26 | 6.06 | 4.53 | 0.95 | 3.90 | 2.64 | 1.24 | 7.36 | 0.07 |

The analysis of the stabilization ratios indicated that OT-001301 is stabilized by all three ligands with increased duration of incubation time with ligand. As expected, 10 µM dose of ligand showed much greater expression of CAR than the 1 µM dose. Regulation was evident even at the lower doses.

Constructs OT-001455, OT-001456 and OT-001457 were cloned into pELNS vectors and transduced into T cells. Three different volumes of lentiviral supernatant were tested on cells i.e. 1 µl, 10 µl and 20 µl. Following transduction, cells were treated with 10 µM Vardenafil or left untreated for 24 hours beginning on day 4. Untransduced cells were also included as negative control. On day 5, the percentage CAR positive cells were measured by FACS using 1 µg /ml CD19 Fc. The results are shown in Figure 7A.

As shown in Figure 7A, all three constructs showed a viral dose dependent increase in the percentage of CAR positive cells, in the presence of Vardenafil. Although similar trends were observed in untreated controls, the percentage of CAR positive cells observed with no vardenafil treatment was substantially lesser than the vardenafil treated controls. Among the three constructs, OT-001457 showed lowest basal expression of the CAR in the absence of ligand and the highest percentage of CAR positive cells in the presence of vardenafil. Similar observations were made when comparing the percentage CAR positive cells obtained with different constructs using 20 µl of virus as shown in Figure 7B, where a shift in the number of cells that are CAR positive was observed in vardenafil treated cells (labelled treatment) compared to untransduced as well as untreated cells.

T cells maintained in culture for over 10 days reach quiescence and accordingly T cells transduced with OT-001456 did not show vardenafil dependent CAR expression at day 11. However, restimulation of the same T cells on day 11 with CD3/CD28 beads (at 3:1 bead: cell ratio, for 24 hours concomitant with ligand treatment) restored vardenafil dependent CAR expression in T cells transduced with 20 µl of virus (Figure 7C). The effect of restimulation on the expression of the CAR was evident at all concentrations of the virus tested Figure 7D. Taken together, these data show that CD19 CARs operably linked to hPDE5 DDs demonstrate ligand dependent CAR expression. Further, ligand responsiveness and subsequent CAR expression may be restored in quiescent T cells by restimulation with CD3/CD28 beads.

To test the effect on different ligands on hPDE5-CAR constructs, the constructs were packaged into plasmids and transduced into T cells using 10 µl of virus. On day 9, T cells expressing either OT-001454, OT-001455, OT-001456 or OT-001457 were treated with varying doses of ligand ranging from 0.1 nM to 10 µM for 24 hours. The constitutive construct OT-001407 was included as the positive control, and the cells transduced with the empty vector (pELNS) served as the negative control. Three different ligands for hPDE5 were tested including, Sildenafil, Tadalafil and Vardenafil. Cells were analyzed by FACS and sorted for singlets/live CAR positive cells. The results are shown in Figure 7E. Among the three ligands tested, all constructs were most responsive to Vardenafil, followed by Tadalafil and Sildenafil. As seen previously, the OT-001456 and OT-001457 showed lower basal expression, and had the higher EC50 values than the other constructs. The experiments were repeated with the OT-001456 construct by transducing T cells with different volumes of virus transduced into T cells using 10µL, 2µL, 0.4µL, or 0.08µL of virus and the CAR expression was measured by FACS, both in the presence of all three ligands and in the absence of the ligands. The results are shown in Table 57, where "basal % CAR+" indicates the percentage of CAR positive cells in the absence of ligand, and the "Max % CAR+," indicates the maximum expression of the CAR in the presence of ligand. The EC50 is defined as the concentration of a drug/ligand that gives half-maximal response. The response herein refers to the expression of the chimeric antigen receptor.

**Table 57. hPDE5 regulation of CD19-CAR**

| **Construct** | **Virus Volume (uL)** | **Basal % CAR**+ | **Max % CAR+** | **Vardenafil EC50 (nM)** | **Tadalafil EC50 (nM)** | **Sildenafil EC50 (nM)** |
|---|---|---|---|---|---|---|
| Empty vector (pELNS) | 1 | 0.2 | 0.2 | - | - | - |
| OT-001407 | 1 | 30.0 | 30.0 | - | - | - |
| OT-001456 | 10 | 3.6 | 37.5 | 140.0 | 1312.2 | >10000.0 |
| OT-001456 | 2 | 2.0 | 29 | 157.0 | 1402.8 | >10000.0 |
| OT-001456 | 0.4 | 0.9 | 16.1 | 197.7 | 2511.9 | >10000.0 |
| OT-001456 | 0.08 | 0.4 | 5.0 | 220.8 | 1857.8 | >10000.0 |

Among the three ligands tested, higher transduction volume increased the maximum CAR expression level achieved, but did not alter ligand EC50 values.

To test how rapidly hPDE5-CAR can be stabilized with ligand, the indicated constructs were tested in T cells. T cells from donor were thawed and activated overnight in the presence of CD3/CD28 Dynabeads at a 3:1 bead: cell ratio. The following day, cells were transduced with 10µL lentivirus prepared from OT-001407, OT-001454, OT-001455, OT-001456, and OT-001457 constructs. Cells were expanded by addition of fresh media over the course of 10 days, maintaining cells around 0.5x10^6 cells/mL, then frozen. Expanded T cells were thawed and restimulated with CD3/CD28 beads for 48 hours. Cells were treated with 10µM Tadalafil or Vardenafil at various times such that all conditions received 48 hr of bead stimulation with either 0, 2, 4, 6, 24, or 48 hours of ligand treatment. CAR surface expression was measured with 1µg/mL CD19-Fc. The results are shown in Figure 7F. In activated T cells, CAR surface expression occurred at nearly maximum levels by 2 hours after ligand addition.

To test whether CD19-CARs showing regulated expression also show regulated cytotoxicity, transduced T cells were tested in a cytotoxicity assay against the CD19-expressing Nalm6 tumor cell line, T cells from a human donor were thawed and activated overnight in the presence of CD3/CD28 Dynabeads at a 3:1 bead: cell ratio. The following day, cells were transduced with 10µL lentivirus from OT-001407, OT-001454, OT-001455, OT-00 1456, and OT-00 1457. Cells were expanded by addition of fresh media over the course of 10 days, maintaining cells around 0.5×10^6 cells/mL, then frozen. Expanded T cells were thawed and cocultured with CD19 expressing target cells, Nalm6-Katushka, at effector: target cell ratio of 10:1 for 6 days, in the absence or presence of Tadalafil. Images captured on the Incucyte Zoom were analyzed for proliferation of target cells by measuring red fluorescence over time (Total NucRed^{®} Area). In the absence of ligand, i.e. DMSO, OT-001456 and OT-001457 showed target cell proliferation as measured by the increase in total NucRed area (red fluorescence). Constructs, OT-001455, OT-001454 showed very low levels of red fluorescence indicating basal activity that was comparable to the constitutively expressed CAR construct OT-001407. All constructs tested showed a reduction in red fluorescence when treated with either 3µM Tadalafil or 10µM Tadalafil that was comparable to the red fluorescence levels observed with the constitutively expressed CAR. As expected, the untransduced cells showed high levels of red fluorescence both in the presence and absence of ligand.

Constructs showing higher basal CAR expression in T cells, OT-001454 and OT-001455, inhibited target cell growth in the absence of tadalafil. T cells transduced with OT-001457, which had the highest Tadalafil EC50 in terms of CAR expression, required higher concentrations of ligand to generate an *in vitro* functional response.

To measure cytokine levels in T cells, supernatants from the previous coculture assay were harvested at 48 hours and analyzed for IFNγ and IL2 levels by MSD assay (Meso Scale Discovery's ELISA). The results are shown in Figure 7G. The cytokine levels at the following effector to target cell rations: 10:1, 3:1,1:1, 0.3:1, and 0.1:1. High levels of both IFNγ and IL2 are produced by OT-001456 transduced T cells when co-cultured with Nalm6 targets only in the presence of 3µM Tadalafil, correlating with the cytotoxicity observed.

To confirm the cytotoxicity and cytokine secretion is directly related to CAR expression, OT-001456 transduced T cells were cocultured with Nalm6-Katushka target cells at 10:1 effector: target cell ratio for 6 days in the presence of a dose titration of Tadalafil from 10 µM to 10 nM. The fluorescence of Nalm6 cells was measured over a period of 6 days in response to the following doses of tadalafil: 0.01µM, 0.04µM, 0.12 µM, 0.37µM, 1.11µM, 3.33 µM, 10 µM. Tadalafil doses at 0.37µM, 1.11µM, 3.33 µM, 10 µM showed a reduction in Nalm6 proliferation over 6 days. Lower concentrations (<0.37µM) tadalafil did not cause a reduction in Nalm6 fluorescence, in fact, Nalm6 cells continue to proliferate over the span of 6 days. Significant CAR expression was detected by FACS using CD19-Fc at Tadalafil concentrations at or above 300nM. This CAR expression corresponded to Tadalafil dose-dependent cytotoxicity (Figure 7H) and cytokine secretion of IFNγ and IL2 (Figure 7I).

The functionality of the CAR transduced T cells was also tested *in vitro.* T cells were transduced with OT-001456 or the constitutive construct OT-001407. Cells were then frozen. Prior to the experiment, T cells were thawed overnight, and cocultured with Nalm6 target cells stably expressing Katushka RFP. Nalm6 cells express high levels of CD19 antigen and are hence ideal target cells in cell killing assay that test CD19 CAR constructs. T cells were mixed with Nalm6 cells at an effector cell to target cell (E: T) ratio of 5:1 for 12 hours in the presence of 10 µM vardenafil or DMSO. Target cell apoptosis was determined by measuring Annexin V fluorescence over time using the Incucyte instrument. Cell killing is measured as the ratio of the total killed target area and to the NALM6-Katushka area (µM²). As shown in Figure 7J, the total killed target cell area increased when Nalm6 cells were co-cultured with OT-00 1456 expressing T cells in the presence of vardenafil. Similar trend was observed in the proliferation of Nalm6 cells (Figure 7K). The same cells did not result in significant cell killing in presence of DMSO, showing that the target cell killing by the T cells is specifically in response to the presence of the ligand. As expected, the constitutive construct, OT-001407 showed increased total killed target cell area both in the presence and absence of ligand, with a trend towards more killing in the presence of the ligand. Nalm6 cells co-cultured with untransduced T cells and Nalm6 cells that were not co-cultured with T cells did not show an increase in the total killed target area. Taken together, these data show that the regulated CAR construct, OT-001456 is active on antigen positive cells only in response to ligand.

The cell supernatants were also collected from the T cell/Nalm6 cytotoxicity co-culture assays at 66 hours and Interferon gamma levels were measured by MSD assays. The results are shown in Table 58.

**Table 58: Interferon gamma production (pg/mL)**

| **Construct** | **DMSO** | **Vardenafil 10 µM** |
|---|---|---|
| OT-001456 | 443.99 | 2282.98 |
| OT-001407 | 3268.72 | 1980.48 |
| Untransduced | 16.57 | 18.48 |

As shown in Table 58, the interferon gamma levels showed a five-fold induction in OT-001456 transduced T cells with the addition of vardenafil. Vardenafil treatment did not induce interferon gamma production in OT-001407 transduced T cells or untransduced T cells, indicating that the ligand specific induction of interferon gamma is related to the increased CAR expression and cell killing observed under similar conditions. The interferon gamma levels in DMSO treated OT-001456 was lower than the levels observed with the constitutive construct indicating that the OT-001456 cells have low basal expression of the CD19 CAR.

### Example 14. In vivo assessment of hPDE5-regulated CD19 CAR

To test the efficacy of ligand regulated CAR efficacy in a tumor rejection model, 5 million CAR positive T cells (25 million total T cells) were injected in Nalm6 tumor bearing female NSG mice. The Nalm6-Luc is a B-cell precursor leukemia cell line used to generate an intravenous disseminated tumor model for studying blood tumors. Mice were treated daily with vehicle or Tadalafil at the following mg/kg body weight or mpk: 10mpk, 30mpk, or 100mpk. Constitutive OT-001407 transduced T cells served as a positive control and untransduced T cells as a negative control. T cells were injected 7 days after the Nalm6 cells were implanted. Animals in the Tadalafil groups received 2 doses of ligand prior to T cells being injected. The objective of the study was to test the ligand dose response regulation of a hPDE5 DD CD19 CAR-T and its impact on efficacy. Further, the study was also used to address if the presence of antigen was necessary for detection and expression of the CAR. Dose groups included (a) untransduced T cells (b) untransduced vehicle treated cells (c) Un-transduced cells dosed with Tadalafil 100mg/kg (d) 5.0 Million CD19+ OT-001407 CAR T cells (e) 5.0 Million CD19 positive Nalm6 cells with OT-001407 CAR T cells (Vehicle) (f) 5.0 Million CD19 positive Nalm6 cells with OT-001407 CAR T cells (Tadalafil 100mg) (g) 5.0 Million CD19 positive Nalm6 cells with OT-001456 CAR T cells (Vehicle) (h) 5.0 Million CD19 positive Nalm6 cells with OT-001456(Tadalafil 10mg/kg) (i) 5.0 Million CD19 positive Nalm6 cells with OT-001456 CAR T cells (Tadalafil 30mg/kg (j) 5.0 Million CD19 positive Nalm6 cells with OT-001456 CAR T cells (Tadalafil 100mg/kg). All animals were dosed once a day, orally for 25 days. Tumor burden assessments were made biweekly using bio luminescent imaging and the fluorescence intensity of Nalm6 Luc cells was observed over time. Two terminal collections were made to assess bone marrow, blood and spleen cell populations. Reduction in tumor burden/ prevalence was measured by decreased luminescence from Nalm6-Luciferase cells, and was observed in Tadalafil treated OT-001456 animals (Figure 8A). In Figure 8A, the construct marked "const" is OT-001407 and the construct marked "regulated" is OT-001456. Taken together these data show that daily oral dosing of tadalafil resulted in dose-dependent suppression of tumor growth and/or reduction in tumor burden. Maximal tumor suppression was comparable to constitutive CAR with very low activity in the absence of drug. The percentage of tumor growth inhibition was >98% across all ligand doses tested for recipients of PDE5-DD-regulated CD19-CART (OT-001456) compared to unmodified T cell recipients.

The mortality of the mice was tracked during the course of the study. Kaplan-Meier analysis, and the log rank test were applied to the survival data. A significant survival advantage was observed in mice treated with OT-001456 transduced T cells relative to the mice treated with unmodified T cells (p< 0.005) with a 50% increase in median survival. The median survival of the mice is provided in Table 59. In Table 59 * indicates undetermined as the mice in this group survived the duration of the study; TDF indicates Tadalafil; VDF denotes vardenafil and "Veh" denotes vehicle. Survival ratio was calculated as ratio of the median survival observed in a group to the median survival of the untransduced- vehicle treatment group.

**Table 59: Median survival (days)**

| Construct | Untransduced | | | OT-001407 | | OT-001456 | | | |
|---|---|---|---|---|---|---|---|---|---|
| Ligand | | Veh | TDF 100mpk | Veh | TDF 100mpk | Veh | TDF 10mpk | TDF 30mpk | TDF 100mpk |
| No. of deaths/ events | 8 | 8 | 8 | 0 | 0 | 8 | 8 | 7 | 8 |
| Median survival | 23 | 26 | 26 | * | * | 29 | 34 | 39 | 37 |
| Survival ratio | - | 1 | 1.13 | - | - | 1.15 | 1.31 | 1.5 | 1.42 |

The median survival was increased in the OT-001456 group treated with either 30mpk or 100 mpk TDF as compared to the untransduced or OT-001407 transduced mice in the presence/absence of ligand. The enhanced survival in the OT-001456 TDF treated groups, was also evident in the observed increase in the survival ratio.

Plasma PK was also established in non-tumor-bearing mice of the hPDE5 inhibitors Tadalafil and Vardenafil after a single dose at the indicated dose levels and is shown in Figure 8B and Figure 8C. To determine pharmacokinetic profiles of PDE5 inhibitors, CD-1 mice were dosed orally once with Vardenafil, while NSG mice were dose with Tadalafil. Plasma samples collected over time and PDE5 inhibitor concentrations were determined by LC/MS/MS (Table 60). The Vardenafil/Tadalafil levels detected in the plasma declined over time and were commensurate with the initial dose orally administered to the animal.

**Table 60: Vardenafil/Tadalafil Plasma levels (µM)**

| **Hours post dose** | **Tadalafil 10mg/kg** | **Tadalafil 30mg/kg** | **Tadalafil 100mg/kg** | **Vardenafil 10mg/kg** | **Vardenafil 100mg/kg** |
|---|---|---|---|---|---|
| 1 | 6.8 | 19.8 | 53.3 | 0.16 | 7.81 |
| 2 | 5.4 | 17.6 | 44.7 | 0.03 | 3.04 |
| 4 | 3.9 | 12.1 | 34.8 | 0.02 | 3.32 |
| 8 | 0.9 | 4.4 | 13.7 | - | 1.18 |
| 12 | 0.1 | 0.5 | 5.6 | - | 0.04 |
| 16 | 0.0 | 0.1 | 1.5 | - | - |
| 24 | - | 0.0 | 0.1 | - | - |

### Example 15. Destabilization Mutant Screening of human PDE5

To identify destabilizing mutations of human PDE5 that are stabilized upon ligand binding to hPDE5, two rounds of mutant screening were performed, using a lentivirus hPDE5 mutant library (PDE5A-MUT-AcGFP_pLVX-IRES-puro) provided by Genscript (China). These methods were used to generate a library of mutant polypeptides using mutagenesis primers and error prone PCR are described herein.

### General Methods

Lentiviral vectors expressing the template, wild-type human PDE5 operably linked to GFP (OT-001075) and hPDE5 (R732L) (OT-001084) were also provided by Genscript along with the hPDE5 mutant library. These constructs were packaged into lentivirus, transduced into NIH3T3 cells and select stable cell pools were derived as described below. Cell pools were selected and treated with sildenafil or vardenafil and subjected to FACS and fluorescence microscopy analyses. Wild-type hPDE5 showed approximately a 1.5- fold increase in mean fluorescence intensity (MFI) after treatment with sildenafil or vardenafil, whereas OT-001084 showed 10 to 20- fold increase in MFI upon treatment with either compound. The hPDE5 mutant library yielded approximately 2-fold increase in MFI with Vardenafil treatment. Vardenafil proved more effective in stabilizing the hPDE5 mutant library and hPDE5 (R732L) mutant than sildenafil and was therefore chosen as the test compound for single-ligand experiments.

For lentiviral production, 293T cells were plated in a 6-well dish (approximately 1×10⁶ cells per well) and allowed to reach around 90% confluence (~1 day). For transfection, growth medium was removed from each well and replaced with 1.5 mL fresh cell culture medium. A lipofectamine dilution was prepared (13.5 µL lipofectamine 3000 in 125 µL Opti-MEM) and mixed thoroughly with a DNA solution (containing 2 µg lentivector, 2 µg Delta 8.9, 0.5 µg VSV-G in 125 µL OptiMEM and 9 µL P3000) and incubated for 10-15 min at room temperature. Lipid-DNA complex was added drop-wise directly to cells and incubated overnight at 37°C, 5% CO₂, the medium was then replaced with 2 mL fresh complete culture medium. Forty-eight hours after transfection, the supernatant (virus) was harvested, filtered and stored in 1 mL aliquots at -80°C.

Lentiviral stocks were used for transduction of NIH3T3 cells and subsequent cell pool selection. The day before transduction, NIH3T3 cells (1.8 × 10⁶) were seeded in a T-25 flask and allowed to reach 60-70% confluency (~1 day). The growth medium in the T-25 flask was then replaced with 1 mL virus (quickly thawed in a 37°C water bath) and 5 µg/mL polybrene. After an 1hr of incubation, with gentle rocking every 15min, 3 mL fresh medium (with 5 µg/mL polybrene) was added to the flask and cells incubated overnight. Transduction medium was removed the following day and replaced with 5 mL fresh medium. At 48hr after transduction, the medium was replaced with media containing 1 µg/mL puromycin. This puromycin medium was replaced every 3-4 days until cell pools were established (~2 weeks).

To identify cell pools containing destabilizing mutants of hPDE5 that are stabilizing when hPDE5 is bound to ligand vardenafil (Selleck S2515) cells were sorted using a BD FACSJazz^{™} cell sorter. Cells were treated with either 1 µM or 10 µM vardenafil for 24 hours then harvested for cell sorting (1-2×10⁷ cells needed for sorting). To enrich cell pools, 3 (low-high-low) or 5 (low-high-low-high-low) rounds of cell sorting were completed as follows, i) 5% of cell population of low AcGFP in absence of ligand (ii) 5% of cell population of high Ac-GFP in presence of ligand (iii) 5% of cell population of low AcGFP in absence of ligand (iv) 5% of cell population of high Ac-GFP in presence of ligand. (v) 5% of cell population of low AcGFP in absence of ligand.

In the first experiment, after final sorting (3^{rd} or 5^{th} round), genomic DNA was isolated from cell pools using the Promega Wizard Genomic DNA Purification kit. An approximately 1kb fragment of hPDE5 was PCR amplified using primers hPDE5A-EI-For (5'ACTAGAGGATCTATTTCCGGTGAATTCATG) (8719) and hPDE5A-BI-Rev (5'GGATCCTCCACCACTGCCTCCA) (SEQ ID No. 8720) and high fidelity Prime STAR HS DNA Polymerase (Takara, Cat R010B), the product was gel-purified, cloned into pEASY-Blunt simple cloning vector (Transgen Cat CB111) and transformed into DH5α. Clones were picked and sent for sequencing of hPDE5. Sequencing results are summarized in Tables 61, Table 62 and Table 63.

**Table 61: Mutants from 3^{rd} sorted pool (low-high-low)**

| **Library clone ID** | **hPDE5 mutation** |
|---|---|
| LibC000009 | hPDE5 (Amino acid 535-860 of WT, L573M, F735L) |
| LibC000010 | hPDE5 (Amino acid 535-860 of WT, R658H) |
| LibC000011 | hPDE5 (Amino acid 535-860 of WT, S562G, L727P, R847T) |
| LibC000012 | hPDE5 (Amino acid 535-860 of WT, Q666H) |
| LibC000013 | hPDE5 (Amino acid 535-860 of WT, L781P) |
| LibC000014 | hPDE5 (Amino acid 535-860 of WT, A767E) |
| LibC000015 | hPDE5 (Amino acid 535-860 of WT, T571S, V585M, T723S) |
| LibC000016 | hPDE5 (Amino acid 535-860 of WT, Q743L) |
| LibC000017 | hPDE5 (Amino acid 535-860 of WT, Y709H, F787V) |
| LibC000018 | hPDE5 (Amino acid 535-860 of WT, N605Y, I715K) |
| LibC000019 | hPDE5 (Amino acid 535-860 of WT, F564I, N662Y, H685L, L693P, F736I) |
| LibC000020 | hPDE5 (Amino acid 535-860 of WT, A722V, F755L, M760I) |
| LibC000021 | hPDE5 (Amino acid 535-860 of WT, V594I) |
| LibC000022 | hPDE5 (Amino acid 535-860 of WT, H592R) |
| LibC000023 | hPDE5 (Amino acid 535-860 of WT, E858V) |
| LibC000024 | hPDE5 (Amino acid 535-860 of WT, T784I) |

**Table 62: Mutants from 5^{th} sorted pool (low-high-low-high-low)**

| **Library clone ID** | **hPDE5 mutation** |
|---|---|
| LibC000001 | hPDE5 (Amino acid 535-860 of WT, S542L, E708V, W772C) |
| LibC000002 | hPDE5 (Amino acid 535-860 of WT, I700F, E796G) |
| LibC000003 | hPDE5 (Amino acid 535-860 of WT, D724G, K848N) |
| LibC000004 | hPDE5 (Amino acid 535-860 of WT, T712M, M758I, Q859R) |
| LibC000005 | hPDE5 (Amino acid 535-860 of WT, I556S, E796D) |
| LibC000006 | hPDE5 (Amino acid 535-860 of WT, I680S) |
| LibC000007 | hPDE5 (Amino acid 535-860 of WT, H613L, D724Y, F755Y) |
| LibC000008 | hPDE5 (Amino acid 535-860 of WT, A611T) |

Select clones from the fifth sort were cloned into expression vectors and operably linked to AcGFP to generate the constructs listed in 63.

**Table 63: Mutant constructs from 5^{th} sorted pool**

| **OT-ID** | **Mutation** |
|---|---|
| OT-001358 | hPDE5 (Amino acid 535- 860 of WT; N661S) |
| OT-001359 | hPDE5 (Amino acid 535-830 of WT, L569M, T833S) |
| OT-001360 | hPDE5 (Amino acid 535-860 of WT, I768N) |
| OT-001361 | hPDE5 (Amino acid 535-860 of WT, R607W) |
| OT-001362 | hPDE5 (Amino acid 535-860 of WT, N620S) |
| OT-001363 | hPDE5 (Amino acid 535-860 of WT, L554R, Q589E, M691I) |
| OT-001364 | hPDE5 (Amino acid 535-860 of WT, W615R, T723R, A762T, E808G) |
| OT-001365 | hPDE5 (Amino acid 535-860 of WT, C574Y) |

Twenty-five full length hPDE5 mutant clones were also ligated in-frame with AcGFP (*Aequorea coerulescens* green fluorescent protein) into a pLVX-EF1α vector and are indicated in Table 64 below by "OT" ID. Some of the clones tested are also identified by "Lib" IDs in Table 64, these clones have the same construct build as the clones identified by "OT" ID; however, the entire insert was not sequenced. Virus was produced for each of the hPDE5 mutants and transduced into NIH3T3 cells as described above, with puromycin based selection of stable cell pools, which were then tested by FACS GFP analysis following treatment with 10 µM vardenafil for 24hr. Destabilizing mutants that provide stability upon binding of hPDE5 and vardenafil were expected to show greater GFP fluorescence by FACS analysis. Results are summarized in Table 64 below. The stabilization ratio was calculated as the fold change in GFP intensity in ligand (vardenafil) treated samples compared to treatment with DMSO (i.e. in the absence of ligand) with the same construct. The destabilizing mutation coefficient was calculated as the fold change in GFP intensity in the PDE5 mutant constructs compared to the PDE5 wildtype construct in the absence of the ligand. Destabilizing mutation coefficients less than 1 are desirable. Stabilization ratios greater than 1 are desired in DDs.

**Table 64: GFP FACS analysis (MFI) of vardenafil-treated mutant cell pools**

| **Library ID/ Construct ID.** | **MFI** | | **Stabilization Ratio** | **Destabilizing mutation co-efficient** |
|---|---|---|---|---|
| | **DMSO** | **10µM VDF** | | |
| LibC000009 | 510 | 4485 | 8.8 | 0.09 |
| LibC000010 | 6713 | 13940 | 2.1 | 1.17 |
| LibC000011 | 57 | 67 | 1.2 | 0.01 |
| LibC000013 | 498 | 723 | 1.5 | 0.09 |
| LibC000014 | 2500 | 6998 | 2.8 | 0.44 |
| LibC000015 | 174 | 1951 | 11.2 | 0.03 |
| LibC000016 | 2895 | 6869 | 2.4 | 0.51 |
| LibC000017 | 2244 | 6530 | 2.9 | 0.39 |
| LibC000018 | 1038 | 3102 | 3.0 | 0.18 |
| LibC000019 | 320 | 285 | 0.9 | 0.06 |
| LibC000020 | 332 | 1459 | 4.4 | 0.06 |
| LibC000021 | 1732 | 4440 | 2.6 | 0.30 |
| LibC000023 | 4386 | 6121 | 1.4 | 0.77 |
| LibC000024 | 5039 | 6905 | 1.4 | 0.88 |
| LibC000002 | 309 | 981 | 3.2 | 0.05 |
| LibC000003 | 656 | 1870 | 2.9 | 0.11 |
| LibC000005 | 271 | 2081 | 7.7 | 0.05 |
| LibC000007 | 234 | 234 | 1.0 | 0.04 |
| OT-001358 | 5726 | 7157 | 1.2 | 1.00 |
| OT-001359 | 5065 | 7544 | 1.5 | 0.88 |
| OT-001360 | 908 | 1735 | 1.9 | 0.16 |
| OT-001362 | 1283 | 2303 | 1.8 | 0.22 |
| OT-001363 | 260 | 1170 | 4.5 | 0.05 |
| OT-001364 | 121 | 141 | 1.2 | 0.02 |
| OT-001084 | 273 | 7463 | 27.3 | 0.05 |
| OT-001084 | 163 | 4127 | 25.3 | 0.03 |
| OT-001084 | 320 | 6531 | 20.4 | 0.06 |
| OT-001075 | 2003 | 3298 | 1.6 | - |
| OT-001075 | 8695 | 13100 | 1.5 | - |
| OT-001075 | 6480 | 9132 | 1.4 | - |
| NIH3T3 control | 62 | - | - | - |
| NIH3T3 control | 55 | - | - | - |
| NIH3T3 control | 72 | - | - | - |

Six clones. mostly with stabilization ratio > 4 and destabilizing mutation co-efficient <0.01 were chosen for further analysis with all three ligands for FACS titration analysis. An 8- concentration, 3- fold dilution titration scheme starting at 100 µM and three ligands: vardenafil, sildenafil (Selleck, S1431) and tadalafil (Selleck, S 1512) were used for the analysis. Mean fluorescence intensity (MFI) data for treatment with each of three ligands are shown in Table 65 and Table 66 below. VDF = vardenafil, SDF = sildenafil, TDF = tadalafil.

**Table 65: GFP FACS analysis (MFI) of ligand-treated clones**

| **Cone. (µM)** | **LibC000013** | | | **LibC000015** | | | **LibC000020** | | | **LibC000005** | | |
|---|---|---|---|---|---|---|---|---|---|---|---|---|
| | **VDF** | **SDF** | **TDF** | **VDF** | **SDF** | **TDF** | **VDF** | **SDF** | **TDF** | **VDF** | **SDF** | **TDF** |
| 100 | 7207 | 8260 | 7614 | 3069 | 630 | 518 | 2499 | 513 | 509 | 4240 | 1136 | 814 |
| 33.333 | 7308 | 7404 | 8353 | 3166 | 432 | 463 | 2144 | 407 | 440 | 3892 | 734 | 711 |
| 11.111 | 7393 | 5539 | 8085 | 2315 | 357 | 374 | 1395 | 373 | 371 | 3066 | 577 | 551 |
| 3.704 | 7330 | 3467 | 6275 | 1383 | 331 | 326 | 733 | 376 | 342 | 1968 | 518 | 453 |
| 1.235 | 6773 | 2076 | 3688 | 756 | 320 | 311 | 399 | 371 | 337 | 1044 | 479 | 418 |
| 0.412 | 5735 | 1459 | 2083 | 492 | 313 | 301 | 290 | 378 | 326 | 603 | 444 | 385 |
| 0.137 | 4220 | 1268 | 1456 | 385 | 313 | 309 | 266 | 375 | 313 | 449 | 447 | 389 |
| 0.046 | 2634 | 1243 | 1222 | 336 | 316 | 295 | 263 | 367 | 335 | 389 | 464 | 410 |
| DMSO | 854 | 943 | 847 | 373 | 301 | 306 | 238 | 338 | 243 | 376 | 335 | 366 |
| NIH3T3 | 58 | 40 | 42 | - | - | - | 58 | 40 | 42 | 58 | 40 | 42 |

**Table 66: GFP FACS analysis (MFI) of ligand -treated pools**

| **Cone. (µM)** | **OT-001363** | | | **OT-001084** | | | **OT-001075** | | |
|---|---|---|---|---|---|---|---|---|---|
| | **VDF** | **SDF** | **TDF** | **VDF** | **SDF** | **TDF** | **VDF** | **SDF** | **TDF** |
| 100 | 1397 | 1356 | 1132 | 8608 | 3103 | 3428 | 12000 | 7082 | 10100 |
| 33.333 | 1563 | 994 | 1077 | 5866 | 2654 | 2832 | 8854 | 7025 | 7688 |
| 11.111 | 1566 | 725 | 816 | 5213 | 1962 | 2493 | 7964 | 6848 | 7388 |
| 3.704 | 1390 | 579 | 552 | 4746 | 1187 | 1941 | 7592 | 6480 | 7039 |
| 1.235 | 1047 | 456 | 405 | 3938 | 598 | 1107 | 7393 | 5740 | 6211 |
| 0.412 | 666 | 418 | 328 | 2662 | 276 | 534 | 7223 | 4806 | 5491 |
| 0.137 | 463 | 404 | 321 | 2047 | 204 | 222 | 7059 | 4245 | 4745 |
| 0.046 | 387 | 431 | 341 | 1104 | 139 | 139 | 6655 | 4647 | 4350 |
| DMSO | 327 | 336 | 284 | 357 | 93.4 | 316 | 7545 | 3140 | 9511 |
| NIH3T3 | 58 | 40 | 42 | 58 | 40 | 42 | 58 | 40 | 42 |

The mean fluorescence intensity (MFI) of GFP at 100 µM concentration treatment was set as 100% hPDE5 stabilization effect (HPE), while DMSO treatment was set as 0% effect (ZPE) of hPDE5 stabilization. The effect of the compound on hPDE5 stabilization was calculated based on the following formula: % PDE5 stabilization effect = (MFI-ZPE)/(HPE-ZPE) x100. Calculated %hPDE5 stabilization effects for mutant cell pools are shown in Table 67 and Table 68 below. VDF = vardenafil, SDF = sildenafil, TDF = tadalafil.

**Table 67: Percent stabilization effect for ligand-treated mutant cell pools**

| **Cone. (µM)** | **LibC000009** | | | **LibC000015** | | | **LibC000020** | | | **LibC000005** | | |
|---|---|---|---|---|---|---|---|---|---|---|---|---|
| | **VDF** | **SDF** | **TDF** | **VDF** | **SDF** | **TDF** | **VDF** | **SDF** | **TDF** | **VDF** | **SDF** | **TDF** |
| 100 | 100 | 100.0 | 100.0 | 100.0 | 100.0 | 100.0 | 100.0 | 100.0 | 100.0 | 100.0 | 100.0 | 100.0 |
| 33.333 | 101.6 | 88.3 | 110.9 | 103.6 | 39.8 | 74.1 | 84.3 | 39.4 | 74.1 | 91.0 | 49.8 | 77.0 |
| 11.111 | 102.9 | 62.8 | 107.0 | 72.0 | 17.0 | 32.1 | 51.2 | 20.0 | 48.1 | 69.6 | 30.2 | 41.3 |
| 3.704 | 101.9 | 34.5 | 80.2 | 37.5 | 9.1 | 9.4 | 21.9 | 21.7 | 37.2 | 41.2 | 22.8 | 19.4 |
| 1.235 | 93.2 | 15.5 | 42.0 | 14.2 | 5.8 | 2.4 | 7.1 | 18.9 | 35.3 | 17.3 | 18.0 | 11.6 |
| 0.412 | 76.8 | 7.1 | 18.3 | 4.4 | 3.6 | -2.4 | 2.3 | 22.9 | 31.2 | 5.9 | 13.6 | 4.2 |
| 0.137 | 53.0 | 4.4 | 9.0 | 0.4 | 3.6 | 1.4 | 1.2 | 21.1 | 26.3 | 1.9 | 14.0 | 5.1 |
| 0.046 | 28.0 | 4.1 | 5.5 | -1.4 | 4.6 | -5.2 | 1.1 | 16.6 | 34.6 | 0.3 | 16.1 | 9.8 |
| DMSO | 0.0 | 0.0 | 0.0 | 0.0 | 0.0 | 0.0 | 0.0 | 0.0 | 0.0 | 0.0 | 0.0 | 0.0 |

**Table 68: Percent stabilization effect for ligand-treated mutant cell pools**

| **Conc. (µM)** | **OT-001363** | | | **OT-001084** | | | **OT-001075** | | |
|---|---|---|---|---|---|---|---|---|---|
| | **VDF** | **SDF** | **TDF** | **VDF** | **SDF** | **TDF** | **VDF** | **SDF** | **TDF** |
| **100** | 100.0 | 100.0 | 100.0 | 100.0 | 100.0 | 100.0 | 100.0 | 100.0 | 100.0 |
| **33.333** | 115.5 | 64.5 | 93.5 | 66.8 | 85.1 | 80.8 | 29.4 | 98.6 | -309.5 |
| **11.111** | 115.8 | 38.1 | 62.7 | 58.9 | 62.1 | 70.0 | 9.4 | 94.1 | -360.4 |
| **3.704** | 99.3 | 23.8 | 31.6 | 53.2 | 36.3 | 52.2 | 1.1 | 84.7 | -419.7 |
| **1.235** | 67.3 | 11.8 | 14.3 | 43.4 | 16.8 | 25.4 | -3.4 | 66.0 | -560.3 |
| **0.412** | 31.7 | 8.0 | 5.2 | 27.9 | 6.1 | 7.0 | -7.2 | 42.3 | -682.5 |
| **0.137** | 12.7 | 6.7 | 4.4 | 20.5 | 3.7 | -3.0 | -10.9 | 28.0 | -809.2 |
| **0.046** | 5.6 | 9.3 | 6.7 | 9.1 | 1.5 | -5.7 | -20.0 | 38.2 | -876.2 |
| **DMSO** | 0.0 | 0.0 | 0.0 | 0.0 | 0.0 | 0.0 | 0.0 | 0.0 | 0.0 |

Plots of the % effect versus concentration were generated with Graphpad Prism 5 and EC₅₀ values were calculated using a four-parameter nonlinear regression model. EC₅₀ data are shown in Table 69. EC₅₀ values were not calculated for HPE/ZPE < 3.

**Table 69: EC₅₀ values**

| **Construct** | **EC₅₀ VDF** | **EC₅₀ SDF** | **EC₅₀ TDF** |
|---|---|---|---|
| LibC000009 (hPDE5 (Amino acid 535-860 of WT, L573M, F735L)) | 0.2 | 7.9 | 1.8 |
| LibC000015 (hPDE5 (Amino acid 535-860 of WT, T571S, V585M, T723S)) | 5.3 | - | - |
| LibC000020 (hPDE5 (Amino acid 535-860 of WT, A722V, F755L, M760I)) | 12.0 | - | - |
| LibC000005 (hPDE5 (Amino acid 535-860 of WT, I556S, E796D)) | 5.7 | 37.3 | - |
| OT-001363 | 0.9 | 18.9 | 8.3 |
| OT-001084 | 0.7 | 6.5 | 2.8 |

Based on this screening of a human PDE5 library (Experiment 1), several clones were identified with destabilizing mutations, which upon ligand binding to PDE5 serve to stabilize the protein. LibC000009, and OT-001363 were identified as the top destabilizing mutants from this experiment, as they demonstrated HPE/ZPE values of >3 for all three ligands.

In a second experiment, cell pools were enriched by 5 rounds of cell sorting as described above. Genomic DNA was isolated, the product gel-purified, cloned into pEASY-Blunt simple cloning vector and transformed into DH5α. Clones were picked and sent for sequencing of hPDE5. Sequencing results are summarized in Table 70 below.

**Table 70: Mutants from 5^{th} sorted pool**

| **Construct ID** | **Mutations** |
|---|---|
| OT-001366 | hPDE5 (535-860 of WT, L554R, Q586P, K710N, K730E |
| OT-001367 | hPDE5 (535-860 of WT, H613R |
| OT-001368 | hPDE5 (535-860 of WT, Q586L, S663Y, A762T, E808G) |
| OT-001369 | hPDE5 (535-860 of WT, V548M, D803N) |
| OT-001370 | hPDE5 (535-860 OF WT, L554R, Q589E, A823T) |
| OT-001371 | hPDE5 (535-860 of WT, T571I, K604R, I706N, E795R) |
| OT-001372 | hPDE5 (535-860 of WT, G659A, T784I |
| OT-001373 | hPDE5 (535-860 of WT, Q666H) |
| OT-001374 | hPDE5 (535-860 of WT, H617L, A722V) |
| OT-001375 | hPDE5 (535-860 of WT, N609Y, I799L) |
| OT-001376 | hPDE5 (535-860 of WT, F561L, G659A, T784I) |
| OT-001377 | hPDE5 (535-860 of WT, K795N) |
| OT-001378 | hPDE5 (535-860 of WT, K770Q, K848N) |
| OT-001379 | hPDE5 (535-860 of WT, N605D) |
| OT-001380 | hPDE5 (535-860 of WT, I680S) |
| OT-001381 | hPDE5 (535-860 of WT, V548M, F820I) |
| OT-001382 | hPDE5 (535-860 of WT, I799L) |
| OT-001383 | hPDE5 (535-860 of WT, R607Q) |
| OT-001384 | hPDE5 (535-860 of WT, S550F, L554R) |

Twenty hPDE5 mutants were selected and sub cloned in-frame with AcGFP into a pLVX-EF1α vector. Lentiviruses expressing these hPDE5 mutants were produced and used to generate cell pools, which were then treated with 10 µM vardenafil for 24hr and tested by FACS for GFP expression. Data were analyzed by ACEA Novocyte NovoExpress 1.2.4 software. Results are summarized in Table 71. The stabilization ratio was calculated as the fold change in GFP intensity in ligand (vardenafil) treated samples compared to treatment with DMSO (i.e. in the absence of ligand) with the same construct. The destabilizing mutation coefficient was calculated as the fold change in GFP intensity in the PDE5 mutant constructs compared to the PDE5 wildtype construct in the absence of the ligand. Destabilizing mutation coefficients less than 1 are desirable. Stabilization ratios greater than 1 are desired in DDs.

**Table 71: GFP FACS analysis (MFI) of vardenafil-treated clones**

| **Clone No.** | **Mean fluorescence intensity (MFI)** | | **Stabilization Ratio** | **Destabilizing mutation co-efficient** |
|---|---|---|---|---|
| | **DMSO** | **10µM VDF** | | |
| OT-001366 | 1620 | 5945 | 3.7 | 0.05 |
| OT-001367 | 9396 | 20753 | 2.2 | 0.32 |
| OT-001368 | 2168 | 10148 | 4.7 | 0.07 |
| OT-001369 | 6864 | 14992 | 2.2 | 0.23 |
| OT-001370 | 2717 | 16617 | 6.1 | 0.09 |
| OT-001371 | 12380 | 33121 | 2.7 | 0.42 |
| OT-001372 | 36071 | 46063 | 1.3 | 1.21 |
| OT-001373 | 32358 | 40657 | 1.3 | 1.09 |
| OT-001374 | 1208 | 1234 | 1.0 | 0.04 |
| OT-001375 | 10926 | 34449 | 3.2 | 0.37 |
| OT-001376 | 3574 | 7332 | 2.1 | 0.12 |
| OT-001377 | 34578 | 51182 | 1.5 | 1.16 |
| OT-001378 | 2050 | 31968 | 15.6 | 0.07 |
| OT-001379 | 6293 | 39197 | 6.2 | 0.21 |
| OT-001380 | 64493 | 92590 | 1.4 | 2.17 |
| OT-001381 | 1400 | 1480 | 1.1 | 0.05 |
| OT-001382 | 37015 | 47513 | 1.3 | 1.25 |
| OT-001383 | 11848 | 39923 | 3.4 | 0.40 |
| OT-001384 | 3009 | 8836 | 2.9 | 0.10 |
| OT-001385 | 975 | 2684 | 2.8 | 0.03 |
| OT-001084 | 1320 | 36657 | 27.8 | 0.04 |
| OT-001084 | 1535 | 35653 | 23.2 | 0.05 |
| OT-001075 | 30404 | 45616 | 1.5 | |
| OT-001075 | 29006 | 43134 | 1.5 | |
| NIH3T3 | 334 | - | - | |

Five clones with stabilization ratios > 4 and destabilizing mutation co-efficient <0.25 were chosen for further FACS titration analysis with the three PDE5 ligands as described previously. Data were analyzed by ACEA Novocyte NovoExpress 1.2.4 and mean fluorescence intensity (MFI) data are shown in Table 72 and Table 73 below. VDF = vardenafil, SDF = sildenafil, TDF = tadalafil.

**Table 72: GFP FACS analysis (MFI) of ligand-treated clones**

| **Cone. (µM)** | **OT-001366** | | | **OT-001368** | | | **OT-001370** | | | **OT-001378** | | |
|---|---|---|---|---|---|---|---|---|---|---|---|---|
| | **VDF** | **SDF** | **TDF** | **VDF** | **SDF** | **TDF** | **VDF** | **SDF** | **TDF** | **VDF** | **SDF** | **TDF** |
| **100** | 10075 | 3958 | 5348 | 16225 | 6454 | 8089 | 22761 | 17471 | 25264 | 47881 | 29835 | 56820 |
| **33.333** | 9430 | 3370 | 4053 | 14792 | 4724 | 6435 | 24683 | 12196 | 21573 | 45279 | 16797 | 39880 |
| **11.111** | 7787 | 3033 | 3280 | 12476 | 3827 | 4822 | 23517 | 7761 | 14127 | 42089 | 8273 | 19260 |
| **3.704** | 5293 | 2852 | 2925 | 9212 | 3384 | 4204 | 20879 | 5502 | 8870 | 34071 | 4837 | 9255 |
| **1.235** | 3566 | 2776 | 2756 | 6223 | 3226 | 3995 | 16399 | 4517 | 6338 | 22789 | 3482 | 5353 |
| **0.412** | 2657 | 2753 | 2961 | 4285 | 3290 | 4029 | 11704 | 3959 | 5497 | 13317 | 3079 | 4077 |
| **0.137** | 2256 | 2777 | 2924 | 3198 | 3183 | 3874 | 7655 | 3559 | 5387 | 6981 | 2874 | 3777 |
| **0.046** | 2044 | 2812 | 2803 | 2679 | 3237 | 3936 | 5515 | 3758 | 5492 | 4477 | 2800 | 3841 |
| **DMSO** | 2228 | 2781 | 2826 | 2657 | 3322 | 3773 | 3543 | 4171 | 5207 | 2625 | 3087 | 4029 |
| **NIH3T3** | 401 | 397 | 462 | 401 | 397 | 462 | 401 | 397 | 462 | 401 | 397 | 462 |

**Table 73: GFP FACS analysis (MFI) of ligand treated clones**

| **Conc. (µM)** | **OT-001379** | | | **OT-001075** | | | **OT-001075** | | |
|---|---|---|---|---|---|---|---|---|---|
| | **VDF** | **SDF** | **TDF** | **VDF** | **SDF** | **TDF** | **VDF** | **SDF** | **TDF** |
| 100 | 50937 | 49449 | 77286 | 66821 | 39638 | 88657 | 68646 | 65696 | 124011 |
| 33.333 | 47373 | 41359 | 73260 | 44931 | 31873 | 97813 | 53578 | 64475 | 136420 |
| 11.111 | 47019 | 29331 | 55450 | 39279 | 20750 | 89646 | 50082 | 62109 | 143351 |
| 3.704 | 44984 | 18084 | 34005 | 36347 | 11248 | 58392 | 49859 | 57186 | 142331 |
| 1.235 | 39930 | 11933 | 18589 | 32753 | 4647 | 28605 | 47543 | 47748 | 121306 |
| 0.412 | 32217 | 9781 | 12030 | 25177 | 2887 | 12329 | 45747 | 37806 | 102964 |
| 0.137 | 21125 | 9355 | 9940 | 15273 | 2161 | 6399 | 45044 | 34518 | 85774 |
| 0.046 | 13855 | 9188 | 10204 | 7877 | 1983 | 5246 | 45287 | 33102 | 73045 |
| DMSO | 7173 | 9629 | 11082 | 3378 | 2114 | 4958 | 29006 | 35174 | 70819 |
| NIH3T3 | 401 | 397 | 462 | 401 | 397 | 462 | 401 | 397 | 462 |

The stabilization effect was calculated based on the following formula: % PDE5 stabilization effect = (MFI-ZPE)/(HPE-ZPE) x100 and the results are shown in Table 74 and Table 75 below.

**Table 74: Percent stabilization effect for ligand-treated mutant clones**

| **Conc. (µM)** | **OT-001366** | | | **OT-001368** | | | **OT-001370** | | | **OT-001378** | | |
|---|---|---|---|---|---|---|---|---|---|---|---|---|
| | **VDF** | **SDF** | **TDF** | **VDF** | **SDF** | **TDF** | **VDF** | **SDF** | **TDF** | **VDF** | **SDF** | **TDF** |
| 100 | 100.0 | 100.0 | 100.0 | 100.0 | 100.0 | 100.0 | 100.0 | 100.0 | 100.0 | 100.0 | 100.0 | 100.0 |
| 33.333 | 91.8 | 50.0 | 48.7 | 89.4 | 44.8 | 61.7 | 110.0 | 60.3 | 81.6 | 94.3 | 51.3 | 67.9 |
| 11.111 | 70.8 | 21.4 | 18.0 | 72.4 | 16.1 | 24.3 | 103.9 | 27.0 | 44.5 | 87.2 | 19.4 | 28.9 |
| 3.704 | 39.1 | 6.0 | 3.9 | 48.3 | 2.0 | 10.0 | 90.2 | 10.0 | 18.3 | 69.5 | 6.5 | 9.9 |
| 1.235 | 17.1 | -0.4 | -2.8 | 26.3 | -3.1 | 5.1 | 66.9 | 2.6 | 5.6 | 44.6 | 1.5 | 2.5 |
| 0.412 | 5.5 | -2.4 | 5.4 | 12.0 | -1.0 | 5.9 | 42.5 | -1.6 | 1.4 | 23.6 | 0.0 | 0.1 |
| 0.137 | 0.4 | -0.3 | 3.9 | 4.0 | -4.4 | 2.3 | 21.4 | -4.6 | 0.9 | 9.6 | -0.8 | -0.5 |
| 0.046 | -2.3 | 2.6 | -0.9 | 0.2 | -2.7 | 3.8 | 10.3 | -3.1 | 1.4 | 4.1 | -1.1 | -0.4 |
| DMSO | 0.0 | 0.0 | 0.0 | 0.0 | 0.0 | 0.0 | 0.0 | 0.0 | 0.0 | 0.0 | 0.0 | 0.0 |
| NIH3T3 | - | - | - | - | - | - | - | - | - | - | - | - |

**Table 75: Percent stabilization effect for ligand-treated mutant clones**

| **Conc. (µM)** | **OT-001379** | | | **OT-001075** | | | **OT-001075** | | |
|---|---|---|---|---|---|---|---|---|---|
| | **VDF** | **SDF** | **TDF** | **VDF** | **SDF** | **TDF** | **VDF** | **SDF** | **TDF** |
| 100 | 100.0 | 100.0 | 100.0 | 100.0 | 100.0 | 100.0 | 100.0 | 100.0 | 100.0 |
| 33.333 | 91.9 | 79.7 | 93.9 | 65.5 | 79.3 | 110.9 | 62.0 | 96.0 | 123.3 |
| 11.111 | 91.0 | 49.5 | 67.0 | 56.6 | 49.7 | 101.2 | 53.2 | 88.2 | 136.4 |
| 3.704 | 86.4 | 21.2 | 34.6 | 52.0 | 24.3 | 63.8 | 52.6 | 72.1 | 134.4 |
| 1.235 | 74.8 | 5.8 | 11.3 | 46.3 | 6.8 | 28.3 | 46.8 | 41.2 | 94.9 |
| 0.412 | 57.2 | 0.4 | 1.4 | 34.4 | 2.1 | 8.8 | 42.2 | 8.6 | 60.4 |
| 0.137 | 31.9 | -0.7 | -1.7 | 18.7 | 0.1 | 1.7 | 40.5 | -2.1 | 28.1 |
| 0.046 | 15.3 | -1.1 | -1.3 | 7.1 | -0.3 | 0.3 | 41.1 | -6.8 | 4.2 |
| DMSO | 0.0 | 0.0 | 0.0 | 0.0 | 0.0 | 0.0 | 0.0 | 0.0 | 0.0 |
| NIH3T3 | - | - | - | - | - | - | - | - | - |

Plots of the % effect versus concentration were generated with Graphpad Prism 5 and EC₅₀ values were calculated using a four-parameter nonlinear regression model. EC₅₀ data are shown in Table 76. EC₅₀ values were not calculated for HPE/ZPE < 3.

**Table 76. EC₅₀ values for mutant cell pools**

| **Construct ID (Description)** | **EC₅₀ VDF** | **EC₅₀ SDF** | **EC₅₀ TDF** |
|---|---|---|---|
| OT-001366 (hPDE5 (535-860 of WT, L554R, Q586P, K710N, K730E) | 5.6 | - | - |
| OT-001368 (hPDE5 (535-860 of WT, Q586L, S663Y, A762T, E808G)) | 4.6 | - | - |
| OT-001370 (hPDE5 (535-860 of WT, L554R, Q589E, A823T)) | 0.8 | 21.4 | 14.8 |
| OT-001378 (hPDE5 (535-860 of WT, K770Q, K848N)) | 1.6 | 29.4 | 19.6 |
| OT-001379 (hPDE5 (535-860 of WT, N605D)) | 0.3 | 13.6 | 6.4 |
| OT-001084 | 0.4 | 14.2 | 2.7 |
| OT-001075 | - | - | - |

Based on this screening of a human PDE5 library (Experiment 2), several clones were identified with destabilizing mutations, which upon ligand binding to PDE5 serve to stabilize the protein. OT-001366, OT-001368, OT-001370, OT-001378 and OT-001379 were all identified as destabilizing mutants that may warrant further characterization.

Select PDE5 clones from both experiment 1 and experiment 2 were directly compared. Cells expressing individual clones were then incubated with vehicle, Dimethylsulfoxide (DMSO), 10 µM Vardenafil, Sildenafil or Tadalafil. Median GFP fluorescence was quantified using FACS and the results from the experiment are presented in Table 77. The stabilization ratio was calculated as the fold change in GFP intensity in ligand treated samples compared to treatment with DMSO (i.e. in the absence of ligand) with the same construct. The destabilizing mutation co-efficient was calculated as the fold change in GFP intensity in the DHFR mutant constructs in the absence of the ligand; to the GFP intensity of the wildtype construct in the absence of the ligand and is represented in Table 78. Destabilizing mutation co-efficient ratio less than 1 and stabilization ratios greater than 1 are desired in DDs. The experiments were conducted using the PDE5 (R732L) mutant as a control.

**Table 77: Fluorescence intensity of PDE5 mutants**

| **Construct ID/Library clone ID** | **DMSO** | **Vardenafil** | **Sildenafil** | **Tadalafil** |
|---|---|---|---|---|
| Parental | 46.47 | - | - | - |
| OT-001084 | 255.47 | 8608 | 3103 | 3428 |
| LibC000020 | 273 | 2499 | 513 | 509 |
| OT-001363 | 315.67 | 1566 | 1356 | 1132 |
| LibC000005 | 359 | 4240 | 1136 | 814 |
| LibC000009 | 881.33 | 7393 | 8260 | 8353 |
| OT-001075 | 6732 | 12000 | 7082 | 10100 |

**Table 78: Ratio calculations for PDE5 mutants**

| **Construct ID/Library clone ID** | **Stabilization ratio** | | | **Destabilizing mutation co-efficient** |
|---|---|---|---|---|
| | **Vardenafil** | **Sildenafil** | **Tadalafil** | |
| Parental | - | - | - | - |
| OT-001075 | 1.78 | 1.05 | 1.50 | - |
| OT-001084 | 33.70 | 12.15 | 13.42 | 0.04 |
| LibC000020 | 9.15 | 1.88 | 1.86 | 0.04 |
| OT-001363 | 4.96 | 4.30 | 3.59 | 0.05 |
| LibC000005 | 11.81 | 3.16 | 2.27 | 0.05 |
| LibC000009 | 8.39 | 9.37 | 9.48 | 0.13 |

As shown in Table 78, all constructs showed destabilizing mutation co-efficient less than one indicating strong destabilization. All constructs also showed ligand dependent stabilization with all three ligands as observed by the stabilization ratios of less than one. It was noted, however, that the ligand dependent stabilization observed for all the mutants generated by random mutagenesis was less than the stabilization ratios observed with the PDE5 (R732L) mutation.

### Example 16. Analysis of IL15-PDES-R732 constructs

A library of mutations was generated by site directed mutagenesis of amino acid 732 of PDE5. The amino acid at the position 732 was mutated to any of the known amino acids, including, but not limited to, aspartic acid, glutamic acid, glutamine, asparagine, histidine, serine, threonine, tyrosine, cysteine, methionine, tryptophan, alanine, isoleucine, leucine, phenylalanine, valine, proline, and glycine. and each of the mutants was fused to a payload of interest, IL 15-IL 15Ra fusion protein via a linker. The constructs were transiently transfected into HEK 293T cells and incubated with 1 µM Vardenafil for 48 hours. The properties of the DDs regulated IL15-IL15Ra payloads were analyzed in the presence and absence of ligands via FACS using the HA antibody as previously described. The results are shown in Table 79, where the stabilization ratio was defined as the ratio of expression, function or level of a protein of interest, i.e. IL15-IL15Ra in response to the stimulus, i.e. Vardenafil; to the expression, of the IL15-IL15Ra in the absence of the stimulus, i.e. DMSO control. The constructs were ordered by their stabilization ratio from highest to the lowest. The destabilizing mutation coefficient was calculated as the fold change in GFP intensity in the PDE5 mutant constructs compared to the PDE5 wildtype construct OT-IL15-082) in the absence of the ligand. Destabilizing mutation coefficients less than 1 are desirable. Stabilization ratios greater than 1 are desired in DDs.

**Table 79: % of HA positive, IL15-IL15Ra positive cells**

| **Construct ID** | **Mutation** | **DMSO** | **1 µM Vardenafil** | **Stabilization ratio** | **Destabilizing mutation co-efficient** |
|---|---|---|---|---|---|
| OT-001341 | R732G | 16 | 45.5 | 2.84 | 0.34 |
| OT-001336 | R732N | 19 | 43.5 | 2.29 | 0.41 |
| OT-001339 | R732E | 17.5 | 40 | 2.29 | 0.37 |
| OT-001333 | R732Y | 20.3 | 46.4 | 2.29 | 0.43 |
| OT-001335 | R732Q | 19.7 | 43.9 | 2.23 | 0.42 |
| OT-001338 | R732D | 19.6 | 43.4 | 2.21 | 0.42 |
| OT-001332 | R732A | 22 | 47.3 | 2.15 | 0.47 |
| OT-001334 | R732H | 18.9 | 39.5 | 2.09 | 0.40 |
| OT-001328 | R732M | 21.7 | 44.9 | 2.07 | 0.46 |
| OT-001329 | R732V | 21.3 | 43.7 | 2.05 | 0.46 |
| OT-001324 | R732L | 23.7 | 47 | 1.98 | 0.51 |
| OT-001340 | R732C | 22.9 | 43.3 | 1.89 | 0.49 |
| OT-001342 | R732W | 23.4 | 44.2 | 1.89 | 0.50 |
| OT-001330 | R732S | 27.3 | 51.4 | 1.88 | 0.58 |
| OT-001327 | R732I | 24.3 | 44.6 | 1.84 | 0.52 |
| OT-001337 | R732K | 27.6 | 44.7 | 1.62 | 0.59 |
| OT-001331 | R732P | 29.8 | 40.8 | 1.37 | 0.64 |
| OT-001321 | WT | 46.8 | 56 | 1.20 | - |

As shown in Table 79, constructs OT-001341, OT-001336, OT-001339, OT-IL15-098, OT-001335, OT-001338, OT-001332, OT-001334, OT-001328, and OT-001329 showed stabilization ratios greater than the initially identified PDE5 DD mutant, R732L indicating superior ligand dependent stabilization. While constructs, OT-001340, OT-001342, OT-001330, OT-001327, OT-001337 and OT-001331 showed ligand dependent stabilization lower than the R723L construct. However, all constructs displayed stabilization ratios greater than wild type construct. Compared to the wildtype control, all constructs tested also showed lower percentage HA positive cells in the absence of ligand and destabilizing mutation co-efficient less than 1 indicating that engineering any of the mutations at position 732 of the full length PDE5, destabilizes the protein. Taken together, mutagenesis of the PDE5-IL15-IL15Ra constructs at R732 of PDE5 identified mutants with reduced basal expression and improved dynamic range.

HA and/or Flag tags were cloned into vectors containing the IL15-IL15Ra fusion constructs to generate constructs OT-001388, OT-001321, OT-001323, OT-001324 and OT-001325. One of the tags was placed in frame with the IL15 sequence while the other was placed in frame with the IL15Ra sequence. The tags were added to facilitate the easy detection of both IL15 and IL15Ra. HEK 293T cells were transiently transfected with the constructs for 24 hours and treated with Vardenafil for 48 hours at 1 µM. The percentage of Flag and HA double positive cells was analyzed by FACS. The results are shown in Table 80. The stabilization ratio was defined as the ratio of expression, function or level of a protein of interest, i.e. IL15-IL15Ra in response to the stimulus, i.e. Vardenafil; to the expression, of the IL15-IL15Ra in the absence of the stimulus, i.e. DMSO control. The destabilization ratio was calculated as the ratio of expression, of IL15Ra in the absence of the stimulus specific to the effector module i.e. Vardenafil to the expression, function or level of the protein of interest, that is expressed constitutively (OT-001388) and in the absence of the stimulus specific to the SRE. The destabilizing mutation co-efficient refers to the expression, function or level or a protein of interest, appended to a particular DD mutant e.g. PDE5 (R732L), in the absence of the stimulus specific to the SRE; to the protein expression, function or level of the protein of interest, appended to the corresponding wildtype sequence from which the particular DD mutant is derived e.g., PDE5 (WT), in the absence of the stimulus. The Stabilization ratios greater than one and destabilization ratio as well as destabilizing mutation co-efficient less than one is desired.

**Table 80: Percentage HA positive and Flag positive cells**

| **Construct** | **Mutation** | **Control** | **Vardenafil** | **Stabilization ratio** | **Destabilization ratio** | **Destabilizing mutation co-efficient** |
|---|---|---|---|---|---|---|
| OT-001388 | No DD | 26 | 25 | 0.96 | - | - |
| OT-001321 | WT | 19.6 | 16.8 | 0.86 | 0.75 | - |
| OT-001323 | F736A | 8.11 | 24.9 | 3.07 | 0.31 | 0.41 |
| OT-001324 | R723L | 9.25 | 26.4 | 2.85 | 0.36 | 0.47 |
| OT-001325 | R732L, F736A | 7.44 | 21.6 | 2.90 | 0.29 | 0.38 |

As expected, the constitutive construct OT-001388 and the PDE5 wildtype construct, OT-001321 did not show any ligand dependent increase in double positive cells. Ligand dependent increase in double positive cells was observed with constructs 084- 086. These data suggest that the tags can be utilized as reliable surrogates to investigate the expression of IL15 and IL15Ra proteins in the constructs. All IL15-IL15Ra constructs appended to a DD mutant showed destabilization ratios and destabilizing mutant co-efficient less than 1, which indicates that the specific mutations engineered can destabilize the protein of interest.

### Example 17. Anti-tumor efficacy of Tadalafil and Vardenafil

To evaluate PDE5 inhibitor small molecule drugs, human T cells were transduced with lentiviral vectors expressing a human CD19-targeting CAR fused to PDE5-DD. To confirm transduction, T cells were cultured with Tadalafil or Vardenafil (ligand) and analyzed by flow cytometry analysis for CD19 CAR expression. To evaluate anti-tumor activity in vivo, immunodeficient (NOD.Cg PrkdcscidIl2rgtm1Wjl/SzJ, NSG) mice were implanted with luciferase-expressing, CD19+ Nalm6 B cell leukemia cell line (tumor). After tumor implantation, tumor burden was assessed by bioluminescent imaging (BLI) on day 5 and mice were distributed into groups to create equivalent tumor burden across cohorts. T cells were thawed and tumor bearing NSG mice were infused with either: (1) unmodified T cells, (2) PDE5-regulated CD19-CART (OT-001456) cells, or (3) Constitutive CD19-CART (OT-001407). Total T cell dose was adjusted to achieve 5 million CD19-CAR⁺ T cells and injected into mice on day 5. Ligand treatment was initiated on day 7 and continued till day 28. Mice were dosed orally Q.D (once a day) or B.I.D (twice a day). for 25 days with Tadalafil or Vardenafil at 0 (vehicle, DMSO alone), or 30 mg/kg (mpk). Tumor growth was monitored by bi-weekly bioluminescent imaging (Figure 9A and Figure 9B). Increased Tadalafil dose-density from a Q.D. dose regime to a B.I.D dose regime significantly increased DD-CAR T cell anti-tumor efficacy in mice transduced with OT-001456. Dosing with Vardenafil in OT-001456 transduced mice reduced tumor burden compared to mice treated with vehicle controls (OT-001456 transduced or untransduced). However, increasing dose density did not improve Vardenafil dependent anti-tumor efficacy of OT-001456 transduced T cells.

### Example 18. Induction of apoptosis by regulated CD19 CAR

Donor derived T cells were thawed and activated overnight in the presence of CD3/CD28 Dynabeads at a 3:1 bead: cell ratio. The following day, cells were transduced with lentivirus corresponding to constructs, OT-001407 and OT-001456. Cells were expanded over the course of 9 days by the addition of fresh media, maintaining cells around 0.5×10^6 cells/mL. The cells were then frozen. To confirm that the transduced T cells are functional, they were thawed and cultured with CD19 expressing target cells, Nalm6-Katushka, at effector: target ratios of 10:1, 3:1, 1:1, 0.3:1, 0.1:1, and 0.03:1 for 6 days. Images captured on the Incucyte Zoom were analyzed for proliferation of target cells by measuring red fluorescence over time, and for apoptosis of target cells by measuring Annexin V-Green reagent over time. OT-001407 effector cells were included as a positive control, which effectively killed Nalm6 targets at 1:1 ratio and above. OT-001456 transduced cells demonstrated regulated CAR activity as evidenced by the killing Nalm6 target cells only in the presence of 3 µm Tadalafil at 10:1 effector: target ratio, but had no activity in the presence of DMSO.

Forty-eight hours after co-cultures were started, supernatants were harvested for cytokines, IL2 and IFN gamma analysis by MSD. Data show that inhibition of proliferation and cytokine secretion are proportional to the amount of CAR expression.

### Example 19. Regulated CAR expression and function with different construct designs

To test regulated CAR expression and function with CD28 costimulatory domain, donor T cells were thawed and activated overnight in the presence of CD3/CD28 Dynabeads at a 3:1 bead: cell ratio. The following day, cells were transduced with lentivirus corresponding to OT-001456 and OT-001508 constructs. Cells were expanded by addition of fresh media over the course of 10 days, maintaining cells around 0.5×10^6 cells/mL. Cells were co-cultured with Nalm6-Katushka cells at T cell: Nalm6 ratios of 10:1, 5:1, and 2.5:1 for 4 days to evaluate CAR function in the presence or absence of 10µm tadalafil. Images captured on the Incucyte Zoom were analyzed for proliferation of target cells by measuring red fluorescence over time. To confirm CAR expression, FACS was performed with CD19-Fc staining on day 7 and day 10 of the T cell expansion, following an overnight incubation with 10µM tadalafil. Constructs with CD28 costimulatory domain appeared to lose expression over time, similar to OT-001456. In contrast to OT-001456, construct with CD28 co-stimulatory domains OT-001508 demonstrated high basal killing even in the absence of tadalafil.

Experiments similar to those described in testing CD28 domain were performed to test for regulated CAR expression and function in constructs where the DD domain is placed internally versus at the C terminal domains. Constructs utilized included OT-001456 with C terminal PDE5 and OT-001511 with PDE5 placed internally. Regulated CAR expression was maintained when the DD was placed internally within the construct. Tadalafil dependent cell killing was also observed with the internal PDE5 DD construct. Similar results were obtained when performed in cells transduced with hPDE5 wildtype with a C terminal PDE5 placement (OT-001512) or an internal PDE5 placement (OT-001510). The wild type hPDE5 constructs demonstrated that the CAR containing the wildtype PDE5 had reduced CAR expression compared to the PDE5 DD regulated constructs. However, the expression of CAR was sufficient to reduce Nalm6 proliferation indicating that the expression levels were functional.

The percentage of CAR positive cells and Median fluorescence intensity of the CAR constructs described in this example are provided in Table 81.

**Table 81: CAR expression**

| Construct | Day 7 | | | | Day 10 | | | |
|---|---|---|---|---|---|---|---|---|
| | DMSO | | Tadalafil | | DMSO | | Tadalafil | |
| | Avg (% CAR +) | Avg (MF) | Avg (% CAR +) | Avg (MF) | Avg (% CAR +) | Avg (MF) | Avg (% CAR +) | Avg (MF) |
| OT-001407 | 66.1 | 3475 | 65.3 | 3736 | 51 | 723 | 51.8 | 980 |
| OT-001512 | 67.7 | 1584 | 67.4 | 1811 | 49.2 | 459 | 51.5 | 547 |
| OT-001456 | 44.5 | 253 | 74.3 | 1944 | 1.7 | 49 | 32.5 | 213 |
| OT-001501 | 62 | 591 | 70.3 | 1721 | 12 | 106 | 39.6 | 279 |
| OT-001510 | 68 | 1245 | 66.7 | 1285 | 35.9 | 144 | 39.8 | 181 |
| OT-001511 | 59.5 | 505 | 69.7 | 1591 | 7.7 | 84 | 22.2 | 121 |
| OT-001509 | 63.5 | 658 | 68.8 | 1621 | 14.5 | 104 | 29.7 | 151 |
| OT-001508 | 23.8 | 90 | 44 | 172 | 0.6 | 36 | 12.6 | 71 |
| Vector | 0.9 | 18 | 1 | 18 | 0.4 | 13 | 0.1 | 12 |
| Untransduced | 0.5 | 6 | 0.4 | 6 | 0.2 | 10 | 0.1 | 10 |

U2OS cells were transduced with various volumes of lentivirus for GFP tagged constitutive (OT-001905) or PDE5-DD (OT-001904) CD19 CAR construct, or HA tagged constitutive (OT-001906) or PDE5-DD (OT-001903) and (OT-001907) CD19 CAR construct, or empty vector. Cells were cultured overnight in the presence or absence of 10µm tadalafil. Expression of CAR was detected by staining cells with 10ug/mL CD19-Fc. A correlation was observed between (a) CAR expression and the GFP tag/HA tag expression, and (b) the regulated expression of CAR and GFP/HA expression by PDE5-DD.

U2OS cells were transduced with lentivirus corresponding to the constitutive (OT-001906) or PDE5-DD (OT-001903) HA-tagged CD19-CAR constructs, or empty vector. Cells were seeded at 5000 cells/well in 96 well plates and cultured overnight. Expression of CAR was detected using an anti-HA antibody and Incucyte Immunocytochemistry (Ann Arbor, Michigan) with Opti-Green background eliminator and FabFluor488 reagents according to manufacturer's protocol, and quantitated using Incucyte Zoom software to measure green fluorescence (antibody binding per well) normalized to cell confluence to account for growth of cells over time. A negative control mouse IgG1 isotype antibody was included. Antibodies were added 5 hours after the initiation of the experiment and an increase in fluorescence was observed in the cells expressing the constitutive construct or the ligand pretreated regulated construct, but not the regulated construct in the absence of ligand. As expected, the negative control mouse IgG1 isotype resulted in no fluorescent signal. At 24 hours, 5µM tadalafil was added to the (OT-001903) group and an increase fluorescence was observed which correlated with the stabilization of the DD.

T cells from transduced with OT-001903 or OT-001906 were treated overnight with 10µM tadalafil or vardenafil or left untreated. Cells were washed 3 times with cell media, then stained for CAR expression using 10µg/mL CD19-Fc 0, 2, 4, and 24hr post-washout. CD19 CAR expression on the surface of T cells began to decrease as early as two hours after ligand withdrawal and by 24 hours CAR expression levels were similar to untreated controls.

To test for regulated CAR expression with GFP and Luciferase tagged constructs, T cells were thawed and activated overnight in the presence of CD3/CD28 Dynabeads at a 3:1 bead: cell ratio. The following day, cells were transduced with lentivirus from indicated constructs. Constructs OT-001602, OT-001598, OT-001456, OT-001902, OT-001901, OT-001900, OT-001899, OT-001898, and OT-001908 were tested with increasing volumes of lentivirus. Cells were expanded by addition of fresh media over the course of 10 days, maintaining cells around 0.5x10^6 cells/mL, then co-cultured with Nalm6-iRFP cells to evaluate CAR function in the presence or absence of 10µM tadalafil. Images captured on the Incucyte Zoom were analyzed for proliferation of target cells by measuring red fluorescence over time. To confirm CAR expression, FACS was performed with CD19-Fc staining on day 7 and day 10 of the T cell expansion, following an overnight incubation with 10µm tadalafil. For GFP containing constructs, CAR expression correlated with increased GFP fluorescence. For luciferase constructs, luminescence was measured following addition of luciferin substrate for 5 minutes and similar to GFP, luciferase showed an increased signal which correlated with CAR expression. The increase in signal for both GFP and luciferase activity also corresponded to higher transduction amounts.

### Example 20. In vivo ligand dosing

An in vivo study was performed to determine if cell dosing and/or various ligand exposure schemas lead to enhanced efficacy against NALM-Luc cells in female NSG mice. The different groups included in the study are provided in Table 82.

**Table 82: CAR expression**

| Group | Construct | in vitro treatment | in vivo treatment | Dosing Schedule | Cell Dose (M= million) |
|---|---|---|---|---|---|
| 1 | Empty Vector | None | Vehicle | | 5M |
| 2 | OT-001407 | None | Vehicle | | 1M |
| 3 | OT-001512 | None | Vehicle | | 5M |
| 4 | OT-001456 | None | Vehicle | | 5M |
| 5 | OT-001456 | None | Tadalafil | BID 50mg/kg | 5M |
| 6 | OT-001456 | None | Tadalafil | BID 100mg/kg (for 7 days); QD 50mg/kg | 5M |
| 7 | OT-001456 | None | Tadalafil | BID 100mg/kg (for 7 days); BID 50mg/kg | 5M |
| 8 | OT-001456 | Tadalafil 10µM (24hr prior to freeze) | Vehicle | | 5M |
| 9 | OT-001456 | Tadalafil 10µM (24hr prior to freeze) | Tadalafil | BID 50mg/kg | 5M |
| 10 | OT-001456 | None | Vehicle | | 5M (day 7) and 5M (day 10) |
| 11 | OT-001456 | None | Tadalafil | BID 50mg/kg | 5M (day7) and 5M (day 10) |
| 12 | OT-001456 | None | Vehicle | | 10M |
| 13 | OT-001456 | None | Tadalafil | BID 50mg/kg | 10M |
| 14 | OT-001455 | None | Vehicle | | 5M |
| 15 | OT-001455 | None | Tadalafil | BID 50mg/kg | 5M |

The average bioluminescence intensity was measured for all groups until day 31. The results are shown in Table 83.

**Table 83: Average BLI**

| **Days Post** | **6** | **13** | **19** | **23** | **26** | **31** |
|---|---|---|---|---|---|---|
| Empty Vector | 5.78E+06 | 1.42E+09 | 1.71E+10 | | | |
| | 2.72E+06 | 4.63E+08 | 3.60E+09 | 4.04E+10 | | |
| | 3.89E+06 | 1.36E+09 | 1.31E+09 | 1.19E+10 | | |
| | 1.11E+06 | 9.48E+07 | 3.16E+09 | 1.14E+10 | | |
| | 3.82E+06 | 8.06E+08 | 1.11E+10 | | | |
| | 2.16E+06 | 5.71E+08 | 9.52E+09 | | | |
| | 2.13E+06 | 2.60E+08 | 1.06E+10 | | | |
| | 2.70E+06 | 7.32E+08 | 9.47E+09 | | | |
| OT-001407 (1M) | 1.31E+06 | 2.86E+07 | 2.44E+08 | 1.08E+09 | 1.03E+10 | |
| | 5.72E+06 | 1.12E+08 | 1.10E+09 | 1.73E+09 | 8.83E+09 | |
| | 3.79E+06 | 9.43E+07 | 1.14E+09 | 5.13E+09 | | |
| | 4.03E+06 | 7.71E+07 | 9.38E+08 | 3.65E+09 | 2.81E+10 | |
| | 2.68E+06 | 5.10E+07 | 4.76E+08 | 1.77E+09 | 1.04E+10 | |
| | 2.77E+06 | 1.12E+08 | 5.20E+08 | 2.72E+09 | 1.81E+10 | |
| | 2.12E+06 | 4.81E+07 | 7.84E+08 | 2.69E+09 | 1.48E+10 | |
| | 2.19E+06 | 1.09E+08 | 5.81E+08 | 1.61E+09 | 1.02E+10 | |
| OT-001512 (5M PDE5 WT) | 2.65E+06 | 8.20E+05 | 1.17E+06 | 5.18E+06 | 1.06E+07 | 5.03E+07 |
| | 1.51E+06 | 1.20E+06 | 1.40E+06 | 1.94E+06 | 5.64E+06 | 9.82E+07 |
| | 5.70E+06 | 1.20E+06 | 3.06E+06 | 6.29E+06 | 1.25E+07 | 7.62E+06 |
| | 4.11E+06 | 1.05E+06 | 9.67E+05 | 7.95E+05 | 1.33E+06 | 1.11E+06 |
| | 2.11E+06 | 9.44E+05 | 1.00E+06 | 8.88E+05 | 9.22E+05 | 1.49E+06 |
| | 2.20E+06 | 9.02E+05 | 9.11E+05 | 1.25E+06 | 2.95E+06 | 1.35E+07 |
| | 3.71E+06 | 4.23E+06 | 1.00E+07 | 8.18E+06 | 1.47E+07 | 8.09E+07 |
| | 2.78E+06 | 9.51E+05 | 7.60E+05 | 5.68E+05 | 5.41E+05 | 8.20E+05 |
| OT-001456 (Vehicle) | 3.68E+06 | 4.13E+08 | 1.24E+10 | | | |
| | 2.05E+06 | 2.65E+08 | 4.08E+09 | | | |
| | 4.13E+06 | 1.17E+07 | 1.72E+10 | | | |
| | 2.64E+06 | 3.08E+08 | 8.41E+09 | | | |
| | 1.54E+06 | 1.19E+08 | 2.58E+09 | 8.37E+09 | | |
| | 5.66E+06 | 9.57E+08 | 3.24E+09 | 4.33E+10 | | |
| | 2.22E+06 | 4.19E+08 | 8.71E+09 | | | |
| | 2.82E+06 | 5.02E+08 | 9.29E+09 | | | |
| OT-001456 (TDF 50mpk BID) | 5.62E+06 | 5.33E+06 | 6.96E+06 | 6.84E+05 | 1.01E+06 | 5.87E+08 |
| | 4.25E+06 | 2.29E+06 | 6.91E+06 | 6.48E+05 | 2.95E+07 | 1.58E+06 |
| | 1.55E+06 | 1.11E+06 | 5.08E+05 | 1.96E+06 | 4.37E+06 | 3.39E+07 |
| | 2.64E+06 | 3.75E+06 | 5.03E+06 | 5.96E+05 | 3.62E+07 | 1.67E+07 |
| | 2.85E+06 | 3.61E+06 | 5.45E+05 | 2.82E+07 | 4.12E+07 | 2.26E+06 |
| | 3.62E+06 | 3.32E+06 | 4.62E+05 | 6.02E+05 | 6.84E+05 | 2.66E+07 |
| | 2.05E+06 | 3.07E+06 | | | | |
| | 2.27E+06 | 2.95E+07 | | | | |
| OT-001456 (TDF 100mpk BID (7 days) followed by 50mpk QD) | 4.35E+06 | 8.29E+06 | 8.71E+06 | 4.15E+07 | 2.10E+08 | 3.46E+09 |
| | 2.31E+06 | 1.39E+06 | 4.54E+06 | 1.17E+07 | 1.10E+06 | 3.63E+06 |
| | 1.58E+06 | 1.45E+06 | 3.38E+06 | 1.50E+07 | 5.32E+07 | 1.61E+08 |
| | 5.54E+06 | 4.62E+06 | 6.09E+05 | 3.80E+07 | 1.84E+08 | 2.00E+08 |
| | 2.03E+06 | 8.08E+05 | 6.17E+05 | 4.72E+07 | 2.46E+06 | 1.88E+09 |
| | 2.85E+06 | 6.72E+05 | 1.42E+07 | 1.42E+06 | 3.70E+08 | 3.60E+09 |
| | 3.59E+06 | 3.27E+06 | 8.86E+06 | 5.04E+07 | 2.05E+08 | 1.49E+09 |
| | 2.63E+06 | 5.69E+05 | 4.40E+06 | 1.03E+07 | 4.85E+07 | 9.72E+07 |
| OT-001456 (TDF 100mpk BID (7 days) followed by 50mpk BID) | 2.86E+06 | 2.89E+06 | 5.46E+05 | 7.61E+06 | 1.06E+07 | 1.13E+08 |
| | 1.59E+06 | 5.11E+05 | 3.47E+06 | 5.86E+05 | 1.95E+07 | 1.66E+07 |
| | 2.00E+06 | 2.40E+06 | 6.36E+06 | 1.72E+07 | 5.24E+07 | 3.75E+08 |
| | 2.31E+06 | 1.92E+06 | 3.08E+06 | 1.40E+07 | 1.24E+07 | 1.10E+07 |
| | 2.62E+06 | 3.73E+06 | 5.78E+05 | 9.21E+06 | 2.69E+06 | 2.76E+08 |
| | 4.43E+06 | 5.66E+05 | 3.14E+06 | 6.16E+06 | 1.00E+07 | 1.90E+07 |
| | 3.59E+06 | 9.95E+06 | 1.53E+07 | 6.86E+05 | 1.13E+08 | 1.89E+09 |
| | 5.51E+06 | 7.59E+06 | 7.38E+06 | 2.13E+07 | 1.46E+06 | 4.44E+08 |
| OT-001456 (10µM in Vitro_Vehicle) | 2.58E+06 | 1.92E+08 | 3.52E+07 | 8.32E+07 | 3.32E+10 | |
| | 2.32E+06 | 1.06E+08 | 2.97E+09 | 9.80E+09 | | |
| | 2.00E+06 | 2.32E+06 | 3.03E+09 | 1.32E+10 | | |
| | 1.59E+06 | 1.22E+08 | 3.38E+09 | 1.28E+10 | | |
| | 4.43E+06 | 4.09E+08 | 5.16E+09 | | | |
| | 5.42E+06 | 5.27E+08 | 3.35E+07 | 6.89E+09 | | |
| | 2.87E+06 | 2.07E+08 | 9.73E+06 | 6.58E+09 | | |
| | 3.55E+06 | | | | | |
| OT-001456 (10µM in Vitro and TDF 50mpk BID) | 1.60E+06 | 1.32E+06 | 1.85E+06 | 5.19E+06 | 1.56E+07 | 1.37E+07 |
| | 1.81E+06 | 1.80E+06 | 4.61E+05 | 9.32E+05 | 8.50E+05 | 1.61E+06 |
| | 2.53E+06 | 1.97E+06 | 4.41E+06 | 4.13E+05 | 6.08E+05 | 6.91E+06 |
| | 3.47E+06 | 2.58E+06 | 9.23E+05 | 4.94E+05 | 3.08E+07 | 3.14E+07 |
| | 2.92E+06 | 1.78E+06 | 4.18E+06 | 1.05E+07 | 1.60E+07 | 3.77E+07 |
| | 5.32E+06 | 1.45E+07 | 1.26E+07 | 4.13E+07 | 6.03E+07 | 1.80E+09 |
| | 4.44E+06 | 1.35E+07 | 6.35E+07 | 1.42E+08 | 2.89E+08 | 3.00E+09 |
| | 2.33E+06 | 1.18E+06 | 1.07E+06 | 2.14E+06 | 1.17E+06 | 5.78E+06 |
| OT-001456 (5M (day 7)+5M (day 10) Vehicle) | 1.79E+06 | 1.30E+08 | 2.19E+07 | 1.43E+09 | 3.14E+07 | 2.96E+10 |
| | 2.93E+06 | 1.85E+08 | 2.36E+09 | 1.12E+10 | | |
| | 1.60E+06 | 7.71E+07 | 4.63E+08 | 4.20E+09 | 9.97E+07 | |
| | 4.46E+06 | 2.89E+08 | 2.98E+09 | 6.01E+09 | | |
| | 5.26E+06 | 4.40E+08 | 5.56E+09 | | | |
| | 2.53E+06 | 9.93E+07 | 2.14E+09 | 8.57E+09 | | |
| | 2.35E+06 | 1.59E+08 | 1.54E+07 | 6.94E+07 | 1.30E+10 | |
| | 3.47E+06 | | | | | |
| OT-001456 (5M (day 7)+5M (day 10) TDF 50mpk BID | 3.44E+06 | | | | | |
| | 4.63E+06 | 1.69E+06 | 4.45E+05 | 4.57E+06 | 4.72E+06 | 8.27E+06 |
| | 5.25E+06 | 4.46E+05 | | | | |
| | 1.63E+06 | 5.02E+05 | 1.05E+06 | 1.24E+06 | 1.52E+06 | 2.16E+06 |
| | 2.49E+06 | 1.16E+06 | 5.10E+05 | 5.34E+05 | 8.27E+05 | 4.90E+05 |
| | 2.93E+06 | 1.15E+06 | 5.07E+05 | 5.13E+05 | 7.71E+05 | 4.80E+05 |
| | 2.37E+06 | 1.24E+06 | 1.31E+06 | 4.30E+05 | | |
| | 1.78E+06 | 9.98E+05 | 1.30E+06 | 1.07E+06 | 1.28E+06 | 9.16E+05 |
| OT-001456 (10M) Vehicle | 3.05E+06 | 1.60E+08 | 5.34E+09 | | | |
| | 2.38E+06 | 1.06E+08 | 3.40E+09 | 1.76E+10 | | |
| | 4.65E+06 | 1.23E+08 | 2.53E+09 | 1.42E+10 | | |
| | 1.64E+06 | 2.81E+07 | 5.84E+08 | | | |
| | 2.48E+06 | 1.34E+08 | 3.02E+09 | 6.21E+09 | | |
| | 1.76E+06 | 9.03E+07 | 1.98E+09 | 4.85E+09 | 1.90E+10 | |
| | 3.32E+06 | 1.77E+08 | 4.17E+09 | | | |
| | 5.23E+06 | 3.56E+08 | 5.38E+09 | | | |
| OT-001456 ((10M) TDF 50mpk BID) | 2.38E+06 | 2.26E+06 | 4.76E+06 | 6.55E+06 | 8.28E+06 | 1.35E+07 |
| | 4.65E+06 | 7.78E+06 | 9.50E+06 | 3.19E+07 | 9.26E+07 | 1.68E+09 |
| | 5.08E+06 | 5.75E+07 | 2.07E+07 | | | |
| | 1.65E+06 | 2.12E+06 | 3.44E+06 | 5.48E+05 | 3.08E+07 | 6.35E+07 |
| | 2.45E+06 | 2.47E+06 | | | | |
| | 3.08E+06 | 1.22E+06 | | | | |
| | 3.29E+06 | 1.21E+06 | 2.15E+06 | 3.37E+06 | 4.84E+06 | 8.80E+06 |
| | 1.72E+06 | 1.56E+06 | 3.71E+06 | 3.56E+06 | 1.29E+07 | 5.89E+05 |
| OT-001455 (Vehicle) | 4.67E+06 | 2.77E+06 | 4.84E+06 | 1.14E+07 | 1.82E+07 | 6.04E+06 |
| | 1.71E+06 | 2.19E+06 | 4.68E+06 | 8.10E+06 | 1.42E+07 | 5.87E+07 |
| | 2.39E+06 | 2.25E+06 | 5.89E+06 | 2.67E+07 | 4.25E+07 | 3.96E+08 |
| | 3.26E+06 | 1.88E+06 | 4.63E+05 | 7.64E+05 | 7.80E+05 | 4.12E+07 |
| | 2.43E+06 | 1.57E+06 | 5.24E+05 | 1.39E+07 | 1.17E+06 | 1.32E+08 |
| | 5.06E+06 | 3.60E+06 | 1.37E+07 | 6.52E+07 | 1.29E+08 | 1.30E+09 |
| | 1.65E+06 | 5.88E+05 | 4.05E+06 | 6.82E+06 | 5.94E+06 | 3.99E+07 |
| | 3.10E+06 | 1.98E+06 | 6.28E+06 | 1.35E+07 | 9.73E+05 | 3.75E+06 |
| OT-001455 (TDF 50mpk BID) | 1.71E+06 | 8.06E+05 | 1.05E+06 | 8.46E+05 | 6.23E+05 | 6.89E+06 |
| | 3.12E+06 | 1.11E+06 | 1.30E+06 | 9.82E+05 | 1.10E+06 | 6.14E+05 |
| | 3.14E+06 | 9.73E+05 | 6.66E+05 | 4.92E+05 | 9.90E+05 | 5.28E+05 |
| | 2.41E+06 | 9.25E+05 | 1.25E+06 | 4.45E+05 | 8.58E+05 | 1.15E+06 |
| | 4.92E+06 | 9.62E+05 | 1.21E+06 | 1.29E+06 | 1.95E+06 | 2.97E+06 |
| | 5.01E+06 | 8.78E+05 | | | | |
| | 2.43E+06 | 9.71E+05 | 8.78E+05 | 4.88E+05 | 8.45E+05 | 7.24E+05 |
| | 1.65E+06 | | | | | |

Table 84 provides the median survival for each of the groups in the cohort.

**Table 84: Median survival**

| Construct | Median survival (days) |
|---|---|
| Empty Vector | 19 |
| OT-001407 (1M) | 26 |
| OT-001512 (5MPDE5 WT) | Undefined |
| OT-001456 (Vehicle) | 20 |
| OT-001456 (TDF 50mpk BID) | Undefined |
| OT-001456 (TDF 100mpk BID > 50mpk QD) | 32 |
| OT-001456 (TDF 100mpk BID > 50mpk BID) | Undefined |
| OT-001456 (10uM in Vitro_Vehicle) | 23 |
| OT-001456 (10uM in Vitro_TDF 50mpk BID) | Undefined |
| OT-001456 ((5M+5M) Vehicle) | 23 |
| OT-001456 ((5M+5M) TDF 50mpk BID) | Undefined |
| OT-001456 ((10M) Vehicle) | 22 |
| OT-001456 ((10M) TDF 50mpk BID) | Undefined |
| OT-001455(Vehicle) | Undefined |
| OT-001455 (TDF 50mpk BID) | Undefined |

### Example 21. Mutagenesis of the amino acid F736 of hPDE5

The analysis of mutants identified by site directed mutagenesis identified amino acid hotspots whose mutation confers destabilization and ligand dependent stabilization properties to hPDE5. The amino acid at position 736 was chosen for the further analysis since the hPDE5 (F736A) mutation possessed the properties described above. To improve the DD characteristics of this DD, the amino acid at the position 736 was mutated to any of the known amino acids, including, but not limited to, aspartic acid, glutamic acid, glutamine, asparagine, histidine, serine, threonine, tyrosine, cysteine, methionine, tryptophan, alanine, isoleucine, leucine, phenylalanine, valine, proline, and glycine. The library of mutations was generated by site directed mutagenesis and each of the mutants in the library was fused to a reporter protein e.g. AcGFP via a linker and transduced into HCT116 cells. The properties of the DDs were analyzed via FACS both in the presence and absence of ligands. Ligands that were evaluated included, but not limited Sildenafil, and Vardenafil. Table 85 shows the MFI of cells treated with 10µM vardenafil for 48 hours. In Table 85 VDF indicates vardenafil.

**Table 85: Vardenafil dependent MFIs**

| **Alias** | **Mutation** | **Group** | **MFI** | **Stabilization ratio** |
|---|---|---|---|---|
| - | | Parental | 1153 | |
| OT-001269 | F736P | Control | 6690 | - |
| | | VDF | 144781 | 21.64 |
| OT-001264 | F736W | Control | 32415 | - |
| | | VDF | 158156 | 4.88 |
| OT-001272 | F736C | Control | 44895 | - |
| | | VDF | 151888 | 3.38 |
| OT-001276 | F736D | Control | 25733 | - |
| | | VDF | 167459 | 6.51 |
| OT-001266 | F736Q | Control | 54247 | - |
| | | VDF | 240810 | 4.44 |
| OT-001267 | F736E | Control | 38227 | - |
| | | VDF | 199986 | 5.23 |
| OT-001275 | F736N | Control | 27563 | - |
| | | VDF | 141843 | 5.15 |
| OT-001274 | F736H | Control | 30463 | - |
| | | VDF | 129332 | 4.25 |
| OT-001263 | F736R | Control | 23282 | - |
| | | VDF | 168331 | 7.23 |
| OT-001265 | F736K | Control | 20219 | - |
| | | VDF | 150258 | 7.43 |
| OT-001268 | F736S | Control | 39511 | - |
| | | VDF | 243585 | 6.16 |
| OT-001277 | F736T | Control | 47397 | - |
| | | VDF | 203516 | 4.29 |
| OT-001273 | F736Y | Control | 69893 | - |
| | | VDF | 193231 | 2.76 |
| OT-001260 | F736A | Control | 25583 | - |
| | | VDF | 124553 | 4.87 |
| OT-001259 | F736G | Control | 19279 | - |
| | | VDF | 144347 | 7.49 |
| OT-001261 | F736L | Control | 66857 | - |
| | | VDF | 169009 | 2.53 |
| OT-001271 | F736I | Control | 75543 | - |
| | | VDF | 221732 | 2.94 |
| OT-001270 | F736V | Control | 81977 | - |
| | | VDF | 333677 | 4.07 |
| OT-001262 | F736M | Control | 110217 | - |
| | | VDF | 353885 | 3.21 |

Among the constructs tested, F736P, F736R, F736K, F736A, F736G showed stabilization ratios greater than 7 indicating strong ligand dependent stabilization. These constructs were chosen for further dose response characterization studies. Dose response studies were performed in HCT-1 16 cells with doses ranging from 0.002 to 30 µM for vardenafil, 0.005 to 100 µM for sildenafil and tadalafil. Cells were treated with the indicated dose for 48 hours and the MFI was measured using FACS. The vardenafil, sildenafil and tadalafil dose responses are shown in Table 86, Table 87 and Table 88.

**Table 86:Vardenafil dose response in F736 mutants**

| Construct | Mutation | Vardenafil Dose (µM) | MFI | Stabilization ratio |
|---|---|---|---|---|
| Parental | - | - | 1035 | - |
| OT-001269 | F736P | 30 | 158216 | 21.26 |
| | | 10 | 144184 | 19.37 |
| | | 3.333 | 130695 | 17.56 |
| | | 1.111 | 114355 | 15.36 |
| | | 0.370 | 92834 | 12.47 |
| | | 0.123 | 61401 | 8.25 |
| | | 0.041 | 32907 | 4.42 |
| | | 0.014 | 16591 | 2.23 |
| | | 0.005 | 10071 | 1.35 |
| | | 0.002 | 8253 | 1.11 |
| | | DMSO | 7443 | - |
| OT-001263 | F736R | 30 | 180839 | 8.45 |
| | | 10 | 177263 | 8.28 |
| | | 3.333 | 165159 | 7.72 |
| | | 1.111 | 152487 | 7.12 |
| | | 0.370 | 130186 | 6.08 |
| | | 0.123 | 103375 | 4.83 |
| | | 0.041 | 64283 | 3.00 |
| | | 0.014 | 37755 | 1.76 |
| | | 0.005 | 25227 | 1.18 |
| | | 0.002 | 22913 | 1.07 |
| | | DMSO | 21406 | - |
| OT-001265 | F736K | 30 | 182761 | 9.82 |
| | | 10 | 156978 | 8.43 |
| | | 3.333 | 151181 | 8.12 |
| | | 1.111 | 141783 | 7.62 |
| | | 0.370 | 118662 | 6.38 |
| | | 0.123 | 100311 | 5.39 |
| | | 0.041 | 56035 | 3.01 |
| | | 0.014 | 32977 | 1.77 |
| | | 0.005 | 22525 | 1.21 |
| | | 0.002 | 19109 | 1.03 |
| | | DMSO | 18611 | - |
| OT-001260 | F736A | 30 | 140875 | 5.51 |
| | | 10 | 127144 | 4.97 |
| | | 3.333 | 125782 | 4.92 |
| | | 1.111 | 125078 | 4.89 |
| | | 0.370 | 115016 | 4.50 |
| | | 0.123 | 100026 | 3.91 |
| | | 0.041 | 75486 | 2.95 |
| | | 0.014 | 46252 | 1.81 |
| | | 0.005 | 30287 | 1.18 |
| | | 0.002 | 25820 | 1.01 |
| | | DMSO | 25560 | - |
| OT-001259 | F736G | 30 | 132814 | 6.44 |
| | | 10 | 128225 | 6.22 |
| | | 3.333 | 120003 | 5.82 |
| | | 1.111 | 121525 | 5.89 |
| | | 0.370 | 113357 | 5.50 |
| | | 0.123 | 91136 | 4.42 |
| | | 0.041 | 60953 | 2.96 |
| | | 0.014 | 37052 | 1.80 |
| | | 0.005 | 25469 | 1.24 |
| | | 0.002 | 22445 | 1.09 |
| | | DMSO | 20615 | - |

**Table 87: Sildenafil dose response in F736 mutants**

| Construct | Mutation | Sildenafil Dose (µM) | MFI | Stabilization ratio |
|---|---|---|---|---|
| Parental | | | 1009 | |
| OT-001269 | F736P | 100 | 90704 | 13.86 |
| | | 33.333 | 97811 | 14.95 |
| | | 11.111 | 76720 | 11.73 |
| | | 3.704 | 49658 | 7.59 |
| | | 1.235 | 26727 | 4.08 |
| | | 0.412 | 15073 | 2.30 |
| | | 0.137 | 9548 | 1.46 |
| | | 0.046 | 6977 | 1.07 |
| | | 0.015 | 6836 | 1.04 |
| | | 0.005 | 6718 | 1.03 |
| | | DMSO | 6543 | - |
| OT-001263 | F736R | 100 | 141542 | 6.77 |
| | | 33.333 | 158814 | 7.59 |
| | | 11.111 | 136805 | 6.54 |
| | | 3.704 | 103181 | 4.93 |
| | | 1.235 | 69456 | 3.32 |
| | | 0.412 | 43588 | 2.08 |
| | | 0.137 | 27361 | 1.31 |
| | | 0.046 | 22043 | 1.05 |
| | | 0.015 | 21600 | 1.03 |
| | | 0.005 | 20051 | 0.96 |
| | | DMSO | 20922 | - |
| OT-001265 | F736K | 100 | 117668 | 6.24 |
| | | 33.333 | 132672 | 7.04 |
| | | 11.111 | 111812 | 5.93 |
| | | 3.704 | 89823 | 4.76 |
| | | 1.235 | 61268 | 3.25 |
| | | 0.412 | 37507 | 1.99 |
| | | 0.137 | 25307 | 1.34 |
| | | 0.046 | 19932 | 1.06 |
| | | 0.015 | 18778 | 1.00 |
| | | 0.005 | 19365 | 1.03 |
| | | DMSO | 18856 | - |
| OT-001260 | F736A | 100 | 93652 | 4.12 |
| | | 33.333 | 110048 | 4.84 |
| | | 11.111 | 103954 | 4.57 |
| | | 3.704 | 85884 | 3.78 |
| | | 1.235 | 67277 | 2.96 |
| | | 0.412 | 48774 | 2.15 |
| | | 0.137 | 31578 | 1.39 |
| | | 0.046 | 25710 | 1.13 |
| | | 0.015 | 22537 | 0.99 |
| | | 0.005 | 24395 | 1.07 |
| | | DMSO | 22727 | - |
| OT-001259 | F736G | 100 | 85769 | 5.03 |
| | | 33.333 | 93490 | 5.48 |
| | | 11.111 | 96630 | 5.66 |
| | | 3.704 | 70643 | 4.14 |
| | | 1.235 | 49434 | 2.90 |
| | | 0.412 | 31138 | 1.83 |
| | | 0.137 | 22228 | 1.30 |
| | | 0.046 | 17226 | 1.01 |
| | | 0.015 | 17591 | 1.03 |
| | | 0.005 | 17329 | 1.02 |
| | | DMSO | 17059 | - |

**Table 88:Tadalafil dose response in F736 mutants**

| Construct | Mutation | Tadalafil Dose (µM) | MFI | Stabilization ratio |
|---|---|---|---|---|
| Parental | | | 1111 | - |
| OT-001269 | F736P | 100 | 130984 | 19.71 |
| | | 33.333 | 108698 | 16.36 |
| | | 11.111 | 87589 | 13.18 |
| | | 3.704 | 53208 | 8.01 |
| | | 1.235 | 26467 | 3.98 |
| | | 0.412 | 14220 | 2.14 |
| | | 0.137 | 9298 | 1.40 |
| | | 0.046 | 7795 | 1.17 |
| | | 0.015 | 7378 | 1.11 |
| | | 0.005 | 6662 | 1.00 |
| | | DMSO | 6646 | - |
| OT-001263 | F736R | 100 | 175124 | 8.27 |
| | | 33.333 | 149164 | 7.05 |
| | | 11.111 | 127766 | 6.03 |
| | | 3.704 | 97047 | 4.58 |
| | | 1.235 | 56547 | 2.67 |
| | | 0.412 | 34314 | 1.62 |
| | | 0.137 | 25603 | 1.21 |
| | | 0.046 | 21049 | 0.99 |
| | | 0.015 | 20533 | 0.97 |
| | | 0.005 | 21220 | 1.00 |
| | | DMSO | 21172 | - |
| OT-001265 | F736K | 100 | 162392 | 8.30 |
| | | 33.333 | 151730 | 7.76 |
| | | 11.111 | 134986 | 6.90 |
| | | 3.704 | 102372 | 5.23 |
| | | 1.235 | 63261 | 3.23 |
| | | 0.412 | 36476 | 1.87 |
| | | 0.137 | 26447 | 1.35 |
| | | 0.046 | 21404 | 1.09 |
| | | 0.015 | 20043 | 1.02 |
| | | 0.005 | 19212 | 0.98 |
| | | DMSO | 19558 | - |
| OT-001260 | F736A | 100 | 131921 | 5.29 |
| | | 33.333 | 120132 | 4.82 |
| | | 11.111 | 116598 | 4.67 |
| | | 3.704 | 108477 | 4.35 |
| | | 1.235 | 76753 | 3.08 |
| | | 0.412 | 53672 | 2.15 |
| | | 0.137 | 34408 | 1.38 |
| | | 0.046 | 25447 | 1.02 |
| | | 0.015 | 24416 | 0.98 |
| | | 0.005 | 24661 | 0.99 |
| | | DMSO | 24943 | - |
| OT-001259 | F736G | 100 | 136173 | 6.85 |
| | | 33.333 | 120303 | 6.05 |
| | | 11.111 | 109416 | 5.50 |
| | | 3.704 | 89786 | 4.51 |
| | | 1.235 | 60600 | 3.05 |
| | | 0.412 | 36777 | 1.85 |
| | | 0.137 | 25357 | 1.27 |
| | | 0.046 | 21222 | 1.07 |
| | | 0.015 | 19748 | 0.99 |
| | | 0.005 | 19490 | 0.98 |
| | | DMSO | 19890 | - |

Dose dependent increase in GFP expression was observed with all constructs tested and upon treatment with all three ligands. However, OT-001269, OT-001263, and OT-001265 showed stronger ligand dependent stabilization when compared to the other two constructs. EC50 values for each of the constructs is shown in Table 89.

**Table 89:EC50 (µM) of F736 mutants**

| **Ligand** | **OT-001269** | **OT-001263** | **OT-001265** | **OT-001260** | **OT-001259** |
|---|---|---|---|---|---|
| Sildenafil | 3.844 | 2.066 | 1.884 | 0.9580 | 1.553 |
| Vardenafil | 0.2724 | 0.1202 | 0.1264 | 0.04424 | 0.06552 |
| Tadalafil | 7.503 | 4.825 | 2.853 | 1.072 | 2.519 |

### Example 22. hPDE5 (R732, F736) selective library screen

Mutant library wherein both the amino acid at the position 732 (R732) as well as at position 736 (F736) were mutated to any of the known amino acids, including, but not limited to, aspartic acid, glutamic acid, glutamine, asparagine, histidine, serine, threonine, tyrosine, cysteine, methionine, tryptophan, alanine, isoleucine, leucine, phenylalanine, valine, proline, and glycine were generated. The library of mutations was generated by mutagenesis and the library was fused to a reporter protein e.g. AcGFP via a linker and transduced into NIH3T3 cells. The library expressing cells were selected using the selectable marker, mCherry. The ligand response of the initial library was characterized using different doses (3 or 10 µM) vardenafil or tadalafil. In general, treatment with ligand shifted the GFP expression of the library expressing cells to the right.

A GFP "low" sort was performed where the bottom 5% of the population was sorted out to keep only the lowest basal expressing clones. The library expressing cells were treated with 3 µM Tadalafil for 24 hours and the top 10% and 10-25% library expressing cells were sorted out. The two groups i.e. 10% and 10-25% were characterized using 3µM Tadalafil. Following characterization, the genomic DNA was extracted from the top 10% library expressing cells' group and inserted into lentiviral vectors by Gibson assembly. Clones were prepared from the library and sequenced. A little under a half of the clones sequenced showed mutations in both R732 and F736. Mutants occurring with a frequency of greater than 5 in the library are described herein. The nature of the R732 mutation and the frequency of occurrence of the mutation in parenthesis are as follows: R732D (24), R732E (12), R732G (8), R732L (11), R732P (10), R732V (15), R732W (17). The nature of F736 mutation and the frequency of occurrence of the mutation in parenthesis are as follows: F736D (12), F736F (6), F736G (22), F736L (6), F736S (23), F736V (12). The frequency of the combination of R732 and F736 mutants is described in Table 90.

**Table 90:EC50 (µM) of R732, F736 mutants**

| **Mutation Combination (with respect to SEQ ID NO:1)** | **Frequency** |
|---|---|
| R732D, F736S | 22 |
| R732E, F736D | 10 |
| R732V, F736G | 9 |
| R732W, F736G | 7 |
| R732W, F736V | 5 |
| R732L, F736W | 4 |
| R732P, F736Q | 4 |
| R732W, F736L | 3 |
| R732G, F736G | 2 |
| R732L, F736F | 2 |
| R732L, F736V | 2 |
| R732L, F736Y | 2 |
| R732P, F736D | 2 |
| R732T, F736P | 2 |
| R732V, F736F | 2 |
| R732Y, F736M | 2 |
| R732A, F736A | 1 |
| R732C, F736V | 1 |
| R732D,F736L | 1 |
| R732D, F736T | 1 |
| R732E, F736C | 1 |
| R732E, F736E | 1 |
| R732F, F736C | 1 |
| R732F, F736V | 1 |
| R732G, F736L | 1 |
| R732G, F736P | 1 |
| R732G, F736R | 1 |
| R732G, F736V | 1 |
| R732I, F736G | 1 |
| R732I, F736V | 1 |
| R732L, F736S | 1 |
| R732M, F736G | 1 |
| R732M, F736H | 1 |
| R732M, F736R | 1 |
| R732P, F736C | 1 |
| R732P, F736F | 1 |
| R732P, F736G | 1 |
| R732P, F736I | 1 |
| R732S, F736G | 1 |
| R732V, F736E | 1 |
| R732V, F736I | 1 |
| R732V, F736R | 1 |
| R732V, F736V | 1 |
| R732W, F736A | 1 |
| R732W, F736F | 1 |
| R732Y, F736L | 1 |

Based on this screen, constructs described in Table 12 were prepared for further characterization.

### Example 23. PDE5 mutant saturation library

Mutant library wherein every amino acid within position 535 to 860 of SEQ ID NO: 1 was mutated were generated. The library of mutations was fused to a reporter protein e.g. AcGFP via a linker and transduced into Jurkat cells. The library expressing cells were selected using the selectable marker, mCherry. The ligand response of the initial library was characterized using different doses (3 or 10 µM) vardenafil or tadalafil. In general, treatment with ligand shifted the GFP expression of the library expressing cells to the right.

A GFP "low" sort was performed where the bottom 5% and 5-15 % of the population was sorted out to keep only the lowest basal expressing clones. The library expressing cells (both 5 and 5-15%) were treated with 10 µM Tadalafil for 24 hours and the top 30% library expressing cells were sorted out and characterized using 10µM Tadalafil. Following characterization, the genomic DNA was extracted from the top 10% library expressing cells' group and inserted into vector. Clones were prepared from the library and sequenced. The nature of the mutation and the frequency of occurrence of the mutation in parenthesis are shown in Table 91. In the table * indicates the translation of the stop codon and X indicates any amino acid.

**Table 91: Frequency of occurrence within saturation library**

| **Mutant** | **Frequency of occurrence** |
|---|---|
| C574N | 6 |
| E536K, I739W | 6 |
| R732V, L756H | 5 |
| H653N | 4 |
| H678F, S702F | 4 |
| R732T, R740Y | 4 |
| A722E | 3 |
| E536N, E708K, E734K, M758I | 3 |
| F735N | 3 |
| F736P | 3 |
| G697F | 3 |
| I576K, M625Y | 3 |
| Q541P, H678L | 3 |
| R732P | 3 |
| T712N | 3 |
| V602S, F736M | 3 |
| A645I, E707K | 2 |
| D764N | 2 |
| D822V | 2 |
| F588N | 2 |
| F619H | 2 |
| F735P | 2 |
| H617K | 2 |
| I739F, R850F | 2 |
| K604F, R732L | 2 |
| L600K, K608I | 2 |
| N583L, F736S | 2 |
| Q635H, R732S,I739M, R740F | 2 |
| R732W, E800K | 2 |
| A628T, A722T, E734L | 1 |
| C574N, L804I | 1 |
| C574N, R732T, R740Y | 1 |
| C846N | 1 |
| E536V, R732T | 1 |
| E539G, R732L | 1 |
| F561I | 1 |
| F579L, C846N | 1 |
| F588K | 1 |
| F619Y | 1 |
| F735P, Q854K | 1 |
| F735Q, K848N | 1 |
| F735R | 1 |
| F736R, F744R | 1 |
| F736S, F744S | 1 |
| F736S, N849S | 1 |
| F744A, R850Y | 1 |
| H617S, I680F | 1 |
| H678I, S702F | 1 |
| H678Y, F736S | 1 |
| H834R, V835G | 1 |
| I73 9W | 1 |
| K603F, R732L | 1 |
| K608S, P771F | 1 |
| K730R, R731G, R732E, G733E, E734N | 1 |
| K741P, R850K | 1 |
| K741T, P749*, L765F | 1 |
| K752N, E753X | 1 |
| L554I, P771F | 1 |
| L554I, R732L, E785G | 1 |
| L582S | 1 |
| L651V, V660X, D791X | 1 |
| L675H, E708K, N742S | 1 |
| N698F, K741I | 1 |
| P549F | 1 |
| P771L | 1 |
| Q718K, E734W | 1 |
| R577Y | 1 |
| R597G, R732W | 1 |
| R732I, R740I | 1 |
| R732L, D788Y | 1 |
| R732V | 1 |
| R740K, P771X | 1 |
| R850N | 1 |
| T621F | 1 |
| V602N, M758I | 1 |

Based on the frequency of occurrence of the mutations, 3 clones were selected for further characterization and inserted into viral vectors namely the C574N bearing (OT-001869), E536K and I739W bearing clone OT-001868 and H678F, and S702F bearing clone OT-001870.

### Example 24. PDE5 Cytosolic Screen

The position of hPDE5 in a construct comprising hPDE5 fused to GFP may affect the resultant library of mutants generated by mutagenesis screening. The effect of positioning the DD at the c terminal construct on the identification of DD clones was tested. PDE5 library generated by random mutagenesis fused GFP to generate AcGFP-PDE5-IRES-Puro constructs. The library plasmid was packaged in lentivirus and used to infect 3T3 at a MOI=0.3. The cells were selected with puromycin (2µg/mL) for 2 weeks. The bottom 10% of cells expressing the lowest GFP in the absence of ligand were collected from the initial sort. In the subsequent sort, 5% of cells expressing the highest GFP levels 48 hours after incubation with 1µM vardenafil or 10µM sildenafil were collected. This was followed by the selection of the 5% of cells expressing the lowest GFP level after the removal of ligand. Approximately 50 clones from vardenafil and sildenafil treated libraries were sequenced. The top 10 clones were selected for characterization based on frequency of mutations. Table 92 shows the mutation position, and its frequency.

**Table 92: Mutation frequency**

| **Clone** | **Point mutation number** | **Frequency** | **AA position** | **Ligand used in screen** |
|---|---|---|---|---|
| OT-001944 | 1 | 8 | 228 | Mutations present in clones of both Screens (Vardenafil and Sildenafil) |
| OT-001941 | 1 | 2 | 102 | |
| OT-001947 | 2 | 4 | 135 | |
| | | 8 | 166 | |
| OT-001946 | 4 | 14 | 126 | |
| | | 14 | 247 | |
| | | 15 | 260 | |
| | | 15 | 291 | |
| OT-001943 | 1 | 6 | 106 | |
| OT-001939 | 3 | 4 | 107 | |
| | | 4 | 163 | |
| | | 4 | 279 | |
| OT-001945 | 1 | 1 (STOP) | 241 | Vardenafil |
| OT-001942 | 3 | 2 | 89 | |
| | | 2 | 120 | |
| | | 3 | 207 | |
| OT-001940 | 1 | 4 | 222 | Sildenafil |
| OT-001948 | 2 | 1 | 98 | |
| | | 5 | 114 | |

Stable cell lines were made with lentivirus transduction of the constructs into 3T3 cells. Cells were treated with ligand (0.1 or 1 µM Vardenafil) for 48 hours, then analyzed by flow cytometry. Ligand dependent and dose dependent regulation was observed with OT-001939, OT-001948, OT-001947 and to a modest extent with OT-001944. Clones OT-001940, OT-001946, OT-001945, OT-001943, and OT-001942did not show an increase in GFP expression in response to ligand. The median fluorescence intensity (MFI) for mutants OT-001948 and OT-001947is shown in Table 93. To calculate the stabilization ratio, the MFI of the parental cells was subtracted from the untreated control.

**Table 93: Mutant MFI**

| **Ligand** | **Description** | **Parental** | **OT-001232 (Wild Type)** | **OT-001226** | **OT-001948** | **OT-001947** |
|---|---|---|---|---|---|---|
| No Vardenafil | MFI | 103 | 15600 | 1530 | 478 | 308 |
| Vardenafil 0.1 µM | MFI | - | 16200 | 11700 | 1363 | 823 |
| | Stabilization ratio | - | 1.04 | 8.1 | 3.4 | 3.5 |
| Vardenafil 1 µM | MFI | - | 16200 | 13800 | 4478 | 2881 |
| | Stabilization ratio | - | 1.04 | 9.6 | 11.7 | 13.6 |

As shown in Table 93, both clones showed ligand dependent stabilization at both 0.1 and 1µM vardenafil. OT-001948 and OT-001947clones were characterized by western blot using all three ligands. Cells were treated with Vardenafil (0.3 and 10 µM), sildenafil and tadalafil (3 and 100 µM) for 24 hours and immunoblotted with anti-GFP antibody. Both OT-001948 and OT-001947 showed ligand dose dependent expression with all three ligands. Samples were also immunoblotted with beta actin and showed even protein loading.

OT-001948 and OT-001947were treated with varying concentration of ligand for 48 hours and then analyzed by flow cytometry (see Table 94, Table 95, Table 96).

**Table 94:Tadalafil dose response**

| **+Tadalafil (µM)** | **OT-001232 (Wild Type)** | **OT-001226** | OT-001947 | OT-001947 |
|---|---|---|---|---|
| 0 | 3.05E+04 | 2627 | 838 | 1244 |
| 0.001 | 3.10E+04 | 2499 | 807 | 1201 |
| 0.003 | 3.09E+04 | 2753 | 783 | 1168 |
| 0.01 | 3.04E+04 | 3.01E+03 | 870 | 1168 |
| 0.03 | 3.12E+04 | 3.42E+03 | 867 | 1231 |
| 0.1 | 3.37E+04 | 7.53E+03 | 900 | 1259 |
| 0.3 | 3.55E+04 | 1.50E+04 | 1194 | 1597 |
| 1 | 3.71E+04 | 2.26E+04 | 1812 | 2197 |
| 3 | 3.87E+04 | 2.62E+04 | 3237 | 3476 |
| 10 | 3.64E+04 | 2.73E+04 | 6499 | 6419 |

**Table 95:Vardenafil dose response**

| **Vardenafil (µM)** | **OT-001232 (Wild Type)** | **OT-001226** | **OT-001947** | OT-001947 |
|---|---|---|---|---|
| 0 | 3.09E+04 | 2345 | 889 | 1321 |
| 0.001 | 3.15E+04 | 3453 | 983 | 1362 |
| 0.003 | 3.27E+04 | 5207 | 1079 | 1407 |
| 0.01 | 3.22E+04 | 1.14E+04 | 1198 | 1708 |
| 0.03 | 3.18E+04 | 1.92E+04 | 1711 | 2365 |
| 0.1 | 3.13E+04 | 2.40E+04 | 2831 | 3892 |
| 0.3 | 3.01E+04 | 2.61E+04 | 4935 | 6919 |
| 1 | 2.93E+04 | 2.64E+04 | 8232 | 1.06E+04 |
| 3 | 3.00E+04 | 2.72E+04 | 1.04E+04 | 1.44E+04 |
| 10 | 3.18E+04 | 2.73E+04 | 1.21E+04 | 1.57E+04 |

**Table 96:Sildenafil dose response**

| **Sildenafil (µM)** | **OT-001232 (Wild Type)** | **OT-001226** | OT-001947 | OT-001947 |
|---|---|---|---|---|
| 0 | 3.09E+04 | 2472 | 762 | 1114 |
| 0.001 | 3.04E+04 | 2401 | 729 | 1089 |
| 0.003 | 2.70E+04 | 2554 | 739 | 1099 |
| 0.01 | 2.96E+04 | 2848 | 740 | 1086 |
| 0.03 | 3.07E+04 | 4053 | 766 | 1136 |
| 0.1 | 3.26E+04 | 8260 | 860 | 1285 |
| 0.3 | 3.27E+04 | 1.54E+04 | 1074 | 1630 |
| 1 | 3.44E+04 | 2.12E+04 | 1744 | 2610 |
| 3 | 3.37E+04 | 2.27E+04 | 2777 | 3888 |
| 10 | 3.43E+04 | 2.34E+04 | 5259 | 6788 |

Ligand dependent regulation was observed with both OT-001947, and OT-001948 clones. The lowest dose of ligand required to achieve detectable expression was higher for clones when compared to OT-001226. The GFP expression in the absence of any of the three ligands tested, was very low indicating low basal expression.

### Example 25. PDE5 regulated CARs

Select PDE5 DDs generated by mutagenesis were cloned into construct OT-001300 and responsiveness to PDE5 ligands was tested. The clones selected for characterization are shown in Table 97.

**Table 97:PDE5 clone mutation frequency**

| **Construct** | **Number of mutations** | **Mutation Frequency** | **AA position** |
|---|---|---|---|
| OT-001922 | 5 | 4 | 251 |
| OT-001910 | 5 | 3 | 230 |
| OT-001926 | 4 | 3 | 229 |
| OT-001929 | 2 | 3 | 214 |
| OT-001924 | 3 | 4 | 175 |
| OT-001925 | 3 | 4/8 | 49/218 |
| OT-001921 | 2 | 5 | 286 |
| OT-001913 | 2 | 4 | 190 |
| OT-001911 | 1 | 8 | 218 |
| OT-001912 | 1 | 4 | 224 |
| OT-001936 | 2 | 2/2 | 194/283 |
| OT-001935 | 1 | 3 | 57 |
| OT-001914 | 1 | 4 | 5 |
| OT-001937 | 4 | 3 | 116 |
| OT-001917 | 1 | 3 | 230 |
| OT-001909 | 3 | 2 | 43 |
| OT-001938 | 2 | 3 | 207 |
| OT-001915 | 2 | 2/2 | 138/302 |
| OT-001916 | 2 | 2 | 101 |
| OT-001934 | 3 | 3 | 25 |

CARs appended to PDE5 clones were tested in 3T3 cells. Ligands were tested at the doses indicated for 48 hours in Table 98. CAR expression was measured using FACS performed with CD19-Fc staining or HA staining. In Table 98, MFI indicates Median Fluorescence Intensity and SR indicates stabilization ratio. Dose responses studies were also performed with vardenafil and tadalafil (Table 99 and Table 100).

**Table 98: Ligand dependent CAR expression**

| Construct | Untreated | Vardenafil (1µM) | | Sildenafil (10µM) | | Tadalafil (10µM) | |
|---|---|---|---|---|---|---|---|
| | MFI | MFI | SR | MFI | SR | MFI | SR |
| OT-001913 | 700 | 1700 | 2.5 | 1300 | 2 | - | - |
| OT-001912 | 600 | 2600 | 4 | 2000 | 3 | - | - |
| OT-001911 | 600 | 2100 | 3.5 | 2100 | 3.5 | - | - |
| OT-001910 | 180 | 2000 | 11 | 1750 | 10 | - | - |
| OT-001918 | 80 | 341 | 4.3 | 204 | 2.6 | 199 | 2.5 |
| OT-001920 | 186 | 698 | 3.8 | 701 | 3.8 | 704 | 3.8 |
| OT-001930 | 58.2 | 51.3 | 0.9 | 65 | 1.1 | 64.2 | 1.1 |
| OT-001933 | 234 | 522 | 2.2 | 428 | 1.8 | 525 | 2.2 |
| OT-001927 | 53.7 | 78 | 1.5 | 65.8 | 1.2 | 10.7 | 0.2 |
| OT-001919 | 225 | 774 | 3.4 | 693 | 3.1 | 645 | 2.9 |
| OT-001928 | 60 | 216 | 3.6 | 160 | 2.7 | 110 | 1.8 |
| OT-001914 | 365 | 1279 | 3.5 | 1260 | 3.5 | 1180 | 3.2 |
| OT-001916 | 312 | 911 | 2.9 | 757 | 2.4 | 487 | 1.6 |
| OT-001915 | 198 | 1156 | 5.8 | 933 | 4.7 | 490 | 2.5 |
| OT-001917 | 249 | 1062 | 4.3 | 1252 | 5 | 823 | 3.3 |

**Table 99:Tadalafil dependent CAR expression**

| **Across: Tadalafil (µM)** | | **Parental** | **0** | **0.3** | **1** | **3** | **10** | **30** |
|---|---|---|---|---|---|---|---|---|
| OT-001920 (Sample A) | MFI | 8.3 | 218.0 | 266.0 | 330.0 | 435.0 | 580.0 | 688.0 |
| | SR | - | - | 1.2 | 1.5 | 2.0 | 2.7 | 3.2 |
| OT-001920 (Sample B) | MFI | 7.2 | 527.0 | 256.0 | 714.0 | 519.0 | 744.0 | 1142.0 |
| | SR | - | - | 0.5 | 1.4 | 1.0 | 1.4 | 2.2 |
| OT-001920 (Sample C) | MFI | 8.3 | 218.0 | 266.0 | 330.0 | 435.0 | 580.0 | 688.0 |
| | SR | - | - | 1.2 | 1.5 | 2.0 | 2.7 | 3.2 |
| OT-001919 | MFI | 8.3 | 93.4 | 108.0 | 158.0 | 236.0 | 335.0 | 370.0 |
| | SR | - | - | 1.2 | 1.7 | 2.5 | 3.6 | 4.0 |
| OT-001911 (EC50: 4.6 µM) | MFI | 12.0 | 327.0 | 371.0 | 469.0 | 725.0 | 1119.0 | 1311.0 |
| | SR | - | - | 1.1 | 1.4 | 2.2 | 3.4 | 4.0 |
| OT-001912 (EC50: 800 nM) | MFI | 11.3 | 267.0 | 707.0 | 1044.0 | 1511.0 | 1611.0 | 1724.0 |
| | SR | - | - | 2.6 | 3.9 | 5.7 | 6.0 | 6.5 |
| OT-001919 (EC50: 5.82 µM) | MFI | 7.2 | 143.0 | 213.0 | 370.0 | 261.0 | 456.0 | 448.0 |
| | SR | - | - | 1.5 | 2.6 | 1.8 | 3.2 | 3.1 |
| OT-001918 | MFI | 7.2 | 32.1 | 74.5 | 87.9 | 125.0 | 186.0 | 280.0 |
| | SR | - | - | 2.3 | 2.7 | 3.9 | 5.8 | 8.7 |
| OT-001919 | MFI | 8.3 | 93.4 | 108.0 | 158.0 | 236.0 | 335.0 | 370.0 |
| | SR | - | - | 1.2 | 1.7 | 2.5 | 3.6 | 4.0 |
| OT-001913 | MFI | 11.3 | 110.0 | 141.0 | 159.0 | 193.0 | 232.0 | 369.0 |
| | SR | - | - | 1.3 | 1.4 | 1.8 | 2.1 | 3.4 |
| OT-001914 | MFI | - | 215.0 | 255.0 | 383.0 | 511.0 | 1030.0 | 1158.0 |
| | SR | - | - | 1.2 | 1.8 | 2.4 | 4.8 | 5.4 |
| OT-001916 | MFI | - | 267.0 | 325.0 | 398.0 | 409.0 | 758.0 | 852.0 |
| | SR | - | - | 1.2 | 1.5 | 1.5 | 2.8 | 3.2 |
| OT-001915 | MFI | - | 132.0 | 95.4 | 102.0 | 150.0 | 253.0 | 348.0 |
| | SR | - | - | 0.7 | 0.8 | 1.1 | 1.9 | 2.6 |
| OT-001917 | MFI | - | 73.9 | 302.0 | 427.0 | 748.0 | 1239.0 | 1141.0 |
| | SR | - | - | 4.1 | 5.8 | 10.1 | 16.8 | 15.4 |

**Table 100: Vardenafil dependent CAR expression**

| **Across: Vardenafil (µM)** | | **Parental** | **0** | **0.03** | **0.1** | **0.3** | **1** | **10** |
|---|---|---|---|---|---|---|---|---|
| OT-001920 (Sample A; EC50: 170 nM) | MFI | 12 | 61 | 204 | 343 | 680 | 936 | 908 |
| | SR | - | - | 3.34 | 5.62 | 11.15 | 15.34 | 14.89 |
| OT-001910 (EC50: 1.6 µM) | MFI | 12 | 61 | 223 | 336 | 764 | 948 | 916 |
| | SR | - | | 3.66 | 5.51 | 12.52 | 15.54 | 15.02 |
| OT-001911 (EC50: 200 nM) | MFI | 12 | 327 | 430 | 647 | 1037 | 1451 | 1442 |
| | SR | - | - | 1.31 | 1.98 | 3.17 | 4.44 | 4.41 |
| OT-001912 (Sample A) | MFI | 12 | 267 | 539 | 653 | 969 | 1299 | 822 |
| | SR | - | - | 2.02 | 2.45 | 3.63 | 4.87 | 3.08 |
| OT-001912 (Sample B) | MFI | 7.24 | 185 | 645 | 775 | 833 | 883 | 878 |
| | SR | - | - | 3.49 | 4.19 | 4.50 | 4.77 | 4.75 |
| OT-001919 (EC50: 56 nM) | MFI | 7.24 | 143 | 271 | 380 | 506 | 544 | 483 |
| | SR | - | - | 1.90 | 2.66 | 3.54 | 3.80 | 3.38 |
| V32 (EC50: 398 nM) | MFI | 7.24 | 32.1 | 91.4 | 116 | 209 | 308 | 401 |
| | SR | - | - | 2.85 | 3.61 | 6.51 | 9.60 | 12.49 |
| OT-001920 (Sample B; EC50: 80 nM) | MFI | 7.24 | 527 | 496 | 881 | 941 | 1185 | 1004 |
| | SR | - | - | 0.94 | 1.67 | 1.79 | 2.25 | 1.91 |
| OT-001913 | MFI | 12 | 108 | 86.5 | 132 | 239 | 331 | 265 |
| | SR | - | - | 0.80 | 1.22 | 2.21 | 3.06 | 2.45 |
| OT-001914 | MFI | - | 215 | 467 | 665 | 1026 | 1234 | 1136 |
| | SR | - | - | 2.17 | 3.09 | 4.77 | 5.74 | 5.28 |
| OT-001916 | MFI | - | 267 | 380 | 382 | 518 | 955 | 1095 |
| | SR | - | - | 1.42 | 1.43 | 1.94 | 3.58 | 4.10 |
| OT-001915 | MFI | - | 132 | 308 | 514 | 719 | 918 | 960 |
| | SR | - | - | 2.33 | 3.89 | 5.45 | 6.95 | 7.27 |
| OT-001917 | MFI | - | 158 | 384 | 624 | 915 | 1123 | 1084 |
| | SR | - | - | 2.43 | 3.95 | 5.79 | 7.11 | 6.86 |

Most of the constructs tested in Table 98 showed ligand dependent stabilization with all three ligands. Exceptions include OT-001930, which did not demonstrate ligand dependent stabilization with any of the ligands tested (Stabilization ratio <1.5) and OT-001927 which only showed regulation in the presence of vardenafil with a stabilization ratio >1.5). All of the clones tested in tested in Table 99 and Table 100 showed ligand dependent regulation. The kinetics of the regulation varied between the clones. While some clones e.g. OT-001917, and OT-001910, showed ligand dependent regulation at lower doses, other clones such as OT-001915 and OT-001916 showed ligand dependent regulation only at higher ligand doses. These results were further confirmed by western blot analysis. Stable 3T3 cell lines PDE5 CAR constructs were treated with 0.3 or 10 µm Vardenafil; or Sildenafil and Tadalafil at 1 or 30 µM for 48 hours and immunoblotted for PDE5 or HA tag. Tested clones included OT-001913, OT-001911, OT-001912, OT-001910, OT-001918, OT-001920, OT-001919, OT-001914, OT-001916, OT-001915, OT-001917. All show clones showed ligand dependent regulation with each of the three ligands.

### Example 26. CAR regulation by hPDE5

To test regulated CAR expression by hPDE5 DDs with Tadalafil *in vitro,* donor T cells were thawed and activated overnight in the presence of CD3/CD28 Dynabeads at a 3:1 bead: cell ratio. The following day, cells were transduced with lentivirus (empty vector), OT-001512, OT-001455, or OT-001456 constructs. Cells were expanded by addition of fresh media over the course of 10 days, maintaining cells around 0.5×10⁶ cells/mL and then they were frozen. As shown in Table 101, CAR expression was confirmed on day 7 of the expansion by culturing with DMSO or 10uM Tadalafil 24 hours prior to FACS staining with 10ug/mL CD19-Fc.

**Table 101: Day 7 Expanded T cells**

| **Construct** | **Day 7** | |
|---|---|---|
| | **DMSO** | **Tadalafil** |
| | **% CAR+ Cells** | **% CAR+ Cells** |
| Empty Vector | 0 | 0 |
| OT-001512 | 48 | 0 |
| OT-001455 | 15 | 53 |
| OT-001456 | 11 | 60 |

Expression was also confirmed in the frozen, expanded T cells by thawing, stimulating with CD3/CD28 Dynabeads (3:1 bead:cell ratio), and treating with DMSO or a dose response of Tadalafil for 24 hours. The results are shown in Table 102.

**Table 102: Tadalafil Titration**

| **Tadalafil (uM)** | **OT-001455** | **OT-001456** |
|---|---|---|
| | **% CAR+ Cells** | **% CAR+ Cells** |
| 0 | 3.1 | 4.2 |
| 0.04 | 16.6 | 5.3 |
| 0.1 | 22.2 | 10.2 |
| 0.4 | 32.9 | 17.6 |
| 1.1 | 33.8 | 35.1 |
| 3.3 | 36.5 | 41.2 |
| 10 | 38.3 | 47.7 |
| 30 | 38.3 | 48.7 |

To confirm that cytotoxicity and cytokine secretion is directly related to CAR expression, transduced T cells were thawed and cocultured with red fluorescent Nalm6 target cells at 10:1, 3:1, and 1:1 effector:target cell ratios for 5 days in the presence or absence of 1uM Tadalafil. The results are shown in Table 103.

**Table 103: Nalm6 Proliferation**

| Construct | 10:1 | | 3:1 | | 1:1 | |
|---|---|---|---|---|---|---|
| | DMSO | Tadalafil | DMSO | Tadalafil | DMSO | Tadalafil |
| Empty Vector | 757794 | 0 | 805634 | 0 | 977464 | 0 |
| OT-001512 | 4485 | 0 | 154256 | 0 | 422304 | 0 |
| OT-001455 | 15351 | 1658 | 185756 | 17319 | 833197 | 265637 |
| OT-001456 | 221126 | 3004 | 468664 | 22179 | 614952 | 402447 |

Coculture supernatants (3:1) were collected after 48 hours and analyzed for IFNg and IL2 levels by MSD. The results are shown in Table 104.

**Table 104: IFNg and IL2 Levels**

| Construct | IFNg | | IL2 | |
|---|---|---|---|---|
| | DMSO | Tadalafil | DMSO | Tadalafil |
| | pg/mL | pg/mL | pg/mL | pg/mL |
| Empty Vector | 8.1 | 0 | 0.4 | 0 |
| OT-001512 | 1023.6 | 0 | 394 | 0 |
| OT-001455 | 161.9 | 1094.5 | 5 | 42.2 |
| OT-001456 | 66.8 | 762.2 | 3 | 110.2 |

As shown in Table 105, Tadalafil regulated CD19 chimeric antigen receptor (OT-001456) controls CD19+ Nalm6-luc tumors in NSG mice.

**Table 105: Tadalafil regulated CD19 chimeric antigen receptor (OT-001456)**

| **Tadalafil (uM)** | **% CAR+** | **Nalm6 cell count** | **IFNg (pg/mL)** | **IL2 (pg/mL)** |
|---|---|---|---|---|
| 10 | 26.7 | 74442 | 1048 | 447 |
| 3.333 | 22.3 | 19624 | 1616 | 394 |
| 1.111 | 13.4 | 37555 | 692 | 298 |
| 0.37 | 6.25 | 72377 | 315 | 105 |
| 0.123 | 3.35 | 1145929 | 20 | 7 |
| 0.041 | 2.92 | 1117625 | 12 | 4 |
| 0.013 | 2.21 | 1150899 | 11 | 2 |
| 0.004 | 1.83 | 1212262 | 8 | 2 |

### Example 27: hPDE5 regulation in HCT116 cells

To test regulated expression in HCT116 cells by PDE5 DDs, HCT116 cells were transduced with hPDE5-GFP constructs (OT-001211, OT-001212, OT-001213, and OT-001214) and incubated with the indicated concentrations of ligand for 48 hours. GFP fluorescence was measured by FACS. The normalized GFP MFI is shown in Table 106, Table 107 and Table 108 for vardenafil, sildenafil and tadalafil respectively.

**Table 106: Vardenafil dose response**

| **Vardenafil (uM)** | **OT-001211** | **OT-001212** | **OT-001213** | **OT-001214** |
|---|---|---|---|---|
| 10000 | 107.2 | 103.2 | 103.8 | 101.8 |
| 3333.3 | 101.8 | 101.3 | 91.6 | 99 |
| 1111.1 | 100.5 | 94.9 | 67.4 | 80.1 |
| 370.4 | 102 | 73.5 | 43.9 | 56.6 |
| 123.5 | 87.3 | 48.6 | 14.4 | 33.6 |
| 41.2 | 82.4 | 14.7 | 2.8 | 13.4 |
| 13.7 | 40.6 | 2.5 | 0.5 | 3.7 |
| 4.6 | 14.9 | 0.2 | 0.2 | 1.7 |
| 1.5 | 3.5 | 0.1 | 0.2 | 0.6 |
| 0.5 | 1.3 | 0 | 0.3 | 0.1 |

**Table 107: Sildenafil dose response**

| **Sildenafil (uM)** | **OT-001211** | **OT-001212** | **OT-001213** | **OT-001214** |
|---|---|---|---|---|
| 10000 | 106.7 | 73.9 | 46.8 | 45.5 |
| 3333.3 | 95.3 | 42.8 | 17.7 | 21.3 |
| 1111.1 | 77.5 | 10.5 | 2.9 | 5.9 |
| 370.4 | 65.4 | 2.2 | 0.7 | 1.7 |
| 123.5 | 29.9 | 0.1 | 0 | 0.7 |
| 41.2 | 7.6 | 0.1 | 0.1 | 0.3 |
| 13.7 | 2.1 | -0.1 | 0 | 0.5 |
| 4.6 | 1.2 | 0 | 0 | 0.1 |
| 1.5 | 0.4 | 2.6 | 0 | 0.3 |
| 0.5 | 0.2 | 0 | -0.2 | 0.2 |

**Table 108: Tadalafil dose response**

| **Sildenafil (uM)** | **OT-001211** | **OT-001212** | **OT-001213** | **OT-001214** |
|---|---|---|---|---|
| 10000 | 106.7 | 88.7 | 42.2 | 42 |
| 3333.3 | 98.3 | 71.4 | 12.1 | 18.9 |
| 1111.1 | 83.7 | 35.1 | 3 | 4.7 |
| 370.4 | 57.4 | 8.6 | 0.6 | 1.3 |
| 123.5 | 29.3 | 1.9 | 0.3 | 0.5 |
| 41.2 | 8.8 | 0.5 | 0.1 | 0 |
| 13.7 | 1.8 | -0.2 | 0.4 | -0.2 |
| 4.6 | 0 | -0.2 | 0 | -0.1 |
| 1.5 | -0.3 | 0 | 0 | 0.2 |
| 0.5 | 0 | 0 | 0.3 | 0.3 |

As shown in Table 106, Table 107 and Table 108, regulation with hPDE5 DDs was observed with OT-001211, OT-001212, OT-001213, and OT-001214 in a dose dependent manner.

### Example 28: hPDE5 regulation in Human T cells

To test regulated CD19CAR protein expression in human T cells by PDE5 DDs, donor T cells were thawed and activated overnight in the presence of CD3/CD28 Dynabeads at a 3:1 bead: cell ratio. The following day, cells were transduced with hPDE5-CD19CAR constructs (OT-001456, OT-001455, OT-001457, or OT-001454) or empty vector at a virus volume of 10 uL. Cells were expanded by addition of fresh media and then they were frozen. The percent of cells expressing CAR after treatment with vardenafil, sildenafil and tadalafil are shown below. The percentage for the empty vector for each ligand dose was between 0.2 and 0.4%.

**Table 109: Vardenafil dose response**

| **Vardenafil (uM)** | **OT-001456** | **OT-001455** | **OT-001457** | **OT-001454** |
|---|---|---|---|---|
| 10000 | 37 | 44.7 | 40.2 | 53.4 |
| 3333.3 | 34.8 | 41.2 | 37.2 | 52.9 |
| 1111.1 | 35.2 | 33.7 | 27.2 | 52.9 |
| 370.4 | 29.3 | 24 | 14.1 | 52.5 |
| 123.5 | 18.6 | 16.9 | 7.8 | 50 |
| 41.2 | 9.4 | 14.5 | 5.5 | 44.9 |
| 13.7 | 5.6 | 13.3 | 3.9 | 40 |
| 4.6 | 4.4 | 12.8 | 3.8 | 33.5 |
| 1.5 | 3.7 | 11.7 | 3.5 | 30.5 |
| 0.5 | 4 | 11.1 | 3.2 | 30.2 |
| 0.2 | 3.1 | 10.7 | 3.1 | 29.2 |

**Table 110: Sildenafil dose response**

| **Sildenafil (uM)** | **OT-001456** | **OT-001455** | **OT-001457** | **OT-001454** |
|---|---|---|---|---|
| 10000 | 21.3 | 45.3 | 6.9 | 49.9 |
| 3333.3 | 10 | 45 | 4.5 | 45.7 |
| 1111.1 | 6.2 | 45.5 | 3.8 | 40.1 |
| 370.4 | 5 | 44.6 | 3.5 | 34.1 |
| 123.5 | 4.1 | 44.1 | 3.9 | 31 |
| 41.2 | 3.7 | 40.9 | 3.8 | 29.4 |
| 13.7 | 3.7 | 33.6 | 4 | 28.9 |
| 4.6 | 3.2 | 23.4 | 3 | 28.1 |
| 1.5 | 3.2 | 17 | 3.2 | 28 |
| 0.5 | 3.5 | 14.1 | 3.5 | 28.5 |
| 0.2 | 2.8 | 12.5 | 3.1 | 28.2 |

**Table 111: Tadalafil dose response**

| **Tadalafil (uM)** | **OT-001456** | **OT-001455** | **OT-001457** | **OT-001454** |
|---|---|---|---|---|
| 10000 | 35.6 | 48.6 | 16.1 | 51.4 |
| 3333.3 | 30.4 | 48.5 | 7.2 | 50 |
| 1111.1 | 19 | 44.7 | 4.9 | 44.8 |
| 370.4 | 9.4 | 39.9 | 3.4 | 40.3 |
| 123.5 | 5.9 | 28.8 | 3.7 | 33 |
| 41.2 | 4.4 | 19 | 3.7 | 28.7 |
| 13.7 | 4 | 15.7 | 3.6 | 29.2 |
| 4.6 | 3.5 | 12.7 | 3.7 | 27.7 |
| 1.5 | 3.4 | 11.8 | 3.6 | 28.2 |
| 0.5 | 3.7 | 11.5 | 3 | 27.6 |
| 0.2 | 3.1 | 10.6 | 3.1 | 27.1 |

As shown in Table 109, Table 110 and Table 111, regulation with hPDE5 DDs was observed with OT-001456, OT-001455, OT-001457, and OT-001454 in a dose dependent manner.

### Example 29: Ligand exposure schemes

Effect of pre-freezing treatment of T cells with tadalafil on expression of CD19-CAR was tested. T cells were thawed and activated overnight in the presence of CD3/CD28 Dynabeads at a 3:1 bead:cell ratio. The following day, cells were transduced with OT-001456. Cells were expanded by addition of fresh media over the course of 10 days, maintaining cells around 0.5×10^6 cells/mL. On day 9 of the expansion, cells were treated with 10uM tadalafil overnight, then frozen. Upon thawing, cells treated with tadalafil prior to freezing had similar CAR expression (37%) to cells that were thawed and treated overnight with 10uM tadalafil (35%), indicating that CAR expression was maintained during the freezing process.

A Nalm6-luciferase tumor model in female NSG mice was used to determine if various ligand exposure schemes lead to enhanced efficacy *in vivo.* The experimental setup was as described in Example 20, Table 82. One week after implantation of Nalm6-luciferase tumors, mice were randomized and injected at indicated cell doses with freshly thawed CD19-CAR T cells that had previously been transduced with OT-001455 or OT-001456 and expanded in vitro for 10 days. Groups 8 and 9 CAR T cells had been treated with 10uM tadalafil overnight prior to cells being frozen so they would express CAR at time of injection. Mice received either tadalafil or vehicle, beginning 12 hours prior to CD19-CAR T injection, then once or twice a day, as indicated, at the listed concentrations. Body weights and tumor BLI measurements were recorded over time, with higher BLI measures indicating high Nalm6-luciferase tumor burden.

As shown in Figure 10, a high initial dose of tadalafil with later shift to maintenance dosing does not improve efficacy of PDE5-regulated CD19 CAR-mediated cytotoxicity

The effect of pre-exposing cells to tadalafil ligand prior to infusing them was also tested. As shown in Figure 11, ligand pre-treatment prior to freezing CAR T cells increases basal CAR activity in vehicle treated animals but does not improve efficacy of ligand-regulated CAR-mediated cytotoxicity.

### Example 30: CAR T cell dose schemes

A Nalm6-luciferase tumor model in female NSG mice was used to determine if various cell dose schemes lead to enhanced efficacy *in vivo.* The experimental setup was as described in Example 20 and Example 29. Groups 8 and 9 CAR T cells had been treated with 10uM tadalafil overnight prior to cells being frozen so they would express CAR at time of injection (see Example 29). Groups 10 and 11 received an additional CD19-CAR T cell dose on day 10 post tumor implantation. Mice received either tadalafil or vehicle, beginning 12 hours prior to CD19-CAR T injection, then once or twice a day, as indicated, at the listed concentrations. Body weights and tumor BLI measurements were recorded over time, with higher BLI measures indicating high Nalm6-luciferase tumor burden.

As shown in Figure 12, increased cell dose fractionated over 3 days led to increased anti-tumor efficacy as compared to a single cell infusion.

### Example 31: Comparison of OT-001455 and OT-001456in vivo

A Nalm6-luciferase tumor model in female NSG mice was used to compare PDE5-regulated CD19 CAR OT-001455 and OT-001456 efficacy *in vivo.* The experimental setup was as described in Example 20, Example 29, and Example 30.

As shown in Figure 13, OT-001455 showed increased efficacy but higher basal activity as compared to OT-001456.

### Example 32: Time course of CAR expression

To evaluate expansion time matched dose response and time course of ligand induced CAR expression, T cells were thawed and activated overnight in the presence of CD3/CD28 Dynabeads at a 3:1 bead:cell ratio. The following day, cells were transduced with OT-001407, OT-001456, and OT-001900, or empty vector. Cells were expanded by addition of fresh media over the course of 10 days, maintaining cells around 0.5×10^6 cells/mL. On day 7 of the expansion, an aliquot of cells was seeded into 96 well plates, the following day treated with a titration of tadalafil or vardenafil, then analyzed for CAR expression after a 24 hr incubation by FACS staining with 10ug/mL CD19-Fc.

EC50 values were calculated in Spotfire by fitting a 4-parameter curve to the % CAR+ cells in a Singlet |Live Cell gate versus the dose of ligand. As shown in Table 112, similar EC50 values were observed for OT-001456 and OT-001900 (PDE5-regulated CD19 CAR with eGFP).

**Table 112: EC50 values for PDE5-regulated CD19 CAR +/- GFP tag**

| **EC50 (uM)** | **Tadalafil** | **Vardenafil** |
|---|---|---|
| **OT-001456** | 1.08 | 0.20 |
| **GT-001900** | 0.84 | 0.13 |

As shown in Figure 14A and Figure 14B, saturating concentrations of ligand (Tadalafil in Figure 14A and Vardenafil in Figure 14B) resulted in similar time courses of CAR expression.

To evaluate the time course of reversible CAR expression following ligand removal, T cells were thawed and activated overnight in the presence of CD3/CD28 Dynabeads at a 3:1 bead:cell ratio. The following day, cells were transduced with 10uL lentivirus from OT-001906 (HA-tagged constitutive CAR) and OT-001903 (HA-tagged PDE5 DD CAR). Cells were expanded by addition of fresh media over the course of 10 days, maintaining cells around 0.5×10^6 cells/mL. T cells on day 6 of transduction/expansion were treated overnight with 10um ligand or vehicle control. Cells were washed 3x with T cell media, then stained for CAR expression using 10ug/mL CD19-Fc 0, 2, 4, and 24hr post-washout.

As shown in Figure 15, PDE5-regulated CD19 CAR expression on the surface of T cells drops rapidly following removal of ligand.

In order to demonstrate the reversible nature and ability for multiple rounds of dosing, T cells were thawed and activated overnight in the presence of CD3/CD28 Dynabeads at a 3:1 bead:cell ratio. The following day, cells were transduced with OT-001456. Cells were expanded by addition of fresh media over the course of 10 days, maintaining cells around 0.5×10^6 cells/mL. On day 6 of the expansion, an aliquot of cells was treated with 10uM tadalafil for 24 hours. The next day this sample was divided into 2 portions: Group 1 stayed in culture with ligand, and Group 2 was washed 3x and cultured in T cell conditioned media without ligand. On day 8 of the expansion, the washed Group 2 cells, along with an aliquot of Group 3 cells that had never been exposed to ligand, were stimulated with 10uM tadalafil for 24 hours. FACS staining with 1ug/mL of CD19-Fc was performed daily just prior to ligand addition or wash steps.

The results are shown in Figure 16, demonstrating that OT-001456 allows reversibility of CAR expression based on ligand exposure time course, along with the ability to perform multiple rounds of pulsed ligand dosing while maintaining equal expression levels.

### Example 33: N-terminal vs. C-terminal PDE5 DD configuration

Human donor T cells were thawed and activated overnight in the presence of 3:1 ratio bead:cell, then transduced with empty vector, OT-001456 (C-terminal PDE5 DD), or OT-001908 (N-terminal PDE5 with furin). Cells were expanded over the course of 10 days with addition of fresh media to maintain cells around 0.5×10^6cells/mL. On day 9, an aliquot of cells was removed, treated with 10uM tadalafil overnight, then stained with 10ug/mL CD19-Fc. The results are provided in Table 113, demonstrating that PDE5 DDs regulated CD19-CAR expression better when placed at the C-terminus.

**Table 113: % CAR+ Cells with N-terminal vs. C-terminal PDE5 DD**

| | **Vehicle (DMSO)** | **Ligand (10uM tadalafil)** |
|---|---|---|
| **Vector** | **0.10** | - |
| **OT-001456 (C-terminal)** | 3.20 | 60.8 |
| **OT-001908 (N-terminal)** | 6.80 | 6.9 |

## Claims

1. An effector module comprising:
(a) a stimulus response element (SRE), wherein the SRE is a destabilizing domain (DD), said DD comprising the cGMP-specific 3',5'-cyclic phosphodiesterasehPDE5 of SEQ ID NO:1, in whole or in part, wherein said whole or part is capable of binding to a stabilizing ligand and wherein the DD further comprises:
i) a mutation at amino acid position 732 (R732) of SEQ ID NO: 1 and
ii) a mutation at amino acid position 736 (F736) of SEQ ID NO: 1, selected from the group consisting of F736G, F736L, F736M, F736R, F736W, F736K, F736Q, F736E, F736S, F736P, F736V, F736I, F736C, F736Y, F736H, F736N, F736D, and F736T; and
(b) at least one payload, which is attached, appended or associated with said SRE; and
wherein the SRE is responsive to one or more stimuli.

2. The effector module of claim 1, wherein the mutations at positions R732 and F736 are selected from the group consisting of R732D, F736S; R732E, F736D; R732V, F736G; R732W, F736G; R732W, F736V; R732L, F736W; R732P, F736Q; R732S, F736G; R732T, F736P; R732M, F736H; R732Y, F736M; R732P, F736D; R732P, F736G; R732W, F736L; R732L, F736S; R732D, F736T; R732L, F736V; and R732G, F736V.

3. The effector module of claim 1 or claim 2, wherein the hPDE5 comprises the amino acid sequence of SEQ ID NO: 8721, 8722, 8723, 8724, 8725, 8726, 8727, 8729, 8730, 8731, 8732, 8733, 8734, 8735, 8736, 8737, 8738, or 8739.

4. The effector module of any one of claims 1-3, wherein the at least one payload is a chimeric antigen receptor (CAR).

5. The effector module of claim 4, wherein the CAR comprises:
(a) an extracellular target moiety;
(b) a hinge and transmembrane domain;
(c) an intracellular signaling domain; and
(d) optionally, one or more co-stimulatory domains.

6. The effector module of claim 4 or claim 5, wherein the CAR is a CD19 CAR.

7. The effector module of any one of claims 1-6, wherein the one or more stimuli comprises a small molecule and wherein the small molecule is selected from Tadalafil, Vardenafil, Sildenafil, Avanafil, Lodenafil, Mirodenafil, Udenafil, Benzamidenafil, Dasantafil, and Beminafil.

8. The effector module of claim 7, wherein the small molecule is:
a) Vardenafil; or
b) Sildenafil; or
c) Tadalafil.

9. A pharmaceutical composition comprising the effector module of any one of claims 1-8 and a pharmaceutically acceptable excipient.

10. A polynucleotide encoding the effector module of any one of claims 1-8.

11. A pharmaceutical composition comprising the polynucleotide of claim 10.

12. A vector comprising a polynucleotide of claim 10.

## Patentansprüche

1. Effektormodul, umfassend:
(a) ein Stimulus-Response-Element (SRE), wobei das SRE eine destabilisierende Domäne (DD) ist, wobei die DD die cGMPspezifische 3',5'-cyclische Phosphodiesterase hPDE5 von SEQ ID NO:1 ganz oder teilweise umfasst, wobei das Ganze oder der Teil in der Lage ist, an einen stabilisierenden Liganden zu binden und wobei die DD ferner Folgendes umfasst:
i) eine Mutation an Aminosäureposition 732 (R732) von SEQ ID NO:1 und
ii) eine Mutation an Aminosäureposition 736 (F736) von SEQ ID NO:1, ausgewählt aus der Gruppe bestehend aus F736G, F736L, F736M, F736R, F736W, F736K, F736Q, F736E, F736S, F736P, F736V, F736I, F736 6C, F736Y, F736H, F736N, F736D und F736T; und
(b) mindestens einer Nutzlast, die an das SRE gebunden, angehängt oder mit diesem assoziiert ist; und
wobei das SRE auf einen oder mehrere Reize anspricht.

2. Effektormodul nach Anspruch 1, wobei die Mutationen an den Positionen R732 und F736 ausgewählt sind aus der Gruppe bestehend aus R732D, F736S; R732E, F736D; R732V, F736G; R732W, F736G; R732W, F736V; R732L, F736W; R732P, F736Q; R732S, F736G; R732T, F736P; R732M, F736H; R732Y, F736M; R732P, F736D; R732P, F736G; R732W, F736L; R732L, F736S; R732D, F736T; R732L, F736V; und R732G, F736V.

3. Effektormodul nach Anspruch 1 oder Anspruch 2, wobei die hPDE5 die Aminosäuresequenz von SEQ ID NO: 8721, 8722, 8723, 8724, 8725, 8726, 8727, 8729, 8730, 8731, 8732, 8733, 8734, 8735, 8736, 8737, 8738 oder 8739 umfasst.

4. Effektormodul nach einem der Ansprüche 1-3, wobei die mindestens eine Nutzlast ein chimärer Antigenrezeptor (CAR) ist.

5. Effektormodul nach Anspruch 4, wobei der CAR Folgendes umfasst:
(a) eine extrazelluläre Zieleinheit;
(b) eine Scharnier- und Transmembrandomäne;
(c) eine intrazelluläre Signaldomäne; und
(d) optional eine oder mehrere kostimulatorische Domänen.

6. Effektormodul nach Anspruch 4 oder Anspruch 5, wobei der CAR ein CD19-CAR ist.

7. Effektormodul nach einem der Ansprüche 1-6, wobei der eine oder die mehreren Reize eine niedermolekulare Verbindung umfassen und wobei die niedermolekulare Verbindung aus Tadalafil, Vardenafil, Sildenafil, Avanafil, Lodenafil, Mirodenafil, Udenafil, Benzamidenafil, Dasantafil und Beminafil ausgewählt ist.

8. Effektormodul nach Anspruch 7, wobei die niedermolekulare Verbindung Folgendes ist:
a) Vardenafil oder
b) Sildenafil; oder
c) Tadalafd.

9. Pharmazeutische Zusammensetzung, umfassend das Effektormodul nach einem der Ansprüche 1-8 und einen pharmazeutisch annehmbaren Hilfsstoff.

10. Polynukleotid, das für das Effektormodul nach einem der Ansprüche 1-8 codiert.

11. Pharmazeutische Zusammensetzung, umfassend das Polynukleotid nach Anspruch 10.

12. Vektor, umfassend ein Polynukleotid nach Anspruch 10.

## Revendications

1. Module effecteur comprenant :
(a) un élément de réponse de stimulus (SRE), dans lequel le SRE est un domaine déstabilisant (DD), ledit DD comprenant la phosphodiestérase cyclique 3',5'-spécifique de cGMP hPDE5 de SEQ ID NO:1, en totalité ou en partie, dans lequel ladite totalité ou partie est capable de se lier à un ligand stabilisant et dans lequel le DD comprend en outre :
i) une mutation en position d'acide aminé 732 (R732) de SEQ ID NO:1 et
ii) une mutation en position d'acide aminé 736 (F736) de SEQ ID NO:1, choisie dans le groupe constitué par F736G, F736L, F736M, F736R, F736W, F736K, F736Q, F736E, F736S, F736P, F736V, F736I, F736C, F736Y, F736H, F736N, F736D, F736T, et ;
(b) au moins une charge utile, qui est rattachée, annexée ou associée audit SRE ; et
dans lequel le SRE est sensible à un ou plusieurs stimuli.

2. Module effecteur selon la revendication 1, dans lequel les mutations aux positions R732 et F736 sont choisies dans le groupe constitué par R732D, F736S ; R732E, F736D ; R732V, F736G ; R732W, F736G ; R732W, F736V ; R732L, F736W ; R732P, F736Q ; R732S, F736G ; R732T, F736P ; R732M, F736H ; R732Y, F736M ; R732P, F736D ; R732P, F736G ; R732W, F736L ; R732L, F736S ; R732D, F736T ; R732L, F736V ; et R732G, F736V.

3. Module effecteur selon la revendication 1 ou la revendication 2, dans lequel la hPDE5 comprend la séquence d'acides aminés SEQ ID NO : 8721, 8722, 8723, 8724, 8725, 8726, 8727, 8729, 8730, 8731, 8732, 8733, 8734, 8735, 8736, 8737, 8738 ou 8739.

4. Module effecteur selon l'une quelconque des revendications 1 à 3, dans lequel l'au moins une charge utile est un récepteur antigénique chimérique (CAR).

5. Module effecteur selon la revendication 4, dans lequel le CAR comprend :
(a) un groupement cible extracellulaire ;
(b) un domaine charnière et transmembranaire ;
(c) un domaine de signalisation intracellulaire ; et
(d) éventuellement, un ou plusieurs domaines costimulateurs.

6. Module effecteur selon la revendication 4 ou la revendication 5, dans lequel le CAR est un CAR de CD19.

7. Module effecteur selon l'une quelconque des revendications 1 à 6, dans lequel l'un ou les plusieurs stimulis comprennent une petite molécule et dans lequel la petite molécule est choisie parmi le Tadalafil, le Vardénafil, le Sildénafil, l'Avanafil, le Lodénafil, le Mirodénafil, l'Udénafil, le Benzamidenafil, le Dasantafil et le Beminafil.

8. Module effecteur selon la revendication 7, dans lequel la petite molécule est :
a) Vardénafil ; ou
b) Sildénafil ; ou
c) Tadalafil.

9. Composition pharmaceutique comprenant le module effecteur selon l'une quelconque des revendications 1 à 8, et un support pharmaceutiquement acceptable.

10. Polynucléotide codant pour le module effecteur selon l'une quelconque des revendications 1 à 8.

11. Composition pharmaceutique comprenant le polynucléotide selon la revendication 10.

12. Vecteur comprenant un polynucléotide selon la revendication 10.
